# EUROPEAN PATENT SPECIFICATION

(11) **EP 1 268 775 B1**
(45) Date of publication and mention of the grant of the patent: **19.11.2008**
(21) Application number: 00941578.7
(22) Date of filing: 21.06.2000
(51) Int. Cl.: C12N 15/12, C12N 15/11, C07K 14/705, A61K 38/17, A61K 48/00, A61K 49/00

(54) **THE HIGH BONE MASS GENE OF 11Q13.3**
DAS HOHE KNOCHENMASSE GEN AUF 11Q13.3
LE GENE DE LA MASSE OSSEUSE ELEVEE DE 11Q13.3

(30) Priority: 05.04.2000 US 543771; 05.04.2000 US 544398
(43) Date of publication of application: 02.01.2003
(60) Divisional application: 08006373.8
(73) Proprietor: Oscient Pharmaceuticals Corporation, Waltham, MA 02415 (US); CREIGHTON UNIVERSITY, Omaha, NE 68178 (US)
(72) Inventor: CARULLI, John, P., Southboro, MA 01772 (US); LITTLE, Randall, D., Newtonville, MA 02460-2118 (US); RECKER, Robert, R., Omaha, NE 68144 (US); JOHNSON, Mark, L., Omaha, NE 68135 (US)
(74) Representative: Duckworth, Timothy John
(86) International application number: PCT/US2000/016951
(87) International publication number: WO 2001/077327

(56) References cited:
- WO-A-00/58496
- WO-A-01/92891
- WO-A-98/46743
- DONG Y ET AL: "MOLECULAR CLONING AND CHARACTERIZATION OF LR3, A NOVEL LDL RECEPTOR FAMILY PROTEIN WITH MITOGENIC ACTIVITY" BIOCHEMICAL AND BIOPHYSICAL RESEARCH COMMUNICATIONS, ACADEMIC PRESS INC. ORLANDO, FL, US, vol. 251, no. 3, 29 October 1998 (1998-10-29), pages 784-790, XP001076809 ISSN: 0006-291X
- HEY P J ET AL: "CLONING OF A NOVEL MEMBER OF THE LOW-DENSITY LIPOPROTEIN RECEPTOR FAMILY" GENE: AN INTERNATIONAL JOURNAL ON GENES AND GENOMES, ELSEVIER SCIENCE PUBLISHERS, BARKING, GB, vol. 216, 1998, pages 103-111, XP002952911 ISSN: 0378-1119
- TROMMSDORFF M ET AL: "Interaction of cytosolic adaptor proteins with neuronal apolipoprotein E receptors and the amyloid precursor protein" JOURNAL OF BIOLOGICAL CHEMISTRY, AMERICAN SOCIETY OF BIOLOGICAL CHEMISTS, BALTIMORE, MD, US, vol. 273, no. 50, 11 December 1998 (1998-12-11), pages 33556-33560, XP002165275 ISSN: 0021-9258

## Description

### FIELD OF THE INVENTION

The present invention relates generally to the field of genetics, genomics and molecular biology. More particularly, the invention relates to methods and materials used to isolate, detect and sequence a high bone mass gene and corresponding wild-type gene, and mutants thereof. The present invention also relates to the high bone mass gene, the corresponding wild-type gene, and mutants thereof. The genes identified in the present invention are implicated in the ontology and physiology of bone development. The invention also provides nucleic acids, proteins, cloning vectors, expression vectors, tramformed non-human hosts, methods of developing pharmaceutical compositions, methods of identifying molecules involved in bone development, and methods of diagnosing and manufacturing medicaments for treating diseases involved in bone development. In preferred embodiment, the present invention is directed to methods for manufacturing medicamentss for treating, diagnosing, preventing and screening for normal and abnormal conditions of bone, including metabolic bone diseases such as osteoporosis.

### BACKGROUND OF THE INVENTION

Two of the most common types of osteoporosis are postmenopausal and senile osteoporosis. Osteoporosis affects men as well as women, and, taken with other abnormalities of bone, presents an ever-increasing health risk for an aging population. The most common type of osteoporosis is that associated with menopause. Most women lose between 20-60% of the bone mass in the trabecular compartment of the bone within 3-6 years after the cessation of menses. This rapid loss is generally associated with an increase of bone resorption and formation. However, the resorptive cycle is more dominant and the result is a net loss of bone mass. Osteoporosis is a common and serious disease among postmenopausal women. There are an estimated 25 million women in the United States alone who are afflicted with this disease. The results of osteoporosis are both personally harmful, and also account for a large economic loss due to its chronicity and the need for extensive and long-term support (hospitalization and nursing home care) from the disease sequelae. This is especially true in more elderly patients. Additionally, while osteoporosis is generally not thought of as a life-threatening condition, a 20-30% mortality rate is related to hip fractures in elderly women. A large percentage of this mortality rate can be directly associated with postmenopausal osteoporosis.

The most vulnerable tissue in the bone to the effects of postmenopausal osteoporosis is the trabecular bone. This tissue is often referred to as spongy bone and is particularly concentrated near the ends of the bone near the joints and in the vertebrae of the spine. The trabecular tissue is characterized by small structures which inter-connect with each other as well as the more solid and dense cortical tissue which makes up the outer surface and central shaft of the bone. This criss-cross network of trabeculae gives lateral support to the outer cortical structure and is critical to the biomechanical strength of the overall structure. In postmenopausal osteoporosis, it is primarily the net resorption and loss of the trabeculae which lead to the failure and fracture of the bone. In light of the loss of the trabeculae in postmenopausal women, it is not surprising that the most common fractures are those associated with bones which are highly dependent on trabecular support, e.g., the vertebrae, the neck of the femur, and the forearm. Indeed, hip fracture, Colle's fractures, and vertebral crush fractures are indicative of postmenopausal osteoporosis.

One of the earliest generally accepted methods for treatment of postmenopausal osteoporosis was estrogen replacement therapy. Although this therapy frequently is successful, patient compliance is low, primarily due to the undesirable side-effects of chronic estrogen treatment. Frequently cited side-effects of estrogen replacement therapy include reinitiation of menses, bloating, depression, and fear of breast or uterine cancer. In order to limit the known threat of uterine cancer in those women who have not undergone a hysterectomy, a protocol of estrogen and progestin cyclic therapy is often employed. This protocol is similar to that which is used in birth control regimens, and often is not tolerated by women because of the side-effects characteristic of progestin. More recently, certain antiestrogens, originally developed for the treatment of breast cancer, have been shown in experimental models of postmenopausal osteoporosis to be efficacious. Among these agents is raloxifene (See, U.S. Patent No. 5,393,763, and Black et al, J. Clin. Invest., 93:63-69 (1994)). In addition, tamoxifene, a widely used clinical agent for the treatment of breast cancer, has been shown to increase bone mineral density in post menopausal women suffering from breast cancer (Love et al, N. Engl. J. Med., 326:852-856 (1992)).

Another therapy for the treatment of postmenopausal osteoporosis is the use of calcitonin. Calcitonin is a naturally occurring peptide which inhibits bone resorption and has been approved for this use in many countries (Overgaard et al, Br. Med. J., 305:556-561 (1992)). The use of calcitonin has been somewhat limited, however. Its effects are very modest in increasing bone mineral density and the treatment is very expensive. Another therapy for the treatment of postmenopausal osteoporosis is the use of bis-phosphonates. These compounds were originally developed for use in Paget's disease and malignant hypercalcemia. They have been shown to inhibit bone resorption. Alendronate, one compound of this class, has been approved for the treatment of postmenopausal osteoporosis. These agents may be helpful in the treatment of osteoporosis, but these agents also have potential liabilities which include osteomalacia, extremely long half-life in bone (greater than 2 years), and possible "frozen bone syndrome," e.g., the cessation of normal bone remodeling.

Senile osteoporosis is similar to postmenopausal osteoporosis in that it is marked by the loss of bone mineral density and resulting increase in fracture rate, morbidity, and associated mortality. Generally, it occurs in later life, i.e., after 70 years of age. Historically, senile osteoporosis has been more common in females, but with the advent of a more elderly male population, this disease is becoming a major factor in the health of both sexes. It is not clear what, if any, role hormones such as testosterone or estrogen have in this disease, and its etiology remains obscure. Treatment of this disease has not been very satisfactory. Hormone therapy, estrogen in women and testosterone in men, has shown equivocal results; calcitonin and bis-phosphonates may be of some utility.

The peak mass of the skeleton at maturity is largely under genetic control. Twin studies have shown that the variance in bone mass between adult monozygotic twins is smaller than between dizygotic twins (Slemenda et al, J. Bone Miner. Res., 6:561-567 (1991); Young et al, J. Bone Miner. Res., 6:561-567 (1995); Pocock et al, J. Clin. Invest., 80:706-710 (1987); Kelly et al, J. Bone Miner. Res., 8:11-17 (1993)), and it has been estimated that up to 60% or more of the variance in skeletal mass is inherited (Krall et al, J. Bone Miner. Res., 10:S367 (1993)). Peak skeletal mass is the most powerful determinant of bone mass in elderly years (Hui et al, Ann. Int. Med., 111:355-361 (1989)), even though the rate of age-related bone loss in adult and later life is also a strong determinant (Hui et al, Osteoporosis Int., 1:30-34 (1995)). Since bone mass is the principal measurable determinant of fracture risk, the inherited peak skeletal mass achieved at maturity is an important determinant of an individual's risk of fracture later in life. Thus, study of the genetic basis of bone mass is of considerable interest in the etiology of fractures due to osteoporosis.

Recently, a strong interest in the genetic control of peak bone mass has developed in the field of osteoporosis. The interest has focused mainly on candidate genes with suitable polymorphisms to test for association with variation in bone mass within the normal range, or has focused on examination of genes and gene loci associated with low bone mass in the range found in patients with osteoporosis. The vitamin D receptor locus (VDR) (Morrison et al, Nature, 367:284-287 (1994)), PTH gene (Howard et al, J. Clin. Endocrinol. Metab., 80:2800-2805 (1995); Johnson et al, J. Bone Miner. Res., 8:11-17 (1995); Gong et al, J. Bone Miner. Res., 10:S462 (1995)) and the estrogen receptor gene (Hosoi et al, J. Bone Miner. Res., 10:S170 (1995); Morrison et al, Nature, 367:284-287 (1994)) have figured most prominently in this work. These studies are difficult because bone mass (the phenotype) is a continuous, quantitative, polygenic trait, and is confounded by environmental factors such as nutrition, co-morbid disease, age, physical activity, and other factors. Also, this type of study design requires large numbers of subjects. In particular, the results of VDR studies to date have been confusing and contradictory (Garnero et al, J. Bone Miner. Res., 10:1283-1288 (1995); Eisman et al, J. Bone. Miner. Res., 10:1289-1293 (1995); Peacock, J. Bone Miner. Res., 10:1294-1297 (1995)). Furthermore, the work thus far has not shed much light on the mechanism(s) whereby the genetic influences might exert their effect on bone mass.

While it is well known that peak bone mass is largely determined by genetic rather than environmental factors, studies to determine the gene loci (and ultimately the genes) linked to variation in bone mass are difficult and expensive. Study designs which utilize the power of linkage analysis, e.g., sib-pair or extended family, are generally more informative than simple association studies, although the latter do have value. However, genetic linkage studies involving bone mass are hampered by two major problems. The first problem is the phenotype, as discussed briefly above. Bone mass is a continuous, quantitative trait, and establishing a discrete phenotype is difficult. Each anatomical site for measurement may be influenced by several genes, many of which may be different from site to site. The second problem is the age component of the phenotype. By the time an individual can be identified as having low bone mass, there is a high probability that their parents or other members of prior generations will be deceased and therefore unavailable for study, and younger generations may not have even reached peak bone mass, making their phenotyping uncertain for genetic analysis.

Regardless, linkage analysis can be used to find the location of a gene causing a hereditary "disorder" and does not require any knowledge of the biochemical nature of the disorder, i.e., a mutated protein that is believed to cause the disorder does not need to be known. Traditional approaches depend on assumptions concerning the disease process that might implicate a known protein as a candidate to be evaluated. The genetic localization approach using linkage analysis can be used to first find the general chromosomal region in which the defective gene is located and then to gradually reduce the size of the region in order to determine the location of the specific mutated gene as precisely as possible. After the gene itself is discovered within the candidate region, the messenger RNA and the protein are identified and, along with the DNA, are checked for mutations.

The genetic localization approach has practical implications since the location of the disease can be used for prenatal diagnosis even before the altered gene that causes the disease is found. Linkage analysis can enable families, even many of those that do not have a sick child, to know whether they are carriers of a disease gene and to evaluate the condition of an unborn child through molecular diagnosis. The transmission of a disease within families, then, can be used to find the defective gene. As used herein, reference to "high bone mass" (HBM) is analogous to reference to a disease state, although from a practical standpoint high bone mass can actually help a subject avoid the disease known as osteoporosis.

Linkage analysis is possible because of the nature of inheritance of chromosomes from parents to offspring. During meiosis, the two parental homologues pair to guide their proper separation to daughter cells. While they are lined up and paired, the two homologues exchange pieces of the chromosomes, in an event called "crossing over" or "recombination." The resulting chromosomes are chimeric, that is, they contain parts that originate from both parental homologues. The closer together two sequences are on the chromosome, the less likely that a recombination event will occur between them, and the more closely linked they are. In a linkage analysis experiment, two positions on the chromosomes are followed from one generation to the next to determine the frequency of recombination between them. In a study of an inherited disease, one of the chromosomal positions is marked by the disease gene or its normal counterpart, i.e., the inheritance of the chromosomal region can be determined by examining whether the individual displays symptoms of the disorder or not. The other position is marked by a DNA sequence that shows natural variation in the population such that the two homologues can be distinguished based on the copy of the "marker" sequence that they possess. In every family, the inheritance of the genetic marker sequence is compared to the inheritance of the disease state. If, within a family carrying an autosomal dominant disorder such as high bone mass, every affected individual carries the same form of the marker and all the unaffected individuals carry at least one different form of the marker, there is a great probability that the disease gene and the marker are located close to each other. In this way, chromosomes may be systematically checked with known markers and compared to the disease state. The data obtained from the different families is combined, and analyzed together by a computer using statistical methods. The result is information indicating the probability of linkage between the genetic marker and the disease allowing different distances between them. A positive result can mean that the disease is very close to the marker, while a negative result indicates that it is far away on that chromosome, or on an entirely different chromosome.

Linkage analysis is performed by typing all members of the affected family at a given marker locus and evaluating the co-inheritance of a particular disease state with the marker probe, thereby determining how often the two of them are co-inherited. The recombination frequency can be used as a measure of the genetic distance between two gene loci. A recombination frequency of 1% is equivalent to 1 map unit, or 1 centiMorgan (cM), which is roughly equivalent to 1,000 kb of DNA. This relationship holds up to frequencies of about 20% or 20 cM.

The entire human genome is 3,300 cM long. In order to find an unknown disease gene within 5-10 cM of a marker locus, the whole human genome can be searched with roughly 330 informative marker loci spaced at approximately 10 cM intervals (Botstein et al, Am. J. Hum. Genet., 32:314-331 (1980)). The reliability of linkage results is established by using a number of statistical methods. The method most commonly used for the analysis of linkage in humans is the LOD score method (Morton, Prog. Clin. Biol. Res., 147:245-265 (1984), Morton et al, Am. J. Hum. Genet., 38:868-883 (1986)) which was incorporated into the computer program LIPED by Ott, Am. J. Hum. Genet., 28:528-529 (1976). LOD scores are the logarithm of the ratio of the likelihood that two loci are linked at a given distance to that they are not linked (>50 cM apart). The advantage of using logarithmic values is that they can be summed among families with the same disease. This becomes necessary given the relatively small size of human families.

By convention, a total LOD score greater than + 3.0 (that is, odds of linkage at the specified recombination frequency being 1000 times greater than odds of no linkage) is considered to be significant evidence for linkage at that particular recombination frequency. A total LOD score of less than - 2.0 (that is, odds of no linkage being 100 times greater than odds of linkage at the specified frequency) is considered to be strong evidence that the two loci under consideration are not linked at that particular recombination frequency. Until recently, most linkage analyses have been performed on the basis of two-point data, which is the relationship between the disorder under consideration and a particular genetic marker. However, as a result of the rapid advances in mapping the human genome over the last few years, and concomitant improvements in computer methodology, it has become feasible to carry out linkage analyses using multi-point data. Multi-point analysis provide a simultaneous analysis of linkage between the disease and several linked genetic markers, when the recombination distance among the markers is known.

Multi-point analysis is advantageous for two reasons. First, the informativeness of the pedigree is usually increased. Each pedigree has a certain amount of potential information, dependent on the number of parents heterozygous for the marker loci and the number of affected individuals in the family. However, few markers are sufficiently polymorphic as to be informative in all those individuals. If multiple markers are considered simultaneously, then the probability of an individual being heterozygous for at least one of the markers is greatly increased. Second, an indication of the position of the disease gene among the markers may be determined. This allows identification of flanking markers, and thus eventually allows isolation of a small region in which the disease gene resides. Lathrop et al, Proc. Natl. Acad. Sci. USA, 81:3443-3446 (1984) have written the most widely used computer package, LINKAGE, for multi-point analysis. WO 98/46743 is concerned with an LDL receptor. Schneider et al (1994) Exp. Cell Res., 214 (1):264-269 is concerned with formation by focal adhesions by ostroblasts adhering to different substrata.

There is a need in the art for identifying the gene associated with a high bone mass phenotype. The present invention is directed to this, as well as other, important ends.

### SUMMARY OF THE INVENTION

The present invention describes the Zmax1 gene and the HBM gene on chromosome 11q13.3 by genetic linkage and mutation analysis. The use of additional genetic markers linked to the genes has aided this discovery. By using linkage analysis and mutation analysis, persons predisposed to HBM may be readily identified. Cloning methods using Bacterial Artificial Chromosomes have enabled the inventors to focus on the chromosome region of 11q13.3 and to accelerate the sequencing of the autosomal dominant gene. In addition, the invention identifies the Zmax1 gene and the HBM gene, and identifies the guanine-to-thymine polymorphism mutation at position 582 in the Zmax1 gene that produces the HBM gene and the HBM phenotype.

The present invention identifies the Zmax1 gene and the HBM gene, which can be used to determine if people are predisposed to HBM and, therefore, not susceptible to diseases characterized by reduced bone density, including, for example, osteoporosis, or are predisposed and susceptible to diseases characterized by abnormally high bone density, such as, for example, osteoporosis. Older individuals carrying the HBM gene express the HBM protein, and, therefore, do not develop osteoporosis. In other words, the HBM gene is a suppressor of osteoporosis. This *in vivo* observation is a strong evidence that treatment of normal individuals with the HBM gene or protein, or fragments thereof, will ameliorate osteoporosis.

Moreover, such treatment will be indicated in the treatment of bone lesions, particularly bone fractures, for bone remodeling in the healing of such lesions. For example, persons predisposed to or suffering from stress fractures (i.e., the accumulation of stress-induced microfractures, eventually resulting in a true fracture through the bone cortex) may be identified and/or treated by means of the medicaments of the invention. Moreover, the medicaments and compositions of the invention will be of use in the treatment of secondary osteoporosis, where the course of therapy involves bone remodeling, such as endocrine conditions accompanying corticosteroid administration, hyperthyroidism, hypogonadism, hematologic malignancies, malabsorption and alcoholism, as well as disorders associated with vitamin D and/or phosphate metabolism, such as osteomalacia and rickets, and diseases characterized by abnormal or disordered bone remodeling, such as Paget's disease, and in neoplasms of bone, which may be benign or malignant.

In various embodiments, the present invention is directed to nucleic acids, proteins, vectors, and transformed non-human hosts of HBM and Zmax 1.

Additionally, the present invention is directed to applications of the above embodiments of the invention including, for example, medicaments for gene therapy, pharmaceutical development, and diagnostic assay for bone development disorders. In preferred embodiments, the present invention is directed to medicaments for treating, diagnosing, preventing and screening for osteoporosis.

Thus, the present invention provides:
- an isolated nucleic acid sequence of SEQ ID NO: 2;
- an isolated amino acid sequence of SEQ ID NO: 4;
- a nucleic acid sequence encoding the amino acid sequence of SEQ ID NO: 4;
- a replicative cloning vector comprising the nucleic acid sequence of the present invention and a replicon operative in an isolated host cell;
- an isolated host cell transformed with the replicative cloning vector of the invention;
- an expression vector comprising the nucleic acid sequence of the invention operably linked to a transcription regulatory region; and
- an isolated host cell transformed with the expression vector of the invention. The present invention additionally provides:

- an *in vitro* method for testing a substance as a therapeutic agent for bone modulation in a host comprising administering the nucleic acid of the invention to a host cell, and assessing whether bone modulation occurs;
- a method of identifying a molecule involved in bone modulation comprising identifying a molecule that binds to, or that inhibits binding of a molecule to, an isolated amino acid sequence of SEQ ID NO: 4 or an amino acid sequence encoded by the nucleic acid sequence of SEQ ID NO:2;
- a method for identifying a protein involved in bone modulation comprising identifying a protein that has an expression level that is different in a first host comprising the Zmax 1 gene of SEQ ID NO: 1 when compared to a second host comprising a nucleic acid sequence of SEQ ID NO: 2 or a nucleic acid sequence encoding the amino acid sequence of SEQ ID NO: 4; and
- a method of testing for high bone mass (HBM) activity comprising immobilizing an HBM protein of SEQ ID NO: 4, binding a protein to the

HBM protein, and measuring the extent of binding.

In a further embodiment, the invention provides a method for identification of a candidate molecule involved in bone modulation comprising
identifying a molecule that binds to, that inhibits binding of a molecule to, the nucleic acid sequence of SEQ ID NO: 1;
identifying a molecule that binds to, or that inhibits binding of a molecule to, the nucleic acid sequence of SEQ ID NO: 2; and
comparing the extent of binding, or the extent of inhibition of binding of the molecule to each nucleic acid sequence, wherein the molecule that binds, or inhibits binding, more or less to the nucleic acid sequence of SEQ ID NO: 2 or the nucleic acid sequence of SEQ ID NO: 1 is the candidate molecule.

The present invention also provides an *in vitro* method of pharmaceutical development for treatment of bone development disorders comprising identifying a molecule that binds to the amino acid sequence of SEQ ID NO: 4.

Additionally, the invention provides an *in vitro* method of pharmaceutical development for treatment of bone development disorders the method comprising
- constructing a first host cell that contains the Zmax1 gene of SEQ ID NO: 1 or protein of SEQ ID NO: 3;
- constructing a second host cell that contains a nucleic acid according to SEQ ID NO: 2 or a protein according to SEQ ID NO: 4;
- analyzing a difference between the first host cell and the second host cell;
- identifying a molecule that, when added to the first host cell, causes the first host cell to exhibit a characteristic feature of the second host cell.

In further embodiments, the invention provides:
- a method for diagnostic screening for a genetic predisposition to a bone development disorder comprising screening a sample form a patient with a nucleic acid sequence of SEQ ID NO: 2 or a nucleic acid sequence encoding the amino acid sequence of SEQ ID NO:4
- a diagnostic assay for bone development disorders comprising an antibody to an amino acid sequence according to SEQ ID NO: 4 or an amino acid sequence encoded by a nucleic acid sequence of SEQ ID NO: 2;
- an isolated nucleic acid sequence comprising at least 15 contiguous nucleotides of SEQ ID NO: 2, wherein said 15 contiguous nucleotides comprise position 582 of SEQ ID NO: 2;
- an isolated nucleic acid sequence comprising at least 15 contiguous nucleotides of SEQ ID NO: 2, wherein said 15 contiguous nucleotides comprise position 582 of SEQ ID NO: 2, and which encodes for an amino acid sequence including a valine corresponding to valine at position 171 of SEQ ID NO: 4 and wherein valine substitution is associated with a high bone mass phenotype.;
- an isolated nucleic acid segment of at least 15 contiguous nucleotides including a polymorphic site from the nucleic acid sequence of SEQ ID NO: 2 in which G at position 582 is replaced by T, and sequences complementary thereto; and
- a method of identifying a molecule involved in bone modulation comprising identifying a molecule that binds to the HBM polypeptide of SEQ ID NO: 4.

The invention further provides an isolated high bone mass (HBM) polynucleotide comprising a nucleic acid selected from the group consisting of:
(a) a nucleic acid having SEQ ID NO: 2;
(b) a nucleic acid encoding a polypeptide of SEQ ID NO: 4;
(c) a nucleic acid which is complementary to a nucleic acid of (a), or (b);
(d) a nucleic acid comprising at least 15 contiguous nucleotides which comprise position 582 of SEQ ID NO: 2 from a polynucleotide of (a), (b), or (c);
wherein said isolated HBM polynucleotide encodes a protein or polypeptide, which when administered to a subject modulates bone mass.

Additionally, the invention provides an isolated nucleic acid sequence comprising at least 15 contiguous nucleotides of SEQ ID NO: 2, wherein said 15 contiguous nucleotides comprise nucleotides which encode for an amino acid substitution at position 171 of SEQ ID NO: 4, which substitution is associated with a high bone mass phenotype.

The invention also provides an isolated high bone mass (HBM) polynucleotide comprising a nucleic acid selected from the group consisting of:
(a) a nucleic acid having SEQ ID NO: 2;
(b) a nucleic acid encoding a polypeptide of SEQ ID NO:4;
(c) a nucleic acid which is complementary to a nucleic acid of (a), (b); and
(d) a nucleic acid comprising at least 15 contiguous nucleotides which comprise position 582 of SEQ ID NO: 2 from a polynucleotide of (a), (b), and (c).

In addition, the present invention provides for:
- the use of an antibody that binds to a polypeptide encoded by a nucleic acid of the invention involved in focal adhesion signaling, for the manufacture of a medicament for modulating bone density; and
- the use of an antibody which binds to a polypeptide encoded by a nucleic acid of the invention involved in focal adhesion signaling, for the manufacture of a medicament for treating bone development disorders.

### BRIEF DESCRIPTION OF THE FIGURES

**Fig. 1** shows the pedigree of the individuals used in the genetic linkage studies. Under each individual is an ID number, the z-score for spinal BMD, and the allele calls for the critical markers on chromosome solid symbols represent"affected"individuals. Symbols containing"N"are"unaffected"individuals. DNA from 37 individuals was genotyped. Question marks denote unknown genotypes or individuals who were not genotyped.

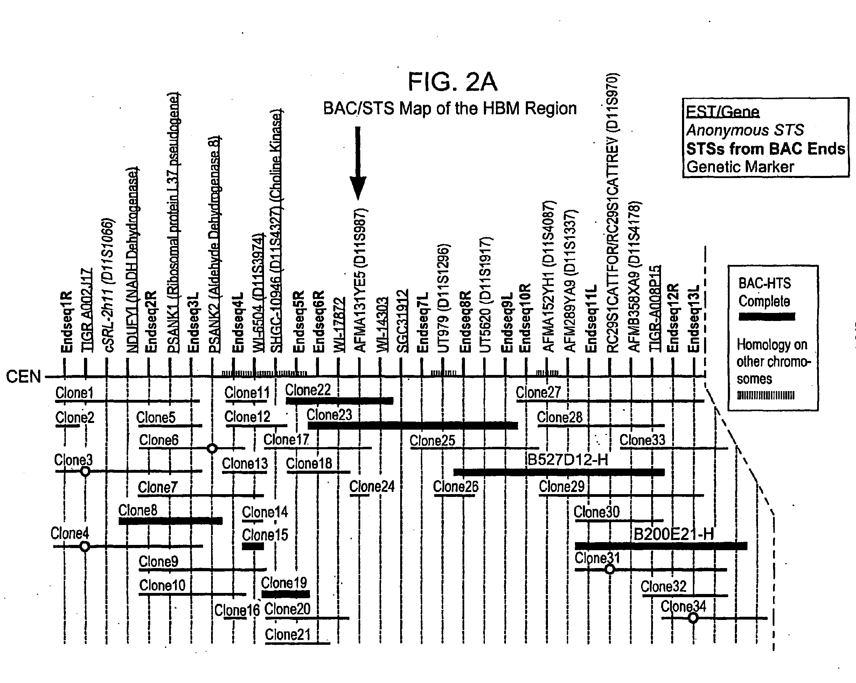

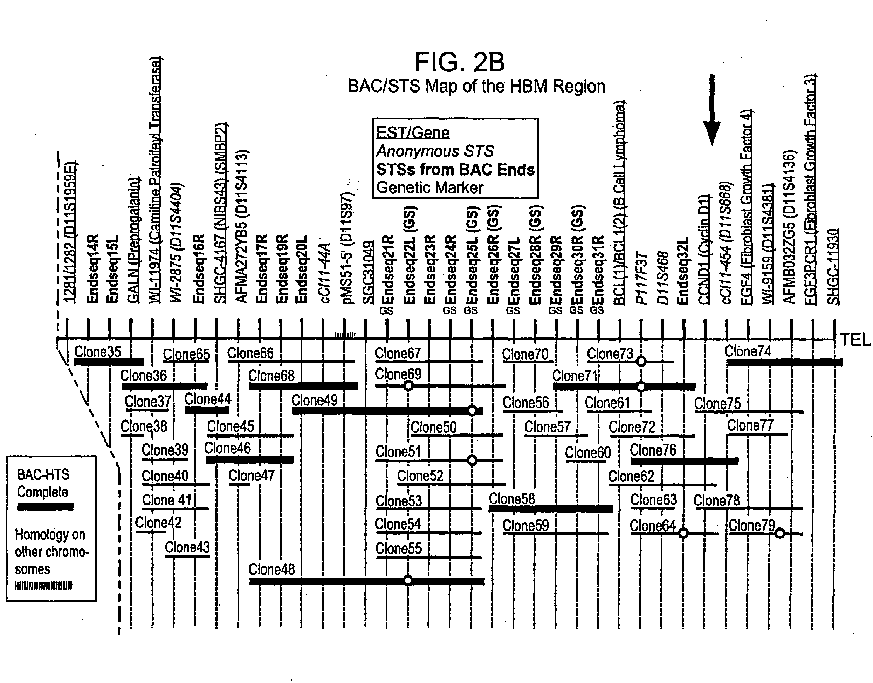

**Fig. 2** depicts the BAC/STS content physical map of the HBM region in 11q13.3. STS markers derived from genes, ESTs, microsatellites, random sequences, and BAC endsequences are denoted above the long horizontal line. For markers that are present in GDB the same nomenclature has been used. Locus names (D11S####) are listed in parentheses after the primary name if available. STSs derived from BAC endsequences are listed with the BAC name first followed by L or R for the left and right end of the clone, respectively. The two large arrows indicate the genetic markers that define the HBM critical region. The horizontal lines below the STSs indicate BAC clones identified by PCR-based screening of a nine-fold coverage BAC library. Open circles indicate that the marker did not amplify the corresponding BAC library address during library screening. Clone names use the following convention: B for BAC, the plate, row and column address, followed by -H indicating the HBM project (i.e.,B36F16-H).

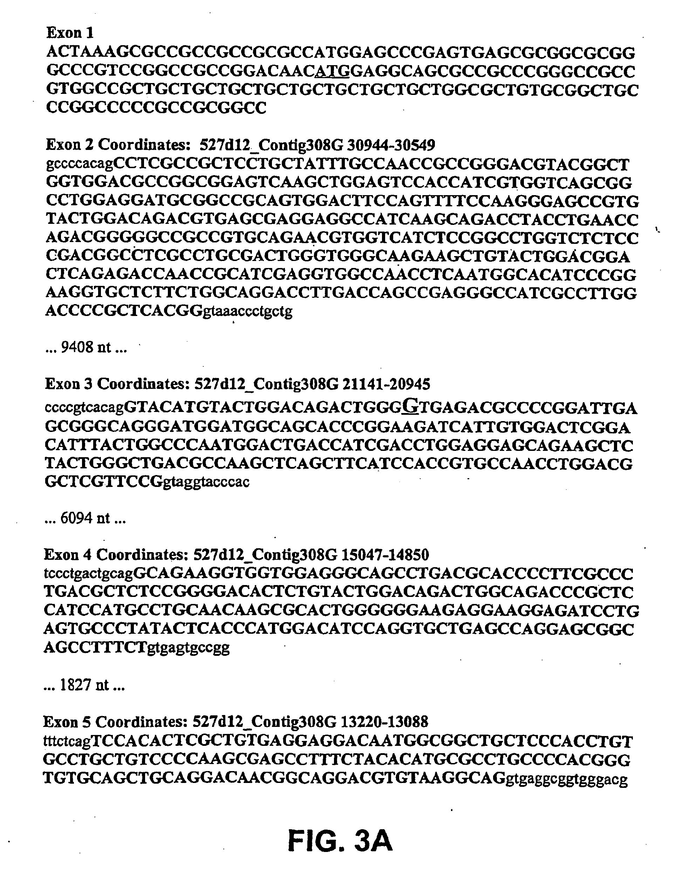

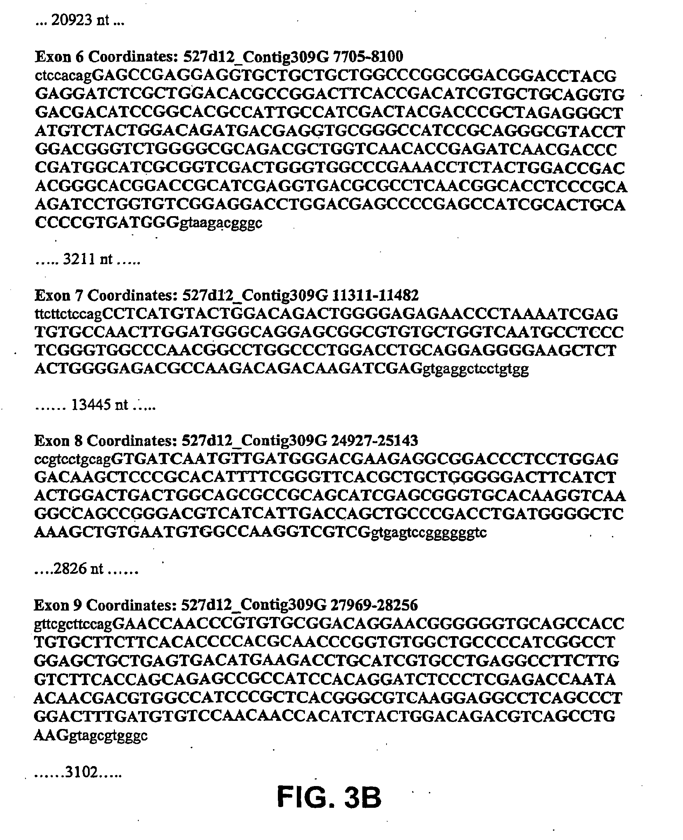

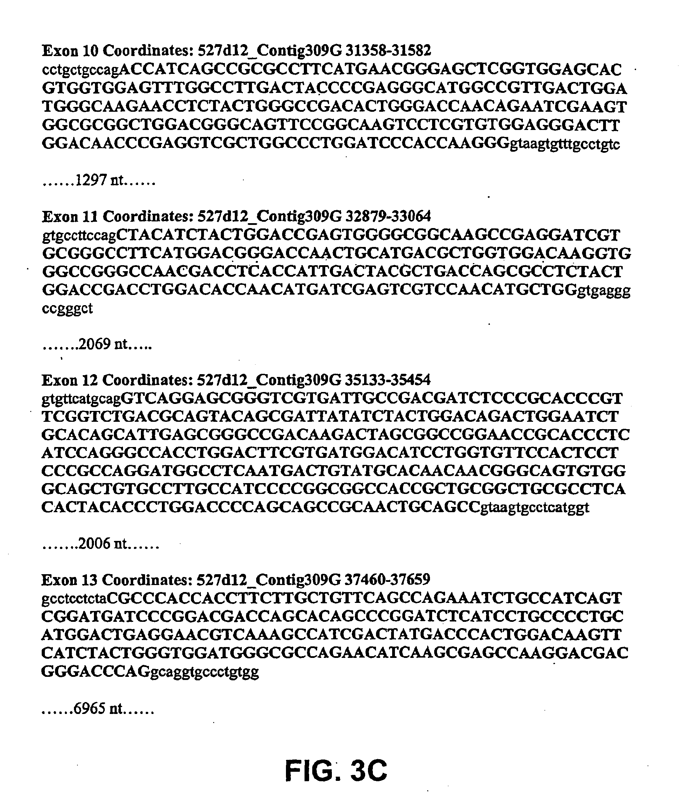

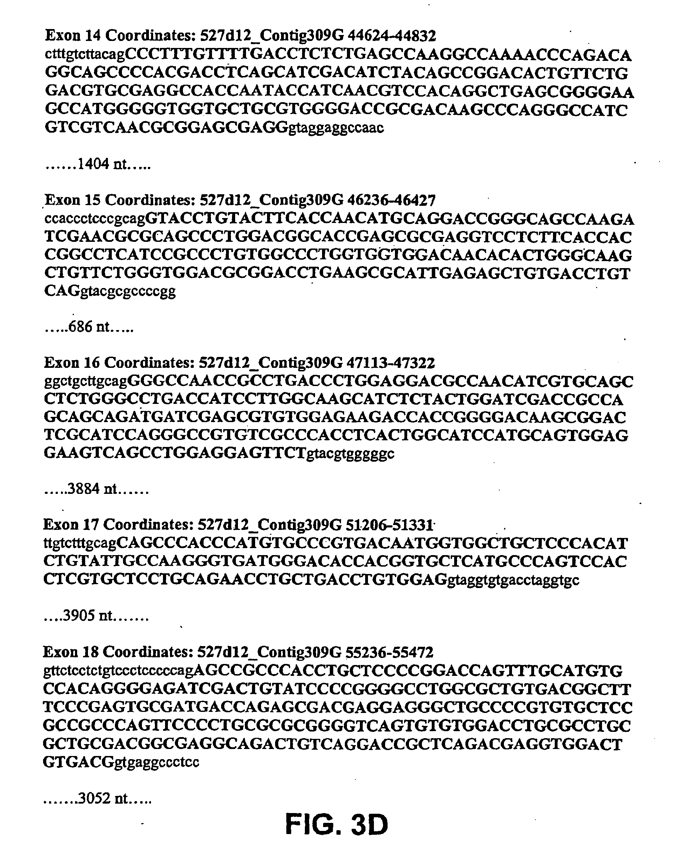

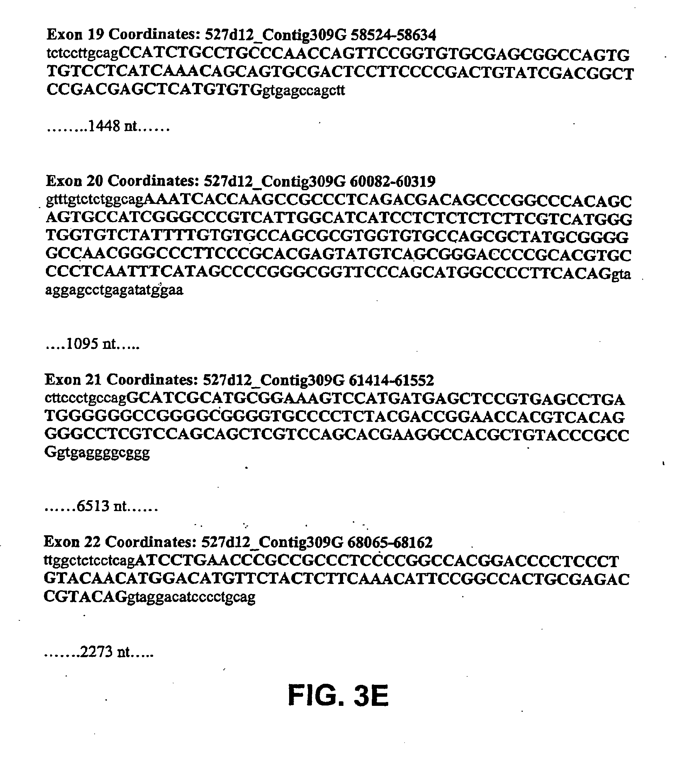

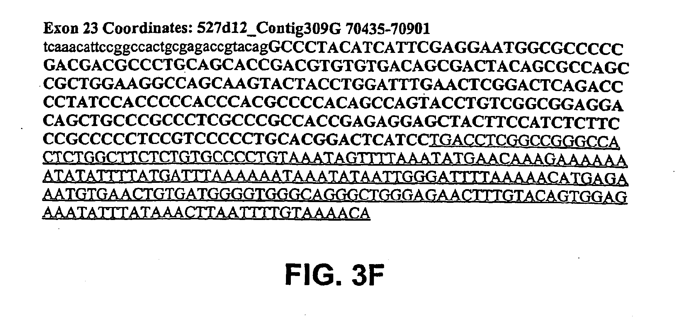

**Figs. 3A-3F** show the genomic structure of Zmax1 with flanking intron sequences. Translation is initiated by the underlined "ATG" in exon 1. The site of the polymorphism in the HBM gene is in exon 3 and is represented by the underlined "G," whereby this nucleotide is a "T" in the HBM gene. The 3' untranslated region of the mRNA is underlined within exon 23 (exon 1, SEQ ID NO:40; exon 2, SEQ ID NO:41; exon 3, SEQ ID NO:42; exon 4, SEQ ID NO:43; exon 5, SEQ ID NO:44; exon 6, SEQ ID NO:45; exon 7, SEQ ID NO:46; exon 8, SEQ ID NO:47; exon 9, SEQ ID NO:48; exon 10, SEQ ID NO:49; exon 11, SEQ ID NO:50; exon 12, SEQ ID NO:51; exon 13, SEQ ID NO:52; exon 14, SEQ ID NO:53; exon 15, SEQ ID NO:54; exon 16, SEQ ID NO:55; exon 17, SEQ ID NO:56; exon 18, SEQ ID NO:57; exon 19, SEQ ID NO:58; exon 20, SEQ ID NO:59; exon 21, SEQ ID NO:60; exon 22, SEQ ID NO:61; and exon 23; SEQ ID NO:62).

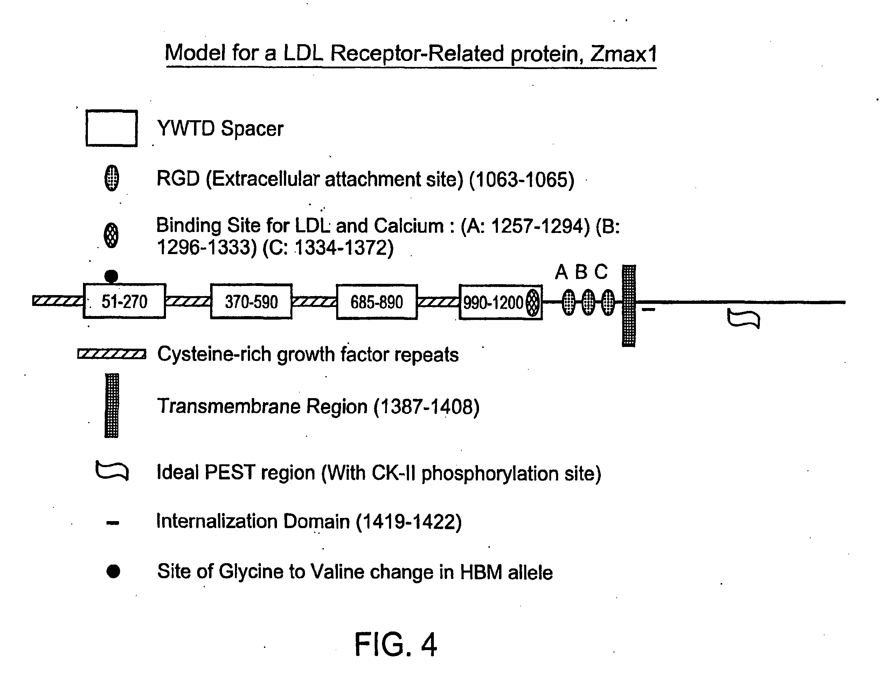

**Fig. 4** shows the domain organization of Zmax1, including the YWTD spacers, the extracellular attachment site, the binding site for LDL and calcium, the cysteine-rich growth factor repeats, the transmembrane region, the ideal PEST region with the CK-II phosphorylation site and the internalization domain. **Fig. 4** also shows the site of the glycine to valine change that occurs in the HBM protein. The signal peptide is located at amino acids 1-22, the extracellular domain is located at amino acids 23-1385, the transmembrane segment is located at amino acids 1386-1413, and the cytoplasmic domain is located at amino acids 1414-1615.

**Fig. 5** is a schematic illustration of the BAC contigs B527D12 and B200E21 in relation to the HBM gene.

**Figs. 6A-6E** are the nucleotide and amino acid sequences of the wild-type gene, Zmax1. The location for the base pair substitution at nucleotide 582, a guanine to thymine, is underlined. This allelic variant is the HBM gene. The HBM gene encodes for a protein with an amino acid substitution of glycine to valine at position 171. The 5' untranslated region (UTR) boundaries bases 1 to 70, and the 3' UTR boundaries bases 4916-5120.

**Figs. 7A** and **7B** are northern blot analyses showing the expression of Zmax1 in various tissues.

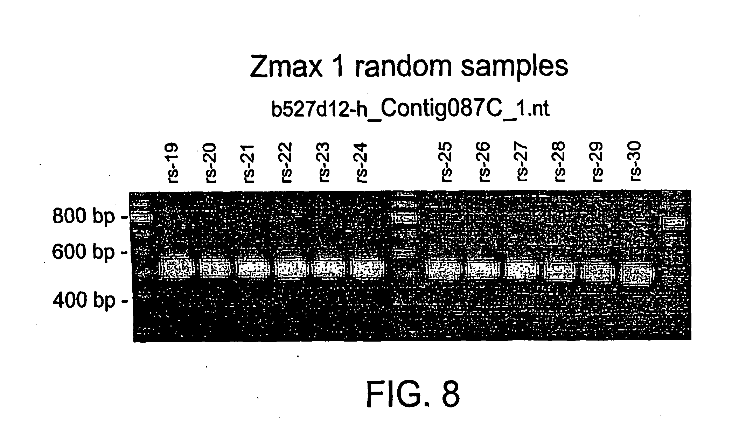

**Fig. 8** is a PCR product analysis.

**Fig. 9** is allele specific oligonucleotide detection of the Zmax1 exon 3 mutation.

**Fig.10** is the cellular localization of mouse Zmax1 by *in situ* hybridization at 100X magnification using sense and antisense probes.

**Fig. 11** is the cellular localization of mouse Zmax1 by *in situ* hybridization at 400X magnification using sense and antisense probes.

**Fig.12** is the cellular localization of mouse Zmax1 by *in situ* hybridization of osteoblasts in the endosteum at 400X magnification using sense and antisense probes.

**Fig. 13** shows antisense inhibition of Zmax1 expression in MC-3T3 cells.

**Fig. 14** shows a Zmax1 Exon3 Allele Specific Oligonucleotide (ASO) assay which illustrates the rarity of the HBM1 allele (right panels; T-specific oligo; 58°C Wash) as compared to the wild-type Zmax1 allele (left panels, G-specific oligo; 55°C Wash). The positive spots appearing in the right panels were positive controls.

**Fig.15** depicts a model representing the potential role of Zmax1 in focal adhesion signaling.

### DETAILED DESCRIPTION OF THE INVENTION

To aid in the understanding of the specification and claims, the following definitions are provided.

"Gene" refers to a DNA sequence that encodes through its template or messenger RNA a sequence of amino acids characteristic of a specific peptide. The term "gene" includes intervening, non-coding regions, as well as regulatory regions, and can include 5' and 3' ends.

"Gene sequence" refers to a DNA molecule, including both a DNA molecule which contains a non-transcribed or non-translated sequence. The term is also intended to include any combination of gene(s), gene fragment(s), non-transcribed sequence(s) or non-translated sequence(s) which are present on the same DNA molecule.

The sequences of the present invention may be derived from a variety of sources including DNA, cDNA, synthetic DNA, synthetic RNA or combinations thereof. Such sequences may comprise genomic DNA which may or may not include naturally occurring introns. Moreover, such genomic DNA may be obtained in association with promoter regions or poly (A) sequences. The sequences, genomic DNA or cDNA may be obtained in any of several ways. Genomic DNA can be extracted and purified from suitable cells by means well known in the art. Alternatively, mRNA can be isolated from a cell and used to produce cDNA by reverse transcription or other means.

"cDNA" refers to complementary or copy DNA produced from an RNA template by the action of RNA-dependent DNA polymerase (reverse transcriptase). Thus, a "cDNA clone" means a duplex DNA sequence complementary to an RNA molecule of interest, carried in a cloning vector or PCR amplified. This term includes genes from which the intervening sequences have been removed.

"Recombinant DNA" means a molecule that has been recombined by *in vitro* splicing cDNA or a genomic DNA sequence.

"Cloning" refers to the use of *in vitro* recombination techniques to insert a particular gene or other DNA sequence into a vector molecule. In order to successfully clone a desired gene, it is necessary to use methods for generating DNA fragments, for joining the fragments to vector molecules, for introducing the composite DNA molecule into a host cell in which it can replicate, and for selecting the clone having the target gene from amongst the recipient host cells.

"cDNA library" refers to a collection of recombinant DNA molecules containing cDNA inserts which together comprise the entire genome of an organism. Such a cDNA library can be prepared by methods known to one skilled in the art and described by, for example, Cowell and Austin, "cDNA Library Protocols," Methods in Molecular Biology (1997). Generally, RNA is first isolated from the cells of an organism from whose genome it is desired to clone a particular gene.

"Cloning vehicle" refers to a plasmid or phage DNA or other DNA sequence which is able to replicate in a host cell. The cloning vehicle is characterized by one or more endonuclease recognition sites at which such DNA sequences may be cut in a determinable fashion without loss of an essential biological function of the DNA, which may contain a marker suitable for use in the identification of transformed cells.

"Expression control sequence" refers to a sequence of nucleotides that control or regulate expression of structural genes when operably linked to those genes. These include, for example, the lac systems, the trp system, major operator and promoter regions of the phage lambda, the control region of fd coat protein and other sequences known to control the expression of genes in prokaryotic or eukaryotic cells. Expression control sequences will vary depending on whether the vector is designed to express the operably linked gene in a prokaryotic or eukaryotic host, and may contain transcriptional elements such as enhancer elements, termination sequences, tissue-specificity elements and/or translational initiation and termination sites.

"Expression vehicle" refers to a vehicle or vector similar to a cloning vehicle but which is capable of expressing a gene which has been cloned into it, after transformation into a host. The cloned gene is usually placed under the control of (i.e., operably linked to) an expression control sequence.

"Operator" refers to a DNA sequence capable of interacting with the specific repressor, thereby controlling the transcription of adjacent gene(s).

"Promoter" refers to a DNA sequence that can be recognized by an RNA polymerase. The presence of such a sequence permits the RNA polymerase to bind and initiate transcription of operably linked gene sequences.

"Promoter region" is intended to include the promoter as well as other gene sequences which may be necessary for the initiation of transcription. The presence of a promoter region is sufficient to cause the expression of an operably linked gene sequence.

"Operably linked" means that the promoter controls the initiation of expression of the gene. A promoter is operably linked to a sequence of proximal DNA if upon introduction into a host cell the promoter determines the transcription of the proximal DNA sequence(s) into one or more species of RNA. A promoter is operably linked to a DNA sequence if the promoter is capable of initiating transcription of that DNA sequence.

"Prokaryote" refers to all organisms without a true nucleus, including bacteria.

"Eukaryote" refers to organisms and cells that have a true nucleus, including mammalian cells.

"Host" includes prokaryotes and eukaryotes, such as yeast and filamentous fungi, as well as plant and animal cells. The term includes an organism or cell that is the recipient of a replicable expression vehicle.

"Fragment" of a gene refers to any variant of the gene that possesses the biological activity of that gene.

"Variant" refers to a gene that is substantially similar in structure and biological activity or immunological characteristics to either the entire gene or to a fragment of the gene. Provided that the two genes possess a similar activity, they are considered variant as that term is used herein even if the sequence of amino acid residues is not identical.

"Amplification of nucleic acids" refers to methods such as polymerase chain reaction (PCR), ligation amplification (or ligase chain reaction, LCR) and amplification methods based on the use of Q-beta replicase. These methods are well known in the art and described, for example, in U.S. Patent Nos. 4,683,195 and 4,683,202. Reagents and hardware for conducting PCR are commercially available. Primers useful for amplifying sequences from the HBM region are preferably complementary to, and hybridize specifically to sequences in the HBM region or in regions that flank a target region therein. HBM sequences generated by amplification may be sequenced directly. Alternatively, the amplified sequence(s) may be cloned prior to sequence analysis.

"Antibodies" may refer to polyclonal and/or monoclonal antibodies and fragments thereof, and immunologic binding equivalents thereof, that can bind to the HBM proteins and fragments thereof or to nucleic acid sequences from the HBM region, particularly from the HBM locus or a portion thereof. The term antibody is used both to refer to a homogeneous molecular entity, or a mixture such as a serum product made up of a plurality of different molecular entities. Proteins may be prepared synthetically in a protein synthesizer and coupled to a carrier molecule and injected over several months into rabbits. Rabbit sera is tested for immunoreactivity to the HBM protein or fragment. Monoclonal antibodies may be made by injecting mice with the proteins, or fragments thereof Monoclonal antibodies will be screened by ELISA and tested for specific immunoreactivity with HBM protein or fragments thereof. Harlow et al, Antibodies: A Laboratory Manual, Cold Spring Harbor Laboratory, Cold Spring Harbor, NY (1988). These antibodies will be useful in assays as well as pharmaceuticals.

"HBM" refers to high bone mass.

"HBM protein" refers to a protein that is identical to a Zmax1 protein except that it contains an alteration of glycine 171 to valine. An HBM protein is defined for any organism that encodes a Zmax1 true homologue. For example, a mouse HBM protein refers to the mouse Zmax1 protein having the glycine 170 to valine substitution.

"HBM gene" refers to the genomic DNA sequence found in individuals showing the HBM characteristic or phenotype, where the sequence encodes the protein indicated by SEQ ID NO: 4. The HBM gene and the Zmax1 gene are allelic. The protein encoded by the HBM gene has the property of causing elevated bone mass, while the protein encoded by the Zmax1 gene does not. The HBM gene and the Zmax1 gene differ in that the HBM gene has a thymine at position 582, while the Zmax1 gene has a guanine at position 582. The HBM gene comprises the nucleic acid sequence shown as SEQ ID NO: 2. The HBM gene may also be referred to as an "HBM polymorphism."

"Normal," "wild-type," "unaffected" and "Zmax1" all refer to the genomic DNA sequence that encodes the protein indicated by SEQ ID NO: 3. The Zmax1 gene has a guanine at position 582. The Zmax1 gene comprises the nucleic acid sequence shown as SEQ ID NO: 1. "Normal," "wild-type," "unaffected" and "Zmax1" also refer to allelic variants of the genomic sequence that encodes proteins that do not contribute to elevated bone mass. The Zmax1 gene is common in the human population, while the HBM gene is rare.

"SYWT+EGF" refers to a repeat unit found in the Zmax1 protein, consisting of five YWT repeats followed by an EGF repeat.

"Bone development" generally refers to any process involved in the change of bone over time, including, for example, normal development, changes that occur during disease states, and changes that occur during aging. "Bone development disorder" particularly refers to any disorders in bone development including, for example, changes that occur during disease states and changes that occur during aging. Bone development may be progressive or cyclical in nature. Aspects of bone that may change during development include, for example, mineralization, formation of specific anatomical features, and relative or absolute numbers of various cell types.

"Bone modulation" or "modulation of bone formation" refers to the ability to affect any of the physiological processes involved in bone remodeling, as will be appreciated by one skilled in the art, including, for example, bone resorption and appositional bone growth, by, inter alia, osteoclastic and osteoblastic activity, and may comprise some or all of bone formation and development as used herein.

"Normal bone density" refers to a bone density within two standard deviations of a Z score of 0.

A "Zmax1 system" refers to a purified protein, cell extract, cell, animal, human or any other composition of matter in which Zmax1 is present in a normal or mutant form.

A "surrogate marker" refers to a diagnostic indication, symptom, sign or other feature that can be observed in a cell, tissue, human or animal that is correlated with the HBM gene or elevated bone mass or both, but that is easier to measure than bone density. The general concept of a surrogate marker is well accepted in diagnostic medicine.

The Zmax1 gene and Zmax1 protein in the forms indicated by SEQ ID NOS: 1 and 3, respectively, and other closely related variants, as well as the adjacent chromosomal regions of Zmax1 necessary for its accurate expression are referred to least 15 contiguous nucleotides of the nucleic acid sequence of SEQ ID NO:1 are referred to in one instance.

The present invention also encompasses the HBM gene and HBM protein in the forms indicated by SEQ ID NO: 2 and 4, respectively, and other closely related variants, as well as the adjacent chromosomal regions of the HBM gene necessary for its accurate expression. In a preferred embodiment, the present invention is directed to at least 15 contiguous nucleotides of the nucleic acid sequence of SEQ ID NO: 2 wherein said is contiguous nucleotides comprise position 582 of SEQ ID No:2. More preferably in one instance, the present invention is therefore directed to at least 15 contiguous nucleotides of the nucleic acid sequence of SEQ ID NO: 2, wherein one of the 15 contiguous nucleotides is the thymine at nucleotide 582.

The invention also relates to the nucleotide sequence of the Zmax1 gene region, as well as the nucleotide sequence of the HBM gene region. More particularly, a preferred embodiment are the BAC clones containing segments of the Zmax1 gene region B200E21-H and B527D12-H. A preferred embodiment is the nucleotide sequence of the BAC clones consisting of SEQ ID NOS: 5-12.

The invention also concerns the use of the nucleotide sequence to identify DNA probes for the Zmax1 gene and the HBM gene, PCR primers to amplify the Zmax1 gene and the HBM gene, nucleotide polymorphisms in the Zmax1 gene and the HBM gene, and regulatory elements of the Zmax1 gene and the HBM gene.

This invention describes the further localization of the chromosomal location of the Zmax1 gene and HBM gene on chromosome 11a13.3 between genetic markers D11S987 and SNP_CONTIG033-6, as well as the DNA sequences of the Zmax1 gene and the HBM gene. The chromosomal location was refined by the addition of more genetic markers to the mapping panel used to map the gene, and by the extension of the pedigree to include more individuals. The pedigree extension was critical because the new individuals that have been genotyped harbor critical recombination events that narrow the region. To identify genes in the region on 11q13.3, a set of BAC clones containing this chromosomal region was identified. The BAC clones served as a template for genomic DNA sequencing, and also as a reagent for identifying coding sequences by direct cDNA selection. Genomic sequencing and direct cDNA selection were used to characterize more than 1.5 million base pairs of DNA from 11q13.3. The Zmax1 gene was identified within this region and the HBM gene was then discovered after mutational analysis of affected and unaffected individuals.

When a gene has been genetically localized to a specific chromosomal region, the genes in this region can be characterized at the molecular level by a series of steps that include: cloning of the entire region of DNA in a set of overlapping clones (physical mapping), characterization of genes encoded by these clones by a combination of direct cDNA selection, exon trapping and DNA sequencing (gene identification), and identification of mutations in these genes by comparative DNA sequencing of affected and unaffected members of the HBM kindred (mutation analysis).

Physical mapping is accomplished by screening libraries of human DNA cloned in vectors that are propagated in *E*. *coli* or *S*. *cereviseae* using PCR assays designed to amplify unique molecular landmarks in the chromosomal region of interest. To generate a physical map of the HBM candidate region, a library of human DNA cloned in Bacterial Artificial Chromosomes (BACs) was screened with a set of Sequence Tagged Site (STS) markers that had been previously mapped to chromosome 11q12-q13 by the efforts of the Human Genome Project.

STSs are unique molecular landmarks in the human genome that can be assayed by PCR. Through the combined efforts of the Human Genome Project, the location of thousands of STSs on the twenty-two autosomes and two sex chromosomes has been determined. For a positional cloning effort, the physical map is tied to the genetic map because the markers used for genetic mapping can also be used as STSs for physical mapping. By screening a BAC library with a combination of STSs derived from genetic markers, genes, and random DNA fragments, a physical map comprised of overlapping clones representing all of the DNA in a chromosomal region of interest can be assembled.

BACs are cloning vectors for large (80 kilobase to 200 kilobase) segments of human or other DNA that are propagated in *E*. *coli.* To construct a physical map using BACs, a library of BAC clones is screened so that individual clones harboring the DNA sequence corresponding to a given STS or set of STSs are identified. Throughout most of the human genome, the STS markers are spaced approximately 20 to 50 kilobases apart, so that an individual BAC clone typically contains at least two STS markers. In addition, the BAC libraries that were screened contain enough cloned DNA to cover the human genome six times over. Therefore, an individual STS typically identifies more than one BAC clone. By screening a six-fold coverage BAC library with a series of STS markers spaced approximately 50 kilobases apart, a physical map consisting of a series of overlapping BAC clones, i.e. BAC contigs, can be assembled for any region of the human genome. This map is closely tied to the genetic map because many of the STS markers used to prepare the physical map are also genetic markers.

When constructing a physical map, it often happens that there are gaps in the STS map of the genome that result in the inability to identify BAC clones that are overlapping in a given location. Typically, the physical map is first constructed from a set of STSs that have been identified through the publicly available literature and World Wide Web resources. The initial map consists of several separate BAC contigs that are separated by gaps of unknown molecular distance. To identify BAC clones that fill these gaps, it is necessary to develop new STS markers from the ends of the clones on either side of the gap. This is clone by sequencing the terminal 200 to 300 base pairs of the BACs flanking the gap, and developing a PCR assay to amplify a sequence of 100 or more base pairs. If the terminal sequences are demonstrated to be unique within the human genome, then the new STS can be used to screen the BAC library to identify additional BACs that contain the DNA from the gap in the physical map. To assemble a BAC contig that covers a region the size of the HBM candidate region (2,000,000 or more base pairs), it is often necessary to develop new STS markers from the ends of several clones.

After building a BAC contig, this set of overlapping clones serves as a template for identifying the genes encoded in the chromosomal region. Gene identification can be accomplished by many methods. Three methods are commonly used: (1) a set of BACs selected from the BAC contig to represent the entire chromosomal region can be sequenced, and computational methods can be used to identify all of the genes, (2) the BACs from the BAC contig can be used as a reagent to clone cDNAs corresponding to the genes encoded in the region by a method termed direct cDNA selection, or (3) the BACs from the BAC contig can be used to identify coding sequences by selecting for specific DNA sequence motifs in a procedure called exon trapping. The present invention includes genes identified by the first two methods.

To sequence the entire BAC contig representing the HBM candidate region, a set of BACs was chosen for subcloning into plasmid vectors and subsequent DNA sequencing of these subclones. Since the DNA cloned in the BACs represents genomic DNA, this sequencing is referred to as genomic sequencing to distinguish it from cDNA sequencing. To initiate the genomic sequencing for a chromosomal region of interest, several non-overlapping BAC clones are chosen. DNA for each BAC clone is prepared, and the clones are sheared into random small fragments which are subsequently cloned into standard plasmid vectors such as pUC18. The plasmid clones are then grown to propagate the smaller fragments, and these are the templates for sequencing. To ensure adequate coverage and sequence quality for the BAC DNA sequence, sufficient plasmid clones are sequenced to yield six-fold coverage of the BAC clone. For example, if the BAC is 100 kilobases long, then phagemids are sequenced to yield 600 kilobases of sequence. Since the BAC DNA was randomly sheared prior to cloning in the phagemid vector, the 600 kilobases of raw DNA sequence can be assembled by computational methods into overlapping DNA sequences termed sequence contigs. For the purposes of initial gene identification by computational methods, six-fold coverage of each BAC is sufficient to yield ten to twenty sequence contigs of 1000 base pairs to 20,000 base pairs.

The sequencing strategy employed in this invention was to initially sequence "seed" BACs from the BAC contig in the HBM candidate region. The sequence of the "seed" BACs was then used to identify minimally overlapping BACs from the contig, and these were subsequently sequenced. In this manner, the entire candidate region was sequenced, with several small sequence gaps left in each BAC. This sequence served as the template for computational gene identification. One method for computational gene identification is to compare the sequence of BAC contig to publicly available databases of cDNA and genomic sequences, e.g. unigene, dbEST, genbank. These comparisons are typically done using the BLAST family of computer algorithms and programs (Altschul et al, J. Mol. Biol., 215:403-410 (1990)). The BAC sequence can also be translated into protein sequence, and the protein sequence can be used to search publicly available protein databases, using a version of BLAST designed to analyze protein sequences (Altschul et al, Nucl. Acids Res., 25:3389-3402 (1997)). Another method is to use computer algorithms such as MZEF (Zhang, Proc. Natl. Acad. Sci., 94:565-568 (1997)) and GRAIL (Uberbacher et al, Methods Enzymo/., 266:259-281 (1996)), which predict the location of exons in the sequence based on the presence of specific DNA sequence motifs that are common to all exons, as well as the presence of codon usage typical of human protein encoding sequences.

In addition to identifying genes by computational methods, genes were also identified by direct cDNA selection (Del Mastro et al, Genome Res. 5(2):185-194 (1995)). In direct cDNA selection, cDNA pools from tissues of interest are prepared, and the BACs from the candidate region are used in a liquid hybridization assay to capture the cDNAs which base pair to coding regions in the BAC. In the methods described herein, the cDNA pools were created from several different tissues by random priming the first strand cDNA from polyA RNA, synthesizing the second strand cDNA by standard methods, and adding linkers to the ends of the cDNA fragments. The linkers are used to amplify the cDNA pools. The BAC clones are used as a template for *in vitro* DNA synthesis to create a biotin labelled copy of the BAC DNA. The biotin labelled copy of the BAC DNA is then denatured and incubated with an excess of the PCR amplified, linkered cDNA pools which have also been denatured. The BAC DNA and cDNA are allowed to anneal in solution, and heteroduplexes between the BAC and the cDNA are isolated using streptavidin coated magnetic beads. The cDNAs that are captured by the BAC are then amplified using primers complimentary to the linker sequences, and the hybridization/selection process is repeated for a second round. After two rounds of direct cDNA selection, the cDNA fragments are cloned, and a library of these direct selected fragments is created.

The cDNA clones isolated by direct selection are analyzed by two methods. Since a pool of BACs from the HBM candidate region is used to provide the genomic DNA sequence, the cDNAs must be mapped to individual BACs. This is accomplished by arraying the BACs in microtiter dishes, and replicating their DNA in high density grids. Individual cDNA clones are then hybridized to the grid to confirm that they have sequence identity to an individual BAC from the set used for direct selection, and to determine the specific identity of that BAC. cDNA clones that are confirmed to correspond to individual BACs are sequenced. To determine whether the cDNA clones isolated by direct selection share sequence identity or similarity to previously identified genes, the DNA and protein coding sequences are compared to publicly available databases using the BLAST family of programs.

The combination of genomic DNA sequence and cDNA sequence provided by BAC sequencing and by direct cDNA selection yields an initial list of putative genes in the region. The genes in the region were all candidates for the HBM locus. To further characterize each gene, Northern blots were performed to determine the size of the transcript corresponding to each gene, and to determine which putative exons were transcribed together to make an individual gene. For Northern blot analysis of each gene, probes were prepared from direct selected cDNA clones or by PCR amplifying specific fragments from genomic DNA or from the BAC encoding the putative gene of interest. The Northern blots gave information on the size of the transcript and the tissues in which it was expressed. For transcripts which were not highly expressed, it was sometimes necessary to perform a reverse transcription PCR assay using RNA from the tissues of interest as a template for the reaction.

Gene identification by computational methods and by direct cDNA selection provides unique information about the genes in a region of a chromosome. When genes are identified, then it is possible to examine different individuals for mutations in each gene.

### I. Phenotyping using DXA Measurements

Spinal bone mineral content (BMC) and bone mineral density (BMD) measurements performed at Creighton University (Omaha, Nebraska) were made by DXA using a Norland Instruments densitometer (Norland XR2600 Densitometer, Dual Energy X-ray Absorptiometry, DXA). Spinal BMC and BMD at other locations used the machinery available. There are estimated to be 800 DXA machines currently operating in the U.S. Most larger cities have offices or imaging centers which have DXA capabilities, usually a Lunar or Hologic machine. Each location that provided spine BMC and BMD data included copies of the printouts from their machines to provide verification that the regions of interest for measurement of BMD have been chosen appropriately. Complete clinical histories and skeletal radiographs were obtained.

The HBM phenotype is defined by the following criteria: very high spinal BMD; a clinical history devoid of any known high bone mass syndrome; and skeletal radiographs showing a normal shape of the appendicular skeleton.

### II. Genotyping of Microsatellite Markers

To narrow the genetic interval to a region smaller than that originally reported by Johnson et al, Am. J. Hum. Genet., 60:1326-1332 (1997), additional microsatellite markers on chromosome 11q12-13 were typed. The new markers included: D11S4191, D11S1883, D11S1785, D11S4113, D11S4136, D11S4139, (Dib, et al, Nature, 380:152-154 (1996), FGF3 (Polymeropolous, et al, Nucl. Acid Res., 18:7468 (1990)), as well as GTC_HBM_Marker_1, GTC_HBM_Marker_2, GTC_HBM_Marker_3, GTC_HBM_Marker_4, GTC_HBM_Marker_5, GTC_HBM_Marker_6, and GTC_HBM_Marker_7 (*See* **Fig. 2**).

Blood (20 ml) was drawn into lavender cap (EDTA containing) tubes by a certified phlebotomist. The blood was stored refrigerated until DNA extraction. DNA has been extracted from blood stored for up to 7 days in the refrigerator without reduction in the quality or quantity of yield. For those subjects that have blood drawn at distant sites, a shipping protocol was successfully used on more than a dozen occasions. Blood samples were shipped by overnight express in a styrofoam container with freezer packs to provide cooling. Lavender cap tubes were placed on individual plastic shipping tubes and then into "zip-lock" biohazard bags. When the samples arrived the next day, they were immediately processed to extract DNA.

The DNA extraction procedure used a kit purchased from Gentra Systems, Inc. (Minneapolis, Minnesota). Briefly, the procedure involved adding 3 volumes of a red blood cell lysis buffer to the whole blood. After incubations for 10 minutes at room temperature, the solution was centrifuged in a Beckman tabletop centrifuge at 2,000 X g for 10 minutes. The white blood cell pellet was resuspended in Cell Lysis Buffer. Once the pellet was completely resuspended and free of cell clumps, the solution was digested with RNase A for 15 minutes at 37°C. Proteins were precipitated by addition of the provided Protein Precipitation Solution and removed by centrifugation. The DNA was precipitated out of the supernatant by addition of isopropanol. This method was simple and fast, requiring only 1-2 hours, and allowed for the processing of dozens of samples simultaneously. The yield of DNA was routinely >8 mg for a 20 ml sample of whole blood and had a MW of >50 kb. DNA was archived by storing coded 50 µg aliquots at -80°C as an ethanol precipitate.

DNA was genotyped using one fluorescently labeled oligonucleotide primer and one unlabeled oligonucleotide primer. Labeled and unlabeled oligonucleotides were obtained from Integrated DNA Technologies, Inc. (Coralville, Iowa). All other reagents for microsatellite genotyping were purchased from Perkin Elmer-Applied Biosystems, Inc. ("PE-ABI") (Norwalk, Connecticut). Individual PCR reactions were performed for each marker, as described by PE-ABI using AmpliTag DNA Polymerase. The reactions were added to 3.5 µl of loading buffer containing deionized formamide, blue dextran and TAMRA 350 size standards (PE-ABI). After heating at 95 °C for 5 minutes to denature the DNA, the samples were loaded and electrophoresed as described in the operator's manual for the Model 377 DNA Sequencer (PE-ABI, Foster City, California). After gel electrophoresis, the data was analyzed using PE-ABI GENESCAN^{™} and GENOTYPER^{™} software. First, within the GENESCAN^{™} software, the lane tracking was manually optimized prior to the first step of analysis. After the gel lane data was extracted, the standard curve profiles of each lane were examined and verified for linearity and size calling. Lanes, which had problems with either of these parameters, were re-tracked and verified. Once all lanes were tracked and the size standards were correctly identified, the data were imported into GENOTYPER^{™} for allele identification To expedite allele calling (binning), the program Linkage Designer from the Internet web-site of Dr. Guy Van Camp (http://alt.www.uia.ac.be/u/dnalab/ld.html) was used. This program greatly facilitates the importing of data generated by GENOTYPER^{™} into the pedigree drawing program Cyrillic (Version 2.0, Cherwell Scientific Publishing Limited, Oxford, Great Britain) and subsequent linkage analysis using the program LINKAGE (Lathrop et al, Am. J. Hum. Genet., 37:482-498 (1985)).

### III. Linkage Analysis

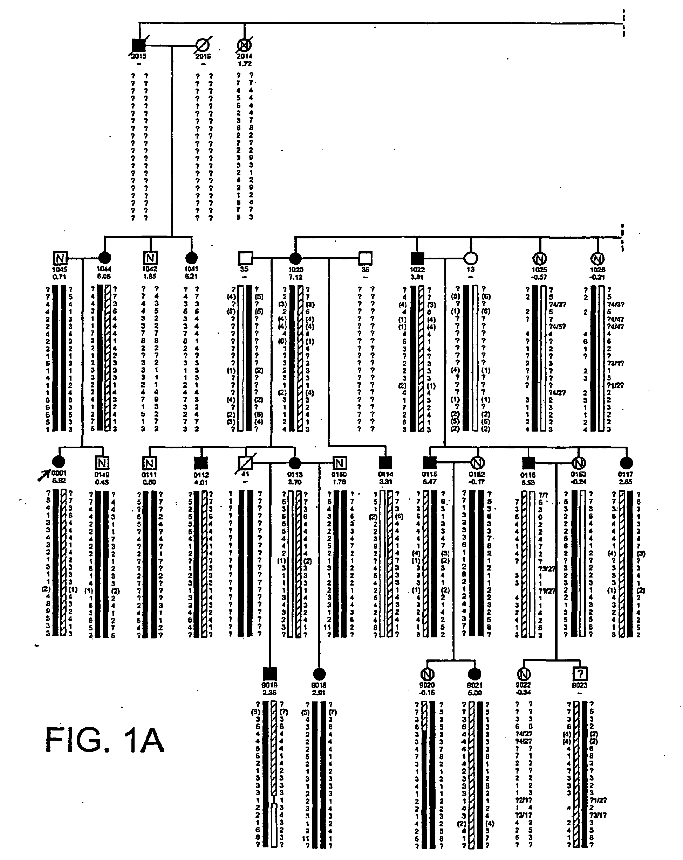

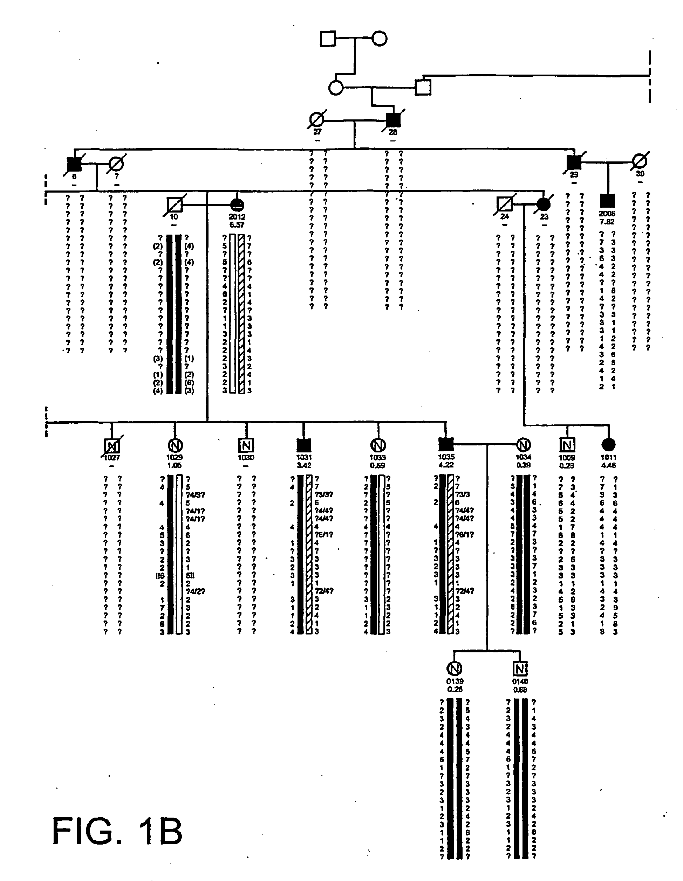

**Fig. 1** demonstrates the pedigree of the individuals used in the genetic linkage studies for this invention. Specifically, two-point linkage analysis was performed using the MLINK and LINKMAP components of the program LINKAGE (Lathrop et al, Am. J. Hum. Genet., 37:482-498 (1985)). Pedigree/marker data was exported from Cyrillic as a pre-file into the Makeped program and converted into a suitable ped-file for linkage analysis.

The original linkage analysis was performed using three models: (i) an autosomal dominant, fully penetrant model, (ii) an autosomal dominant model with reduced penetrance, and (iii) a quantitative trait model. The HBM locus was mapped to chromosome 11q12-13 by analyzing DNA for linked markers from 22 members of a large, extended kindred. A highly automated technology was used with a panel of 345 fluorescent markers which spanned the 22 autosomes at a spacing interval ranging from 6-22 cM. Only markers from this region of chromosome 11 showed evidence of linkage (LOD score ∼3.0). The highest LOD score (5.74) obtained by two-point and multipoint analysis was D11S987 (map position 55 in **Fig. 2**). The 95% confidence interval placed the HBM locus between markers D11S905 and D11S937 (map position 41-71 in **Fig. 2**). Haplotype analysis also places the Zmax1 gene in this same region. Further descriptions of the markers D11S987, D11S905, and D11S937 can be found in Gyapay et al, Nature Genetics, Vol. 7, (1994).

In this invention, the inventors report the narrowing of the HBM interval to the region between markers D11S987 and GTC_HBM_Marker_5. These two markers lie between the delimiting markers from the original analysis (D11S11S905 and D11S937) and are approximately 3 cM from one another. The narrowing of the interval was accomplished using genotypic data from the markers D11S4191, D11S1883, D11S1785, D11S4113, D11S4136, D11S4139, (Dib et al, Nature, 380:152-154 (1996)), FGF3 (Polymeropolous et al, Nucl. Acid Res., 18:7468 (1990)) (information about the genetic markers can be found at the internet site of the Genome Database, http://gdbwww.gdb.org/), as well as the markers GTC_HBM_Marker_1, GTC_HBM_Marker_2, GTC_HBM_Marker_3, GTC_HBM_Marker_4, GTC_HBM_Marker_5, GTC_HBM_Marker_6, and GTC_HBM_Marker_7.

As shown in **Fig. 1****,** haplotype analysis with the above genetic markers identifies recombination events (crossovers) in individuals 9019 and 9020 that significantly refine the interval of chromosome 11 to which the Zmax1 gene is localized. Individual 9019 is an HBM-affected individual that inherits a portion of chromosome 11 from the maternal chromosome with the HBM gene, and a portion from the chromosome 11 homologue. The portion inherited from the HBM gene-carrying chromosome includes markers D11S935, D11S1313, GTC_HBM_Marker_4, D11S987, D11S1296, GTC_HBM_Marker_6, GTC_HBM_Marker_2, D11S970, GTC_HBM_Marker_3, D11S4113, GTC_HBM__Marker_1, GTC_HBM_Marker_7 and GTC_HBM_Marker_5. The portion from D11S4136 and continuing in the telomeric direction is derived from the non-HBM chromosome. This data places the Zmax1 gene in a location centromeric to the marker GTC_HBM_Marker_5. Individual 9020 is an unaffected individual who also exhibits a critical recombination event. This individual inherits a recombinant paternal chromosome 11 that includes markers D11S935, D11S1313, GTC_HBM_Marker_4, D11S987, D11S1296 and GTC_HBM_Marker_6 from her father's (individual 0115) chromosome 11 homologue that carries the HBM gene, and markers GTC_HBM_Marker_2, D11S970, GTC_HBM_Marker_3, GTC_HBM_Marker_1, GTC_HBM_Marker_7, GTC_HBM_Marker_5, D11S4136, D11S4139, D11S1314, and D11S937 from her father's chromosome 11 that does not carry the HBM gene. Marker D11S4113 is uninformative due to its homozygous nature in individual 0115. This recombination event places the centromeric boundary of the HBM region between markers D11S1296 and D11S987.

Two-point linkage analysis was also used to confirm the location of the Zmax1 gene on chromosome 11. The linkage results for two point linkage analysis under a model of full penetrance are presented in Table 1 below. This table lists the genetic markers in the first column and the recombination fractions across the top of the table. Each cell of the column shows the LOD score for an individual marker tested for linkage to the Zmax1 gene at the recombination fraction shown in the first row. For example, the peak LOD score of 7.66 occurs at marker D11S970, which is within the interval defined by haplotype analysis.

**TABLE 1**

| | | | | | | | | | |
|---|---|---|---|---|---|---|---|---|---|
| Marker | 0.0 | 0.05 | 0.1 | 0.15 | 0.2 | 0.25 | 0.3 | 0.35 | 0.4 |
| D11S935 | - infinity | 0.39 | 0.49 | 0.47 | 0.41 | 0.33 | 0.25 | 0.17 | 0.10 |
| D11S1313 | - infinity | 2.64 | 2.86 | 2.80 | 2.59 | 2.30 | 1.93 | 1.49 | 1.00 |
| D11S987 | - infinity | 5.49 | 5.18 | 4.70 | 4.13 | 3.49 | 2.79 | 2.03 | 1.26 |
| D11S4113 | 4.35 | 3.99 | 3.62 | 3.24 | 2.83 | 2.40 | 1.94 | 1.46 | 0.97 |
| D11S1337 | 2.29 | 2.06 | 1.81 | 1.55 | 1.27 | 0.99 | 0.70 | 0.42 | 0.18 |
| D11S970 | 7.66 | 6.99 | 6.29 | 5.56 | 4.79 | 3.99 | 3.15 | 2.30 | 1.44 |
| D11S4136 | 6.34 | 5.79 | 5.22 | 4.61 | 3.98 | 3.30 | 2.59 | 1.85 | 1.11 |
| D11S4139 | 6.80 | 6.28 | 5.73 | 5.13 | 4.50 | 3.84 | 3.13 | 2.38 | 1.59 |
| FGF3 | 0.59 | 3.23 | 3.15 | 2.91 | 2.61 | 2.25 | 1.84 | 1.40 | 0.92 |
| D11S1314 | 6.96 | 6.49 | 5.94 | 5.34 | 4.69 | 4.01 | 3.27 | 2.49 | 1.67 |
| D11S937 | -infinity | 4.98 | 4.86 | 4.52 | 4.06 | 3.51 | 2.88 | 2.20 | 1.47 |

A single nucleotide polymorphism (SNP) further defines the HBM region. This SNP is termed SNP_Contig033-6 and is located 25 kb centromeric to the genetic marker GTC_HBM_Marker_5. This SNP is telomeric to the genetic marker GTC_HBM_Marker_7. SNP_Contig033-6 is present in HBM-affected individual 0113. However, the HBM-affected individual 9019, who is the son of 0113, does not carry this SNP. Therefore, this indicates that the crossover is centromeric to this SNP. The primer sequence for the genetic markers GTC_HBM_Marker_5 and GTC_HBM_Marker_7 is shown in Table 2 below.

**TABLE 2**

| Marker | Primer (Forward) | Primer (Reverse) |
|---|---|---|
| GTC_HBM_ Marker_5 | TTTTGGGTACACAATTCAGTCG | AAAACTGTGGGTGCTTCTGG |
| GTC_HBM_ Marker_7 | GTGATTGAGCCAATCCTGAGA | TGAGCCAAATAAACCCCTTCT |

The kindred described have several features of great interest, the most important being that their bones, while very dense, have an absolutely normal shape. The outer dimensions of the skeletons of the HBM-affected individuals are normal, and, while medullary cavities are present, there is no interference with hematopoiesis. The HBM-affected members seem to be resistant to fracture, and there are no neurologic symptoms, and no symptoms of impairment of any organ or system function in the members examined. HBM-affected members of the kindred live to advanced age without undue illness or disability. Furthermore, the HBM phenotype matches no other bone disorders such as osteoporosis, osteoporosis pseudoglioma, Engelmann's disease, Ribbing's disease, hyperphosphatasemia, Van Buchem's disease, melorheostosis, osteopetrosis, pycnodysostosis, sclerostenosis, osteopoikilosis, acromegaly, Paget's disease, fibrous dysplasia, tubular stenosis, osteogenesis imperfecta, hypoparathyroidism, pseudohypoparathyroidism, pseudopseudohypoparathyroidism, primary and secondary hyperparathyroidism and associated syndromes, hypercalciuria, medullary carcinoma of the thyroid gland, osteomalacia and other diseases. Clearly, the HBM locus in this family has a very powerful and substantial role in regulating bone density, and its identification is an important step in understanding the pathway(s) that regulate bone density and the pathogenesis of diseases such as osteoporosis.

In addition, older individuals carrying the HBM gene, and therefore expression of the HBM protein, do not show loss of bone mass characteristic of normal individuals. In other words, the HBM gene is a suppressor of osteoporosis. In essence, individuals carrying the HBM gene are dosed with the HBM protein, and, as a result, do not develop osteoporosis. This *in vivo* observation is strong evidence that treatment of normal individuals with the HBM gene or protein, or a fragment thereof, will ameliorate osteoporosis.

### IV. Physical Mapping

To provide reagents for the cloning and characterization of the HBM locus, the genetic mapping data described above were used to construct a physical map of the region containing Zmax1 on chromosome 11q13.3. The physical map consists of an ordered set of molecular landmarks, and a set of BAC clones that contain the Zmax1 gene region from chromosome 11q13.3.

Various publicly available mapping resources were utilized to identify existing STS markers (Olson et al, Science, 245:1434-1435 (1989)) in the HBM region. Resources included the GDB, the Whitehead Institute Genome Center, dbSTS and dbEST (NCBI), 11db, the University of Texas Southwestern GESTEC, the Stanford Human Genome Center, and several literature references (Courseaux et al, Genomics, 40:13-23 (1997), Courseaux et al, Genomics, 37:354-365 (1996), Guru et al, Genomics, 42:436-445 (1997), Hosoda et al, Genes Cells, 2:345-357 (1997), James et al, Nat. Genet., 8:70-76 (1994), Kitamura et al, DNA Research, 4:281-289 (1997), Lemmens et al, Genomics, 44:94-100 (1997), Smith et al, Genome Res., 7:835-842 (1997)). Maps were integrated manually to identify markers mapping to the region containing Zmax1.

Primers for existing STSs were obtained from the GDB or literature references are listed in Table 3 below. Thus, Table 3 shows the STS markers used to prepare the physical map of the Zmax1 gene region.

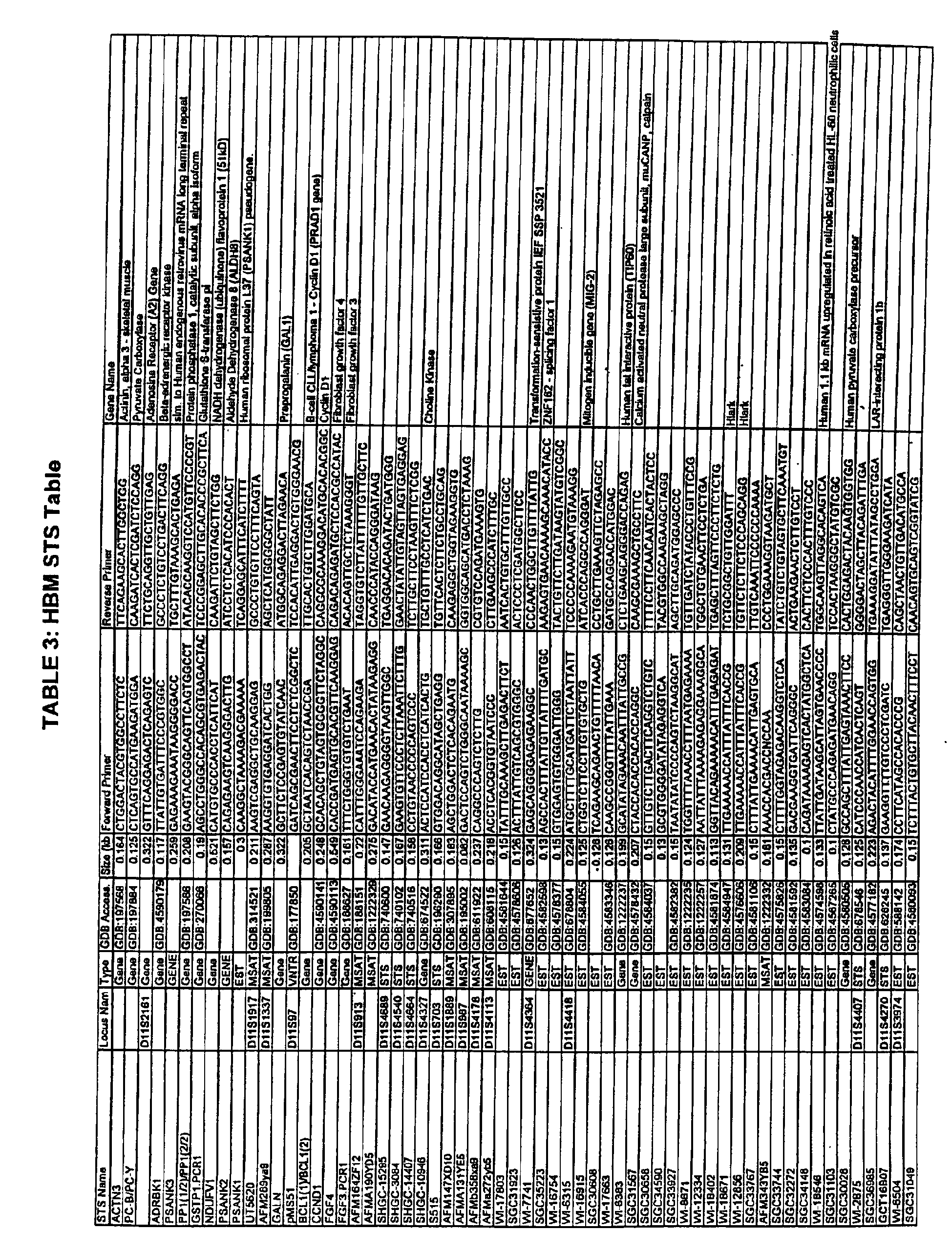

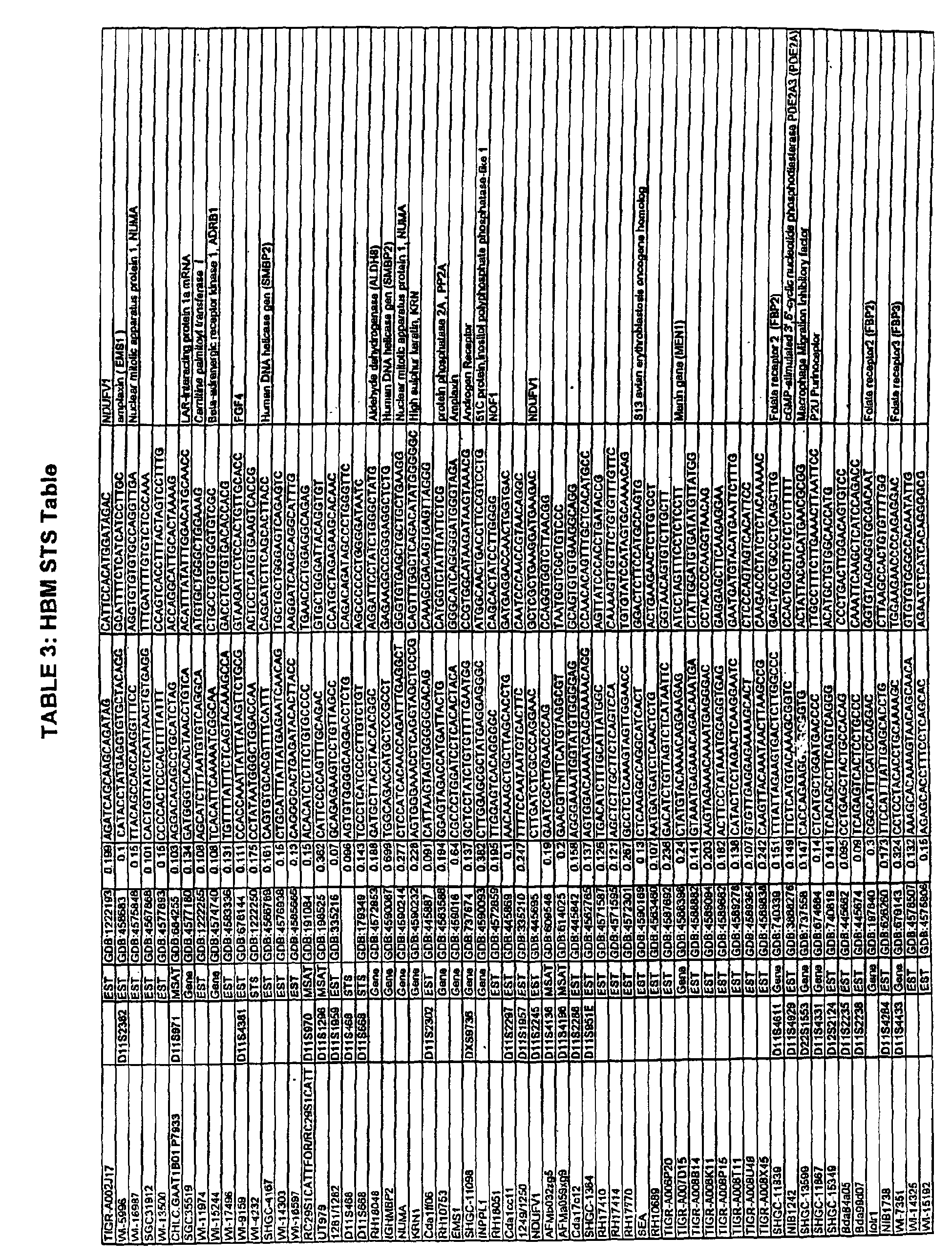

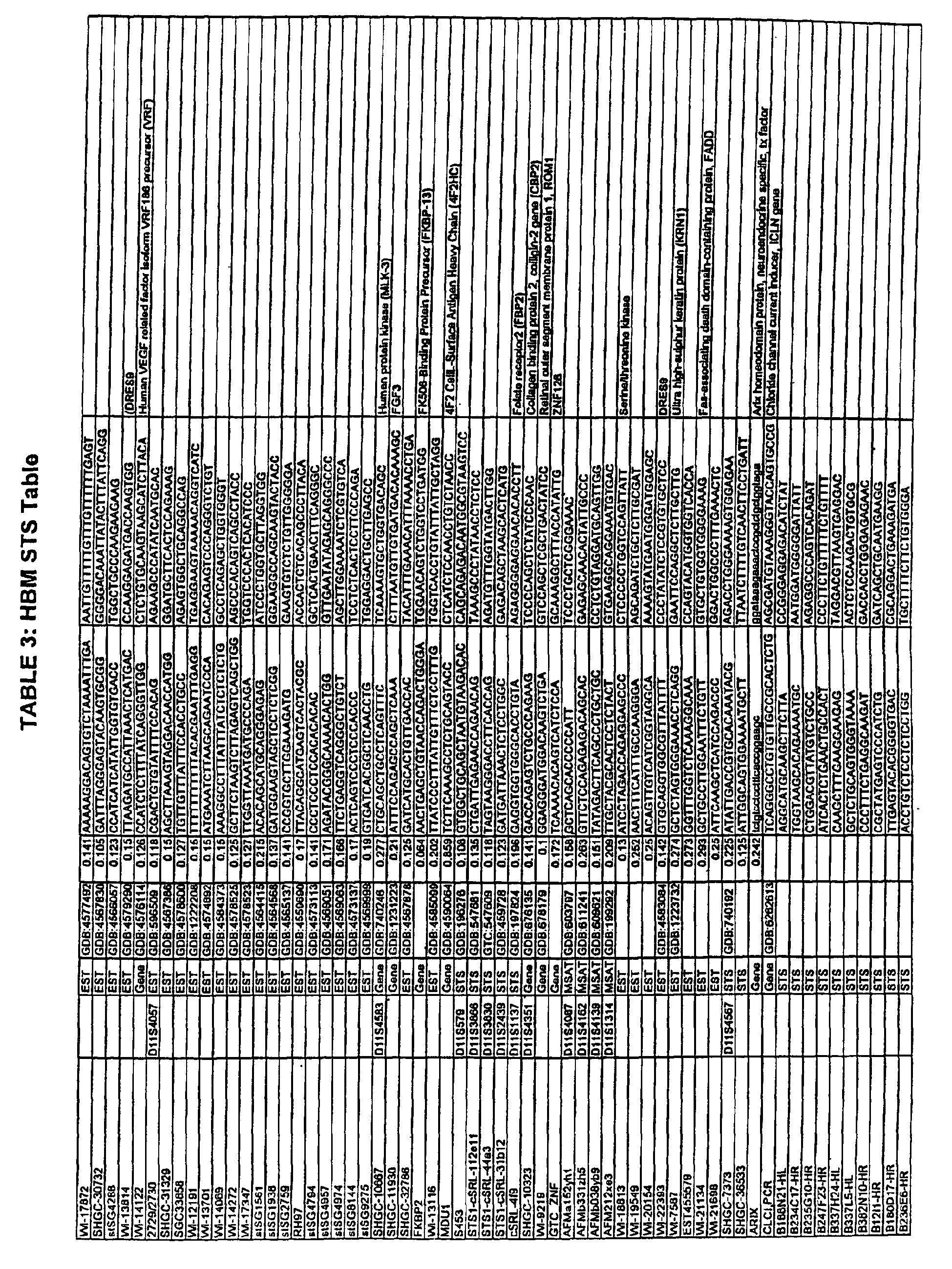

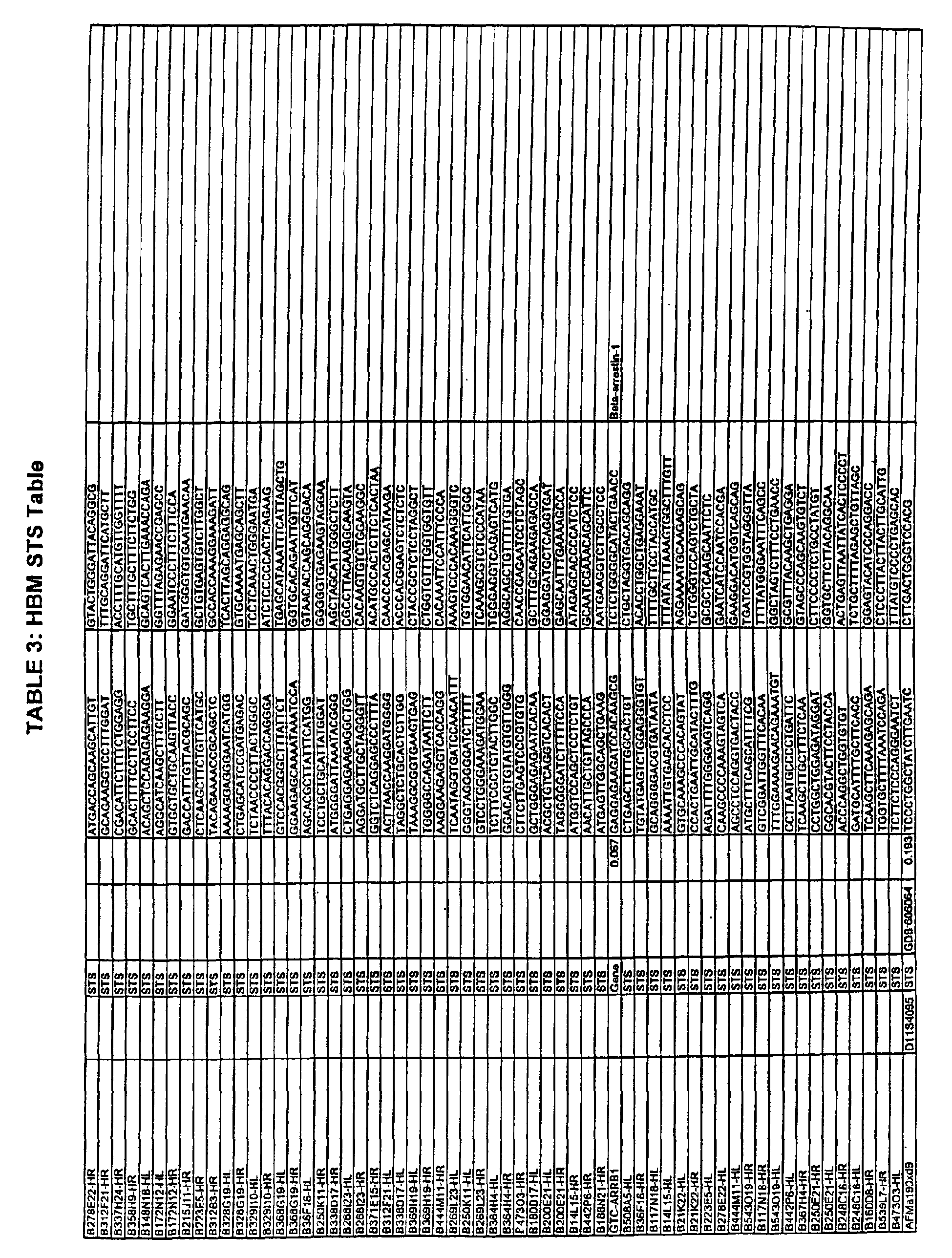

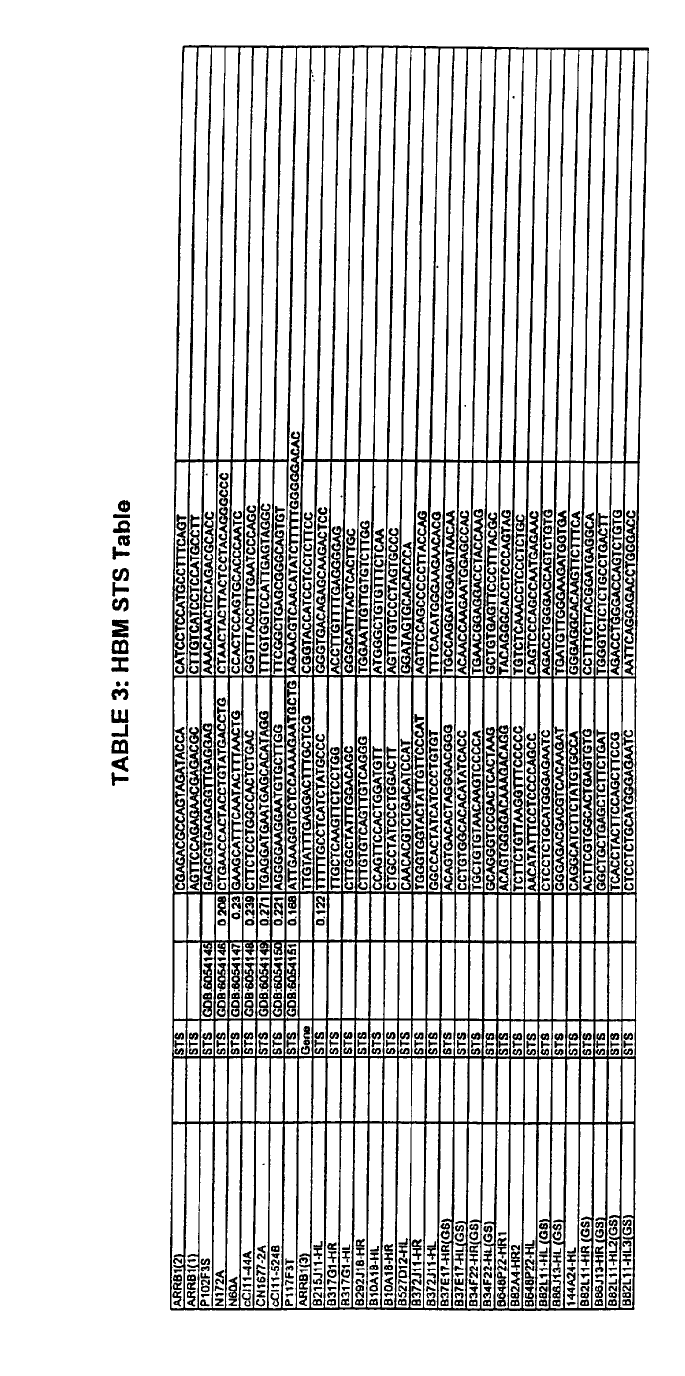

Novel STSs were developed either from publicly available genomic sequence or from sequence-derived BAC insert ends. Primers were chosen using a script which automatically performs vector and repetitive sequence masking using Cross_match (P. Green, U. of Washington) and subsequent primer picking using Primer3 (Rozen, Skaletsky (1996, 1997). Primer3 is available at www.genome.wi.mit. edu/genome_software/other/primer3.html.

Polymerase chain reaction (PCR) conditions for each primer pair were initially optimized with respect to MgCl₂ concentration. The standard buffer was 10 mM Tris-HCl (pH 8.3), 50 mM KCl, MgCl₂, 0.2 mM each dNTP, 0.2 µM each primer, 2.7 ng/µl human DNA, 0.25 units of AmpliTaq (Perkin Elmer) and MgCl₂ concentrations of 1.0 mM, 1.5 mM, 2.0 mM or 2.4 mM. Cycling conditions included an initial denaturation at 94°C for 2 minutes followed by 40 cycles at 94°C for 15 seconds, 55 °C for 25 seconds, and 72°C for 25 seconds followed by a final extension at 72°C for 3 minutes. Depending on the results from the initial round of optimization the conditions were further optimized if necessary. Variables included increasing the annealing temperature to 58°C or 60°C, increasing the cycle number to 42 and the annealing and extension times to 30 seconds, and using AmpliTaqGold (Perkin Elmer).

BAC clones (Kim et al, Genomics, 32:213-218 (1996), Shizuya et al, Proc. Natl. Acad. Sci. USA, 89:8794-8797 (1992)) containing STS markers of interest were obtained by PCR-based screening of DNA pools from a total human BAC library purchased from Research Genetics. DNA pools derived from library plates 1-596 were used corresponding to nine genomic equivalents of human DNA. The initial screening process involved PCR reactions of individual markers against superpools, i.e., a mixture of DNA derived from all BAC clones from eight 384-well library plates. For each positive superpool, plate (8), row (16) and column (24) pools were screened to identify a unique library address. PCR products were electrophoresed in 2% agarose gels (Sigma) containing 0.5 µg/ml ethidium bromide in 1X TBE at 150 volts for 45 min. The electrophoresis units used were the Model A3-1 systems from Owl Scientific Products. Typically, gels contained 10 tiers of lanes with 50 wells/tier. Molecular weight markers (100 bp ladder, Life Technologies, Bethesda, MD) were loaded at both ends of the gel. Images of the gels were captured with a Kodak DC40 CCD camera and processed with Kodak 1D software. The gel data were exported as tab delimited text files; names of the files included information about the library screened, the gel image files and the marker screened. These data were automatically imported using a customized Perl script into Filemaker™ PRO (Claris Corp.) databases for data storage and analysis. In cases where incomplete or ambiguous clone address information was obtained, additional experiments were performed to recover a unique, complete library address.

Recovery of clonal BAC cultures from the library involved streaking out a sample from the library well onto LB agar (Maniatis et al, Molecular Cloning: A Laboratory Manual., Cold Spring Harbor Laboratory, Cold Spring Harbor, NY (1982)) containing 12.5 µg/ml chloramphenicol (Sigma). Two individual colonies and a portion of the initial streak quadrant were tested with appropriate STS markers by colony PCR for verification. Positive clones were stored in LB broth containing 12.5 µg/ml chloramphenicol and 15% glycerol at -70°C.

Several different types of DNA preparation methods were used for isolation of BAC DNA. The manual alkaline lysis miniprep protocol listed below (Maniatis et al, Molecular Cloning: A Laboratory Manual, Cold Spring Harbor Laboratory, Cold Spring Harbor, NY (1982)) was successfully used for most applications, i.e., restriction mapping, CHEF gel analysis, FISH mapping, but was not successfully reproducible in endsequencing. The Autogen and Qiagen protocols were used specifically for BAC DNA preparation for endsequencing purposes.

Bacteria were grown in 15 ml Terrific Broth containing 12.5 µg/ml chloramphenicol in a 50 ml conical tube at 37°C for 20 hrs with shaking at 300 rpm. The cultures were centrifuged in a Sorvall RT 6000 D at 3000 rpm (∼1800 g) at 4°C for 15 min. The supernatant was then aspirated as completely as possible. In some cases cell pellets were frozen at -20°C at this step for up to 2 weeks. The pellet was then vortexed to homogenize the cells and minimize clumping. 250 µl of P1 solution (50 mM glucose, 15 mM Tris-HCl, pH 8, 10 mM EDTA, and 100 µg/ml RNase A) was added and the mixture pipetted up and down to mix. The mixture was then transferred to a 2 ml Eppendorf tube. 350 µl of P2 solution (0.2 N NaOH, 1% SDS) was then added, the mixture mixed gently and incubated for 5 min. at room temperature. 350 µl of P3 solution (3 M KOAc, pH 5.5) was added and the mixture mixed gently until a white precipitate formed. The solution was incubated on ice for 5 min. and then centrifuged at 4°C in a microfuge for 10 min. The supernatant was transferred carefully (avoiding the white precipitate) to a fresh 2 ml Eppendorf tube, and 0.9 ml of isopropanol was added, the solution mixed and left on ice for 5 min. The samples were centrifuged for 10 min., and the supernatant removed carefully. Pellets were washed in 70% ethanol and air dried for 5 min. Pellets were resuspended in 200 µl of TE8 (10 mM Tris-HCl, pH 8.0, 1.0 mM EDTA), and RNase A (Boehringer Mannheim) added to 100 µg/ml. Samples were incubated at 37°C for 30 min., then precipitated by addition of C₂H₃O₂Na·3H₂O to 0.5 M and 2 volumes of ethanol. Samples were centrifuged for 10 min., and the pellets washed with 70% ethanol followed by air drying and dissolving in 50 µl TE8. Typical yields for this DNA prep were 3-5 µg/15 ml bacterial culture. Ten to 15 µl were used for HindIII restriction analysis; 5 µl was used for NotI digestion and clone insert sizing by CHEF gel electrophoresis.

BACs were inoculated into 15 ml of 2X LB Broth containing 12.5 µg/ml chloramphenicol in a 50 ml conical tube. 4 tubes were inoculated for each clone. Cultures were grown overnight (∼16 hr) at 37°C with vigorous shaking (>300 rpm). Standard conditions for BAC DNA isolation were followed as recommended by the Autogen 740 manufacturer. 3 ml samples of culture were placed into Autogen tubes for a total of 60 ml or 20 tubes per clone. Samples were dissolved finally in 100 µl TE8 with 15 seconds of shaking as part of the Autogen protocol. After the Autogen protocol was finished DNA solutions were transferred from each individual tube and pooled into a 2 ml Eppendorf tube. Tubes with large amounts of debris (carry over from the pelleting debris step) were avoided. The tubes were then rinsed with 0.5 ml of TE8 successively and this solution added to the pooled material. DNA solutions were stored at 4°C; clumping tended to occur upon freezing at -20°C. This DNA was either used directly for restriction mapping, CHEF gel analysis or FISH mapping or was further purified as described below for use in endsequencing reactions.

The volume of DNA solutions was adjusted to 2 ml with TE8, samples were then mixed gently and heated at 65 °C for 10 min. The DNA solutions were then centrifuged at 4°C for 5 min. and the supernatants transferred to a 15 ml conical tube. The NaCl concentration was then adjusted to 0.75 M (∼0.3 ml of 5 M NaCl to the 2 ml sample). The total volume was then adjusted to 6 ml with Qiagen column equilibration buffer (Buffer QBT). The supernatant containing the DNA was then applied to the column and allowed to enter by gravity flow. Columns were washed twice with 10 ml of Qiagen Buffer QC. Bound DNA was then eluted with four separate 1 ml aliquots of Buffer QF kept at 65°C. DNA was precipitated with 0.7 volumes of isopropanol (∼2.8 ml). Each sample was then transferred to 4 individual 2.2 ml Eppendorf tubes and incubated at room temperature for 2 hr or overnight. Samples were centrifuged in a microfuge for 10 min. at 4°C. The supernatant was removed carefully and 1 ml of 70% ethanol was added. Samples were centrifuged again and because the DNA pellets were often loose at this stage, the supernatant removed carefully. Samples were centrifuged again to concentrate remaining liquid which was removed with a micropipet tip. DNA pellets were then dried in a desiccator for 10 min. 20 µl of sterile distilled and deionized H₂O was added to each tube which was then placed at 4°C overnight. The four 20 µl samples for each clone were pooled and the tubes rinsed with another 20 µl of sterile distilled and deionized H₂O for a final volume of 100 µl. Samples were then heated at 65°C for 5 min. and then mixed gently. Typical yields were 2-5 µg/60 ml culture as assessed by NotI digestion and comparison with uncut lambda DNA.

3 ml of LB Broth containing 12.5 µg/ml of chloramphenicol was dispensed into autoclaved Autogen tubes. A single tube was used for each clone. For inoculation, glycerol stocks were removed from -70°C storage and placed on dry ice. A small portion of the glycerol stock was removed from the original tube with a sterile toothpick and transferred into the Autogen tube; the toothpick was left in the Autogen tube for at least two minutes before discarding. After inoculation the tubes were covered with tape making sure the seal was tight. When all samples were inoculated, the tube units were transferred into an Autogen rack holder and placed into a rotary shaker at 37°C for 16-17 hours at 250 rpm. Following growth, standard conditions for BAC DNA preparation, as defined by the manufacturer, were used to program the Autogen. Samples were not dissolved in TE8 as part of the program and DNA pellets were left dry. When the program was complete, the tubes were removed from the output tray and 30 µl of sterile distilled and deionized H₂O was added directly to the bottom of the tube. The tubes were then gently shaken for 2-5 seconds and then covered with parafilm and incubated at room temperature for 1-3 hours. DNA samples were then transferred to an Eppendorf tube and used either directly for sequencing or stored at 4°C for later use.

### V. BAC Clone Characterization for Physical Mapping

DNA samples prepared either by manual alkaline lysis or the Autogen protocol were digested with HindIII for analysis of restriction fragment sizes. This data were used to compare the extent of overlap among clones. Typically 1-2 µg were used for each reaction. Reaction mixtures included: 1X Buffer 2 (New England Biolabs), 0.1 mg/ml bovine serum albumin (New England Biolabs), 50 µg/ml RNase A (Boehringer Mannheim), and 20 units of HindIII (New England Biolabs) in a final volume of 25 µl. Digestions were incubated at 37°C for 4-6 hours. BAC DNA was also digested with NotI for estimation of insert size by CHEF gel analysis (see below). Reaction conditions were identical to those for HindIII except that 20 units of NotI were used. Six µl of 6X Ficoll loading buffer containing bromphenol blue and xylene cyanol was added prior to electrophoresis.

HindIII digests were analyzed on 0.6% agarose (Seakem, FMC Bioproducts) in 1X TBE containing 0.5 µg/ml ethidium bromide. Gels (20 cm X 25 cm) were electrophoresed in a Model A4 electrophoresis unit (Owl Scientific) at 50 volts for 20-24 hrs. Molecular weight size markers included undigested lambda DNA, HindIII digested lambda DNA, and HaeIII digested _X174 DNA. Molecular weight markers were heated at 65 °C for 2 min. prior to loading the gel. Images were captured with a Kodak DC40 CCD camera and analyzed with Kodak 1D software.

NotI digests were analyzed on a CHEF DRII (BioRad) electrophoresis unit according to the manufacturer's recommendations. Briefly, 1% agarose gels (BioRad pulsed field grade) were prepared in 0.5X TBE, equilibrated for 30 minutes in the electrophoresis unit at 14°C, and electrophoresed at 6 volts/cm for 14 hrs with circulation. Switching times were ramped from 10 sec to 20 sec. Gels were stained after electrophoresis in 0.5 µg/ml ethidium bromide. Molecular weight markers included undigested lambda DNA, HindIII digested lambda DNA, lambda ladder PFG ladder, and low range PFG marker (all from New England Biolabs).

BAC DNA prepared either by the manual alkaline lysis or Autogen protocols were labeled for FISH analysis using a Bioprime labeling kit (BioRad) according to the manufacturer's recommendation with minor modifications. Approximately 200 ng of DNA was used for each 50 µl reaction. 3 µl were analyzed on a 2% agarose gel to determine the extent of labeling. Reactions were purified using a Sephadex G50 spin column prior to *in situ* hybridization. Metaphase FISH was performed as described (Ma et al, Cytogenet. Cell Genet., 74:266-271 (1996)).

### VI. BAC Endsequencing

The sequencing of BAC insert ends utilized DNA prepared by either of the two methods described above. The DYEnamic energy transfer primers and Dynamic Direct cycle sequencing kits from Amersham were used for sequencing reactions. Ready made sequencing mix including the M13 -40 forward sequencing primer was used (Catalog # US79730) for the T7 BAC vector terminus; ready made sequencing mix (Catalog # US79530) was mixed with the M13 -28 reverse sequencing primer (Catalog # US79339) for the SP6 BAC vector terminus. The sequencing reaction mixes included one of the four fluorescently labeled dye-primers, one of the four dideoxy termination mixes, dNTPs, reaction buffer, and Thermosequenase. For each BAC DNA sample, 3 µl of the BAC DNA sample was aliquoted to 4 PCR strip tubes. 2 µl of one of the four dye primer/termination mix combinations was then added to each of the four tubes. The tubes were then sealed and centrifuged briefly prior to PCR. Thermocycling conditions involved a 1 minute denaturation at 95 °C, 15 second annealing at 45°C, and extension for 1 minute at 70°C for 35 total cycles. After cycling the plates were centrifuged briefly to collect all the liquid to the bottom of the tubes. 5 µl of sterile distilled and deionized H₂O was then added into each tube, the plates sealed and centrifuged briefly again. The four samples for each BAC were then pooled together. DNA was then precipitated by adding 1.5 µl of 7.5 M NH₄OAc and 100 µl of -20°C 100% ethanol to each tube. Samples were mixed by pipetting up and down once. The plates were then sealed and incubated on ice for 10 minutes. Plates were centrifuged in a table top Haraeus centrifuge at 4000 rpm (3,290 g) for 30 minutes at 4°C to recover the DNA. The supernatant was removed and excess liquid blotted onto paper towels. Pellets were washed by adding 100 µl of -20°C 70% ethanol into each tube and recentrifuging at 4000 rpm (3,290 g) for 10 minutes at 4°C. The supernatant was removed and excess liquid again removed by blotting on a paper towel. Remaining traces of liquid were removed by placing the plates upside down over a paper towel and centrifuging only until the centrifuge reached 800 rpm. Samples were then air dried at room temperature for 30 min. Tubes were capped and stored dry at -20°C until electrophoresis. Immediately prior to electrophoresis the DNA was dissolved in 1.5 µl of Amersham loading dye. Plates were then sealed and centrifuged at 2000 rpm (825 g). The plates were then vortexed on a plate shaker for 1-2 minutes. Samples were then recentrifuged at 2000 rpm (825 g) briefly. Samples were then heated at 65°C for 2 min. and immediately placed on ice. Standard gel electrophoresis was performed on ABI 377 fluorescent sequencers according to the manufacturer's recommendation.

### VII. Sub-cloning and Sequencing of HBM BAC DNA

The physical map of the Zmax1 gene region provides a set of BAC clones that contain within them the Zmax1 gene and the HBM gene. DNA sequencing of several of the BACs from the region has been completed. The DNA sequence data is a unique reagent that includes data that one skilled in the art can use to identify the Zmax1 gene and the HBM gene, or to prepare probes to identify the gene(s), or to identify DNA sequence polymorphisms that identify the gene(s).

BAC DNA was isolated according to one of two protocols, either a Qiagen purification of BAC DNA (Qiagen, Inc. as described in the product literature) or a manual purification which is a modification of the standard alkaline lysis/Cesium Chloride preparation of plasmid DNA (see e.g., Ausubel et al, Current Protocols in Molecular Biology, John Wiley & Sons (1997)). Briefly for the manual protocol, cells were pelleted, resuspended in GTE (50 mM glucose, 25 mM Tris-Cl (pH 8), 10 mM EDTA) and lysozyme (50 mg/ml solution), followed by NaOH/SDS (1% SDS/0.2 N NaOH) and then an ice-cold solution of 3 M KOAc (pH 4.5-4.8). RnaseA was added to the filtered supernatant, followed by Proteinase K and 20% SDS. The DNA was then precipitated with isopropanol, dried and resuspended in TE (10 mM Tris, 1 mM EDTA (pH 8.0)). The BAC DNA was further purified by Cesium Chloride density gradient centrifugation (Ausubel et al, Current Protocols in Molecular Biology, John Wiley & Sons (1997)).

Following isolation, the BAC DNA was sheared hydrodynamically using an HPLC (Hengen, Trends in Biochem. Sci., 22:273-274 (1997)) to an insert size of 2000-3000 bp. After shearing, the DNA was concentrated and separated on a standard 1% agarose gel. A single fraction, corresponding to the approximate size, was excised from the gel and purified by electroelution (Sambrook et al, Molecular Cloning: A Laboratory Manual, Cold Spring Harbor Laboratory, Cold Spring, NY (1989)).

The purified DNA fragments were then blunt-ended using T4 DNA polymerase. The blunt-ended DNA was then ligated to unique BstXI-linker adapters (5' GTCTTCACCACGGGG and 5' GTGGTGAAGAC in 100-1000 fold molar excess). These linkers were complimentary to the BstXI-cut pMPX vectors (constructed by the inventors), while the overhang was not self-complimentary. Therefore, the linkers would not concatemerize nor would the cut-vector religate itself easily. The linker-adapted inserts were separated from the unincorporated linkers on a 1% agarose gel and purified using GeneClean (BIO 101, Inc.). The linker-adapted insert was then ligated to a modified pBlueScript vector to construct a "shotgun" subclone library. The vector contained an out-of-frame lacZ gene at the cloning site which became in-frame in the event that an adapter-dimer is cloned, allowing these to be avoided by their blue-color.

All subsequent steps were based on sequencing by ABI377 automated DNA sequencing methods. Only major modifications to the protocols are highlighted. Briefly, the library was then transformed into DH5α competent cells (Life Technologies, Bethesda, MD, DH5α transformation protocol). It was assessed by plating onto antibiotic plates containing ampicillin and IPTG/Xgal. The plates were incubated overnight at 37°C. Successful transformants were then used for plating of clones and picking for sequencing. The cultures were grown overnight at 37°. DNA was purified using a silica bead DNA preparation (Ng et al, Nucl. Acids Res., 24:5045-5047 (1996)) method. In this manner, 25 µg of DNA was obtained per clone.

These purified DNA samples were then sequenced using ABI dye-terminator chemistry. The ABI dye terminator sequence reads were run on ABI377 machines and the data was directly transferred to UNIX machines following lane tracking of the gels. All reads were assembled using PHRAP (P. Green, Abstracts of DOE Human Genome Program Contractor-Grantee Workshop V, Jan. 1996, p.157) with default parameters and quality scores. The initial assembly was done at 6-fold coverage and yielded an average of 8-15 contigs. Following the initial assembly, missing mates (sequences from clones that only gave one strand reads) were identified and sequenced with ABI technology to allow the identification of additional overlapping contigs. Primers for walking were selected using a Genome Therapeutics program Pick_primer near the ends of the clones to facilitate gap closure. These walks were sequenced using the selected clones and primers. Data were reassembled with PHRAP into sequence contigs.

### VIII. Gene Identifcation by Computational Methods

Following assembly of the BAC sequences into contigs, the contigs were subjected to computational analyses to identify coding regions and regions bearing DNA sequence similarity to known genes. This protocol included the following steps.
1. Degap the contigs: the sequence contigs often contain symbols (denoted by a period symbol) that represent locations where the individual ABI sequence reads have insertions or deletions. Prior to automated computational analysis of the contigs, the periods were removed. The original data was maintained for future reference.
2. BAC vector sequences were "masked" within the sequence by using the program cross match (Phil Green, http:\\chimera.biotech.washington.edu\UWGC). Since the shotgun libraries construction detailed above leaves some BAC vector in the shotgun libraries, this program was used to compare the sequence of the BAC contigs to the BAC vector and to mask any vector sequence prior to subsequent steps. Masked sequences were marked by an "X" in the sequence files, and remained inert during subsequent analyses.
3. *E*. *coli* sequences contaminating the BAC sequences were masked by comparing the BAC contigs to the entire *E. coli* DNA sequence.
4. Repetitive elements known to be common in the human genome were masked using cross match. In this implementation of crossmatch, the BAC sequence was compared to a database of human repetitive elements (Jerzy Jerka, Genetic Information Research Institute, Palo Alto, CA). The masked repeats were marked by X and remained inert during subsequent analyses.
5. The location of exons within the sequence was predicted using the MZEF computer program (Zhang, Proc. Natl. Acad. Sci., 94:565-568 (1997)).
6. The sequence was compared to the publicly available unigene database (National Center for Biotechnology Information, National Library of Medicine, 38A, 8N905, 8600 Rockville Pike, Bethesda, MD 20894; www.ncbi.nlm.nih.gov) using the blastn2 algorithm (Altschul et al, Nucl. Acids Res., 25:3389-3402 (1997)). The parameters for this search were: E=0.05, v=50, B=50 (where E is the expected probability score cutoff, V is the number of database entries returned in the reporting of the results, and B is the number of sequence alignments returned in the reporting of the results (Altschul et al, J. Mol. Biol., 215:403-410 (1990)).
7. The sequence was translated into protein for all six reading frames, and the protein sequences were compared to a non-redundant protein database compiled from Genpept Swissprot PIR (National Center for Biotechnology Information, National Library of Medicine, 38A, 8N905, 8600 Rockville Pike, Bethesda, MD 20894; www.ncbi.nlm.nih.gov). The parameters for this search were E=0.05, V=50, B= 50, where E, V, and B are defined as above.
8. The BAC DNA sequence was compared to the database of the cDNA clones derived from direct selection experiments (described below) using blastn2 (Altschul et al, Nucl. Acids. Res., 25:3389-3402 (1997)). The parameters for this search were E=0.05, V=250, B=250, where E, V, and B are defined as above.
9. The BAC sequence was compared to the sequences of all other BACs from the HBM region on chromosome 11q12-13 using blastn2 (Altschul et al, Nucl. Acids. Res., 25:3389-3402 (1997)). The parameters for this search were E=0.05, V=50, B=50, where E, V, and B are defined as above.
10. The BAC sequence was compared to the sequences derived from the ends of BACs from the HBM region on chromosome 11q12-13 using blastn2 (Altschul et al, Nucl. Acids. Res., 25:3389-3402 (1997)). The parameters for this search were E=0.05, V=50, B=50, where E, V, and B are defined as above.
11. The BAC sequence was compared to the Genbank database (National Center for Biotechnology Information, National Library of Medicine, 38A, 8N905, 8600 Rockville Pike, Bethesda, MD 20894; www.ncbi.nlm.nih.gov) using blastn2 (Altschul et al, Nucl. Acids. Res., 25:3389-3402 (1997)). The parameters for this search were E=0.05, V=50, B=50, where E, V, and B are defined as above.
12. The BAC sequence was compared to the STS division of Genbank database (National Center for Biotechnology Information, National Library of Medicine, 38A, 8N905, 8600 Rockville Pike, Bethesda, MD 20894; www.ncbi.nlm.nih.gov) using blastn2 (Altschul et al, 1997). The parameters for this search were E=0.05, V=50, B= 50, where E, V, and B are defined as above.
13. The BAC sequence was compared to the Expressed Sequence (EST) Tag Genbank database (National Center for Biotechnology Information, National Library of Medicine, 38A, 8N905, 8600 Rockville Pike, Bethesda, MD 20894; www.ncbi.nlm.nih.gov) using blastn2 (Altschul et al, Nucl. Acids. Res., 25:3389-3402 (1997)). The parameters for this search were E=0.05, V=250, B=250, where E, V, and B are defined as above.

### IX. Gene Identification by Direct cDNA Selection

Primary linkered cDNA pools were prepared from bone marrow, calvarial bone, femoral bone, kidney, skeletal muscle, testis and total brain. Poly (A) + RNA was prepared from calvarial and femoral bone tissue (Chomczynski et al, Anal. Biochem., 162:156-159 (1987); D'Alessio et al, Focus, 9:1-4 (1987)) and the remainder of the mRNA was purchased from Clontech (Palo Alto, California). In order to generate oligo(dT) and random primed cDNA pools from the same tissue, 2.5 µg mRNA was mixed with oligo(dT) primer in one reaction and 2.5 µg mRNA was mixed with random hexamers in another reaction, and both were converted to first and second strand cDNA according to manufacturers recommendations (Life Technologies, Bethesda, MD). Paired phosphorylated cDNA linkers (see sequence below) were annealed together by mixing in a 1:1 ratio (10 µg each) incubated at 65°C for five minutes and allowed to cool to room temperature.
Paired linkers oligo1/2
   OLIGO 1: 5'CTG AGC GGA ATT CGT GAG ACC3' (SEQ ID NO:12)
   OLIGO 2: 5'TTG GTC TCA CGT ATT CCG CTC GA3' (SEQ ID NO:13)
Paired linkers oligo3/4
   OLIGO 3: 5'CTC GAG AAT TCT GGA TCC TC3' (SEQ ID NO:14)
   OLIGO 4: 5'TTG AGG ATC CAG AAT TCT CGA G3' (SEQ ID NO:15)
Paired linkers ohgo5/6
   OLIGO 5: 5'TGT ATG CGA ATT CGC TGC GCG3' (SEQ ID NO:16)
   OLIGO 6: 5'TTC GCG CAG CGA ATT CGC ATA CA3' (SEQ ID NO:17)
Paired linkers oligo7/8
   OLIGO 7: 5'GTC CAC TGA ATT CTC AGT GAG3' (SEQ ID NO:18)
   OLIGO 8: 5'TTG TCA CTG AGA ATT CAG TGG AC3' (SEQ ID NO:19)
Paired linkers oligo11/12
   OLIGO 11: 5'GAA TCC GAA TTC CTG GTC AGC3' (SEQ ID NO:20)
   OLIGO 12: 5'TTG CTG ACC AGG AAT TCG GAT TC3' (SEQ ID NO:21)
   Linkers were ligated to all oligo(dT) and random primed cDNA pools (see below) according to manufacturers instructions (Life Technologies, Bethesda, MD).

Oligo 1/2 was ligated to oligo(dT) and random primed cDNA pools prepared from bone marrow. Oligo 3/4 was ligated to oligo(dT) and random primed cDNA pools prepared from calvarial bone. Oligo 5/6 was ligated to oligo(dT) and random primed cDNA pools prepared from brain and skeletal muscle. Oligo 7/8 was ligated to oligo(dT) and random primed cDNA pools prepared from kidney. Oligo 11/12 was ligated to oligo(dT) and random primed cDNA pools prepared from femoral bone.

The cDNA pools were evaluated for length distribution by PCR amplification using 1 µl of a 1:1, 1:10, and 1:100 dilution of the ligation reaction, respectively. PCR reactions were performed in a Perkin Elmer 9600, each 25 µl volume reaction contained 1 µl of DNA, 10 mM Tris-HCl (pH 8.3), 50 mM KCl, 1.5 mM MgCl2, 0.001 % gelatin, 200 mM each dNTPs, 10 µM primer and 1 unit Taq DNA polymerase (Perkin Elmer) and was amplified under the following conditions: 30 seconds at 94°C, 30 seconds at 60°C and 2 minutes at 72°C for 30 cycles. The length distribution of the amplified cDNA pools were evaluated by electrophoresis on a 1% agarose gel. The PCR reaction that gave the best representation of the random primed and oligo(dT) primed cDNA pools was scaled up so that ∼2-3 µg of each cDNA pool was produced. The starting cDNA for the direct selection reaction comprised of 0.5 µg of random primed cDNAs mixed with 0.5 µg of oligo(dT) primed cDNAs.

The DNA from the 54 BACs that were used in the direct cDNA selection procedure was isolated using Nucleobond AX columns as described by the manufacturer (The Nest Group, Inc.).

The BACs were pooled in equimolar amounts and 1 µg of the isolated genomic DNA was labeled with biotin 16-UTP by nick translation in accordance with the manufacturers instructions (Boehringer Mannheim). The incorporation of the biotin was monitored by methods that could be practiced by one skilled in the art (Del Mastro and Lovett, Methods in Molecular Biology, Humana Press Inc., NJ (1996)).

Direct cDNA selection was performed using methods that could be practiced by one skilled in the art (Del Mastro and Lovett, Methods in Molecular Biology, Humana Press Inc., NJ (1996)). Briefly, the cDNA pools were multiplexed in two separate reactions: In one reaction cDNA pools from bone marrow, calvarial bone, brain and testis were mixed, and in the other cDNA pools from skeletal muscle, kidney and femoral bone were mixed. Suppression of the repeats, yeast sequences and plasmid in the cDNA pools was performed to a Cot of 20. 100 ng of biotinylated BAC DNA was mixed with the suppressed cDNAs and hybridized in solution to a Cot of 200. The biotinylated DNA and the cognate cDNAs was captured on streptavidin-coated paramagnetic beads. The beads were washed and the primary selected cDNAs were eluted. These cDNAs were PCR amplified and a second round of direct selection was performed. The product of the second round of direct selection is referred to as the secondary selected material. A Galanin cDNA clone, previously shown to map to 11q12-13 (Evans, Genomics, 18:473-477 (1993)), was used to monitor enrichment during the two rounds of selection.

The secondary selected material from bone marrow, calvarial bone, femoral bone, kidney, skeletal muscle, testis and total brain was PCR amplified using modified primers of oligos 1, 3, 5, 7 and 11, shown below, and cloned into the UDG vector pAMP10 (Life Technologies, Bethesda, MD), in accordance with the manufacturer's recommendations.
Modified primer sequences:
   Oligo1-CUA: 5'CUA CUA CUA CUA CTG AGC GGA ATT CGT GAG ACC3' (SEQ ID NO:22)
   Oligo3-CUA: 5'CUA CUA CUA CUA CTC GAG AAT TCT GGA TCC TC3' (SEQ ID NO:23) .
   Oligo5-CUA: 5'CUA CUA CUA CUA TGT ATG CGA ATT CGC TGC GCG3' (SEQ ID NO:24)
   Oligo7-CUA: 5'CUA CUA CUA CUA GTC CAC TGA ATT CTC AGT GAG3' (SEQ ID NO:25)
   Oligo11-CUA: 5'CUA CUA CUA CUA GAA TCC GAA TTC CTG GTC AGC3' (SEQ ID NO:26)

The cloned secondary selected material, from each tissue source, was transformed into MAX Efficiency DH5a Competent Cells (Life Technologies, Bethesda, MD) as recommended by the manufacturer. 384 colonies were picked from each transformed source and arrayed into four 96 well microtiter plates. All secondarily selected cDNA clones were sequenced using M13 dye primer terminator cycle sequencing kit (Applied Biosystems), and the data collected by the ABI 377 automated fluorescence sequencer (Applied Biosystems). All sequences were analyzed using the BLASTN, BLASTX and FASTA programs (Altschul et al, J. Mol. Biol., 215:403-410 (1990), Altschul et al, Nucl. Acids. Res., 25:3389-3402 (1997)). The cDNA sequences were compared to a database containing sequences derived from human repeats, mitochondrial DNA, ribosomal RNA, E. *coli* DNA to remove background clones from the dataset using the program cross_match. A further round of comparison was also performed using the program BLASTN2 against known genes (Genbank) and the BAC sequences from the HBM region. Those cDNAs that were >90% homologous to these sequences were filed according to the result and the data stored in a database for further analysis. cDNA sequences that were identified but did not have significant similarity to the BAC sequences from the HBM region or were eliminated by cross_match were hybridized to nylon membranes which contained the BACs from the HBM region, to ascertain whether they hybridized to the target.

Hybridization analysis was used to map the cDNA clones to the BAC target that selected them. The BACs that were identified from the HBM region were arrayed and grown into a 96 well microtiter plate. LB agar containing 25 µg/ml kanamycin was poured into 96 well microtiter plate lids. Once the agar had solidified, pre-cut Hybond N+ nylon membranes (Amersham) were laid on top of the agar and the BACs were stamped onto the membranes in duplicate using a hand held 96 well replica plater (V&P Scientific, Inc.). The plates were incubated overnight at 37°C. The membranes were processed according to the manufacturers recommendations.

The cDNAs that needed to be mapped by hybridization were PCR amplified using the relevant primer (oligos 1, 3, 5, 7 and 11) that would amplify that clone. For this PCR amplification, the primers were modified to contain a linkered digoxigenin molecule at the 5' of the oligonucleotide. The PCR amplification was performed under the same conditions as described in Preparation of cDNA Pools (above). The PCR products were evaluated for quality and quantity by electrophoresis on a 1% agarose gel by loading 5 µl of the PCR reaction. The nylon membranes containing the stamped BACs were individually pre-hybridized in 50 ml conical tubes containing 10 ml of hybridization solution (5x SSPE, 0.5x Blotto, 2.5% SDS and 1 mM EDTA (pH 8.0)). The 50 ml conical tubes were placed in a rotisserie oven (Robbins Scientific) for 2 hours at 65°C. Twenty-five ng of each cDNA probe was denatured and added into individual 50 ml conical tubes containing the nylon membrane and hybridization solution. The hybridization was performed overnight at 65°C. The filters were washed for 20 minutes at 65°C in each of the following solutions: 3x SSPE, 0.1% SDS; 1x SSPE, 0.1% SDS and 0.1x SSPE, 0.1% SDS.

The membranes were removed from the 50 ml conical tubes and placed in a dish. Acetate sheets were placed between each membrane to prevent them from sticking to each other. The incubation of the membranes with the Anti-DIG-AP and CDP-Star was performed according to manufacturers recommendations (Boehringer Mannheim). The membranes were wrapped in Saran wrap and exposed to Kodak Bio-Max X-ray film for 1 hour.

### X. cDNA Cloning and Expression Analysis

To characterize the expression of the genes identified by direct cDNA selection and genomic DNA sequencing in comparison to the publicly available databases, a series of experiments were performed to further characterize the genes in the HBM region. First, oligonucleotide primers were designed for use in the polymerase chain reaction (PCR) so that portions of a cDNA, EST, or genomic DNA could be amplified from a pool of DNA molecules (a cDNA library) or RNA population (RT-PCR and RACE). The PCR primers were used in a reaction containing genomic DNA to verify that they generated a product of the size predicted based on the genomic (BAC) sequence. A number of cDNA libraries were then examined for the presence of the specific cDNA or EST. The presence of a fragment of a transcription unit in a particular cDNA library indicates a high probability that additional portions of the same transcription unit will be present as well.

A critical piece of data that is required when characterizing novel genes is the length, in nucleotides, of the processed transcript or messenger RNA (mRNA). One skilled in the art primarily determines the length of an mRNA by Northern blot hybridization (Sambrook et al, Molecular Cloning: A Laboratory Manual, Cold Spring Harbor Laboratory, Cold Spring Harbor NY (1989)). Groups of ESTs and direct-selected cDNA clones that displayed significant sequence similarity to sequenced BACs in the critical region were grouped for convenience into approximately 30 kilobase units. Within each 30 kilobase unit there were from one up to fifty ESTs and direct-selected cDNA clones which comprised one or more independent transcription units. One or more ESTs or direct-selected cDNAs were used as hybridization probes to determine the length of the mRNA in a variety of tissues, using commercially available reagents (Multiple Tissue Northern blot; Clontech, Palo Alto, California) under conditions recommended by the manufacturer.

Directionally cloned cDNA libraries from femoral bone, and calvarial bone tissue were constructed by methods familiar to one skilled in the art (for example, Soares in Automated DNA Sequencing and Analysis, Adams, Fields and Venter, Eds., Academic Press, NY, pages 110-114 (1994)). Bones were initially broken into fragments with a hammer, and the small pieces were frozen in liquid nitrogen and reduced to a powder in a tissue pulverizer (Spectrum Laboratory Products). RNA was extracted from the powdered bone by homogenizing the powdered bone with a standard Acid Guanidinium Thiocyanate-Phenol-Chloroform extraction buffer (e.g. Chomczynski and Sacchi, Anal. Biochem., 162:156-159 (1987)) using a polytron homogenizer (Brinkman Instruments). Additionally, human brain and lung total RNA was purchased from Clontech. PolyA RNA was isolated from total RNA using dynabeads-dT according to the manufacturer's recommendations (Dynal, Inc.).

First strand cDNA synthesis was initiated using an oligonucleotide primer with the sequence: 5'-AACTGGAAGAATTCGCGGCCGCAGGAATTTTTTTTT TTTTTTTTT-3' (SEQ ID NO:27). This primer introduces a NotI restriction site (underlined) at the 3' end of the cDNA. First and second strand synthesis were performed using the "one-tube" cDNA synthesis kit as described by the anufacturer (Life Technologies, Bethesda, MD). Double stranded cDNAs were treated with T4 polynucleotide kinase to ensure that the ends of the molecules were blunt (Soares in Automated DNA Sequencing and Analysis, Adams, Fields and Venter, Eds., Academic Press, NY, pages 110-114 (1994)), and the blunt ended cDNAs were then size selected by a Biogel column (Huynh et al in DNA Cloning, Vol. 1, Glover, Ed., IRL Press, Oxford, pages 49-78 (1985)) or with a size-sep 400 sepharose column (Pharmacia, catalog # 27-5105-01). Only cDNAs of 400 base pairs or longer were used in subsequent steps. EcoRI adapters (sequence: 5' OH-AATTCGGCACGAG-OH 3' (SEQ ID NO:28), and 5' p-CTCGTGCCG-OH 3' (SEQ ID NO:29)) were then ligated to the double stranded cDNAs by methods familiar to one skilled in the art (Soares, 1994). The EcoRI adapters were then removed from the 3' end of the cDNA by digestion with NotI (Soares, 1994). The cDNA was then ligated into the plasmid vector pBluescript II KS+ (Stratagene, La Jolla, California), and the ligated material was transformed into *E. coli* host DH10B or DH12S by electroporation methods familiar to one skilled in the art (Soares, 1994). After growth overnight at 37°C, DNA was recovered from the *E. coli* colonies after scraping the plates by processing as directed for the Mega-prep kit (Qiagen, Chatsworth, California). The quality of the cDNA libraries was estimated by counting a portion of the total numbers of primary transformants and determining the average insert size and the percentage of plasmids with no cDNA insert. Additional cDNA libraries (human total brain, heart, kidney, leukocyte, and fetal brain) were purchased from Life Technologies, Bethesda, MD.

cDNA libraries, both oligo (dT) and random hexamer (N₆) primed, were used for isolating cDNA clones transcribed within the HBM region: human bone, human brain, human kidney and human skeletal muscle (all cDNA libraries were made by the inventors, except for skeletal muscle (dT) and kidney (dT) cDNA libraries). Four 10 x 10 arrays of each of the cDNA libraries were prepared as follows: the cDNA libraries were titered to 2.5 x 10⁶ using primary transformants. The appropriate volume of frozen stock was used to inoculate 2 L of LB/ampicillin (100 mg/ml). This inoculated liquid culture was aliquotted into 400 tubes of 4 ml each. Each tube contained approximately 5000 cfu. The tubes were incubated at 30°C overnight with gentle agitation. The cultures were grown to an OD of 0.7-0.9. Frozen stocks were prepared for each of the cultures by aliquotting 100 µl of culture and 300 µl of 80% glycerol. Stocks were frozen in a dry ice/ethanol bath and stored at -70°C. The remaining culture was DNA prepared using the Qiagen (Chatsworth, CA) spin miniprep kit according to the manufacturer's instructions. The DNAs from the 400 cultures were pooled to make 80 column and row pools. The cDNA libraries were determined to contain HBM cDNA clones of interest by PCR. Markers were designed to amplify putative exons. Once a standard PCR optimization was performed and specific cDNA libraries were determined to contain cDNA clones of interest, the markers were used to screen the arrayed library. Positive addresses indicating the presence of cDNA clones were confirmed by a second PCR using the same markers.

Once a cDNA library was identified as likely to contain cDNA clones corresponding to a specific transcript of interest from the HBM region, it was manipulated to isolate the clone or clones containing cDNA inserts identical to the EST or direct-selected cDNA of interest. This was accomplished by a modification of the standard "colony screening" method (Sambrook et al, Molecular Cloning: A Laboratory Manual, Cold Spring Harbor Laboratory, Cold Spring Harbor NY (1989)). Specifically, twenty 150 mm LB+ampicillin agar plates were spread with 20,000 colony forming units (cfu) of cDNA library and the colonies allowed to grow overnight at 37°C. Colonies were transferred to nylon filters (Hybond from Amersham, or equivalent) and duplicates prepared by pressing two filters together essentially as described (Sambrook et al, Molecular Cloning: A Laboratory Manual, Cold Spring Harbor Laboratory, Cold Spring Harbor NY (1989)). The "master" plate was then incubated an additional 6-8 hours to allow the colonies to grow back. The DNA from the bacterial colonies was then affixed to the nylon filters by treating the filters sequentially with denaturing solution (0.5 N NaOH, 1.5 M NaCl) for two minutes, neutralization solution (0.5 M Tris-Cl pH 8.0, 1.5,M NaCl) for two minutes (twice). The bacterial colonies were removed from the filters by washing in a solution of 2X SSC/0.1% SDS for one minute while rubbing with tissue paper. The filters were air dried and baked under vacuum at 80°C for 1-2 hours.

A cDNA hybridization probe was prepared by random hexamer labeling (Fineberg and Vogelstein, Anal. Biochem., 132:6-13 (1983)) or by including gene-specific primers and no random hexamers in the reaction (for small fragments). Specific activity was calculated and was >5X10⁸ cpm/10⁸ µg of cDNA. The colony membranes were then prewashed in 10 mM Tris-Cl pH 8.0, 1 M NaCl, 1 mM EDTA, 0.1% SDS for 30 minutes at 55 °C. Following the prewash, the filters were prehybridized in > 2 ml/filter of 6X SSC, 50 % deionized formamide, 2% SDS, 5X Denhardt's solution, and 100 mg/ml denatured salmon sperm DNA, at 42°C for 30 minutes. The filters were then transferred to hybridization solution (6X SSC, 2% SDS, 5X Denhardt's, 100 mg/ml denatured salmon sperm DNA) containing denatured α³²P-dCTP-labeled cDNA probe and incubated at 42°C for 16-18 hours.

After the 16-18 hour incubation, the filters were washed under constant agitation in 2X SSC, 2% SDS at room temperature for 20 minutes, followed by two washes at 65°C for 15 minutes each. A second wash was performed in 0.5 X SSC, 0.5% SDS for 15 minutes at 65 °C. Filters were then wrapped in plastic wrap and exposed to radiographic film for several hours to overnight. After film development, individual colonies on plates were aligned with the autoradiograph so that they could be picked into a 1 ml solution of LB Broth containing ampicillin. After shaking at 37°C for 1-2 hours, aliquots of the solution were plated on 150 mm plates for secondary screening. Secondary screening was identical to primary screening (above) except that it was performed on plates containing ∼250 colonies so that individual colonies could be clearly identified for picking.

After colony screening with radiolabeled probes yielded cDNA clones, the clones were characterized by restriction endonuclease cleavage, PCR, and direct sequencing to confirm the sequence identity between the original probe and the isolated clone. To obtain the full-length cDNA, the novel sequence from the end of the clone identified was used to probe the library again. This process was repeated until the length of the cDNA cloned matches that estimated to be full-length by the northern blot analysis.

RT-PCR was used as another method to isolate full length clones. The cDNA was synthesized and amplified using a "Superscript One Step RT-PCR" kit (Life Technologies, Gaithersburg, MD). The procedure involved adding 1.5 µg of RNA to the following: 25 µl of reaction mix provided which is a proprietary buffer mix with MgSO₄ and dNTP's, 1 µl sense primer (10 µM) and 1 µl anti-sense primer (10 µM), 1 µl reverse transcriptase and Taq DNA polymerase mix provided and autoclaved water to a total reaction mix of 50 µl. The reaction was then placed in a thermocycler for 1 cycle at 50°C for 15 to 30 minutes, then 94°C for 15 seconds, 55-60°C for 30 seconds and 68-72°C for 1 minute per kilobase of anticipated product and finally 1 cycle of 72°C for 5-10 minutes. The sample was analyzed on an agarose gel. The product was excised from the gel and purified from the gel (GeneClean, Bio 101). The purified product was cloned in pCTNR (General Contractor DNA Cloning System, 5 Prime - 3 Prime, Inc.) and sequenced to verify that the clone was specific to the gene of interest.

Rapid Amplification of cDNA ends (RACE) was performed following the manufacturer's instructions using a Marathon cDNA Amplification Kit (Clontech, Palo Alto, CA) as a method for cloning the 5' and 3' ends of candidate genes. cDNA pools were prepared from total RNA by performing first strand synthesis, where a sample of total RNA sample was mixed with a modified oligo (dT) primer, heated to 70°C, cooled on ice and followed by the addition of: 5x first strand buffer, 10 mM dNTP mix, and AMV Reverse Transcriptase (20 U/µl). The tube was incubated at 42°C for one hour and then the reaction tube was placed on ice. For second strand synthesis, the following components were added directly to the reaction tube: 5x second strand buffer, 10 mM dNTP mix, sterile water, 20x second strand enzyme cocktail and the reaction tube was incubated at 16°C for 1.5 hours. T4 DNA Polymerase was added to the reaction tube and incubated at 16°C for 45 minutes. The second-strand synthesis was terminated with the addition of an EDTA/Glycogen mix. The sample was subjected to a phenol/chloroform extraction and an ammonium acetate precipitation. The cDNA pools were checked for quality by analyzing on an agarose gel for size distribution. Marathon cDNA adapters (Clontech) were then ligated onto the cDNA ends. The specific adapters contained priming sites that allowed for amplification of either 5' or 3' ends, depending on the orientation of the gene specific primer (GSP) that was chosen. An aliquot of the double stranded cDNA was added to the following reagents: 10 µM Marathon cDNA adapter, 5x DNA ligation buffer, T4 DNA ligase. The reaction was incubated at 16°C overnight. The reaction was heat inactivated to terminate the reaction. PCR was performed by the addition of the following to the diluted double stranded cDNA pool: 10x cDNA PCR reaction buffer, 10 µM dNTP mix, 10 µM GSP, 10 µM AP1 primer (kit), 50x Advantage cDNA Polymerase Mix. Thermal Cycling conditions were 94 °C for 30 seconds, 5 cycles of 94°C for 5 seconds, 72 °C for 4 minutes, 5 cycles of 94°C for 5 seconds, 70°C for 4 minutes, 23 cycles of 94°C for 5 seconds, 68 °C for 4 minutes. After the first round of PCR was performed using the GSP to extend to the end of the adapter to create the adapter primer binding site, exponential amplification of the specific cDNA of interest was observed. Usually a second nested PCR is performed to confirm the specific cDNA. The RACE product was analyzed on an agarose gel and then excised and purified from the gel (GeneClean, BIO 101). The RACE product was then cloned into pCTNR (General Contractor DNA Cloning System, 5' - 3', Inc.) and the DNA sequence determined to verify that the clone is specific to the gene of interest.

### XI. Mutation Analysis

Comparative genes were identified using the above procedures and the exons from each gene were subjected to mutation detection analysis. Comparative DNA sequencing was used to identify polymorphisms in HBM candidate genes from chromosome 11q12-13. DNA sequences for candidate genes were amplified from patient lymphoblastoid cell lines.

The inventors developed a method based on analysis of direct DNA sequencing of PCR products amplified from candidate regions to search for the causative polymorphism. The procedure consisted of three stages that used different subsets of HBM family to find segregating polymorphisms and a population panel to assess the frequency of the polymorphisms. The family resources result from a single founder leading to the assumption that all affected individuals will share the same causative polymorphism.

Candidate regions were first screened in a subset of the HBM family consisting of the proband, daughter, and her mother, father and brother. Monochromosomal reference sequences were produced concurrently and used for comparison. The mother and daughter carried the HBM polymorphism in this nuclear family, providing the ability to monitor polymorphism transmission. The net result is that two HBM chromosomes and six non-HBM chromosomes were screened. This allowed exclusion of numerous frequent alleles. Only alleles exclusively present in the affected individuals passed to the next level of analysis.

Polymorphisms that segregated exclusively with the HBM phenotype in this original family were then re-examined in an extended portion of the HBM pedigree consisting of two additional nuclear families. These families consisted of five HBM and three unaffected individuals. The HBM individuals in this group included the two critical crossover individuals, providing the centromeric and telomeric boundaries of the critical region. Tracking the heredity of polymorphisms between these individuals and their affected parents allowed for further refining of the critical region. This group brought the total of HBM chromosomes screened to seven and the total of non-HBM chromosomes to seventeen.

When a given polymorphism continued to segregate exclusively with the HBM phenotype in the extended group, a population panel was then examined. This panel of 84 persons consisted of 42 individuals known to have normal bone mineral density and 42 individuals known to be unrelated but with untyped bone mineral density. Normal bone mineral density is within two standard deviations of BMD Z score 0. The second group was from the widely used CEPH panel of individuals. Any segregating polymorphisms found to be rare in this population were subsequently examined on the entire HBM pedigree and a larger population.

Polymerase chain reaction (PCR) was used to generate sequencing templates from the HBM family's DNA and monochromosomal controls. Enzymatic amplification of genes within the HBM region on 11q12-13 was accomplished using the PCR with oligonucleotides flanking each exon as well as the putative 5' regulatory elements of each gene. The primers were chosen to amplify each exon as well as 15 or more base pairs within each intron on either side of the splice. All PCR primers were made as chimeras to facilitate dye primer sequencing. The M13-21F (5'- GTA A CGA CGG CCA GT -3') (SEQ ID NO:30) and -28REV (5'- AAC AGC TAT GAC CAT G -3') (SEQ ID NO:31) primer binding sites were built on to the 5' end of each forward and reverse PCR primer, respectively, during synthesis. 150 ng of genomic DNA was used in a 50 µl PCR with 2 U AmpliTaq, 500 nM primer and 125 µM dNTP. Buffer and cycling conditions were specific to each primer set. TaqStart antibody (Clontech) was used for hot start PCR to minimize primer dimer formation. 10% of the product was examined on an agarose gel. The appropriate samples were diluted 1:25 with deionized water before sequencing.

Each PCR product was sequenced according to the standard Energy Transfer primer (Amersham) protocol. All reactions took place in 96 well trays. 4 separate reactions, one each for A, C, G and T were performed for each template. Each reaction included 2 µl of the sequencing reaction mix and 3 µl of diluted template. The plates were then heat sealed with foil tape and placed in a thermal cycler and cycled according to the manufacturer's recommendation. After cycling, the 4 reactions were pooled. 3 µl of the pooled product was transferred to a new 96 well plate and 1 µl of the manufacturer's loading dye was added to each well. All 96 well pipetting procedures occurred on a Hydra 96 pipetting station (Robbins Scientific, USA). 1 µl of pooled material was directly loaded onto a 48 lane gel running on an ABI 377 DNA sequencer for a 10 hour, 2.4 kV run.

*Polyphred* (University of Washington) was used to assemble sequence sets for viewing with *Consed* (University of Washington). Sequences were assembled in groups representing all relevant family members and controls for a specified target region. This was done separately for each of the three stages. Forward and reverse reads were included for each individual along with reads from the monochromosomal templates and a color annotated reference sequence. *Polyphred* indicated potential polymorphic sites with a purple flag. Two readers independently viewed each assembly and assessed the validity of the purple-flagged sites.

A total of 23 exons present in the mature mRNA and several other portions of the primary transcript were evaluated for heterozygosity in the nuclear family of two HBM-affected and two unaffected individuals. 25 SNPs were identified, as shown in the table below.

**TABLE 4: Single Nucleotide Polymorphisms in the Zmax1 Gene and Environs**

| **Exon Name** | **Location** | **Base Change** |
|---|---|---|
| b200e21-h_Contig1_1.nt | 69169 (309G) | C/A |
| b200e21-h_Contig4_12.nt | 27402 (309G) | A/G |
| b200e21-h_Contig4_13.nt | 27841 (309G) | T/C |
| b200e21-h_Contig4_16.nt | 35600 (309G) | A/G |
| b200e21-h_Contig4_21.nt | 45619 (309G) | G/A |
| b200e21-h_Contig4_22.nt-a | 46018 (309G) | T/G |
| b200e21-h_Contig4_22.nt-b | 46093 (309G) | T/G |
| b200e21-h_Contig4_22.nt-c | 46190 (309G) | A/G |
| b200e21-h_Contig4_24.nt-a | 50993 (309G) | T/C |
| b200e21-h_Contig4_24.nt-b | .51124 (309G) | C/T |
| b200e21-h_Contig4_25.nt | 55461 (309G) | C/T |
| b200e21-h_Contig4_33.nt-a | 63645 (309G) | C/A |
| b200e21-h_Contig4_33.nt-b | 63646 (309G) | A/C |
| b200e21-h_Contig4_61.nt | 24809 (309G) | T/G |
| b200e21-h_Contig4_62.nt | 27837 (309G) | T/C |
| b200e21-h_Contig4_63.nt-a | 31485(309G) | C/T |
| b200e21-h_Contig4_63.nt-b | 31683 (309G) | A/G |
| b200e21-h_Contig4_9.nt | 24808 (309G) | T/G |
| b527d12-h_Contig030g_1.nt-a | 31340 (308G) | T/C |
| b527d12-h_Contig030g_1.nt-b | 32538 (308G) | A/G |
| b527d12-h_Contig080C_2.nt | 13224 (308G) | A/G |
| b527d12-h_Contig087C_1.nt | 21119 (308G) | C/A |
| b527d12-h_Contig087C_4.nt | 30497 (308G) | G/A |
| b527d12-h_Contig088C_4.nt | 24811 (309G) | A/C |
| b527d12-h_Contig089_1HP.nt | 68280 (309G) | G/A |

In addition to the polymorphisms presented in Table 4, two additional polymorphisms can also be present in SEQ ID NO:2. These is a change at position 2002 of SEQ ID NO:2. Either a guanine or an adenine can appear at this position. This polymorphism is silent and is not associated with any change in the amino acid sequence. The second change is at position 4059 of SEQ ID NO:2 corresponding in a cytosine (C) to thymine (T) change. This polymorphism results in a corresponding amino acid change from a valine (V) to an alanine (A). Other polymorphisms were found in the candidate gene exons and adjacent intron sequences. Any one or combination of the polymorphisms listed in Table 4 or the two discussed above could also have a minor effect on bone mass when present in SEQ ID NO:2.

The present invention encompasses the nucleic acid sequences having the nucleic acid sequence of SEQ ID NO: 1 with the above-identified point mutations.

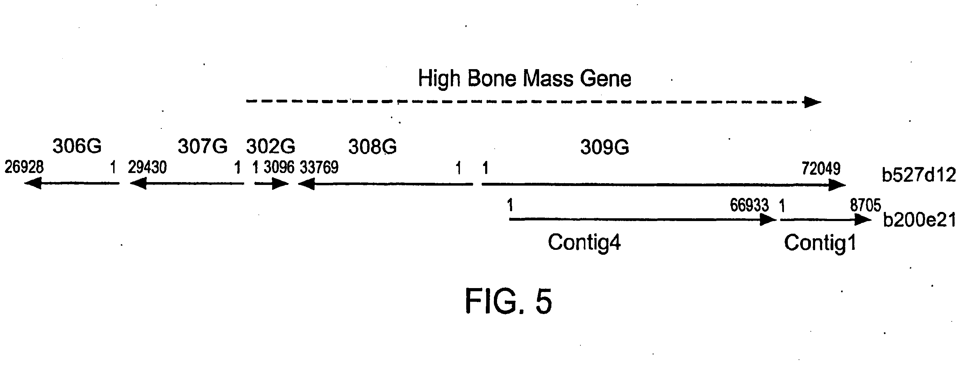

Preferably, the present invention encompasses the nucleic acid of SEQ ID NO: 2. Specifically, a base-pair substitution changing G to T at position 582 in the coding sequence of Zmax1 (the HBM gene) was identified as heterozygous in all HBM individuals, and not found in the unaffected individuals (i.e., b527d12-h_Contig087C_1.nt). Fig. 5 shows the order of the contigs in B527D12. The direction of transcription for the HBM gene is from left to right. The sequence of contig308G of B527D12 is the reverse complement of the coding region to the HBM gene. Therefore, the relative polymorphism in contig 308G shown in Table 4 as a base change substitution of C to A is the complement to the G to T substitution in the HBM gene. This mutation causes a substitution of glycine 171 with valine (G171V).

The HBM polymorphism was confirmed by examining the DNA sequence of different groups of individuals. In all members of the HBM pedigree (38 individuals), the HBM polymorphism was observed in the heterozygous form in affected (i.e., elevated bone mass) individuals only (N=18). In unaffected relatives (N=20) (BMDZ<2.0) the HBM polymorphism was never observed. To determine whether this polymorphism was ever observed in individuals outside of the HBM pedigree, 297 phenotyped individuals were characterized at the site of the HBM gene. None were heterozygous at the site of the HBM polymorphism. In an unphenotyped control group, none of 64 individuals were observed to be heterozygous at position 582. Taken together, these data prove that the polymorphism observed in the kindred displaying the high bone mass phenotype is strongly correlated with the G→T polymorphism at position 582 of Zmax1. Furthermore, these results coupled with the ASO results described below, establish that the HBM polymorphism genetically segregates with the HBM phenotype, and that both the HBM polymorphism and phenotype are rare in the general population.

### XII. Allele Specific Oligonucleotide (ASO) Analysis

The amplicon containing the HBM1 polymorphism was PCR amplified using primers specific for the exon of interest. The appropriate population of individuals was PCR amplified in 96 well microtiter plates as follows. PCR reactions (20 µl) containing 1X Promega PCR buffer (Cat. # M1883 containing 1.5 mM MgCl₂), 100mM dNTP, 200 nM PCR primers (1863F:
CCAAGTTCTGAGAAGTCC and 1864R: AATACCTGAAACCATACCTG), 1 U Amplitaq, and 20 ng of genomic DNA were prepared and amplified under the following PCR conditions: 94°C, 1 minute, (94°C, 30 sec.; 58°C, 30 sec.; 72°C, 1 min.) X35 cycles), 72°C, 5', 4°C, hold. Loading dye was then added and 10 µl of the products was electrophoresed on 1.5% agarose gels containing 1 µg/ml ethidium bromide at 100-150 V for 5-10 minutes. Gels were treated 20 minutes in denaturing solution (1.5 M NaCl, 0.5 N NaOH), and rinsed briefly with water. Gels were then neutralized in 1 M Tris-HCl, pH 7.5, 1.5 M NaCl, for 20 minutes and rinsed with water. Gels were soaked in 10 X SSC for 20 minutes and blotted onto nylon transfer membrane (Hybond N+- Amersham) in 10X SSC overnight. Filters were the rinsed in 6X SSC for 10 minutes and UV crosslinked.

The allele specific oligonucleotides (ASO) were designed with the polymorphism approximately in the middle. Oligonucleotides were phosphate free at the 5' end and were purchased from Gibco BRL. Sequences of the oligonucleotides are:
2326 Zmax1.ASO.g: AGACTGGGGTGAGACGC
2327 Zmax1.ASO.t: CAGACTGGGTTGAGACGCC
The polymorphic nucleotides are underlined. To label the oligos, 1.5 µl of 1 µg/µl ASO oligo (2326.Zmax1.ASO.g or 2327.Zmax1.ASO.t), 11 µl ddH₂O, 2 µl 10X kinase forward buffer, 5 µl γ³²P-ATP (6000 Ci/mMole), and 1 µl T4 polynucleotide kinase (10 U/µl) were mixed, and the reaction incubated at 37°C for 30-60 minutes. Reactions were then placed at 95 °C for 2 minutes and 30 ml H₂O was added. The probes were purified using a G25 microspin column (Pharmacia).

Blots were prehybridized in 10 ml 5X SSPE, 5X Denhardt's, 2% SDS, and 100 µg/ml, denatured, sonicated salmon sperm DNA at 40°C for 2 hr. The entire reaction mix of kinased oligo was then added to 10 ml fresh hybridization buffer (5X SSPE, 5X Denhardt's, 2% SDS) and hybridized at 40°C for at least 4 hours to overnight.

All washes done in 5X SSPE, 0.1 % SDS. The first wash was at 45 °C for 15 minutes; the solution was then changed and the filters washed 50°C for 15 minutes. Filters were then exposed to Kodak biomax film with 2 intensifying screens at -70°C for 15 minutes to 1 hr. If necessary the filters were washed at 55°C for 15 minutes and exposed to film again. Filters were stripped by washing in boiling 0.1X SSC, 0.1% SDS for 10 minutes at least 3 times.

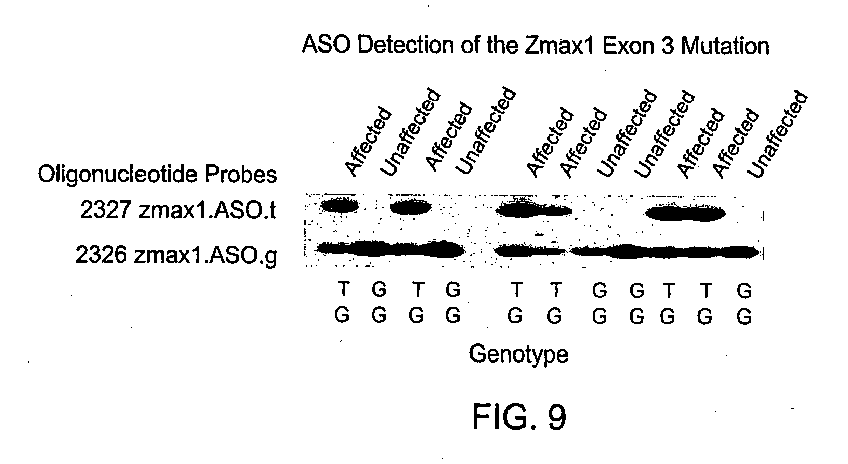

The two films that best captured the allele specific assay with the 2 ASOs were converted into digital images by scanning them into Adobe PhotoShop. These images were overlaid against each other in Graphic Converter and then scored and stored in FileMaker Pro 4.0 (see Fig. 9).

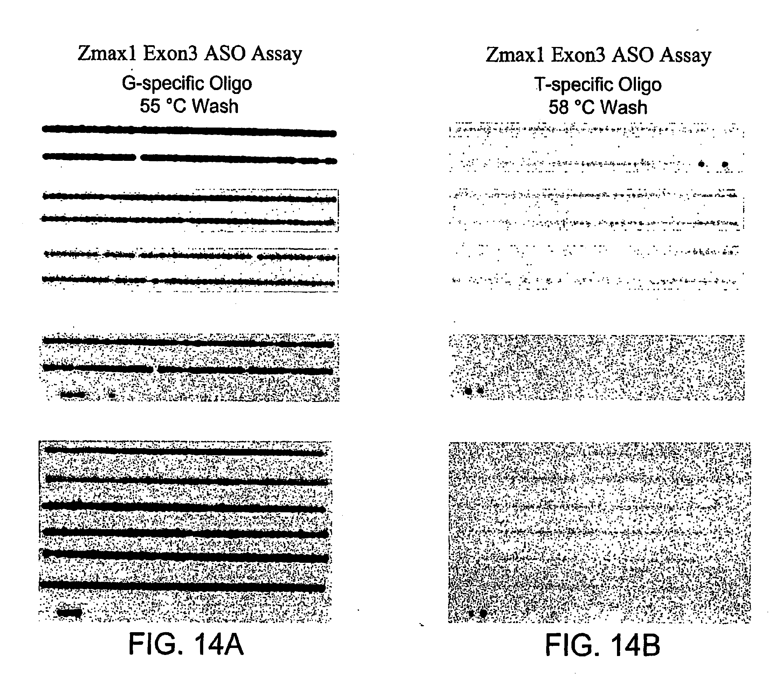

In order to determine the HBM1 allele frequency in ethnically diverse populations, 672 random individuals from various ethnic groups were typed by the allele specific oligonucleotide (ASO) method. This population included 96 CEPH grandparents (primarily Caucasian), 192 Caucasian, 192 African-American, 96 Hispanic, and 96 Asian individuals. No evidence was obtained for the presence of the HBM1 polymorphism in any of these individuals. Overall, a total of 911 individuals were typed either by direct sequencing or ASO hybridization; all were homozygous GG at the site of the HBM polymorphism (Fig. 14). This information illustrates that the HBM1 allele is rare in various ethnic populations.

Thus this invention provides a rapid method of identifying individuals with the HBM1 allele. This method could be used in the area of diagnostics and screening of an individual for susceptibility to osteoporosis or other bone disorder. The assay could also be used to identify additional individuals with the HBM1 allele or the additional polymorphisms described herein.

### XIII. Cellular Localization of Zmax1

### A. Gene Expression in Rat tibia by non isotopic In Situ Hybridization

*In situ* hybridization was conducted by Pathology Associates International (PAI), Frederick, MD. This study was undertaken to determine the specific cell types that express the Zmax1 gene in rat bone with particular emphasis on areas of bone growth and remodeling. Zmax1 probes used in this study were generated from both human (HuZmax1) and mouse (MsZmax1) cDNAs, which share an 87% sequence identity. The homology of human and mouse Zmax1 with rat Zmax1 is unknown.

For example, gene expression by non-isotopic *in situ* hybridization was performed as follows, but other methods would be known to the skilled artisan. Tibias were collected from two 6 to 8 week old female Sprague Dawley rats euthanized by carbon dioxide asphyxiation. Distal ends were removed and proximal tibias were snap frozen in OCT embedding medium with liquid nitrogen immediately following death. Tissues were stored in a -80°C freezer.

Probes for amplifying PCR products from cDNA were prepared as follows. The primers to amplify PCR products from a cDNA clone were chosen using published sequences of both human LRP5 (Genbank Accession No. ABO17498) and mouse LRP5 (Genbank Accession No. AFO64984). In order to minimize cross reactivity with other genes in the LDL receptor family, the PCR products were derived from an intracellular portion of the protein coding region. PCR was performed in a 50 µl reaction volume using cDNA clone as template. PCR reactions contained 1.5 mM MgCl₂, 1 unit Amplitaq, 200 µM dNTPs and 2 µM each primer. PCR cycling conditions were 94°C for 1 min., followed by 35 cycles of 94°C for 30 seconds, 55°C for 30 seconds, 72°C for 30 seconds; followed by a 5 minute extension at 72°C. The reactions were then run on a 1.5% agarose Tris-Acetate gel. DNA was eluted from the agarose, ethanol precipitated and resuspended in 10 mM Tris, pH 8.0. Gel purified PCR products were prepared for both mouse and human cDNAs and supplied to Pathology Associates International for *in situ* hybridizations.

The sequence of the human and mouse PCR primers and products were as follows:
Human Zmax 1 sense primer (HBM1253)
   CCCGTGTGCTCCGCCGCCCAGTTC
Human Zmax 1 antisense primer (HBM1465)
   GGCTCACGGAGCTCATCATGGACTT
Human Zmax1 PCR product
Mouse Zmax 1 Sense primer (HBM1655)
   AGCGAGGCCACCATCCACAGG
Mouse Zmax 1 antisense primer (HBM1656)
   TCGCTGGTCGGCATAATCAAT
Mouse Zmax1 PCR product

Riboprobes were synthesized as follows. The PCR products were reamplified with chimeric primers designed to incorporate either a T3 promoter upstream, or a T7 promoter downstream of the reamplification products. The resulting PCR products were used as template to synthesize digoxigenin-labeled riboprobes by *in vitro* transcription (IVT). Antisense and sense riboprobes were synthesized using T7 and T3 RNA polymerases, respectively, in the presence of digoxigenin-11-UTP (Boehringer-Mannheim) using a MAXIscript IVT kit (Ambion) according to the manufacturer. The DNA was then degraded with Dnase-1, and unincorporated digoxigenin was removed by ultrafiltration. Riboprobe integrity was assessed by electrophoresis through a denaturing polyacrylamide gel. Molecular size was compared with the electrophoretic mobility of a 100-1000 base pair (bp) RNA ladder (Ambion). Probe yield and labeling was evaluated by blot immunochemistry. Riboprobes were stored in 5 µl aliquots at -80°C.

The *in situ* hybridization was performed as follows. Frozen rat bone was cut into 5 µM sections on a Jung CM3000 cryostat (Leica) and mounted on adhesive slides (Instrumedics). Sections were kept in the cryostat at 20°C until all the slides were prepared in order to prevent mRNA degradation prior to post-fixation for 15 minutes in 4% paraformaldehyde. Following post-fixation, sections were incubated with 1 ng/µl of either antisense or sense riboprobe in Pathology Associates International (PAI) customized hybridization buffer for approximately 40 hours at 58°C. Following hybridization, slides were subjected to a series of post-hybridization stringency washes to reduce nonspecific probe binding. Hybridization was visualized by immunohistochemistry with an anti-digoxigenin antibody (FAB fragment) conjugated to alkaline phosphatase. Nitroblue tetrazolium chloride/bromochloroindolyl phosphate (Boehringer-Mannheim), a precipitating alkaline phosphatase substrate, was used as the chromogen to stain hybridizing cells purple to nearly black, depending on the degree of staining. Tissue sections were counter-stained with nuclear fast red. Assay controls included omission of the probe, omission of probe and anti-digoxigenin antibody.

Specific cell types were assessed for demonstration of hybridization with antisense probes by visualizing a purple to black cytoplasmic and/or peri-nuclear staining indicating a positive hybridization signal for mRNA. Each cell type was compared to the replicate sections, which were hybridized with the respective sense probe. Results were considered positive if staining was observed with the antisense probe and no staining or weak background with the sense probe.

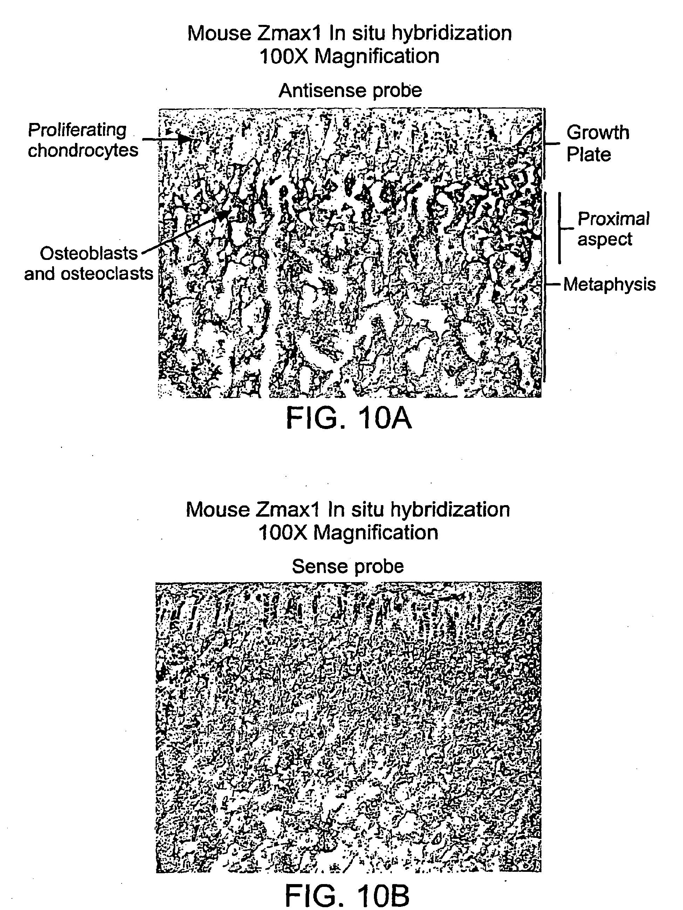

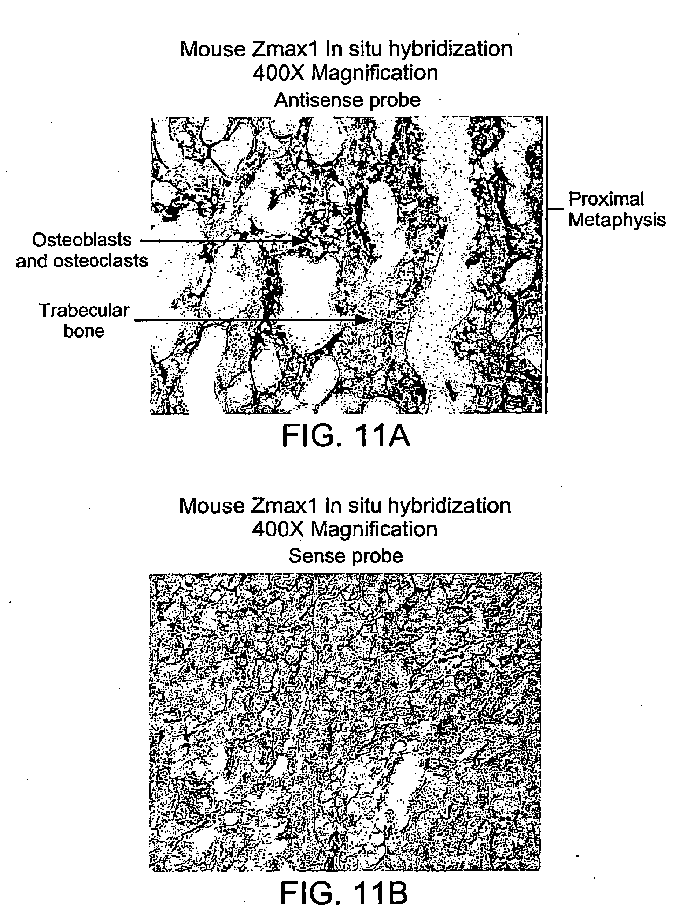

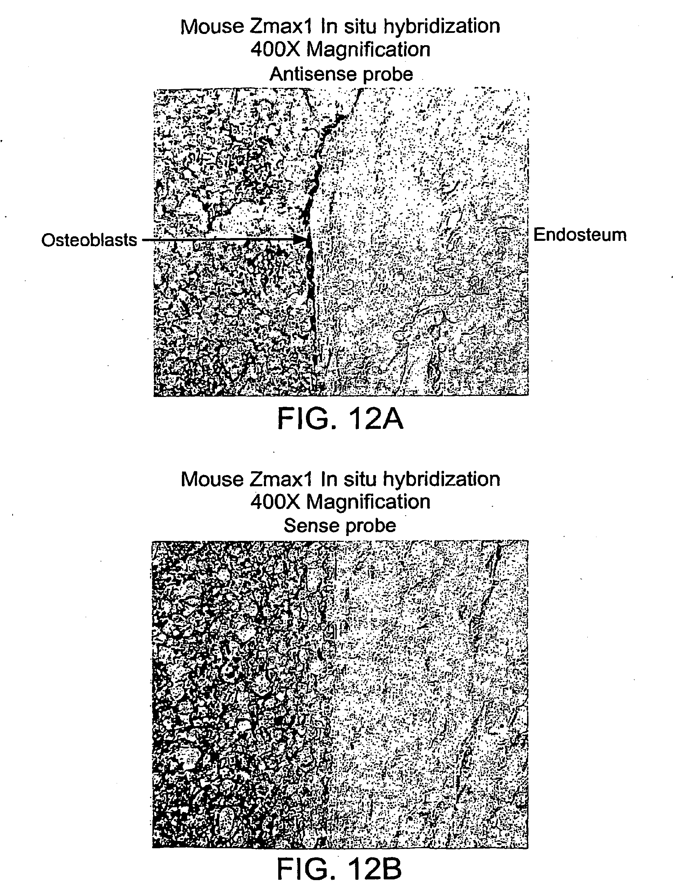

The cellular localization of the hybridization signal for each of the study probes is summarized in Table 5. Hybridization for Zmax1 was primarily detected in areas of bone involved in remodeling, including the endosteum and trabecular bone within the metaphysis. Hybridization in selected bone lining cells of the periosteum and epiphysis were also observed. Positive signal was also noted in chondrocytes within the growth plate, particularly in the proliferating chondrocytes. See Figs. 10, 11 and 12 for representative photomicrographs of *in situ* hybridization results.

**TABLE 5 Summary of Zmax1 in situ hybridization in rat tibia**

| **PROBE** | **SITE** | **ISH SIGNAL** |
|---|---|---|
| Hu Zmax1 | Epiphysis | |
| | Osteoblasts | + |
| | Osteoclasts | _ |
| | Growth Plate | |
| | resting chondrocytes | - |
| | proliferating chondrocytes | + |
| | hypertrophic chondrocytes | - |
| | Metaphysis | |
| | osteoblasts | + |
| | osteoclasts | + |
| | Diaphysis | - |
| | Endosteum | |
| | osteoblasts | + |
| | osteoclasts | + |
| | Periosteum | - |
| | | |
| MsZmax1 | Epiphysis | |
| | Osteoblasts | + |
| | Osteoclasts | - |
| | Growth Plate | |
| | resting chondrocytes | - |
| | proliferating chondrocytes | + |
| | hypertrophic chondrocytes | + |
| | Metaphysis | |
| | osteoblasts | + |
| | osteoclasts | + |
| | Diaphysis | - |
| | Endosteum | |
| | osteoblasts | + |
| | osteoclasts | + |
| | Periosteum | + |
| | | |

| | | |
|---|---|---|
| Legend: "+" = hybridization signal detected "-" = no hybridization signal detected "ISH" - *In situ* hybridization | | |

These studies confirm the positional expression of Zmax1 in cells involved in bone remodeling and bone formation. Zmax1 expression in the zone of proliferation and in the osteoblasts and osteoclasts of the proximal metaphysis, suggests that the Zmax1 gene is involved in the process of bone growth and mineralization. The activity and differentiation of osteoblasts and osteoclasts are closely coordinated during development as bone is formed and during growth as well as in adult life as bone undergoes continuous remodeling. The formation of internal bone structures and bone remodeling result from the coupling of bone resorption by activated osteoclasts with subsequent deposition of new material by osteoblasts. Zmax1 is related to the LDL receptor gene, and thus may be a receptor involved in mechanosensation and subsequent signaling in the process of bone remodeling. Therefore, changes in the level of expression of this gene could impact on the rate of remodeling and degree of mineralization of bone.

### XIV. Antisense

Antisense oligonucleotides are short synthetic nucleic acids that contain complementary base sequences to a targeted RNA. Hybridization of the RNA in living cells with the antisense oligonucleotide interferes with RNA function and ultimately blocks protein expression. Therefore, any gene for which the partial sequence is known can be targeted by an antisense oligonucleotide.

Antisense technology is becoming a widely used research tool and will play an increasingly important role in the validation and elucidation of therapeutic targets identified by genomic sequencing efforts.

Antisense technology was developed to inhibit gene expression by utilizing an oligonucleotide complementary to the mRNA that encodes the target gene. There are several possible mechanisms for the inhibitory effects of antisense oligonucleotides. Among them, degradation of mRNA by RNase H is considered to be the major mechanism of inhibition of protein function. This technique was originally used to elucidate the function of a target gene, but may also have therapeutic applications, provided it is designed carefully and properly.

An example of materials and methods for preparing antisense oligonucleotides can be performed as follows. Preliminary studies have been undertaken in collaboration with Sequiter (Natick, MA) using the antisense technology in the osteoblast-like murine cell line, MC3T3. These cells can be triggered to develop along the bone differentiation sequence. An initial proliferation period is characterized by minimal expression of differentiation markers and initial synthesis of collagenous extracellular matrix. Collagen matrix synthesis is required for subsequent induction of differentiation markers. Once the matrix synthesis begins, osteoblast marker genes are activated in a clear temporal sequence: alkaline phosphatase is induced at early times while bone sialoprotien and osteocalcin appear later in the differentiation process. This temporal sequence of gene expression is useful in monitoring the maturation and mineralization process. Matrix mineralization, which does not begin until several days after maturation has started, involves deposition of mineral on and within collagen fibrils deep within the matrix near the cell layer-culture plate interface. The collagen fibril-associated mineral formed by cultured osteoblasts resembles that found in woven bone in vivo and therefore is used frequently as a study reagent.

MC3T3 cells were transfected with antisense oligonucleotides for the first week of the differentiation, according to the manufacturer's specifications (U.S. Patent No. 5,849,902).

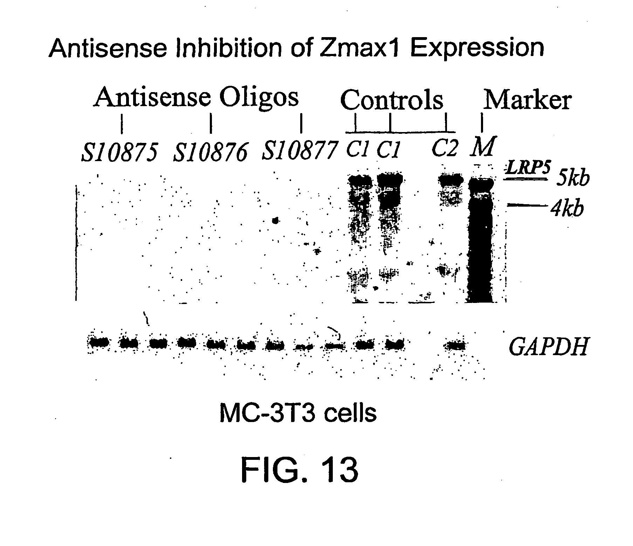

The oligonucleotides designed for Zmax1 are given below:
**10875:** AGUACAGCUUCUUGCCAACCCAGUC
**10876:** UCCUCCAGGUCGAUGGUCAGCCCAU
**10877:** GUCUGAGUCCGAGUUCAAAUCCAGG
Fig. 13 shows the results of antisense inhibition of Zmax1 in MC3T3 cells. The three oligonucleotides shown above were transfected into MC3T3 and RNA was isolated according to standard procedures. Northern analysis clearly shows markedly lower steady state levels of the Zmax1 transcript while the control gene GAPDH remained unchanged. Thus, antisense technology using the primers described above allows for the study of the role of Zmax1 expression on bone biology.

### XV. Yeast Two Hybrid

In order to identify the signaling pathway that Zmax1 participates in to modulate bone density, the yeast two hybrid protein interaction technology was utilized. This technique facilitates the identification of proteins that interact with one another by coupling tester proteins to components of a yeast transcription system (Fields and Song, 1989, Nature 340: 245-246; U.S. Pat. No. 5,283,173 by Fields and Song; Johnston, 1987, Microbiol. Rev. 51: 458-476; Keegan et al, 1986, Science 231: 699-704; Durfee et al, 1993, Genes Dev. 7: 555-569; Chien et al, 1991, Proc. Natl. Acad. Sci USA 88: 9578-9582; Fields et al., 1994, Trends in Genetics 10: 286-292; and Gyuris et al., 1993, Cell 75: 791-803). First a "bait" protein, the protein for which one seeks interacting proteins, is fused to the DNA binding domain of a yeast transcription factor. Second, a cDNA library is constructed that contains cDNAs fused to the transcriptional activation domain of the same yeast transcription factor; this is termed the prey library. The bait construct and prey library are transformed into yeast cells and then mated to produce diploid cells. If the bait interacts with a specific prey from the cDNA library, the activation domain is brought into the vicinity of the promoter via this interaction. Transcription is then driven through selectable marker genes and growth on selective media indicates the presence of interacting proteins.

The amino acid sequence used in the yeast two hybrid experiments discussed herein consisted of the entire cytoplasmic domain and a portion of the transmembrane domain and is shown below (amino to carboxy orientation):

The last 6 amino acids of the putative transmembrane domain are indicated in bold. Putative SH3 domains are underlined. Additional amino acid sequences of 50 amino acids or greater in either the proteins encoded by the Zmax1 or HBM alleles can also be used as bait. The upper size of the polypeptide used as bait is limited only by the presence of a complete transmembrane domain (see Fig. 4), which will render the bait to be nonfunctional in a yeast two hybrid system. These additional bait proteins can be used to identify additional proteins which interact with the proteins encoded by HBM or Zmax1 in the focal adhesion signaling pathway or in other pathways in which these HBM or Zmax1 proteins may act. Once identified, methods of identifying agents which regulate the proteins in the focal adhesion signaling pathway or other pathways in which HBM acts can be performed as described herein for the HBM and Zmax1 proteins.

In order to identify cytoplasmic Zmax1 signaling pathways, the cytoplasmic domain of Zmax1 was subcloned into two bait vectors. The first vector was pDBleu, which was used to screen a brain, and Hela prey cDNA library cloned into the vector pPC86 (Clontech). The second bait vector used was pDBtrp, which was used to screen a cDNA prey library derived from the TE85 osteosarcoma cell line in vector pOP46. Standard techniques known to those skilled in the art were used as described in Fields and Song, 1989, Nature 340: 245-246; U.S. Pat. No. 5,283,173 by Fields and Song; Johnston, 1987, Microbiol. Rev. 51: 458-476; Keegan et al., 1986, Science 231: 699-704; Durfee et al., 1993, Genes Dev. 7: 555-569; Chien et al., 1991, Proc. Natl. Acad. Sci USA 88: 9578-9582; Fields et al., 1994, Trends in Genetics 10: 286-292; and Gyuris et al., 1993, Cell 75: 791-803. The bait construct and prey cDNA libraries were transformed into yeast using standard procedures.

To perform the protein interaction screen, an overnight culture of the bait yeast strain was grown in 20 ml SD selective medium with 2% glucose (pDBLeu, SD -Leu medium, pDBtrp, SD -trp medium). The cultures were shaken vigorously at 30°C overnight. The cultures were diluted 1 : 10 with complete medium (YEPD with 2% glucose) and the cultures then incubated with shaking for 2 hrs at 30°C.

The frozen prey library was thawed, and the yeast cells reactivated by growing them in 150 ml YEPD medium with 2% glucose for 2 hrs at 30°C. A filter unit was sterilized with 70% ethanol and washed with sterile water to remove the ethanol. The cell densities of both bait and prey cultures were measured by determining the OD at 600 nm. An appropriate volume of yeast cells that corresponded to a cell number of 1 ml of OD 600 = 4 of each yeast strain, bait and prey (library) was placed in a 50 ml Falcon tube. The mixture was then filtered through the sterilized filter unit. The filter was then transferred onto a prewarmed YEPD agar plate with the cell side up, removing all air bubbles underneath the filter. Plates were then incubated at 30 °C for 6 hrs. One filter was transferred into a 50 ml Falcon tube, and 10 ml of SD with 2% Glucose was added; cells were resuspended by vortexing for 10 sec.

The number of primary diploid cells (growth on SD -Leu, -Trp plates) versus the numbers of colony forming units growing on SD -Trp and SD -Leu plates only was then titered. Different dilutions were plated and incubated at 30°C for two days. The number of colony forming units was then counted. The number of diploid colonies (colonies on SD -Leu -Trp plates) permits the calculation of whether or not the whole library of prey constructs was mated to the yeast expressing the bait. This information is important to judge the quality of the screen.

### A. Indirect selection

Resuspended cells from 5 filtermatings were then pooled and the cells sedimented by centrifugation in a 50 ml Falcon tube. Cells were then resuspended in 16 ml SD medium with 2% Glc. Two ml of this cell suspension was plated onto 8 square plates each (SD -Leu, -Trp) with sterile glass beads and selected for diploid cells by incubating at 30°C for 18 - 20 hrs.

Cells were then scraped off the square plates, the cells centrifuged and combined into one 50 ml Falcon tube. The cell pellet was then resuspended in 25 ml of SD medium with 2% glucose. The cell number was then determined by counting of an appropriate dilution (usually 1:100 to 1:1000) with a Neugebauer chamber. Approximately 5 x 10⁷ diploid cells were plated onto the selective medium. The observations about the growth of the bait strain together with irrelevant prey vectors helps to determine which selective plates will have to be chosen for the library screen. Generally, all screens were plated on one square plate each with SD -Leu, - Trp, -His; SD -Leu, -Trp, His, 5 mM 3AT, and SD -Leu, -Trp, -His, -Ade is recommended.

The yeast cells were then spread homogeneously with sterile glass beads and incubated at 30°C for 4 days. The number of colony forming yeast cells was titered by plating different dilutions of the scraped cell suspension onto SD -Leu, -Trp plates. Usually, plating of 100 µl of a 10⁻³ and 10⁻⁴ dilution gave 100 -1000 colonies per plate.

### B. Direct selection

Five filters with the mated yeast cells were each transferred into separate 50 ml Falcon tubes and the cells resuspended with 10 ml SD medium with 2% Glc, each, followed by vortexing for 10 sec. The resuspended cells were combined and centrifuged in a Beckman centrifuge at 3000 rpm. The supernatant was discarded and the cells resuspended in 6 ml of SD medium with 2% Glc. Two ml of the suspension was spread onto each selective square plate and incubated at 30°C for 4 - 5 days.

### C. Isolation of Single Colonies

Yeast cells from an isolated colony were picked with a sterile tooth pick and transferred into individual wells of a 96 well plate. The cells were resuspended in 50 µl of SD -Leu, -Trp, -His medium and incubated at 30°C for one day. The yeast cells were then stamped onto a SD -Leu, -Trp, -His plate in 96 well format and incubated at 30°C for 2 days. Yeast cells were also stamped onto a Nylon filter covering a YEPD plate and incubated at 30°C for one day. The cells on the Nylon filter were used for the analysis of the β - Gal reporter activity.

Yeast colonies were scraped from the SD -Leu, -Trp, -His plate with a sterile tooth pick, and reconfigured, if necessary, according to the β - Gal activity and then resuspended in 20 % glycerol. This served as a master plate for storage at -80°C.

For DNA preparation, yeast cells from the glycerol stock were stamped onto a SD -Trp plate and incubated at 30°C for 2 days. After two days of incubation, the yeast colonies were ready for colony PCR and sequencing. Standard colony PCR conditions were used to amplify inserts from preys recovered from the interaction screen. Sequencing was done using standard sequencing reactions and ABI377 (Perkin Elmer) fluorescent sequencing machines.

### D. Verification of baitlprey interaction

Glycerol stocks of the prey of interest were thawed and inoculated in a 10 ml overnight culture of SD with glucose -Trp. After overnight growth, plasmid DNA preparation was performed using the BIO 101 RPM Yeast Plasmid Isolation Kit with 10 ml of culture. The culture was centrifuged and transfered to a 1.5 ml microcentrifuge tube. Yeast Lysis Matrix was then added to the pellet followed by 250 µl of Alkaline Lysis Solution. Samples were then vortexed for 5 minutes. 250 µl Neutralizing Solution was added and the sample mixed briefly. Samples were centrifuged for 2 minutes at room temperature in a microcentrifuge. The supernatant was transferred to a Spin Filter avoiding debris and Lysis Matrix. 250 µl of Glassmilk Spin Buffer was added, and the tubes inverted to mix. Samples were centrifuged for 1 min and the liquid in the Catch Tube was discarded. 500 µl of Wash Solution was added, the samples were centrifuged for 1 min, and the wash solution was discarded. The wash step was repeated once followed by a 1 min dry centrifugation to drive the remaining liquid out of the Spin Filter. The filter was transferred to a new Catch Tube and 100 µl of sterile H₂O was added; samples were then vortexed briefly to resuspend and centrifuged for 30 seconds to collect the DNA in the bottom of the Catch Tube.

Five µl of DNA was then transformed into DH10B Electromax cells using standard procedures and glycerol stocks prepared. Miniprep DNA was prepared using the Qiagen QIAprep Spin Miniprep Kit. DNA was finally eluted with 30 µl of Qiagen EB buffer. One µl of the plasmid DNA samples was then used to transform yeast cells using standard procedures. After 2 days of growth on SD -trp media, colonies were picked and patched onto fresh media. Similarly, bait colonies were patched onto SD -Leu media. Both were grown overnight at 30°C.

For mating, cells from bait and prey patches were spread together on YAPD media and incubated at 30°C for 12 hr. This plate was then replicaplated onto an SD Agar-Leu-Trp plate and grown for 2 days at 30°C. To test the strength of interaction these plates were replicaplated onto SD Agar-Leu-Trp-His, SD Agar-Leu-Trp-His with 5 mM 3AT and 10 mM 3AT, SD Agar-Leu-Trp-His-Ade, and SD Agar-Leu-Trp-Ura media and grown for 2 days at 30°C.

### E. Galacton Star β-Galactosidase Activity Assay

After streaking and replica plating positive interactors on selection plates, colonies were placed in a 96 well dish with 200 µl of SD-medium, leaving wells 1 and 96 blank. Ten microliters from the first 96 well dish was plated into another flat bottom 96 well dish containing 100 µl of SD-medium. Controls consisted of a negative control and a very weak positive control. The cell density was measured at OD₆₀₀ (a value of 1 corresponds to 1x10⁷ cells utilizing a 96 well spectrophotometer). The OD was usually between 0.03 and 0.10. Using microplates specifically for the luminometer, 50 µl of reaction mixture were pipetted into each well. Fifty microliters of culture were then added and mixed by pipetting up and down twice. The reaction was incubated for 30 minutes at room temperature followed by measurement of Relative Light Units using a luminometer.

Table 6 lists the genes identified in the yeast two hybrid screens from the 3 prey libraries tested. Two genes, zyxin and axin, were found to interact with the cytoplasmic domain of Zmax1 in all three screens. Three genes, alpha-actinin, TCB and S1-5 interacted in two of the three screens.

A variety of proteins found at sites of cell-cell and cell-matrix contact (focal contacts/adesion plaques) were shown to interact with the cytoplasmic domain of Zmax1. These include alpha-actinin, Trio, Pinch-like protein, and Zyxin. PINCH is a LIM domain-containing protein that is known to interact with integrin-linked kinase, an early signaler in integrin and growth factor signaling pathways. The finding of a closely related gene in the yeast two hybrid screen raises the possibility of a novel pathway linked to integrin signaling from extracellular matrix signals. Trio, also known to localize to focal adhesions, is thought to play a key role in coordinating cell-matrix interactions and cytoskeletal rearrangements involved in cell movement. Zyxin, another LIM domain-containing protein, is also localized to adhesion plaques and is thought to be involved in reorganization of the cytoskeleton when triggers are transmitted via integrin signaling pathways. Zyxin also interacts with alpha actinin, which we identified as interacting with Zmax1. Other LIM domain containing proteins identified include the human homologue of mouse ajuba, LIMD1, and a novel LIMD1-like protein.

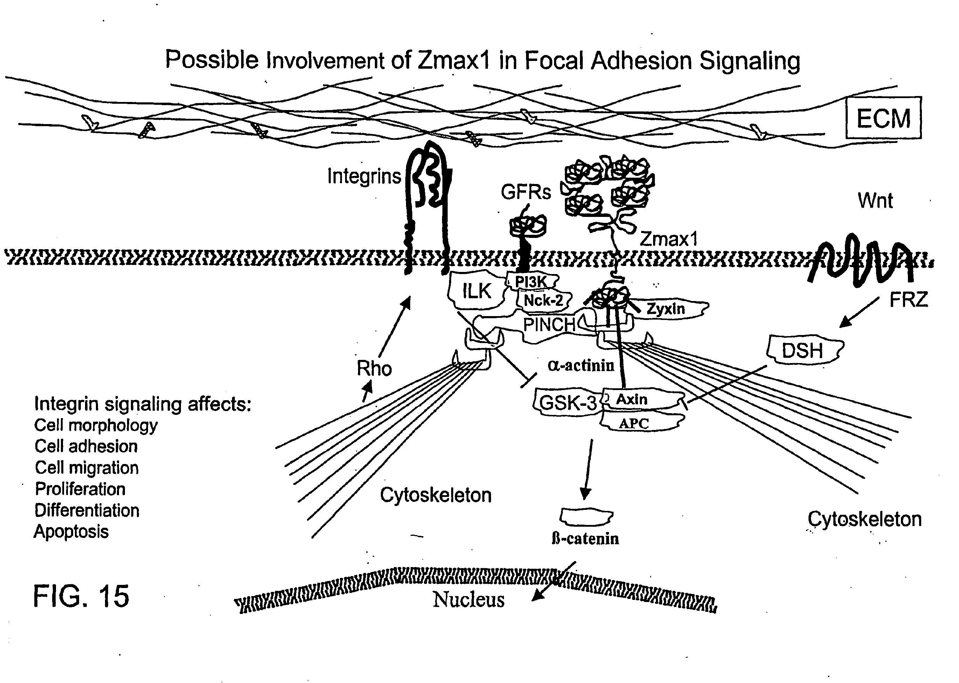

Axin was also identified from the two hybrid experiments. This protein is involved in inhibition of the Wnt signaling pathway and interacts with the tumor suppressor APC. There is a link here with the focal adhesion signaling described above: one common step in the two pathways involves inhibition of glycogen synthase kinase 3, which in turn results in the activation of β-catenin/Lef-1 and AP-1 transcription factors. Axin/APC are involved in this as well as integrin linked kinase. The Wnt pathway has a role in determining cell fates during embryogenesis. If inappropriately activated, the Wnt pathway may also lead to cancer. The Wnt pathway also seems to have a role in cytoskeletal rearrangements. A model depicting Zmax1 involvement in focal adhesion signaling is depicted in Fig. 15.

This data coupled with other studies suggest that integrin signaling pathways have a role in cellular responses to mechanical stress and adhesion. This provides an attractive model for the mechanism of action of Zmax1 in bone biology. It is possible that Zmax1 is involved in sensing either mechanical stress directly or binding a molecule in the extracellular matrix that is related to mechanical sensation. Signaling through subsequent pathways may be involved in bone remodeling due to effects on cell morphology, cell adhesion, migration, proliferation, differentiation, and apoptosis in bone cells.

**Table 6: Yeast Two Hybrid Results**

| **Gene Symbol** | **Gene** | **Genbank Accession #** | **NT SEQ ID NO:** | **AA SEQ ID NO:** |
|---|---|---|---|---|
| ACTN1 | alpha-actinin | NM_001102 | 63 | |
| AES | amino-teminal enhancer of | NM_001130.3 | 64 | |
| AIP4 | atrophin-1 interacting protein | AF038564.1 | 65 | |
| Novel | Ajuba | | 66 | |
| AXIN | Wnt signaling | AF009674.1 | 67 | |
| CDC23 | cell division cycle 23, yeast, homolog | NM_004661.1 | 68 | |
| HSM800944 | Similar to TRIO | AL117435.1 | 69 | |
| HSM800936 | | AL117427.1 | 70 | |
| Novel | Similar to LIM domains containing protein 1 | | 71 | |
| DEEPEST | mitotic spindle coiled-coil related protein | NM_006461.1 | 72 | |
| ECM1 | extracellular matrix protein 1 | U65932.1 | 73 | |
| EF1A | elongation factor 1-alpha | X16869.1 | 74 | |
| FN | fibronectin | X02761.1 | 75 | |
| HOXB 13 | homeodomain protein | U81599.1 | 76 | |
| Novel | Glu-Lys Rich protein | | 77 | |
| LIMD1 | LIM domains containing 1 | NM_014240.1 | 78 | |
| Novel | PINCH-like | | 79 | |
| RANBPM | centrosomal protein | NM_005493.1 | 80 | |
| Sl-5 | extracellular protein | U03877.1 | 81 | |
| TCB | gene encoding cytosolic thyroid hormone-binding | M26252.1 | 82 | |
| TID | tumorous imaginal discs | NM_005147.1 | 83 | |
| ZYX | Zyxin | NM_003461.1 | 84 | |
| TRIO | GTPase | U42390.1 | 85 | |
| HUMPITPB | phosphatidylinositol transfer protein | D30037.1 | 86 | |
| ACTN1 | alpha-actinin | NP_001093.1 | | 87 |
| AES | amino-terminal enhancer of | NP_001121.2 | | 88 |
| AIP4 | atrophin-1 interacting protein | AAC04845.1 | | 89 |
| Novel | Ajuba | | | 90 |
| AXIN | Wnt signalling | AAC51624.1 | | 91 |
| CDC23 | cell division cycle 23, yeast homolog | NP_004652.1 | | 92 |
| Novel | Similar to TRIO CAB55923.1 | | | 93 |
| Novel | Similar to LIM domains containing protein 1 | | | 94 |
| DEEPEST | mitotic spindle coiled-coil related protein | NP_006452.1 | | 95 |
| ECM1 | extracellular matrix protein 1 | AAB05933.1 | | 96 |
| EF1A | elongation factor 1-alpha | CAA34756.1 | | 97 |
| FN | fibronectin | CAA26536.1 | | 98 |
| Novel | Glu-Lys rich protein | | | 99 |
| HOXB13 | homeodomain protein B13 | AAB39863.1 | | 100 |
| LIMD1 | LIM domains containing 1 | Np_055055.1 | | 101 |
| Novel | PINCH-like | | | 102 |
| RANBPM | centrosomal protein | NP_005484.1 | | 103 |
| S1-5 | extracellular protein | AAA65590.1 | | 104 |
| TCB | cytosolic thyroid hormone-binding protein | AAA36672.1 | | 105 |
| TID | tumorous imaginal discs | NP_005138.1 | | 106 |
| ZYX | Zyxin | NP_003452.1 | | 107 |
| TRIO | GTPase | AAC34245.1 | | 108 |
| PTDINSTP | phosphatidylinositol transfer protein beta isoform | P48739 | | 109 |

In light of the model depicted in Fig. 15 and the results shown in Table 6, another aspect contemplated by the invention would be to regulate bone density and bone mass disorders by the regulating focal adhesion signaling. The regulation can occur by regulating the DNA, mRNA transcript or protein encoded by any of the members involved in the focal adhesion signaling pathway as identified by the yeast two hybrid system.

Also contemplated are the novel nucleic acids and proteins identified by the HBM. yeast two hybrid system. These include but are not limited to SEQ ID NO: 66 (Ajuba), SEQ ID NO: 71 (a gene similar to a gene encoding LIM domains containing protein 1), SEQ ID NO: 77 (Glu-Lys Rich protein), SEQ ID NO: 79 (PINCH-like gene), SEQ ID NO: 90 (Ajuba protein), SEQ ID NO: 93 (protein similar to TRIO), SEQ ID NO: 94 0, SEQ ID NO: 99 (Glu-Lys rich protein) and SEQ ID NO: 102 (PINCH-like protein).

### XVI. Potential Function

The protein encoded by Zmax1 is related to the Low Density Lipoprotein receptor (LDL receptor). *See*, Goldstein et al, Ann. Rev. Cell Biology, 1:1-39 (1985); Brown et al, Science, 232:34-47 (1986). The LDL receptor is responsible for uptake of low density lipoprotein, a lipid-protein aggregate that includes cholesterol. Individuals with a defect in the LDL receptor are deficient in cholesterol removal and tend to develop artherosclerosis. In addition, cells with a defective LDL receptor show increased production of cholesterol, in part because of altered feedback regulation of cholesterol synthetic enzymes and in part because of increased transcription of the genes for these enzymes. In some cell types, cholesterol is a precursor for the formation of steroid hormones.

Thus, the LDL receptor may, directly or indirectly, function as a signal transduction protein and may regulate gene expression. Because Zmax1 is related to the LDL receptor, this protein may also be involved in signaling between cells in a way that affects bone remodeling.

The glycine 171 amino acid is likely to be important for the function of Zmax1 because this amino acid is also found in the mouse homologue of Zmax1. The closely related LRP6 protein also contains glycine at the corresponding position (Brown et al, Biochemical and Biophysical Research Comm., 248:879-888 (1988)). Amino acids that are important in a protein's structure or function tend to be conserved between species, because natural selection prevents mutations with altered amino acids at important positions from arising.

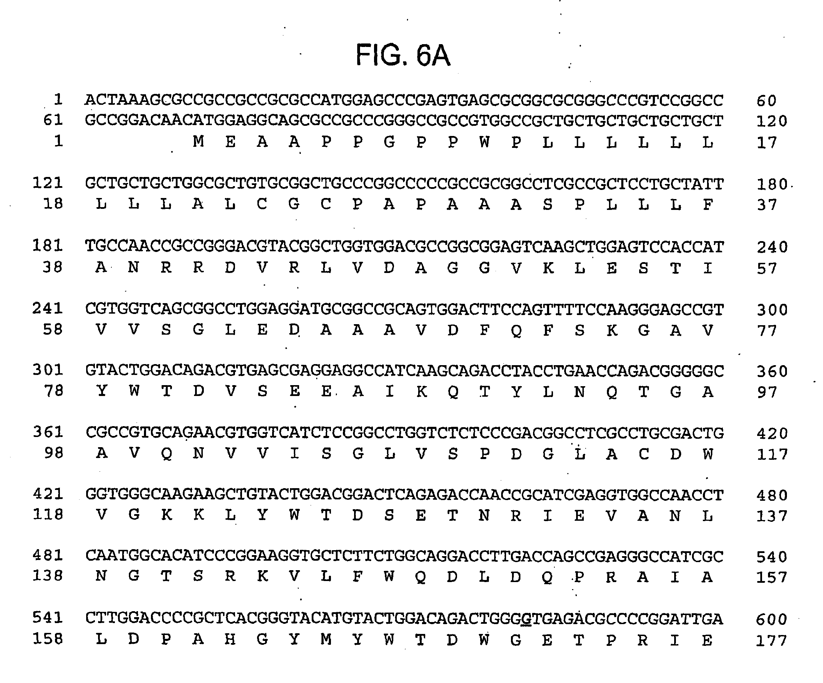

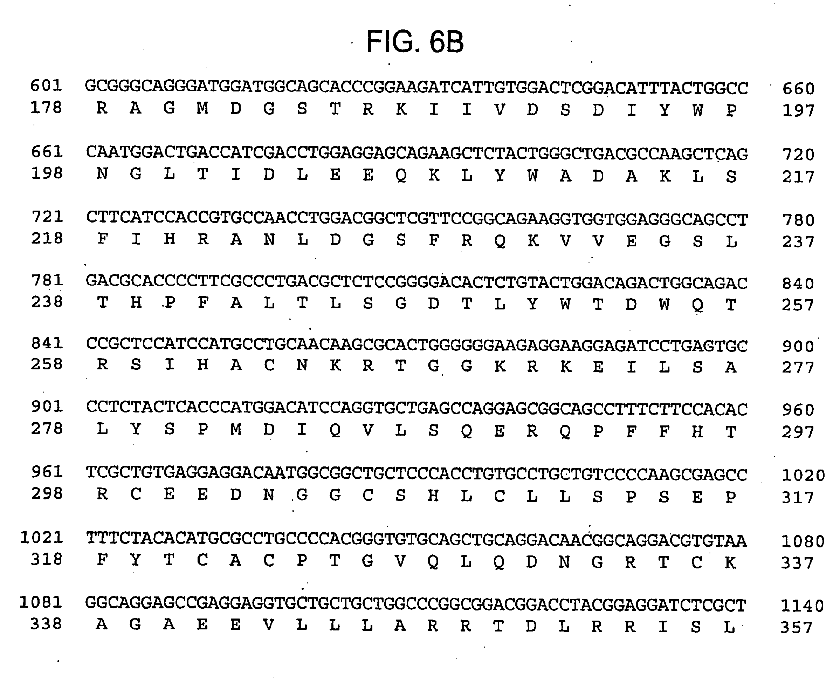

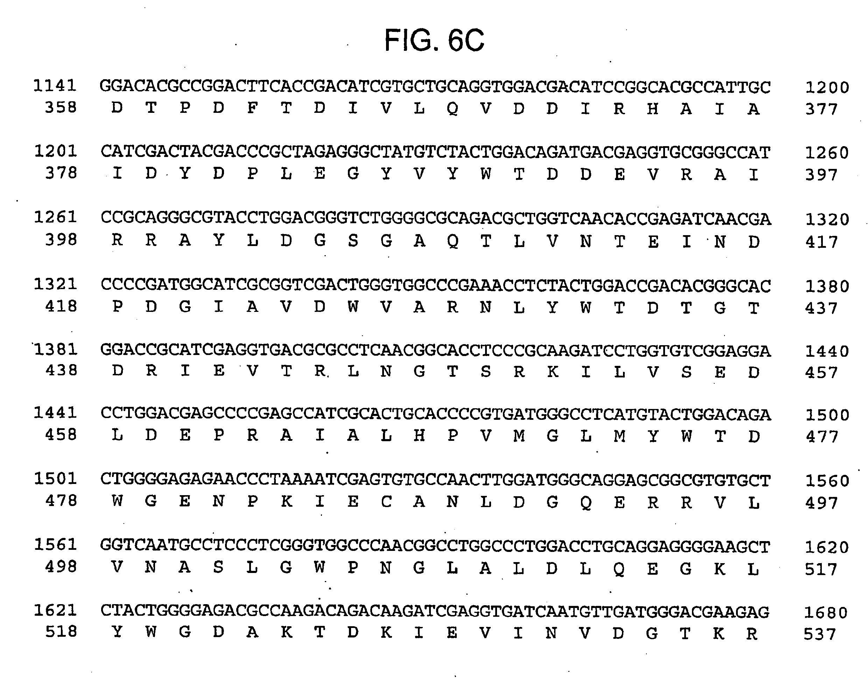

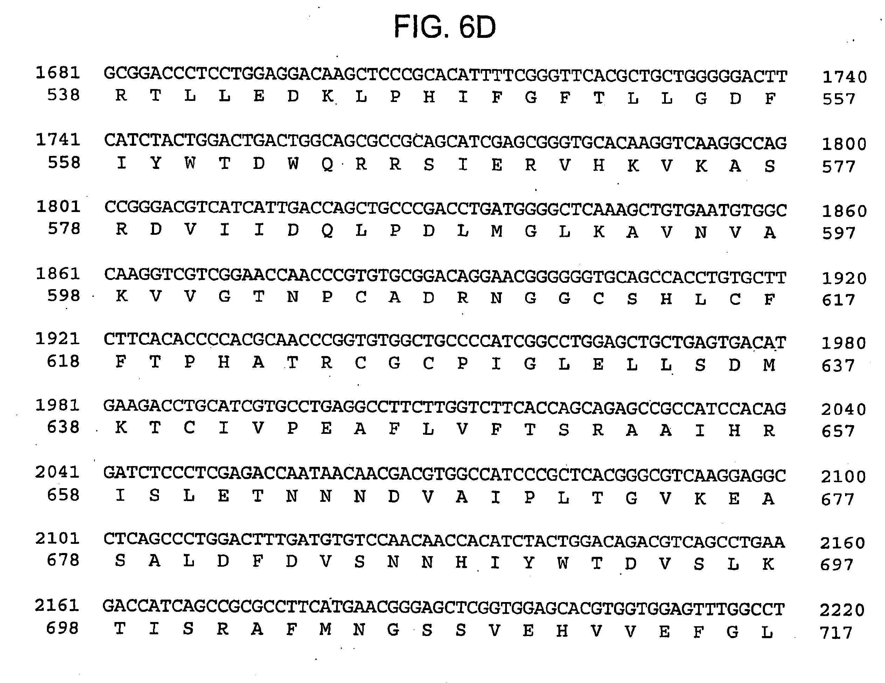

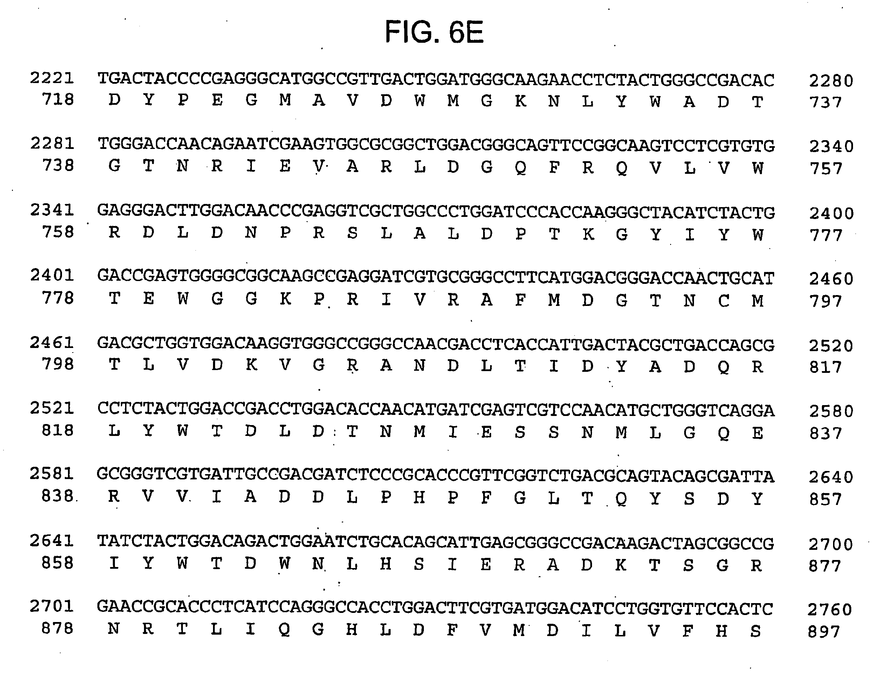

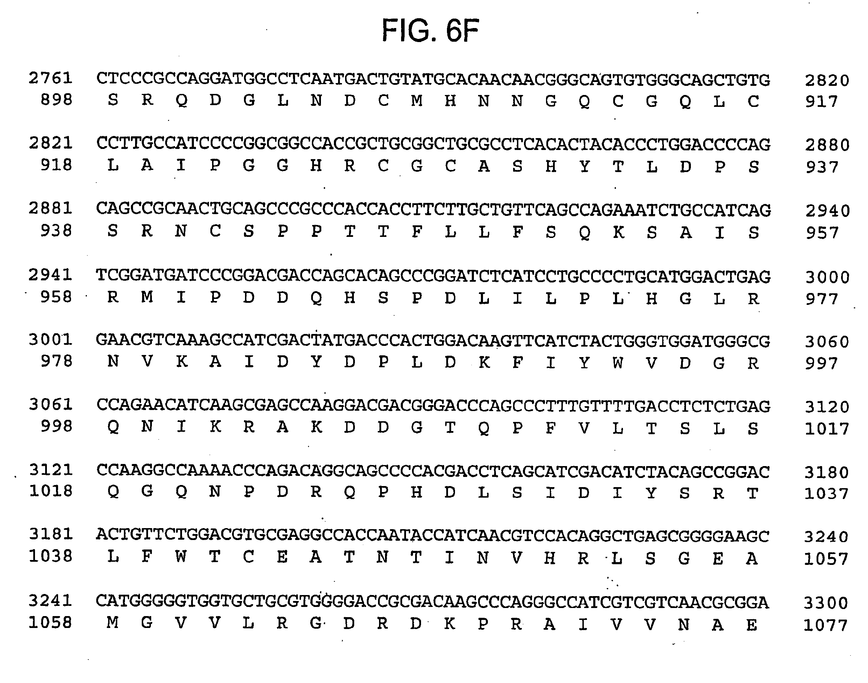

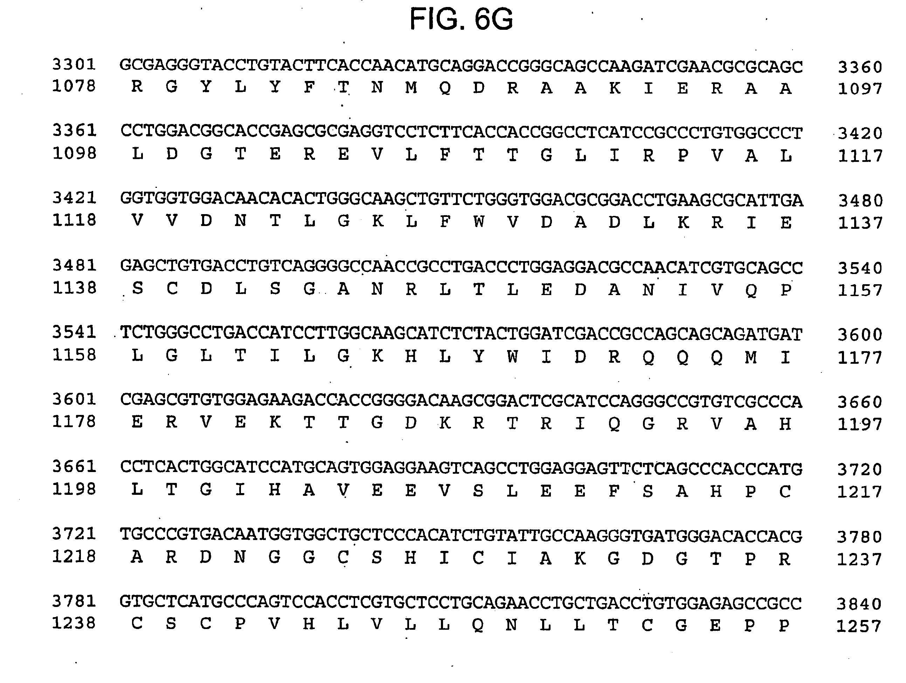

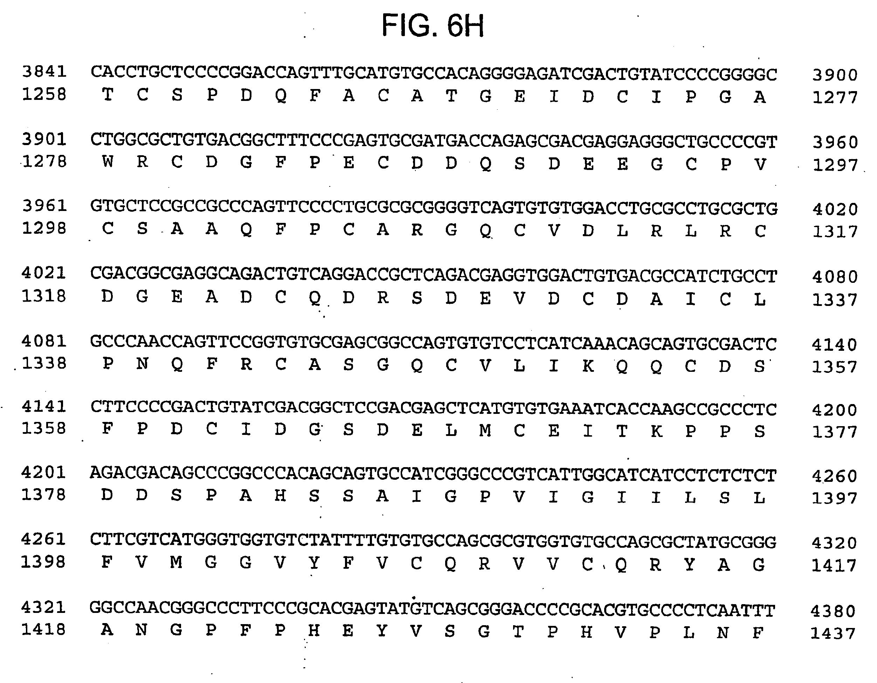

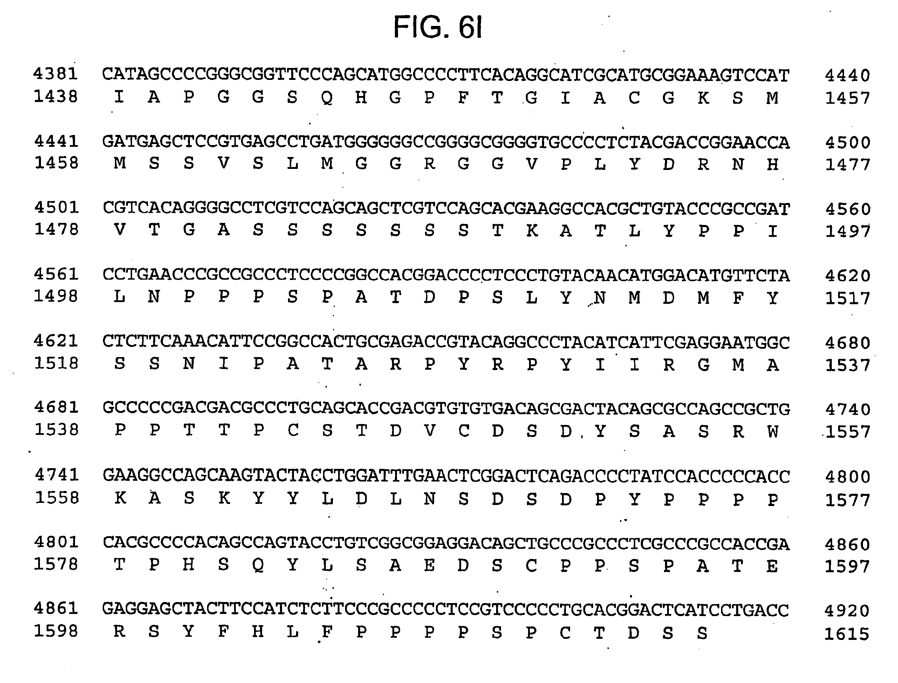

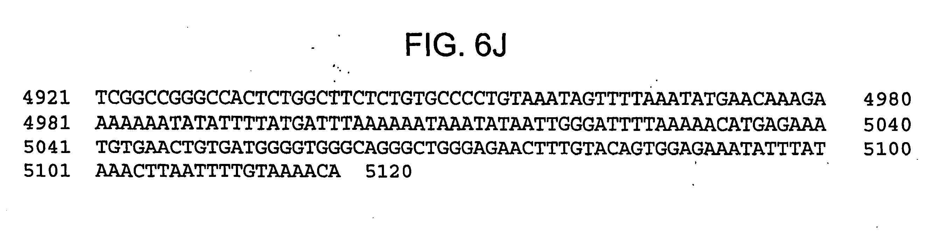

In addition, the extracellular domain of Zmax1 contains four repeats consisting of five YWTD motifs followed by an EFG motif. This 5YWTD+EGF repeat is likely to form a distinct folded protein domain, as this repeat is also found in the LDL receptor and other LDL receptor-related proteins. The first three 5YWTD+EGF repeats are very similar in their structure, while the fourth is highly divergent. Glycine 171 occurs in the central YWTD motif of the first 5YWTD+EGF repeat in Zmax1. The other two similar 5YWTD+EGF repeats of Zmax1 also contain glycine at the corresponding position, as does the 5YWTD+EGF repeat in the LDL receptor protein. However, only 17.6% of the amino acids are identical among the first three 5YWTD+EGF repeats in Zmax1 and the single repeat in the LDL receptor. These observations indicate that glycine 171 is essential to the function of this repeat, and mutation of glycine 171 causes a functional alteration of Zmax1. The cDNA and peptide sequences are shown in Figs. 6A-6E. The critical base at nucleotide position 582 is indicated in bold and is underlined.

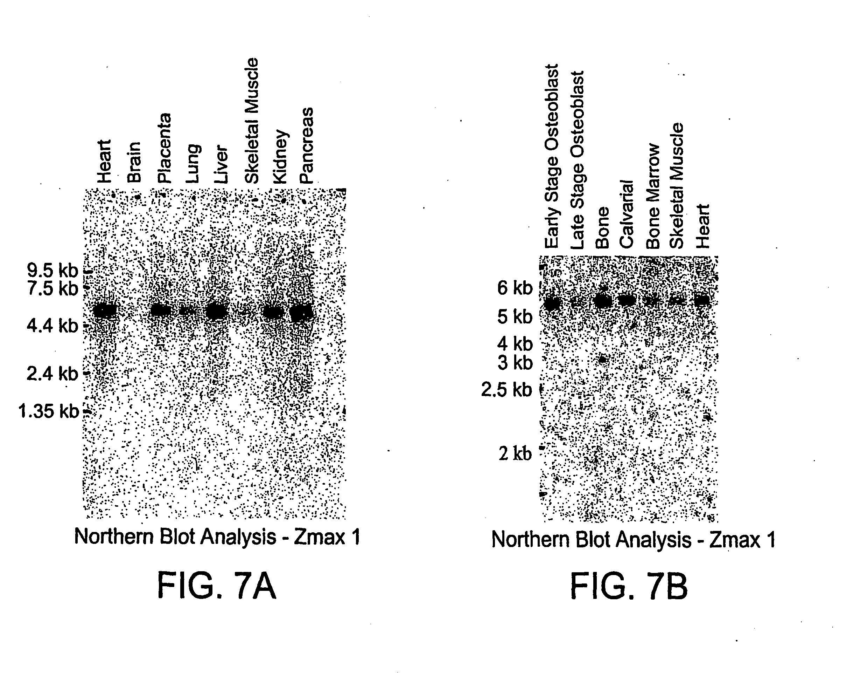

Northern blot analysis (Figs. 7A-B) reveals that Zmax1 is expressed in human bone tissue as well as numerous other tissues. A multiple-tissue Northern blot (Clontech, Palo Alto, CA) was probed with exons from Zmax1. As shown in Fig. 7A, the 5.5 kb Zmax1 transcript was highly expressed in heart, kidney, lung, liver and pancreas and is expressed at lower levels in skeletal muscle and brain. A second northern blot, shown in Fig. 7B, confirmed the transcript size at 5.5 kb, and indicated that Zmax1 is expressed in bone, bone marrow, calvaria and human osteoblastic cell lines.

Taken together, these results coupled with the yeast two hybrid results indicate that the HBM polymorphism in the Zmax1 gene is responsible for the HBM phenotype, and that the Zmax1 gene is important in bone development. In addition, because mutation of Zmax1 can alter bone mineralization and development, it is likely that molecules that bind to Zmax1 may usefully alter bone development. Such molecules may include, for example, small molecules, proteins, RNA aptamers, peptide aptamers, and the like.

### XVII. Preparation of Nucleic Acids, Vectors, Transformations and Host Cells

Large amounts of the nucleic acids of the present invention may be produced by replication in a suitable host cell. Natural or synthetic nucleic acid fragments coding for a desired fragment will be incorporated into recombinant nucleic acid constructs, usually DNA constructs, capable of introduction into and replication in a prokaryotic or eukaryotic cell. Usually the nucleic acid constructs will be suitable for replication in a unicellular host, such as yeast or bacteria, but may also be intended for introduction to (with and without integration within the genome) cultured mammalian or plant or other eukaryotic cell lines. The purification of nucleic acids produced by the methods of the present invention is described, for example, in Sambrook et al, Molecular Cloning. A Laboratory Manual, 2nd Ed. (Cold Spring Harbor Laboratory, Cold Spring Harbor, NY (1989) or Ausubel et al, Current Protocols in Molecular Biology, J. Wiley and Sons, NY (1992).

The nucleic acids of the present invention may also be produced by chemical synthesis, e.g., by the phosphoramidite method described by Beaucage et al, Tetra. Letts., 22:1859-1862 (1981) or the triester method according to Matteucci,et al, J. Am. Chem. Soc., 103:3185 (1981), and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

Nucleic acid constructs prepared for introduction into a prokaryotic or eukaryotic host cell may comprise a replication system recognized by the host, including the intended nucleic acid fragment encoding the desired protein, and will preferably also include transcription and translational initiation regulatory sequences operably linked to the protein encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals may also be included where appropriate, whether from a native HBM or Zmax1 protein or from other receptors or from secreted proteins of the same or related species, which allow the protein to cross and/or lodge in cell membranes, and thus attain its functional topology, or be secreted from the cell. Such vectors may be prepared by means of standard recombinant techniques well known in the art and discussed, for example, in Sambrook et al, Molecular Cloning. A Laboratory Manual, 2nd Ed. (Cold Spring Harbor Laboratory, Cold Spring Harbor, NY (1989) or Ausubel et al, Current Protocols in Molecular Biology, J. Wiley and Sons, NY (1992).

An appropriate promoter and other necessary vector sequences will be selected so as to be functional in the host cell, and may include, when appropriate, those naturally associated with Zmax1 or HBM genes. Examples of workable combinations of cell lines and expression vectors are described in Sambrook et al, Molecular Cloning. A Laboratory Manual, 2nd Ed. (Cold Spring Harbor Laboratory, Cold Spring Harbor, NY (1989) or Ausubel et al, Current Protocols in Molecular Biology, J. Wiley and Sons, NY (1992). Many useful vectors are known in the art and may be obtained from such vendors as Stratagene, New England BioLabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in EP 73,675A. Appropriate non-native mammalian promoters might include the early and late promoters from SV40 (Fiers et al, Nature, 273:113 (1978)) or promoters derived from murine Moloney leukemia virus, mouse tumor virus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences, see also Enhancers and Eukaryotic Gene Expression, Cold Spring Harbor Press, Cold Spring Harbor, NY (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encode proteins that a) confer resistance to antibiotics or other toxic substances, e.g. ampicillin, neomycin, methotrexate, etc.; b) complement auxotrophic deficiencies, or c) supply critical nutrients not available from complex media, e.g., the gene encoding D-alanine racemase for Bacilli. The choice of the proper selectable marker will depend on the host cell, and appropriate markers for different host cells are well known in the art.

The vectors containing the nucleic acids of interest can be transcribed *in vitro*, and the resulting RNA introduced into the host cell by well-known methods, e.g., by injection (see, Kubo et al, FEBS Letts. 241:119 (1989)), or the vectors can be introduced directly into host cells by methods well known in the art, which vary depending on the type of cellular host, including electroporation; transfection employing calcium chloride, rubidium chloride, calcium phosphate, DEAE-dextran, or other substances; microprojectile bombardment; lipofection; infection (where the vector is an infectious agent, such as a retroviral genome); and other methods. See generally, Sambrook et al., 1989 and Ausubel et al., 1992. The introduction of the nucleic acids into the host cell by any method known in the art, including those described above, will be referred to herein as "transformation." The cells into which have been introduced nucleic acids described above are meant to also include the progeny of such cells.

Large quantities of the nucleic acids and proteins of the present invention may be prepared by expressing the HBM nucleic acid or portions thereof in vectors or other expression vehicles in compatible prokaryotic or eukaryotic host cells. The most commonly used prokaryotic hosts are strains of *Escherichia coli*, although other prokaryotes, such as *Bacillus subtilis* or *Pseudomonas* may also be used.

Mammalian or other eukaryotic host cells, such as those of yeast, filamentous fungi, plant, insect, or amphibian or avian species, may also be useful for production of the proteins of the present invention. Propagation of mammalian cells in culture is per se well known. See, Jakoby and Pastan (eds.), Cell Culture. Methods in Enzymology, volume 58, Academic Press, Inc., Harcourt Brace Jovanovich, NY, (1979)). Examples of commonly used mammalian host cell lines are VERO and HeLa cells, Chinese hamster ovary (CHO) cells, and WI38, BHK, and COS cell lines, although it will be appreciated by the skilled practitioner that other cell lines may be appropriate, e.g., to provide higher expression desirable glycosylation patterns, or other features.

Clones are selected by using markers depending on the mode of the vector construction. The marker may be on the same or a different DNA molecule, preferably the same DNA molecule. In prokaryotic hosts, the transformant may be selected, e.g., by resistance to ampicillin, tetracycline or other antibiotics. Production of a particular product based on temperature sensitivity may also serve as an appropriate marker.

Prokaryotic or eukaryotic cells transformed with the nucleic acids of the present invention will be useful not only for the production of the nucleic acids and proteins of the present invention, but also, for example, in studying the characteristics of Zmax1 or HBM proteins.

Antisense nucleic acid sequences arc useful in preventing or diminishing the expression of Zmax1 or HBM, as will be appreciated by one skilled in the art. For example, nucleic acid vectors containing all or a portion of the Zmax1 or HBM gene or other sequences from the Zmax1 or HBM region may be placed under the control of a promoter in an antisense orientation and introduced into a cell. Expression of such an antisense construct within a cell will interfere with Zmax1 or HBM transcription and/or translation and/or replication.

The probes and primers based on the Zmax1 and HBM gene sequences disclosed herein are used to identify homologous Zmax1 and HBM gene sequences and proteins in other species. These Zmax1 and HBM gene sequences and proteins are used in the diagnostic/prognostic, therapeutic and drug screening methods described herein for the species from which they have been isolated.

### XVILL. Protein Expression and Purification

Expression and purification of the HBM protein of the invention can be performed essentially as outlined below, To facilitate the cloning, expression and purification of membrane and secreted protein from the HBM gene, a gene expression system, such as the pET System (Novagen), for cloning and expression of recombinant proteins in E. *coli* was selected. Also, a DNA sequence encoding a peptide tag, the His-Tap, was fused to the 3' end of DNA sequences of interest to facilitate purification of the recombinant protein products. The 3' end was selected for fusion to avoid alteration of any 5 terminal signal sequence.

Nucleic acids chosen, for example, from the nucleic acids set forth in SEQ , ID NOS: 1, 3 and 5-12 for cloning HBM were prepared by polymerase chain reaction (PCR). Synthetic oligonucleotide primers specific for the 5' and 3' ends of the HBM nucleotide sequence were designed and purchased from Life Technologies (Gaithersburg, MD). All forward primers (specific for the 5' end of the sequence) were designed to include an NcoI cloning site at the 5' terminus. These primers were designed to permit initiation of protein translation at the methionine residue encoded within the NcoI site followed by a valine residue and the protein encoded by the HBM DNA sequence. All reverse primers (Specific for the 3' end of the sequence) included an EcoRI site at the 5' terminus to permit cloning of the HBM sequence into the reading frame of the pET-28b. The pET-28b vector provided a sequence encoding an additional 20 carboxyl-teminal amino acids including six histidine residues (at the C-terminus), which comprised the histidine affinity tag.

Genomic DNA prepared from the HBM gene was used as the source of template DNA for PCR amplification (Ausubel et al, Current Protocols in Molecular Biology, John Wiley & Sons (1994)). To amplify a DNA sequence containing the HBM nucleotide sequence, genomic DNA (50 ng) was introduced into a reaction vial containing 2 mM MgCl₂, 1 µM synthetic oligonucleotide primers (forward and reverse primers) complementary to and flanking a defined HBM, 0.2 mM of each of deoxynucleotide triphosphate, dATP, dGTP, dCTP, dTTP and 2.5 units of heat stable DNA polymerase (Amplitaq, Roche Molecular Systems, Inc., Branchburg, NJ) in a final volume of 100 microliters.

Upon completion of thermal cycling reactions, each sample of amplified DNA was purified using the Qiaquick Spin PCR purification kit (Qiagen, Gaithersburg, MD). All amplified DNA samples were subjected to digestion with the restriction endonucleases, e.g., NcoI and EcoRI (New England BioLabs, Beverly, MA) (Ausubel et al, Current Protocols in Molecular Biology, John Wiley & Sons, Inc. (1994)). DNA samples were then subjected to electrophoresis on 1.0% NuSeive (FMC BioProducts, Rockland, ME) agarose gels. DNA was visualized by exposure to ethidium bromide and long wave UV irradiation. DNA contained in slices isolated from the agarose gel was purified using the Bio 101 GeneClean Kit protocol (Bio 101, Vista, CA).

The pET-28b vector was prepared for cloning by digestion with restriction endonucleases, e.g., NcoI and EcoRI (New England BioLabs, Beverly, MA) (Ausubel et al, Current Protocols in Molecular Biology, John Wiley & Sons, Inc. (1994)). The pET-28a vector, which encodes the histidine affinity tag that can be fused to the 5' end of an inserted gene, was prepared by digestion with appropriate restriction endonucleases.

Following digestion, DNA inserts were cloned (Ausubel et al, Current Protocols in Molecular Biology, John Wiley & Sons, Inc. (1994)) into the previously digested pET-28b expression vector. Products of the ligation reaction were then used to transform the BL21 strain of *E. coli* (Ausubel et al, Current Protocols in Molecular Biology, John Wiley & Sons, Inc. (1994)) as described below.

Competent bacteria, *E. coli* strain BL21 or *E. coli* strain BL21 (DE3), were transformed with recombinant pET expression plasmids carrying the cloned HBM sequence according to standard methods (Ausubel et al, Current Protocols in Molecular Biology, John Wiley & Sons, Inc. (1994)). Briefly, 1 µl of ligation reaction was mixed with 50 µl of electrocompetent cells and subjected to a high voltage pulse, after which samples were incubated in 0.45 ml SOC medium (0.5% yeast extract, 2.0% tryptone, 10 mM NaCl, 2.5 mM KCI, 10 mM MgCl₂, 10 mM MgS0₄ and 20 mM glucose) at 37°C with shaking for 1 hour. Samples were then spread on LB agar plates containing 25 µg/ml kanamycin sulfate for growth overnight. Transformed colonies of BL21 were then picked and analyzed to evaluate cloned inserts, as described below.

Individual BL21 clones transformed with recombinant pET-28b HBM nucleotide sequences were analyzed by PCR amplification of the cloned inserts using the same forward and reverse primers specific for the HBM sequences that were used in the original PCR amplification cloning reactions. Successful amplification verifies the integration of the HBM sequence in the expression vector (Ausubel et al, Current Protocols in Molecular Biology, John Wiley & Sons, Inc. (1994)).

Individual clones of recombinant pET-28b vectors carrying properly cloned HBM nucleotide sequences were picked and incubated in 5 ml of LB broth plus 25 µg/ml kanamycin sulfate overnight. The following day plasmid DNA was isolated and purified using the Qiagen plasmid purification protocol (Qiagen Inc., Chatsworth, CA).

The pET vector can be propagated in any *E. coli* K-12 strain, e.g., HMS 174, HB101, JM109, DH5 and the like, for purposes of cloning or plasmid preparation. Hosts for expression include *E. coli* strains containing a chromosomal copy of the gene for T7 RNA polymerase. These hosts were lysogens of bacteriophage DE3, a lambda derivative that carries the ladI gene, the lacUV5 promoter and the gene for T7 RNA polymerase. T7 RNA polymerase was induced by addition of isopropyl-β-D-thiogalactoside (IPTG), and the T7 RNA polymerase transcribes any target plasmid containing a functional T7 promoter, such as pET-28b, carrying its gene of interest. Strains include, for example, BL21(DE3) (Studier et al, Meth. Enzymol., 185:60-89 (1990)).

To express the recombinant HBM sequence, 50 ng of plasmid DNA are isolated as described above to transform competent BL21(DE3) bacteria as described above (provided by Novagen as part of the pET expression kit). The lacZ gene (β-galactosidase) is expressed in the pET-System as described for the HBM recombinant constructions. Transformed cells were cultured in SOC medium for 1 hour, and the culture was then plated on LB plates containing 25 µg/ml kanamycin sulfate. The following day, the bacterial colonies were pooled and grown in LB medium containing kanamycin sulfate (25 µg/ml) to an optical density at 600 nM of 0.5 to 1.0 O.D. units, at which point 1 mM IPTG was added to the culture for 3 hours to induce gene expression of the HBM recombinant DNA constructions.

After induction of gene expression with IPTG, bacteria were collected by centrifugation in a Sorvall RC-3B centrifuge at 3500 x g for 15 minutes at 4°C. Pellets were resuspended in 50 ml of cold mM Tris-HCI, pH 8.0, 0.1 M NaCI and 0.1 mM EDTA (STE buffer). Cells were then centrifuged at 2000 x g for 20 minutes at 4°C. Wet pellets were weighed and frozen at -80°C until ready for protein purification.

A variety of methodologies known in the art can be used to purify the isolated proteins (Coligan et al, Current Protocols in Protein Science, John Wiley & Sons (1995)). For example, the frozen cells can be thawed, resuspended in buffer and ruptured by several passages through a small volume microfluidizer (Model M-110S, Microfluidics International Corp., Newton, MA). The resultant homogenate is centrifuged to yield a clear supernatant (crude extract) and, following filtration, the crude extract is fractioned over columns. Fractions are monitored by absorbance at OD₂₈₀ nm and peak fractions may be analyzed by SDS-PAGE.

The concentrations of purified protein preparations are quantified spectrophotometrically using absorbance coefficients calculated from amino acid content (Perkins, Eur. J. Biochem., 157:169-180 (1986)). Protein concentrations are also measured by the method of Bradford, Anal. Biochem., 72:248-254 (1976) and Lowry et al, J. Biol. Chem., 193:265-275 (1951) using bovine serum albumin as a standard.

SDS-polyacrylamide gels of various concentrations were purchased from BioRad (Hercules, CA), and stained with Coomassie blue. Molecular weight markers may include rabbit skeletal muscle myosin (200 kDa), *E. coli* β-galactosidase (116 kDa), rabbit muscle phosphorylase B (97.4 kDa), bovine serum albumin (66.2 kDa), ovalbumin (45 kDa), bovine carbonic anyhdrase (31 kDa), soybean trypsin inhibitor (21.5 kDa), egg white lysozyme (14.4 kDa) and bovine aprotinin (6.5 kDa).

Once a sufficient quantity of the desired protein has been obtained, it may be used for various purposes. A typical use is the production of antibodies specific for binding. These antibodies may be either polyclonal or monoclonal, and may be produced by *in vitro* or *in vivo* techniques well known in the art. Monoclonal antibodies to epitopes of any of the peptides identified and isolated as described can be prepared from murine hybridomas (Kohler, Nature, 256:495 (1975)). In summary, a mouse is inoculated with a few micrograms of HBM protein over a period of two weeks. The mouse is then sacrificed. The cells that produce antibodies are then removed from the mouse's spleen. The spleen cells are then fused with polyethylene glycol with mouse myeloma cells. The successfully fused cells are diluted in a microtiter plate and growth of the culture is continued. The amount of antibody per well is measured by immunoassay methods such as ELISA (Engvall, Meth. Enzymol., 70:419 (1980)). Clones producing antibody can be expanded and further propagated to produce HBM antibodies. Other suitable techniques involve *in vitro* exposure of lymphocytes to the antigenic polypeptides, or alternatively, to selection of libraries of antibodies in phage or similar vectors. See Huse et al, Science, 246:1275-1281 (1989). For additional information on antibody production see Davis et al, Basic Methods in Molecular Biology, Elsevier, NY, Section 21-2 (1989).

### XIX. Methods of Use: Gene Therapy

In recent years, significant technological advances have been made in the area of gene therapy for both genetic and acquired diseases. (Kay et al, Proc. Natl. Acad. Sci. USA, 94:12744-12746 (1997)) Gene therapy can be defined as the deliberate transfer of DNA for therapeutic purposes. Improvement in gene transfer methods has allowed for development of gene therapy protocols for the treatment of diverse types of diseases. Gene therapy has also taken advantage of recent advances in the identification of new therapeutic genes, improvement in both viral and nonviral gene delivery systems, better understanding of gene regulation, and improvement in cell isolation and transplantation.

The preceding experiments identify the HBM gene as a dominant mutation conferring elevated bone mass. The fact that this mutation is dominant indicates that expression of the HBM protein causes elevated bone mass. Older individuals carrying the HBM gene, and, therefore expressing the HBM protein; do not suffer from osteoporosis. These individuals are equivalent to individuals being treated with the HBM protein. These observations are a strong experimental indication that therapeutic treatment with the HBM protein prevents osteoporosis. The bone mass elevating activity of the HBM. gene is termed "HBM function."

Therefore, according to the present invention, a method is also provided of supplying HBM function to mesenchymal stem cells (Onyia et al, J. Bone Miner. Res., 13:20-30 (1998); Ko et al, Cancer Res., 56:4614-4619 (1996)). Supplying such a function provides protection against osteoporosis. The HBM gene or a part of the gene may be introduced into the cell in a vector such that the gene remains extrachromosomal. In such a situation, the gene will be expressed by the cell from the extrachromosomal location.

Vectors for introduction of genes both for recombination and for extrachromosomal maintenance are known in the art, and any suitable vector may be used. Methods for introducing DNA into cells such as electroporation, calcium phosphate co-precipitation, and viral transduction are known in the art, and the choice of method is within the competence of one skilled in the art (Robbins, Ed., Gene Therapy Protocols, Human Press, NJ (1997)). Cells transformed with the HBM. gene can be used as model systems to study osteoporosis and drug treatments that promote bone growth.

As generally discussed above, the HBM gene or fragment, where applicable, may be used in the manufacture of medicaments for use in gene therapy methods in order to increase the amount of the expression products of such genes in mesenchymal stem cells. It may be useful also to increase the level of expression of a given HBM protein, or a fragment thereof, even in those cells in which the wild type gene is expressed normally. Gene therapy would be carried out according to generally accepted methods as described by, for example, Friedman, Therapy for Genetic Diseases, Friedman, Ed., Oxford University Press, pages 105-121 (1991).

A virus or plasmid vector containing a copy of the HBM gene linked to expression control elements and capable of replicating inside mesenchymal stem cells, is prepared. Suitable vectors are known and described, for example, in U.S. Patent No. 5,252,479 and WO 93/07282, the disclosures of which are incorporated by reference herein in their entirety. The vector is then injected into the patient, either locally into the bone marrow or systemically (in order to reach any mesenchymal stem cells located at other sites, i.e., in the blood). If the transfected gene is not permanently incorporated into the genome of each of the targeted cells, the treatment may have to be repeated periodically.

Gene transfer systems known in the art may be useful in the practice of the medicaments for gene therapy of the present invention. These include viral and non-viral transfer methods. A number of viruses have been used as gene transfer vectors, including polyoma, i.e., SV40 (Madzak et al, J. Gen. Virol., 73:1533-1536 (1992)), adenovirus (Berkner, Curr. Top. Microbiol. Immunol., 158:39-61 (1992); Berkner et al, Bio Techniques, 6:616-629 (1988); Gorziglia et al, J. Virol., 66:4407-4412 (1992); Quantin et al, Proc. Natl. Acad. Sci. USA, 89:2581-2584 (1992); Rosenfeld et al, Cell, 68:143-155 (1992); Wilkinson et al, Nucl. Acids Res., 20:2233-2239 (1992); Stratford-Peiricaudet et al, Hum. Gene Ther., 1:241-256 (1990)), vaccinia virus (Mackett et al, Biotechnology, 24:495-499 (1992)), adeno-associated virus (Muzyczka, Curr. Top. Microbiol. Immunol., 158:91-123 (1992); Ohi et al, Gene, 89:279-282 (1990)), herpes viruses including HSV and EBV (Margolskee, Curr. Top. Microbiol. Immunol., 158:67-90 (1992); Johnson et al, J. Virol., 66:2952-2965 (1992); Fink et al, Hum. Gene Ther., 3:11-19 (1992); Breakfield et al, Mol. Neurobiol., 1:337-371 (1987;) Fresse et al, Biochem. Pharmacol., 40:2189-2199 (1990)), and retroviruses of avian (Brandyopadhyay et al, Mol. Cell Biol., 4:749-754 (1984); Petropouplos et al, J. Virol., 66:3391-3397 (1992)), murine (Miller, Curr. Top. Microbiol. Immunol., 158:1-24 (1992); Miller et al, Mol. Cell Biol., 5:431-437 (1985); Sorge et al, Mol. Cell Biol., 4:1730-1737 (1984); Mann et al, J. Virol., 54:401-407 (1985)), and human origin (Page et al, J. Virol., 64:5370-5276 (1990); Buchschalcher et al, J. Virol., 66:2731-2739 (1992)). Most human gene therapy protocols have been based on disabled murine retroviruses.

Non-viral gene transfer methods known in the art include chemical techniques such as calcium phosphate coprecipitation (Graham et al, Virology, 52:456-467 (1973); Pellicer et al, Science, 209:1414-1422 (1980)), mechanical techniques, for example microinjection (Anderson et al, Proc. Natl. Acad. Sci. USA, 77:5399-5403 (1980); Gordon et al, Proc. Natl. Acad. Sci. USA, 77:7380-7384 (1980); Brinster et al, Cell, 27:223-231 (1981); Constantini et al, Nature, 294:92-94 (1981)), membrane fusion-mediated transfer via liposomes (Felgner et al, Proc. Natl. Acad. Sci. USA, 84:7413-7417 (1987); Wang et al, Biochemistry, 28:9508-9514 (1989); Kaneda et al, J. Biol. Chem., 264:12126-12129 (1989); Stewart et al, Hum. Gene Ther., 3:267-275 (1992); Nabel et al, Science, 249:1285-1288 (1990); Lim et al, Circulation, 83:2007-2011 (1992)), and direct DNA uptake and receptor-mediated DNA transfer (Wolff et al, Science, 247:1465-1468 (1990); Wu et al, BioTechniques, 11:474-485 (1991); Zenke et al, Proc. Natl. Acad. Sci. USA, 87:3655-3659 (1990); Wu et al, J. Biol. Chem., 264:16985-16987 (1989); Wolff et al, BioTechniques, 11:474-485 (1991); Wagner et al, 1990; Wagner et al, Proc. Natl. Acad. Sci. USA, 88:4255-4259 (1991); Cotten et al, Proc. Natl. Acad. Sci. USA, 87:4033-4037 (1990); Curiel et al, Proc. Natl. Acad. Sci. USA, 88:8850-8854 (1991); Curiel et al, Hum. Gene Ther., 3:147-154 (1991)). Viral-mediated gene transfer can be combined with direct *in vivo* vectors to the mesenchymal stem cells and not into the surrounding cells (Romano et al, In Vivo, 12(1):59-67 (1998); Gonez et al, Hum. Mol. Genetics, 7(12):1913-9 (1998)). Alternatively, the retroviral vector producer cell line can be injected into the bone marrow (Culver et al, Science, 256:1550-1552 (1992)). Injection of producer cells would then provide a continuous source of vector particles. This technique has been approved for use in humans with inoperable brain tumors.

In an approach which combines biological and physical gene transfer methods, plasmid DNA of any size is combined with a polylysine-conjugated antibody specific to the adenovirus hexon protein, and the resulting complex is bound to an adenovirus vector. The trimolecular complex is then used to infect cells. The adenovirus vector permits efficient binding, internalization, and degradation of the endosome before the coupled DNA is damaged.

Liposome/DNA complexes have been shown to be capable of mediating direct *in vivo* gene transfer. While in standard liposome preparations the gene transfer process is non-specific, localized *in vivo* uptake and expression have been reported in tumor deposits, for example, following direct *in situ* administration (Nabel, Hum. Gene Ther., 3:399-410 (1992)).

### XX. Methods of Use: Transformed Host cells, Development of Pharmaceuticals and Research Tools

Cells and non-human animals that carry the HBM gene can be used as model systems to study and test for substances that have potential as therapeutic agents (Onyia et al, J. Bone Miner. Res., 13:20-30 (1998); Broder et al, Bone, 21:225-235 (1997)). The cells are typically cultured mesenchymal stem cells. These may be isolated from individuals with somatic or germline HBM genes. Alternatively, the cell line can be engineered to carry the HBM gene, as described above. After a test substance is applied to the cells, the transformed phenotype of the cell is determined. Any trait of transformed cells can be assessed, including formation of bone matrix in culture (Broder et al, Bone, 21:225-235 (1997)), mechanical properties (Kizer et al, Proc. Natl. Acad. Sci. USA, 94:1013-1018 (1997)), and response to application of putative therapeutic agents.

Non-human animals for testing therapeutic agents can be selected after treatment of germline cells or zygotes. Such treatments include insertion of the Zmax1 gene, as well as insertion of the HBM gene and disrupted homologous genes. Alternatively, the inserted Zmax1 gene(s) and/or HBM gene(s) of the non-human animals may be disrupted by insertion or deletion mutation of other genetic alterations using conventional techniques, such as those described by, for example, Capechi, Science, 244:1288 (1989); Valancuis et al, Mol. Cell Biol., 11:1402 (1991); Hasty et al, Nature, 350:243 (1991); Shinkai et al, Cell, 68:855 (1992); Mombaerts et al, Cell, 68:869 (1992); Philpott et al, Science, 256:1448 (1992); Snouwaert et al, Science, 257:1083 (1992); Donehower et al, Nature, 356:215 (1992). After test substances have been administered to the non-human animals, the growth of bone must be assessed. If the test substance enhances the growth of bone, then the test substance is a candidate therapeutic agent. These non-human animal models provide an extremely important vehicle for potential therapeutic products.

Individuals carrying the HBM gene have elevated bone mass. The HBM gene causes this phenotype by altering the activities, levels, expression patterns, and modification states of other molecules involved in bone development. Using a variety of established techniques, it is possible to identify molecules, preferably proteins or mRNAs, whose activities, levels, expression patterns, and modification states are different between systems containing the Zmax 1 gene and systems containing the HBM gene. Such systems can be, for example, cell-free extracts, cells, tissues or living organisms, such as mice or humans. For a mutant form of Zmax1, a complete deletion of Zmax1, mutations lacking the extracellular or intracellular portion of the protein, or any other mutation in the Zmax1 gene may be used. It is also possible to use expression of antisense Zmax1 RNA or oligonucleotides to inhibit production of the Zmax1 protein. For a mutant form of HBM, a complete deletion of HBM, mutations lacking the extracellular or intracellular portion of the HBM protein, or any other mutation in the HBM gene may be used. It is also possible to use expression of antisense HBM RNA or ' oligonucleotides to inhibit production of the HBM protein.

Molecules identified by comparison of Zmax1 systems and HBM systems can be used as surrogate markers in pharmaceutical development or in diagnosis of human or animal bone disease. Alternatively, such molecules may be used in treatment of bone disease. *See*, Schena et al, Science, 270:467-470 (1995).

For example, a transgenic mouse carrying the HBM gene in the mouse homologue is constructed. A mouse of the genotype HBM/+ is viable, healthy and has elevated bone mass. To identify surrogate markers for elevated bone mass, HBM/+ (i.e., heterozygous) and isogenic +/+ (i.e., wild-type) mice are sacrificed. Bone tissue mRNA is extracted from each animal, and a "gene chip" corresponding to mRNAs expressed in the +/+ individual is constructed. mRNA from different tissues is isolated from animals of each genotype, reverse-transcribed, fluorescently labeled, and then hybridized to gene fragments affixed to a solid support. The ratio of fluorescent intensity between the two populations is indicative of the relative abundance of the specific mRNAs in the +/+ and HBM/+ animals. Genes encoding mRNAs over- and under-expressed relative to the wild-type control are candidates for genes coordinately regulated by the HBM gene.

One standard procedure for identification of new proteins that are part of the same signaling cascade as an already-discovered protein is as follows. Cells are treated with radioactive phosphorous, and the already-discovered protein is manipulated to be more ore less active. The phosphorylation state of other proteins in the cell is then monitored by polyacrylamide gel electrophoresis and autoradiography, or similar techniques. Levels of activity of the known protein may be manipulated by many methods, including, for example, comparing wild-type mutant proteins using specific inhibitors such as drugs or antibodies, simply adding or not adding a known extracellular protein, or using antisense inhibition of the expression of the known protein (Tamura et al, Science, 280(5369):1614-7 (1998); Meng, EMBO J., 17(15):4391-403 (1998); Cooper et al, Cell, 1:263-73 (1982)).

In another example, proteins with different levels of phosphorylation are identified in TE85 osteosarcoma cells expressing either a sense or antisense cDNA for Zmax1. TE85 cells normally express high levels of Zmax1 (Dong et al, Biochem. & Biophys. Res. Comm., 251:784-790 (1998)). Cells containing the sense construct express even higher levels of Zmax1, while cells expressing the antisense construct express lower levels. Cells are grown in the presence of ³²P, harvested, lysed, and the lysates run on SDS polyacrylamide gels to separate proteins, and the gels subjected to autoradiography (Ausubel et al, Current Protocols in Molecular Biology, John Wiley & Sons (1997)). Bands that differ in intensity between the sense and antisense cell lines represent phosphoproteins whose phosphorylation state or absolute level varies in response to levels of Zmax1. As an alternative to the ³²P-labeling, unlabeled proteins may be separated by SDS-PAGE and subjected to immunoblotting, using the commercially available anti-phosphotyrosine antibody as a probe (Thomas et al, Nature, 376(6537):267-71 (1995)). As an alternative to the expression of antisense RNA, transfection with chemically modified antisense oligonucleotides can be used (Woolf et al, Nucleic Acids Res., 18(7):1763-9 (1990)).

Many bone disorders, such as osteoporosis, have a slow onset and a slow response to treatment. It is therefore useful to develop surrogate markers for bone development and mineralization. Such markers can be useful in developing treatments for bone disorders, and for diagnosing patients who may be at risk for later development of bone disorders. Examples of preferred markers are N- and C-terminal telopeptide markers described, for example, in U.S. Patent Nos. 5,455,179, 5,641,837 and 5,652,112, the disclosures of which are incorporated by reference herein in their entirety. In the area of HIV disease, CD4 counts and viral load are useful surrogate markers for disease progression (Vlahov et al, JAMA, 279(1):35-40 (1998)). There is a need for analogous surrogate markers in the area of bone disease.

A surrogate marker can be any characteristic that is easily tested and relatively insensitive to non-specific influences. For example, a surrogate marker can be a molecule such as a protein or mRNA in a tissue or in blood serum. Alternatively, a surrogate marker may be a diagnostic sign such as sensitivity to pain, a reflex response or the like.

In yet another example, surrogate markers for elevated bone mass are identified using a pedigree of humans carrying the HBM gene. Blood samples are withdrawn from three individuals that carry the HBM gene, and from three closely related individuals that do not. Proteins in the serum from these individuals are electrophoresed on a two dimensional gel system, in which one dimension separates proteins by size, and another dimension separates proteins by isoelectric point (Epstein et al, Electrophoresis, 17(11):1655-70 (1996)). Spots corresponding to proteins are identified. A few spots are expected to be present in different amounts or in slightly different positions for the HBM individuals compared to their normal relatives. These spots correspond to proteins that are candidate surrogate markers. The identities of the proteins are determined by microsequencing, and antibodies to the proteins can be produced by standard methods for use in diagnostic testing procedures. Diagnostic assays for HBM proteins or other candidate surrogate markers include using antibodies described in this invention and a reporter molecule to detect HBM in human body fluids, membranes, bones, cells, tissues or extracts thereof. The antibodies can be labeled by joining them covalently or noncovalently with a substance that provides a detectable signal. In many scientific and patent literature, a variety of reporter molecules or labels are described including radionuclides, enzymes, fluorescent, chemi-luminescent or chromogenic agents (U.S. Patent Nos. 3,817,837; 3,850,752; 3,939,350; 3,996,345; 4,277,437; 4,275,149; and 4,366,241).

Using these antibodies, the levels of candidate surrogate markers are measured in normal individuals and in patients suffering from a bone disorder, such as osteoporosis, osteoporosis pseudoglioma, Engelmann's disease, Ribbing's disease, hyperphosphatasemia, Van Buchem's disease, melorheostosis, osteopetrosis, pychodysostosis, sclerosteosis, osteopoikilosis, acromegaly, Paget's disease, fibrous dysplasia, tubular stenosis, osteogenesis imperfecta, hypoparathyroidism, pseudohypoparathyroidism, pseudopseudohypoparathyroidism, primary and secondary hyperparathyroidism and associated syndromes, hypercalciuria, medullary carcinoma of the thyroid gland, osteomalacia and other diseases. Techniques for measuring levels of protein in serum in a clinical setting using antibodies are well established. A protein that is consistently present in higher or lower levels in individuals carrying a particular disease or type of disease is a useful surrogate marker.

A surrogate marker can be used in diagnosis of a bone disorder. For example, consider a child that present to a physician with a high frequency of bone fracture. The underlying cause may be child abuse, inappropriate behavior by the child, or a bone disorder. To rapidly test for a bone disorder, the levels of the surrogate marker protein are measured using the antibody described above.

Levels of modification states of surrogate markers can be measured as indicators of the likely effectiveness of a drug that is being developed. It is especially convenient to use surrogate markers in creating treatments for bone disorders, because alterations in bone development or mineralization may require a long time to be observed. For example, a set of bone mRNAs, termed the "HBM-inducible mRNA set" is found to be overexpressed in HBM/+ mice as compared to +/+ mice, as described above. Expression of this set can be used as a surrogate marker. Specifically, if treatment of +/+ mice with a compound results in overexpression of the HBM-inducible mRNA set, then that compound is considered a promising candidate for further development.

This invention is particularly useful for screening compounds by using the Zmax1 or HBM protein or binding fragment thereof in any of a variety of drug screening techniques.

The Zmax1 or HBM protein or fragment employed in such a test may either be free in solution, affixed to a solid support, or borne on a cell surface. One method of drug screening utilizes eukaryotic or prokaryotic host cells which are stably transformed with recombinant nucleic acids expressing the protein or fragment, preferably in competitive binding assays. Such cells, either in viable or fixed form, can be used for standard binding assays. One may measure, for example, for the formation of complexes between a Zmax1 or HBM protein or fragment and the agent being tested, or examine the degree to which the formation of a complex between a Zmax1 or HBM protein or fragment and a known ligand is interfered with by the agent being tested.

Thus, the present invention provides methods of screening for drugs comprising contacting such an agent with a HBM protein or fragment thereof and assaying (i) for the presence of a complex between the agent and the HBM protein or fragment, or (ii) for the presence of a complex between the HBM protein or fragment and a ligand, by methods well known in the art. In such competitive binding assays the HBM protein or fragment is typically labeled. Free HBM protein or fragment is separated from that present in a protein:protein complex, and the amount of free (i.e., uncomplexed) label is a measure of the binding of the agent being tested to HBM or its interference with HBM: ligand binding, respectively.

Another technique for drug screening provides high throughput screening for compounds having suitable binding affinity to the Zmax1 or HBM proteins and is described in detail in WO 84/03564. Briefly stated, large numbers of different small-peptide test compounds are synthesized on a solid substrate, such as plastic pins or some other surface. The peptide test compounds are reacted with Zmax1 or HBM proteins and washed. Bound Zmax1 or HBM protein is then detected by methods well known in the art. Purified Zmax1 or HBM can be coated directly onto plates for use in the aforementioned drug screening techniques. However, non-neutralizing antibodies to the protein can be used to capture antibodies to immobilize the Zmax1 or HBM protein on the solid phase.

This invention also contemplates the use of competitive drug screening assays in which neutralizing antibodies capable of specifically binding the HBM protein compete with a test compound for binding to the HBM protein or fragments thereof. In this manner, the antibodies can be used to detect the presence of any peptide that shares one or more antigenic determinants of the HBM protein.

A further technique for drug screening involves the use of host eukaryotic cell lines or cells (such as described above) that have a nonfunctional Zmax1 or HBM gene. These host cell lines or cells are defective at the Zmax1 or HBM protein level. The host cell lines or cells are grown in the presence of drug compound. The rate of growth of the host cells is measured to determine if the compound is capable of regulating the growth of Zmax1 or HBM defective cells.

The goal of rational drug design is to produce structural analogs of biologically active proteins of interest or of small molecules with which they interact (e.g., agonists, antagonists, inhibitors) in order to fashion drugs which are, for example, more active or stable forms of the protein, or which, e.g., enhance or interfere with the function of a protein in vivo. See, e.g., Hodgson, BiolTechnology, 9:19-21 (1991). In one approach, one first determines the three-dimensional structure of a protein of interest (e.g., Zmax1 or HBM protein) or, for example, of the Zmax1- or HBM-receptor or ligand complex, by x-ray crystallography, by computer modeling or most typically, by a combination of approaches. Less often, useful information regarding the structure of a protein may be gained by modeling based on the structure of homologous proteins. An example of rational drug design is the development of HIV protease inhibitors (Erickson et al, Science, 249:527-533 (1990)). In addition, peptides (e.g., Zmax1 or HBM protein) are analyzed by an alanine scan (Wells, Methods in Enzymol., 202: 390-411 (1991)). In this technique, an amino acid residue is replaced by Ala, and its effect on the peptide's activity is determined. Each of the amino acid residues of the peptide is analyzed in this manner to determine the important regions of the peptide.

It is also possible to isolate a target-specific antibody, selected by a functional assay, and then to solve its crystal structure. In principle, this approach yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced banks of peptides. Selected peptides would then act as the pharmacore.

Thus, one may design drugs which have, e.g., improved Zmax1 or HBM protein activity or stability or which act as inhibitors, agonists, antagonists, etc. of Zmax1 or HBM protein activity. By virtue of the availability of cloned Zmax1 or HBM sequences, sufficient amounts of the Zmax1 or HBM protein may be made available to perform such analytical studies as x-ray crystallography. In addition, the knowledge of the Zmax1 or HBM protein sequence provided herein will guide those employing computer modeling techniques in place of, or in addition to x-ray crystallography.

### XXI. Methods of Use: Avian and Mammalian Animal Husbandry

The HBM DNA and HBM protein can be used for vertebrate and preferably human therapeutic agents and for avian and mammalian veterinary agents, including for livestock breeding. Birds, including, for example, chickens, roosters, hens, turkeys, ostriches, ducks, pheasants and quails, can benefit from the identification of the gene and pathway for high bone mass. In many examples cited in literature (for example, McCoy et al, Res. Vet. Sci., 60(2): 185-186 (1996)), weakened bones due to husbandry conditions cause cage layer fatigue, osteoporosis and high mortality rates. Additional therapeutic agents to treat osteoporosis or other bone disorders in birds can have considerable beneficial effects on avian welfare and the economic conditions of the livestock industry, including, for example, meat and egg production.

### XXII. Methods of use: Diagnostic assays using Zmax1-specific oligonucleotides for detection of genetic alterations affecting bone development.

In cases where an alteration or disease of bone development is suspected to involve an alteration of the Zmax1 gene or the HBM gene, specific oligonucleotides may be constructed and used to assess the level of Zmax1 mRNA or HBM mRNA, respectively, in bone tissue or in another tissue that affects bone development.

For example, to test whether a person has the HBM gene, which affects bone density, polymerase chain reaction can be used. Two oligonucleotides are synthesized by standard methods or are obtained from a commercial supplier of custom-made oligonucleotides. The length and base composition are determined by standard criteria using the Oligo 4.0 primer Picking program (Wojchich Rychlik, 1992). One of the oligonucleotides is designed so that it will hybridize only to HBM DNA under the PCR conditions used. The other oligonucleotide is designed to hybridize a segment of Zmax1 genomic DNA such that amplification of DNA using these oligonucleotide primers produces a conveniently identified DNA fragment. For example, the pair of primers CCAAGTTCTGAGAAGTCC (SEQ ID NO:32) and AATACCTGAAACCATACCTG (SEQ ID NO:33) will amplify a 530 base pair DNA fragment from a DNA sample when the following conditions are used: step 1 at 95°C for 120 seconds; step 2 at 95°C for 30 seconds; step 3 at 58°C for 30 seconds; step 4 at 72°C for 120 seconds; where steps 2-4 are repeated 35 times. Tissue samples may be obtained from hair follicles, whole blood, or the buccal cavity.

The fragment generated by the above procedure is sequenced by standard techniques. Individuals heterozygous for the HBM gene will show an equal amount of G and T at the second position in the codon for glycine 171. Normal or homozygous wild-type individuals will show only G at this position. Other amplification techniques besides PCR may be used as alternatives, such as ligation-mediated PCR or techniques involving Q-beta replicase (Cahill et al, Clin. Chem., 37(9):1482-5 (1991)). For example, the oligonucleotides AGCTGCTCGT AGCTG TCTCTCCCTGGATCACGGGTACATGTACTGGACAGACTGGGT (SEQ ID NO:34) and TGAGACGCCCCGGATTGAGCGGGCAGGGATAGCTTA TTCCCTGTGCCGCATTACGGC (SEQ ID NO:35) can be hybridized to a denatured human DNA sample, treated with a DNA ligase, and then subjected to PCR amplification using the primer oligonucleotides AGCTGCTCGTAGCTGTCT CTCCCTGGA (SEQ ID NO:36) and GCCGTAATGCGGCACAGGGAATAAGCT (SEQ ID NO:37). In the first two oligonucleotides, the outer 27 bases are random sequence corresponding to primer binding sites, and the inner 30 bases correspond to sequences in the Zmax1 gene. The T at the end of the first oligonucleotide corresponds to the HBM gene. The first two oligonucleotides are ligated only when hybridized to human DNA carrying the HBM gene, which results in the formation of an amplifiable 114 bp DNA fragment.

Products of amplification can be detected by agarose gel electrophoresis, quantitative hybridization, or equivalent techniques for nucleic acid detection known to one skilled in the art of molecular biology (Sambrook et al, Molecular Cloning: A Laboratory Manual, Cold Spring Harbor Laboratory, Cold Spring, NY (1989)).

Other alterations in the Zmax1 gene or the HBM gene may be diagnosed by the same type of amplification-detection procedures, by using oligonucleotides designed to identify those alterations. These procedures can be used in animals as well as humans to identify alterations in Zmax1 or HBM that affect bone development.

Expression of Zmax1 or HBM in bone tissue may be accomplished by fusing the cDNA of Zmax1 or HBM, respectively, to a bone-specific promoter in the context of a vector for genetically engineering vertebrate cells. DNA constructs are introduced into cells by packaging the DNA into virus capsids, by the use of cationic liposomes, electroporation, or by calcium phosphate transfection. Transfected cells, preferably osteoblasts, may be studied in culture or may be introduced into bone tissue in animals by direct injection into bone or by intravenous injection of osteoblasts, followed by incorporation into bone tissue (Ko et al, Cancer Research, 56(20):4614-9 (1996)). For example, the osteocalcin promoter, which is specifically active in osteoblasts, may be used to direct transcription of the Zmax1 gene or the HBM gene. Any of several vectors and transfection methods may be used, such as retroviral vectors, adenovirus vectors, or vectors that are maintained after transfection using cationic liposomes, or other methods and vectors described herein.

Alteration of the level of functional Zmax1 protein or HBM protein affects the level of bone mineralization. By manipulating levels of functional Zmax1 protein or HBM protein, it is possible to affect bone development and to increase or decrease levels of bone mineralization. For example, it may be useful to increase bone mineralization in patients with osteoporosis. Alternatively, it may be useful to decrease bone mineralization in patients with osteopetrosis or Paget's disease. Alteration of Zmax1 levels or HBM levels can also be used as a research tool. Specifically, it is possible to identify proteins, mRNA and other molecules whose level or modification status is altered in response to changes in functional levels of Zmax1 or HBM. The pathology and pathogenesis of bone disorders is known and described, for example, in Rubin and Farber (Eds.), Pathology, 2nd Ed., S.B. Lippincott Co., Philadelphia, PA (1994).

A variety of techniques can be used to alter the levels of functional Zmax1 or HBM. For example, intravenous or intraosseous injection of the extracellular portion of Zmax1 or mutations thereof, or HBM or mutations thereof, will alter the level of Zmax1 activity or HBM activity, respectively, in the body of the treated human, animal or bird. Truncated versions of the Zmax1 protein or HBM protein can also be injected to alter the levels of functional Zmax1 protein or HBM protein, respectively. Certain forms of Zmax1 or HBM enhance the activity of endogenous protein, while other forms are inhibitory.

In a preferred embodiment, the HBM protein is used to treat osteoporosis. In a further preferred embodiment, the extracellular portion of the HBM protein is used. This HBM protein may be optionally modified by the addition of a moiety that causes the protein to adhere to the surface of cells. The protein is prepared in a pharmaceutically acceptable solution and is administered by injection or another method that achieves acceptable pharmacokinetics and distribution.

In a second embodiment of this method, Zmax1 or HBM levels are increased or decreased by gene therapy techniques. To increase Zmax1 or HBM levels, osteoblasts or another useful cell type are genetically engineered to express high levels of Zmax1 or HBM as described above. Alternatively, to decrease Zmax1 or HBM levels, antisense constructs that specifically reduce the level of translatable Zmax1 or HBM mRNA can be used. In general, a tissue-nonspecific promoter may be used, such as the CMV promoter or another commercially available promoter found in expression vectors (Wu et al, Toxicol. Appl. Pharmacol., 141(1):330-9 (1996)). In a preferred embodiment, a Zmax1 cDNA or its antisense is transcribed by a bone-specific promoter, such as the osteocalcin or another promoter, to achieve specific expression in bone tissue. In this way, if a Zmax1-expressing DNA construct or HBM-expressing construct is introduced into non-bone tissue, it will not be expressed.

In a third embodiment, antibodies against Zmax1 or HBM are used to inhibit its function. Such antibodies are identified herein.

In a fourth embodiment, drugs that inhibit Zmax1 function or HBM function are used. Such drugs are described herein and optimized according to techniques of medicinal chemistry well known to one skilled in the art of pharmaceutical development.

Zmax1 and HBM interact with several proteins, such as ApoE. Molecules that inhibit the interaction between Zmax1 or HBM and ApoE or another binding partner are expected to alter bone development and mineralization. Such inhibitors may be useful as drugs in the treatment of osteoporosis, osteopetrosis, or other diseases of bone mineralization. Such inhibitors may be low molecular weight compounds, proteins or other types of molecules. *See,* Kim et al, J. Biochem. (Tokyo), 124(6):1072-1076 (1998).

Inhibitors of the interaction between Zmax1 or HBM and interacting proteins may be isolated by standard drug-screening techniques. For example, Zmax1 protein, (or a fragment thereof) or HBM protein (or a fragment thereof) can be immobilized on a solid support such as the base of microtiter well. A second protein or protein fragment, such as ApoE is derivatized to aid in detection, for example with fluorescein. Iodine, or biotin, then added to the Zmax1 or HBM in the presence of candidate compounds that may specifically inhibit this protein-protein domain of Zmax1 or HBM, respectively, and thus avoid problems associated with its transmembrane segment. Drug screens of this type are well known to one skilled in the art of pharmaceutical development.

Because Zmax1 and HBM are involved in bone development, proteins that bind to Zmax1 and HBM are also expected to be involved in bone development. Such binding proteins can be identified by standard methods, such as coimmunoprecipitation, co-fractionation, or the two-hybrid screen (Ausubel et al, Current Protocols in Molecular Biology, John Wiley & Sons (1997)). For example, to identify Zmax1-interacting proteins or HBM-interacting proteins using the two-hybrid system, the extracellular domain of Zmax1 or HBM is fused to LexA and expressed for the yeast vector pEG202 (the "bait") and expressed in the yeast strain EGY48. The yeast strain is transformed with a "prey" library in the appropriate vector, which encodes a galactose-inducible transcription-activation sequence fused to candidate interacting proteins. The techniques for initially selecting and subsequently verifying interacting proteins by this method are well known to one skilled in the art of molecular biology (Ausubel et al, Current Protocols in Molecular Biology, John Wiley & Sons (1997)).

In a preferred embodiment, proteins that interact with HBM, but not Zmax1, are identified using a variation of the above procedure (Xu et al, Proc. Natl. Acad. Sci. USA, 94(23):12473-8 (Nov. 1997)). This variation of the two-hybrid system uses two baits, and Zmax1 and HBM are each fused to LexA and TetR, respectively. Alternatively, proteins that interact with the HBM but not Zmax1 are also isolated. These procedures are well known to one skilled in the art of molecular biology, and are a simple variation of standard two-hybrid procedures.

As an alternative method of isolating Zmax1 or HBM interacting proteins, a biochemical approach is used. The Zmax1 protein or a fragment thereof, such as the extracellular domain, or the HBM protein or a fragment thereof, such as the extracellular domain, is chemically coupled to Sepharose beads. The Zmax1- or HBM-coupled beads are poured into a column. An extract of proteins, such as serum proteins, proteins in the supernatant of a bone biopsy, or intracellular proteins from gently lysed TE85 osteoblastic cells, is added to the column. Non-specifically bound proteins are eluted, the column is washed several times with a low-salt buffer, and then tightly binding proteins are eluted with a high-salt buffer. These are candidate proteins that bind to Zmax1 or HBM, and can be tested for specific binding by standard tests and control experiments. Sepharose beads used for coupling proteins and the methods for performing the coupling are commercially available (Sigma), and the procedures described here are well known to one skilled in the art of protein biochemistry.

As a variation of the above procedure, proteins that are eluted by high salt from the Zmax1- or HBM-Sepharose column are then added to an HBM-Zmax1-sepharose column. Proteins that flow through without sticking are proteins that bind to Zmax1 but not to HBM. Alternatively, proteins that bind to the HBM protein and not to the Zmax1 protein can be isolated by reversing the order in which the columns are used.

### XXIII. Method of Use: Transformation-Associated Recombination (TAR) Cloning

Essential for the identification of novel allelic variants of Zmax1 is the ability to examine the sequence of both copies of the gene in an individual. To accomplish this, two "hooks," or regions of significant similarity, are identified within the genomic sequence such that they flank the portion of DNA that is to be cloned. Most preferably, the first of these hooks is derived from sequences 5' to the first exon of interest and the second is derived from sequences 3' to the last exon of interest. These two "hooks" are cloned into a bacterial/yeast shuttle vector such as that described by Larionov et al, Proc. Natl. Acad. Sci. USA, 94:7384-7387 (1997). Other similar vector systems may also be used. To recover the entire genomic copy of the Zmax1 gene, the plasmid containing the two "hooks" is linearized with a restriction endonuclease or is produced by another method such as PCR. This linear DNA fragment is introduced into yeast cells along with human genomic DNA. Typically, the yeast *Saccharomyces cerevisiae* is used as a host cell, although Larionov et al (in press) have reported using chicken host cells as well. During and after the process of transformation, the endogenous host cell converts the linear plasmid to a circle by a recombination event whereby the region of the human genomic DNA homologous to the "hooks" is inserted into the plasmid. This plasmid can be recovered and analyzed by methods well known to one skilled in the art. Obviously, the specificity for this reaction requires the host cell machinery to recognize sequences similar to the "hooks" present in the linear fragment. However, 100% sequence identity is not required, as shown by Kouprina et al, Genomic, 53(1):21-28 (October 1998), where the author describes using degenerate repeated sequences common in the human genome to recover fragments of human DNA from a rodent/human hybrid cell line.

In another example, only one "hook" is required, as described by Larionov et al, Proc. Natl. Acad. Sci. USA, 95(8):4469-74 (April 1998). For this type of experiment, termed "radial TAR cloning," the other region of sequence similarity to drive the recombination is derived from a repeated sequence from the genome. In this way, regions of DNA adjacent to the Zmax1 gene coding region can be recovered and examined for alterations that may affect function.

### XXIV. Methods of Use: Genomic Screening

The use of polymorphic genetic markers linked to the HBM gene or to Zmax1 is very useful in predicting susceptibility to osteoporosis or other bone diseases. Koller et al, Amer. J. Bone Min. Res., 13:1903-1908 (1998) have demonstrated that the use of polymorphic genetic markers is useful for linkage analysis. Similarly, the identification of polymorphic genetic markers within the high bone mass gene will allow the identification of specific allelic variants that are in linkage disequilibrium with other genetic lesions that affect bone development. Using the DNA sequence from the BACs, a dinucleotide CAn repeat was identified and two unique PCR primers that will amplify the genomic DNA containing this repeat were designed, as shown below:
B200E21C16_L: GAGAGGCTATATCCCTGGGC (SEQ ID NO:38)
B200E21C16_R: ACAGCACGTGTTTAAAGGGG (SEQ ID NO:39)
and used in the genetic mapping study.

This method has been used successfully by others skilled in the art (e.g., Sheffield et al, Genet., 4:1837-1844 (1995); LeBlanc-Straceski et al, Genomics, 19:341-9 (1994); Chen et al, Genomics, 25:1-8 (1995)). Use of these reagents with populations or individuals will predict their risk for osteoporosis. Similarly, single nucleotide polymorphisms (SNPs), such as those shown in Table 4 above, can be used as well to predict risk for developing bone diseases or resistance to osteoporosis in the case of the HBM gene.

### XXV. Methods of Use: Modulators of Tissue Calcification

The calcification of tissues in the human body is well documented. Towler et al, J. Biol. Chem., 273:30427-34 (1998) demonstrated that several proteins known to regulate calcification of the developing skull in a model system are expressed in calcified aorta. The expression of Msx2, a gene transcribed in osteoprogenitor cells, in calcified vascular tissue indicates that genes which are important in bone development are involved in calcification of other tissues. Treatment with HBM protein, agonists or antagonists is likely to ameliorate calcification (such as the vasculature, dentin and bone of the skull visera) due to its demonstrated effect on bone mineral density. In experimental systems where tissue calcification is demonstrated, the over-expression or repression of Zmax1 activity permits the identification of molecules that are directly regulated by the Zmax1 gene. These genes are potential targets for therapeutics aimed at modulating tissue calcification. For example, an animal, such as the LDLR -/-, mouse is fed a high fat diet and is observed to demonstrate expression of markers of tissue calcification, including Zmax1. These animals are then treated with antibodies to Zmax1 or HBM protein, antisense oligonucleotides directed against Zmax1 or HBM cDNA, or with compounds known to bind the Zmax1 or HBM protein or its binding partner or ligand. RNA or proteins are extracted from the vascular tissue and the relative expression levels of the genes expressed in the tissue are determined by methods well known in the art. Genes that are regulated in the tissue are potential therapeutic targets for pharmaceutical development as modulators of tissue calcification.

The nucleic acids, proteins, peptides, amino acids, small molecules or other pharmaceutically useful compounds of the present invention that are to be given to an individual may be administered in the form of a composition with a pharmaceutically acceptable carrier, excipient or diluent, which are well known in the art. The individual may be a mammal or a bird, preferably a human, a rat, a mouse or bird. Such compositions may be administered to an individual in a pharmaceutically effective amount. The amount administered will vary depending on the condition being treated and the patient being treated. The compositions may be administered alone or in combination with other treatments.

### EXAMPLES

The present invention is described by reference to the following Examples, which are offered by way of illustration and are not intended to limit the invention in any manner. Standard techniques well known in the art or the techniques specifically described below were utilized.

### Example 1

The propositus was referred by her physicians to the Creighton Osteoporosis Center for evaluation of what appeared to be unusually dense bones. She was 18 years old and came to medical attention two years previous because of back pain, which was precipitated by an auto accident in which the car in which she was riding as a passenger was struck from behind. Her only injury was soft tissue injury to her lower back that was manifested by pain and muscle tenderness. There was no evidence of fracture or subluxation on radiographs. The pain lasted for two years, although she was able to attend school full time. By the time she was seen in the Center, the pain was nearly resolved and she was back to her usual activities as a high school student. Physical exam revealed a normal healthy young woman standing 66 inches and weighing 128 pounds. Radiographs of the entire skeleton revealed dense looking bones with thick cortices. All bones of the skeleton were involved. Most importantly, the shapes of all the bones were entirely normal. The spinal BMC was 94.48 grams in L1-4, and the spinal BMD was 1.667 gm/cm² in L1-4. BMD was 5.62 standard deviations (SD) above peak skeletal mass for women. These were measured by DXA using a Hologic 2000∼. Her mother was then scanned and a lumbar spinal BMC of 58.05 grams and BMD of 1.500 gm/cm² were found. Her mother's values place her 4.12 SD above peak mass and 4.98 SD above her peers. Her mother was 51 years old, stood 65 inches and weighed 140 pounds. Her mother was in excellent health with no history of musculoskeletal or other symptoms. Her father's lumbar BMC was 75.33 grams and his BMD was 1.118 gm/cm². These values place him 0.25 SD above peak bone mass for males. He was in good health, stood 72 inches tall, and weighed 187 pounds.

These clinical data suggested that the propositus inherited a trait from her mother, which resulted in very high bone mass, but an otherwise normal skeleton, and attention was focused on the maternal kindred. In U.S. Patent No. 5,691,153, twenty- two of these members had measurement of bone mass by DXA. In one case, the maternal grandfather of the propositus, was deceased, however, medical records, antemortem skeletal radiographs and a gall bladder specimen embedded in paraffin for DNA genotyping were obtained. His radiographs showed obvious extreme density of all of the bones available for examination including the femur and the spine, and he was included among the affected members. In this invention, the pedigree has been expanded to include 37 informative individuals. These additions are a significant improvement over the original kinship (Johnson et al, Am. J. Hum. Genet., 60:1326-1332 (1997)) because, among the fourteen individuals added since the original study, two individuals harbor key crossovers. X-linkage is ruled out by the presence of male-to-male transmission from individual 12 to 14 and 15.

### Example 2

The present invention describes DNA sequences derived from two BAC clones from the HBM gene region, as evident in Table 7 below, which is an assembly of these clones. Clone b200e21-h (ATCC No. 980812; SEQ ID NOS: 10-11) was deposited at the American Type Culture Collection (ATCC), 10801 University Blvd., Manassas, VA 20110-2209 U.S.A., on December 30, 1997. Clone b527d12-h (ATCC No. 980720; SEQ ID NOS: 5-9) was deposited at the American Type Culture Collection (ATCC), 10801 University Blvd., Manassas, VA 20110-2209 U.S.A., on October 2, 1998. These sequences are unique reagents that can be used by one skilled in the art to identify DNA probes for the Zmax1 gene, PCR primers to amplify the gene, nucleotide polymorphisms in the Zmax1 gene, or regulatory elements of the Zmax1 gene.

**TABLE 7**

| **Contig** | **ATCC No.** | **SEQ ID NO.** | **Length (base pairs)** |
|---|---|---|---|
| b527d12-h_contig302G | 980720 | 5 | 3096 |
| b527d12-h_contig306G | 980720 | 6 | 26928 |
| b527d12-h_contig307G | 980720 | 7 | 29430 |
| b527d12-h_contig308G | 980720 | 8 | 33769 |
| b527d12-h_contig309G | 980720 | 9 | 72049 |
| b200e21-h_contig1 | 980812 | 10 | 8705 |
| b200e21-h_contig4 | 980812 | 11 | 66933 |

Although the invention has been set forth in detail, one skilled in the art will recognize that numerous changes and modifications can be made.

This application claims priority to U.S. Application Nos. 09/543,771 and 09/544,398 filed on April 5, 2000, which are a continuation-in-part of Application No. 09/229,319, filed January 13, 1999, which claims benefit of U.S. Provisional Application No. 60/071,449, filed January 13, 1998, and U.S. Provisional Application No. 60/105,511, filed October 23, 1998, .

## Claims

1. An isolated nucleic acid sequence of SEQ ID NO: 2.

2. The isolated nucleic acid sequence of claim 1, wherein the nucleic acid sequence is DNA.

3. An isolated amino acid sequence of SEQ ID NO: 4.

4. A nucleic acid sequence encoding the amino acid sequence of SEQ ID NO: 4.

5. A replicative cloning vector comprising the nucleic acid sequence of claim 1 and a replicon operative in an isolated host cell.

6. An isolated host cell transformed with the replicative cloning vector of claim 5.

7. An expression vector comprising the nucleic acid sequence of claim 1 operably linked to a transcription regulatory region.

8. An isolated host cell transformed with the expression vector of claim 7.

9. An in vitro method for testing a substance as a therapeutic agent for bone modulation in a host comprising administering the nucleic acid of claim 1 to a host cell, and assessing whether bone modulation occurs.

10. A method of identifying a molecule involved in bone modulation comprising identifying a molecule that binds to, or that inhibits binding of a molecule to, an isolated amino acid sequence of SEQ ID NO: 4 or an amino acid sequence encoded by the nucleic acid sequence of SEQ ID NO: 2.

11. A method for identifying a protein involved in bone modulation comprising identifying a protein that has an expression level that is different in a first host comprising the Zmax1 gene of SEQ ID NO:1 when compared to a second host comprising a nucleic acid sequence of SEQ ID NO: 2 or a nucleic acid sequence encoding the amino acid sequence of SEQ ID NO: 4.

12. A method of testing for high bone mass (HBM) activity comprising immobilizing an HBM protein of SEQ ID NO:4, binding a protein to the HBM protein, and measuring the extent of binding.

13. A method for identification of a candidate molecule involved in bone modulation comprising
identifying a molecule that binds to, or that inhibits binding of a molecule to, the nucleic acid sequence of SEQ ID NO: 1;
identifying a molecule that binds to, or that inhibits binding of a molecule to, the nucleic acid sequence of SEQ ID NO: 2; and
comparing the extent of binding, or the extent of inhibition of binding, of the molecule to each nucleic acid sequence, wherein the molecule that binds, or inhibits binding, more or less to the nucleic acid sequence of SEQ ID NO: 2 or the nucleic acid sequence of SEQ ID NO: 1 is the candidate molecule.

14. An *in vitro* method of pharmaceutical development for treatment of bone development disorders comprising identifying a molecule that binds to the amino acid sequence of SEQ ID NO: 4.

15. An *in vitro* method of pharmaceutical development for treatment of bone development disorders the method comprising
constructing a first host cell that contains the Zmax1 gene of SEQ ID NO:1 or protein of SEQ ID NO:3;
constructing a second host cell that contains a nucleic acid according to SEQ ID NO: 2 or a protein according to SEQ ID NO: 4;
analyzing a difference between the first host cell and the second host cell;
identifying a molecule that, when added to the first host cell, causes the first host cell to exhibit a characteristic feature of the second host cell.

16. A method for diagnostic screening for a genetic predisposition to a bone development disorder comprising screening a sample from a patient with a nucleic acid sequence of SEQ ID NO: 2 or a nucleic acid sequence encoding the amino acid sequence of SEQ ID NO: 4.

17. A diagnostic assay for bone development disorders comprising an antibody to an amino acid sequence according to SEQ ID NO: 4 or an amino acid sequence encoded by a nucleic acid of SEQ ID NO: 2.

18. An isolated nucleic acid sequence comprising at least 15 contiguous nucleotides of SEQ ID NO: 2, wherein said 15 contiguous nucleotides comprise position 582 of SEQ ID NO: 2.

19. An isolated nucleic acid sequence comprising at least 15 contiguous nucleotides of SEQ ID NO: 2, wherein said 15 contiguous nucleotides comprise position 582 of SEQ ID NO: 2, and which encodes for an amino acid sequence including a valine corresponding to valine at position 171 of SEQ ID NO: 4 and wherein valine substitution is associated with a high bone mass phenotype.

20. An isolated nucleic acid segment of at least 15 contiguous nucleotides including a polymorphic site from the nucleic acid sequence of SEQ ID NO: 2 in which G at position 582 is replaced by T, and sequences complementary thereto.

21. A method of identifying a molecule involved in bone modulation comprising identifying a molecule that binds to HBM polypeptide of SEQ ID NO:4

22. An isolated high bone mass (HBM) polynucleotide comprising a nucleic acid selected from the group consisting of:
(a) a nucleic acid having SEQ ID NO: 2;
(b) a nucleic acid encoding a polypeptide of SEQ ID NO: 4;
(c) a nucleic acid which is complementary to a nucleic acid of (a), or (b);
(d) a nucleic acid comprising at least 15 contiguous nucleotides which comprise position 582 of SEQ ID NO:2 from a polynucleotide of (a), (b), or (c); wherein said isolated HBM polynucleotide encodes a protein or polypeptide,
which when administered to a subject modulates bone mass.

23. An isolated nucleic acid sequence comprising at least 15 contiguous nucleotides of SEQ ID NO: 2, wherein said 15 contiguous nucleotides comprise nucleotides which encode for an amino acid substitution at position 171 of SEQ ID NO: 4; which substitution is associated with a high bone mass phenotype.

24. An isolated high bone mass (HBM) polynucleotide comprising a nucleic acid selected from the group consisting of:
(a) a nucleic acid having SEQ ID NO: 2;
(b) a nucleic acid encoding a polypeptide of SEQ ID NO: 4;
(c) a nucleic acid which is complementary to a nucleic acid of (a), (b); and
(d) a nucleic acid comprising at least 15 contiguous nucleotides which comprise position 582 of SEQ ID NO: 2 from a polynucleotide of (a), (b), and (c).

25. The use of an antibody that binds to a polypeptide encoded by a nucleic acid according to any of claims 18, 20, 22 or 24 involved in focal adhesion signaling, for the manufacture of a medicament for modulating bone density.

26. The use of an antibody which binds to a polypeptide encoded by a nucleic acid of any of claims 18, 20, 22 or 24 involved in focal adhesion signaling, for the manufacture of a medicament for treating bone development disorders.

27. Use according to claim 25 or 26 wherein the polypeptide is SEQ ID NO:4.

28. An antibody that binds to a polypeptide encoded by a nucleic acid according to any of claims 18, 20, 22 or 24 involved in focal adhesion signaling, for modulating bone density.

29. An antibody which binds to a polypeptide encoded by a nucleic acid of any of claims 18, 20, 22 or 24 involved in focal adhesion signaling, for treating bone development disorders.

30. An antibody according to claim 28 or 29 wherein the polypeptide is SEQ ID NO:4.

## Patentansprüche

1. Isolierte Nucleinsäuresequenz gemäß SEQ ID NO:2.

2. Isolierte Nucleinsäuresequenz nach Anspruch 1, wobei es sich bei der Nucleinsäuresequenz um DNA handelt.

3. Isolierte Aminosäuresequenz gemäß SEQ ID NO:4.

4. Nucleinsäuresequenz, die für die Aminosäuresequenz von SEQ ID NO:4 kodiert.

5. Replikativer Klonierungsvektor, umfassend die Nucleinsäuresequenz von Anspruch 1 und ein in einer isolierten Wirtszelle operatives Replikon.

6. Isolierte Wirtszelle, die mit dem replikativen Klonierungsvektor von Anspruch 5 transformiert ist.

7. Expressionsvektor, umfassend die Nucleinsäuresequenz von Anspruch 1 in funktioneller Verknüpfung mit einer regulatorischen Transkriptionsregion.

8. Isolierte Wirtszelle, die mit dem Expressionsvektor von Anspruch 7 transformiert ist.

9. In vitro-Verfahren zum Testen einer Substanz als therapeutisches Mittel zur Knochenmodulation in einem Wirt, umfassend die Verabreichung der Nucleinsäure von Anspruch 1 an eine Wirtszelle und die Feststellung, ob eine Knochenmodulation eintritt.

10. Verfahren zum Identifizieren eines an der Knochenmodulation beteiligten Moleküls, umfassend das Identifizieren eines Moleküls, das an eine isolierte Aminosäuresequenz gemäß SEQ ID NO:4 oder an eine durch die Nucleinsäuresequenz von SEQ ID NO: 2 kodierte Aminosäuresequenz bindet, oder die Bindung eines Moleküls an eine isolierte Aminosäuresequenz gemäß SEQ ID NO:4 oder an eine durch die Nucleinsäuresequenz von SEQ ID NO:2 kodierte Aminosäuresequenz hemmt.

11. Verfahren zum Identifizieren eines an der Knochenmodulation beteiligten Proteins, umfassend das Identifizieren eines Proteins, das einen Expressionsgrad aufweist, der in einem ersten Wirt, der das Zmaxl-Gen gemäß SEQ ID NO:1 umfasst, unterschiedlich ist im Vergleich zu einem zweiten Wirt, der eine Nucleinsäuresequenz gemäß SEQ ID NO:2 oder eine für die Aminosäuresequenz gemäß SEQ ID NO:4 kodierende Nucleinsäuresequenz umfasst.

12. Verfahren zum Testen auf eine hohe Knochenmasseaktivität (HBM-Aktivität), umfassend das Immobilisieren eines HBM-Proteins gemäß SEQ ID NO:4, das Binden eines Proteins an das HBM-Protein und das Messen des Ausmaßes der Bindung.

13. Verfahren zum Identifizieren eines als Kandidat in Frage kommenden Moleküls, das an der Knochenmodulation beteiligt ist, umfassend
das Identifizieren eines Moleküls, das an die Nucleinsäuresequenz gemäß SEQ ID NO:1 bindet oder die Bindung eines Moleküls an die Nucleinsäuresequenz von SEQ ID NO:1 hemmt;
das Identifizieren eines Moleküls, das an die Nucleinsäuresequenz gemäß SEQ ID NO:2 bindet oder die Bindung eines Moleküls an die Nucleinsäuresequenz gemäß SEQ ID NO:2 hemmt; und
das Vergleichen des Ausmaßes der Bindung oder des Ausmaßes der Hemmung der Bindung des Moleküls an die jeweilige Nucleinsäuresequenz,
wobei das Molekül, das mehr oder weniger an die Nucleinsäuresequenz gemäß SEQ ID NO:2 oder die Nucleinsäuresequenz gemäß SEQ ID NO:1 bindet oder die Bindung daran hemmt, das als Kandidat in Frage kommende Molekül darstellt.

14. In vitro-Verfahren zur Entwicklung eines Arzneimittels zur Behandlung von Knochenentwicklungsstörungen, umfassend das Identifizieren eines Moleküls, das an die Aminosäuresequenz gemäß SEQ ID NO:4 bindet.

15. In vitro-Verfahren zur Entwicklung eines Arzneimittels zur Behandlung von Knochenentwicklungsstörungen, wobei das Verfahren folgendes umfasst:
das Konstruieren einer ersten Wirtszelle, die das Zmaxl-Gen gemäß SEQ ID NO:1 oder das Protein von SEQ ID NO:3 enthält;
das Konstruieren einer zweiten Wirtszelle, die eine Nucleinsäure gemäß SEQ ID NO:2 oder ein für SEQ ID NO:4 kodierendes Protein enthält;
das Analysieren einer Differenz zwischen der ersten Wirtszelle und der zweiten Wirtszelle; und
das Identifizieren eines Moleküls, das bei Zugabe zur ersten Wirtszelle bewirkt, dass die erste Wirtszelle ein charakteristisches Merkmal der zweiten Wirtszelle aufweist.

16. Verfahren zum diagnostischen Screening auf eine genetische Veranlagung für eine Knochenentwicklungsstörung, umfassend das Screening einer Probe eines Patienten mit einer Nucleinsäuresequenz von SEQ ID NO:2 oder einer Nucleinsäuresequenz, die für die Aminosäuresequenz von SEQ ID NO:4 kodiert.

17. Diagnostischer Test für Knochenentwicklungsstörungen, umfassend einen Antikörper gegen eine Aminosäuresequenz gemäß SEQ ID NO:4 oder eine durch eine Nucleinsäure gemäß SEQ ID NO:2 kodierte Aminosäuresequenz.

18. Isolierte Nucleinsäuresequenz, umfassend mindestens 15 zusammenhängende Nucleotide von SEQ ID NO:2, wobei die 15 zusammenhängenden Nucleotide die Position 582 von SEQ ID NO:2 umfassen.

19. Isolierte Nucleinsäuresequenz, umfassend mindestens 15 zusammenhängende Nucleotide von SEQ ID NO:2, wobei die 15 zusammenhängenden Nucleotide die Position 582 von SEQ ID NO:2 umfassen und wobei die isolierte Nucleinsäuresequenz für eine Aminosäuresequenz kodiert, die ein Valin entsprechend dem Valin in Position 171 von SEQ ID NO:4 umfasst und wobei die Valin-Substitution mit einem Phänotyp von hoher Knochenmasse verbunden ist.

20. Isoliertes Nucleinsäuresegment mit mindestens 15 zusammenhängenden Nucleotiden, umfassend eine polymorphe Stelle aus der Nucleinsäuresequenz von SEQ ID NO:2, wobei G in Position 582 durch T ersetzt ist, sowie hierzu komplementäre Sequenzen.

21. Verfahren zum Identifizieren eines Moleküls, das an der Knochenmodulation beteiligt ist, umfassend das Identifizieren eines Moleküls, das an das HBM-Polypeptid gemäß SEQ ID NO:4 bindet.

22. Isoliertes HBM (hohe Knochenmasse)-Polynucleotid, umfassend eine Nucleinsäure, ausgewählt aus der Gruppe, bestehend aus:
(a) einer Nucleinsäure mit der Sequenz gemäß SEQ ID NO:2;
(b) einer Nucleinsäure, die für ein Polypeptid gemäß SEQ ID NO:4 kodiert;
(c) einer Nucleinsäure, die komplementär zu einer Nucleinsäure gemäß
(a) oder (b) ist; und
(d) einer Nucleinsäure, die mindestens 15 zusammenhängende Nucleotide umfasst, die die Position 582 von SEQ ID NO:2 umfassen, aus einem Polynucleotid gemäß (a), (b) oder (c);
wobei das isolierte HBM-Polynucleotid für ein Protein oder ein Polypeptid kodiert, das bei Verabreichung an ein Subjekt die Knochenmasse moduliert.

23. Isolierte Nucleinsäuresequenz, umfassend mindestens 15 zusammenhängende Nucleotide von SEQ ID NO:2, wobei die 15 zusammenhängenden Nucleotide Nucleotide umfassen, die für eine Aminosäuresubstitution in Position 171 von SEQ ID NO:4 kodieren, wobei die Substition mit einem Phänotyp von hoher Knochenmasse verbunden ist.

24. Isoliertes HBM (hohe Knochenmasse)-Polynucleotid, umfassend eine Nucleinsäure, ausgewählt aus der Gruppe, bestehend aus:
(a) einer Nucleinsäure mit der Sequenz gemäß SEQ ID NO:2;
(b) einer Nucleinsäure, die für ein Polypeptid gemäß SEQ ID NO:4 kodiert;
(c) einer Nucleinsäure, die komplementär zu einer Nucleinsäure gemäß
(a) oder (b) ist; und
(d) einer Nucleinsäure, die mindestens 15 zusammenhängende Nucleotide umfasst, die die Position 582 von SEQ ID NO:2 umfassen, aus einem Polynucleotid gemäß (a), (b) und (c).

25. Verwendung eines Antikörpers, der an ein Polypeptid bindet, das durch eine Nucleinsäure gemäß einem der Ansprüche 18, 20, 22 oder 24 kodiert wird und an der fokalen Adhäsionsignalgebung beteiligt ist, zur Herstellung eines Arzneimittels zur Modulation der Knochendichte.

26. Verwendung eines Antikörpers, der an ein Polypeptid bindet, das durch eine Nucleinsäure gemäß einem der Ansprüche 18, 20, 22 oder 24 kodiert wird und an der fokalen Adhäsionssignalgebung beteiligt ist, zur Herstellung eines Arzneimittels zur Behandlung von Knochenentwicklungsstörungen.

27. Verwendung nach Anspruch 25 oder 26, wobei das Polypeptid die Sequenz gemäß SEQ ID NO:4 aufweist.

28. Antikörper, der an ein Polypeptid bindet, das durch eine Nucleinsäure gemäß einem der Ansprüche 18, 20, 22 oder 24 kodiert wird und an der fokalen Adhäsionssignalgebung beteiligt ist, zur Modulation der Knochendichte.

29. Antikörper, der an ein Polypeptid bindet, das durch eine Nucleinsäure gemäß einem der Ansprüche 18, 20, 22 oder 24 kodiert wird und an der fokalen Adhäsionssignalgebung beteiligt ist, zur Behandlung von Knochenentwicklungsstörungen.

30. Antikörper nach Anspruch 28 oder 29, wobei das Polypeptid die Sequenz gemäß SEQ ID NO:4 aufweist.

## Revendications

1. Séquence d'acide nucléique isolée de SEQ ID NO :2.

2. Séquence d'acide nucléique isolée selon la revendication 1, qui est un ADN.

3. Séquence d'acide aminé isolée de SEQ ID NO :4.

4. Séquence d'acide nucléique codant la séquence en acides aminés de SEQ ID NO :4.

5. Vecteur de clonage réplicatif comprenant la séquence d'acides nucléiques de la revendication 1 et un réplicon fonctionnel dans une cellule-hôte isolée.

6. Cellule-hôte isolée transformée avec le vecteur de clonage réplicatif de la revendication 5.

7. Vecteur d'expression comprenant la séquence en acides nucléiques de la revendication 1, lié de façon efficace à une région de régulation de la transcription.

8. Cellule-hôte isolée transformée avec le vecteur d'expression de la revendication 7.

9. Procédé *in vitro* pour tester une substance en tant qu'agent thérapeutique pour la modulation de l'os dans un hôte, comprenant le fait d'administrer l'acide nucléique de la revendication 1 à une cellule-hôte, et le fait de tester si une modulation de l'os intervient.

10. Procédé d'indentification d'une molécule impliquée dans la modulation de l'os, comprenant le fait d'identifier une molécule qui se fixe ou inhibe la fixation d'une molécule à une séquence d'acides aminés isolée de SEQ ID NO :4 ou une séquence en acides aminés codée par la séquence d'acides nucléiques de SEQ ID NO':2.

11. Procédé d'identification d'une protéine impliquée dans la modulation de l'os, comprenant le fait d'identifier une protéine qui a un niveau d'expression différent, dans un premier hôte comprenant le gène Zmax1 de SEQ ID NO :1, quand on le compare à un second hôte comprenant une séquence d'acides nucléiques de SEQ ID NO :2 ou une séquence d'acides nucléiques codant la séquence en acides aminés de SEQ ID NO :4.

12. Procédé pour tester l'activité de haute masse osseuse (HBM) comprenant le fait d'immobiliser une protéine HBM de SEQ ID NO :4, le fait de fixer une protéine à la protéine HBM, et le fait de mesurer l'importance de la fixation.

13. Procédé d'identification d'une molécule candidate impliquée dans la modulation de l'os, comprenant
- le fait d'identifier une molécule qui se fixe ou inhibe la fixation d'une molécule à la séquence d'acides nucléiques de SEQ ID NO :1,
- le fait d'identifier une molécule qui se fixe ou inhibe la fixation d'une molécule à la séquence d'acides nucléiques de SEQ ID NO :2, et
- le fait de comparer l'importance de la fixation, ou l'importance de l'inhibition de la fixation de la molécule sur chaque séquence d'acides nucléiques, dans laquelle la molécule qui se fixe ou inhibe plus ou moins la fixation à la séquence d'acides nucléiques de SEQ ID NO :2, ou à la séquence d'acides nucléiques de SEQ ID NO :1 est la molécule candidate.

14. Procédé *in vitro* de développement pharmaceutique pour le traitement de troubles du développement de l'os comprenant l'identification d'une molécule qui se fixe à la séquence en acides aminés de SEQ ID NO :4.

15. Procédé *in vitro* de développement pharmaceutique pour le traitement de troubles du développement de l'os, comprenant :
- le fait de construire une première cellule-hôte qui contient le gène Zmax1 de SEQ ID NO :1 ou la protéine de SEQ ID NO : 3 ;
- le fait de construire une seconde cellule-hôte qui contient un acide nucléique selon SEQ ID NO :2 ou une protéine selon SEQ ID NO :4 ;
- le fait d'analyser la différence entre la première cellule-hôte et la seconde cellule-hôte ; et
- le fait d'identifier une molécule qui, lorsqu'elle est ajoutée à la première cellule-hôte, provoque dans cette première cellule-hôte l'expression d'une caractéristique de la seconde cellule-hôte.

16. Procédé de test de diagnostic d'une prédisposition génétique à un trouble du développement de l'os, comprenant le fait de tester un échantillon d'un patient avec une séquence d'acides nucléiques de SEQ ID NO :2 ou une séquence d'acides nucléiques codant la séquence en acides aminés de SEQ ID NO :4.

17. Procédé de diagnostic pour les troubles du développement de l'os, comprenant un anticorps dirigé contre une séquence en acides aminés selon SEQ ID NO :4, ou une séquence en acides aminés codée par un acide nucléique de SEQ ID NO :2.

18. Séquence d'acides nucléiques isolée comprenant au moins 15 nucléotides contigus de SEQ ID NO :2, qui comprend la position 582 de SEQ ID NO :2.

19. Séquence d'acides nucléiques isolée comprenant au moins 15 nucléotides contigus de SEQ ID NO :2, dans laquelle lesdits 15 nucléotides contigus comprennent la position 582 de SEQ ID NO :2, et qui code une séquence en acides aminés incluant une valine correspondant à la valine à la position 171 de SEQ ID NO :4 et dans laquelle la substitution de la valine est associée à un phénotype de haute masse osseuse.

20. Segment d'acides nucléiques isolé d'au moins 15 nucléotides contigus incluant un site polymorphe, de la séquence d'acides nucléiques de SEQ ID NO :2 dans lequel le G à la position 582 est remplacé par un T, ainsi que ses séquences complémentaires.

21. Procédé d'identification d'une molécule impliquée dans la modulation de l'os, comprenant le fait d'identifier une molécule qui se fixe au polypeptide HBM de SEQ ID NO :4.

22. Polynucléotide isolé de HBM comprenant un acide nucléique choisi dans le groupe constitué de :
(a) un acide nucléique ayant la séquence SEQ ID NO :2 ;
(b) un acide nucléique codant un polypeptide de SEQ ID NO :4 ;
(c) un acide nucléique complémentaire d'un acide nucléique de
(a) ou (b) ;
(d) un acide nucléique comprenant au moins 15 nucléotides contigus, qui comprend la position 582 de SEQ ID NO :2 d'un polynucléotide de (a), (b) ou (c) ;
dans lequel ledit polynucléotide HBM isolé code une protéine ou un polypeptide qui, lorsqu'il est administré à un sujet, module la masse osseuse.

23. Séquence d'acides nucléiques isolée comprenant au moins 15 nucléotides contigus de SEQ ID NO :2, dans laquelle lesdits 15 nucléotides contigus comprennent des nucléotides qui codent une substitution de l'acide aminé à la position 171 de SEQ ID NO :4, ladite substitution étant associée à un phénotype de haute masse osseuse.

24. Polynucléotide HBM isolé comprenant un acide nucléique choisi dans le groupe constitué de :
(a) un acide nucléique ayant la séquence de SEQ ID NO :2 ;
(b) un acide nucléique codant un polypeptide de SEQ ID NO :4 ;
(c) un acide nucléique complémentaire d'un acide nucléique de (a), (b) ; et
(d) un acide nucléique comprenant au moins 15 nucléotides contigus qui comprennent la position 582 de SEQ ID NO :2 d'un polynucléotide de (a), (b) ou (c).

25. Utilisation d'un anticorps qui se fixe à un polypeptide codé par un acide nucléique selon l'une quelconque des revendications 18, 20, 22 ou 24, impliqué dans le signal d'adhésion focale pour la fabrication d'un médicament afin de moduler la densité osseuse.

26. Utilisation d'un anticorps qui se fixe à un polypeptide codé par un acide nucléique selon l'une des revendications 18, 20, 22 ou 24, impliqué dans le signal d'adhésion focale pour la fabrication d'un médicament pour le traitement des troubles du développement osseux.

27. Utilisation selon l'une quelconque des revendications 25 ou 26, dans laquelle le polypeptide est SQ ID NO :4.

28. Anticorps qui se fixe à un polypeptide codé par un acide nucléique selon l'une quelconque des revendications 18, 20, 22 ou 24, impliqué dans le signal d'adhésion focale pour moduler la densité osseuse.

29. Anticorps qui se fixe à un polypeptide codé par un acide nucléique selon l'une quelconque des revendications 18, 20, 22 ou 24, impliqué dans le signal d'adhésion focale pour le traitement de troubles du développement de l'os.

30. Anticorps selon l'une des revendications 28 ou 29, dans lequel le polypeptide est SEQ ID NO :4.


### SEQUENCE LISTING

<110> John P. Carulli et al.
   <120> THE HIGH BONE MASS GENE OF 11q13.3
   <130> 032796-021
<150> US 09/544,398
   <151> 2000-04-05
   <150> US 09/543,771
   <151> 2000-04-05
   <150> US 09/229,319
   <151> 1999-01-13
   <150> US 60/071,449
   <151> 1998-01-13
   <150> US 60/105,511
   <151> 1998-10-23
   <160> 109
<210> 1
   <211> 5120
   <212> DNA
   <213> Homo sapiens
<400> 1
   actaaagcgc cgccgccgcg ccatggagcc cgagtgagcg cggcgcgggc ccgtccggcc 60
   gccggacaac atg gag gca gcg ccg ccc ggg ccg ccg tgg ccg ctg ctg 109
   ctg ctg ctg ctg ctg ctg ctg gcg ctg tgc ggc tgc ccg gcc ccc gcc 157
   gcg gcc tcg ccg ctc ctg cta ttt gcc aac cgc cgg gac gta cgg ctg 205
   gtg gac gcc ggc gga gtc aag ctg gag tcc acc atc gtg gtc agc ggc 253
   ctg gag gat gcg gcc gca gtg gac ttc cag ttt tcc aag gga gcc gtg 301
   tac tgg aca gac gtg agc gag gag gcc atc aag cag acc tac ctg aac 349
   cag acg ggg gcc gcc gtg cag aac gtg gtc atc tcc ggc ctg gtc tct 397
   ccc gac ggc ctc gcc tgc gac tgg gtg ggc aag aag ctg tac tgg acg 445
   gac tca gag acc aac cgc atc gag gtg gcc aac ctc aat ggc aca tcc 493
   cgg aag gtg ctc ttc tgg cag gac ctt gac cag ccg agg gcc atc gcc 541
   ttg gac ccc gct cac ggg tac atg tac tgg aca gac tgg ggt gag acg 589
   ccc cgg att gag cgg gca ggg atg gat ggc agc acc cgg aag atc att 637
   gtg gac tcg gac att tac tgg ccc aat gga ctg acc atc gac ctg gag 685
   gag cag aag ctc tac tgg gct gac gcc aag ctc agc ttc atc cac cgt 733
   gcc aac ctg gac ggc tcg ttc cgg cag aag gtg gtg gag ggc agc ctg 781
   acg cac ccc ttc gcc ctg acg ctc tcc ggg gac act ctg tac tgg aca 829
   gac tgg cag acc cgc tcc atc cat gcc tgc aac aag cgc act ggg ggg 877
   aag agg aag gag atc ctg agt gcc ctc tac tca ccc atg gac atc cag 925
   gtg ctg agc cag gag cgg cag cct ttc ttc cac act cgc tgt gag gag 973
   gac aat ggc ggc tgc tcc cac ctg tgc ctg ctg tcc cca agc gag cct 1021
   ttc tac aca tgc gcc tgc ccc acg ggt gtg cag ctg cag gac aac ggc 1069
   agg acg tgt aag gca gga gcc gag gag gtg ctg ctg ctg gcc cgg cgg 1117
   acg gac cta cgg agg atc tcg ctg gac acg ccg gac ttc acc gac atc 1165
   gtg ctg cag gtg gac gac atc cgg cac gcc att gcc atc gac tac gac 1213
   ccg cta gag ggc tat gtc tac tgg aca gat gac gag gtg cgg gcc atc 1261
   cgc agg gcg tac ctg gac ggg tct ggg gcg cag acg ctg gtc aac acc 1309
   gag atc aac gac ccc gat ggc atc gcg gtc gac tgg gtg gcc cga aac 1357
   ctc tac tgg acc gac acg ggc acg gac cgc atc gag gtg acg cgc ctc 1405
   aac ggc acc tcc cgc aag atc ctg gtg tcg gag gac ctg gac gag ccc      1453
   cga gcc atc gca ctg cac ccc gtg atg ggc ctc atg tac tgg aca gac      1501
   tgg gga gag aac cct aaa atc gag tgt gcc aac ttg gat ggg cag gag      1549
   cgg cgt gtg ctg gtc aat gcc tcc ctc ggg tgg ccc aac ggc ctg gcc      1597
   ctg gac ctg cag gag ggg aag ctc tac tgg gga gac gcc aag aca gac      1645
   aag atc gag gtg atc aat gtt gat ggg acg aag agg cgg acc ctc ctg      1693
   gag gac aag ctc ccg cac att ttc ggg ttc acg ctg ctg ggg gac ttc      1741
   atc tac tgg act gac tgg cag cgc cgc agc atc gag cgg gtg cac aag      1789
   gtc aag gcc agc cgg gac gtc atc att gac cag ctg ccc gac ctg atg      1837
   ggg ctc aaa gct gtg aat gtg gcc aag gtc gtc gga acc aac ccg tgt 1885
   gcg gac agg aac ggg ggg tgc agc cac ctg tgc ttc ttc aca ccc cac 1933
   gca acc cgg tgt ggc tgc ccc atc ggc ctg gag ctg ctg agt gac atg 1981
   aag acc tgc atc gtg cct gag gcc ttc ttg gtc ttc acc agc aga gcc 2029
   gcc atc cac agg atc tcc ctc gag acc aat aac aac gac gtg gcc atc 2077
   ccg ctc acg ggc gtc aag gag gcc tca gcc ctg gac ttt gat gtg tcc 2125
   aac aac cac atc tac tgg aca gac gtc agc ctg aag acc atc agc cgc 2173
   gcc ttc atg aac ggg agc tcg gtg gag cac gtg gtg gag ttt ggc ctt 2221
   gac tac ccc gag ggc atg gcc gtt gac tgg atg ggc aag aac ctc tac 2269
   tgg gcc gac act ggg acc aac aga atc gaa gtg gcg cgg ctg gac ggg 2317
   cag ttc cgg caa gtc ctc gtg tgg agg gac ttg gac aac ccg agg tcg 2365
   ctg gcc ctg gat ccc acc aag ggc tac atc tac tgg acc gag tgg ggc 2413
   ggc aag ccg agg atc gtg cgg gcc ttc atg gac ggg acc aac tgc atg 2461
   acg ctg gtg gac aag gtg ggc cgg gcc aac gac ctc acc att gac tac 2509
   gct gac cag cgc ctc tac tgg acc gac ctg gac acc aac atg atc gag 2557
   tcg tcc aac atg ctg ggt cag gag cgg gtc gtg att gcc gac gat ctc 2605
   ccg cac ccg ttc ggt ctg acg cag tac agc gat tat atc tac tgg aca 2653
   gac tgg aat ctg cac agc att gag cgg gcc gac aag act agc ggc cgg 2701
   aac cgc acc ctc atc cag ggc cac ctg gac ttc gtg atg gac atc ctg 2749
   gtg ttc cac tcc tcc cgc cag gat ggc ctc aat gac tgt atg cac aac 2797
   aac ggg cag tgt ggg cag ctg tgc ctt gcc atc ccc ggc ggc cac cgc 2845
   tgc ggc tgc gcc tca cac tac acc ctg gac ccc agc agc cgc aac tgc 2893
   agc ccg ccc acc acc ttc ttg ctg ttc agc cag aaa tct gcc atc agt 2941
   cgg atg atc ccg gac gac cag cac agc ccg gat ctc atc ctg ccc ctg 2989
   cat gga ctg agg aac gtc aaa gcc atc gac tat gac cca ctg gac aag 3037
   ttc atc tac tgg gtg gat ggg cgc cag aac atc aag cga gcc aag gac 3085
   gac ggg acc cag ccc ttt gtt ttg acc tct ctg agc caa ggc caa aac 3133
   cca gac agg cag ccc cac gac ctc agc atc gac atc tac agc cgg aca 3181
   ctg ttc tgg acg tgc gag gcc acc aat acc atc aac gtc cac agg ctg 3229
   agc ggg gaa gcc atg ggg gtg gtg ctg cgt ggg gac cgc gac aag ccc 3277
   agg gcc atc gtc gtc aac gcg gag cga ggg tac ctg tac ttc acc aac 3325
   atg cag gac cgg gca gcc aag atc gaa cgc gca gcc ctg gac ggc acc 3373
   gag cgc gag gtc ctc ttc acc acc ggc ctc atc cgc cct gtg gcc ctg 3421
   gtg gtg gac aac aca ctg ggc aag ctg ttc tgg gtg gac gcg gac ctg 3469
   aag cgc att gag agc tgt gac ctg tca ggg gcc aac cgc ctg acc ctg 3517
   gag gac gcc aac atc gtg cag cct ctg ggc ctg acc atc ctt ggc aag 3565
   cat ctc tac tgg atc gac cgc cag cag cag atg atc gag cgt gtg gag 3613
   aag acc acc ggg gac aag cgg act cgc atc cag ggc cgt gtc gcc cac 3661
   ctc act ggc atc cat gca gtg gag gaa gtc agc ctg gag gag ttc tca 3709
   gcc cac cca tgt gcc cgt gac aat ggt ggc tgc tcc cac atc tgt att 3757
   gcc aag ggt gat ggg aca cca cgg tgc tca tgc cca gtc cac ctc gtg 3805
   ctc ctg cag aac ctg ctg acc tgt gga gag ccg ccc acc tgc tcc ccg 3853
   gac cag ttt gca tgt gcc aca ggg gag atc gac tgt atc ccc ggg gcc 3901
   tgg cgc tgt gac ggc ttt ccc gag tgc gat gac cag agc gac gag gag 3949
   ggc tgc ccc gtg tgc tcc gcc gcc cag ttc ccc tgc gcg cgg ggt cag 3997
   tgt gtg gac ctg cgc ctg cgc tgc gac ggc gag gca gac tgt cag gac 4045
   cgc tca gac gag gtg gac tgt gac gcc atc tgc ctg ccc aac cag ttc 4093
   cgg tgt gcg agc ggc cag tgt gtc ctc atc aaa cag cag tgc gac tcc 4141
   ttc ccc gac tgt atc gac ggc tcc gac gag ctc atg tgt gaa atc acc 4189
   aag ccg ccc tca gac gac agc ccg gcc cac agc agt gcc atc ggg ccc 4237
   gtc att ggc atc atc ctc tct ctc ttc gtc atg ggt ggt gtc tat ttt 4285
   gtg tgc cag cgc gtg gtg tgc cag cgc tat gcg ggg gcc aac ggg ccc 4333
   ttc ccg cac gag tat gtc agc ggg acc ccg cac gtg ccc ctc aat ttc 4381
   ata gcc ccg ggc ggt tcc cag cat ggc ccc ttc aca ggc atc gca tgc 4429
   gga aag tcc atg atg agc tcc gtg agc ctg atg ggg ggc cgg ggc ggg 4477
   gtg ccc ctc tac gac cgg aac cac gtc aca ggg gcc tcg tcc agc agc 4525
   tcg tcc agc acg aag gcc acg ctg tac ccg ccg atc ctg aac ccg ccg 4573
   ccc tcc ccg gcc acg gac ccc tcc ctg tac aac atg gac atg ttc tac 4621
   tct tca aac att ccg gcc act gcg aga ccg tac agg ccc tac atc att 4669
   cga gga atg gcg ccc ccg acg acg ccc tgc agc acc gac gtg tgt gac 4717
   agc gac tac agc gcc agc cgc tgg aag gcc agc aag tac tac ctg gat 4765
   ttg aac tcg gac tca gac ccc tat cca ccc cca ccc acg ccc cac agc 4813
   cag tac ctg tcg gcg gag gac agc tgc ccg ccc tcg ccc gcc acc gag 4861
   agg agc tac ttc cat ctc ttc ccg ccc cct ccg tcc ccc tgc acg gac 4909
   tca tcc tgacctcggc cgggccactc tggcttctct gtgcccctgt aaatagtttt 4965
   aaatatgaac aaagaaaaaa atatatttta tgatttaaaa aataaatata attgggattt 5025
   taaaaacatg agaaatgtga actgtgatgg ggtgggcagg gctgggagaa ctttgtacag 5085
   tggagaaata tttataaact taattttgta aaaca 5120
<210> 2
   <211> 5120
   <212> DNA
   <213> Homo sapiens
<400> 2
   actaaagcgc cgccgccgcg ccatggagcc cgagtgagcg cggcgcgggc ccgtccggcc 60
   gccggacaac atg gag gca gcg ccg ccc ggg ccg ccg tgg ccg ctg ctg 109
   Met Glu Ala Ala Pro Pro Gly Pro Pro Trp Pro Leu Leu
   1 5 10
   ctg ctg ctg ctg ctg ctg ctg gcg ctg tgc ggc tgc ccg gcc ccc gcc 157
   Leu Leu Leu Leu Leu Leu Leu Ala Leu Cys Gly Cys Pro Ala Pro Ala
   15 20 25
   gcg gcc tcg ccg ctc ctg cta ttt gcc aac cgc cgg gac gta cgg ctg 205
   Ala Ala Ser Pro Leu Leu Leu Phe Ala Asn Arg Arg Asp Val Arg Leu
   30 35 40 45
   gtg gac gcc ggc gga gtc aag ctg gag tcc acc atc gtg gtc agc ggc      253
   Val Asp Ala Gly Gly Val Lys Leu Glu Ser Thr Ile Val Val Ser Gly
                   50                  55                  60
   ctg gag gat gcg gcc gca gtg gac ttc cag ttt tcc aag gga gcc gtg      301
   Leu Glu Asp Ala Ala Ala Val Asp Phe Gln Phe Ser Lys Gly Ala Val
               65                  70                  75
   tac tgg aca gac gtg agc gag gag gcc atc aag cag acc tac ctg aac      349
   Tyr Trp Thr Asp Val Ser Glu Glu Ala Ile Lys Gln Thr Tyr Leu Asn
           80                  85                  90
   cag acg ggg gcc gcc gtg cag aac gtg gtc atc tcc ggc ctg gtc tct      397
   Gln Thr Gly Ala Ala Val Gln Asn Val Val Ile Ser Gly Leu Val Ser
       95                  100                 105
   ccc gac ggc ctc gcc tgc gac tgg gtg ggc aag aag ctg tac tgg acg      445
   Pro Asp Gly Leu Ala Cys Asp Trp Val Gly Lys Lys Leu Tyr Trp Thr
   110                 115                 120                 125
   gac tca gag acc aac cgc atc gag gtg gcc aac ctc aat ggc aca tcc      493
   Asp Ser Glu Thr Asn Arg Ile Glu Val Ala Asn Leu Asn Gly Thr Ser
                   130                 135                 140
   cgg aag gtg ctc ttc tgg cag gac ctt gac cag ccg agg gcc atc gcc      541
   Arg Lys Val Leu Phe Trp Gln Asp Leu Asp Gln Pro Arg Ala Ile Ala
               145                 150                 155
   ttg gac ccc gct cac ggg tac atg tac tgg aca gac tgg gtt gag acg      589
   Leu Asp Pro Ala His Gly Tyr Met Tyr Trp Thr Asp Trp Val Glu Thr
           160                 165                 170
   ccc cgg att gag cgg gca ggg atg gat ggc agc acc cgg aag atc att      637
   Pro Arg Ile Glu Arg Ala Gly Met Asp Gly Ser Thr Arg Lys Ile Ile
       175                 180                 185
   gtg gac tcg gac att tac tgg ccc aat gga ctg acc atc gac ctg gag 685
   Val Asp Ser Asp Ile Tyr Trp Pro Asn Gly Leu Thr Ile Asp Leu Glu
   190 195 200 205
   gag cag aag ctc tac tgg gct gac gcc aag ctc agc ttc atc cac cgt 733
   Glu Gln Lys Leu Tyr Trp Ala Asp Ala Lys Leu Ser Phe Ile His Arg
   210 215 220
   gcc aac ctg gac ggc tcg ttc cgg cag aag gtg gtg gag ggc agc ctg 781
   Ala Asn Leu Asp Gly Ser Phe Arg Gln Lys Val Val Glu Gly Ser Leu
   225 230 235
   acg cac ccc ttc gcc ctg acg ctc tcc ggg gac act ctg tac tgg aca 829
   Thr His Pro Phe Ala Leu Thr Leu Ser Gly Asp Thr Leu Tyr Trp Thr
   240 245 250
   gac tgg cag acc cgc tcc atc cat gcc tgc aac aag cgc act ggg ggg 877
   Asp Trp Gln Thr Arg Ser Ile His Ala Cys Asn Lys Arg Thr Gly Gly
   255 260 265
   aag agg aag gag atc ctg agt gcc ctc tac tca ccc atg gac atc cag 925
   Lys Arg Lys Glu Ile Leu Ser Ala Leu Tyr Ser Pro Met Asp Ile Gln
   270 275 280 285
   gtg ctg agc cag gag cgg cag cct ttc ttc cac act cgc tgt gag gag 973
   Val Leu Ser Gln Glu Arg Gln Pro Phe Phe His Thr Arg Cys Glu Glu
   290 295 300
   gac aat ggc ggc tgc tcc cac ctg tgc ctg ctg tcc cca agc gag cct 1021
   Asp Asn Gly Gly Cys Ser His Leu Cys Leu Leu Ser Pro Ser Glu Pro
   305 310 315
   ttc tac aca tgc gcc tgc ccc acg ggt gtg cag ctg cag gac aac ggc 1069
   Phe Tyr Thr Cys Ala Cys Pro Thr Gly Val Gln Leu Gln Asp Asn Gly
   320 325 330
   agg acg tgt aag gca gga gcc gag gag gtg ctg ctg ctg gcc cgg cgg 1117
   Arg Thr Cys Lys Ala Gly Ala Glu Glu Val Leu Leu Leu Ala Arg Arg
   335 340 345
   acg gac cta cgg agg atc tcg ctg gac acg ccg gac ttc acc gac atc 1165
   Thr Asp Leu Arg Arg Ile Ser Leu Asp Thr Pro Asp Phe Thr Asp Ile
   350 355 360 365
   gtg ctg cag gtg gac gac atc cgg cac gcc att gcc atc gac tac gac 1213
   Val Leu Gln Val Asp Asp Ile Arg His Ala Ile Ala Ile Asp Tyr Asp
   370 375 380
   ccg cta gag ggc tat gtc tac tgg aca gat gac gag gtg cgg gcc atc 1261
   Pro Leu Glu Gly Tyr Val Tyr Trp Thr Asp Asp Glu Val Arg Ala Ile
   385 390 395
   cgc agg gcg tac ctg gac ggg tct ggg gcg cag acg ctg gtc aac acc 1309
   Arg Arg Ala Tyr Leu Asp Gly Ser Gly Ala Gln Thr Leu Val Asn Thr
   400 405 410
   gag atc aac gac ccc gat ggc atc gcg gtc gac tgg gtg gcc cga aac 1357
   Glu Ile Asn Asp Pro Asp Gly Ile Ala Val Asp Trp Val Ala Arg Asn
   415 420 425
   ctc tac tgg acc gac acg ggc acg gac cgc atc gag gtg acg cgc ctc 1405
   Leu Tyr Trp Thr Asp Thr Gly Thr Asp Arg Ile Glu Val Thr Arg Leu
   430 435 440 445
   aac ggc acc tcc cgc aag atc ctg gtg tcg gag gac ctg gac gag ccc 1453
   Asn Gly Thr Ser Arg Lys Ile Leu Val Ser Glu Asp Leu Asp Glu Pro
   450 455 460
   cga gcc atc gca ctg cac ccc gtg atg ggc ctc atg tac tgg aca gac 1501
   Arg Ala Ile Ala Leu His Pro Val Met Gly Leu Met Tyr Trp Thr Asp
   465 470 475
   tgg gga gag aac cct aaa atc gag tgt gcc aac ttg gat ggg cag gag 1549
   Trp Gly Glu Asn Pro Lys Ile Glu Cys Ala Asn Leu Asp Gly Gln Glu
   480 485 490
   cgg cgt gtg ctg gtc aat gcc tcc ctc ggg tgg ccc aac ggc ctg gcc 1597
   Arg Arg Val Leu Val Asn Ala Ser Leu Gly Trp Pro Asn Gly Leu Ala
   495 500 505
   ctg gac ctg cag gag ggg aag ctc tac tgg gga gac gcc aag aca gac 1645
   Leu Asp Leu Gln Glu Gly Lys Leu Tyr Trp Gly Asp Ala Lys Thr Asp
   510 515 520 525
   aag atc gag gtg atc aat gtt gat ggg acg aag agg cgg acc ctc ctg 1693
   Lys Ile Glu Val Ile Asn Val Asp Gly Thr Lys Arg Arg Thr Leu Leu
   530 535 540
   gag gac aag ctc ccg cac att ttc ggg ttc acg ctg ctg ggg gac ttc 1741
   Glu Asp Lys Leu Pro His Ile Phe Gly Phe Thr Leu Leu Gly Asp Phe
   545 550 555
   atc tac tgg act gac tgg cag cgc cgc agc atc gag cgg gtg cac aag 1789
   Ile Tyr Trp Thr Asp Trp Gln Arg Arg Ser Ile Glu Arg Val His Lys
   560 565 570
   gtc aag gcc agc cgg gac gtc atc att gac cag ctg ccc gac ctg atg 1837
   Val Lys Ala Ser Arg Asp Val Ile Ile Asp Gln Leu Pro Asp Leu Met
   575 580 585
   ggg ctc aaa gct gtg aat gtg gcc aag gtc gtc gga acc aac ccg tgt 1885
   Gly Leu Lys Ala Val Asn Val Ala Lys Val Val Gly Thr Asn Pro Cys
   590 595 600 605
   gcg gac agg aac ggg ggg tgc agc cac ctg tgc ttc ttc aca ccc cac 1933
   Ala Asp Arg Asn Gly Gly Cys Ser His Leu Cys Phe Phe Thr Pro His
   610 615 620
   gca acc cgg tgt ggc tgc ccc atc ggc ctg gag ctg ctg agt gac atg 1981
   Ala Thr Arg Cys Gly Cys Pro Ile Gly Leu Glu Leu Leu Ser Asp Met
   625 630 635
   aag acc tgc atc gtg cct gag gcc ttc ttg gtc ttc acc agc aga gcc 2029
   Lys Thr Cys Ile Val Pro Glu Ala Phe Leu Val Phe Thr Ser Arg Ala
   640 645 650
   gcc atc cac agg atc tcc ctc gag acc aat aac aac gac gtg gcc atc 2077
   Ala Ile His Arg Ile Ser Leu Glu Thr Asn Asn Asn Asp Val Ala Ile
   655 660 665
   ccg ctc acg ggc gtc aag gag gcc tca gcc ctg gac ttt gat gtg tcc 2125
   Pro Leu Thr Gly Val Lys Glu Ala Ser Ala Leu Asp Phe Asp Val Ser
   670 675 680 685
   aac aac cac atc tac tgg aca gac gtc agc ctg aag acc atc agc cgc 2173
   Asn Asn His Ile Tyr Trp Thr Asp Val Ser Leu Lys Thr Ile Ser Arg
   690 695 700
   gcc ttc atg aac ggg agc tcg gtg gag cac gtg gtg gag ttt ggc ctt 2221
   Ala Phe Met Asn Gly Ser Ser Val Glu His Val Val Glu Phe Gly Leu
   705 710 715
   gac tac ccc gag ggc atg gcc gtt gac tgg atg ggc aag aac ctc tac 2269
   Asp Tyr Pro Glu Gly Met Ala Val Asp Trp Met Gly Lys Asn Leu Tyr
   720 725 730
   tgg gcc gac act ggg acc aac aga atc gaa gtg gcg cgg ctg gac ggg 2317
   Trp Ala Asp Thr Gly Thr Asn Arg Ile Glu Val Ala Arg Leu Asp Gly
   735 740 745
   cag ttc cgg caa gtc ctc gtg tgg agg gac ttg gac aac ccg agg tcg 2365
   Gln Phe Arg Gln Val Leu Val Trp Arg Asp Leu Asp Asn Pro Arg Ser
   750 755 760 765
   ctg gcc ctg gat ccc acc aag ggc tac atc tac tgg acc gag tgg ggc 2413
   Leu Ala Leu Asp Pro Thr Lys Gly Tyr Ile Tyr Trp Thr Glu Trp Gly
   770 775 780
   ggc aag ccg agg atc gtg cgg gcc ttc atg gac ggg acc aac tgc atg 2461
   Gly Lys Pro Arg Ile Val Arg Ala Phe Met Asp Gly Thr Asn Cys Met
   785 790 795
   acg ctg gtg gac aag gtg ggc cgg gcc aac gac ctc acc att gac tac 2509
   Thr Leu Val Asp Lys Val Gly Arg Ala Asn Asp Leu Thr Ile Asp Tyr
   800 805 810
   gct gac cag cgc ctc tac tgg acc gac ctg gac acc aac atg atc gag 2557
   Ala Asp Gln Arg Leu Tyr Trp Thr Asp Leu Asp Thr Asn Met Ile Glu
   815 820 825
   tcg tcc aac atg ctg ggt cag gag cgg gtc gtg att gcc gac gat ctc 2605
   Ser Ser Asn Met Leu Gly Gln Glu Arg Val Val Ile Ala Asp Asp Leu
   830 835 840 845
   ccg cac ccg ttc ggt ctg acg cag tac agc gat tat atc tac tgg aca 2653
   Pro His Pro Phe Gly Leu Thr Gln Tyr Ser Asp Tyr Ile Tyr Trp Thr
   850 855 860
   gac tgg aat ctg cac agc att gag cgg gcc gac aag act agc ggc cgg 2701
   Asp Trp Asn Leu His Ser Ile Glu Arg Ala Asp Lys Thr Ser Gly Arg
   865 870 875
   aac cgc acc ctc atc cag ggc cac ctg gac ttc gtg atg gac atc ctg 2749
   Asn Arg Thr Leu Ile Gln Gly His Leu Asp Phe Val Met Asp Ile Leu
   880 885 890
   gtg ttc cac tcc tcc cgc cag gat ggc ctc aat gac tgt atg cac aac 2797
   Val Phe His Ser Ser Arg Gln Asp Gly Leu Asn Asp Cys Met His Asn
   895 900 905
   aac ggg cag tgt ggg cag ctg tgc ctt gcc atc ccc ggc ggc cac cgc 2845
   Asn Gly Gln Cys Gly Gln Leu Cys Leu Ala Ile Pro Gly Gly His Arg
   910 915 920 925
   tgc ggc tgc gcc tca cac tac acc ctg gac ccc agc agc cgc aac tgc 2893
   Cys Gly Cys Ala Ser His Tyr Thr Leu Asp Pro Ser Ser Arg Asn Cys
   930 935 940
   agc ccg ccc acc acc ttc ttg ctg ttc agc cag aaa tct gcc atc agt 2941
   Ser Pro Pro Thr Thr Phe Leu Leu Phe Ser Gln Lys Ser Ala Ile Ser
   945 950 955
   cgg atg atc ccg gac gac cag cac agc ccg gat ctc atc ctg ccc ctg 2989
   Arg Met Ile Pro Asp Asp Gln His Ser Pro Asp Leu Ile Leu Pro Leu
   960 965 970
   cat gga ctg agg aac gtc aaa gcc atc gac tat gac cca ctg gac aag 3037
   His Gly Leu Arg Asn Val Lys Ala Ile Asp Tyr Asp Pro Leu Asp Lys
   975 980 985
   ttc atc tac tgg gtg gat ggg cgc cag aac atc aag cga gcc aag gac 3085
   Phe Ile Tyr Trp Val Asp Gly Arg Gln Asn Ile Lys Arg Ala Lys Asp
   990 995 1000 1005
   gac ggg acc cag ccc ttt gtt ttg acc tct ctg agc caa ggc caa aac 3133
   Asp Gly Thr Gln Pro Phe Val Leu Thr Ser Leu Ser Gln Gly Gln Asn
   1010 1015 1020
   cca gac agg cag ccc cac gac ctc agc atc gac atc tac agc cgg aca 3181
   Pro Asp Arg Gln Pro His Asp Leu Ser Ile Asp Ile Tyr Ser Arg Thr
   1025 1030 1035
   ctg ttc tgg acg tgc gag gcc acc aat acc atc aac gtc cac agg ctg 3229
   Leu Phe Trp Thr Cys Glu Ala Thr Asn Thr Ile Asn Val His Arg Leu
   1040 1045 1050
   agc ggg gaa gcc atg ggg gtg gtg ctg cgt ggg gac cgc gac aag ccc 3277
   Ser Gly Glu Ala Met Gly Val Val Leu Arg Gly Asp Arg Asp Lys Pro
   1055 1060 1065
   agg gcc atc gtc gtc aac gcg gag cga ggg tac ctg tac ttc acc aac 3325
   Arg Ala Ile Val Val Asn Ala Glu Arg Gly Tyr Leu Tyr Phe Thr Asn
   1070 1075 1080 1085
   atg cag gac cgg gca gcc aag atc gaa cgc gca gcc ctg gac ggc acc 3373
   Met Gln Asp Arg Ala Ala Lys Ile Glu Arg Ala Ala Leu Asp Gly Thr
   1090 1095 1100
   gag cgc gag gtc ctc ttc acc acc ggc ctc atc cgc cct gtg gcc ctg 3421
   Glu Arg Glu Val Leu Phe Thr Thr Gly Leu Ile Arg Pro Val Ala Leu
   1105 1110 1115
   gtg gtg gac aac aca ctg ggc aag ctg ttc tgg gtg gac gcg gac ctg 3469
   Val Val Asp Asn Thr Leu Gly Lys Leu Phe Trp Val Asp Ala Asp Leu
   1120 1125 1130
   aag cgc att gag agc tgt gac ctg tca ggg gcc aac cgc ctg acc ctg 3517
   Lys Arg Ile Glu Ser Cys Asp Leu Ser Gly Ala Asn Arg Leu Thr Leu
   1135 1140 1145
   gag gac gcc aac atc gtg cag cct ctg ggc ctg acc atc ctt ggc aag 3565
   Glu Asp Ala Asn Ile Val Gln Pro Leu Gly Leu Thr Ile Leu Gly Lys
   1150 1155 1160 1165
   cat ctc tac tgg atc gac cgc cag cag cag atg atc gag cgt gtg gag 3613
   His Leu Tyr Trp Ile Asp Arg Gln Gln Gln Met Ile Glu Arg Val Glu
   1170 1175 1180
   aag acc acc ggg gac aag cgg act cgc atc cag ggc cgt gtc gcc cac 3661
   Lys Thr Thr Gly Asp Lys Arg Thr Arg Ile Gln Gly Arg Val Ala His
   1185 1190 1195
   ctc act ggc atc cat gca gtg gag gaa gtc agc ctg gag gag ttc tca 3709
   Leu Thr Gly Ile His Ala Val Glu Glu Val Ser Leu Glu Glu Phe Ser
   1200 1205 1210
   gcc cac cca tgt gcc cgt gac aat ggt ggc tgc tcc cac atc tgt att 3757
   Ala His Pro Cys Ala Arg Asp Asn Gly Gly Cys Ser His Ile Cys Ile
   1215 1220 1225
   gcc aag ggt gat ggg aca cca cgg tgc tca tgc cca gtc cac ctc gtg 3805
   Ala Lys Gly Asp Gly Thr Pro Arg Cys Ser Cys Pro Val His Leu Val
   1230 1235 1240 1245
   ctc ctg cag aac ctg ctg acc tgt gga gag ccg ccc acc tgc tcc ccg 3853
   Leu Leu Gln Asn Leu Leu Thr Cys Gly Glu Pro Pro Thr Cys Ser Pro
   1250 1255 1260
   gac cag ttt gca tgt gcc aca ggg gag atc gac tgt atc ccc ggg gcc 3901
   Asp Gln Phe Ala Cys Ala Thr Gly Glu Ile Asp Cys Ile Pro Gly Ala
   1265 1270 1275
   tgg cgc tgt gac ggc ttt ccc gag tgc gat gac cag agc gac gag gag 3949
   Trp Arg Cys Asp Gly Phe Pro Glu Cys Asp Asp Gln Ser Asp Glu Glu
   1280 1285 1290
   ggc tgc ccc gtg tgc tcc gcc gcc cag ttc ccc tgc gcg cgg ggt cag 3997
   Gly Cys Pro Val Cys Ser Ala Ala Gln Phe Pro Cys Ala Arg Gly Gln
   1295 1300 1305
   tgt gtg gac ctg cgc ctg cgc tgc gac ggc gag gca gac tgt cag gac 4045
   Cys Val Asp Leu Arg Leu Arg Cys Asp Gly Glu Ala Asp Cys Gln Asp
   1310 1315 1320 1325
   cgc tca gac gag gtg gac tgt gac gcc atc tgc ctg ccc aac cag ttc 4093
   Arg Ser Asp Glu Val Asp Cys Asp Ala Ile Cys Leu Pro Asn Gln Phe
   1330 1335 1340
   cgg tgt gcg agc ggc cag tgt gtc ctc atc aaa cag cag tgc gac tcc 4141
   Arg Cys Ala Ser Gly Gln Cys Val Leu Ile Lys Gln Gln Cys Asp Ser
   1345 1350 1355
   ttc ccc gac tgt atc gac ggc tcc gac gag ctc atg tgt gaa atc acc 4189
   Phe Pro Asp Cys Ile Asp Gly Ser Asp Glu Leu Met Cys Glu Ile Thr
   1360 1365 1370
   aag ccg ccc tca gac gac agc ccg gcc cac agc agt gcc atc ggg ccc 4237
   Lys Pro Pro Ser Asp Asp Ser Pro Ala His Ser Ser Ala Ile Gly Pro
   1375 1380 1385
   gtc att ggc atc atc ctc tct ctc ttc gtc atg ggt ggt gtc tat ttt 4285
   Val Ile Gly Ile Ile Leu Ser Leu Phe Val Met Gly Gly Val Tyr Phe
   1390 1395 1400 1405
   gtg tgc cag cgc gtg gtg tgc cag cgc tat gcg ggg gcc aac ggg ccc 4333
   Val Cys Gln Arg Val Val Cys Gln Arg Tyr Ala Gly Ala Asn Gly Pro
   1410 1415 1420
   ttc ccg cac gag tat gtc agc ggg acc ccg cac gtg ccc ctc aat ttc 4381
   Phe Pro His Glu Tyr Val Ser Gly Thr Pro His Val Pro Leu Asn Phe
   1425 1430 1435
   ata gcc ccg ggc ggt tcc cag cat ggc ccc ttc aca ggc atc gca tgc 4429
   Ile Ala Pro Gly Gly Ser Gln His Gly Pro Phe Thr Gly Ile Ala Cys
   1440 1445 1450
   gga aag tcc atg atg agc tcc gtg agc ctg atg ggg ggc cgg ggc ggg 4477
   Gly Lys Ser Met Met Ser Ser Val Ser Leu Met Gly Gly Arg Gly Gly
   1455 1460 1465
   gtg ccc ctc tac gac cgg aac cac gtc aca ggg gcc tcg tcc agc agc 4525
   Val Pro Leu Tyr Asp Arg Asn His Val Thr Gly Ala Ser Ser Ser Ser
   1470 1475 1480 1485
   tcg tcc agc acg aag gcc acg ctg tac ccg ccg atc ctg aac ccg ccg 4573
   Ser Ser Ser Thr Lys Ala Thr Leu Tyr Pro Pro Ile Leu Asn Pro Pro
   1490 1495 1500
   ccc tcc ccg gcc acg gac ccc tcc ctg tac aac atg gac atg ttc tac 4621
   Pro Ser Pro Ala Thr Asp Pro Ser Leu Tyr Asn Met Asp Met Phe Tyr
   1505 1510 1515
   tct tca aac att ccg gcc act gcg aga ccg tac agg ccc tac atc att 4669
   Ser Ser Asn Ile Pro Ala Thr Ala Arg Pro Tyr Arg Pro Tyr Ile Ile
   1520 1525 1530
   cga gga atg gcg ccc ccg acg acg ccc tgc agc acc gac gtg tgt gac 4717
   Arg Gly Met Ala Pro Pro Thr Thr Pro Cys Ser Thr Asp Val Cys Asp
   1535 1540 1545
   agc gac tac agc gcc agc cgc tgg aag gcc agc aag tac tac ctg gat 4765
   Ser Asp Tyr Ser Ala Ser Arg Trp Lys Ala Ser Lys Tyr Tyr Leu Asp
   1550 1555 1560 1565
   ttg aac tcg gac tca gac ccc tat cca ccc cca ccc acg ccc cac agc 4813
   Leu Asn Ser Asp Ser Asp Pro Tyr Pro Pro Pro Pro Thr Pro His Ser
   1570 1575 1580
   cag tac ctg tcg gcg gag gac agc tgc ccg ccc tcg ccc gcc acc gag 4861
   Gln Tyr Leu Ser Ala Glu Asp Ser Cys Pro Pro Ser Pro Ala Thr Glu
   1585 1590 1595
   agg agc tac ttc cat ctc ttc ccg ccc cct ccg tcc ccc tgc acg gac 4909
   Arg Ser Tyr Phe His Leu Phe Pro Pro Pro Pro Ser Pro Cys Thr Asp
   1600 1605 1610
   tca tcc tgacctcggc cgggccactc tggcttctct gtgcccctgt aaatagtttt 4965
   Ser Ser
   1615
   aaatatgaac aaagaaaaaa atatatttta tgatttaaaa aataaatata attgggattt 5025
   taaaaacatg agaaatgtga actgtgatgg ggtgggcagg gctgggagaa ctttgtacag 5085
   tggagaaata tttataaact taattttgta aaaca 5120
<210> 3
   <211> 1615
   <212> PRT
   <213> Homo sapiens
<400> 3
   Met Glu Ala Ala Pro Pro Gly Pro Pro Trp Pro Leu Leu Leu Leu Leu
   1 5 10 15
   Leu Leu Leu Leu Ala Leu Cys Gly Cys Pro Ala Pro Ala Ala Ala Ser
   20 25 30
   Pro Leu Leu Leu Phe Ala Asn Arg Arg Asp Val Arg Leu Val Asp Ala
   35 40 45
   Gly Gly Val Lys Leu Glu Ser Thr Ile Val Val Ser Gly Leu Glu Asp
   50 55 60
   Ala Ala Ala Val Asp Phe Gln Phe Ser Lys Gly Ala Val Tyr Trp Thr
   65 70 75 80
   Asp Val Ser Glu Glu Ala Ile Lys Gln Thr Tyr Leu Asn Gln Thr Gly
   85 90 95
   Ala Ala Val Gln Asn Val Val Ile Ser Gly Leu Val Ser Pro Asp Gly
   100 105 110
   Leu Ala Cys Asp Trp Val Gly Lys Lys Leu Tyr Trp Thr Asp Ser Glu
   115 120 125
   Thr Asn Arg Ile Glu Val Ala Asn Leu Asn Gly Thr Ser Arg Lys Val
   130 135 140
   Leu Phe Trp Gln Asp Leu Asp Gln Pro Lys Ala Ile Ala Leu Asp Pro
   145 150 155 160
   Ala His Gly Tyr Met Tyr Trp Thr Asp Trp Gly Glu Thr Pro Arg Ile
   165 170 175
   Glu Arg Ala Gly Met Asp Gly Ser Thr Arg Lys Ile Ile Val Asp Ser
   180 185 190
   Asp Ile Tyr Trp Pro Asn Gly Leu Thr Ile Asp Leu Glu Glu Gln Lys
   195 200 205
   Leu Tyr Trp Ala Asp Ala Lys Leu Ser Phe Ile His Arg Ala Asn Leu
   210 215 220
   Asp Gly Ser Phe Arg Gln Lys Val Val Glu Gly Ser Leu Thr His Pro
   225 230 235 240
   Phe Ala Leu Thr Leu Ser Gly Asp Thr Leu Tyr Trp Thr Asp Trp Gln
   245 250 255
   Thr Arg Ser Ile His Ala Cys Asn Lys Arg Thr Gly Gly Lys Arg Lys
   260 265 270
   Glu Ile Leu Ser Ala Leu Tyr Ser Pro Met Asp Ile Gln Val Leu Ser
   275 280 285
   Gln Glu Arg Gln Pro Phe Phe His Thr Arg Cys Glu Glu Asp Asn Gly
   290 295 300
   Gly Trp Ser His Leu Cys Leu Leu Ser Pro Ser Glu Pro Phe Tyr Thr
   305 310 315 320
   Cys Ala Cys Pro Thr Gly Val Gln Met Gln Asp Asn Gly Arg Thr Cys
   325 330 335
   Lys Ala Gly Ala Glu Glu Val Leu Leu Leu Ala Arg Arg Thr Asp Leu
   340 345 350
   Arg Arg Ile Ser Leu Asp Thr Pro Asp Phe Thr Asp Ile Val Leu Gln
   355 360 365
   Val Asp Asp Ile Arg His Ala Ile Ala Ile Asp Tyr Asp Pro Leu Glu
   370 375 380
   Gly Tyr Val Tyr Trp Thr Asp Asp Glu Val Arg Ala Ile Arg Arg Ala
   385 390 395 400
   Tyr Leu Asp Gly Ser Gly Ala Gln Thr Leu Val Asn Thr Glu Ile Asn
   405 410 415
   Asp Pro Asp Gly Ile Ala Val Asp Trp Val Ala Arg Asn Leu Tyr Trp
   420 425 430
   Thr Asp Thr Gly Thr Asp Arg Ile Glu Val Thr Arg Leu Asn Gly Thr
   435 440 445
   Ser Arg Lys Ile Leu Val Ser Glu Asp Leu Asp Glu Pro Arg Ala Ile
   450 455 460
   Ala Leu His Pro Val Met Gly Leu Met Tyr Trp Thr Asp Trp Gly Glu
   465 470 475 480
   Asn Pro Lys Ile Glu Cys Ala Asn Leu Asp Gly Gln Glu Arg Arg Val
   485 490 495
   Leu Val Asn Ala Ser Leu Gly Trp Pro Asn Gly Leu Ala Leu Asp Leu
   500 505 510
   Gln Glu Gly Lys Leu Tyr Trp Gly Asp Ala Lys Thr Asp Lys Ile Glu
   515 520 525
   Val Ile Asn Val Asp Gly Thr Lys Arg Arg Thr Leu Leu Glu Asp Lys
   530 535 540
   Leu Pro His Ile Phe Gly Phe Thr Leu Leu Gly Asp Phe Ile Tyr Trp
   545 550 555 560
   Thr Asp Trp Gln Arg Arg Ser Ile Glu Arg Val His Lys Val Lys Ala
   565 570 575
   Ser Arg Asp Val Ile Ile Asp Gln Leu Pro Asp Leu Met Gly Leu Lys
   580 585 590
   Ala Val Asn Val Ala Lys Val Val Gly Thr Asn Pro Cys Ala Asp Arg
   595 600 605
   Asn Gly Gly Cys Ser His Leu Cys Phe Phe Thr Pro His Ala Thr Arg
   610 615 620
   Cys Gly Cys Pro Ile Gly Leu Glu Leu Leu Ser Asp Met Lys Thr Cys
   625 630 635 640
   Ile Val Pro Glu Ala Phe Leu Val Phe Thr Ser Arg Ala Ala Ile His
   645 650 655
   Arg Ile Ser Leu Glu Thr Asn Asn Asn Asp Val Ala Ile Pro Leu Thr
   660 665 670
   Gly Val Lys Glu Ala Ser Ala Leu Asp Phe Asp Val Ser Asn Asn His
   675 680 685
   Ile Tyr Trp Thr Asp Val Ser Leu Lys Asn Ile Ser Arg Ala Phe Met
   690 695 700
   Asn Gly Ser Ser Val Glu His Val Val Glu Phe Gly Leu Asp Tyr Pro
   705 710 715 720
   Glu Gly Met Ala Val Asp Trp Met Gly Lys Asn Leu Tyr Trp Ala Asp
   725 730 735
   Thr Gly Thr Asn Arg Ile Glu Val Ala Arg Leu Asp Gly Gln Phe Arg
   740 745 750
   Gln Val Leu Val Trp Arg Asp Leu Asp Asn Pro Arg Ser Leu Ala Leu
   755 760 765
   Asp Pro Thr Lys Gly Tyr Ile Tyr Trp Thr Glu Trp Gly Gly Lys Pro
   770 775 780
   Arg Ile Val Arg Ala Phe Met Asp Gly Thr Asn Cys Met Thr Leu Val
   785 790 795 800
   Asp Lys Val Gly Arg Ala Asn Asp Leu Thr Ile Asp Tyr Ala Asp Gln
   805 810 815
   Arg Leu Tyr Trp Thr Asp Leu Asp Thr Asn Met Ile Glu Ser Ser Asn
   820 825 830
   Met Leu Gly Gln Glu Arg Val Val Ile Ala Asp Asp Leu Pro His Pro
   835 840 845
   Phe Gly Leu Thr Gln Tyr Ser Asp Tyr Ile Tyr Trp Thr Asp Trp Asn
   850 855 860
   Leu His Ser Ile Glu Arg Ala Asp Lys Thr Ser Gly Arg Asn Arg Thr
   865 870 875 880
   Leu Ile Gln Gly His Leu Asp Phe Val Met Asp Ile Leu Val Phe His
   885 890 895
   Ser Ser Arg Gln Asp Gly Leu Asn Asp Cys Met His Asn Asn Gly Gln
   900 905 910
   Cys Gly Gln Leu Cys Leu Ala Ile Pro Gly Gly His Arg Cys Gly Cys
   915 920 925
   Ala Ser His Tyr Thr Leu Asp Pro Ser Ser Arg Asn Cys Ser Pro Pro
   930 935 940
   Thr Thr Phe Leu Leu Phe Ser Gln Lys Ser Ala Ile Ser Arg Met Ile
   945 950 955 960
   Pro Asp Asp Gln His Ser Pro Asp Leu Ile Leu Pro Leu His Gly Leu
   965 970 975
   Arg Asn Val Lys Ala Ile Asp Tyr Asp Pro Leu Asp Lys Phe Ile Tyr
   980 985 990
   Trp Val Asp Gly Arg Gln Asn Ile Lys Arg Ala Lys Asp Asp Gly Thr
   995 1000 1005
   Gln Pro Phe Val Leu Thr Ser Leu Ser Gln Gly Gln Asn Pro Asp Arg
   1010 1015 1020
   Gln Pro His Asp Leu Ser Ile Asp Ile Tyr Ser Arg Thr Leu Phe Trp
   1025 1030 1035 1040
   Thr Cys Glu Ala Thr Asn Thr Ile Asn Val His Arg Leu Ser Gly Glu
   1045 1050 1055
   Ala Met Gly Val Val Leu Arg Gly Asp Arg Asp Lys Pro Arg Ala Ile
   1060 1065 1070
   Val Val Asn Ala Glu Arg Gly Tyr Leu Tyr Phe Thr Asn Met Gln Asp
   1075 1080 1085
   Arg Ala Ala Lys Ile Glu Arg Ala Ala Leu Asp Gly Thr Glu Arg Glu
   1090 1095 1100
   Val Leu Phe Thr Thr Gly Leu Ile Arg Pro Val Ala Leu Val Val Asp
   1105 1110 1115 1120
   Asn Thr Leu Gly Lys Leu Phe Trp Val Asp Ala Asp Leu Lys Arg Ile
   1125 1130 1135
   Glu Ser Cys Asp Leu Ser Gly Ala Asn Arg Leu Thr Leu Glu Asp Ala
   1140 1145 1150
   Asn Ile Val Gln Pro Leu Gly Leu Thr Ile Leu Gly Lys His Leu Tyr
   1155 1160 1165
   Trp Ile Asp Arg Gln Gln Gln Met Ile Glu Arg Val Glu Lys Thr Thr
   1170 1175 1180
   Gly Asp Lys Arg Thr Arg Ile Gln Gly Arg Val Ala His Leu Thr Gly
   1185 1190 1195 1200
   Ile His Ala Val Glu Glu Val Ser Leu Glu Glu Phe Ser Ala His Pro
   1205 1210 1215
   Cys Ala Arg Asp Asn Gly Gly Cys Ser His Ile Cys Ile Ala Lys Gly
   1220 1225 1230
   Asp Gly Thr Pro Arg Cys Ser Cys Pro Val His Leu Val Leu Leu Gln
   1235 1240 1245
   Asn Leu Leu Thr Cys Gly Glu Pro Pro Thr Cys Ser Pro Asp Gln Phe
   1250 1255 1260
   Ala Cys Ala Thr Gly Glu Ile Asp Cys Ile Pro Gly Ala Trp Arg Cys
   1265 1270 1275 1280
   Asp Gly Phe Pro Glu Cys Asp Asp Gln Ser Asp Glu Glu Gly Cys Pro
   1285 1290 1295
   Val Cys Ser Ala Ala Gln Phe Pro Cys Ala Arg Gly Gln Cys Val Asp
   1300 1305 1310
   Leu Arg Leu Arg Cys Asp Gly Glu Ala Asp Cys Gln Asp Arg Ser Asp
   1315 1320 1325
   Glu Val Asp Cys Asp Ala Ile Cys Leu Pro Asn Gln Phe Arg Cys Ala
   1330 1335 1340
   Ser Gly Gln Cys Val Leu Ile Lys Gln Gln Cys Asp Ser Phe Pro Asp
   1345 1350 1355 1360
   Cys Ile Asp Gly Ser Asp Glu Leu Met Cys Glu Ile Thr Lys Pro Pro
   1365 1370 1375
   Ser Asp Asp Ser Pro Ala His Ser Ser Ala Ile Gly Pro Val Ile Gly
   1380 1385 1390
   Ile Ile Leu Ser Leu Phe Val Met Gly Gly Val Tyr Phe Val Cys Gln
   1395 1400 1405
   Arg Val Val Cys Gln Arg Tyr Ala Gly Ala Asn Gly Pro Phe Pro His
   1410 1415 1420
   Glu Tyr Val Ser Gly Thr Pro His Val Pro Leu Asn Phe Ile Ala Pro
   1425 1430 1435 1440
   Gly Gly Ser Gln His Gly Pro Phe Thr Gly Ile Ala Cys Gly Lys Ser
   1445 1450 1455
   Met Met Ser Ser Val Ser Leu Met Gly Gly Arg Gly Gly Val Pro Leu
   1460 1465 1470
   Tyr Asp Arg Asn His Val Thr Gly Ala Ser Ser Ser Ser Ser Ser Ser
   1475 1480 1485
   Thr Lys Ala Thr Leu Tyr Pro Pro Ile Leu Asn Pro Pro Pro Ser Pro
   1490 1495 1500
   Ala Thr Asp Pro Ser Leu Tyr Asn Met Asp Met Phe Tyr Ser Ser Asn
   1505 1510 1515 1520
   Ile Pro Ala Thr Ala Arg Pro Tyr Arg Pro Tyr Ile Ile Arg Gly Met
   1525 1530 1535
   Ala Pro Pro Thr Thr Pro Cys Ser Thr Asp Val Cys Asp Ser Asp Tyr
   1540 1545 1550
   Ser Ala Ser Arg Trp Lys Ala Ser Lys Tyr Tyr Leu Asp Leu Asn Ser
   1555 1560 1565
   Asp Ser Asp Pro Tyr Pro Pro Pro Pro Thr Pro His Ser Gln Tyr Leu
   1570 1575 1580
   Ser Ala Glu Asp Ser Cys Pro Pro Ser Pro Ala Thr Glu Arg Ser Tyr
   1585 1590 1595 1600
   Phe His Leu Phe Pro Pro Pro Pro Ser Pro Cys Thr Asp Ser Ser
   1605 1610 1615
<210> 4
   <211> 1615
   <212> PRT
   <213> Homo sapiens
<400> 4
   Met Glu Ala Ala Pro Pro Gly Pro Pro Trp Pro Leu Leu Leu Leu Leu
   1 5 10 15
   Leu Leu Leu Leu Ala Leu Cys Gly Cys Pro Ala Pro Ala Ala Ala Ser
   20 25 30
   Pro Leu Leu Leu Phe Ala Asn Arg Arg Asp Val Arg Leu Val Asp Ala
   35 40 45
   Gly Gly Val Lys Leu Glu Ser Thr Ile Val Val Ser Gly Leu Glu Asp
   50 55 60
   Ala Ala Ala Val Asp Phe Gln Phe Ser Lys Gly Ala Val Tyr Trp Thr
   65 70 75 80
   Asp Val Ser Glu Glu Ala Ile Lys Gln Thr Tyr Leu Asn Gln Thr Gly
   85 90 95
   Ala Ala Val Gln Asn Val Val Ile Ser Gly Leu Val Ser Pro Asp Gly
   100 105 110
   Leu Ala Cys Asp Trp Val Gly Lys Lys Leu Tyr Trp Thr Asp Ser Glu
   115 120 125
   Thr Asn Arg Ile Glu Val Ala Asn Leu Asn Gly Thr Ser Arg Lys Val
   130 135 140
   Leu Phe Trp Gln Asp Leu Asp Gln Pro Lys Ala Ile Ala Leu Asp Pro
   145 150 155 160
   Ala His Gly Tyr Met Tyr Trp Thr Asp Trp Val Glu Thr Pro Arg Ile
   165 170 175
   Glu Arg Ala Gly Met Asp Gly Ser Thr Arg Lys Ile Ile Val Asp Ser
   180 185 190
   Asp Ile Tyr Trp Pro Asn Gly Leu Thr Ile Asp Leu Glu Glu Gln Lys
   195 200 205
   Leu Tyr Trp Ala Asp Ala Lys Leu Ser Phe Ile His Arg Ala Asn Leu
   210 215 220
   Asp Gly Ser Phe Arg Gln Lys Val Val Glu Gly Ser Leu Thr His Pro
   225 230 235 240
   Phe Ala Leu Thr Leu Ser Gly Asp Thr Leu Tyr Trp Thr Asp Trp Gln
   245 250 255
   Thr Arg Ser Ile His Ala Cys Asn Lys Arg Thr Gly Gly Lys Arg Lys
   260 265 270
   Glu Ile Leu Ser Ala Leu Tyr Ser Pro Met Asp Ile Gln Val Leu Ser
   275 280 285
   Gln Glu Arg Gln Pro Phe Phe His Thr Arg Cys Glu Glu Asp Asn Gly
   290 295 300
   Gly Trp Ser His Leu Cys Leu Leu Ser Pro Ser Glu Pro Phe Tyr Thr
   305 310 315 320
   Cys Ala Cys Pro Thr Gly Val Gln Met Gln Asp Asn Gly Arg Thr Cys
   325 330 335
   Lys Ala Gly Ala Glu Glu Val Leu Leu Leu Ala Arg Arg Thr Asp Leu
   340 345 350
   Arg Arg Ile Ser Leu Asp Thr Pro Asp Phe Thr Asp Ile Val Leu Gln
   355 360 365
   Val Asp Asp Ile Arg His Ala Ile Ala Ile Asp Tyr Asp Pro Leu Glu
   370 375 380
   Gly Tyr Val Tyr Trp Thr Asp Asp Glu Val Arg Ala Ile Arg Arg Ala
   385 390 395 400
   Tyr Leu Asp Gly Ser Gly Ala Gln Thr Leu Val Asn Thr Glu Ile Asn
   405 410 415
   Asp Pro Asp Gly Ile Ala Val Asp Trp Val Ala Arg Asn Leu Tyr Trp
   420 425 430
   Thr Asp Thr Gly Thr Asp Arg Ile Glu Val Thr Arg Leu Asn Gly Thr
   435 440 445
   Ser Arg Lys Ile Leu Val Ser Glu Asp Leu Asp Glu Pro Arg Ala Ile
   450 455 460
   Ala Leu His Pro Val Met Gly Leu Met Tyr Trp Thr Asp Trp Gly Glu
   465 470 475 480
   Asn Pro Lys Ile Glu Cys Ala Asn Leu Asp Gly Gln Glu Arg Arg Val
   485 490 495
   Leu Val Asn Ala Ser Leu Gly Trp Pro Asn Gly Leu Ala Leu Asp Leu
   500 505 510
   Gln Glu Gly Lys Leu Tyr Trp Gly Asp Ala Lys Thr Asp Lys Ile Glu
   515 520 525
   Val Ile Asn Val Asp Gly Thr Lys Arg Arg Thr Leu Leu Glu Asp Lys
   530 535 540
   Leu Pro His Ile Phe Gly Phe Thr Leu Leu Gly Asp Phe Ile Tyr Trp
   545 550 555 560
   Thr Asp Trp Gln Arg Arg Ser Ile Glu Arg Val His Lys Val Lys Ala
   565 570 575
   Ser Arg Asp Val Ile Ile Asp Gln Leu Pro Asp Leu Met Gly Leu Lys
   580 585 590
   Ala Val Asn Val Ala Lys Val Val Gly Thr Asn Pro Cys Ala Asp Arg
   595 600 605
   Asn Gly Gly Cys Ser His Leu Cys Phe Phe Thr Pro His Ala Thr Arg
   610 615 620
   Cys Gly Cys Pro Ile Gly Leu Glu Leu Leu Ser Asp Met Lys Thr Cys
   625 630 635 640
   Ile Val Pro Glu Ala Phe Leu Val Phe Thr Ser Arg Ala Ala Ile His
   645 650 655
   Arg Ile Ser Leu Glu Thr Asn Asn Asn Asp Val Ala Ile Pro Leu Thr
   660 665 670
   Gly Val Lys Glu Ala Ser Ala Leu Asp Phe Asp Val Ser Asn Asn His
   675 680 685
   Ile Tyr Trp Thr Asp Val Ser Leu Lys Asn Ile Ser Arg Ala Phe Met
   690 695 700
   Asn Gly Ser Ser Val Glu His Val Val Glu Phe Gly Leu Asp Tyr Pro
   705 710 715 720
   Glu Gly Met Ala Val Asp Trp Met Gly Lys Asn Leu Tyr Trp Ala Asp
   725 730 735
   Thr Gly Thr Asn Arg Ile Glu Val Ala Arg Leu Asp Gly Gln Phe Arg
   740 745 750
   Gln Val Leu Val Trp Arg Asp Leu Asp Asn Pro Arg Ser Leu Ala Leu
   755 760 765
   Asp Pro Thr Lys Gly Tyr Ile Tyr Trp Thr Glu Trp Gly Gly Lys Pro
   770 775 780
   Arg Ile Val Arg Ala Phe Met Asp Gly Thr Asn Cys Met Thr Leu Val
   785 790 795 800
   Asp Lys Val Gly Arg Ala Asn Asp Leu Thr Ile Asp Tyr Ala Asp Gln
   805 810 815
   Arg Leu Tyr Trp Thr Asp Leu Asp Thr Asn Met Ile Glu Ser Ser Asn
   820 825 830
   Met Leu Gly Gln Glu Arg Val Val Ile Ala Asp Asp Leu Pro His Pro
   835 840 845
   Phe Gly Leu Thr Gln Tyr Ser Asp Tyr Ile Tyr Trp Thr Asp Trp Asn
   850 855 860
   Leu His Ser Ile Glu Arg Ala Asp Lys Thr Ser Gly Arg Asn Arg Thr
   865 870 875 880
   Leu Ile Gln Gly His Leu Asp Phe Val Met Asp Ile Leu Val Phe His
   885 890 895
   Ser Ser Arg Gln Asp Gly Leu Asn Asp Cys Met His Asn Asn Gly Gln
   900 905 910
   Cys Gly Gln Leu Cys Leu Ala Ile Pro Gly Gly His Arg Cys Gly Cys
   915 920 925
   Ala Ser His Tyr Thr Leu Asp Pro Ser Ser Arg Asn Cys Ser Pro Pro
   930 935 940
   Thr Thr Phe Leu Leu Phe Ser Gln Lys Ser Ala Ile Ser Arg Met Ile
   945 950 955 960
   Pro Asp Asp Gln His Ser Pro Asp Leu Ile Leu Pro Leu His Gly Leu
   965 970 975
   Arg Asn Val Lys Ala Ile Asp Tyr Asp Pro Leu Asp Lys Phe Ile Tyr
   980 985 990
   Trp Val Asp Gly Arg Gln Asn Ile Lys Arg Ala Lys Asp Asp Gly Thr
   995 1000 1005
   Gln Pro Phe Val Leu Thr Ser Leu Ser Gln Gly Gln Asn Pro Asp Arg
   1010 1015 1020
   Gln Pro His Asp Leu Ser Ile Asp Ile Tyr Ser Arg Thr Leu Phe Trp
   1025 1030 1035 1040
   Thr Cys Glu Ala Thr Asn Thr Ile Asn Val His Arg Leu Ser Gly Glu
   1045 1050 1055
   Ala Met Gly Val Val Leu Arg Gly Asp Arg Asp Lys Pro Arg Ala Ile
   1060 1065 1070
   Val Val Asn Ala Glu Arg Gly Tyr Leu Tyr Phe Thr Asn Met Gln Asp
   1075 1080 1085
   Arg Ala Ala Lys Ile Glu Arg Ala Ala Leu Asp Gly Thr Glu Arg Glu
   1090 1095 1100
   Val Leu Phe Thr Thr Gly Leu Ile Arg Pro Val Ala Leu Val Val Asp
   1105 1110 1115 1120
   Asn Thr Leu Gly Lys Leu Phe Trp Val Asp Ala Asp Leu Lys Arg Ile
   1125 1130 1135
   Glu Ser Cys Asp Leu Ser Gly Ala Asn Arg Leu Thr Leu Glu Asp Ala
   1140 1145 1150
   Asn Ile Val Gln Pro Leu Gly Leu Thr Ile Leu Gly Lys His Leu Tyr
   1155 1160 1165
   Trp Ile Asp Arg Gln Gln Gln Met Ile Glu Arg Val Glu Lys Thr Thr
   1170 1175 1180
   Gly Asp Lys Arg Thr Arg Ile Gln Gly Arg Val Ala His Leu Thr Gly
   1185 1190 1195 1200
   Ile His Ala Val Glu Glu Val Ser Leu Glu Glu Phe Ser Ala His Pro
   1205 1210 1215
   Cys Ala Arg Asp Asn Gly Gly Cys Ser His Ile Cys Ile Ala Lys Gly
   1220 1225 1230
   Asp Gly Thr Pro Arg Cys Ser Cys Pro Val His Leu Val Leu Leu Gln
   1235 1240 1245
   Asn Leu Leu Thr Cys Gly Glu Pro Pro Thr Cys Ser Pro Asp Gln Phe
   1250 1255 1260
   Ala Cys Ala Thr Gly Glu Ile Asp Cys Ile Pro Gly Ala Trp Arg Cys
   1265 1270 1275 1280
   Asp Gly Phe Pro Glu Cys Asp Asp Gln Ser Asp Glu Glu Gly Cys Pro
   1285 1290 1295
   Val Cys Ser Ala Ala Gln Phe Pro Cys Ala Arg Gly Gln Cys Val Asp
   1300 1305 1310
   Leu Arg Leu Arg Cys Asp Gly Glu Ala Asp Cys Gln Asp Arg Ser Asp
   1315 1320 1325
   Glu Val Asp Cys Asp Ala Ile Cys Leu Pro Asn Gln Phe Arg Cys Ala
   1330 1335 1340
   Ser Gly Gln Cys Val Leu Ile Lys Gln Gln Cys Asp Ser Phe Pro Asp
   1345 1350 1355 1360
   Cys Ile Asp Gly Ser Asp Glu Leu Met Cys Glu Ile Thr Lys Pro Pro
   1365 1370 1375
   Ser Asp Asp Ser Pro Ala His Ser Ser Ala Ile Gly Pro Val Ile Gly
   1380 1385 1390
   Ile Ile Leu Ser Leu Phe Val Met Gly Gly Val Tyr Phe Val Cys Gln
   1395 1400 1405
   Arg Val Val Cys Gln Arg Tyr Ala Gly Ala Asn Gly Pro Phe Pro His
   1410 1415 1420
   Glu Tyr Val Ser Gly Thr Pro His Val Pro Leu Asn Phe Ile Ala Pro
   1425 1430 1435 1440
   Gly Gly Ser Gln His Gly Pro Phe Thr Gly Ile Ala Cys Gly Lys Ser
   1445 1450 1455
   Met Met Ser Ser Val Ser Leu Met Gly Gly Arg Gly Gly Val Pro Leu
   1460 1465 1470
   Tyr Asp Arg Asn His Val Thr Gly Ala Ser Ser Ser Ser Ser Ser Ser
   1475 1480 1485
   Thr Lys Ala Thr Leu Tyr Pro Pro Ile Leu Asn Pro Pro Pro Ser Pro
   1490 1495 1500
   Ala Thr Asp Pro Ser Leu Tyr Asn Met Asp Met Phe Tyr Ser Ser Asn
   1505 1510 1515 1520
   Ile Pro Ala Thr Ala Arg Pro Tyr Arg Pro Tyr Ile Ile Arg Gly Met
   1525 1530 1535
   Ala Pro Pro Thr Thr Pro Cys Ser Thr Asp Val Cys Asp Ser Asp Tyr
   1540 1545 1550
   Ser Ala Ser Arg Trp Lys Ala Ser Lys Tyr Tyr Leu Asp Leu Asn Ser
   1555 1560 1565
   Asp Ser Asp Pro Tyr Pro Pro Pro Pro Thr Pro His Ser Gln Tyr Leu
   1570 1575 1580
   Ser Ala Glu Asp Ser Cys Pro Pro Ser Pro Ala Thr Glu Arg Ser Tyr
   1585 1590 1595 1600
   Phe His Leu Phe Pro Pro Pro Pro Ser Pro Cys Thr Asp Ser Ser
   1605 1610 1615
<210> 5
   <211> 3096
   <212> DNA
   <213> Homo sapiens
<400> 5
   catcttctca cacgatctct cgcttcgcac tccttccttt gattggtttt caccatttac 60
   tcagacgacg gtccttcttc gatctttgca cattcttcta tcatctacta ccttcatacc 120
   cagctccgtc ccctaatatt catgcgcgga tggcccattc cgtggtgaaa attcccttct     180
   actctgctaa tctgctgttc tctctccctc ccgtcgggtt ctgctcctgc cacgttctcc     240
   cctctcccca ccaaaggctg ggttttcttt gtcagggctc ctttcccctt tggaagaagg     300
   ggggctgtat ggccttggtg cgaggccctc cagtgacagg atcccccatc acccagagtt     360
   ccacaggccc tggtagggag gagggggagc agaagaggag gtgccatctt tgcctgctgg      420
   ggaagggcag gggccaccca cacagagctc tcccatttgc tgtggaccct ggggccactg     480
   cccagttcct tccaaaggaa agccagctcc ccaggtggtg ggagagtgat atggcttcct     540
   cttaaactta gggaattgag tgtgtggttg cttctaagtg ccttagaagc cgggagcggc     600
   tcctggaaag agcctgcctg ccacagcggg ccttaccctg gctgtgccca cagatgtccc     660
   tggggcctgc cgctcctgcc cggctctcct ggcctccccc ggtgtgggtt gggaaaagca     720
   cagcaaatta aaaaacacct ccatctctgg cctttgaaga atgcatctga acagccgaga     780
   gtgtaaaccg tggtgaaatg tggtctttcc agtttgggga gaagcagggc agagctgggg     840
   cttttgtacc cagggtttcc aagagctcct gcctccctcg gctgggctgg ccagggcccc     900
   ccgctgggac ctccagctgt aatagggaag gttttactgg gttgctggcc actgtggact     960
   gcccctaagg gcaggtatgc ctgcctttac ccgggttccc ctcctgcctg gaagatacag     1020
   cccatgggag gcctgttgtc tgtgggatcc tccagcatca gagacactgg ggccagcgtc     1080
   tgcctggtga ggtgcaggcc tggcaggccc ggtcccccac ctgcttgagc acccacggtg     1140
   gtgggggctc gctgcctccc gagacaatct atgtcattgt tgtccaagga agctaattta     1200
   gagtagaaag ttccgtgtcc agtcccactc tgtgcgtgtg ttagcagggg actctcgggc     1260
   cggagctggg tccaccctgg tagggggact tcatggggcc tgggcgacag cactgtgtat      1320
   ttgtgtgtgt gtgtgtttgt gtgtgtgtgt gtctgaggag gtggaccagt ttctcaaaag     1380
   gcctgtgacc ccaagaacca aggaatttca gcctgggtgg atcacacctt cactggtgag     1440
   tgggacaagc tgggggccct cgccacagga gcagccaggg catggggcac agttggcctc     1500
   attcacaaaa tgggagtata agtgatccct gctctggcgg ccaggacgat gagtgggaac     1560
   acaccgtgtg ggggctgcct ggcctgggtg tgccgcgggt gtccttgttg gtgatggttc    1620
   cacctgcttg tgccaccagt gccctctggg tctcacacac aactctcttc ccagcgaagg     1680
   cccctcctgc cctcaggcct cagtgctgct tccgtctcgg aaggccccag gagctcctgc     1740
   atcctgggcg tgattcctgt gtgcctgcag accccctcgc ggctgccatc tcatcctttg 1800
   gtgcacctgt tggccagacc tcctggtagc gggtgctgca ctcccctgaa tgtgccgggg 1860
   cctgggggca gggacctggg ctcctccctc actgagtgga gggaactcag tgtcttggag 1920
   ttggggtgcc tgcaggctgg gtggtgcagg tgaaatgcag acctctcagc tggtgttcca 1980
   gagcagctgc cttcccccgc ccgagggact tcacccgcag cccagtcagg ggtggcgcct 2040
   gggtgcatcg cccgcaggct gggtaggggt ggagcctggg tggccctgcc tgtgagctgc 2100
   atagttgtcg cctttgaccc tgagttttct tcgttatctg tttggacctg tttggggcag 2160
   gcaggggatg agatctgaag ataaatgcct tagctgtgac catctccttt tgtgagaggt 2220
   caatgtccag ttccgctgca gttataacat cccatttttt gatttctttt tattttttcc 2280
   tttttctttt tgagatggag tctcgctctg tcacccaggc tggagtgcaa tggggtgacc 2340
   tcagctcact gcaacctcca cttctcgggt tcaagtgatt ctcctgcctc agcctcctga 2400
   ctagcagggg ttacaggcgt gagccaccac gcccagctaa tttttgtatt tttagtagag 2460
   gcaaggtttc gtcatgttgg ccaggctggt ctcaaactcc tggccttaag tgatctgccc 2520
   gcctcggcct cccaaagtgc tgagatgaca ggtgtgagcc accgtgcccg gcccagaact 2580
   ctttaattcc cacctgaaac ttgccgcctt aagcaggtcc ccagtctccc tcccctagtc 2640
   cctggtccca ccattctgct ttctgtctca atgaatttgc ctaccgtaag tacctcatat 2700
   aaattgaatc ataaagtatt tgtcttttta tatctggctt atttcactta gcataacatt 2760
   cttaagtttc atccatgttg tagcatgtgt cagaatctct ctcttttttt tttttttttt 2820
   tttttttttt ttttgcagac agagtctcgc tctgtcatct agactggagt tcagtggcac 2880
   gatctcggtt cactgcaaca tctgcctcct gggtccaagc aattctcctg cctcagcctc 2940
   cttagcagct ggaactacag gcgcgtgcca ccatgccttg ctaatttttg tatttttatg 3000
   tggaggcagg gtttcaccat cttggccagg ctggtctcga attcctggtc ttcaccacgg 3060
   gggcccgaag gacccgggca aagcgtggag gggagg 3096
<210> 6
   <211> 26928
   <212> DNA
   <213> Homo sapiens
<220>
   <221> unsure
   <222> (12044) , (12489), (26433), (26434) , (26435) , (26436) , (26439) , (26441)
   <223> Identity of nucleotide sequences at the above locations are unknown.
<400> 6
   gaagaccaag ggcacacagc gaggcagttt cagggcgggc agcctggggc cccacggggc 60
   ggccccggac acttgttctc acctgtggag ggcagagaag ggaacaggga gagaagtggc 120
   cggctgggag tggaggtggg tttgaggttt tactgtaaac taaatgtgta ccctctacct 180
   tagttatgaa ttatgagaca cgaagactgc gaaacagaca cactcctcta aaagtgcctc 240
   taggctgaca gggagaaagt cccgccaggc tcccagacgc cacctttgag tccttcaaca 300
   agcccgccag ggcctcttgc ccaccggtgt cagctcagcc actgaaccct ccaggaagaa 360
   gacgtgctgg taggagaaga atctcaccca ggcacagcct ggaaggggca cagaaggggc 420
   tccggaacca gcaagcccaa gttggaactc ccagtctgct actttctaga acgactgtgc 480
   ccttggcggg tctaagtaga acctctccgc gcactctttc ctcctttgta aagtggggac 540
   agcaatggcc accttgcagg ttcagagagg gcttgcagta cctcacagaa ctgagtgccc 600
   gtgaacgtgt gtgttcctcc agatttgtga cagctttgcc aggctggagt caggctgaac 660
   gcctctgccc tcatggggtt tatattctag gaagaccaac aaaaacaaga agacggaaaa 720
   ttaaaacaac aaaagcccca ttgacaggcc gtgaagaatg ccatgaaaaa tgaatggcgt 780
   tgtgctgcag tctttgggga aacgggctta cggaaagaag gacacttgag ctgctaccaa 840
   tgagcagccg tccggtggga gggcagttca ggaagagcag acatccactg aggaggcgct 900
   ggggcagagg gcagcctggt cgctggattc gggggaggaa ccacatcagg ccatgagctg 960
   gagctggtgg tagaatgtac aggagaggcc agccagggcc agctcatgtc agacctcaag 1020
   cggggaagat gaatcgagaa tgcaccccac gagcaatggg aagccagtct acgatttaag 1080
   cagcaaaaat attttccctt cttccaccct gcatccagct ctaccagcac agcctggggt 1140
   tctattttca agatagaata gacccagact cccagctctt cttacacttc tactactgcc    1200
   acctgtcacc cactcatgcg tccccacttg cagcctcgac ccccttccac ctgatctcat    1260
   ggcagccagg gaagctccag ggctcgtgag ggctgccatc tcaggaaaga agcaaaagcc 1320
   ttcggcacct gcagggcctg ctccaaccac acttcttcct tgacctctca gcttccttag 1380
   ccactccctt cccacatctc accctgctcc agccacagtg gtgtctctgt gggttctcaa 1440
   acacaccagg tgcactcctg cctcagggcc tttgtgcttg ctgttctctg ctgggactct 1500
   tttttttttt tttttttttg agacagggtc tcactctgtg gcccaggctg gagtgtagtg 1560
   gtgtgatcgt agctcattgc aacctcaaac tcctgggctc aagcaatcct cccacctcag 1620
   cctctcaagt agttagcttt tgttgttttg ttttgagatg ggatctcact ctgttgccca 1680
   ggctggagtg cagtggggca atcttggctc accacaacct ctgcctccca ggctcaagca 1740
   attctcctgc ctcagcctcc caagtagctg ggattacagg catgtgccac cacgcccagc 1800
   ttatttttgt atttttagta gagacagggt ttcaccatgt tggtctggct ggtcttgaac 1860
   tcctggcctc agatgatcca cctgcctcgg cctcccaaag tgctgggatg acaggcatga 1920
   gcctgtctct agtagttagg actacagaga ggggccatca tgcctggtga tcctcccacc 1980
   ttttctgctc caactctttc accccactta gcctcgtggc tcactctctt acctcttcag 2040
   ctcctcagtc aggcctgagg acccctgttg aaaattgcaa accacacccc ccaccaccac 2100
   cacccactat tgccagcact ttctactcca tttctctgct ttacttttct cctttgtact 2160
   catcaccacc tgactcatta catgtttacg tatctttctt ctctccacta gcatggaagc 2220
   tccaggagag cagagagtgt agttttattc cctgatgtgt ttcctgtgcc cgtaccaggg 2280
   cctagcacac agtaggtgct cagtaaatgt gtgttggatg aacaaataca gtgaaaggat 2340
   ctgatctaca tttataaaga aggcactctg gctgctgagt ggggatgaga ctgtcaggag 2400
   gaaagaggcc cctgtggggg cctggccagc aggtgggtac aatggtagca gccaggagag 2460
   agggcctctt ggactcaagt ggatggggcc tgctcagggc tccggccaca ggaacaaagg 2520
   gaagggggcc caggatggcc tgtcatagag gacacattac aactggccca aagttcaagt 2580
   caggtttcta aatttgggaa gggatacaga aaaactaaag actctactgg acagtcagtt 2640
   attgaaatga ttacatagaa aatgtaccaa gaattaaaaa aaaaaaaaaa aagcattatg 2700
   aaggggccac cagagactcc cagagaggaa agggactatg ggctggatgc ggtgactcac 2760
   acctataatc ccagcacttt gggaggccga ggagggtgga tcacgaggtc aggagttcaa 2820
   aaccagccta ggcaacatgg taaaaccccc gtttctacta aaaatacaaa aaattagctg 2880
   ggcatggcag catgtgcctg taatcccagc tactcgggag gctgaggcag gagagttgct 2940
   agaacccagg aggcagaggt tgcagtgagc cgagattgag ccactatgct ccagcttggg 3000
   cgacagagca agactccgtc tctaaaaaaa agaaaaaaaa ggccagatga ggtggctcat 3060
   gcctgtaatc ccagcacttt gggaggccga ggtgggtgga tcacgaggtc aggagatcga 3120
   gaccatcctg gctaacatgg tgaaactcca tctctactta aaatacaaaa aattagccgg 3180
   gcgtggtggc gggcacctgt agtcccagct acttgggagg ctgaggcagg agaatggcgt 3240
   gaacctggga ggcggagctt gcagtgagcc gagattgcgc cactgcactc catccagcct 3300
   gggcgacaga gttagactcc gtctcaaaaa aaaaaaaaaa aaaaaaatta gctgattagt 3360
   tgggcttggt ggcgggcgcc tgtaatccca actactcggg aggctgaggc gggagaatca 3420
   cttgaacccg ggaggcagag gttgcaatga gccgatatca cgccactaca ctccagcctg 3480
   ggcgacagag caagactcca tctcaaaaaa gaaaaaaaaa aagaaagggg ctgtgctgtg 3540
   gcctgggacc caaagcacac tactgcaagg tcccagggtg cctgactcca accggagcct 3600
   tgagaacatt catttgcaaa gaatgaatta aaattcagca ctattttatt ctgcaggatt 3660
   ccagcacccc aaggacagtc atttttagac ccttcagtaa cgtaataagt aaccggagga 3720
   tgtgctgagc ttccacttcc ccagacggtt gcctgtcaca gctcatcagg ccaacaaact 3780
   tttcttaggc ctcaaatttg gaaatgttca ctctcagttc gttccttaga tgcaagtcca 3840
   tcccaatgaa gtaacagggg ctcagcacct gtccaatctc attgcttccg gggacagggg 3900
   cccatgagga tgtcgtttca gcccggtgac acttgggcaa agtgcctttt ggtttccctc 3960
   ccaggctgga acgtgctggc tctgtgaagt tacgctgggc acaagagccc cccccaaccc 4020
   ggcaggactg actgctgtgg tcagaggcgc ccctggggct ttgggagcca cagaatcttc 4080
   ctgagggcag cgccggagga ggccccagtg agagtgccca ctgccaggct cattcctcag 4140
   gctgccgcag gcctctcccc aaaacaggca atgcttctca gcaacctgcc ccaggagcag 4200
   gccagggaag gccgccatcg gcctacagtg ctgggctctg gagggcttgg ttggtaacag 4260
   gccatggttt ctatgagcca gctggggtgt gaaggacaca ggctggattc acctctctgg 4320
   gcctcagttt ctgcattcaa aaagtgggaa tcatgatatc tgctctattt cttatctctc 4380
   agtgctgatg tgaacctcca ataagacttt taaaaatact ctttctacct tacttttatt 4440
   tttcatttat tttaagataa tgtctagctg tctcacccag gctggagtgc agtggtgtga 4500
   ttacggctca ctacagcctt aacctcccag gctcaagtga tcctcctacc acagcctccc 4560
   aagtagctgg aactacaggc atgcaccacc gcacctggat aattttttct tttgagacaa 4620
   ggtttcactc tgttgcccag gctggagtgc agtggtgcac tcttggctca ctgcagcctc 4680
   aacctccctg ggcttaggtg atcctcacac ttcagtctcc caagtagctg ggactacagg 4740
   tatgtgccag tacacccagc taatattttt gaaggatggg gtttcactat attgcccagg 4800
   ctggtcttga actccagggt ttaagcaatc taccttcctc agcctgccaa agtgctagga 4860
   ttataggtat gagccacccc ccggcctata atcctaccac tttaaaaaag cctgtaattt 4920
   tagcacttta aaaaattttt ctaaattttt tatagagatg ggggacagct gtggtctcac 4980
   tgtgttgccc aggctggtct tgaactccta ggatcaagcc atcctcctgg cctggcctcc 5040
   caaagtgttg ggattataag cataagcctt accttacctt ttttttttga gttgcagttt 5100
   tgttcttgtt gctcaggctg gagtgcaatg gcaagatctt ggctcactgc aacctccacc 5160
   tcccgggttc aagcaattct cctgcctcag cctcccgagt agctgggatt acaggcatgc 5220
   gccaccacac ccagctaatt ttgtattttt agtagagatg gggtttctct atatacctta 5280
   attttaaagc actgcattca tgtaaattgt gattaacatg gattcaagag agggagtgag 5340
   gatgaatgag ccaggcagtc acctcggctg tcaccctcca cttctctcct ccttctgaca 5400
   gtcatcgtcc atccgtttct gcagctgttt gtttgactct cctgatcatt ttgcttgcca 5460
   cataacttgc ctcctgggaa agaatgccct gggcaggccc acatgagtag tgaaaaataa 5520
   tctgcagtga aaaataaaac taagtagtct ggtccacaga gcagtcttat tttttcactg 5580
   cagatgaagg agttgacatt caggcttcat tctcatttat aagtgtttta aagacacata 5640
   cagtggattg aacagtggcc ttcaaaaaga tgtatctaca tcctaatccc tgggacctgt 5700
   gaatgttaac caagttagga aaagggtctt cccgggtgtc attaagttag agatcttgag 5760
   atgaggagct catcgtggat tatccaggtg gaccctgcat ccaaggacaa atggtcctta 5820
   gaaaagaaaa gcagaggctg ggcacagtgg ctcaagcctg taatcccagc actttgagag 5880
   gccgaggtgg gtggatcacc taaggtcatg agttcgagag cagcctggcc aacatgatga 5940
   aatcccatct ctactaaaaa tacaaaaatt agcaaggcat ggtggcgggt gcctataatc 6000
   ccagctactc aggaagctga ggcaggagaa tggcttgcac ctgggaggcg gaggttgcag    6060
   tgagccaaga tcgcgccact gcactccagc ctgagggaga aaagtgaaac tctgtctcat    6120
   aaaagaaaag aaaagcagac agagatctga gacagaagag gagagtgaag gaaaaaaggc    6180
   catgtgaaga tgaggcagag gttggagcca tgcagccaca agccaaggaa tacctggagc    6240
   cccagaagtt gcaagaggta ggaagaagcc tcccctagag cctccagacg gagcacagcc    6300
   ctgccaacac ctccacctca gacttctggc ctccagcact gtgagataat caactgctgt    6360
   tgttttaagc caccagattt gtggtaattt gttatggcag ccacaggaaa ctaatacagt    6420
   acctaatctt cacaaaccca tcttacagaa aaggaaactg aagtcagaga ggtagtggct    6480
   tgtgcagtgt gttaggccat tcttgtatta ctataaagaa atacctgagg ccgggcatgg    6540
   tggctcacgc ctgtaatccc agcactttgg gaggccaagg tgagtggatc acttgaggtc    6600
   aggagttcaa gaccagcctg gacaacatgg tgaaacccca tttctactga aaatatgaaa    6660
   attagccagg catggtggcg tgcatctgta gtcccagcta ctcaggaggc tgaggcagga    6720
   gaatcacttg cgcccgggag gaggaggttg tagtgagcca agattgtgcc actgcactcc    6780
   agcctgggag acaagagaga aaccctgtct caaaataaat aaaaaacaaa taaacacctg    6840
   agactgggta gtttataaag aaaggggtta actggctccc ggttctgcag gctgtacaag    6900
   catggtgccg gcatctgctt ggttgctggg aaggcttcag ggagttttac tcatcgtgga    6960
   aggcagagcc agagcaggtg catcacacag caaaagcagg agcgagagag agagagagca    7020
   gggaggtgtg cacactttta aatgagcaga tctcacgaga actcaccatt gcaaggacag   7080
   caccaagcca cgaggggtct gcccccatga cccaaacctc ccactaggcc ccacccccaa   7140
   cattgggaat tacagttcaa catgaggttt ggggggacaa atatccaaac tatatcattc    7200
   cacccctggc cccccagatc tcatgttctt ctcacattgc aaaatatagt catgccttcc    7260
   cagtagcccc ccaaagtctt aactcatccc agcattaact caaaaatccc attcccaagt    7320
   ccaacgtctc atctgaagat gagttccttt cacctacaag actgtaaaaa tgaaaacagt    7380
   tatttactgc tgagatacaa tgggggcata ggcattaggt aaacattcct gttccaaaag    7440
   ggagaaatcg gtcaaaagaa aggggctata ggccccaagc aagtccaaaa cccagcagag    7500
   caatcattca atcttaaagc tccaaaataa cctccttaaa ctccatgtcc catagccagg    7560
   gcacactggt gcaaggggca ggctcccaag gccttgggca gctctattcc tgcggctttg    7620
   cagaattcag tccccatggc tgctcttaca gattggagat gagggcctgc ggcttttcca 7680
   ggtgcagggt gcaagctgct ggtgatctac cattctgggg tgtggatggt ggcggccccg 7740
   tcccgcagct ccactaggca ttgtcccagt ggggactcta tgtggggcct ccaaccccac 7800
   atttcccctc caatgggaag gctctgcccc tgcagcagcc ttcttcctgg gctcccaggc 7860
   tttctcatac atcctctgac atctaggtgg atggtgtcaa gcttccttca ctcttgcact 7920
   ctgcacacct acaggcttaa caccacatgg aagctgccaa ggtgtatggc tggaaccctc 7980
   tgaagcagca gcctgagctg tgactatggc cctttgagcc aaggctggag ctggaacagt 8040
   ctagatgcag gcagggagca gtgtcctgag gctgtgcaga gcagcagggc cctgtgcctg 8100
   gacaatgaaa ccattctttc ctcctcatcc tctgggcctg tgatgggagg gttgtggaag 8160
   atctctgaaa tgcctttgag gcctttttgc ctctgaggcc tatttcctat tgtctcagtt 8220
   attggcagtc ggctcctttt tagttatgca aatcctctag caagaggtta ctccactgcc 8280
   ggcttgaact cctctcctga aaaagctttt tctttctttg tcacatggcc aggctgcaaa 8340
   ttttccaaac ttttatgctc tgttttacct ttaaatataa cttctaactt taattcattt 8400
   atttgctcct gcatttgagc atagggaatt caaagaagct gggccacatc ttgaatgctt 8460
   tgctgcttca aaatttatgg ccacgcttgg tggctcacac ctgtaatccc agcactttgg 8520
   gaggcctagg tgggcagatc acgagatcag gagatcgaga ccatcctggt caacatggtg 8580
   aaacccatct ctactaaaaa tacaaaaaaa ttagcttggt gtggtggcgc agacctgtag 8640
   tcccagctac tggagaggct gaggcaggag aattacttga acctgggagg cagaggttgc 8700
   agtgagccca gatcatgcca ctgcactcca gcctggtgac agaataagat ttgatctcga 8760
   aaggaaggaa ggaaggagga agggaagaaa tgtcttcccc ccagatgtcc tgggtcatcc 8820
   ctcttatgtt caaacttcaa cagatcccta gggcatgaaa ataatacagc caaattattt 8880
   gctaaggcat aacgaaagtg acctttgctc cagttcccaa taagttcctc atttccatct 8940
   gagactcatc accctggcct tggcttgtcc atatcactgt cagcattttg gtcacaatca 9000
   tttaaccagc taatcgggag gctgaggcaa gaggatcact tgaacccagg aggttgaggc 9060
   tgcagtgagc tgtgatcaca tcactgcagt ccagcttggg caacagagca agatcctgtc 9120
   tcaataaata aataaataaa tacataaata acttaagttt atttaaagct gcatctttgc 9180
   caccatggag aaaggccagg ccagctcctt ctctctttct gcacgtgttc ctcccacctc 9240
   agctgcctct gctcctcaag gaggaacaga gggagtagga aaggccatcc caggaggccc 9300
   agcaccccat gacctggctc tggggccttg tgggtttatg gattcccagt gctgagtcat 9360
   ccctcacagg ctcttgtggg caccttggac attggtcaga agcatgtggt ccccgggaac 9420
   acaccttttc ctgatcatct gggaagggca gcttgtgcca gcgaggccac ctgttcagcg 9480
   ccacggcccg ccagacagct gcagccacag ccttgccttt gatcagagca aacaccagac 9540
   atgtgtgtca tgcccccaac ccatctccag gggacacatg tcctttcttg ccaggcctga 9600
   gatgaacaag agagggacaa gtccccaagc ctctctctcc ttcctgcctc acccactccg 9660
   ctgttagatt ctcaaggtgg atggtgggct aactagggca accgaccatc ctggtttacc 9720
   tagaactgag ggggcatttt caggaataaa actgcaaaag tctggagcaa acaggagcaa 9780
   gttggtcact ctggggctgg tggagtcagg tttccttctg caggccccct ccccgcaagc 9840
   atgggtggaa cccaggacag gaacacagag caggccccag gaccgggctt gtcacttaca 9900
   agtctttttt tttttttttt ttttgagatg gagtcttgct ctgtcatcag ggctggagta 9960
   cagtggtgcc atcttagctc actgcaacct ctgccttctg ggttcaagtg atccccctgc 10020
   ctcagcctcc tgagtagctg ggactacagg tggcaccacc acgcccagct aattttttgt 10080
   atttctagta gagatgagat ggccaggctg gtcttgaact cctgacctca agtgatctgc 10140
   ccgccttggc ctcccaaagt gctgggatta caggtgtgag ccactgtgcc tggccccact 10200
   cacaagtctt aaaccatgcc tcagcacatc aatgccattt acaaaaaggt agagggattt 10260
   tccaggcaaa aatagatgaa agacatagga tgattgatca tgtcctgctt aaacataggt 10320
   ctgatgctat taagaattga gggctgggag cggtggctca cgcctgtaat cccagcactt 10380
   tgggaggccg aggcgggcgg atcacgaggt caggagatcg agaccatcct ggctaacacg 10440
   gtgaaacccc atctctacta aaaatacaaa aaatggccgc gcgcggtgac tcacgcctgt 10500
   aatcccagca ctttgggagg ccaaggcggg cggatcacga ggtcaggaga tcgagaccat 10560
   cctggctaac acagtgaagc cccgtctcta ctaaaaaata caaaaaaaat tagccaggca 10620
   tggtggcggg cgcctgtagt cccagcaact tgggaggctg aggcaggaga agaatggtgt 10680
   gaacctggga ggtggagctt ccagtgagcc gagatcacac cactgcactc cagcctgggc 10740
   gacagagtga aactccatct caaaaaaaaa ataaataaat aaataagaat tgttagtatt 10800
   ttgcaggtgt gacaaatgat tctgtttctg tggcagaatg ttctcaggag atctcttttg 10860
   aactctcatg gaaagcatca tgctgttggc aacatcacat ttatttttat ttatttatta 10920
   ttttttagag acagggtctt gctctgttgc ccaggctgga gtgcagtggc acaatcacag 10980
   ctcactgcag cctcaacctc ctgggctcaa gcaatcctcc tgcctcagcc tcccaaagta 11040
   gctgggacca caggcgtgag ccactgcact cagcccaatg taccttcaat atttacattt 11100
   ctggcaaagg tagcaaaacc ttaacaaatt ttgaatctag ataataaaat tatgaggctg 11160
   ggtgcagtgg ccctgacagg gatggctcac atctgtaatc tcaacatttt gggaggccaa 11220
   ggtaggcgga tcacctgagg ccaggagttt gagaccagcc tggccaacat ggtgtaaccc 11280
   tgtctctaac aaaaatacaa aaaaattagc cagacgtggt ggtgcacgtc tgtcatccca 11340
   gctactaggg aggctgaggc aggagaattg cttgaacccg agaggcagag gttgtgatga 11400
   gccgagatcg cgtcattgca ctccagcctg ggcaaaagca agagcgaaac tctctctcca 11460
   aaaaataaaa aaaaaataaa ttaatgaatt aattaaaata aaataaaata atggatagtc 11520
   actgtaaaga aaaaataaat gtatatatca gccaacaagt gatggaatag agcaccccat 11580
   ctccctggct ggacagatac atcccacaac acctggaagg cggctccatg tagaactttc 11640
   tggactgctt gaggtgctgt gctggagcac ggtgacagag gagctggacc atggacctcc 11700
   cccggccccc accaagggcg aggtccccct gtggtgggtc tgagggaggc atccgtatgg 11760
   cctctgcggc ttgggcaggg aatttggggt ccaagtactt ggtgcaaagc ctggaaagag 11820
   ggtttgggtg ctgagggcat atcccctggg ccacatgggg gcagaagtgg ggccccctga 11880
   agcttggagt cctgggcagg ggcatctatt ttgctgtctg aggccttcag tacttgaagc 11940
   aaaatggagg cagaatgtcc caccttaatg cccctgattc ctccaaacca attccagaga 12000
   cagcaagggc cagaacaggg atggccctgc ccagggtcat gcancgagga agtggccagg 12060
   ctgggatctg aacccaggct aatcccctcc cttgtcctcc tccaggccct cacccctgca 12120
   tagagccctc cagctcactc atcctcggcc agctccatct cctcagcttg taaacccccc 12180
   cgggattttc ctttcttaaa aaacaaaggc ttggccaggc acggtggctc acgcctgtac 12240
   tttgggggtg gctcccagca ctttgggagg ccaaggtggg cggatcatga ggtcaagaga 12300
   ttgagaccat tctggccagc atggtgaaac cctgtattta ctaaaaaaaa aaaaattaac 12360
   tgggcatggt ggctagctac ttaggaggct gaggcaggag aatcgcttga acctgggaga 12420
   aagaggttgc agtgagccaa gatcgcgcca ctccacttta acctggcaac agaacaagat 12480
   tccgtttcna aaaacaaaca aacaaacaaa taaacaaaaa aaggcggagc gcgatggctc 12540
   gcgcctgcaa tcccagcact ttgggaggct gaggcgggcg gatcacttga ggttaggagt 12600
   ttgagaccag cttggccaac atggtgaaac cccatttcca ctaaaagtac aaaaatcagc 12660
   caggtgtggt ggtgggtgcc tgtaatccca gctactcagg aggctgaggc aggagaatcg 12720
   cttgaaccca tgacctggag gctacagtga gctgagattg cgccactgta ctccagcttg 12780
   ggcaacaaga tttgtttctc taaaaaaaaa aaaaaaaaga ctggcccttc cccttcagct 12840
   cttcctcagg gtccctgagc actctacacc cccgtctaca ctgagcactc caccctgctg 12900
   tctacactga gcactccacc ctgccatcta cactgaggac tccaccccac tgtctacact 12960
   ggctgcctcc cgccctcacc tcctgctaag gccattcccc gctgcatctg tcttctagat 13020
   tctgcagcct tcagcacgct gggcccctcc tttgtcccct tgagccacct ccagcctccc 13080
   cctgagctgc tactcctctc ccagcagcct ccacccaagc ccctccagtc cccaagctgt 13140
   cccttgcatc cagcactgcc cttccacgtg ccccttccct ccagcttcac agcagggtgg 13200
   ggcctccagg ccctgcccac tgtgcccatc cacaagttgt ggtgggagct ccgaggggag 13260
   gcaggggtgt gcatggactt gggacgtcca agtctgggac caggggcagc tggttggtgg 13320
   agtgtggagg gggataggga ctttcaggta gagaggctgt aggggcaaga tcgggacggc 13380
   ggatgtccct aaggagggct ctgacctggg aaatattgtg cagcttcctc tttgccattc 13440
   ctggagctca gacactggcc ggctctcacc ccgcccttcc tgcaggacac agctccatcc 13500
   cagtgagttc ctagtgtaga catctccagc agcacggatg ggaaaggaag tcatcaaagg 13560
   tgcccaggac cggaggcttt ttctggaggt ggcagaggag ggtgtgggtc tcagggctct 13620
   ggctgagggc aagcgtggga ggtcttaggt ctgcaccagc cccgtgaagg cccctcctgc 13680
   tccctggtgg agtcctagag ggaacagcag cccctaggct ctagcaggag tgggtagggg 13740
   cttttctggc ttcctactgt gccagcagga tagctgggcc tggcactgag cccaaagatc 13800
   acatgccggg gcattggcgc agtgaggaac agacccttgc caaagctggc aaagaagacc 13860
   ccatggggtg cagctggtga agctgagagc tcaatgtttg ggggagcctg gcaaaagggg 13920
   tcctcccctc cctctgcagg ccaggatcgc aggttttccc tacatgttgg taattctcaa 13980
   acaatcccat ggccactgga gcaaagatca cagtgggcgg cggcctcggg agcagtggac 14040
   agggcacgca gtgcctttga tgccagagcc ctcgccccaa agtcaacaaa ctctgcagcg 14100
   gactttgcac ccggactttg ttttcaccat acaaggaaag ggacagatca caggccctct 14160
   cgctgccctc gctgagccgg aagctgcagc gtgagctctc tcaagcccca tttctaggtt 14220
   ccccaggcgc acccctgagc ccctactcgc ctattaagtt ctcctaatag cccttcaagg 14280
   tcttaatgta tgtccattag acagagggga aaactgaggc gagggcaagt gacttgaccg 14340
   aggttcctcg gcgagcaggg cgtggagctg agaacctcgt tattactgct ccccacacaa 14400
   ccctctggcc gttcttggaa gaaggctgag ccccgggggg gccagagtga cccaaacacc 14460
   atgggccgcc tgcggtaaca cgtgcggcca cgaaggggca gcagtttccc gcccggccgg 14520
   gctctctccg gcgctcagta tccgtcccag gccaagaaga agaaactcgg ggaggagggc 14580
   ggagggggct gcgtgggagg gcgtggaaga tggacgtggc caggggagtg gcagctgcac 14640
   acagtggatg ctgttaagat gaagggaaag aacgtgggct ccgagatcac tggacacggt 14700
   tccacctttc ttcccgctca ctgcatggcc ctgggcgggt tgttgaaccc ttggaaacct 14760
   gtttttcctt ttttcctttt tttttgagac agggtcttgc tctgtggccc agactggagt 14820
   gccgtggcac gatcttggct cactgctgcc tcccaggttc aagtgatcct cccagctcag 14880
   cctcctgcgt agctgggacc ccaggtatgt gtcaccacag ccggctaatt tttgtatttt 14940
   tttgtagaga cgggatttcg ccgtattgcc caggctggtc tcaaactcct gagttcaccg 15000
   gatcttcctg cctcagcctc ccaaagtgct gggattactg gcatgagcca ccgcacccag 15060
   cagagacctc agttttctaa cctgtgccag caggaataat gatagctgcc tagcttggct 15120
   gtgctgggaa ttaagtaaga tgaccgggta gcaaatatga agtattactg gacacagagg 15180
   gccccaggct gggttagcag cggtggtcag ggctgctgct tcctggcctg agctcgaagg 15240
   agggccctca ttaccacctg ggtgagtcct cgtccaagcc tggcactgct gcgtgggaat 15300
   aacttctgcc acccaagttg gcagattgtg tgcaaagtta agtcctgact ctgtggggtg 15360
   gacttcgagg cctcttcatc ggacctgctt ccggtgactg cattcgcacc tcctcctgtt 15420
   cctggtttaa cacagcccag ctttcctcct gctgagccct ccctgggcct gctgtcaccc 15480
   tcgtgccgct gtgcctcgca gtgccactcc ctgtaccctg aatactttgc cctgcctctc 15540
   cacccagctg agagtcaggg cccctgtgag gctctgccca gcccgtcctc cgggtttctg 15600
   cctctgctga gcacttccct gcatgattgc ttctgagagt ccccccagcc tgtgagcttc 15660
   tcaggactgg gacagcttct caggaccgag gcttcctggt ctgcttgcaa ttttacaggc 15720
   gggcacattt tcccttggcc aacatcagag actggacatc tgcagatctg tgctagccac 15780
   tgagcaccca ggcaccccag caggtagctc tgtaaccaac ccattctgta aagctgaggc 15840
   tcagagaggt gaagcgcctg gcctggggcc acagcctgcg tcagctgcag agccaggagc 15900
   tgagatatgc acctgcggct ctgctcacag ggtcctgcac agactgctgc tggagccacc 15960
   tatgtagagt caagagagtt catgttaact ccctctcaca tccctcagcc agggtggggg 16020
   ctgacgatag acactcaggg atggcctacc ctccccaaca acccccgtca ggtttgccgg 16080
   atctccttgg aagaaaagtt ctgggcagaa ttccaccgtt ggcctggcct acactctcct 16140
   tagtggctta ggaccctcag cggtggataa gttgtgggca gaagagatgc aatcaggatt 16200
   ctcacccact caccccttgc cagccccaat aagctcaata agctgggctc ggtctgagga 16260
   agtgtccagg aaatgtgcaa atggcctggg acagccctgt gttcctttca gtaaggttgc 16320
   tgaaggtgag gctgaaagtt ggagaaacag aagccagtgc ttatggtttt aattaagata 16380
   atggaatgta tgtatgtatg tatgtatgta tgtatgtatt tatgtattta tctttagaga 16440
   tagagtctca ctctgttgcc caggctggaa tgcggtgaca caatcatagc tccttgcagc 16500
   ctcgacttcc tatgcccaaa tgatcctcct acctcagcct cctgagtagc tgggactaca 16560
   gacacacgcc aactatgcct agctaatttt tatttctatt ttttgtggag actgggttct 16620
   cactttgttg cccaggctgg tcttgaaccc ctagcttcaa gcaatcctcc tgcctcagcc 16680
   tcccaaagtg gagggattac aggtgtgagc caccacacct ggcctggaat ttatttgtat 16740
   tctgcttata aaattaatac attcttattg cagaaaagtt tgaaaataaa agaaaggaca 16800
   aagaacaaaa agcgtatata atttcacagc tcagatctca ctgctattaa catttttatt 16860
   tactttcagg cttttttctt tctaggtaca tatgcagaga ttattttatt ttatttattt 16920
   tattttatat tttattttat attttttatt tcattatttt attttatttt attttattat 16980
   ttttagagac agggcctcac tctgtcaccc aggctggagt acaatggagt gatcatagct 17040
   cactgcagcc tcaaacacct gggctcaagc aatcccccca ctcagccttc tgagtagttg 17100
   ggactaaagt gtgagtctgg ctaatttttt ttactttttg tattgacaga ggtctcacta 17160
   tgttgcccag gctgatctca aactcctggg ttcaagcgat cctcccacct tggactccca 17220
   aagtgctggg attacaggca tgagccacca tgcctggcct aaaatgccac tttttgtcat 17280
   ttactaaaat cccatggaca ctttgacatg tctgtattct atgctattga tctgactgtt 17340
   ggcatctaca tcattatggc catctatcat ctatcataat ccattttaac attaaaattg 17400
   tgctgctgct tagatttttc tggcctgtct cctatttgta ttcttccaga taaattttag 17460
   aatcatttta tcaaattccc cttgcagaaa aagccctatt ggattttggt tgaaaaatac 17520
   tgaattttta cattaactta ggaaagggct gggcacggtg gctcacgcct gtaatcccta 17580
   cacttttcga ggccaaggca ggtggatcac ttgaggttgg gagtttgaga ccagcctggc 17640
   caacatggtg aaactcggtc tttactaaaa atacaaaaat tgccaggcgc attggctcac 17700
   ctgtaatccc agcactttgg gaggccgagg tgggtggatc acgaggtcag gagatagaga 17760
   ccatcctggc taacacggtg caaccccgtc tctcctaaaa atacaaaaaa ttagccaggc 17820
   gtggtggtgg gcgcctgtgg tctcagctac ttaggaggct gaggcaggag aatggtgtga 17880
   acccaggagg cggagcttgc agtgagccaa gatcgcgcca ctgcactcca gcctgggcga 17940
   cagagtgaga ctccatctca aaaaaaaata ataataataa tacaaaaatt agccgggggt 18000
   cgtggcgtgc acctataatc ccagttactt gggaggctga ggcaggagaa tcgcttgaat 18060
   ccaggaggtg gaggttgcaa tgagcagaga tcgtgccact gtactccagc ctgggtgaca 18120
   gagtgacact ctgtgaaaaa aaaaaaaaaa ttctgaagga ttgagactct tagactctta 18180
   ggtcttccta tccaagagca caatatagct tttcatgtat tcaagccttt ttcaatgcat 18240
   caacagaatt ttacagtttt tttcatgata tcctgctatt tcttataaaa tgtattccta 18300
   gatattctgc atgttttccg gttgtttgtt aataaatatt tttcatttgt cattatttcc 18360
   taattggctg ttatttgtat atatgacatc tgttgaattt tttgattact ttgaaaatgg 18420
   ccattctttt gtgttttttt ttaactttct attttgagat aattttgact tacagaagat 18480
   ttgcaaaaat agtacagaga gttcctgttt cccccttatg ttaacccagt ttctccttat 18540
   gttaacatct tacataacta cagaacaatt gtcaaatcta agaatcaacc tgggcacaat 18600
   gctattaact aaactgcaga agctgttcag atctcaccag ttcttctact gctccccttt 18660
   tctcttccag tgttcaatcc ggaatcctac attatattta gttgtcattt ctctttggtg 18720
   tcttccaatc tgtgacagtt cctcagtctt tctttgtctt tcatgacttt cattttttta 18780
   tacttttgaa aaatactggc cggttgtttt gtagaacgcc ctcagtttgg gtttgcctga 18840
   agttttttgt gattagatcg aggtcatgca ttattggaga gggtgccacc gcctcgatgt 18900
   gcaagctcaa tgcatcatat cagagggttt gtaatgtcag tttataccgc cggagaccct 18960
   aacctggagc atttcgtgaa ggtgctgtct gccaggattc tccactagaa agttactatt 19020
   tttccctttt taattactga atgtctgagg ggaaatactt tgagactatg caaatatcct 19080
   gtttctgctt taacttcggc tcactaagtt tagcattcat ctatggatct cgcttatagc 19140
   aagtattact gtggagttct aatggtaatt ttctgtttct ctcattcctt caacctttat 19200
   taatatgctt cttcctcact tattcatttt gtttcagttg tttataccaa catggatttg 19260
   tggatattgg ttttattctt tgggttgcaa ttgaatccta tcattatttt gttagtcagt 19320
   tgttccatcc gaccttggtc attaggagcc cttgaaattt ggctcccatg cctttttttt 19380
   tttttttgag accgagtctc actctgtcac ccaggtttga gtgcagtggc atgatcttgg 19440
   cttcctgcaa cctccgcctc ccaggttcaa gcaattctcc tgcctcagcc tcctgagtag 19500
   ctggtattat aggcgctcca ccaccttgcc cggctaattt tttgtatttt tagtagagat 19560
   ggggttttat tatgttggcc aggctggtct caaactcctg acctcaggtg atctgcccgc 19620
   ctcggcctcc caaagtgctg ggactacagg cgtgagccac cacacctggc ctcctatgcc 19680
   attttaacat gcccgtcttt tctttttctt tcctactttc tgtgactgta agaagctcca 19740
   ggatacattt ttgctgccct agacttagcc tcaatcagtt ctcagaaaag ctctggttct 19800
   ttttatggga tacttagaaa actagctctg tatggcctgg cgcggtggct cacgcctgta 19860
   atcccagtac tttgggaggc cgaggtgggc agatcacaga tcacgaagtc aggagatcaa 19920
   gaccatcctg gctaacatgg tgaaactctg tctctactaa acatacaaaa aattagtcca 19980
   ggcgcggtgg cgggcgcctg tagtcccagc tactcaggag gctgaggcag gagaacggca 20040
   tgaacccggg aggcggagct tgcagtgagc cgagatcggc agccactgca ctccagcctg 20100
   ggccacagag cgagactccg tctcaaaaaa aaaaaaagga aaaagaaaaa agaaaactag 20160
   ctctgtatgc tagttttttt tttaagacag ggtctctctt gccccagctg gagtgtagca 20220
   gcacgatcac agctcactgt agcctcaacc ttctgggctc aagcaatcct cctgcctcag 20280
   tctcctaagt agctgggtct acaggcatgc accaccgtac gtggcaattt ttaaaaactg 20340
   tttgtagaga tggagtctcc ctatgttgcc tggtctggaa ctcctggcct caagtgatcc 20400
   tcctgcctcg gcctcccaaa gtgctgagat tacaggcatg agccactgta cctggcctgg 20460
   ccaaggtctg tcttttttta aaagaagttg ttgtatagtt gttttttttt ttattttttt 20520
   ttctgagacg gagtctcgct ctgtcgccca ggctggagtg cagtggtgcg atctcggctc 20580
   actgcaagct ccgcctccca ggttcacgcc attctcctgc ctcagcctcc cgagtagctg 20640
   ggcctacagg cgcccgctac cacgcccggc taattttttg catttttagt agagacgggg 20700
   tttcaccgtg ttagccagga tggtctcgat ctcctgacct cgtgatccgc ccgcctcggc 20760
   ctcccaaagt gctgggatta caggcgtgag ccaccgcgcc cggcctgttg tatagttttt 20820
   atctcgagtt ttctagcgat ttaatcatat tggttacaaa aaaggatgat tttactacct 20880
   cctttccaat gtttctacat attttttcat tttatctaac tgcattttaa aataaacttt 20940
   taattttaga atggtttcat atttacagaa aatgtgcaaa gatagtacag agagttcctg 21000
   tgtactccac acccggtttc cttattatta tcttaacgtg atacacaatt aataaaccag 21060
   taacattatt attcactgaa gtccacactt tctttttttt tttttctgag acggagtcta 21120
   cttctgtcac ccaggctgga gtgcagtggc gcaatctcgg ctcactgcaa cctccacctc 21180
   ctgggttcag gcaattctgt ggctcagcat cccaagtagc tgggaataca ggtgcccgcc 21240
   accacgcccg gctaattttt tgtattttta gtagagatgg ggtttcacca tgttagccag 21300
   gatggtcttg aactcctgac ctcgtgatct gcctgcctca gcctcccaaa gtgctgggat 21360
   tacaggcgtg agccaccgcg cccggcgtcc atactttctt tagatatcct tcctttttac 21420
   ctaacgtcct tcttctggtt caggatccca tccagaaagc aacattaccc ctcgccatca 21480
   cgtcttcaca ggctcccctt gacgggaaga gttcctcaga ctttccttgt ttttgttgac 21540
   cttgacagtt ttgaggagga ctggtatctt agtctgtttt gtgctgctat cacagactag 21600
   ctgagaccga tacatgatac atgaaaaaaa atgtattctt acagttgtgg aggctgggaa 21660
   gttcaagacg aagttgctgg ttggtttggt ctctggtttc aagatggcgc cttgctgctg 21720
   catcctctgg agaagaagaa tgcggtgtcc tctcactgca gaagatggaa gcgctaaaag 21780
   gaatgaactc cctttgccaa gccattttat aatgggcatt aatccacaaa ggatgaaacc 21840
   ctgagaaaca tcaagcttta aagcactggt tctcaacctt tttggtctca ggagcccttt 21900
   atactcttaa aacgttttga ggatcccaaa aaaaggcttc tacaggttcc atcttttaat 21960
   atttaccata tcaaaaatta aactgaaaaa attttaaatt atttattcat ttaaaataac 22020
   aaggataaac ccattacatg ctaacataaa tcatgtattt tatgaaaaat agctatattt 22080
   atcaaaacaa aaattagtga gaagagtggc atgtataatt ttttttgttt attttttgtt 22140
   tttagatgga atcttattct gtcgcccagg ctggagtgca gtggtgtgat ctcggctcac 22200
   tgcaagctct gcctcccagg ttcacaccat tctcctgcct cagcctcctg agtagctggg 22260
   actgcaggtg cctgccacca cgcccggcta attttttgta tttttagtag agatggagtt 22320
   tcaccgtgtt agccaggatg gtcttgatct cctgaccttg tgatccaccc gcctcagcct 22380
   cccaaagtgc tgggattaca ggcttgagcc actgcgtctg gcctaaattt ttgtgaatgt 22440
   ctttaatgcc tgccttctca tatttgtttc tgcattcaag ttattgcaaa atgttgtgtt 22500
   ggttgaagtt tgtaaagaaa atgtggcctc atacagttgt gtagttggaa aggcaagagt 22560
   attttgattc tctcttcaaa caactatgga caacctgctg ttacaaaacc agaatgcaaa 22620
   aagttgtagt aaatacaggt taggtgtagt gtggaatctg aaagcatgtg aatgaacttt 22680
   ctgagttttg taacattaaa gtccagttgc gttaagctac tgtgatagca tatagcattg 22740
   tcctaatact ggaattagta tcagaagtgg ggtgctactg ttaataaata aaaagaaata 22800
   aataaatcat gtgatactgg ctcagaagtc aggcagtagg ctgtgtggaa cctgacatca 22860
   cgccatgtaa tacattggca accatttgat ccagctgtct gtcatgatga cttggaaagt 22920
   caaccacata cttacagagc ctgtagacat aggggaaaat agtataaaac agaatactaa 22980
   cagtggacct tggttcttgc cagttgcatt tagccaaata ttaaacaaaa gagatattct 23040
   tgggcagcaa ctggaccatc ttcaagtaaa agtgaaaggt aataaacaga gtccagacat 23100
   ttgtgcccat gcgggttaag aaaaatccag ttgcttctag acaccgtata tgaaaacaac 23160
   gctgaaaaca agcctttgag tggtaaaggc cgattaacac tcagcgcggt aacaaagacc 23220
   aggtgggcta acccgaaatg aaatgagaag cctgtggtga tgaggaggca gagaagtaaa 23280
   atcaagtttg agcatttcgt ttaggagagt ttgggctctg attacttgca catgcaaacg 23340
   aactggaaac aaacagatca gatgtctacc acttcttcga gggaattgca ttgccaaaga 23400
   agtcatgaaa gcagactcta tactgattag gcattaaaac aaaaacaatc tttaggcccc 23460
   taaacttgca tgggcaggaa gtgggctgtc aaagctgttc atcctctaag gtggacctag 23520
   ttcctagtcc ccagtataca cttcagatgt ggccctggag gacactggac atggaggacc 23580
   tcccagagga tgaggctagg gcttcatttc tccaatgacc tcagctgcct ctatttcccc 23640
   ttcttcctct ggaagtccta tcatcgttat tattattatt atcatcattt ttattttgag 23700
   ataaggtctc gctctgttgc ccaggctgga gtgcagtgac atgatcatgg ctcactgcag 23760
   ccctcccagg ctcaagtgat cctcctgcct cagcctcctg agtagctggg agtacaggca 23820
   catgccacca tgcttggcta tttttttttt cagtagagat agggctctca ctatgttgcc 23880
   agggctgatc tcaacctcct gggttcaaga gatcctccta cctcagctcc tgagtagctg 23940
   ggattcgggt gcacaccacc atgccaacta atttttaatt tttttttgta tggacaggat 24000
   gtacagtgtt agaaatggat tgcttgcaga ggcaggagga tcacttgagc ccaggagttt 24060
   gatcacactg tgaaccatga tcgcacccct gcactccaat ctgggcaaca gagtgagacc 24120
   ttgtctcaaa aaaaaaaaaa aagagagaga gagagagact caaagatagg caaaaaagtg 24180
   ggaaagcttt atagtggaca aaaaggaacg ctctaagtct gccctattgg catggtgctg 24240
   aaggtgggct aactagagat agggggtact atgtggttga ctatgggtgc atctttggct 24300
   ttccctgggt gatcctaagt tggaagcagg gacaaaaatt agggaagctg ttagttattc 24360
   atcacgttct ggcagtagtg gactggttgt gatagaagtt attgttttgg ccaggtgcgg 24420
   tggctcatgc ctgtaatcct agccctttca gagttcaacg tgggtggatc aggaaggagg 24480
   gaggatttgg gaggtcagga gttagcctgg ctaacctggc gaaatcccat ctctactaaa 24540
   aatacaaaaa ttagctgggc gtggtggtgc atgcctataa tcccagctac tcgggacgct 24600
   gaggcaggag aatcagttga acctggggag gcggaggttg cagtgagcca agatcgtgcc 24660
   caatttcatc tcaaaaaaaa aaaaaaagtt atcgtttagc ttcctcgatt gttactggac 24720
   gtagtaatct ggcttcctgc aagtctaact ttcagcagac tggctacatg ggctgtgtac 24780
   tgtagataag gcagtaagta aagcaaaaat tgatagagca tcaaggataa atagaaaatc 24840
   cgtaatcaag cagaagattt gaacacttca ctttcagtaa ctgataaaac aagtagacaa 24900
   aaaaaatcag taaggatgta gaagatttga acaacgtaat taacaaactt gacttgattt 24960
   acacgtctag aaccctgcag aacacacact ttttcaagca tactcagaac atttatataa 25020
   agtgaccata tggtggacca taaagcagtt tcaacaaatc tcacaggagt aaaataacag 25080
   accgtgtttt ctgaccgtaa gtacagttaa cctagaaatt gaaaacaaaa agctagaaaa 25140
   accccatgta tctggaaatt ttaatataca ctttgaaata acaaatggat cagagattaa 25200
   ttcaaatagg aatttagaaa taccttgaac tgaaaaataa tgagaatact ataccccaaa 25260
   actgtggggt gcagctgaac agtatataga cgaaaagtat actcatatgt gcatacctta 25320
   aggagcgggg aggattgaaa gttaatggga ggcaaaagca ggtggatcac ttgaggttag 25380
   gagttcaaga tcagcctggc taacagggtg aaaccccatc tctactaaaa atacaaaaaa 25440
   ttatccaggc gtagtgaggc tgaggcaaga gaatcgttgg aacccaggag gcagaggttg 25500
   cagtgagccg cgattgcgcc actgcacccc agcctgggag acagagcgag actccatctc 25560
   aagaaagaaa aaaaaaaaag aaaaggccag gcgcggtggc tcatgcctgt aatcccagca 25620
   ttttgggagg ccgaggtggg cggatcacga ggtcaggaga tcgagactat cctggctagc 25680
   acggtgaaac cccgcctcta ctaaaaatac aaaaaaatta gccaggcgtg gtggcgggtg 25740
   cctgtagtcc cagctactca ggaggctgag gcaggagaat gtcatgaacc caggaggcag 25800
   agcttgcagt gagccgagat cgcgccactg tactccagcc tgggcaacag agagagactc 25860
   tgtctcaaaa aaaaaaaaaa gttaatggga taaacatcca tctcaagaag ttagaaagga 25920
   atgacaaata aaccaaaaaa aaaaaaatca aaagaagaaa atcataaggt caagactata 25980
   aagagagtgg ctgggtgcag tggctcaggc ctgtaatctc agcattttgg gaagcagagg 26040
   tgggcagatc acttgagccc aggagttcaa gaccagcctg agtaacatag agagacctca 26100
   tctttgctga aaataaaaat aaaaaattag ccaggcatgg tggtactgag gtgggaggat 26160
   cacttgagcc taggaggttg aggctgcagt aagccatgat tgtgccactg cacttcagcc 26220
   tgggtgacag agtgggaccc tgtctctaaa aaactaaaat aaggctgggc gcggtggctc 26280
   aaatctgtaa tcccaccact ttgggaggcc aaggctgagg tcagcagttt gagaacagct 26340
   tggccaacaa gatgaaacct catctctact aaaaatacaa aaaattagtt gggtgtggtg 26400
   gcatgtgcct gtaatcccag ctacttagga ggnnnnctnt ngattatatt ttctccttcc 26460
   tacgtcgtta ttggactgaa ttcagaatga tgactctcat tggagctctt cctgtctcct 26520
   aactacagtg gcttccgacc ccactctggt tttcacttca cccctctgct gctcatacga 26580
   gtagatactt ccttccttct ttctcacttg ttgctcttcc tcaacccccc ccgttggtgt 26640
   cccctcctct ttatcttttt ctcgcgacac ctgcgttctc ttgccctctt atcatccctt 26700
   tctcgaggcg gtcctttcct ttatccagct taaatacctt ctcctctgtt tatttggggg 26760
   ttgggttttt atctctcacc ctccctctaa tttctttcct ctttccgcac ccatcaagcc 26820
   tctcgtggtt tctcttcctc tactctcggg tcccccccct ctccccttct ttttttcttc 26880
   acccccccaa gcgctttgcc ttttttttct ttgcccttta ttcccccc 26928
<210> 7
   <211> 29430
   <212> DNA
   <213> Homo sapiens
<220>
   <221> unsure
   <222> (4336), (4345) , (4349) , (4392) , (4447) , (4490)
   <223> Identity of nucleotide sequences at the above locations are unknown.
<400> 7
   aggggaaggg ccggctccgt agctcacacc tataatccca gcactttccg aggagagagg     60
   atcatctcag gccaggagtt caagaccagc ctgggcaaca cagcaagacc gcatctctac    120
   aaaaacttct tttaaagctt aaaaaaaaaa aaaaaagcaa agaggacagt tcaggagaaa    180
   agcctgtaga ggcagcacac taaggaggag acgcagccca ggcaccagga ggggctggcc    240
   atgggcactc actcctccag caggcgagtg cccagcacca gctggcccac ccagacaccc    300
   aggacacggc ctgaatggct ccgtattcac gtgggtggta ataaacaagc aatacacata    360
   gccaataagg acaccttagt aatgttacat cataaacgct gcagatcagg gaaatggtgc    420
   agggtgaagt gggttggggg gctgcatgct acatgagaag tgggtcgggg ggctgcatgc    480
   tacctgagac agagcaggcc ttgctgggaa agaaggagcc ggcaggcctg ggcaaaggtc    540
   ctggggtggg agcacactgg agcagagtgt gggggtagca tggcgggtgc tggtcctctg    600
   ggcgccttcc caccacgtca tgtgcccatg tgcccaaggt ctctcgtttc acagccccct    660
   gaagctcagg ggtcacagct acacagcccc cagatacctt ggcctgcccc aggtcattcc    720
   atccagtgat ggacctgctg acctctagcc tgacctctgg gcagcgtaat ttgagaagga    780
   ggagaaggga gggcaacaga cctggggcga tgagggatgc acagggtggc agacacctga    840
   ggctgcacct tggagcctca gttctgggtg tgggtggggg atggacaggc tgagggctga    900
   agcagctggg cccggccacc atcacacccc aggacccacc agatcaccat gaaaaaccga    960
   atgtcaactg gcagcccaga gtgcagaaca aacctttcag aaacacggtg gtgactgccg   1020
   catcatgaac ataaaataat tacgccctct ccccagggat cacccctgca ggagtttgtc 1080
   ccaagaaaca ccagaaagaa ggaaaacgtc tgagtcacaa tatttgctga ggccttattt 1140
   gtaatagcaa aaaaaaaaaa aaaaaaagaa caatctccag cggcaggggt aactagacta 1200
   ttgtctccgt ggaaaggtag caccaattaa ctagtaacaa aatgactgcg gtaacaacaa 1260
   aacgttcgac atgtcaacac caaaaaccac acacccagca taaccgtgaa ccatgatttc 1320
   tactagaatg aatggcagtt atgagaaagc accagcggag acaaagattg aaaaagtaaa 1380
   ggtggcctca ttagggagac aagtctctgg gtaatatatt gtaatactgg taaatatata 1440
   gtttttaata tattttttaa ttccaaattc catatatgtt cctatgaagc tatttctgca 1500
   aatatttttt tcaggaccgt acatcacaaa ggcaaaaggg ccaggtcagc tctccagctg 1560
   agagtgacca cttcagagca gacggcagac tccagggtta gcaagcctgg ctgagacctg 1620
   gcccatgaca atcactcaac ccctctgacc tcaacatcct gtctgtgaaa tggggataat 1680
   tactgcacct ccacatcaca gagtgcgagg cttaaacagg atgcttcata gaaaagcgct 1740
   caagaggtaa cagccgggag ggggtagtgg ttttcattaa ttaaatgttg ccttcatcca 1800
   gccctgggcc agctccaaca caaagcacac accatccact cagactcagt tgcctggatt 1860
   caaagcccgg cctggcctcc agctgtgaga ttccgggcag gatttcccat ctcccagagc 1920
   ctcagtttcc tcattcatga aacaggaagt gatcattcct tttattttta tttttatttt 1980
   tattttgaga cggagtttca ctctagttgc ccaggctgga gtatgatggc gcaatctcag 2040
   ctcactgcaa cctcggcctc ccagtttcaa gcgattctcc cacctcagtc tcctgagtag 2100
   ctgggattac aggcacacgc caccacgccc agctaatttt gtatttttag tagagacggg 2160
   gttttgccat gttggtcagg ctggtctcga actcctgacc tcaggtgatc cgcccgcctt 2220
   ggcatcccaa agtgctggga ttacaggtgt gagccaccaa gcccagttga caactgcttt 2280
   taaagacacc tctggctgct gtggaaaaca gcctggtagt gcctcaaaaa gttacacata 2340
   gaatgatcct atgaccagta attccactcc tacatatata cccaaaagaa ctgaacccct 2400
   ctactcatgt atgtacacat acaggtacac gcatgttaac agcagtgttc acaaagccaa 2460
   aacatggaaa cagctcaaat gtccataacc gatgaacgga taaatgaaac gtagtctatt 2520
   caccacctga cggaggtgag aggggccata aaaaggaatg atgcataaaa acgaatatta 2580
   tggccaggta tggtggctca cgcctgtaat cccaggactt tgggaggctg aggcgggcgg 2640
   atcacgaggt aaggagttcg agaccagcct ggccaacacg gtgaaacccc atctctacta 2700
   aaaatacaca aattagctgg gcatggtgga gggcgcctgt aataccagct actccggagg 2760
   ctgaggcaag agaatccctt gaacctggga aacagaggtt gcagtgagct gagattgcac 2820
   cactgcactc cagcctgggc gacagaccaa aactccgttt cggaaaaaaa agaaaaaatt 2880
   agccaggtgt ggtggcgggt gggtccctgt aatcccagct ctacttggga tactgaggca 2940
   ggagaaccac ttgaacccgg gaggtggagg tagcggtgag ctgagattgt gccactgcgc 3000
   tccagcctgt gtgacagaag gagactctgt ctctaaaaaa caaaaacaaa aaaggcccga 3060
   cgcggtgtct tacacctgta atgccaacac tttgggaagc caaggcaggc agatcatctg 3120
   aggtcaggag tttgagagca gcctgggcaa cacggtgaaa ccccatctct actaaaaata 3180
   cagaaattag ccaggtgtgg tggcacatgc ctgtaatccc agctactcgg gaggctgagg 3240
   caggagaatc gcttgaaccc aggaagcgga ggttgcagtg agccgacatt gcaccattat 3300
   actccagcct gggtgacaga gtgagattct gtctcaaaaa aaaaaaaaaa aaaaaaaaaa 3360
   ctaaacaaaa gcaaaaaaac caatgagtaa tgttgtcaag tgaacttcat cccaatggga 3420
   atgcagataa tttgtttaaa aggcaccatg cacactgggc aggctggctt cccctgggaa 3480
   cgtcttcttt tgcctggatt cccagttggt ttaatcgggc gtagaacact ttcttcaatc 3540
   cgggattcag gcacccctgc tcagcacaaa ctcagtacac cccgcactct gctgtgggtt 3600
   cttggcacta ttaggagaat gtgagggggt gattcagatc tatctctagt gggtgcatgt 3660
   ctgccactcc caggaacgcc cacttctggc aagtcagtgt cagagaaagg ccagctcgtg 3720
   gcccctcctg ccttgagtcc caggacccgt gatcagtcct acccggagca gaatcaggag 3780
   tttgaaaacc caagtgccaa caatctcatt ttaacccatg taagcatatc caatatttat 3840
   atatagaatt cataacagat gtctgggctt ccattccaat agcctatatt ttacactgtt 3900
   tatttacatg gttacaccaa acaagactca attcaaggta acccaatcct ttgctactat 3960
   accaaaataa gcaacatttt cagtccatgc cttatatata ttcaccaagc attacactag 4020
   gcctccaact gctcatcgga gcaagctgca gcctggacac aagctagaga ttaatcagtc 4080
   aggaatgatc ctgcgtccag tgccagcatg atggaagaga cagagaaaca gaagacatca 4140
   gggctccaga gtcaaggagc ctgcaggtta gttgggcagg atatacacac atacacacac 4200
   acacgcacac acaaaaccac ccaagaagaa aaggtgggat gaatgcatgg acaggtaatg 4260
   cctggagcct ggggatggat aagctgactg caggtggccc aggcaggctt cctggaggaa 4320
   gaagacctgg ctgtangtgg ggtangcang ctttctaaat ggggaaaatc tggctgtggg 4380
   tggagttggc angtttccga aaagaagaaa agctgactat gggtacacct ggctgttggt 4440
   ggaacangca ggcttcttgg aagaagaaaa tctggctgtg ggtggatcan gcaagcttct 4500
   tggaagaagt aaacctgact atgggtggac caggcaggct tcctagagga agaagaccgg 4560
   ctgtgggtga accaggcagg cttcctagac agaggaagat ctggctgcgg ttagagtggg 4620
   caggcttcta agaagaggaa gggctgactg tgggtagacc tggctgtggg tagactgggc 4680
   aggcttcctg gaggaggaag agctggagca ttgaaaaaca aacatgactt ggtgaatgtt 4740
   gagcatgccc aggcctgatc cccagaggca attacgcact caagttactt aattctactc 4800
   acaatgcctc acaaacaact tctctgacac ctaacacagc tctgggcacc ttctagcttc 4860
   agctcctcaa agcagttatt cacgctacta ccctgcacac ctcctcacac cccaacccca 4920
   gggacaggag ttctgccaga tgccaaagct cctgatgcca aagcctgggt ctgcttccgg 4980
   gctcctcttg gtctaactgt ccaccccgca tcggcatgat gtgcaaaaac aaggctttgc 5040
   aatctgccct gatgcctggc ggagcgagtc cctcccgatt cgtctccttc agaaacacct 5100
   gggctgccct ggtcctgtta tacccccaac acattctaca gtcagctccg caagttccac 5160
   aaagatcaac gctggcgttt ttatggcatt ttatttacag tttttacaat ataaaaaagg 5220
   aaggatgcca cagctcagcc agcaggacag acagagatct atgatgcttc tgctgcacca 5280
   ttgtttgtgg tcaagaaagt ctgttttcaa tgatttatta aattgtggtg ggagatggat 5340
   ggtggcagtg gttaccagca acatgaatgt tcttaatgcc actgaacttc acacttacaa 5400
   atggttacga cgataagtgt tatatgtatt ttaccacaat taaaaacagg taaatgcagg 5460
   ccgggcacgg tggctcacga ctgtaatctc agcactttgg gaggccaagg caggcagatc 5520
   acctgaggtc aggggttcga gaccagtctc gccaacacgg tgaaactctg tctctattaa 5580
   aaatacaaaa attagccaga tgtggtggtg catgcctgta atcccagctt ctcaggaggc 5640
   tgaggcagga aaatagcttg aaaccgggag gcagaggttg ccatgagctg agattgtacc 5700
   attgcactcc agcctgggtg acaaaagcaa aactctgtct caaaaaaata aaataaaata 5760
   aaaataggta aatgcaaaca tatggtatag taatattatg ggctattatg agctacaaaa 5820
   aagaatgact tgggactaca gttacagccc tcattcagga atttgtttta aatgtgggtt 5880
   ggtcgctaag gcatgtacac aacattttga cgttcaaata ttcctagatt tggacagtga    5940
   gcacccctct aagctggctc ttctgtccca gaggtcccca ccagtcctcc agaacttctt    6000
   tgctttctta cacaataaga tgccccatgc tcggcttgta cctttccttg ccccagccct    6060
   agaaccagct tcttcgtgga caagctctga ctcctttggg tggagaatgg tattcagaaa    6120
   cccagacctg ggctctggtg tgctcactgc tacttggggt cattgcttct aggcctctct    6180
   gctgatggag gtaggatata cacgtacagt cttccctctt cccagattcc gtacttgagc    6240
   tcgcctactt gctaacattt atttatatcc cccaaattaa acctcacagc acttctgcaa    6300
   tcactcactg acttgcagag tgtgaaaaaa ctgagtcacc atcacacgtt ccaaactgag    6360
   gtcaactgag gccacaacgc cccatcttct tgctccggct gtcgagatgt aagcaagtgt    6420
   ccttctctcg gtctagctag tgccatgctt tccacatcac tgtgcttttt gtgggcaatt    6480
   ttgctgtata aaatgtcccc tgcacatatg ctgctgtgta gtgctcctag gtgcatgagg    6540
   ctgccccacg ccttacagag agaatatgca tgagaggctt tattcaggta tgagttatag    6600
   cgtagttggc catgaattca atgttaatga atcaacaata tacagtaaat aaggtgcttt    6660
   ttagagacag ggtctcactc tgtcacccag gctttagagt ccagtggtgt gaccttggct    6720
   cactgccgcc tcaacctcct gggctcaagt gatcctccca cctcagcctc ccaaactgtt    6780
   gggattacag gcgtgagcta ctgcactcag cctaaataag gtgtcttaga aacacacata    6840
   agacaaggtt atgggctgag tgcggtggct catgcctgta atcccaacac tttgggaggc    6900
   caaggtggga ggttcacttg aggccagaag tttgagacta gcctgggcaa catggcaaga    6960
   cctcatctgt atattttttt aaatcagaca ggtgtggtgg tgcatgccta tagtcccagc    7020
   tactggagag gctgaggcag gaaaatggcc tgagcccagg aggtcaaggc tgcagtgacc    7080
   catgattgta ccactgcatt ccagcctggg gtgacacagc aagacgctgt cttaaaaaaa    7140
   aaaaaaaaaa aagccaggtc aggtatcgaa cagttggcaa aaacgttgtg acctgaggct    7200
   cacaggaacc tagcccgatg tttcccctag gagcaatggt tcagtattca ataattcagg    7260
   gttcccagtg actttatgga gcataacttt caagaataac aagaaccaac tgtacgtgtg    7320
   tatgtatact cacactttta ttttatttta ttttattttt tgagacagag tctcactctg    7380
   tcacccaggc tggagtaaaa tggcgtgatc tcgactcact gcaacctccg cctcccaggt    7440
   tcaagtgatt ctcagcctcc caagtagctg ggattacagg tgtgccccca caaccggcta    7500
   atttctgtat ttttagtaga gacggagttt cgccacattg gccacgctgg tctcaaactc 7560
   ctaacctcaa gtgatccacc cacctcagcc tcccaaagtg ctggaattac aggcatgagc 7620
   tgccgtgcct agcctacata cacttttata cacacatgca tctatgacta tttctctatt 7680
   tctgtgcatg tgtgcgtggc agtacctaca gtttcagcta tgtgtctggg tactgtctcg 7740
   tccaagtttg taagcacctt ctccaaagtg caaagcctgg cttgtgttac tatccatatg 7800
   tttacttatt tgctcaatca atttacttat tagctccata accagcttcc catctgctcc 7860
   agtagcctct gctgtcagtc acctctgcac cctaccccac cttgcttccg gatgctggat 7920
   gccaatcacc cccgacacct ctacatagca ccaccctcga catgctgctt ctttatttct 7980
   tatttatttg tttgagatgg agtcttactc tgttgcccag gctggagtgc agtggcacga 8040
   tccaggctca ctgcaacgtc cgcctcctgg gttcaagtga ttctcctgcc tcagcttctc 8100
   aaatagctgg gattacaggt gcccaccacc acgcccagct aatttttgta tttttagtag 8160
   agatggggtt tcaccatgtt ggccaggctg gtctcgaact cctgacctca agtgatccac 8220
   cttggcctct caaagtgctg ggattacagg tgtgagccac cgcgcctggt ctgcttcttt 8280
   aaatgccagg caccaacatt tgtgcaatgg ggtgggagga aagaacaggg aggagagcac 8340
   actgccggcc cctgcactga atccactgat caatctgggg gcaactgcca tctccatctc 8400
   ctgtcttcct atccgtgaac atctactgca gtcctctcca atgtccttct gtaaagttgt 8460
   attatgtttt gcatacaggc cttgcatatt agttctcaga tataatccat atactttata 8520
   taaaattcaa accacattta aaaaaataaa actagcatga ctataacgga gtctgcaaca 8580
   ttctcacaga ctttatgata aaacatgaaa cttcaaagat acttagggtg gggcagggac 8640
   aatgtttaag gctgcctgga agcctcccca tccctgagcc agaaagtcct atctcccctt 8700
   caaggggaaa tgcttgaaaa agcactgatc aggctaaaat gacagggatc agggagtaat 8760
   caaagtacaa gtgagctggt ctcctccatt ctgagcacag caaagttcag tctctccaag 8820
   tccaagaatc atacacctgt ttgccaagaa tgaagttcag gtgtctacaa gtggctgaaa      8880
   atattcattg ctgggccatt aacaacattc ttggcaaaac cataccttag cttctcgtgg      8940
   aaatttctta aggtagaaga aacaggaaac acccaggctc gcttttatgt agacagttcc 9000
   atgaagccag ggaccttccc cacatccacg tttcaattac ctgcacgcag ctcacagtgt 9060
   attcaacatc tacgcgtctc tcctactggg gtggcggtgg ccactcaaac cctcatgcag 9120
   ctacgatgac cgcaattttg gcaacataat ttcatgtttt tccttgggct tttacccaag 9180
   tcagtgacac aattctgcag ttgtctaaag attcaaaatg agggacttga catttacaac 9240
   aataataaaa tcttgggttt cctttaacca agcacatgtt ctgcctttta gagaaagctc 9300
   tgcaaactca agctggagtg ggatacttgc tgacatcttc aagcacccca ggaatagctc 9360
   tactccccca tttccacctt ggctgaacca tctatatccc accaattccc ccaacatccc 9420
   tccatccgtc catccatcca cccaaggacc tgctaagcca ggaggtctct cccatctacc 9480
   ccacagcctg gcctcagccc acaagggctc tctctacatg aatcccaccg caccagagta 9540
   gaccaagtct cccgtagact ccaccctgac cacctccatg cctccagcca ttcccacccc 9600
   taaaaaccct ccctggtctc tacacccagc tgatgaatac ttggctgaat gtgacctggc 9660
   ctcctggacc caggtgaagc ccacgtcctc cgtaagcccg ccagctcacc ctgcctctgc 9720
   accttcactg gagagagccc gcacttcacc tcctcagggc aggcatggct gatgccaccc 9780
   agtggaatct ggtgcaaagc agggcccggt gcagagcagg gctgcctgca gagcaaggcc 9840
   ctggtgctgg ggccgagcac ctccaatgct ggccgtggaa ccatccctcc cattccaggt 9900
   gctgtctcca tcaagaatga gcgagctgct gacatttgca tgacaataat gaataaatac 9960
   catattttgc ttcaaatcca gaatagatgt ggccagggtt ggcatatgac tgttgggaaa 10020
   ggacagtttg cctcttccca aaccaacttg gattataaaa agcttttctt aacgaccaca 10080
   agagcggagg agctcagggg cagacaaaag gaaggctggc tgcagaaggc gggagagtgg 10140
   ggccttcagg ggcgggtggg gagagagaaa gcctggagct gcacccccaa ggtctgtgta 10200
   catcaggtgc tacagaataa caccacctct tccagcttgg cccccacctg ccctctccca 10260
   gcccagtcac ccagacagca ccccactccc cacacacacc tcacatctgc ccgcctcaca 10320
   ctcaccagct tcggctctca atgcaacctg gaacctgccc ttggcctctc agctcagcca 10380
   cccccattcc tgttggcccc tggcccccca tcgaattctc tctaatccta atgcacacac 10440
   ttgcacactc aaacacacac acacacacac acacacacag cccagaggaa aaccataatt 10500
   gactgaggtc caggcaagtt tcccgagcag ggaccacatt tcaaaggtca gggaagcagg 10560
   cgaacaggaa acatacaggg ggcacgtttg ggggtggagc aggaaataag aaatcacttg 10620
   caaaagataa aaagaaaatg aggtagctgg tttcagacac ctcggagcac acagaacagg 10680
   acaggcgcct ccgggtcttc cctcaacagg gagatgggcc aggcaggtcc ctgctgctcc 10740
   accgcagagc tgggggctat ggccctgaca ccaaggccct ggggcaggcg gggaggcagc 10800
   tgttctcctg cctgtgctcc cgggcagggc ctggccccac aagggaactg gccgaaggct 10860
   ctgcttggct actccggaaa gtcctgggag acaagcaaag gacttgctag gtcactccaa 10920
   acggcccaga tgtgacaact gtgaagaagc cacaccaaag caaggtgaca gaacaatgtt 10980
   ggtgacgtca ggttatcagc ttacgctcaa ctccacttac ccggactcac ccgtaacctg 11040
   ccgtctcttc ccaaccagta aaggatgcct aggtagaggg gcacaaggcc tggagcataa 11100
   ttaccatttt aaaggctctg agaagtcctg cggtgaggaa gcctagttca ctttctctcc 11160
   cctaggattt cccaactgcg cctgatcaca gaacattttt tcatttccac tcaggaaaca 11220
   tattttgaaa aacactggcc tagaggcaga agtgaaatgg aaaacacaaa agtaaaactg 11280
   aacaggaggc actgggcaga gaacggtcag aggcgccctg aatcctggac cggtggagat 11340
   ccccagcttg gcatgctccc ctccctgggc ccagaccgcc tccccccatt tcctggataa 11400
   gaaggctaat gcgcatcagg gtgaagggct tgcctgggct acacccccag gctcgcccca 11460
   caccaatcgc gctcctgcga gagccagtga ctttcttgat ttggctactg tggaattgtt 11520
   tgcaactaac caccccagat acagatacaa atgacaggat gatcagatgt aaaggaccca 11580
   caggtctctg tgatacggct tcatgcagcc agcatggcta gtgccgtgca gaatgagaat 11640
   gaccccaggc aagtccttgc ctcccagacc cagaacccca tggagcccac cagggctggt 11700
   tcacaagcac tgtctgggtc gggcagagat tccagcaaga ggagggaaca tccatgcacc 11760
   ggagccagtt accagaagca aatcgcctct tccaaaaccc aggctattaa tggagtccac 11820
   tgttgagtgg agctggggtc tagctatgga atactgcaca gcagagatct tcctgagaga 11880
   aagcagtttt ccctgaaagc catgtgtcct ccactaactg tgttttaatt gggcgaacgt 11940
   ctgtatctca ttgcagtggc cgcgcatgtg ctgacaaggg gctgggggcg gggtggggag 12000
   cagaagctca ggggcctggg agggaaggaa acaggccacc agggctcccc agaaggcatg 12060
   tatctctctc acaaacacac gcatgcacac acacgtgcac acatactctg caagccctga 12120
   gttagcaact gtggaatgtg accagctcag tgatcccagg acaagctgct agggaatatg 12180
   acatttgatt gatgtctgca aatgtgcgtt ttcactaatt agaaggttta gggcagagca 12240
   gagaaaaata tgtatttcag agtcccagtt tgacctgcca gaaaccagcc cattactaac 12300
   attcttattt tcaacaaaat atagcattct gattacatac catcttggtt ccacgcctcc 12360
   tgccttgcca agcccccgga agcggcccaa ggccatggca aatagtgaga gaaacagttc 12420
   cagggtggag actgactcag gggtgtcagt cagtggggcg ctgatggccg gtgggaggcc 12480
   agcagtcatc accctctcct tgggacagtt gagtagctct cccccagggt catgtggcca 12540
   ctcaggttca tatgggaggc gagaggagtg gcagagtcca ggagagtggc tccgaagtca 12600
   ctgttccctc caggcctcag tgtcttcatc cattaaatgg gtaggctgag gtctgggatg 12660
   acaaggaggg cttgcactta ctgaaaccca tgggaggctg ttcgccgatt tcttttattg 12720
   atggaagaaa acactcgtat aattcaagta ccaattaaaa ggcaggcact ggaaccaccg 12780
   tctgccaatt cctagttttg cctataccaa atttgagcaa gttaattgac ctctcccagc 12840
   ctcagtttct tcgtctgtaa aatgagggta gggatggccc ccagcccaca gggcagctgg 12900
   aaggattaaa gaaatcaaac atctcttaga gcccacctgg cacactgtga tacacaacaa 12960
   atgttagcta tttttgtcta tgaagtctag attttatatc ttgggtgttc taaagcagga 13020
   tacatttatt taaaaacaag gattttcatt aaacacgtac cccacagaca gcaaccccat 13080
   ggagactgct cttaattcag gccagtatcg aaacgactct aactacaagc tttatacagg 13140
   tctcttggct gtccttcaaa tccaactaag gtggtacttc tgaagcactg tgcacatgtg 13200
   tgtgtgcatg cacacgtgtg ggaagggcgg gctcacggat ccctcaggta ccccacccac 13260
   gcagtctcaa gtcacaaagc gacagagcag ccgaggaagg tctgtgcccc actggaccct 13320
   cgtgaagcca ccaactctac ctctgcgccg tgtcctgcag actgggctac cctttgggtg 13380
   gggaccagca tttgatgcaa gaaaggcaga cagaaaagga aaagggcaag ttcgactcca 13440
   gataacacag acagtaccaa gccccagggt ccataaatgc cacgcagatg gaagcattta 13500
   ctgcgaggcc acacagcaaa cgcacggatc cagggacgga ggtgcagact gcggtgcccc 13560
   tgagccatga ccctgcaaat taccaccatg ggaaaggagg ctgccaaacc ccccgacagt 13620
   cggctgggct ggcacagact cgtggtttcc atcgaggtgg gaggaggtgg gacgtcccag 13680
   cccctccccc atgcccactg cagagggaag cggccgtttc ccctgtgtgg ttacaaaggt 13740
   ctcattgttc ttcctcacag ggaggaaact ggaggaccga gctcagaacg cattttagaa 13800
   ctggcagaaa agaacatctg gggaaggaaa cacatttcag aaacaaacat acctttgtac 13860
   cagcttttat tttctttaag tgttgaaaaa ataataataa taaagacatg ccaaatttat 13920
   catcgctcta caaaatccct ttattgagca aaacgtggca gctctacttt caaatgatta 13980
   ctgttcctgg aaaattgcag caacgtggat gccaaggccc gaaggccgcc atcagcagcc 14040
   aaacaaaaga tgccacctcg ggctccgcga cactgtacca tgccagggaa ctggacagat 14100
   ttggggaatg ccacggtttg cctttaaccc cttgcctcct ggtctcctga tgcatctcag 14160
   aggctaacat tctttgagga actggcattt cttagttgta aatatgcatg tgggtttggg 14220
   agctgcctgc aaagtccagt gttgacgatc agctttgatt tccttggaat caagtttacg 14280
   tgtcgagtct ggaagttaag aagaatttgg agaagctgag cactatggtg ttgcaggccc 14340
   tgggtgaact cttccaccaa gcattcattg tggactgaca gcgtgcgagg ggctctgcag 14400
   gcaggtgcac aggacgaaac acattccgtc cgggggaaac ctgcaggaaa gctccctctt 14460
   cttcctaagg tgccgggcct agcttcatgg gtccctaccc tccacgcctg tcacactttc 14520
   tgagtctcat gtgggagctg cttctggttc ctgacttcac tcagtcctca taggaggtgg 14580
   aactactgtc accccatttt acagatgggg agactgggca caaggggacc aagaaaccaa 14640
   tgcaaagtca cacttgtggg atcagtgaca ggggagatca attcccaggt tctttctgca 14700
   agagttaaat tgttttcatg ctgcctaagg gggggcaact gaaagaccac tgcatatctt 14760
   tgccaaaagg gtcaagcaca ggagccgcag ccagtgggtc agatccgcag aggcgctggg 14820
   gtgaccctcc ccatacctgg agggatgctt gtcccctcct ggccttcact gggtcccctc 14880
   atgaccgtgg cctcccagga cctcagcaca atcccggtcc tgtgctccag gacaagccct 14940
   ccgtccccaa gactgtgagg aaatggaacg aagaggggct cgctgcagcc cagcacccac 15000
   actgcccctt ctcaggggca agaaccgtcc tggaggactt ggctttggag ggggagcctg 15060
   ggaggccagt aagtcaacaa gcctctactg ctcatgggtg ggatcccacc gcaggccccc 15120
   acctgctggg gcgggcaggg acgggcggca cagcttggcc agggcagata acccccacct 15180
   tggccagggc gaaggcagga cacgtgggct ccagcctggc cccaccatcc ctgcacaaca 15240
   ctgggcaaag tccacgtttt cctcaactgg gtgttgacat ctgcaggaca ggggcatgga 15300
   ggtacagagc gctgaagcca cacagcaacc taggagcgag actccatgcc tccccgggga 15360
   cccctcccca ccatgaggac catgaaggct tcccatgtgc cgcaaggact ctggtgtgga 15420
   gacacacgtc tcctacacag ccaggcctaa cgctcttgta actgggtggt cccacctggg 15480
   ctcacagctg gagggccagg agctcaaggc ttcgcagggt ctgctctcat cccagaggcg 15540
   atggggagcc acagcaggct gcaggagaga gggtgggccc cctccacttc agaggcccca 15600
   tctggcccac agactggaga gcacatctct cagcaaccac ggagcgccaa ctgcgcacag 15660
   ggcctggtcg tcagagcggg gcaaaggcac tgaccgtcac ggccagggcg agggaagacg 15720
   ggtgggcagg gaccttgggc agagggggaa gaacctggtg cccaggctgg ccctgccttc 15780
   agcagtgaag ctgagtgggg aggcgctgat gcagggggcc agaaagggct gctggtcagc 15840
   cgggaggagc cccccacaga ggaagcagcc agcccagacg cagatggcag ggtcccctca 15900
   acaatgtcct ctgaaaagga gaggcgggga ctgctctggt gacacctaca aatagatagt 15960
   cagccctcag ccccctgcca tacttctgac aaagcagagg cccccagggg aggcgcaccc 16020
   gaaggtacct gcacctgtcc cccagactcc tagagcccac ctgaccccat cccaccaggg 16080
   ctccagctac aaaataaatg ccgaggccag ctaggcaagg acgcacactc ggtaccgact 16140
   gaataggctc cacgttgtca tgagcgcaac ccacaggcca ccaggccaca ctatgcagag 16200
   ctgagatggt ttcggccaag cagcctctca gctgagctga acaagtccag agtccccggg 16260
   gggtcgtcac tatggagtaa caattgcgat gcgatggtaa ccctaacagc taaccgtcac 16320
   tgagccaggc cctgagctag gtacttttca acgctgcctc tctgcagcct caggacgagc 16380
   ctgtgggagc ataaagatca ttccctatca cggatgggga aactgagctc tgaagcagtt 16440
   aacgtgcttg tcccagaccg cagagctagg agcaggacac aacagcaggt caggcaggaa 16500
   cgggtgaggg gggcctgcat gggcttctct ggaggctgcg catacacgca acccccagga 16560
   ccccgaccct gcacctgcag ctcgctactg ccccctcagt gactccagca aacctcgggg 16620
   taggggaagg aggctgggaa tacctcgggt gtccgaaaca gcagcttctg cttggaggcc 16680
   actgctgcat aatggttgct gcccagcaca ccccaagcca cctgtgccac ctgtggtgac 16740
   cttccagcat gccttggtga ccaagctggc cttaggtgct gtgggcagcc aagaatagaa 16800
   cagggcccac ccctcctctt cacactaaca caaagcaaga ggcgggcact tcgactgagt 16860
   gcatccctct agctcaaggg cctcacggat cacaggggtc agggcaagat cccaattctg 16920
   cattcccgtc tgcctttcat cctgctctgc caacaacagc cagtgaggct ggggacatcc 16980
   ctgaacctgt ttctcacctg aaacacatca taccattgga ccccagccct ccgggagagg 17040
   ccctaatccc tgactgtggt gagatcagat cactggttaa gtacccagaa gggccttggt 17100
   caggggctcc aggggtgggg ggtgatgggc gtggtggtat cccgctctgg gctatagtcc 17160
   accctgatgg aggaggtctg tggtcagaac cgggctgtgc agggcacagg agcccagagg 17220
   gacccccaga gctcacctgg tggtctctga gcagggctcc ctcaaccctc agagaaaagc 17280
   acagcaagga ggccgcccag agcccagcgc ctagcaccca gtggcgtgcc agacctgcct 17340
   ggatcctgga gatctctcat caccctccaa gtcagtcatg cccaacccag ggacccacag 17400
   cccacggggc cgtgaaggtg tgctgagtcc aagaaggcct tcgacactgg gaagccaagt 17460
   ggcacctcct ggtgtggagc aggcggaatc ccaccagcct ctgctctgcc agtgggcaca 17520
   gctggacgat gagcagaagg ggctgttgct taataaacgt catttcctta agaggataaa 17580
   acctttcaaa acagatggaa attttttttt aattaaaact ggtggccaaa gagatggaaa 17640
   gcaccccttg tgcctccctc ccatcgtgac ccatcctctg cacacctcaa gctgttcgct 17700
   gcccaggtgt ctcctgaggc actgggggcg ggtgagaatc cgtgagccct cggccagccg 17760
   tggctctctg gagctctgcc ccaggccatc agggcacacg ccgggcaccc tgggggccac 17820
   acagggcaga gcccagctgg gtcagcacac agggccacac tgggcacaca agtctctgag 17880
   cctcccctgt ggacgcagct ctcactatcc caccccacta ggtcccgggg atctgtccca 17940
   cagggtgata tgctgtcaca gaccactacc agagccatgg cctgctgttc cgcccgcagc 18000
   caggtagtca cttgctccac agggacaggc aacgccgcac ttgggggctg ctctgcggca 18060
   ggactagagc tccagcagct cagccctcct gagaaggaga actccatgct ctaagaggca 18120
   gacgcagcgg acggcaccaa agccaccaca agcccacggg gccctgcatg gcaggtcagg 18180
   agtccctgac cactcgctct ttgtaaccag agctgcagtg gagtctacga ggcaaggact 18240
   gtgggcggca gtggccacag caaatgaatg agtgtcccaa gggagcaggc ggctgcgggg 18300
   aggcacagcc gggacccagg agtcctccgg cactgcagca aactccctgg gccccctgag 18360
   cagcgaccag gtggcaagtg catgaactcc cgggggcata acctgggagg gtgacactct 18420
   cttcgtgttc aaattcttga gaacgcatta aaaatatcac tcagtcacct actctatagt 18480
   tttaactcaa aagtaccaaa gtagccaggc gcggtggctc acgcctataa tcccagtact 18540
   ttgggaagct gaggcaagag gatcacttaa gcccaggagt tccaaatgaa cctgggcaac 18600
   atggagggac cccatttcta caaaaaaagt gttttaaaaa attacctggg cctggtggtg 18660
   tgtgcctgta gtcccagcta ctcaggaggc tgaggcggga gaaccacatg aacccagggg 18720
   aggtagaggc tgcagtaggc tgtgatggca ccactgcact ccagcctggg taacagagtc 18780
   agactctatc tcaaaataaa tttaaaaagc accaagccag gcttggtggc tcacacctgt 18840
   aatcccagca ctcagggagg ctgaggcaag tggatcacct gagtcagaag ttcgagacca 18900
   gcccagccaa catggtgaaa ctccatctcc actaaaaata caaaaattac ccaggcgtgg 18960
   tggcgggtgc ctgtaatccc agctactcag gaagctgagg caggagaact gcttgaaccc 19020
   aggaggcaga ggttgcagtg agccaagact gtgctactgc actcaagcct gggagacaga 19080
   acgagactcc atctcaaaaa ataaataaat caatcaaaac caccaagact ttttaatata 19140
   aacatttatt attccataat tccttttttg catgattaaa aatgtttata taaagtttcc 19200
   tgaaaatggt aagaatgcca agtgaaggct gcaaatgccc aagcccccac cgtggcatct 19260
   cacggagtct gggccctagg aggctggtgg gtaccacgtg gacccgagac ttcacagtca 19320
   agtccctttg gggtacactg ggtttcccac accccagaaa tatgggctct tactgcagga 19380
   ccatgggggt cctcacactt ggcccagaag ctgtcacata gccagacagg tgttctacaa 19440
   cctaggctag agggagctca tgctccagca gaattcgagc cagaggaggt aaaagatggg 19500
   taagatctgc tccctggaca gatgaggcct tggcctcaga acagttactg atcatctacc 19560
   agacatcaca ctagaggcag aggggcgcag acgaagacag cccctgtcct caaggccctc 19620
   ccaggttggg tggaccatgg aaggttccag acagatctgg caagagaagt gcccacacca 19680
   ggggcagaag atgggcaggt ctgctcaggg cggcacggcc tgccaggcca aaaagttcca 19740
   acttcagatg ctggagaatg ggcacgactg tctgagaaag ggaaggatgt gatgaaaact 19800
   acttggagaa aaattaatct ggccagagca taagataaat gggcaaaggg gaggttccag 19860
   aaagcaagga gaccaagtaa aagctgatgt cattggctct gaatctaggc tttcactgaa 19920
   tatgcaccgc agggcctgta ggtaaagcct cagagcccag ggagtctgag tggaggagag 19980
   ggcaggggac agagctgggg cctgtgtcta cagtgctcag gaggaatagg catggacgtc 20040
   agctcggagg ctccagctga agtgaggagg cggccagggc agcacggcca cgcccggatc 20100
   cagactcctt ttgggaagca agttcgctct gggggaaagt ttggagaaat ggcctttacc 20160
   cgcagaagca agccccagaa catatcttgc tccaaaacta tctcgtacag tgaggacgtt 20220
   aagcttcagg tcccctagag gagacagtct gctccttcct ggggcagaac ccaaggtggc 20280
   cagagcctgg aaggcaccca gcacccaggc tggtgtgttc cagcccaggc cacacgctca 20340
   gatagctatt aatgccccgt tgagcaattt cctgagagct ttgccaggca ggtaccgcct 20400
   ccccatctga actaatacag gggtacatcc caaggaagaa atgaaaggtg cccacatttt 20460
   gctctgggat taactaggga ggggagtgat aattaactca gtaattatat ttgccatcgg 20520
   gctaatgcta aaattagtgt gcattagaat ttctttcctg agcagacacc ggagtgagtt 20580
   gggcagcagg agtggctcgg gcaagtcggc acaaagggca cctccagagc cttccacaaa 20640
   tgtcagcaaa acccacaaat gtcaaggccg gctccactgc acccagcaga tgaattcact 20700
   tccacagcct gagaccgcca gctcatcgga ggccatttaa aatccagccc tctgacacct 20760
   gctggatatc accatttacc gtccccagat caagagatca aagggtggaa cctgatagga 20820
   cggctctgaa gttcaccaca aaagcataaa cgtgcaagca gagccaatac gtcttttgaa 20880
   aaggacaatg aggtgggaat ttacataact gatcttaaaa tatgttctga tgcttcagag 20940
   atggagacag cagcattccg gtacacaaag acactcacag gcagtggagc acagtgaagg 21000
   gtctggaatc aggacccagg tgtctgtgga cactacacat aaaagagcag catttacaat 21060
   gaatggatag gatggaccat cccaccaagg tgttggacaa ctccctattc actggccaga 21120
   cccctacctc ataccatata caaaaaaaaa aaaaaaaaaa aaacccagac agaataatgt 21180
   ctgaatgtaa aacataaaac agtaacagtc ctggaagaaa ataatggagg atatatttat 21240
   aatctggaga tggagtaaca agggatagga aaaaagccat agggaaaaag tagagttatg 21300
   attatatgaa gcttcttaat atctttatga taatgtacca ccagaaacaa ggatgaagga 21360
   ctagctacag accagcagtg aaacctgaaa caaacagaac aaagaattaa agtccatacc 21420
   aaataaagac ctcccacaaa tctataagaa aaagataaac aggctggcac cgtggcttat 21480
   gtctgtaatc ccagcacttt gggaggcgga gatgggtagg tcacttgagg tcaggagttc 21540
   gagaccagcc tggccaacat ggtgaaaccc tgtctctacc aaaaatacaa aaattagcca 21600
   ggcgtggtgg cgcatgcctg tagtcccagc tacttgggag gctgagccag gagaacagct 21660
   ggaacccggg aggcagaggt tgcagtgaac caagatggca atcgcgccac tgcactccag 21720
   cctggaggac acagcgagac tctgtctcaa aaaaaaaaaa aaaagaagaa gaagaaaaaa 21780
   gaaaagaaaa agacaacaga aaaatgggcc aaggataagt gtaggcaatt tgcagaaaag 21840
   taaataccaa taaaccagaa atgagggttg tgcaaatcaa aaggtgttat aatttttaac 21900
   caaactggac caaagaaaac accaaaaacc aaaatcttgt aattgccagc atcagagagg 21960
   atataggaaa gtgtgtgttc tcgtagatgc ttgcaggtat gaactgctac agccttttag 22020
   gagttatgta tgtatgtatg cttgtatgta tgtatttgag acagggtctc gctctgttgc 22080
   ccaggctaga tctgttgcag tgctgtgatc atggcttact gcagccttga cctcctgagc 22140
   tcaatagatt ttcccacctc agcctttcaa gtagctgaga ctacaggagt gtgcaatcat 22200
   actcagctaa ttttttaaat tttttgtaga catggggggt ctcccaattt tgcccaggct 22260
   ggtctcgaac tcctggactc aagtgatcct cctgcctcaa cctcccaaag tgctgggatt 22320
   acctggatga gccactgtgc ccggcctcaa tatctttaaa aacagaaatg gacacactct 22380
   ttgactagga atgtatccta taaaaacact tatacacatg cagagacaca cgagcaagca 22440
   tgctttgtaa tagcaatgaa ggctggaaaa actcctcaat caggtaaatg ctgtcaagtg 22500
   cacctgtgta ctatgaaatg gcacttggct tttaacaaga gcaaagacag aaaagcaaaa 22560
   gtacaaagta gggtgtgatg gcacatgcct gcagtcccag ctactcagga ggctgaggca 22620
   ggaagatcct ttgagcccag gagttggagg ccaggagctg ggcaatagtg agaaaaaata 22680
   aaattaaata ataataataa taaaataggc tgggcacagc ggctcatgcc tgtaatccca 22740
   acactttggg aggctgaggt gggaggatcg cttgatccca ggagttcaag gccagcctgg 22800
   gcagcaaagc aagacaccca tctcaacgac aaattttaaa aaatcagcca ggcaggctgg 22860
   gcatggtggc tcacgcctgt aatcccagca ctttgggagg ccgaggcagg cagatcactt 22920
   gaggtcagga gttcgagacc agcctggcca acgtggcaaa accctgtctc tactaaaaat 22980
   acaaaaatta gctgggcatg gtggcagatg cctgtagtcc cagctactga ggcacaagaa 23040
   tcgcttgaac cagggtggca gaagttacag tgagccgaga tcgtgccacc gcactccatc 23100
   ctgggcgtga gtgagactcc tgtctcaaaa aaaaaaaaaa aaaaaaaaca aggagccagg 23160
   cacggtgggg tgagggaggg cacagaagca gcgcctcttc tgggggcacc cccaatctct 23220
   agcgatccag aggcctcagg atcctgaagg gagaaaaaac gtgaagctcc gtgctagaag 23280
   agaccataga gattggaatc agctggttct attttacaaa aaaaggaaac tgaggccctc 23340
   agaaggtgag tgcctctcaa tgccccacag ggaggcaggg agagggctct gagccctgca 23400
   gggccctgga ttcttgcaat ggggtggagt ggagcctgtg ccgcccccac caggcacctt 23460
   ctcaggagag gagccgttgt catatccttg aaggggtcct tgagcccctc aaaaggctaa 23520
   aaaccacttt cctccttgag tgaaccttca cctcagttta accacaagaa aaactacatt 23580
   aaggcccagc gcagtggctc atgtctgtaa tcccagcact ttgggaggct gaggtgggtg 23640
   gatcgcttga gcccaggagt tcaagaccag cctgggcaac atagtgaaac cctgtctcta 23700
   caaaaaacaa caaaatcagc tgggcgtggt ggtgcacacc tgaggtccca actacttgcg 23760
   ggctgaggtg agaggattgc ttcagcccag gaggtagagg ctgcagtaag cggtgactga 23820
   atcactgcac tccagcctca gcaacagagc aagactcaaa aaaaaaaaaa aaagcaggcc 23880
   gggtgtggtg gctcacgcct gtaatcccag caccttggga ggccgagcgg gaggatcagg 23940
   agatggagac catcctggct aacacggtga aaccccgtct ctactaaaaa tgcaaaaaat 24000
   tagccgggcg tggtggcggg tgcctgtagt tccagctact caggaggctg aggcaggaga 24060
   aaggcgtgac cctgggaggt ggagcttgca gtgagctgag atcacaccgc tgcactccag 24120
   cctgggcgac agagcaagac tccatctcaa aaaaaaaaaa attaaatctc aaaaaaaatt 24180
   acattaaggc aaactaaaag atgtttaaaa tatatatatt aaattaaata cactccaata 24240
   gagcaaatac gaaaataccc agaaaacaca atccccgcac ccccaggaca acctcccagg 24300
   gggtccacag caagagaccc caagcacgag agacagagaa cagtgtccct gtggcggaac 24360
   ctctggccca tcaggctcta ttagaaaata aggctcttgc cactgagaga aagaggcaca 24420
   gtcgcccagc agccacgggc tctggcacac cacgagtcag gccagcaaag tgtcaactgc 24480
   cccctacaag gtgacaaact aggacaaact ggaaaccaga ggctggacct ggagcacagg 24540
   gaccaccaca tggggctggg gaatgggcag ggacctcaga gcgccaccca catgcctaag 24600
   agcagcgcgt atgcgcatgc ctctgcatgg cttagggaca cagggagctc cccccacccc 24660
   caacccagga aggcagcccc cactacccag gtagggaacg gataggacca gcaccccgtt 24720
   ctgctcgtaa ctcagggctc caggccccct cgggggcaac cagcacagag ctcagacccc 24780
   aaatatcttc acccacctcc tggtccccat ctggacaagg gtgctgggga ctggctctca 24840
   gtcacaccct cggggtactc ttcaaaggac agctggatgc cccagggcag gagcttttgg 24900
   cccccagctc cctcacccca gacaccagct cttgggaccc caccagcatg ggcaaggtgg 24960
   acaccatcgt cccgattttg cagatgagga aactgaggct gagggctggc acacggctct 25020
   ccagagctga agagaatgca gagagcagcc ggagccagcc ggtgggtccc tgaggccggc 25080
   tcgtagcaag ccacagctgc ctccgcccat cacacttgga cctcactggc cccaggacag 25140
   ccctccaggg cggcctggca cagagcccac accctgctgc ttcctgaaca aataagtgaa 25200
   caaggccacc aagccgagga cctggatgta gccccggctc ccgccagggc ctccccaaca 25260
   gactccccat ttggagagcg cattaagtgt ttccaaagcc tcacaaacca cagatgtccg 25320
   gctgtctcac ggcttctgta acctgaactt ggccctcact ctgccctccc agcactcctc 25380
   tcagggccca ggcccctcct ctgagatgcc agcactgact ccccaacttg tccccatcac 25440
   ctggctcgtt cctgaacctc ggcaggagag tctcaggcca gatcctccca ccagccacct 25500
   ccaccaggat gcaggaggca tgagacctgc tcgtgccggc tgggagatgc aaccaaccaa 25560
   gatcaatcca atcagcggat gaactgacaa atataatgtg gtccctccac acaatggaat 25620
   attattcagc cacaaaaagg gctgaaatag gccgggcgtg atggctcaca cctgtaatcc 25680
   cagcactttg ggaggccgag gccggcagct cacttgaggt caggagttca agaccagcct 25740
   ggccaacatg gtgaaatccc gtctctacta aaaatacaaa aattagctgg gcgtggtggc 25800
   gggcacctgt aatgcaagct acttgggagc ctgaggcagg agaatcactt aaacccagga 25860
   ggcagaagtt gcagtgagcc aagatcgcac caccgcactc caacctgggc aacagagcaa 25920
   gactccattt caaaaaaaaa ataaaaggct gaaacaccca tacgtggtac tacttggatg 25980
   actcctgaaa acgttacagt aaccaaggaa gtcagccacg aagacgcatt gtaagattcc 26040
   cttcatgcaa aatgcccaga acaggcagaa ccacagaggc agaaagtcga ctggtgttca 26100
   ccaggggatc cggggagagg gaacgggaag tcaccgtgta atgggtatgg gttttatttt 26160
   ggggtgatgg aaatctctta taacttgata gaagagaggg ttgtaaacac tgtgaatgta 26220
   ccaaatgcct gccttctata ctttaatatt ttatattata taagtttcac ctcaatttaa 26280
   aaaaaaaaca actcgacacc tttcacctag gaaagatctg gctttagctt gcatttcctg 26340
   taactcctgc ctaaagcctt ccagaagctt ccgctgcctt gtggatcaca accagactcc 26400
   acaccatgat ctggcctcta agggcctctc gcaggacacc ccgagggtga aggagcaccc 26460
   gtgggcccac ctctgcatag ctgcaaagct tctttccctg tcctcccctc tacatgggaa 26520
   gctctgcccg caggggcggg gccttatctg ccattctatc gcactcaacc ctagcacttc 26580
   actcggtagc agacaccaaa gcaaaacagc aacagcatta taccgggcca ggtgcacgtt 26640
   aactcactga attcatggta ggaaggattc tattcccatt ttacaggtga gaaaactgag 26700
   gcacacaaag gtagcatcag cttcctaagc ctcccagcac aggaagcggc caggctggaa 26760
   tcagaccctg ggcgcagggg ctctgtccac agtgctaact aactactcct gcccccgagg 26820
   gctgcagcgg tgagtgagtg agtttgtcag tggactggat gtccaaggtc atacaggaaa 26880
   aatccagact attgtaataa cagcctctag accggctggg gccagaaaga tcgaggacgc 26940
   tgacacacaa ctgcgctcac tgcagctctg ccagggatgg ggctaaaggt ctcacacagg 27000
   gcagttaggg ctccccatag cctgggagag gaacggggtg agataacaga aactaggtat 27060
   ggtgcccgaa gtcaaacagc cactgagcat gtaaacccag gtgggtctga ccccaaaccc 27120
   ctccaccccc atcagccctg caacccgtcg ctgcaaggga gaaagcaact cagaggcctc 27180
   acctgcctac atcccccacc cgtgtgtgtg agttctacta aatgcctgag cagtgacaca 27240
   gcacggctga aattaaacgg gttccaaaaa cgacaggaag cacgaagtga atctccccag 27300
   gaaagtgctg aacaaatgct ggatcgggtt caccggcgaa tttcttggaa ctgaagaggg 27360
   gagctaaaca cacggggccc tgctttggag gggactctct cagggtgctc cacacagcac 27420
   ttggttaacc ccactcagcc cttctgggct ctcccagagg gcccggcctt ggccttgggc 27480
   atctacagga ggaacctcca gggggagagg gggtgcctgg acaggccggc cctggaacaa 27540
   gcacttgggc cccgaggaga gaggactagg gcttgggagc tggggaagtt ctcagcactg 27600
   ggaccactag aacaaagcca tttccgtgcg ttcacagctt ccaattgcaa caggaagcaa 27660
   tcaggaaaaa taattagcgg cccacttact ggcttcgctg aggtccgagg catgtatttc 27720
   acacagtaaa accagggata taacatcaaa accgttctgc agaaagattc ctccctttcc 27780
   ttccatttta ggcctggatc accacattca ctggggctcc caggccttgc tgcctaatgt 27840
   taaaataatc aactctattt ttgcctcaca cacaactgaa ctctacagct ataattcttt 27900
   ctcctcaggg gctcgaacca catggacgac aggcatttga ctccagcaac atcaccccaa 27960
   aacgtgcaca aaacccaaaa ctgcaatgag gtgaaaggca acgcggtcgg cctagaaacc 28020
   ccccctttaa aacaaacagt ttccccaaaa ccccttttgc ctccttgacc caggcatttc 28080
   cggaaaaagg agcggcgctg gcctgtactc cccagatact gtcgctgttt tgtcttcacc 28140
   ttgttttgct agctccagac aaggccccac aatgtaaaca cgctcctgaa agaggcagat 28200
   ttggggtgaa actgtccata gaatctctag gcttgggtca gaggcaggag gacgtgaaac 28260
   aaactccaag ctcctcctgt tccccgctgt cccccacacc tccaagcaga ggctgcagcc 28320
   tgggggatct gactacaggg ccaccccgct gcaccattca cactggaaat attcagggag 28380
   acagctgttt gccttaagga ggcccagaca aaggggcccg aggtcctccc cgctaaactg 28440
   ccacaaacag aacaggagcc gcggcgtgca caggcacttg cggccgtgcc acttggccag 28500
   ccatactcca gaaaaacaaa acacgcacat ccgaagagaa tgatttaggt agcaagaggc 28560
   ttgcttgaaa aaccacatgg caatctccaa attaaaagaa catgtgtagc gtttcacgac 28620
   tgcttaagtt tcctgagtcc tcctgacctc aactccaccc cctgggaaac accaaaagtt 28680
   ggatgagaaa gttcccccgc cctacctctc cccacgggag tgtacaactg aggcacaagc 28740
   ctgcctcccc cactgccccg cgatctggga ccacgtctcc tccgcgtagc cgacccgggg 28800
   atggacacta tctggggacc cggcggccac acggggcatt cgggtcgccc gggcacctgg 28860
   caggtgtcag tccgcttgga aacccacagc cacgcggctc acaggagcag cgccaccggc 28920
   taggccgccc cgcgcccggg ctcagaactt tctcgctgcc acttcagccc gtcctcggag 28980
   cacgcggggc ggccgcgcgg ccgctggaaa caggcttgcg aaccggctcc ccgggccagg 29040
   cccgcctccg cgccccaagt ccccgctcgg tgcccggccc gggccacacg ggcccagcgc 29100
   gggctcggct cggctcccgg cttcccgcgg gctcgggcag gtgaggaccc gcccgcgccg 29160
   cacctggcgg agcgggcgcc ctcctcgcca gcccgggacg cagcgtcccc ggggagggcc 29220
   cgggtgggga gacaaagggc ccgcgcgtgg cggggacgcc ggggacggca gggggatccc 29280
   gggcgcgcgc cccaactcgc tcccaactcg ccaagtcgct tccgagacgg cggcggcgcc 29340
   cgcgcacttg gccgcggggc cgcccgggcc attgtccgag caacccgcgg cccgtcttac 29400
   acgccgggcg cgggaaggta tcgaatcagg 29430
<210> 8
   <211> 33769
   <212> DNA
   <213> Homo sapiens
<220>
   <221> unsure
   <222> (33739), (33749) , (33758)
   <223> Identity of nucleotide sequences at the above locations are unknown.
<400> 8
   cttcccctta cactggtcct tcgacccgcc tcggatgaaa actgaatggg tttagcctta 60
   gaggctctcg gtctctaagg gaggtgggtc aggatgccgg ggacagggtc ctcttcctgg 120
   ggcaacgtgg gggaacgagc cacctacccc tccactgaat tgccctgggg tgtgggtacc 180
   gacggctcat tcggtgtcca gggtctgaga tgtgttgaca ggaagaatga aaggggatgg 240
   gagggatggg gcgaaagaag ccacctgcag ccccaggaac tatctggcca gcacaccgtc 300
   acccagcggc ctgagccacc cctgccagag ccaggaggag accctgccaa tgggtcacca 360
   gtgtgcagga actcagaagg tcatcacagt taataccctc catgccccaa tgtgggaaaa 420
   caggtttttt cacaacaaac aagataattt ttgttatttt ggcaaaagga ggcagggcag 480
   ccccggacac ctccatccca cctcatcacc cagccgcagg gccccggcca tccctgcaga 540
   cagagtggat gtcacaacct ccctgcaccg aaccaagtgc agctcccagg ccacaggcca 600
   cccaggaaag gtccagtggc ccccggaggc tcccaccgca ggcctcccac cacagccggc 660
   accaacccag gatagctgtg ttctcctggc ttcttttcac acgggtagca gaaagctgag 720
   atccggggaa agctgagatc cagggaaagc tgagaatcgg cctctgctgc ccggacgccc 780
   acccccagct ctgctcccag ctccagggcc tccttctcag gtgcccttac aggaggcaga 840
   gggcttgagc cacctcctgg gcctggggca cgcaggatga acggggtcac ggtgcaggcc 900
   actgtccact gcgcagatcc caaggccata aacagcctgg ccacagtggc ttcccagctg 960
   gcaggcggcc agattatttt tgttgtttag caattgatta agtttctccg ctgcccccag 1020
   gggtaagtgg tggggcaaat gccgcaaccg cagcatttga cccgggatcc tgtgccaagt 1080
   gaccataggg tcacaaagca caagggaagt ggctgggccc gatgctggct ctgctggaac 1140
   ctgaggccgg ccactgtcac ctgcacggtg cctgggacct tccagcaagc acagagaagc 1200
   tatggccctc caggagcagc tggcaggcac cttggcctgc agtcaggggc tctgtctgct 1260
   cagctctaaa acaggaaagt cgctgctctg cctggggtca gggcagccag agagtgacca 1320
   agtcagtgcc ggcctcagga agggacctgc aggcgggtcc cttcctctcc catccctcgg 1380
   tgccagccag cccctcctgt ggccccccac tgcctgcctc tgcccccatg ccccaccaca 1440
   acctcaggcc catggctgca tggccactcc ccaggcaggc agtggggatg ggatttcacc 1500
   atgttggcca ggctggtctc gaactcctga cctcaggtga ggagttccta aagtgctggg 1560
   attacaggcg tgagccaccg cgccagccct ccctgtggta ctaaacactc acaccccctt 1620
   gctggggacc ctggtgaggg aacacagcct cacaagtgaa gtgtggtttt gttgagcaaa 1680
   tgacgcctgg gcagccctct catctttgcc taaaactgaa gaatttaggg gcgtggatgt 1740
   ataaaacagt tggtgactta aatgaaaaag aaggccacac tccccccttt aggcaggcgg 1800
   cctaattctt taaaagccag cacagggtgc ctttctgaac ccaggcacac agtaggtgtt 1860
   caatggacag cagcggttac ttgtactgct catgacaccc tgtctgtggc ctctgcagct 1920
   ggctccagcc tgacgcatgg ctgcgcccct ccgcaaggcc accccggtat acatggaaac 1980
   tctgtggaga aggccttggg ggccggccag gacgccaggc ccagatccca tctgcgccct 2040
   tcctccatag acctcagcga gctctcggca ccatgtgcct caggcccatt taagaagtag 2100
   ggccggccag gcatggtggc tcatgcctgt aatcccagca ctttgggagg cccaaggtgg 2160
   gtggatcacg agatggtcag gagatcgaga ccatcctggc taacacggtg aaaccccatc 2220
   tctactaaaa atacaaaaaa taagccgagt gtggtggcgg gtgcctatag tccaagctac 2280
   tcgggaggct gaggcaggat aatcgcttga gctcagcagg cagaggttgc agttagcgga 2340
   gatcgcgcca ttgcactcca gcctaggtga cagagagaga ctctgtctca attaaaaaaa 2400
   aaaaaaataa aaaaaagaag cagggccagc cacggacgac ccctcacaca gctcccagga 2460
   cgcgtgcctg ggtatagggc tcaggaccat gaccgctgca gtggccccca agaaacgtta 2520
   cttttgtcac ccaccccgcc tcagtggcag tagccaaaat aacggattag aatggaacca 2580
   tgtgacaatg ccactgcccc aactgacaga agatggctat cagcagttca cgcggcccca 2640
   cctatcacaa gtgcagggca ctctacaact tatgcatcct tccccagaca ccgtcctttc 2700
   gaccctccca ggtcagcaag gcacacaggg cctacatttc acagccacac agcagagggc 2760
   tgaggctgga actcggatgc tctgatttcc gttcaatcac atccccagag gtggcacaga 2820
   gacggggggc ttctcttgac aaagtcaaga aagtcactgc cagctccact gaagaccaaa 2880
   gaacctcagc tctcaaaccc tcttgaaggt gttaccgaac tctcccagcc tgtttcctgg 2940
   gtcccgatgt tggtcccgtg ggacacagga agaggaagaa gctccctaga gcagagcctg 3000
   gtgcacctgc cacactctca gagggctgcg cacgggcgga ggagccgtgt gcaggagtgg 3060
   ggtctggatg gaggggcgct gtggccgggg gcagggggca ggggaagggt gctccaggtg 3120
   gtgggcacag cacgagcagg ggcagggagg tccacactca gatgtgcaca gggagaaaca 3180
   aatcgtgcat ttccattgga ataggcggta aaaggtagaa aaacagagtg ggggccagga 3240
   agggagtcgg agccttctag tgtctctctg caggtgagcg gcagcccgag gtgtcagctc 3300
   agcagacttg gggtccaggg gccgtgtctt ctatcactga ccccagggca cacggaactg 3360
   gggagggaga gcagaggcac agggcacggt cagtgaaacg aaacaaggag tcatcaccaa 3420
   atgcggaaag ggcaaggagt gcccgcagcc gcacaagggt tctgtctggg caacgtgggc 3480
   gtcccaccag gccccgcacc ctgcaagcgc aaagctcgcc actgaagata aagggaagct 3540
   gttggagctg cggagctggt ctggggtccg catggagctg ggcttatgct gcagtcacaa 3600
   gggggacatg gaagaggctg caggggacaa aaccagtgac cacagtctaa ctctgagcct 3660
   gtggaaaggc gcccacagca ttcacccatc ccagagatgc cattccccct gtgcccccgc 3720
   tccacggtga cagcgttctc caggaatatg atgcgcccct ctcctcttgc atcagccctg 3780
   acagtgagta ttcaggccaa aaagcagaag agcacagctg cgtggttcca tttccatgta 3840
   gttctggaac aggcaacgct aatccaaggt gatagaagtc aggagagtgg tggagggggc 3900
   gggggttgag gatggcaaag gggcaccggg aactttccca gtggtagaaa tgttctctgt 3960
   ctggaccgtg tggtagttat gcagacatat gcagctgtca aagttaatcc aaatgtacac 4020
   gttaaaatgt gtgcgtttta ttgcctgcaa gttatacctc aattaaaaaa ataaagttag 4080
   cactcaggct tcttccacaa cttcctgaac cgtgtgagct gattttcttg ctattaaaaa 4140
   ttcacggtcc atggctgaga acagcagctg ccttctgttt gcaaagtcaa cgccaatcac 4200
   tgcccggccg cggcagactc ggccccacag gacctccttt ctttttcccc tttgacctac 4260
   ttccctgata agtgacaaga cagccagact ctgggaacaa acgcccgtta ttcggccccg 4320
   agctgagcgg gccctgcttc ctgagctaat ccgcccggac agacggaggg acgtgagggg 4380
   ctttgccgtc ggctccagct gtcagtctgc ccgtcagact cgacagtggc cccctctgtt 4440
   cctcccgctg cccccactcc atccccgact tctttttgtt tcctgtccct gacagacgaa 4500
   catctgttaa aactctgtct gggtgagctg tggccagcgg cccacaaatc cccaagccgc 4560
   accccagcct catctgggcg ctgccgggag cactgcctgg ccaccctctg gacatagctc 4620
   tgagagccac cggccagggc acgtgtggcc cgagtggcat ggtgcacgcc gctaagccca 4680
   ctgcccaaag gcccccaagc aggagggatg tgcaggagac aaaagtcaaa agaacagggg 4740
   cacgttccac agaggatggg gctggagggg tggcagtgag gaacagcagc ttccgaggat 4800
   ggcggtggca actcccaaat aaggcctcac tcctgctgtt tttagctcat tccacataat 4860
   tggaaaaaca tggcagaaac cgaagccagc tgctgccttg gtcctggggc tgtgtggagg 4920
   gggtggggag gccggaggcc caggctctgc actcgactgc tggggatgag agtgactctg 4980
   agctgcagag agcagcatcg cagccgccat ggtcccattg agccccggcc acgctgggcg 5040
   gcagaggctc gtgggatata cctgccctgt ctcatggggg tcacttcagg aggggcgggg 5100
   gagccaggac acagcccagg gctagcggtc accctgcagc tcaggggcca cgtaaatagt 5160
   gccaccttga aggcacacag cagtgcgggg ccccccccgc caccaacgca tccctacctc 5220
   taggaggccg cctgtgtgcc cctgggaacg ctgctccctg tcccttgggg tcctggtgtg 5280
   accaccctct cagccccttc cttggggaag gcacctgact ccctacaccc agctggcttt 5340
   catttgctca aaatcaggaa aaagcagaat tcaagacatc acagaaatgt cttcgcctgt 5400
   aactccatga aagataaacg gtcagacacc caggagggag tcccagggac ccttgagtct 5460
   cacctgaggc tctggcttca aacctcgaga tgtttccagc catgctagcg ccgcccccca 5520
   caacctgccc cacacagtcc tcccttggga actcacagat ttggccccca cctgccccgt 5580
   ttcttctggt ggagtgggtg cgttgggttg gggtggggct ggggactctg gatgtgtctt 5640
   aagagtctga gtgattctga cacagccagg ccctgccccc ctcctgacct tcgccccaca 5700
   ggaaagggag ccacacgcct gaagcgccca gcacaccccc ctccgtcctc cccaggtcac 5760
   ccgctggccg tgtgagccgt gctccccact gccccttcac ccaccccagc tcctcctggc 5820
   agcacccagc cttggaagct acttctgatt acaaccgccg aaggaagact cgctccctcg 5880
   gcactgaccc agacagcctg caccatcacg ctgctcagca caacccacac agccttcctc 5940
   caaaccccat ggagcgggga gtataatcac cccctttcta ccaacggaca aactgaagca 6000
   cagagaggtt aagtcacttt cctaagctcc caacacgatg acaaaaaata gaaggtcagc 6060
   ccgcaagtgg aactaggtgc tccaagtccc cggtctgcct gacactgcac ctcctcgccg 6120
   ccacggtccc gggtccgcct gacactgcac ctcctcgccg ccacggtccc gggtccgcct 6180
   gacactgcac ctcctcgccg ccacggtccc gggtccgcct gacactgcac ctcctcgccg 6240
   ccacggtccc gggtccgcct gacactgcac ctcctcgccg ccacggtccc gggtccgcct 6300
   gacactgcac ctcctcgccg ccacggtccc gggtccgcct gacactgcac ctcctcgccg 6360
   ccacggtccc gggtctgcct gacactgcac ctcctcaaca ccaccacggt cccgggtctg 6420
   cctgacactg cacctcctca ccaccaccac agtcccgggt ctgcctgaca ctgcatttcc 6480
   tcatcaccac agtcccgggt ctgcctgaca ctgcatttcc tcatcaccac ggtcccgggt 6540
   ctgcctgaca ctgcacctcc tcaccgccac ggtcccgggt ctgcctgaca ctgcactttc 6600
   tcaacaccac tccttggccg gctcccaact acaaaccaag ccatgtcttc catcctgaat 6660
   cctcttggcc taaacatcac tcacaatgcc tccctcggga acaggcacaa gtcccaccag 6720
   cacagcctcc ttcgttacct gcgtttccgc tagcccaggg ccagctccag agccctcacc 6780
   acagagcctc tatccttcac ccccggacac tggacctcac caacccatag cctggaggag 6840
   atccctgtgt gaccccaggg cctcctctgc ccgactctga atttcactgc ccaacgtgac 6900
   acctcggaag gctctctggg cactggcagc cctccatggg caccgctcct tctggccagc 6960
   tctgacatcc cggctggtga ggtgccctgc acgaggcctc tgcccactgg gacctcacag 7020
   ccgtgctgtc agctgcaaca agcgacagaa tttcacgttt tcttcacgtt gcccctgggt 7080
   gagcagctcc aggtagtttt cagtcgaggc gaggcgtccc gtcagcagcc aggcggcaca 7140
   gctaattcat gcccgccggg cgcacggccg caataccaat gggcacctgc agcctggaaa 7200
   gccacagagg aaccgagaac agcgactgtg ctcaggtgac aggactgtgg tcttttaaca 7260
   aaacattttc ctttaacgtg atattttacg gcaaggaatg aaacctggag ggcaggacat 7320
   ttggatacta aagccccagg ctgccgcgtg gtctgctttg tgaagtctga agcccgcgcc 7380
   ccattctggc cccgctcaca ggtccggctc tgactcacca gcttcaatgc taggccgtgc 7440
   ctgtcctcca accagaacat gacttcctta aggacaaagc cgtttctcgc ccatccccat 7500
   ctccctctgg attaagaaat atgggaagat cttctagaac cacctcaaat ttgcagagag 7560
   ccatcctggt gacaaaccct tgaaatgctt ctaagaagag tttaggtttc ttctcaactc 7620
   taaaacctct agaaaactct atttccacac cagctgcccc tggaacactt cagcttcaaa 7680
   agggcccagg gcagggagac ggaggagcca gcatccacac cgagcaccag cctgttaatt 7740
   aacgggaagc gggtggggcc catctccagg cagctctgag gtcagactgg ggaaccatgc 7800
   ttacaaaaaa aagtgaactg aaacgctcac gtcctcatgc aaaaccagac tcccagttgc 7860
   atctttctgt ctcattgagg agctttttcc tccctttgac agaacaccct acacacggca 7920
   tctggaacca aagcagaaag attcaggctc agagtaaaac agtccccaca ctggctgcat 7980
   gtggacgttc ccggcccaga gtctcgccca agcagggcct ataaatgaca caaaatgttt 8040
   ttctcctgcg tgccagtcat gctccaactg agttatgtgt aaaagtgcct ctcacggctg 8100
   agggcaaaaa cagttcccac aagactagag aaaggtgacc cctgacggct gagtctctag 8160
   ggagcgtgga gctgcgtgct cagccctgcg gccctgacgg ctctggaatg gaaaagctat 8220
   ccaactggaa gggcagggct cgctgctagt ccagcggtcc aaccccacag gtgtctgtgg 8280
   tgtcagctcc atgccacaga gcccagggct ggggccagag ccaccaggcc ccctgccagc 8340
   ctgcaggggc ctcctcctct gggtagccta accaccccct gtgagcgcag gcagcctcct 8400
   ctaatcacca cagggcctgt ccccccctct cccccgcttg caggaaaatg agccctgagg 8460
   actccccagg gctgctctgg gcctggacat ggagactggg aattacattt gcagaaggag 8520
   cgcaatgccc ttgaagggct cagccacgag cagccagtcc ccagggctca gaaggcccag 8580
   ctgttagaac cctgggagcc agcaaagagc caggggctcc acctaagtct atagcccctg 8640
   cctcttctgg ttgggaaaga aatcaacgcc cctttactgg ctcccactga cagcccactc 8700
   ccccaggtat gggaggattc tgggacgatg caggcaaacc tggaccctga gtgaacctgc 8760
   cccagctctc acgggcctgg caccagccac agcacctaag gcgccggtca tggtgacaac 8820
   atgaaggtga taagggcatg gacagtggac atggcagctg gacactgggc acccactgga 8880
   tgccaggcac ccagcacggc tccgtcaccc ctggatgagc agtggccctt tgcaagccag 8940
   ggtagcctgg gcaagttatt tgggggtctc caagcttgtc cagctgtgcg acttcactga 9000
   gccatgagtc tgggatttta tcagggccca cacccgttcc tggaactctg atacgtgagg 9060
   gagccacaca gggaccctta acaaaagctc ccagggcaac atgttctctt gcctcagtct 9120
   cccaaatagc tgggattaca ggcgcacgac taccgcccgg ctaatttttg tatttttagt 9180
   agagacaggg tttcaccatg ttggccaggc tggtcttgaa cccctgacct caaatgatcc 9240
   ttccactgtt agggcaaggc acctgacagg cacgactgca cgatctgctt gttgggggct 9300
   gtgtccattc cccactcctt cgacaaatgt ccacacccag ccttgctttg acaccccaag 9360
   aacagagatg gtgacacctg cttcctacat gcccattgct ctcccaaggc agacatcccc 9420
   agcagatgca acacagtgtt taggcagaca tcaccaatcg atggtggcaa cagacaccag 9480
   gccctgctcc ctctaactcc agtggccagg ccccaagcca gctctcacct gcccactccc 9540
   aacccacagc agcaagactc agaaatggca aaaacacaaa gagaacagaa acgccccata 9600
   gcgggaggat gactaaaaga catgtcttga taagatattg ttcaggcata ggccaggcac 9660
   agtggctcat gcctgtgatc ctagaacttt aggaggctga ggtaggtgga tcacctgagg 9720
   ttaggagttc aagaccagcc tagccaacat ggtgaaaccc catctctact aaacatacaa 9780
   aaattagcca gacatagtag cgggcgcctg taatcccagc tgcttgggag gctgaggcag 9840
   gagaattgct tgaacctggg aggtggaagc tgctgtgagc cactgtactc caacctggac 9900
   aacagagcaa gactctgtct caaaaaaaaa aaaaaaaaaa gatatccttc actaaaactc 9960
   atgtctttga tacatattta cctcctgcaa tcgcaaatgc ttctgcagtg cataaagtga 10020
   aataaatagc aggaagcctt acggttcgat cacccacaca gacacacagt cacatacagg 10080
   aaaaacgcag ggagggctgg ggaacaaaaa aacagaagat aaaatgtgga gacagacaca 10140
   ccaagagagt aagagaccac ctccagacct cccttcagct tctcaaacac acgagccggg 10200
   cccgttacag aatttgcggg gaccgctgca aaatggaagt gcagacagcc ccttactcaa 10260
   aaggtaggaa tttcaggtca acaacagagc tcacctcata tgactacaca ggtcacacag 10320
   cccgtgaagt cggtcccaac accagcatgc tcctgcctca aagccgctgc acgtgctgtt 10380
   ccttctcgcc tttccctctt ttagtccttc agatctcagg cctcctgaga gagacctctg 10440
   acctgccggc tcaggcggcc acacccccag tacaggagtc tccggctcag cccctgctgt 10500
   gttccgtacc cgatccaggt ctgtcctatg tccatctgtg tgccggcttg cttcctgaca 10560
   tggcccccac cacacgtgtg cctcggggca ggggaacagg cccgtctcat taactgcttt 10620
   cttctcagat attttctgga atatttgtgg atattgggca acatatatgc tccacctttt 10680
   tcagactagc caggacgagc tgcatttttt tttttttttt tttgagacag ggtctcactc 10740
   tgttgcccag gctggagtat agcggcatga tcttggctca gtgcaacctc cgcctcctag 10800
   gctcaagcaa ttctcctgcc tcagtctccc aagtagctgg gattacaggc ccgtgccact 10860
   actgcccagc taatttttat atttttagta gagatggagt ttcaccatgt tggccaggct 10920
   ggtcttgaac tcctgacctc aaatgatcca cctgccttgg actcccaaat tgttgggatt 10980
   acaggcgtga gccactgcgc ccggcccgag ctgcctgttt tacacctttg ccatattccg 11040
   gtgattctct ctcccctccg tcccccggcc ctgactgtgg tggccactcc ctgccgtcat 11100
   gagcccgtat gtcctcactc tttccctttc cgccaggact tcaaccaaca ctgcagagcg 11160
   cagggtccag ctccagcact gagttcagcc tcttctcacc aacagacagg caggaaagaa 11220
   aacaaactct gagaaggcca aggttcccgg gcagccagca agccaagcat ccttctccgc 11280
   tgaggcttgt gcagccgagg caccccctcc tccagggagc aggcagcgtc ctggggcagt 11340
   ctgcgaggga gaccagggcc cttgctccac cagggcccca ggtatggggg cagcagcaaa 11400
   ctcatggctc tgggagccag accccacctg ctagaaccta ctatgccacc tgctgtgggc 11460
   aaccccaggc tggtgacttg ccctggcctc ctctgtaaac aaagggctca tccaacctgg 11520
   tcaaaccact cctccccttc aagggtctat aatcctccct taacctgctt ggtccaaacc 11580
   cctggtgtcg ccaggtcact caggaggcag ctcatctgga ctccttccct gggtccagtt 11640
   tctctctcaa cattgccttt gaggccgagg tgaacggtca acagcgaagg gccccagagg 11700
   tgatggagga gcgggtgtcc aagacactca ccctttctaa tgcactgact ccctcgtgga 11760
   ctcacttgtg ccgtctcccc cacccaccca gccccagagc ccagagtgcg agcgccagag 11820
   gcccgggatt ctgtctgcac cgcggggtcc ccagtgcctc ggagcaatgc cagcacccgg 11880
   caagtgttcg acaaatgcct gctgaatgag caaatggatg gatgaacgaa tgaatgagca 11940
   agcagatgaa tgaatggggt gctgtccaga gccgtgagga ctaggccgcc caagtcccca 12000
   tttctcaaat tctccttctc ccgacttggg aaacaagatg cttggtcggg gaggctctcc 12060
   aaccatcccc tgcagcagcc ggcacagcgg acagaccctt tgatgtaaca gccatgtctt 12120
   cattaaagat gccctgctct cagaaagaga aagacaaata caaacctgga aaatcctcac 12180
   caaacgcagg acccctgcca gggagcagag aaaagaccca cacgccacgg gcgccacgac 12240
   cacacacaca ccccagccgc tgcacacaaa cacagaccct agccagcaag aacaggggga 12300
   ccaggaaact gttcctaaag tcaggacccc catgtgctca gacagcagtg agagcaagga 12360
   cacttctcca tccaccggat gccaggagag tccttttagg gggccccaca ccgagactct 12420
   gcccttagga ctgttcctga gtgtggaagc cagcccactt ggaagccccc tgccctcccg 12480
   agtgggacac cggcacagga agcaggccct gtcccccacc actttctgca agctgggccc 12540
   catcacgcta cagaaacggg gaggactggt cccagggatg gcgctttcct gacacctctc 12600
   gttaccccct cgcttgccag gccccagggt cagccccaga ggccagactg gctatcccag 12660
   gcccgggagc atccccgaag gcgagctgca tcctgaacgt gtgtgatttc ccgaagggcc 12720
   cgccccgaac cgacacctgg aaagaaagat cctcagccgg tgccccagag gagaagagcc 12780
   atgcctcact gcaacacagt cccaggaagc accaagtgcc tgaggaccaa ggcggagagt 12840
   aaaaaagtgg aaaatatctg gggcaaaaat aaaacaaaac aaaacaggat tgacctcctg 12900
   ggctcaagca atcctcccaa ctcagcttcc cgagtagctg ggaccacaga cttgaatcac 12960
   cacacccgcc aagtggatca tttcgaacgg gtttgccgag gttccttctg gggcaccccc 13020
   ggcggccgca acccattccc gccaggcccc gccccgcccg cccgccccgt cccgtcccac 13080
   cgcctcacct gccttacacg tcctgccgtt gtcctgcagc tgcacacccg tggggcaggc 13140
   gcatgtgtag aaaggctcgc ttggggacag caggcacagg tgggagcagc cgccattgtc 13200
   ctcctcacag cgagtgtgga ctgagaaaac caggacagac tgagagaagg ttccagaaga 13260
   ggaccgtcac ttgtttctga atgagtcaca tcctgcctcg tcccccgtga cagcctccag 13320
   tgtgtccctc tgcccaaaca tcggcctcaa gtggcatcag ggacctcccc gcgggcacca 13380
   ttccacctgc ctcatcgctg gccccgtcca catggggccc tcagcctggc cagacggcct 13440
   gcaatttccc caaaaccagc cgtgaccttc ctggccaccc tcacacccag atgtgacctg 13500
   cccatggagt gacatcctcc ccatctgctt cctcccacca agctcctatg actagaacac 13560
   cctccccagc tcctcggagc ccccaaagga cacccctctg caaaggctgc cccccacgct 13620
   ccaatggccg gggtcaggac ctgcctgtgt ggtagtgacg ggaaccccag agacaatggg 13680
   ctcctgggca aaaggcttgt cttgtctttg tgctatgtgt ggacccagca gcttccatag 13740
   gaacactgtc cttcttgctg ggatggccaa gcttgtcact ctcccaagcc ctcctatgac 13800
   caacagcaat tgaacggaac tcgataaatg cttccagcac ctcattcaaa ccaggggaaa 13860
   gctgggtgta gcagccccaa aatacggata taactggaac aacaaactca tcaaaatgaa 13920
   cctctccctc cctcatgctg ccccaagtgt agatgggttt tgtgaccacg actttctcac 13980
   caggaaacag ctccagagag ccccaccctc ctgtgtcctg ctctgggaac agctggcacc 14040
   cctaggcccc acatttcaat tcaaagtcca aaccttccat aatggcctgg ccagaaatct 14100
   ccatccctgg tccctgtggg agtgggccac tgtccccaga gccgcagccc cactgtcaca 14160
   gaagctggtg catttcccca tcagggacct ctgtcacaac ccagcgtggc ccccaggctg 14220
   agaactgctg attctgggca gattattcat tgataaatac gcgacttgca gggccaagca 14280
   tggtggctca tacctgtgac cccagcactt tgggaagtca aggtgtgagg atcactggag 14340
   cccacgagtt tgagacaagc ctgggcaacg tggcaaaatc tctcatctct attaaaaata 14400
   catacacaca cacacacaca cacacacaca cacatatata tgtatatata aataaccata 14460
   tatatatata cacacatacg tgtatgtgta tataaataca tatacacaca cacacagaca 14520
   acttcttctg ggccttgaaa acgaggcaac cttccttgga aatccccttg ccactgctga 14580
   gcctgaaata gcccccatga gctctgcaga ggggtcctct gcaggcccgt gtcccccagc 14640
   cagccacaca cctccctcca ttgcagcagg taccccttta gagagggggc cccccagagc 14700
   atgggcttct gcagggaggg gtcacctgcc cccccacccc acccacgccc gcgcaccccc 14760
   acgcccccgc atcctcccac tcccctgccc cgcgcccccg ctccccccag ccccctcacc 14820
   ctctcccccg tgccccaacc ggcactcaca aaaaggctgc cgctcctggc tcagcacctg 14880
   gatgtccatg ggtgagtata gggcactcag gatctccttc ctcttccccc cagtgcgctt 14940
   gttgcaggca tggatggagc gggtctgcca gtctgtccag tacagagtgt ccccggagag 15000
   cgtcagggcg aaggggtgcg tcaggctgcc ctccaccacc ttctgcctgc agtcagggaa 15060
   gcggggtgga ggagccatca ggagggtccc ccgacagtca ttgctgctga cccaattaat 15120
   ttcttttttt ttttttgaga tggagtctcg gtctgtcgcc caggctggag tgcagtgatg 15180
   taatctcagc tcactgcaac ctccgcctcc cgggttcaag caattatcct gcctcagcct 15240
   cccgagtagc tgggatcact gatgcccacc actacgccca gatgattttt gtatttttag 15300
   tagagacagg gtttcatcat gttggcaagg ctggtctcga actcctgacc tcaggtgatc 15360
   cacccacctc agcctctcaa agcgctggga ttacaggcgt gcgccaccat gccaggcttc 15420
   ccatttgctt tcaaccagac aagtgaggcc aggtcaagag ccccaggagc tggcgccctc 15480
   gtacatttct cccggcgtgc acagggcacc tcccaaacac agcctgtgat ggtgacacac 15540
   gggctccccc aggtcaagtg gcaaagtctc ccccagggaa gaaaggagga agccatgcct 15600
   ggcaaaaagc acacctctcc tgcccaacgc tttaacctct gtatacaaat caggccatgt 15660
   gcactcgctc cttcttacaa tgctcataat ttatactttc agagtaaatg aaacttggca 15720
   tcaacccgag aaacagctat tcttttctag atgcttacag tgcccagcaa atgaggactc 15780
   gggtgtaatg agattatgga cactggaaac aggatcataa tgtgacgtgg tcggtaatgt 15840
   gcagttttat ttgcttaatg accctcgccc cgtgacaggc tccctgaggg tgggcctggg 15900
   ggcagaggtc cccgccacgt ccccagccct cagcacagtt gccaggagag ggtgacactc 15960
   atgaagtggc acagggaaga tgggagctgt gggctctgca gatccaccac ctcttctgtt 16020
   catttttgtt gatgctgttt tttaagaaaa ttattgaagt aaaattcaca ggacatacgt 16080
   ttactttttt tttttttttt ggagatgggg tctcactctg tcacccaggt tggagtgcag 16140
   tggtgtgatc tcagctcact gcaacctctg cctcccaggt tcaagcgatt ctcccacctc 16200
   cgcctccaga gtagctggga ccacaggcgt gcaccaccac acccagctaa tttttggggg 16260
   gtatcttttt ggtagagaca gggtttcgcc atgttgccca aggctggtct tgaagccctg 16320
   agctcaggcg atccacccgc cttggcctct caaagtgctg ggattacagg cataagccac 16380
   tgcacccagc ctaaatttac cactttaaag tgaatagtgt tacctagtgc attcgcaagg 16440
   cggtgcagcc tccacttctg tctagttcca aagcacttcc attgccccac aggcaaaccc 16500
   cacacccggc agcagtcatg ccccagtccc cgcccccagc cccggcaaac acttttgatg 16560
   gacttaacta cacacattct caacatctca tataaacgga atcacaatat acagcctctg 16620
   atgtctgtct tctttgactt ggcaccatgt tttcgaggtt catccaggct gtagcatgtc 16680
   agtgcttcat cccgttttag gggtgaacca tattccagtg tgcagacaga aaccaatctg 16740
   tgcatccatt cacccactgg gggacctttg tgtcatttcc accctcggct gttgtgcaca 16800
   gtgctgctac ggacattact gtccattcac attttgtgtg aagacctgtt ttcgattctt 16860
   aagagtatac agctaggagc ggaattgctg ggtcatacgt aaatcaatgt ttacgtctca 16920
   aggaatcaac aaactgtttt ccacaatgtt gtcttttttg tttgttttct gagacagggt 16980
   cttgctctgt cacccaggct ggagtgcggt ggtgtgatca tggctcactg cagcctcaat 17040
   ctcctaagct caatccatcc tcctgcctca gcctcctgag tagctgggaa cacaggtatg 17100
   taccaccatg gccagctaat tttctaattt tatttttttt tgtttttgtt tttttgagac 17160
   agagtctcgc tctgtcgccc aggctggagt gcagtggtgc catctcagct cactgcaagc 17220
   tctgcctccc gggttcacac cattctcctg cctcagcctc ccgagtggct gggactatag 17280
   tcaccggcca ccacgcctgg ctaatttttt tgtattttta gtagagatgg ggtttcaccg 17340
   tgttacccag gatggtctcg atctcctaac ttcatgatcc acctgccttg gcctcccaaa 17400
   gttctgggat tacaggcgtg agccaccacg cccgacctta cttttaattt tttaatttta 17460
   ttattttatt ttattttttt tttttttgag acagagtctc gctctgtagc ccaggctgga 17520
   gtgcagtggc gggatctcag ctcactgcaa gctccacctc ccaggttcac gccattctcc 17580
   tgcctcagcc tcccgagtag ctgggactac aggtgcccac cacgatgccc ggctaatttt 17640
   ttgtattttt agtagagaca gggtttcact gtgttagcca ggatgatctc aatctcctga 17700
   cctcgtgatc cgcccgtctc agcctcccaa agtgctggga ttacaggcgt gagccaccgc 17760
   gcccagcctt tttttttttt tttttttttt ttttgagata gagtcttgct ctgtcgccca 17820
   ggctggagtg cagtggcggg atctcagctc actgcaagct ccgcctccca ggttcacgcc 17880
   attctcctgc ctcagcctcc cgagtagctg ggactacagg cacccaccac cacacctggc 17940
   taatgttttg tatttttagt agagacgagg tttcaccgtg ttagccagga tggtctcgat 18000
   ctcctgacct cgtaatccgc ccgcctcggc ctcccaaagt gctgggatta cacgcgtaag 18060
   ccatggcgcc cagcccatgt ggccattttt cagtgagaga agccagaggc ccatcactct 18120
   cggttgctcc ctgggccatg ctctgcctca gccagaagca ctgagggaag gtcagcctcg 18180
   gcccttgccc cagccacagt cacagataaa ggggcctgca caggtctgtg tggctccaga 18240
   gctcgtcacc caacacacga cgcttccatg tgaatagccc caggtgcatc atgaagagcg 18300
   atggccgctg cagaggcaga agaatcccgc ggggaagcag gtgggagaga ggctgagaac 18360
   agaccagacc ctggagctac agaccctatg ttccaaccct ggctgggact agctgtgtgg 18420
   ctctgggcaa attcacatgc ttctctgtgc acaggggatc aaaatagcaa acacaggcta 18480
   ggcacagtgg ttcacaccta taatcccagt gctttgagag gccgaggtgg acacatggct 18540
   taagctcagg agtttgagac cagcctgggc aacatggtga aacctcgtct ctacaaaaaa 18600
   aataccaaat aaattagcca ggcgtggtgg tacgtgcctg tggtctcagc tacttggaag 18660
   gctgaggcgg gaggaacact tgagcccaag aagtcaaggc tgtggccgcg tgtggtggct 18720
   cacgcctgta atcccagcac tttgagaggc tcaggtgggt ggatcacttg tgatcaggag 18780
   ttcaagacca gcctggccaa catggtgaaa ccccgtccct actaaaaaaa tacaacaatt 18840
   tgccaggcgt ggtggcgggc acctgtaatc ccagctactt gggaggctga ggcaggagaa 18900
   tagttagaac ttgggaggtg gaggttgtag ttagccaaga tggtgccgct gcactccagc 18960
   cagggggaca gagcaagact ccatcccaaa aaaaaaaaaa acaaacaaac aaacaaaaaa 19020
   agaggtcaag gctgcagtga accatgattg tgccaatgca ctccagcctg ggtgacaaag 19080
   tgagaccctg cctcaaaaca ataaaaatat aaataaaaat aaaacataat agcaaacgtt 19140
   tcatagaggt ggtatgagca ttaaatgaac tgataaacgt ccctggaaaa cagtaagtgc 19200
   tatggaagga ttcgctgccg ccaccgccac caccattagc atgtttcaac ctccatcacc 19260
   ctcactgtcc cctgtcacca tcctttgacc agggcactcc cagctgcagc ctttctatcc 19320
   tcttgtccac ccttcataac tgtaagatca ctcagctccc aagaaccaca gtctacaggg 19380
   taaccacatt tccaaatctc aaaccagacc cgctggtctg cacttccagg gacaacagga 19440
   tattttcaaa ccagcccaaa agagatgtgt ggctcagcat aagaggaaca ggagaaactg 19500
   aggcctcttg ccctgagaat gagcttggaa gtggatgtcc cggcctcact caaaccttca 19560
   gatgactgag gcccagccag gagcttgagt gtaccctcag gtcataccct gagccagaag 19620
   cacccagcta atccactcct catcactgac tccctcccca taaaaaacct gtttgctgtt 19680
   tcaggctgtt aagttgtggg ctgttttgtt acacagcaat ggataactaa cacacgaggc 19740
   ctggcaagtg tggagcaaag ctgcccaagc cctcaagtct gttcatgtgg gtgttggcct 19800
   gtgtttgcag aaatccagcc actgagtcct cccatgcagt cactactgcc ctctgcacag 19860
   acacctgcca catccctgcc tgggccagga gctccactag tgcaggaatg gggtctgccg 19920
   tcccaggagg atccctgaca cctagcacag ggctagcagc aggcagcact tggttagtga 19980
   ataaactgcc cttcacctgt acacagaagg gatgtttcta taaggggtaa ttaagtacag 20040
   agctgggaag ctatgctgac cagaaggctc taaaagcaat taaccaacga ggggaaaacc 20100
   cttcctactc attctcggcc cattttattg agcactgacc atgtggaagg ccccctggtg 20160
   agactgggga atgcaccaat aactgagaca gcttccggct gttgccctca ggatgcctga 20220
   gctgggatag ggccagggtg ggggtggtgc gtgtgacagg gttactgttc acaaccctgc 20280
   cgggccataa gccctcccca acaattccaa aatccaaaac gctctgaaga tggaaagctt 20340
   ttgttgctca tctggtgaca aaacctcatt tggtgcatgg gccgggtgcg gtggctcacg 20400
   cctgtaatcc cagcactctg ggagccgagg ggaaggatcc cttgagctta ggagtttgag 20460
   accagcctga gcaacatgtg agaccccgtc tctaccaaaa atacaaaaat tagccaggtg 20520
   tggtggcgca ctcctgtagt cccagctact cgggaggctg aggcgggagg atcgcttgag 20580
   cctgggaggt gggggctgca gtgagctgag attatgacat tgcactccag cctgggtgaa 20640
   agagtgagac tctgtctcaa aaaaacaaag ttaaaaaaaa aaaaactgtg catgggtgtg 20700
   ggctacagat agtcttttct gccctactta gaatgaacgt gccacatttg ctatagaaat 20760
   attcaagggc tggtggcaaa tgccacacag accctgacgc tgttccaagt tctgagaagt 20820
   cctgcattcc tcagggcccc agagtttcag agaagagtct gtaggcctga gttaagaagg 20880
   aacgccttca aaagccctgg ggacaaaggg gaaaggggtg ccccaggact gcgtgggtac 20940
   ctaccggaac gagccgtcca ggttggcacg gtggatgaag ctgagcttgg cgtcagccca 21000
   gtagagcttc tgctcctcca ggtcgatggt cagtccattg ggccagtaaa tgtccgagtc 21060
   cacaatgatc ttccgggtgc tgccatccat ccctgcccgc tcaatccggg gcgtctcacc 21120
   ccagtctgtc cagtacatgt acctgtgacg ggggcagggc aagagaagca gctaacacag 21180
   atctgttttt tgtttttgtc tgcatagatg cagacatgaa acaacagaca gtgaacttgc 21240
   cctaaaatct cacccatcgg aaataaccaa caggtatggt ttcaggtatt cctgccttaa 21300
   gctgggcaat caaaatatac tatttccaac ttgttctcag ttaacagtaa attctgggca 21360
   ccttcccttc ttgtggatag aaagattcct tgttcttttg atgattgcct agtgtactct 21420
   gctgtaagtt ttttaaagaa cttcaggtta tttctgattt ttttgctacc atgaaaatgc 21480
   tgtaaatgaa cctctaaaag gcaattcaaa acactcagga tggaatatta tttagtggta 21540
   taaagaaatg agctatcggc tgggcccagt ggctcacacc tctaatccca gcactttggg 21600
   aggccaaggc gggtggatca cgaggtcggg agatcaagac catcctggct aacacagtga 21660
   aaccccgtcc ctactaaaaa tacaaaacat tagccaggcg tggtagtgag cacctgtagt 21720
   cccagctact taggaggctg aggcaggaga atcatttgaa cccgggaggg ggaggttgca 21780
   gtgagcagaa atcgcaccat tgcactccat cctgggcgac agagcgagac tccatctcaa 21840
   aaaaaaaaaa aagaaaagaa aagaaatgat ctatcaagcc atgaaaagac atggaggaaa 21900
   cttaaatgca tgttagtagg tgaaagagcc aatctgtatg agtccagttc taaacactct 21960
   ggaaaaagca aatacacaga gacagtaaag catcagtggt tgccaggagt tggagaggag 22020
   agggatgaat gagtggagca cagaaaatca gggcagtgga actatcctgt atgacatgga 22080
   atggtgggtg catgtcctta ctcatctgtc taaaccaaga atgtacaaat caagggcgaa 22140
   ccctcgtgta aacgtggatt ttgggtgatg gtgcgtcagc cagctttcat cagttgtaac 22200
   aaatgtacca ccctgcacag gatgctgaca gttgggaagg ctgtgtgggt gtgaggacag 22260
   ggatgtatag gaactcagta cctgctgctc atcaattttg ctgtgaacct acaactgttt 22320
   gaaaaaatta agtctattta aaaacaacaa aacatggcca ggcacgatgg cttgcacctg 22380
   taattccagt acttcgggag gctgaggtgg gtgggtcact tgagccaccc tgggcaacat 22440
   ggcaaaatcc cacctctaca aaaaataaaa attaaaaaaa agttagctgg gcatggtggc 22500
   acactcttgt agtcccagct acttgggagg ctgacgtggg aggatccctt cagccctggg 22560
   aggtcgaggc tgcagtgagc tgtgactgta ccactgcact ccagcctgga tgacagagtg 22620
   agaccctgcc taaaaaaaaa aaaaaaaagg ctgggtgcgg tggctcatgc ctgtaattcc 22680
   agcgctttgg gaggccgaga tgggcggatc acgaggtcag gagatcgaga ccatcctggc 22740
   taacacggtg aaaccccgtc tctactaaaa gtacaaaaaa aaaaattagc cgggcatggt 22800
   ggcggacacc tgtagtcaca gctactcggg aggctgaggc aggagaatgg cgtgaacccg 22860
   ggaggcggag cttgcagtga gccaagatca caccactgca ctctcagcct gggagacagc 22920
   aacactccgt ctcaaaaaaa aaagaataaa acccatggct gggatggacc ctgaacctgc 22980
   agctgcagct gttcctgggt aggtctgtgg gcgacgtggc tttgcttctc catgttccca 23040
   agagacaagc atcacccatc catgagaaac aagcacatcc tcagggcgcc cttacgtgat 23100
   ctctggccaa tgaaccaaga caaagtgagc agacaccagg tctgggatgg caggtcccac 23160
   ccccaccagt gcccagtgtg ccctgtttgg aggtgaccac agggtgtgtg cccagaggct 23220
   gggcgtgact ctcagcggag accagagggg aaccacacca gcttggagga ctcagttccc 23280
   atcccagcca gctgggatga gccacaggac acaagggctg gcagacctat tgtgttttgt 23340
   ccacccttca cagcagagaa aggggacagt gcccagaatg tcctctgagg agcctcctcc 23400
   cactcttggt ccttgtaaaa tggtgctgac tcccttgctc ccttcttcct ggggtgggcg 23460
   gcaaacccca ttcccctcag ccttagcaag tgatttagaa acaggcagct cgcccaagcc 23520
   aggcatgaga gtgatcccgg gacacaggga gaacaagccc cgctttgccc tctgggggtc 23580
   tccattcagc agaagaggca aatgacagac acacagccgc ctcctccccc accatggtgc 23640
   tctgcagcct caggagcctc aggtgcacca agggccaccc catccagggg gccatgcttc 23700
   cttgagtggt atcgttcctg agcgagtacc atctccacct tccagagggg ctgtgacaag 23760
   atcaacaaga atgagggcat aggagcctcg aaccaaacat gccctcttcc ctgcagaggc 23820
   tgactgcgcc cagctgctat caccaagccc ctgctcctcc ggccccgtgg ggacagggta 23880
   agaggggtgt cacatggaac agctctccaa acagtccctc tcaagctgct gtctcctgtg 23940
   catctagtga gaacccaacc aacaaaggga aggtgggaat tgctattccc attaggcaga 24000
   tgagaaaact gaggccccga aaggctggcc tgttccaggt tacaggcgct gagcggctgc 24060
   tctgggaaca cacttggtgt ctgctgaggg cccgagcccg gccatcatat gactcaccct 24120
   tcgccagcaa agcccgggtg tgggtgaact tttcctggca gcctgggact ccaaggtgct 24180
   ggcagccagc ccagggaagg ctcccgcgtg cctgcggcag acgccttgct ttacctgcac 24240
   gtccccaccc ctaggagcct ggacagagcc cagaccctcc gccacctcct gagaaggtat 24300
   caggggcatc agtctggact tgggggggaa tccacacagg ccttccccaa atgctccacc 24360
   gtggcccatg gaaaaggctg gaaaacgtgc aggagcagga gcctccgcat ggagcataat 24420
   tcacattcct tccccgagtt tcataacaga ggcctgctgg tttccttaaa tggggaattt 24480
   gcgagccagt cggtgaccag agactggttg gcgtggacgt gctcttgcag agtctcaaac 24540
   gctaccacaa gcccagccaa attccacgga ggaaaatcga cttccgaaga aaagagctgc 24600
   agcatggcct tcgtgcagag ccagctgcgg ttgtggttgt gtgttatttt agggaagggc 24660
   cattttgcat tttaaagagg gggttgggtt tcaccctggc tttaatttga gacccggggg 24720
   ccactgcagc cccttgtcag gctggtacag gccggggact cctcccatgc taagccagtg 24780
   tctttctggc cccagatcct caggggccag agggtcatcc ccagagcccg ctctgccacc 24840
   cacatgggta ccctgggcct gggagggatg tgccttccct caaccctgcc tggatgtccg 24900
   cacggggcca cctgcattgc tgaaactgca acgaagtcga gtctcaggag gggcccccct 24960
   ggctgcaggg ctcttgatcc ttttggccac gtgcacactg aggtggacgc tcggacccag 25020
   agaccccctt catgatgatg gccggggcag gaaccccctc ctctgaggaa ggaccctggt 25080
   gggggacagc actgcaggag ggcacaggag atgacggggg ctctagcagg gccgggagga 25140
   aggccaagat gctcctcgca accgtgtgcc tgtggccagg acagaggaca aacccaccct 25200
   ccactgtccc cactctcagg acagcagtcc tgccccagga ctcagcgccc acacttatgc 25260
   ctgaggacca ctattcaagt cagtatttgg cgagcagggg ttgctgccgc gggcgctgtg 25320
   acaggctgga atcctctccc tctccctctc cctctccgga gacatggagc ctacagggac 25380
   agagtcagca cctcagggta ggaccatggc tggcgtcatc agcatcactg gatctgatga 25440
   gtgggagccg gcatctcact gttttcactc tctcattcaa atgactggag caaagggaag 25500
   gtgtggggag aggcccagga atcaacacta aggtcaactt tgcccccagg ggcaggggtg 25560
   ggagtgaaca gccacaggtg tgatcctggg gagggcttct gggagagaat tcagaggcaa 25620
   gcatgtagag gaaccatttc aaatagttaa gaaaagccag agccaaacag ggacagttgg 25680
   ctcgcagaga tgatgcaggc aaagccagct cagatctgag catgggaaag actactccca 25740
   accaagggcc cagcatctcc caaccaagca ccaagtacct cccaaccaaa tgccaagcac 25800
   ctcccaatca aatacctccc aaccaagcac ctagcacctc tcaactggac accaactact 25860
   cccaaccagg caccaagtac ctcccaacca agtgccaagc acctcccaac caagtaccaa 25920
   ttacctccca accaagcgcc tagcacctcc caactgagca tcatgcacct cccaacagag 25980
   catctagcac ctcccaactg atcacctccc aacctagcac cgagcacctc ccaaccaagt 26040
   gcagagcacc tcccaaccaa gtgccaagca cctcccaatc aaatacctcc caaccaagca 26100
   cctagcacct ctcaactgga caccaacaac tcccaaccaa gcgccaagca cctcctaaca 26160
   aagtaccaat caccttccaa ccgagcacct agcacctccc aactgagcat catgcacctc 26220
   ccaacaaatc acctagcacc tcccgactga tcacctccca acctagcact gagcacttcc 26280
   caaccaacat agcaaaagcc ataaagaagt aaaaagacaa aaccacgtag gcatggagac 26340
   tggacttctg gtggcgagga aagggcattt ttattataac gacagctaac atttgttgaa 26400
   ctcacaaact gttcttggtg ttttcctcat gacatgcagc atggtcacgc ctctgtacag 26460
   acaaggatac tgaggcacag agtggcaccg tgccaacctt gtctcatctt tttatcgaac 26520
   ctacatgcag agtgccagca aatccagctg tcttttctct tcagaacaga tcccaaatct 26580
   cgccactcct tacccccaca agtgaggtgt ccccgctgct gctttctgtc gccaggatcc 26640
   cggtaataac cgtggagagg gctcctgccc ccacgccacc caccccacag ctcactctcg 26700
   ctccagccac caggggatgc cttccagcac gagtcagagc tggcacctcc tctgctcgag 26760
   acctcatgtg tcctctcctc acaccttggg ccctgtttcc ctacattctg ctacagcccc 26820
   tcaaacaggc cccgccccaa accagcccag ggcctttgca ctggctgatc cctctgcctg 26880
   gaccgcgctg cccccagaca gccacacggt tctcagcctc atctgcttcc agtctcgact 26940
   caaaagtcac caagaggcct tcccagcacc tgagctccga cggaagcccc tcgccacagc 27000
   acccaagcac tgctttatcc ccctacgcac acgtcccttt caaatactat tcatttacca 27060
   tctcctccca ctcactgaaa gggccagaga ctgggctata cccgctgcgt ggggagcagg 27120
   accaggcgca agggctcaca aatgcagtgg atgcctggtt gggaggtgag ggagctgcag 27180
   cgacccacgc tgggagggaa cgcaatgaca ggaggagcgc aggtcctggc gacacgatgg 27240
   ccatggcagc cgctggtgag caaccgcagg ccggccctgg gagagggctt ctagcaagct 27300
   gctatcttca gcctctccga ctactgcaga tgccccctcc tagccagaga cactgctaca 27360
   ccagccgacc cttccaaaaa gaaggtcagt aaccccgcga ctcctggagc cacagtgcag 27420
   ggggagaggg ctgagagggc aacagttcac caagcggaac agaggctgcc ccggaggtca 27480
   gctggctccc cggcagctgc aggggtggct agcccactcg gagggcagcg agggcatacg 27540
   aggggctcca gggatgagtg gttgcccagc acagcacccc tgggaggccg ggggcacttc 27600
   tcaggtagtg ggggcacgag gctgctctgg cctgacctca gggactcaaa atactttggc 27660
   gataaattcc accgtgtccc acccctgctg gtaccccata cttacacaca gactggttca 27720
   gatgcagaca ctctcgcgca catactcgct cacacgggca catacacgtg cacacacagt 27780
   cacatgcgca cactcataca cacacaaata tccactcaca cgcatgcatg cacacacacg 27840
   gacacacaca ggctcacacg tatgcacgca tatgcgtgca cacgcacaca cacacacaca 27900
   cgctcacatc ctcccactcc cacactcagt tgctcagaca cacacacgcc tggctctcac 27960
   acaaacctgt tgggctctga aaggctccag cccttcccat gctcgtcaga agccagtcaa 28020
   tggcttccta agtcaccaca cagatcaaag aggtgaactt ggccacatgg cactctgctt 28080
   cctgagctcc caaacaccag ccttggtgag gacagaccct caccccacac cctcattccc 28140
   actaccctgg gcaggcccag aggaggggca tctgcaggat ctggcaacca gcccctcccg 28200
   cccggctcct gcagccggca ccatgggagt cagggggagg tcactgcaaa gggcaacagc 28260
   aagttggtgg ccccaggact agagcccagg ggtcttcagt cctactccag agcttggaca 28320
   ctgtcccaca gggcatggcc aagggaaggg cttccagagc cctgacttca gggaggaggg 28380
   caggcgggct cctgtggcag gcctggatgc atggccgccc actcctggga ctttctaacc 28440
   tagaatatct aggtcaggct gggtgcagtg gctcacgcct gcaatcccaa cactttggga 28500
   ggccgaggag ggtggatcac ttgaggttag gagtttgaga ccagcctggc caacatggcg 28560
   aaaccctgtg tctactaaaa atacaaaacc tagccaggtg tggtagtgca cgcctgtaat 28620
   cacagctact caggaggctg aggcaggaga atcacttgaa ctcgggaggt ggaggttgca 28680
   gtgagctgag atcgtgccat tgcgcaaaga agatctaggc cggcccctca accggtgagg 28740
   tccaggctgg gagtgctgag agactgtggt gacactgaat gaactaacag gcaaagggct 28800
   tccaactgag cctgggggtg gtgggaaatg gctcttgtgt tctagtcaag acctctgcca 28860
   accagttctg acactgaccc agcacagaac ctgacaggtc agcaagggcc agggcttagc 28920
   acagcccagg taagggtgtg tgtacggccc ccagagtcac tcccaggctg caagaaaagg 28980
   gacaaaggag ggacaagggg tggccaagca aactgttccc tctgctcggg agtctggggt 29040
   gacctggcct agctggccag tggagctggg ccacctcccc ttaaactctc caccccggac 29100
   ttcgactcca aagctttcct gccacccacg ctctccccac ctgggatcac ggccaggccc 29160
   tgagccttca agggcccagg tgaactcagc cagactagga gctgaggagg acacagggca 29220
   gcttccagaa cggacccgag aaccactccc agcaggttct gcttccagac aaggagctgc 29280
   actttttcag ccaatgcaat tagaaagcca ggagaaggtg caaattccac ctgcctgagc 29340
   gtccgcactt cccaggccgc ccaccataca cacagcaaag atgtgtttaa ccattcaaac 29400
   ccatggccaa ccacatcggt tgcctcagac atgcaagttt taaaaaggaa cataactatg 29460
   ggccaggcac ggtggttcac gtctgtaatc ccagcacttt gggaggccga ggtgggtgga 29520
   tcacctgagg tcaggagttc gagaccagcc tagacaccat ggtgaaaccc catctgtacc 29580
   aaaactacaa aaattagctg ggcgtggtgg tgggcgcctg taatcccagc tacttgggaa 29640
   gctgaggcag gagaatcact tgaacccggg aggcgaaggt tgcagtgagc cgagattgtg 29700
   ccactgcact ccagcctggg caacaaggga gactccatct caattaaaaa aaaaaaaaaa 29760
   aaaaaggaac ataactatgg agtctcaagg ggaagtaatt ccttcaacaa taacaaatct 29820
   tgaaagctga gctctttttt ttttttgaga caggatctcc tcactttgtc gcccaggctg 29880
   gagtgcagtg gtgggatcac agctcactgc agcctcgatc tcccaggctc aaatgatcct 29940
   cctacctcag cctcccaaga agctgggatt acaggtgcat accatcacac ccgattcatt 30000
   tttgtatact ttgaagagat ggggtctcac catgttgccc agtgtggtct tgaattcctg 30060
   gactcaggtg atctgcccgc cttggcctcc cagagtgctg ggattacagg cctgagccaa 30120
   cacccccacg ggttcatttt cagagtcgca ccgagtgctg gggttacagg cctgagccaa 30180
   cccccccacg ggttcatttt aagagtgaca ccgagtgctg gggttacagg cctgaaccaa 30240
   cccccccacg agttcatttt cagagtcgca ccgagtgctg gggttacagg cctgagccaa 30300
   cccccccacg ggttcatttt aagagtgaca ccgagtgctg gggttacagg cctgagccaa 30360
   cacccccacg ggttcatttt cagagtcaca ccgagtgctg gggttacagg cctgagccaa 30420
   cccccccacg ggttcatttt cagagtcaca ccctttttct gaaaaacaac ttgggctcat 30480
   gcaaattcga gagagagatg gtgacactcc ccgccccctg gacccaggtg gagtcgcagc 30540
   agggtttacc cgtgagcggg gtccaaggcg atggccctcg gctggtcaag gtcctgccag 30600
   aagagcacct tccgggatgt gccattgagg ttggccacct cgatgcggtt ggtctctgag 30660
   tccgtccagt acagcttctt gcccacccag tcgcaggcga ggccgtcggg agagaccagg 30720
   ccggagatga ccacgttctg cacggcggcc cccgtctggt tcaggtaggt ctgcttgatg 30780
   gcctcctcgc tcacgtctgt ccagtacacg gctcccttgg aaaactggaa gtccactgcg 30840
   gccgcatcct ccaggccgct gaccacgatg gtggactcca gcttgactcc gccggcgtcc 30900
   accagccgta cgtcccggcg gttggcaaat agcaggagcg gcgaggctgt ggggcagaag 30960
   caaaccgtga gggccactgg ctaagccagc aagatacaca gccctgggat ggagcactat 31020
   gcccagagca ctcctggtac tgccctgccc atgcccaaga cctccagttc cttcctccca 31080
   cccctaaggc gttgtcagga agttgcctgg gcagccccgg cccgcatcat tcagaggctc 31140
   ctgcagcgca gcaaacagcc ttcttcccac attcggtgac agcacctgtt tgtttaccaa 31200
   ctgttacgtc tgttccccca gatatgggtg acccttcctg ccatgcccaa aacctcccac 31260
   atcgtcctcc agaggctaca ggggccctgt cctgttctgc agagaagcca catccccttt 31320
   gttggcctga cacaggggat ggggacatgc aggcacagca ctggccatgc tgctcgctac 31380
   agacccagcc acagggccac attttttgag gggttcagag cccaggccag acagagcctc 31440
   aagattccct tacaagtctt tgaccactgt ccaagctcag gcccgtttcc ttggccgtgg 31500
   catcagcttc ccatccaccc ctgtattcca tgtttctccc accctgcttc tggacattcc 31560
   tacatttaaa gggtcactct ggaatgccac cccttggctc agacaccttc cacagctccc 31620
   tgtgccagtg ccatgcagaa caaggtcaga ccccctagcc tggcctccaa ggccttggcc 31680
   tctggcctca cctacacttc tctccaccac cccaccccaa gcattcctga tctgcctgcg 31740
   gccaggctgg ctccctcacc tccctgtgca ccgcagccct cagccccttc tgcctgtgca 31800
   agaagcctca tctcacagac aacggtctca ttcccacaac gggctcaatg agaaatcagg 31860
   agaggccttc agaccatcac cccaccagac acctcagacg tcggaccagg agggtccagc 31920
   aacccccaac acagactcag agggactaag aagccacatg aggagtgaac acaagatgtg 31980
   gacaggagga ggttaagggc ctccagggag ctccatcagt ccgtgttctg ctgtcagcag 32040
   ggttaggctg ggctggccac aaacaccccc aaaaaacatc tgaagccttg gcttgaaaca 32100
   gctgacattc ctcatgaaaa ctgcagaccc ctgggtcctc ctgcgcagat gggggagccc 32160
   agccaacccc acactcccac cttcaccaag aaagagaaag ccaaaacaaa ctcaactcag 32220
   ccaatgacaa tcacagaact gaatcctgta gttagttcag ttggtttcat ttcagcaggg 32280
   gaaagatttg cagcctctat gagggtagct gggaacacaa agggccagag catggcccag 32340
   gagaccccag cgcagtgggg tagatggttc cgagcacagg cctccctgcc aagacaagca 32400
   ctggctcaaa tcctggcccc tcccattccc aggagacatg ctccacagga tgggaggaca 32460
   cacagaggac ctgaggccag gaaaatgaca gcggcgcctc cgccgcccca cccgtgctgt 32520
   catcatctta ggtctacagt tctttgtggc aacgagggac actgtgaaag tcaaacaaca 32580
   ggaaggcata ggccacaaat aaagacaaac gggacttcat gggaagctaa agattttgtg 32640
   catcaaaaga cactatcgag agagtaaaaa ggcaacccac agaatgagag aaaatatttc 32700
   caaatcatag atctactaag agattaatat ccatgaaata cagagaactc ctaaaactca 32760
   acaatgagaa aacaactaag ccaactcaaa aatgggcaaa caacttgaac agacatttct 32820
   ccaaagatga catataaatg gccaataaac acatcaaaac aggcttaata tatccctaat 32880
   catcagggaa atgcaaatca aaactacaat aagataccat cttgcaccaa ttaggacggc 32940
   tactatcaaa aaaacaaaat agcaagtgtt ggtgaggatc tggagcaact ggaacccttg 33000
   tgcaccactg gcaaaaatgt gaaatggtgc agctactatg gaaaacagca tggcagttcc 33060
   ccaaaaactt aaacacagaa ttaccatatg acccagcaat ttcgctttgg gttatatacc 33120
   caaaagaact gaaaacaggg acacaatcag atatgcatac accttggatc acagcagcat 33180
   ccttcccaac agctaaaaca tggaggcagc caggcatggt ggctcacgcc tgtaatccca 33240
   gcactttggg aggctgaggc gggtggatca cctgaggtca ggagttcgag accagcctgg 33300
   ccaacatggt gaaaccccgt ctctactaaa atacaaaaat tagctgggcg tagtgacggg 33360
   cacctgtaat cccagctact cacaagtctg aggcaggaga atcacttgaa ccctggaagt 33420
   ggacgttgca gtgagccaag attgcgccac tgcattccag cctgggtgac acagcgagac 33480
   tctgtctcaa aaaacagcaa aacaaaaaca aaaaaacaaa caaacatgga agcaacccaa 33540
   gcgtccctct actgagggat gaatagcggg gcaaaatctg ctccatccac acaatggagt 33600
   actattcagt ctcaaaaagg aaaaagattc tggtcaggca cggtggctca tgcctgtaat 33660
   cccagcactt ggggaggctg aggcgggtgg atcacctgaa gtcaggaatt caaggcccgc 33720
   ctggccaaga ctggcaccna gctacacana aagtatangg ccccggaaa 33769
<210> 9
   <211> 72049
   <212> DNA
   <213> Homo sapiens
<220>
   <221> unsure
   <222> (8356) , (8385) , (38585)
   <223> Identity of nucleotide sequences at the above locations are unknown.
<400> 9
<210> 10
   <211> 8705
   <212> DNA
   <213> Homo sapiens
<400> 10
   ggactcaggg gcagcaggga ggtacaccca tggttagtgg gcggaccata gggggtaatg 60
   agagggtgaa tcgatggaac ctgggggaca caatcgaagt ggttccagag tcgggctgta 120
   ctaattaaag agacggggca gtggacaggc attttcagtt gactgcccag ggagtgttct 180
   gcccaacagg gaggatatgc gtacagaatc atactcgatc agcatgagtc caattcagac 240
   cgtacatcag tggagatatg ggtcccccga tgactccgtg gaacactgat gtttgtgaca 300
   ggggagtaca gcaccagcca tcagcaggcc agtaaatcat accggcctgc gaaattggac 360
   tcagacccgg atccaccctg accgacgtcc caagccccca ccccccaccc cccaccatgg 420
   gccgagatcc agtcctcttt gaatagggcc tggccgtggt tcacgggaca tctgagacat 480
   tgccgaggcg ctgcattggt ggatcttgcc agaagtttgc ccagtgcaga tttgggcaga 540
   atctcaaact gccttgggat gtaggagaga aaccaggcct ggtcaagttc atgggaagag 600
   gtggaaacag accccatagg ctggggcttg ggcagctgta ggaagccctc tctgctgcct 660
   ccctgcctgc tctctgcttt gaagcatctt ccccagtgcc cccagtctca tgccctctca 720
   acgttggggt caaatcctga ggaataccca gactggctct ctgggccaaa gaggaccctc 780
   tccagaaaga gcagggccca gtgcggcttc ctaaagggca ggggaagggc ctggccactc 840
   cccagaggct actcaccagc catcaggata gccccaggaa gcaggccttc tcgagcccat 900
   tttattactt tattttatta ttttatttaa ttttaaattt attttttgag acagagtctc 960
   actctgttgc ccaggctgga gtgcagtggt gcgatctcaa cccactgcag cctctgcctc 1020
   cagggttcaa gggattctcc cacctcagcc tcccaagtag ctgggattac aggtgcccgc 1080
   caccacaccc ggctaatttt catattttta gtagagatga ggtttcacca tgttggccag 1140
   gctggtctcg aactcctgac ctcaagtgat ccgcccgcct cggcctccca aagtgctagg 1200
   tcaagcccat tttaaagttg aagaaactga ggctgaggta aattccctcc ccagggatcc 1260
   tgctgcagcc agaaggtggt aaaacaggac ttcacccggg tctgtctggc gtgaaaggca 1320
   gtgttcttgt accaccctag ggggcctgag agaactgagt ccctcgggca taactgacag 1380
   ttctgttccc attattccgc aggggctcgg atctggctgt atgctttcca ggatggcctt 1440
   ggagacccac ataagcccta caccctttgg gaagctgcat gttgggttgg ggtgccgtca 1500
   gtggcacttg tggaaggtgc agacctgtgt gggtgtgtgg gcccagggcc cctggtccct 1560
   tcctcccttt gtagggctgg ttgtgtgctg cctggacctg gggggcacgt tcacgtggtg 1620
   aatttgtcta tttactatcc ccgctttggg gctggtgcca gcacaggccc ttgtgaaggg 1680
   ggtgcctttg tctggagtgg gactgtggcc cctccctcag cgtggtgact tctgtgtcag 1740
   ggcttcagca gggacgcaga gcccctgagt gttcggaaca agggcgtcat tgcaggagtt 1800
   agactgtgtg tgatggaggg aggaggggca ggaggaaagg tcagaaggag agttcctggg 1860
   aaggtccctg aggagcctgg tgaggtgcta actggtgtgg aggacactca gggcctgtgg 1920
   ggacatctcc tactgctggg ggccagccac aaagggaact ggccgaagtc ctgtccccgc 1980
   cttcacagcc cagcatctgg tcacaaggca ggtacttgga agggcgcggg cacctgggcc 2040
   aaaagtgcct gggttccctt tgcctttcac tgagatgacc ttcggggcag gtggctgctg 2100
   cctcccctcc tgtccccagg ttttgccaac tggccagagg aaggggtcct gggaagcagg 2160
   ggggccagaa gccctctctg caaggaaagc ccgaggggtg tgggaggaag gaaggaatgc 2220
   ccaggctggc gaggctctaa gtcaccctgg cttggctctc ctcagatcct gaacccgccg 2280
   ccctccccgg ccacggaccc ctccctgtac aacatggaca tgttctactc ttcaaacatt 2340
   ccggccactg cgagaccgta caggtaggac atcccctgca gccctccatg gccattgggt 2400
   tcccgccagc ccgtggtgga ggggcctaat ccccatgcca ctgatgaggg gaggtattct 2460
   gggtgctaat gggcaggtgc cgggcccagc cctgcctccc tctgctctgc caaccacact 2520
   aggctgcctc cccagacaag ctcagcgggc actgcatgtt gggttcagaa atcagcagaa 2580
   ctccacgttc tgagctgctc ttcaagttgc tcctatgggg gttactttta agctgggaaa 2640
   tggctgtggc gtcgaggggc cgggggcttg ggctccagag tctgactgtg tgtttgagtc 2700
   cggctgtgga aacctagcca ttgagatgcc ccctcttggt ggctctgtcc tcttaggatg 2760
   ggacaagtct gtgaaggctg ctgcagcacc caccgtagac ccctaatcgt gtgacgtcac 2820
   caggatggtc cgggctgctc acttgccaca gtggcctgtt tgagcccggg aagccaacgg 2880
   ggctgctcag ctggacacca gccccccgag ctgcccatgt tggggtcaca ggccccacct 2940
   ccctggttgg ggaggggcaa ctgagagtgt ggagaggtgg gacccaggtg tgctggtctc 3000
   cgcaggggct ggatcagagc ctgggatggg cagggtgagc ctcctgacct ttaacccagt 3060
   ggtgtcaggc aacgtggccc acccgccagc cgcaccaggc cccacccccg caggtgaagg 3120
   ggtgggatag gctgggcctg ggccaggaca cctctggacc acgcattcct cattgcttgg 3180
   gtccctggag cagcagggcc tcccgagtgt ggtgccgcct gccacctagt ggccatttcc 3240
   acgaactccc aggcctggct ggggagccgg aactgcagcc tccatttcca ccccactccg 3300
   ggtcgggcca cctccctgat gcctcagtat tatatcaaac tgtcacagtc tgtcccacag 3360
   ccttacagac cactgtctcc agaatggtca catccacact gggcagccca gtctcgctag 3420
   ttcctcgtcc cacctcctgc ctttgctcat gcccgtcctg ctctgggccc accgcggaca 3480
   catcttcccc ccgcccgccg tctgacctca cagcagctgg gccccaagag gagtatcctg 3540
   tcctgctgca cttttctcaa cacccggtgt tggctgcacc ttcccaccca ttgcaggccc 3600
   ctctgtgaca ggacgggggc tcctaaacac accacagttc cgagtctgaa ctcacacagt 3660
   gggatgcggc gtttctgggc cacagttggg tgcaggtagc ctctgggagg atgggaggtc 3720
   aggagccatc ttgcgagtca ggttgcttga actcaggatg gaagtgttcc gggcccattg 3780
   gttgctgtat tagcctgttc tcacgctgct aataaagaca tacccaagac tgggtaattg 3840
   taaaggaaag aggtttaacg gactcacagt tccacctgcc tggggtggcc tcacaatcat 3900
   ggtagaagac aaggaggagc aagtcacatc ttacatggct tcagggaaca gacagcatga 3960
   gaaccaagcg aaaggggttt ccccttgtaa aaccatcaag tctagtgaga tttattcact 4020
   accacgagaa cagtatgggg ggaaccaccc ccatgattca atcatctccc actgggtccc 4080
   tcccacagca cgtgggaatt atgggagtac aattcaagat gagatttggg tggggacaca 4140
   gccaaaccct atcggttgcc aacatttaca gtaacagtgt taggtgaaca gttgtccagt 4200
   ctcctgtttt gtcggacact gtttctagca ccttccaggc agaatctcat gtatccttca 4260
   ctttcgaaat gggtactatt tcatccccac ttttatcaat gagaaactaa agctcgaaga 4320
   ggtcaagtaa gttcctggcc aaggtcagct agcaggctct agaggcctcg ttctccttag 4380
   aggcagcctt gccagggccc aggcttggca ggctgcaggg caggtgcggg catgcccatg 4440
   gtagaggtgg gaccattgag gctcagagag ggtaagtgat gagccctggc gacacagcgg 4500
   ggtgggtcca gagtccggcc tgcatcttct ggagctggcc agtggacagg cctttcccgt 4560
   tcacagcccc ggggctgctg tgcccaccag ggcggatgtg cctaccgaat cccactcctc 4620
   tgtgtgtgtc cctttcaggc cctacatcat tcgaggaatg gcgcccccga cgacgccctg 4680
   cagcaccgac gtgtgtgaca gcgactacag cgccagccgc tggaaggcca gcaagtacta 4740
   cctggatttg aactcggact cagaccccta tccaccccca cccacgcccc acagccagta 4800
   cctgtcggcg gaggacagct gcccgccctc gcccgccacc gagaggagct acttccatct 4860
   cttcccgccc cctccgtccc cctgcacgga ctcatcctga cctcggccgg gccactctgg 4920
   cttctctgtg cccctgtaaa tagttttaaa tatgaacaaa gaaaaaaata tattttatga 4980
   tttaaaaaat aaatataatt gggattttaa aaacatgaga aatgtgaact gtgatggggt 5040
   gggcagggct gggagaactt tgtacagtgg agaaatattt ataaacttaa ttttgtaaaa 5100
   cagaactgcc attctttcgt gccctgtgtg catttgagtt gtgtgtcccc gtggagggaa 5160
   tgccgacccc cggaccacca tgagagtcct cctgcacccg ggcgtccctc tgtccggctc 5220
   ctgcagggaa gggctggggc cttgggcaga ggtggatatc tcccctggga tgcatccctg 5280
   agctgcaggc cgggccggct ttatgtgcgt gtggcctgtg ccgtcagaaa gggccctggg 5340
   cttcatcacg ctgttgctgt tcgtcttcct cagattctta gtcttttttt tttttttttt 5400
   ttttttgaga cggagtcttt ctctgtcatc caggctggag tgcagtggta caatctcagc 5460
   tcactgcaag ctccgactcc caggttcaag tgagtctcct gcctcagcct cccgagtagc 5520
   tgggactaca ggtgcgcgcc accacacccg cccagctaat ttttgtattt ttagtagaga 5580
   tggggtttca ccatgttggc caggatgatc tcgatctctt gacctcgtga tccgcccacc 5640
   tcggcctccc aaagtgctgg gattataggc atgagccact gtacccagct gactcttagt 5700
   cacttttaag aaggggactg tgccttcatt tttcactggg ccctgcagaa tatatgcctg 5760
   ggctctgggc tcttctgaac ctgtgttggc ttccatctga cctctctgtg ccagcccaag 5820
   gctgctgctc ttcctgaggg caaggagccc catgactgcg tgttgactcg ctggatgggg 5880
   ctgctgagcc cactctgcca caccacgtgc ccctggcagg gagggaatcc ctgggtcctc 5940
   acaggaacag tcagcaagcc acacctgacg cctgctgtgg gcccatccct gcggtgctgg 6000
   agaagacaga caaggcctgg tcactgcctc tgcagggtcc ccagtccgtg gaaggagaca 6060
   gtaatctagg cattttcggt ggggaagctg agctgttctc gtgtcctgaa ggccaggcgg 6120
   gaacagccgt cttcagaggg aagggagaaa atgcacatcg catcagtgga gaagggcctg 6180
   acttccctca gcatggtgga gggaggtcag aaaacagtca agcttgttgc tgggtgacag 6240
   tgcatttaat aatcaaaata taggctgggt acggtggctc atgcctgtaa tcccagcact 6300
   ttgggaggct gaggcaggtg gatcacttga ggccaggagt ttgagaccgg cctggccaac 6360
   atggcaaaac ctcaactact aaaatacaaa aactagccgg gcgtggtggt gcacgcctgt 6420
   aatcccagct acttgggagg ctgaggcagg agaattgctt gaacctggga ggcggaggct 6480
   gcagtgagcc gagattgtgc cactgcactc cagcctgggc aacagagcaa gactctgtct 6540
   caaaaaaaaa aaaaaaaaaa gcaatacaaa atacaaatat cactttcact aaaagaaggg 6600
   atggaagacc caaaacaaac agaaaacaac aaaatggcag gagtaagtcc ccacttatca 6660
   ataataacat tgactgtaaa taggctaagc tctgcaatca aaagagtggg ccaggagcgg 6720
   tggctcacgc ctgtaattcc aacgctttgg gaggctgagg cggatggatc atttgatgtc 6780
   acgagtttta agaccagcct ggccaacaag gtgaaacccc atctgtacta aaaatacaaa 6840
   aattagccag gcggtagtgg cacgcacctg taatcccagc tacttgtgag gctgaggcag 6900
   gagaatcact ggaggctggg aagcggaggt tgctgtgagc caagatggag ccactgcact 6960
   cccacctggg cgacagagtg agatcctgtc ttaagaaaaa aaagagtgga tgaatggatc 7020
   aaaaaacaag acccaaccat ctcttgcata caagaaacac actttaccta taaaaacaca 7080
   ctaggccagg tgtggtggct cacacctgta atcccagccc tttgggaggc ctgactggca 7140
   gatcacctga ggccaggagt ttcagaccag cttgaccgac atggcaaaac cccatctctc 7200
   ctaaaaatac aaaaaaacaa aaaaaagaaa aaggctggaa gtagtgatgt gtgcctgtag 7260
   ccccagctac ttgggaggct gaggcaggag aattgcttga atccgggaag tggaggttgc 7320
   agtgagccag gatggtgcca ctgcactcca gcctgggtga cagagcgaga ccctgtcata 7380
   aaaaaaaaaa gaaaagaaaa gaaaaacgag aaaaacaaac acaaaattag tagaagaaaa 7440
   gaaataataa agatcagaac aggccaggct catgggcaca gtggctcaac tcctacctgc 7500
   tcaggagttt gagaccagtc tggccaacat ggcaaaaccc catctctcct aaaaatatga 7560
   aaaaaaaaaa ataggctgga tgtggtgatg tgtgtgtgcc tgtagcccca gctacttggg 7620
   aggctgaggt gggagaatca cttgagccca ggaagtggag gctgcagcga gtcatgaatg 7680
   caccctgcac tctagctggg taactggagt gagattctgt ctcaaaaaag caaagaccag 7740
   agcagaaata aatgaaatgg aaatgaagga aacaatgcaa aatgatacaa aaagtttttt 7800
   cgaaaagata aacaaaatca acaaaccttt agccagatta agaaaaaaag agagaagacc 7860
   caaataaata aaatccgaga ttaaaaagga gacattacca ctgataccac agaaattcaa 7920
   aggatcatta gaggcaacta tgtgcaacta tatgctaatg aactggaaaa cctagaagaa 7980
   ctgggtaaat ttctagacac atacaaccta tcaagattga accatgaaga aatccaaaac 8040
   ctgaacaggc cgggcacggt ggcttacgcc tgtaatccca gcactttgga aggcctgaga 8100
   tcaggagttc gagaccagcc tggccaacat ggtgaaaccc catctctact gaaaaaatat 8160
   aaaaattagc cgggcgtggt ggcgggtgcc tctaatgtca gccactcggg aggctgaggc 8220
   aggaaaatca cttgaacctg ggaggcatag gttgcagcga gccgaggttg caccactgca 8280
   ctccagcctt ggcgacagag ccagactcca tctcaaaaaa attaaaataa caaaaacctg 8340
   aacagaccaa taacaagtaa tgcgatgaaa actgtaataa aatgtttccc aacaaagaaa 8400
   gcccaggaac aaatggcttc actgctgaat tttaccaaac attttttttt ttttgagacg 8460
   gagtctcgct ctgtcgccca ggctggagtg cagtggtgta acctcggttc gctggtaact 8520
   tatgcctctc aggctgcaag tgattttcct gcttcaggcc ccccgagtgg ctggaaatta 8580
   gatggtactt gtcaaacaag gcctggctaa atttctatat ttccttcaag tagaagatgt 8640
   gcttccaaca aaggttgggt tacggctggc ttctgaaaat cttggatttc aaggctcccc 8700
   aaaag 8705
<210> 11
   <211> 66933
   <212> DNA
   <213> Homo sapiens
<400> 11
   tataatcaag cgcgttccgt ccagtccggt gggaagattt tcgatatgct tcgtgatctg 60
   ctcaagaacg ttgatcttaa agggttcgag cctgatgtac gtattttgct taccaaatac 120
   agcaatagta atggctctca gtccccgtgg atggaggagc aaattcggga tgcctgggga 180
   agcatggttc taaaaaatgt tgtacgtgaa acggatgaag ttggtaaagg tcagatccgg 240
   atgagaactg tttttgaaca ggccattgat caacgctctt caactggtgc ctggagaaat 300
   gctctttcta tttgggaacc tgtctgcaat gaaattttcg atcgtctgat taaaccacgc 360
   tgggagatta gataatgaag cgtgcgcctg ttattccaaa acatacgctc aatactcaac 420
   cggttgaaga tacttcgtta tcgacaccag ctgccccgat ggtggattcg ttaattgcgc 480
   gcgtaggagt aatggctcgc ggtaatgcca ttactttgcc tgtatgtggt cgggatgtga    540
   agtttactct tgaagtgctc cggggtgata gtgttgagaa gacctctcgg gtatggtcag    600
   gtaatgaacg tgaccaggag ctgcttactg aggacgcact ggatgatctc atcccttctt    660
   ttctactgac tggtcaacag acaccggcgt tcggtcgaag agtatctggt gtcatagaaa    720
   ttgccgatgg gagtcgccgt cgtaaagctg ctgcacttac cgaaagtgat tatcgtgttc    780
   tggttggcga gctggatgat gagcagatgg ctgcattatc cagattgggt aacgattatc    840
   gcccaacaag tgcttatgaa cgtggtcagc gttatgcaag ccgattgcag aatgaatttg    900
   ctggaaatat ttctgcgctg gctgatgcgg aaaatatttc acgtaagatt attacccgct    960
   gtatcaacac cgccaaattg cctaaatcag ttgttgctct tttttctcac cccggtgaac   1020
   tatctgcccg gtcaggtgat gcacttcaaa aagcctttac agataaagag gaattactta   1080
   agcagcaggc atctaacctt catgagcaga aaaaagctgg ggtgatattt gaagctgaag   1140
   aagttatcac tcttttaact tctgtgctta aaacgtcatc tgcatcaaga actagtttaa   1200
   gctcacgaca tcagtttgct cctggagcga cagtattgta taagggcgat aaaatggtgc   1260
   ttaacctgga caggtctcgt gttccaactg agtgtataga gaaaattgag gccattctta   1320
   aggaacttga aaagccagca ccctgatgcg accacgtttt agtctacgtt tatctgtctt   1380
   tacttaatgt cctttgttac aggccagaaa gcataactgg cctgaatatt ctctctgggc   1440
   ccactgttcc acttgtatcg tcggtctgat aatcagactg ggaccacggt cccactcgta   1500
   tcgtcggtct gattattagt ctgggaccac ggtcccactc gtatcgtcgg tctgattatt   1560
   agtctgggac cacggtccca ctcgtatcgt cggtctgata atcagactgg gaccacggtc   1620
   ccactcgtat cgtcggtctg attattagtc tgggaccatg gtcccactcg tatcgtcggt   1680
   ctgattatta gtctgggacc acggtcccac tcgtatcgtc ggtctgatta ttagtctgga   1740
   accacggtcc cactcgtatc gtcggtctga ttattagtct gggaccacgg tcccactcgt   1800
   atcgtcggtc tgattattag tctgggacca cgatcccact cgtgttgtcg gtctgattat   1860
   cggtctggga ccacggtccc acttgtattg tcgatcagac tatcagcgtg agactacgat   1920
   tccatcaatg cctgtcaagg gcaagtattg acatgtcgtc gtaacctgta gaacggagta   1980
   acctcggtgt gcggttgtat gcctgctgtg gattgctgct gtgtcctgct tatccacaac   2040
   attttgcgca cggttatgtg gacaaaatac ctggttaccc aggccgtgcc ggcacgttaa   2100
   ccgggctgca tccgatgcaa gtgtgtcgct gtcgacgagc tcgcgagctc ggacatgagg 2160
   ttgccccgta ttcagtgtcg ctgatttgta ttgtctgaag ttgtttttac gttaagttga 2220
   tgcagatcaa ttaatacgat acctgcgtca taattgatta tttgacgtgg tttgatggcc 2280
   tccacgcacg ttgtgatatg tagatgataa tcattatcac tttacgggtc ctttccggtg 2340
   atccgacagg ttacggggcg gcgacctcgc gggttttcgc tatttatgaa aattttccgg 2400
   tttaaggcgt ttccgttctt cttcgtcata acttaatgtt tttatttaaa ataccctctg 2460
   aaaagaaagg aaacgacagg tgctgaaagc gagctttttg gcctctgtcg tttcctttct 2520
   ctgtttttgt ccgtggaatg aacaatggaa gtccgagctc atcgctaata acttcgtata 2580
   gcatacatta tacgaagtta tattcgatgc ggccgcaagg ggttcgcgtc agcgggtgtt 2640
   ggcgggtgtc ggggctggct taactatgcg gcatcagagc agattgtact gagagtgcac 2700
   catatgcggt gtgaaatacc gcacagatgc gtaaggagaa aataccgcat caggcgccat 2760
   tcgccattca ggctgcgcaa ctgttgggaa gggcgatcgg tgcgggcctc ttcgctatta 2820
   cgccagctgg cgaaaggggg atgtgctgca aggcgattaa gttgggtaac gccagggttt 2880
   tcccagtcac gacgttgtaa aacgacggcc agtgaattgt aatacgactc actatagggc 2940
   gaattcgagc tcggtacccg gggatcctct agagtcgacc tgcaggcatg caagcttctc 3000
   ttgtgccggt tgtacgctgt caggtcacac tggtgagtta ggcagggcac agatgcccag 3060
   agcagaggga actttccttg gggattcaac acgtgcaagt cttaggggct ggcaaatcct 3120
   gccctcagct agagaggggg cttttatttg agaccagaat cacctgagca tcctcctgtc 3180
   cccagctgtg tccagcctgt ctgcagggac atcctgagag gaccaggctc tcccctcatc 3240
   cacctgccta agtgccactc tgaaccctgt ccacctgtgc cgtggagggg cgtgacctca 3300
   agctgctcag ccagcagcag gcttggccct ggggggcagc agagacccag gtggctgtgg 3360
   ggtgggtgct tcgtggcgtg gttctgaaac ttcgttggaa gtgtgtggac agtgccttgc 3420
   ctgttctctg tgggacccta tttagaaacg aggtctgagt tactgggggt catcactgtg 3480
   ttctgatggc ccagctgtgt ggaggccgcg gtgcagcccc atccaaggag ccagggccct 3540
   gggtctagcc gtgaccagaa tgcatgcccc ggaggtgttt ctcatctcgc acctgtgttg 3600
   cctggtgtgt caagtggtcg tgaaactctg tgttagctct tggtgttcct gaaagtgccc 3660
   ccgggtctca ggcctcagaa ccagggtttc ccttcatctc ggtggcctgg gagcatctgg 3720
   gcagttgagc aaagagggcg attcacttga aggatgtgtc tggccctgcc taggagcccc 3780
   ccggcacggt gctggggcct gaagctgccc tcgggtggtg gagaggaggg agcgatgaag 3840
   tggcgtcgag ctgggcagga agggtgagcc cctgcaaggt gggcatgctg gggacgctga 3900
   gcagcatggc cagcagctgg gtctgcagcc tggtacccgg cgggacttgt ggttggggct 3960
   ggtttgtggc caggagaggg gctggcagga gacaaggggg actgtgaggc agctcccacc 4020
   cagcagctga agcccaatgg cctggctgtg tggctctcag ctgcgtgcat aacctctcag 4080
   tgcttcagtt ctctcatttg taaaatgagg aaacaaacag tgccagcctc ccagaggtgt 4140
   catgaggatg aacgagtgac catgtagcat gggctgggtg cgtgtcacct aacatcacca 4200
   gcctttgcaa ggagagccct gggggcctgg ctgagtattt cccttgcccg gcccacccca 4260
   ggcctagact tgtgcctgct gcaggccctt gacccctgac cccattgcac ctgtctccac 4320
   aggagccgag gaggtgctgc tgctggcccg gcggacggac ctacggagga tctcgctgga 4380
   cacgccggac ttcaccgaca tcgtgctgca ggtggacgac atccggcacg ccattgccat 4440
   cgactacgac ccgctagagg gctatgtcta ctggacagat gacgaggtgc gggccatccg 4500
   cagggcgtac ctggacgggt ctggggcgca gacgctggtc aacaccgaga tcaacgaccc 4560
   cgatggcatc gcggtcgact gggtggcccg aaacctctac tggaccgaca cgggcacgga 4620
   ccgcatcgag gtgacgcgcc tcaacggcac ctcccgcaag atcctggtgt cggaggacct 4680
   ggacgagccc cgagccatcg cactgcaccc cgtgatgggg taagacgggc gggggctggg 4740
   gcctggagcc agggccaggc caagcacagg cgagagggag attgacctgg acctgtcatt 4800
   ctgggacact gtcttgcatc agaacccgga ggagggcttg ttaaaacacc ggcagctggg 4860
   ccccaccccc agagcggtga ttcaggagct ccagggcggg gctgaagact tgggtttcta 4920
   acaagcaccc cagtggtccg gtgctgctgc tgggtccatg cgtagaaagc cctggagacc 4980
   tggagggagc cctttgttcc cctggcttca gtttcctcat ctgtagaatg gaacggtcca 5040
   tctgggtgat ttccaggatg acagtagtga cagtaagggc agcctctgtg acactgacca 5100
   cagtacaggc caggcctctt tttttctttt tttttttttg agatggagtc tcactctgtc 5160
   gcccaggctg gagtgcagtg gtgtgatctc agctcactac aacctctgcc tcctgggctc 5220
   aagtgattct cctgcctcag cctcctgagt agctgggatt acaggtgcct gccactgtgc 5280
   ttggctaatg tttgtatttt tggtagagat ggggtttcac cgtcttggcc aggctggtcg 5340
   caaactcctg acctcaggtg atccacctgc ctcagcctcc caaagtgctg ggattacagg 5400
   catgagccac cacgcccggt caggccaggc ctcttttgaa cactttgcac accatgggtc 5460
   ttttcatcca ggggggtagg tacagttgta cagttgagga cactgaagcc cagagaggct 5520
   cagggacttg cccagggtca cacagcagga tgtggcaggt gtggggctgg gcctggcagc 5580
   gtggctccag ctttccagca tagaaatctg tgaaagcaga tagtttgtcg gtcggtaggg 5640
   gagactttct gagacccgcc ccagcggctc agagggtagt agccaggggc cttcctgggg 5700
   gctcataacc cagaacactg aatgggaaaa ccctgatgga ggaggcgcag tggagctgtg 5760
   ggtgccgatg ggaagtccca gaggagctgg gaggtcagta gcggtgctgc cctctgtgga 5820
   gcacttagtg ggcaccaggt gtgtttccag gttcatggcc ctgggacctg aagctcagaa 5880
   ggtgaagtaa cttgcccagg gcacccgtcg ggcagcggcg ggcagaggat ttgtgggctg 5940
   tggagcctgt gctcgtggcc cagccctggg ggttgtgagt gtgctggccg gggagctttt 6000
   cctgcaagtg gactggtgtc taggagccag catgtcaggc agcaggcagc gggagtgcag 6060
   caggcagcgg gagcacagca ggcagagggc ggggctcgag cagccatccg tggaccctgg 6120
   ggcacggagg catgtgggag agggctgctc catggcagtg gctgaagggc tgggttgtgc 6180
   cccgaggagg gtggatgagg gtaagaagtg gggtccccag gggctttagc aagaggaggc 6240
   ccaggaactg gttgccagct acagtgaagg gaacacggcc ctgaggtcag gagcttggtc 6300
   aagtcactgt ctacatgggc ctcggtgtcc tcatctgtga aaaaggaagg gatggggaag 6360
   ctgactccaa ggcccctcct agccctggtt tcatgagtct gaggatccca gggacatggg 6420
   cttggcagtc tgacctgtga ggtcgtgggg tccagggagg ggcaccgagc tggaagcggg 6480
   aggcagaggg gctggccggc tgggtcagac acagctgaag cagaggctgt gacttggggc 6540
   ctcagaacct tcacccctga gctgccaccc caggatctgg gttccctcct tggggggccc 6600
   cagggaacaa gtcacctgtc ctttgcatag gggagccctt cagctatgtg cagaaggttc 6660
   tgctctgccc cttcctccct ctaggtgctc agctcctcca gcccactagt cagatgtgag 6720
   gctgccccag accctgggca gggtcatttc tgtccactga cctttgggat gggagatgag 6780
   ctcttggccc ctgagagtcc aagggctggt gtggtgaaac ccgcacaggg tggaagtggg 6840
   catccctgtc ccaggggagc ccccagggac tctggtcact gggcttgccg ctggcatgct 6900
   cagtcctcca gcacttactg acaccagcat ctactgacac caacatttac aaacaccgac 6960
   attgaccgac accgacattt accgacactg acatttacca acactgttta ccaacactga 7020
   catctactga cactggcatc taccaacact gacatttacc gacactgaca tttaccaaca 7080
   ctatttacca acactgacat ctactgacat tggcatctac caacaccaac atttaccgac 7140
   accaacattt accaacactg aaatttaccg acaccgacat ttaccgacac cgtttaccaa 7200
   caccgacgtt taccgacacc gacatttacc gacactgata tttaccaaca ctgacatcta 7260
   ctgacgctgg catctactga caccgatgcc agcatctacc aacaccgaca tttaccaaca 7320
   ctgacattta ctgacactga tatctactga cactggcatc tactgacacc aacatttacc 7380
   aacaccagca tctaccaaca ccgacattta ccaacaccag catttaccaa caccgatgtt 7440
   taccaacgcc gacgtttacc gacgccagca tctaccaaca ctgacattta ccgacaccga 7500
   catttaccga cactgacatt tactgacact gacatctact gatactggca tctaccgaca 7560
   ctgatattta ccaacgccag catctactga cactgatgtt taccaacacc gacatttacg 7620
   agcaccgaca tttactgaca ccaatattta ctgacatcaa catttagcca tgtgatgggg 7680
   gccggcttgg gggcaggcct tgctcttggc actggggatg ctgcagagac cagacagact 7740
   catggggtca tggacttctg cttcttctcc agcctcatgt actggacaga ctggggagag 7800
   aaccctaaaa tcgagtgtgc caacttggat gggcaggagc ggcgtgtgct ggtcaatgcc 7860
   tccctcgggt ggcccaacgg cctggccctg gacctgcagg aggggaagct ctactgggga 7920
   gacgccaaga cagacaagat cgaggtgagg ctcctgtgga catgtttgat ccaggaggcc 7980
   aggcccagcc accccctgca gccagatgta cgtattggcg aggcaccgat gggtgcctgt 8040
   gctctgctat ttggccacat ggaatgcttg agaaaatagt tacaatactt tctgacaaaa 8100
   acgccttgag agggtagcgc tatacaacgt cctgtggtta cgtaagatgt tatcattcgg 8160
   ccaggtgcct gtagacacag ctacttggag actgaggtgg gaggatcgct ggagtccaag 8220
   agtttgaggc cagcccgggc aaaggggaca caggaatcct ctgcactgct tttgccactt 8280
   actgtgagat ttaaattatt tcacaataca aaattaagac aaaaagttaa tcacatatcc 8340
   actgccctgc ttaagacaga aaacatgggt gttgttgaag ccagaggcag ctgctggcct 8400
   gagtttggtg attggttcct aagcagttga aggcagtttt gtttttccat agatgtctgt 8460
   tctccctttg ctgggtgcag cctcgccctg ctgctgtggt cgggtttcag tggcctcgtc 8520
   ccgtggacgc agcctcgccc tgccgctgtg gtcgggtttc agtggcctcg tcccgtggac 8580
   gcagcctcgc cctgctgctg tggtcgggtt tcagtggcct cgtcccgtgg acgcagcctc 8640
   gccctgccgc tgtggtcggg tttcagtggc ctcgtcccgt ggacgcagcc tcgccctgcc 8700
   gctgtggtcg ggtttcagtg gcctcgtccc atgggcgtgc tttggcagct ttttgctcac 8760
   ctgtggagcc tctcttgagc ttttttgttt gttgtttgtt tttgtttgat tttgtttgat 8820
   tgtttgtttt tgttgtcgtt gttgttgccc aggctggagt gcagtggcgc gatctcagct 8880
   cactgaaacc tctgcctcct tgggttcatg ccattctcct gcctcagcct cccacatagc 8940
   tgggattaca agtgcccgcc accacgcctg gctaaatttt gtatttttag tagacagggg 9000
   gtttcaccat gttggtcagg ctggtctgga actcctggtc tcacatgatc cacctgcctc 9060
   ggcctcccaa agtgttggga ttacaggcgt gagccaccgc gcccagcctc tgttgagcat 9120
   attttgaggt tctcttggtg ccagtgatat gtacatgtgt ccccatcgca ccatcgtcac 9180
   ccattgaggt gacattggtg cctctcctcg gggtggatgc ctccctctgt ttccagcaac 9240
   ttctgaagga ttttcctgag ctgcatcagt ccttgttgac gtcaccatcg gggtcacctt 9300
   tgctctcctc agggctccca ggggaggccc gaatcaggca gcttgcaggg cagggcagga 9360
   tggagaacac gagtgtgtgt ctgtgttgca ggatttcaga ccctgcttct gagcgggagg 9420
   agtctcagca ccttcagggt ggggaaccca gggatggggg aggctgagtg gacgcccttc 9480
   ccacgaaaac cctaggagct gcaggtgtgg ccatttcctg ctggagctcc ttgtaaatgt 9540
   tttgtttttg gcaaggccca tgtttgcggg ccgctgagga tgatttgcct tcacgcatcc 9600
   ccgctacccg tgggagcagg tcagggactc gcgtgtctgt ggcacaccag gcctgtgaca 9660
   ggcgttgttc catgtactgt ctcagcagtg gttttcttga gacagggtct cgctcgctca 9720
   cccaggcgag agtgcagtgg cgcaatcacg gctcgctgta gcctcaatct ccctgggctc 9780
   aggtgatcct cctgcctcac cctctgagta gctgggacta cagacacata ccaccacacc 9840
   cagctagttt ttgtgtattt tttgtggggg gagatggggt ttcgctgtgg tgcccaagct 9900
   gatctcaaac tcctgaggca caagcgatcc acctgcctcg gcctcccaaa gtgctgggat 9960
   gacaggcatc agccgtcaca cgcagctcaa tgattttatt gtggtaaaat aaacatagca 10020
   caaaattgat gattttaacc attttaaagt gaacagttca ggctgggcgt ggtggcttat 10080
   gcttgtaatc ccagtacttt gagaggctga ggtgggcaga tcacctgagg tcaggagttt 10140
   gagaccagcc tggccaacat gatgaaatcc agtctctact aaaaatacaa aaattagccg 10200
   ggcatggtgg caggtgcctg taatcccagc tactcgggag gctgaggcag gagaatcgct 10260
   tgagcccggg aggtggaggt tgcagtgatc tgagatcatg ccactgcact ccaatctgtg 10320
   tgacagagca agactctgtc ttgaaaaata aataaataaa aaaaatttta aaaagtgaac 10380
   aattcagggc atttagtatg aggacaatgt ggtgcaggta tctctgctac tatctacttc 10440
   tagaacactt tcttctgccc tgaaggaaac cccatgccca ccggcactca cgcccattct 10500
   cccctctctc ccagcctctg tcaaccacta atctactttc tgtctctggg ggttcacttc 10560
   ttctggacgt tttgtgtgac tggaatcctg caatatgtgg tccctgcgtg tggcttcttt 10620
   ccatagcatt gtgttttcca gattcaccca cacattgtcg cacgttatca gaatctcatt 10680
   cctgactggg tgcagtgggt taggcctgta atcctaacat tctgggaggc caaggcggga 10740
   cgatcacttg aggcaggagt ttgagaccag cctggccagc ctagcaagac cccagctacc 10800
   aaaaaatttt aaaagttaac tgaacgtggt ggtggtgggc acttgtggtt cccagctacc 10860
   tgggaggctg aggtgggagg atcgcttaag cccaggaggt caaggctgca gtgagctatg 10920
   atcgcaccac tgcactccag cctggacaac agagcaagac cctgtctgaa aaaaaaaaca 10980
   aaaaaaaaag ttcctttctt tttgtggctg gatgacatcc cattgtatgg ccacagcaca 11040
   ttttgtttgt ctgtttatcg ggtggtgggc agtggtttcc accttttgtc tcctgtgaat 11100
   aatgctgctg tgaacatttg aattcaagtt tttgtttgaa cacctgttgt gaattatttg 11160
   gatatatgtg taggggtagg attgctgagt cctatggtaa tgttaggttt gacttactga 11220
   ggaaccatta aactgttttc aacagtggct gcgccgttct gcatccccac cggcagtgtg 11280
   tgagggttct gactttacct cctcacaaac gcttcttttc catttaaaaa aatattcagc 11340
   caggtgctct ggctcacgcc tgtaatccca gcactttggg aggccgtggc gggcggatca 11400
   cctgaggtca ggagttcgag acgagcctgg ccaacatggt gtaaccccat ctctaccaaa 11460
   aatataaaaa ttagccgggt gtggcagcgg gcgcctgtaa tcccagctac ttgggaggct 11520
   gaggcaggag aatcacttga acccgggagg cagaggttgc agtgagccaa gatcgcgcca 11580
   ctacactcca gcctgggtga caagagtgaa actccatcta aaataaaaca aaaataaaaa 11640
   taaataaaaa tttattaaaa cattcatcac agccagccta gtgggtgtcc catgtggctt 11700
   tgcctcgcat ttccctgata actaggatgc tgagcgtctt gtcccaggct tgccacacct 11760
   cagcactttg agatacgtcg cacagtcccc atttgcgaac gagaaatgag gtttagggaa 11820
   cagcagctgt gtcatgtcac acagcgagca gggggtctct gagccgtctg accccacagc 11880
   cgaccaagct ccaatcctta ccgcctccta gtgttgtgga tgtagcccag ggtgctccca 11940
   catttttcag atgagaacac cgaagctcaa aacaggagcg ttttgtccac attggataca 12000
   cgatgtctgt ggtttggtcc tgaagtcact ttatatctca gtggtccaga ctggagtagg 12060
   acagggggtt ctggggaatg gggaaggtgt ctcaggtgaa aggaaggaat tccagattct 12120
   ccatactgtc cttgggaagt tagaagactc agagggtctg gcaaagtcag acaaagcaag 12180
   agaaatgcag tcaggaggaa gcggagctgt ccaggaacag gggggtcgca ggagctcacc 12240
   cccaggaact acacttgctg gggccttcgt gtcacaatga cgtgagcact gcgtgttgat 12300
   tacccacttt tttttttttt ttgaggtgga gtctcgctct cttgcccagt ctggagtgca 12360
   gtggcacgat ctcggctcac tgcaagctct gcctcccggg ttcatgccat tctcctgcct 12420
   cagcctcccg cgtagctggg actacaggcg cctgccaccg cgcccggcta atttttgtat 12480
   ttttagtaga gatgggattt cactacatta gccaggatgg tctcgatctc ctgacctcat 12540
   gatccgcccg tctcggcctc ccaaagtgct gggattacag gcgtgagcca ccgcgcccgg 12600
   cccgatttcc cactttaaga atctgtctgt acatcctcaa agccctatac acagtgctgg 12660
   gttgctatag ggaatatgag gcttacaggc catggtgctg gacacacaga agggacggag 12720
   gtcaggaggt agaagggcgg agagagggaa caggcggagg tcacatcctt ggctttcaaa 12780
   atgggccagg gagagacacc ctctgagcat ggtaggacag gaaagcaaga ttggaacaca 12840
   ttgagagcaa ccgaggtggc tgggcgtggt ggcttacgcc tgtaatccca acactttgga 12900
   aagctgaggt gggtggattg cttgaggcca ggagttcaag accagcctgg ccaacatggt 12960
   gagaccccgt ctctactaaa tatacaaaaa ttagccaggc gtgatggtgc atacctgtaa 13020
   tcccagctgc ttgggaggct gaggcaggag aattgcttaa acctgggagg cggaggttgc 13080
   agtgagccga gatcccgcca ctgcactcca gcctgggcca cagagtgaga ctccatctca 13140
   aaaaaaaaaa aaaaaaaaga taaaaagacc aaccgaggaa ttgaagtggg ggggcgtcac 13200
   agtagcagaa gggggatcgt ggagcaggcc accctgtggt catgcactgg aagctcatta 13260
   cctgacgatt tggagctcat cactgggggc ctaaggagaa tagatactga aggatgagga 13320
   gtgatggcgc ggggcacggg tgtctttggt ggccagaact tggggactgc tggggtgcct 13380
   cactgcaggc cttctcagcg ccctttatat gcttacacag gctgtttcta agagggggat 13440
   acattgcata agcgttttca gactacctca tcatgggtcc ctttctttac cctctgtggc 13500
   cctggtggcg cactctctgg gaaggtgcag gtggatgccc agacccgccc tgccatccac 13560
   ctgcacgtcc agagctgact tagcctcgag attgctgctg gcacctcctg ccccgggaca 13620
   cctcggatgt gcccgtggag atgctggctc tgtgttttct gctggagttt ggtgcgtctt 13680
   ttcctcctgc aagtggccac cgctcttggg tatgtcctca ggcttctgcg agtcatggct 13740
   gcttctcagg tccttgccca gcgccaggag caaaccctcc tggcactttg ttcaggggtg 13800
   gatgcgccag tgttcctgct gtggaccccc atctcacatg agggtcttgg gcctgcaggc 13860
   tcgttcagga aacacccgct gagtacgcag tgtgtgccag ctgtgtccca ggcaatggcg 13920
   gggacagtgg ctgctgctgg ggttgtggtg gcttctgggg actctgggga cagctgaggt 13980
   gcaaggagcc acggctcctt gaggatgcag ttggactcca ggtggaaggg atggttgggg 14040
   gaggtataaa tggggtcagg gaggagacac atttggaaca atgggaacat ttttaagatg 14100
   ctatgtcggg aggcaacaag gtggccaacc caggtgctga ggagcccaca ccagccctgg 14160
   acgtgttttg ccgctcacct ttgctgggga gtggtgggag agaggattcc gttccacgtg 14220
   gtggtgtgcg cagctgggct gtgtggagct gggcgctagg aggaaggtgc tttctgcggg 14280
   gctagccggg ctctgccttt gaacacaatc aggctccagg ttttcagcat ccagtgcatg 14340
   agaggacttc acgggcagct gtggctgatc ccttgatgaa ttgggagaag aacaaaggtc 14400
   tatgaaatga ggtttcatgt agatggcatt agagacgccc acaacagatt tacagagtgg 14460
   agcggagacg gcggatgggt ctgggaggcc cctcctgctg gccttgactg tgacagctgt 14520
   cctgggaatc agcttccagg ccgccccagc agcctgactg acacacacag gggttttagc 14580
   cccatcctgc gaccagctgt tgccatcatc agtgacagct gggagtggcg gtggttccag 14640
   ccctgggcac cctccccacc tgctggggcc cacccagggc agtcctgaca cctacaggtt 14700
   gcttggagcc gcatccgagt cctgccccac cacgtgtgaa gcccgagtgg tcgtgggctg 14760
   aggtcccctg attgcatccc cacttccctt ctgcttcaca tagctgcctc ttctcaccgt 14820
   ttttccagcc tcctgggcta ggaattccag tgttgtgctg gctttgcccc aggacacctc 14880
   cttagccctc ttcctgagtc tagagccccg ggggttggaa gttctggccc ctgggacacc 14940
   tgcagccaca ctcagcttct cctgtgagcc tccagcatgt cccctcagga ccaagccctc 15000
   acgttcttgc ctccccgccc acctgggctc agccagggga aggcctggct gggagcgtct 15060
   cccctctgcc ctgcccttct cccctctacc ctgcccttct ctcctctgcc ccgccatggc 15120
   ttttatatcc tgtgccacaa gacatggctg tgtgtgaaag tggcagggtc tggcatctct 15180
   gtgggtctct gaggcccacg ctccagtgcc actcttccca cccgctggcc gtgccctcat 15240
   gctggaggga cagcccagcc ctctcccgaa ccccagcccc atgtgcccag ctgcccccgg 15300
   ccctctcccc tggaagccgg ggtcactcca gccgtatgcc atggtgggga catcctgctt 15360
   ccttggcctt ccagggaagg tcctctttcc aaatggcgac acctggtccc tgcctggagg 15420
   ctggaagctg tggcccttgt atgcccctcc agggtctgtg cgctcggttg gcccgagttc 15480
   ccatcaccgt catcatcacc atcatcattg tcatttcgct tgtctgtgag ccggcctggt 15540
   ctcccagagc agagaccctc tgaggtccag cctgagttgg ggtctccgtg ctgacccctg 15600
   acggggactc aggacgtacc aggtctgggt caggagtgac ccccaaacct cgtgcccttt 15660
   gacaggcacc cctgactttt gctaagtggg tggaggtgac atcacttaca gcgggagtga 15720
   tgggacaggg tctgttggct gcactgtgct cccagggatc tggggagagg ctatatccct 15780
   gggctttggc actgcagagc tgtgtgtgtt tgtgtgtgtg tgtgtgtgtg tgtgtgtgtg 15840
   tgtgtgtgtg tgtgtgtgtg tttgcgtgcg cgcacatgtg tataagatct ttttttatta 15900
   catgaagcaa gataactgtt gctgtttcct tttgggtttt gtgttcaaca gagtggggta 15960
   cttcttccct cagacaacag aactctcccc tttaaacacg tgctgtcaga gggtgggtct 16020
   tgggctcatg tctgtttgca cagccgagtc agaggaaaca cagggttctt cataaaaaca 16080
   ctgcacagca ggcgactgtc cagagtcagc ctgcaggacg gcagcagccc tgcccctcag 16140
   agcacagcta gggtgggctg ctttgggatc tcccgtcatt ccctcccagc tggcagccgg 16200
   cggccggccc attccttggt gtgctggtca ggggggcgtg cgcctgctct gctcaccctg 16260
   ggaatgggac agaagctggc agctcggaga ggacagggct ggacccttgg gtggcctctg 16320
   gctggaccat ctcattgtcc tcagacacag cctctcgggt ctagtttcat ttcctgaaaa 16380
   acaagtgcac agaactagag caggagtcga gagctacggc ccccgggcca gatccagccc 16440
   tgccacctgt tttcacacca tgctcaagct gagtgggttt tacatttttt aattacttga 16500
   aaaaaaaaaa gccaaaggag gtttcatgac ccatgaaaat tatatggaat tcaaaaaaaa 16560
   aaaattatat ggaattcaaa tttcagtgtc cataaataat ttcttgagac agggtctcgc 16620
   tctgtcaccc aggctggagt gcagtgctat ggcatggctc gctgtaccct tgacctccca 16680
   ggctcaagcg atcctcctgt ctcagcctcc tgagtagctg ggactacggg tgtgtgccac 16740
   caagcccggc taattttttt ttaattttag taaagacagg gtctttctat gttgcccagg 16800
   cttttctgga actccatctt ggcctcccaa agtgctggga ttacaggctc gagccacgga 16860
   gcccagcctg tttttgtttt ttcactgata aagttttgcc gggtgtggta gtgtgtgcct 16920
   ctagcgattt gggaggctga ggtgggagga tcgcttaagc ccaggagttt gaggctgggc 16980
   tcaagtgatc aggaggtgaa ctatgatcat gtcattgcat tccagcctgg gtgacagagc 17040
   aagaacctat ctcttaaaaa tatatattta aaaagtattg ggtgtggtgg ctcacgcctg 17100
   tggtcccagc tacttaggca tctgaggtgg gaggatggct tgagcccagg agtttgaggt 17160
   tgcagcgagc caagatcgtg tcactacact ctagcctggg tgacagagcc cagaccctgc 17220
   ctctttaaaa aaaaaaacca aaaaacatgt attggaacac agccatgcct gttcagtcac 17280
   gtgctctcca tgctgctttc tgctccagag acccttatgg cctgaaagct gaaaatattt 17340
   tctatccttt acaaaaaagt ttgctgacct ctgtcctgga aaattcatct cccaagttct 17400
   cttccggcac tggcgttcct gggtgtccta aatttggccc ctgttatttc tgaactctgt 17460
   tttggctctg ttccctccca ggagccagga caggcacgtt ctctgcatct tgtcccctga 17520
   cgcccagagg cttggctcgg ctcaggcatt cttggaaata tctggctcca ggaaaggcag 17580
   aggcctcctg agtcagccca gagggaacct gccccaggtc tgggggaggc ctgacccagc 17640
   agagtggctt ttgccgatgg gttgggccgg tcaagatgtg ctgaaagttg tcctcagaag 17700
   gccactttgg gattccttcc tccagtatta gagcaactga gagctgctca ttgcaagcct 17760
   gatgttttcc cagttggccg ggtccaccgg gtgccctggg attctgggat ctgggtggaa 17820
   agtagggggc ttgggggagt gtcctgggtt ctggaatcca ggtggcaagt ggtgaggttc 17880
   agggagtggc ttctgagcca ccataggggt ctctgtggga ggctctgccc atccaggaga 17940
   ttccgcaggc cctgccggcc cagagccagc gtcttgcgct tgccgaggct acagccagcc 18000
   ccagccgggt ggaacagccc gtcgcctcct ctcactttgt tttggggcca cctgggagtg 18060
   tggagcaagg gtagagaggg aggaagtggc tgccggccgc tgcccagcac ccttgtttgc 18120
   cttgggccct ctgtgggctc ctttttattg ctcttcaatg aagccaggga aatggacttc 18180
   cttgcctcac ttcagttcaa catgtctgga agtttggtat taaaattaag aaagtgtgga 18240
   aatagagcaa gaagagaaaa atctctccaa gagataatag tgacctctga gctgggcgcg 18300
   gtggctcacg cctgtaaatc ccagtacttt gggaggctga ggcgggcaga tcacctgagg 18360
   tcgggagttt gtgaccggcc tgaccaagat ggagaaaccc cgtctctact aaaaataaat 18420
   aaataaataa ataaataaat acaaaattag ccaggcatgg tggcgcctgc ctataatccc 18480
   agctaaggca ggagaatcgc ttgaacctgg gaggcaaagg ttgcagtgag ccaagatcac 18540
   gccattgcac tctagtctgg gcaacaagag tgaaactccg tctcaaaaaa aataaataaa 18600
   taaaaaataa aaatagtgac ctctggccag gtgtggcagc tcatacccgt aatcccagca 18660
   ctttggaagg aaggccgaga tgggcagatt gctttagcac aggagtttga gaccagcctg 18720
   gccaacatgg tggaacccca tctctacaaa aatagaataa aatttaagag gtaatagtga 18780
   ccttttggta gatcgaaacc tggattgctt tctttttcta aatgctgatt cttttctttg 18840
   tggtgtttgt gttctgtgcc gatgtccctc ccccagccct gttattgtga gtggaagaag 18900
   gggaaagggt tcgcccgcta ctgtgagccc ctcctctcac gctgggtgtc cttggagaag 18960
   cctgcacttc ttcattgtac gccagggctg ggtccctccc tggagtggtt ctgtgctgct 19020
   gggatggggc caacccctca gatgttttct gagtgtcaca cacaggtgtg tgcattcatg 19080
   gcctttgcgt gtcttcctgt tgtggaggca aaaatgtgaa gaaccctaga tgattttggg 19140
   accagggctc catcacctgc tgttcattgc acaccggagc atccaggcat gggtggagag 19200
   ctcagacttc caggcacggt cgcaggggct ggtctaacca tgttcccgcc cgcctgctcg 19260
   tcagaaccgc ctgttgggag ctgttatcat gataccatac ctgggccctg ggctatccga 19320
   ttctgactta attgctccag gttggggcca ggccgttgtt tgctgttttg ttgtttcttc 19380
   tgtgacgtta gccactgggc taatctgagc ccctcagtta caggtggaga aactgagacc 19440
   catgggggtg caaggacttg ccgaggaccc agagcccctt gggggcagag ctgaggcggg 19500
   gcctggcttt gggtcccaga gcttccagtc cccttcccgc tctcctaaca gctttttttt 19560
   ttgagacaag atctcaccct gtcacccagg ctggagtgca atggcatgat ctcggctcac 19620
   tgcaatcttc gctagctgcg ttccagcgat tctcctgcct cagcctcccg agcagctggg 19680
   attacaggtg tgtgccgcca tgcccagctc gttttttttt gtacttttag tagagatagg 19740
   gtttcaccat gttggccagg ctgatctcga actcctgacc tcaaatgatc cgcctgcctc 19800
   ggcctcccaa agtgctagga ttacaggctg ggatcacact gtgcctggcc ctagcagctt 19860
   tgtcctgtgc catccaacaa cagatgaccg aagtctttgt ttcttaacat gcattccatc 19920
   tgccttacag ttttgccacc tgcaaaacag aggacttgtc gcttttctgg taagctggaa 19980
   atgtaatctg gtagcaggag gcctgtggaa gcttgccttt aatggccttg tgtctctttc 20040
   atcctgtcct gagagccgga gaacttggat gttgcaccta actcaacctt cctgttaaca 20100
   tacagttctg caggctcatg gatcatcaga accacgtcct atctcacgcg gctgtatgct 20160
   tccgttggtt caggtgtttt taccttgaca gtattttctc ctcggtggct tttgcggtgg 20220
   ttgcttttaa tcagcattga ctcttcaaga aaaatattta gctgctacat ctcagaggag 20280
   acagggtgga aagcatctga gacctgcagg ctcagactta gaaccagaag tgccctcaga 20340
   gttcatccgg ccctgaccca gcgggaaatg agttcacaga gaagcgggag aactttgccc 20400
   caggccctgc cgttgctcat aactgcccca ggtccttaca tttgctccag gtcctgcccc 20460
   aggccctgca gttgctcata actgccccag gtccttatat ttgctccagg tcctgcccca 20520
   ggtcctgcag ttgctctgtg tggtgggtgt gatctggagc cctccgccca ttgctgcacc 20580
   tggggcaggc attgctaatt gatcccagga ctccttcctg cggagcacgc cctggttctc 20640
   caggcagccg ctgcctgtca gcctgcagtg gttcgggaga ggacacctgc ttgcctggtc 20700
   tgttccaaat cttgcttctc atcccagcac aggtaggggg tgctatggga aagggatcct 20760
   cagttggccc tgtcactgct ctatcagctg gggacgtggc atcctagtga aaacatcatg 20820
   gccgggcgcg gtggctcacg cctggaatcc cagcactttg ggaggctgag gagggtggat 20880
   cacttgaggt cagaagttcg agaccagcct ggtcaacatg gtgaaaccca tctctactaa 20940
   aaatacaaaa attcgccagg tgtggtggcg ggtacctgta atccgagcta ctcgggaggc 21000
   tgaggcagga gaatcgcttg aacctgggag gtggagcttg cagtgagccg agatcttgcc 21060
   actgcactcc agcctgggca acagagtgag acgctgtctc aaaatctcaa acaaacaaac 21120
   aaacaaaaaa caaacaaaca aagcgtcatt tatccagcac ccctggggaa ccatgctacc 21180
   tggtgtttta tggtacctgg caaggtgcag gtgaagttgc tgctcttggg cattgaaccc 21240
   gtcttgtttg gggcagctca ggccccaggc agggtccggg ttggctctcg ttggtgtggc 21300
   cctggcccat ccagacctat atttctgccg tcctgcaggt gatcaatgtt gatgggacga 21360
   agaggcggac cctcctggag gacaagctcc cgcacatttt cgggttcacg ctgctggggg 21420
   acttcatcta ctggactgac tggcagcgcc gcagcatcga gcgggtgcac aaggtcaagg 21480
   ccagccggga cgtcatcatt gaccagctgc ccgacctgat ggggctcaaa gctgtgaatg 21540
   tggccaaggt cgtcggtgag tccggggggt cccaagccat ggctcagcca tgcagacttg 21600
   catgaggagg aagtgacggg tccatgcctg ggcataagtg ttgagctcag gtgccccgac 21660
   ctggggaagg gcaggacagg aaaggtgaca gtatctggcc aaggacagat gggaagggac 21720
   caagggagct gattagggag tggttatgga ctaggaatgt cggtaacaat ggttagaaag 21780
   tgactaacat ttgttgagca cctgctgtgt gcccggccct ggccgggagc cttcgtgccc 21840
   acagtgaccc cgtctgcaaa tgtagttcct tgccctactc gcactgggga gcaggacgca 21900
   gagccgtgca tctcacaggt gccaagctca ggactccctc ctgggtctgc ctgggctggg 21960
   ctgtgcttgt tgcccctgtg gcccacgcat gtgcaccttc cacctgaaag ccaggatctt 22020
   caggacgctc cccgaggagg tcgttgtctg gcacaatgat ttgtctcttc ctgaaaaggt 22080
   gacagagtta cactggagag agcagcatcc aggtgcggca gggacaggcc tggggctcgc 22140
   gggcagggac tctgtgtcct gccggggtcc cacactgcac ctgcttgtca gaggcactca 22200
   gtcaatcttt gctgatgaag gatgagagga cagaggacgt gatgcttgct gctgcattgc 22260
   ctgcagtcct gggtgagatg cccgggttga ctctgctgcc cgtcgggtgg atgtgatgtc 22320
   agatccccgg ctttaaaata cgagggagct gggaattgag ggagcaggtt ggggcagaaa 22380
   gcacagcccc gtggaagcct ggagctgagg cagtgtgggc gacccctgga gcagtgagtg 22440
   cttccttcat ggccttcatc gcaccctgca gtcctcatgt aggggatgcc atccatgaat 22500
   ttagttttcc cagcctcctt taaaaacgcg ttcatgctgg ggccggggca gtgcagtggc 22560
   tcacatctga aatcccacca ctttgggagg ccgaggcggg tggatcatga ggtcaggaga 22620
   tcgagaccat cctggctaac aaggtgaaac cccgtctcta ctaaaaatac aaaaaattag 22680
   ccgggtgcgg tggcgggcgc ctgtagtccc agctactcgg gaggctgagg caggagaatg 22740
   gcgtgaaccc gggaagcgga gcttgcagtg agccgagatt gcgccactgc agtccgcagt 22800
   ccggcctggg cgacagagcg agactccgtc tcaaaaaaaa aaaaaaaagt acaaaaaaaa 22860
   aaaaattagt ctgggtgtgg tatcacgcgc ctataatctc actactcgag aggctgaggc 22920
   ggagaattgc ttgaacccag gaggtagagg ttgtagtgag cccgtatcgt accactgccc 22980
   tccacctggg caatagagcg agactctgtc tcaaaaagaa aaaaaaaaaa agaacattta 23040
   tgccaggtgt ggtggctcat gcctgaaatc ccagaacttt ggaagactga ggcaggagga 23100
   tcacttgagc ccagaaattt gagagtgtct tccctgggca acatagagag acctcatctc 23160
   taccagaaaa aaaaaaatta gcccggcatg gtggcatatc cctgtggtcc cagctactta 23220
   gggggctgac gtggcaggat cacctgagtc tggaggcaga ggttgaagtg agctgagatc 23280
   atgccactgc actccagcct gggtgacaga cagagaccct gtctcaaaaa aaaaaaaaaa 23340
   aaaaagcatt tactatccac catggaaggt gagactgacc tgtgagtgat tgttcaaaga 23400
   acaaaaaata aaccccagag ataagacaaa agggtgcctc catgggggtg tgatttaaag 23460
   ctgagaaatt gggcttcttc cccctcccct ctcaccccgt ggtttgctaa aggagatggg 23520
   aaaaaggatt ctttttttgg ctgaaatatt taacactaaa ttaaagccaa ttttaacagc 23580
   actttggttg atgagtgaaa ttaacagact ggccaaaaat aaacgaacgg tctgtactat 23640
   gtgaaaaaga ggcagctttg gccatgctgg gccaatgtga gttttcaggg ttgctgggaa 23700
   tgtctgtgaa tcggaggaag ggcctagctg ggactctcag gagccaaggc cctgaggggc 23760
   aacttgcctg gtccctgccc tgaggcgttc actgctttct tcctgggcca gatcacaggc 23820
   ccggaggctg gaccactggg ctggcactct tgccgagctg ctccctgact tcctgaccat 23880
   gctcctttca gcagccttgc tgcactttag tttccttgaa tgaaaaatgg ggatgagaat 23940
   agctcctacc tccaaggtga atggagtgag ttcggacagg tgactccctg ggaccagtgc 24000
   ctggcgcctg acaaggtcca gtcagagccc gcactgctgt tactgatacc cttggctgta 24060
   ccaggggaga acttggttgc cattgccagg tgttctccca ccacccccac tactgtccct 24120
   gtttgatgtg tggcgggaat aaagctgtgc acattggagc ttttggcaca tcctggcttt 24180
   caggtgaaag gtgcgtgtgt gtttgagggt ttagcctggc caacccagcc atgaggtcgg 24240
   acctgacctg ggggtgagtc ctgagctcgg cacccctgag ctgtgtggct cacggcagca 24300
   ttcattgtgt ggcttggccg cacccctttc cctgctgggc tgttgatgtt tagactggag 24360
   cctctgtgtt cgcttccagg aaccaacccg tgtgcggaca ggaacggggg gtgcagccac 24420
   ctgtgcttct tcacacccca cgcaacccgg tgtggctgcc ccatcggcct ggagctgctg 24480
   agtgacatga agacctgcat cgtgcctgag gccttcttgg tcttcaccag cagagccgcc 24540
   atccacagga tctccctcga gaccaataac aacgacgtgg ccatcccgct cacgggcgtc 24600
   aaggaggcct cagccctgga ctttgatgtg tccaacaacc acatctactg gacagacgtc 24660
   agcctgaagg tagcgtgggc cagaacgtgc acacaggcag cctttatggg aaaaccttgc 24720
   ctctgttcct gcctcaaagg cttcagacac ttttcttaaa gcactatcgt atttattgta 24780
   acgcagttca agctaatcaa atatgagcaa gcctatttaa aaaaaaaaaa gatgattata 24840
   atgagcaagt ccggtagaca cacataaggg cttttgtgaa atgcttgtgt gaatgtgaaa 24900
   tatttgttgt ccgttgagct tgacttcaga caccccaccc actcccttgt cggtgcccgt 24960
   ttgctcagca gactctttct tcatttatag tgcaaatgta aacatccagg acaaatacag 25020
   gaagactttt tttttttttt tttgagacag agtcttactc tgttgcccag gctggagtac 25080
   cgtagcgtga gctcagctca ctgcaacctc cgcctcccag gttcaagcga ttcttctgcc 25140
   tcagcctcct gagtagctgg gactacagac atgcaccacc acacccagct aatttttttt 25200
   atatttttag tagagacagg gtttcatcat gttggccagg ctggtcttga actcctgacc 25260
   tcaggtgatc tgcccgcctc ggcctcccaa agtgctgaga taacaggtgt gagccaccgt 25320
   tcccggcata ggaaaacttt ttgccttcta aagaagagtt tagcaaacta gtctgtgggc 25380
   tggccttctg attctgtaaa gaaagtttga ttggtggctg ggtgcggtgg ctcacacctg 25440
   taatcccatc actttgggag gccgacgtgg gcatatcacc tgatgtcggg acttcgagac 25500
   cagcctcacc aacgtggaga aaccccgtct ctactaaaaa tacaaaaaaa aaattaaccg 25560
   ggcatggcgg cgcctgcctg taatcgcagc tactcaggag gctgaagcag gagaattgct 25620
   tgaacctggg aggcggaggt tgtggtgagc tgagatggca ccattgcact ccagcctggg 25680
   caacaaaagt gaaactccgt ctcagaaaaa aaaaagtttg attggtgtaa ccaaagcgca 25740
   tttgtttatg gattgtctgt ggcagctttt gttctgccga gatgagttgt gacagatctg 25800
   tatgggctct aaagcctaaa acatgtgcca tccgcccctt tacagaaaaa gtgtgctgac 25860
   ctctgttcta aagtattgga caactacaat gtttgctcat ttattattct atgatttgtt 25920
   ttctgctttt tgttgttgtt gttgttgttg agatagggtt tccctctgtc actcaggctg 25980
   gagtgcagtg gtgtaatctc agctcactgc agcctcgacc tcctgggctc tagtgatcct 26040
   ctcatctcag cctccctagt agctgggact acaggcacac accaccactc ctggctgatt 26100
   tttttttttt tttttttttt ttgtggagac agggtttccg catgttgccc aggctggttt 26160
   caaactccta ggctcaaaca cccacctcag cctcccaaag tgctgggatt acaggcgtga 26220
   gccaccatgc ccagcctatt ctactgtttg tattacatag ctttaaaaga ttttttatga 26280
   ctttaagtca caagggttct ttgtagaaaa aaatatatat ataggaaagt ataaaaagaa 26340
   agtaaaaatt gtccataacc tctccagcca gagacgaccg ttgctgacac ctcagcatat 26400
   tgcctttaag tcttttttct ctaagatagc atttctcttc atcacagtca tatgctacgc 26460
   agaattctgt atcctgattt tttcacttga cattacaaca ggtatttgat ggcgctgtga 26520
   caaactcttt ggcacaatct tttaaatgta tgaaatactc cactgcacag atgtttgctt 26580
   ttaggcttaa ctgttctttt attttgcgtg tgctggttac agccgggcac agtggctcat 26640
   gcctgtaatc acaacacttt gagagggtga ggcaggagga tcacttgagc ccagaagttt 26700
   gagaccggcc tgggcaacat agtgagaccc catctctaca aaaaactttt ttaataagtc 26760
   gggcgtagtg gtgcatagct gtagtcccag ccaccaagga ggctgagttg ggaggattgc 26820
   ttgagcccca ggaggttgat gctgcagtga cctgagatta ctccactgta ctccaacctg 26880
   agcgacagag caagacttgt ctggggaaaa aaaaaaaaaa aatatatata tatatatata 26940
   tatatataca tatatacata cacgcacaca cacataatat aaaaatatat atttataaat 27000
   atataatata taatataaaa atatatattt ataaataaaa tttataaatt atatttataa 27060
   gtaaatatat aatatataat ataaaaatat atattatata atatataata aaatatataa 27120
   tataaaaata tatatttata aataatatat aatacatact tataagtata tatttaaaat 27180
   atatgtaatg tatatttttt aatgtatgat atataatata catttataaa tacacattta 27240
   tattatttta tataaaatat atataaaatc tccaagttgc tttttccaaa aaggtgtctt 27300
   gctgcatttc aaacattcat ttaaaaactt gaatgctggt gatctggtcc agaatgtgtt 27360
   cagtagctgc tgccagtggc caagcatctc gggagatgtc tacaaaacac gctggttctg 27420
   gcctggcgtg gtggctcacg cctgtaatct cagcactttg ggaggctgag gcaggtggat 27480
   caactgaggt ctggatttcg agaccagcct tgccagcttg gtgaaacccc atctctacta 27540
   agaatacaaa aaaattagcc aggcgtggtg gcatgtgcct gtaatcccac ctacttggga 27600
   ggctaaggct ggagaatcgc ttgaacccag ggggcagagg ttgcagtgag ccgagatcgc 27660
   accattgcac tccaggctgg gcaagaagag cgaaactccg tctcaaaaaa aaaaaaaaag 27720
   atgctggttc ctaaaatgtg gcccttttcc tcctcacctg ctgccagacc atcagccgcg 27780
   ccttcatgaa cgggagctcg gtggagcacg tggtggagtt tggccttgac taccccgagg 27840
   gcatggccgt tgactggatg ggcaagaacc tctactgggc cgacactggg accaacagaa 27900
   tcgaagtggc gcggctggac gggcagttcc ggcaagtcct cgtgtggagg gacttggaca 27960
   acccgaggtc gctggccctg gatcccacca aggggtaagt gtttgcctgt cccgtgcgtc 28020
   cttgtgttca cctcgtatga gacagtgcgg gggtgccaac tgggcaaggt ggcaggctgt 28080
   ccgtgtggcc ctcagtgatt agagctgtac tgatgtcatt agccttgatg gtggccagga 28140
   ctggtagggc cctcagaggt catggagttc cttcgtggag cgggtgctga ggctgtatca 28200
   ggcacagtgc tggctgcttt cacctgggcc gtctcaccga agtgtccatg gagcctgcgt 28260
   agggtgggta tctgtgtcga ttttacagat gcagaaacag gctcagagaa accgagtgac 28320
   ttccctaagg tcacataccc agttagagca gagctgggcc aggaagtgct gtctcaggct 28380
   cctgaccagg tctccttgct ttgcactctt gccaaaacca tgatccagaa ctgactttga 28440
   ggtccccgga cctcaggctc ctccgaaatg gcctcttgga ggctgctgag ccacagctta 28500
   ggacccacct cgagaggcaa atgtgctttg agctgccagg cgtcctgggg gccctgcctt 28560
   gggcacgggg ttcagacagg ccccagatgt gtggggcgtc tttctggact tgagttttct 28620
   tttctgtgtg gtggacacag tgctcacccc ttaaagcacc tgtgatgtgt gcagcagccc 28680
   aatccctgcc tgtcgcctgt tctgctaggg aaggaaggaa gacttcagga tggcaggaca 28740
   acagaaagag gtccaggttt tagagcaagg gcaggtcaaa cttagaaaat tctggaatga 28800
   ggatgtgcat ttcctcttct ggatctgcta aaagaagagg gaaggagggg ctgctggggg 28860
   aggagcccag agccgagttt acatccggat cccgcaaggc ctcccctgcc ctgaggtctt 28920
   gttttgtgat gtgcttgtgt ccatcctggt ttctgccgtg tccccaacat ccggccaagc 28980
   ttaggtggat gttccagcac acactcaccc tgtctgtgca cctgtttttg tgtccgtaag 29040
   tgggtattta ctcaccttac gagtgagcca ctgtgggaat tcagggaggt ggcgcagtga 29100
   ccacccctgg agggatatgt gtgtggcagg ggtcgagggt ctcgcccttc cctgcttcct 29160
   gcgcgtggct ttctccagga cggggagggc tgagctgaag aggtggggac agttgcgtcc 29220
   ccccgccacc cactgtcctg cggtgagagc agactcactg agcctgccct tctcccttgt 29280
   gccttccagc tacatctact ggaccgagtg gggcggcaag ccgaggatcg tgcgggcctt 29340
   catggacggg accaactgca tgacgctggt ggacaaggtg ggccgggcca acgacctcac 29400
   cattgactac gctgaccagc gcctctactg gaccgacctg gacaccaaca tgatcgagtc 29460
   gtccaacatg ctgggtgagg gccgggctgg ggccttctgg tcatggaggg cggggcagcc 29520
   gggcgttggc cacctcccag cctcgccgca cgtaccctgt ggcctgcaag ttccccaacc 29580
   tggcaggagc tgtggccaca cccacgactg cccagcagcc tcaccctctg ctgtgggagt 29640
   tgtccccgtc cacccctggg tgcctttgct gcagttatgt cgggagaggc tctggtgaca 29700
   gctgtttcct gtgcacctgc tgggcactag gtcccagcta atccctgtgc caggactcta 29760
   atttcaccct aacacacatg gtggttttca ttgctgggga agctgaggcc tgagcacatg 29820
   acttgcctta ggtcacatag ctggtgagtt caggatcccc cagagatacc agggccagca 29880
   ctcgatcccc acccagccct gaaccccacc atgtgctggg attgtgctgg gagtgtccac 29940
   acgcctggga ccccagggct ggtgctctca tctccttttt ccagatcatg agaatgaggc 30000
   tcagggaagt ttgaaaaaaa cctatcccaa gtcacacagc aacaggagca ggatttgaac 30060
   ccagaaaagg ggaccgcaca ctctgttctg ctagagtagt tagctgtcct gggtgatatg 30120
   gcaggtgaca ggggcaactg tgcttaacaa aggaaccccc atcccccctg ccaagttggg 30180
   agactagaag gtcaggggca gaagctctga agggccaggt gcagtggctg acacctctaa 30240
   tcccagcact ttgtgaggcc aaggcgggca gatgatttga gcccaggagt tcaagatcag 30300
   cctgggtaat gtagtgagac gccatctcta caaaaaaatt ttttaaaaat tagctgggca 30360
   tggtggttca tgcctgtagt ccaagctact tgggaggctc aggtgggagg attgcttgag 30420
   cccaggaggt tgaggttgtg gtgagctgtg atcatgccac tgcactccag cctgggcaat 30480
   agagtgagac cgtctccaaa aaaaaaaaaa gaagaagaaa aagaagctct gaggctccaa 30540
   gtccccaggc accccttggc ttgagggcag acaagggagg agagggtcac ctgggcagcc 30600
   ctgacttttg tcccctggca aagggacctt cagtgacctt ggccctagga gagcctctga 30660
   gcacgtcagc catgtcgaac cgctcaggaa gggcagcaag aatttggctt ctgacctctg 30720
   cctctcctac tcgccatctg cactgggtgt ggttgtgccc attttacaga tgaggaggct 30780
   ggggcatcga ccagctgaat gccttgtccc aggtactgcg taggcagagc tggcagttga 30840
   accccgtgtc ctggttgtcg ctgggggtgg gctgcaccct gacttgtgag gccagtagca 30900
   aggtttgcac gtgacttcgt gaccgtcacc cagctctgca gcacatcccg tgacccagct 30960
   catccaggcc gcatgcaaac ctgttgccag gcgagaaacc agtcaccgca cagctgtggt 31020
   tgcctgaaat gattaagctc attaatcacc ccggagtgag gacagactca gatgaaaacc 31080
   agcaaaagcc ctggaaactc atgtgaccct gccaatgagg gcggccatgt gcattgcagc 31140
   ctggccgtca ctcctcggta cgtgttttgg acttaaacgc tccggatgtt tactgagtgc 31200
   ttgattaata acatggaagg cctggtctca ttgctgtggg agtgaaggat gcacagccag 31260
   gcctgacatg atgagaacaa gaacctggag tctcgctgcc tgggtggtaa tcctggccct 31320
   gccacttagc aactgtgtga ctgtagccag gtcacttaat tttgctagat cctgcctgcg 31380
   cttcagtgga tcttgctggt tttccaaggt ggccaaacac tttaaggcat tcatgtggtc 31440
   gctaggctgc agggttgaac cctggctcac cccgcagggc gccgtgtgct ctgtggcctg 31500
   gctgtgcctt tgctgacacc gtgcccgtgt gtgttcatgc aggtcaggag cgggtcgtga 31560
   ttgccgacga tctcccgcac ccgttcggtc tgacgcagta cagcgattat atctactgga 31620
   cagactggaa tctgcacagc attgagcggg ccgacaagac tagcggccgg aaccgcaccc 31680
   tcatccaggg ccacctggac ttcgtgatgg acatcctggt gttccactcc tcccgccagg 31740
   atggcctcaa tgactgtatg cacaacaacg ggcagtgtgg gcagctgtgc cttgccatcc 31800
   ccggcggcca ccgctgcggc tgcgcctcac actacaccct ggaccccagc agccgcaact 31860
   gcagccgtaa gtgcctcatg gtcccccgca cctcactccc tcgttagatc aggctggttc 31920
   tgggagctga cgctgaaagg agcttctcat ctggggttcc tgggtgtaca tagatggttg 31980
   ggtaggttgt gcactgcaca agctgcatga tgctacctgg gggtccaggt ccaggctgga 32040
   tggacttgtt gcttcatcag gacatagata aatggccaaa actcctcagc tggaaggtcc 32100
   tgggcaggat ctttgggtgt gaaaaccagt cacaggggaa gggtgcttgc tcatactgcc 32160
   agcacagtgc tgagtgcttt ccatagcgct cgtttactcc tcaagcctgg agggtgggga 32220
   gtagcatggt cccatttcac gtacaaggaa cccgatgcac agagaggtgt ggcaacccat 32280
   ccaaggccat acaactgggg tgggttgagc cggggttgac tgtggcaggc tggctcaaga 32340
   gtccctgctc ctgaaccctt gccaggcagc ctggcatcag ctcggggaat ttttgccctg 32400
   acccttggaa gcaagtgggc ctctttgttc tcatgtcagt gatgagaaga gtgactttcc 32460
   tatggcccct ctggagtaca ggtgtttcct gttggcgggc tcttccccca tgacatcagc 32520
   agcgagctgg ttatgattcc ctacgcagaa cttgatagtt tataaagctc tttgtcatcc 32580
   aggccccgtt ggagtctcac gcagacctgg tcgcaggcgg ggctggtctt gcctgtccca 32640
   gctgcatgga tggggaactt gaggcttgca aaggttaagg ggctgttcga ggcccaggct 32700
   ggcaggagat gggcctgggc cagagtctgg gacttcccat gcctgggctg tctttggtcc 32760
   tgttgctcac catccctccc tggggccatg accttagaga gccaaatgga ggtgcaggta 32820
   acccacggca aggaggggtt gccatgactc agagtccccg tcctgtggcc ggcagtacct 32880
   ggtgcaacga cttggatttc agaccagcca ctgtagcccg ctgacggtgc gctcgaagtg 32940
   ccacagcttc tgaagccagg caggactcag gccaggagac tctgttagct gttgagaggg 33000
   agaggccaac ggatgttctg gttctgctag agagctggtt cttcggatcc tggtaccagt 33060
   gcactgagag gaggcccagc ttgattctgg ggctgccttg tggtggcatg tgctgctcac 33120
   tgacaccctc gaggagtgtc ttctctcggg cttgttgact gtgcccggtt ttccgcagtt 33180
   cactggtgca cacataggca catagcaaac cgcacacaca gtcgtgggta tgagtttcac 33240
   tacattccac caccagtgtt cactaccatt acctgccttc cgtcttaagt gttcatcatt 33300
   taaaaataaa tttattgggc tggacgcggt ggctcatgac tgttatccca gcactttggg 33360
   aggctgaggc gggcagatca cctgaggtca ggagttcaag accagcctgg ccaatatggt 33420
   gaaactccat ctctactaaa aatacaaaat tagctgggca tggtggggca tgcctataat 33480
   cccagctact caggaggctg aggcaggaga atggcgtgaa cccgagaggc agagcttaca 33540
   gtgagcccag atagcaccac tgcagtccag cgtgggcaac agtgcgagac tccatctcaa 33600
   aaaaaaaata aataaataaa agaaaaataa atttatgatc tatttcaaaa ataacacatg 33660
   tactttgaaa cagcagagac acatatgaca cggagaatga aattccccat agcgcacccc 33720
   caagagacag ccctggtccc cccgtctttc ccgtggacct ccagcggggc agatgctgag 33780
   ccgcctgttg tcgagtggcg tgctatcccg tcctccagct cctctgtggc ttacagacac 33840
   ccacctgcag ccctgtcttt gcctcctcta gcgcccacca ccttcttgct gttcagccag 33900
   aaatctgcca tcagtcggat gatcccggac gaccagcaca gcccggatct catcctgccc 33960
   ctgcatggac tgaggaacgt caaagccatc gactatgacc cactggacaa gttcatctac 34020
   tgggtggatg ggcgccagaa catcaagcga gccaaggacg acgggaccca ggcaggtgcc 34080
   ctgtgggaag ggtgcggggt gtgcttccca aggcgctcct cttgctggtt tccaggctgc 34140
   tgcccctgtc cttagcagag ggaggaaaca gaggatggct ctgggtgaat gatgacttgg 34200
   gcttcgatta tgtagtcaca gggtatgacc ctgagatgcg tggaaccccg agactgtgat 34260
   tatatgtaga aactgggttt ccccgttgtt taagtagtca tggtggggtc agaccccaca 34320
   ggacttttgt cttttcaaga aagaaaatgg tcgtgtgtca tgcaggggta gttggtactg 34380
   gttaatccag gtttatcctt tattttgtgg gaactgtaca gtcatttctg ctacaatgct 34440
   gtatatgctc ttctgaaaga cacctatgca aaatcgcaca gtaaaaatga cacaactcat 34500
   agggaaagcg gggccagggc acagccctca aaatctccat caatgacatg taagaaaaga 34560
   gaggaacctg ggaaatagca aagtgccttt tgcacattaa atggttagct atatcccaca 34620
   atactgtgca ttcgtaaacg ttaatgctgc aataaatacg gcacttcacc ttgggaagat 34680
   ctggagttgg cttatgagtg tggaagggtg tagcgcatga gtttttgtga aacactggaa 34740
   ggaggattgt gggaaatcaa atggaaagtt ctcaccccag gcgtggagaa gagtgggtca 34800
   tggccccagc agtgagccca gggaggtcag agacggaggt gtgtgtgtgg gtgtgaccct 34860
   gcgcagttcc ctgccggctg tagttttttg cattcgctta atgtttctcg tggaggaaat 34920
   tgtgcatgag caaatgtgaa accgtgctgt gctcaaattg tcctaataca tcattgcatt 34980
   ggaacagatt ggcttttttt tttttttttt tttttttttt tttttgagat ggagtctcac 35040
   tctgtcacca gcctggagtg cagtggcatg atcttggctc actgcaacct ttgcctccta 35100
   tgttcaagtg attttcctgc ctcagcctcc tgagtaactg ggattacagg catgagccac 35160
   cgcggccggc cagatttgca tttttgaaac aactgctagg ctgggcgcgg tggctcacac 35220
   ctgtaatccc agcactgtgg gaggccgagg caggtggatc acctgaggtc aggggttcga 35280
   gaccagcctg gccaacatgg tgaaaccccg tctctactga atatacaaaa atcagctggg 35340
   tgtggtggcg ggtgcctgta atcccagcta ctcaggaggc tgaggcagga gaattgcttg 35400
   aacccaggag gcagaggttg cggtgagccg agatcacacc attgcactcc agcctgggca 35460
   acaagagcaa aactccatct caaaaaataa aaaatagaaa aacaagtgct gtagcggaag 35520
   tgagcacttt gcggagtcag gcttgtgtgg cctgttccac aaatgatgtg ctcacggtgg 35580
   cctcaggccc acctggagtc tgcagcatgg ggcacaacag gttcattagt gtagaattcc 35640
   aggacaggcc tggctcctaa gcagccttct tttacaaaaa ctgcagagcc cgcctgtatc 35700
   ctagcacttt gggaggccga agtgggtgga tcacgaggtc aggagttcaa gaccagcctg 35760
   gccaacatgg tgaaacccca tctctactaa atatacgaaa attagctggg tgtggtggca 35820
   cgcgcctgta gtcccagcta ctcgggaggc tgaggcagaa ttgcttgaac ctgggaggtg 35880
   gaggttgcag ggatctgaga ccatgtcatt gcactccagc ctgggcaaca gagcgagacg 35940
   ccatctcaaa aaaaaaaaac ctacagagcc acacggcctc tttctccacc gagtgttggt 36000
   gtgggagctt gtgttattgt ggtgaaatct tggtactttc ttgaggcaga gagaggctga 36060
   gcgcctggag agactttcac atgggtcgcc atgtccgccg tcggtttcgc tgttgtgctc 36120
   cccatctgaa ggctggtgcc gtccagacag gctggacgcc cctttccacc agatccttcc 36180
   tcccgcagca gtttctagtt acgttgtact gtgaggtctg tgtccttggt tgatggcaaa 36240
   agtcagccga attgaaattc agagccatgc ctggctccct ggagcttctc tcctgggcag 36300
   ctgtgatcat tgcctctgct gtggtgtggg tggtggaaat ggattccttt catcttgctt 36360
   gctacaggtg actgtcacgt ggagtccttt ggagagaggg acgtgttaat tgatggatgt 36420
   ggctcccatg ctgagaaagc tcctgggcgt acattgcctt agagtttcat tggagctgcg 36480
   ttcttttatg gtgtctgcta ggcagaagtg atgaagactt ggaagaaaac ccagaaggtt 36540
   ttccacttaa tttggaaaat gtgcttttcc cctcctgtgt cttttgctaa ggtccagcct 36600
   cctgcagcct ccccgctctg tggactctgg ctttgattct ttattaggag tccccctgct 36660
   cccccaaaag atggtgtcta aattatcatc caattggccg aggttttgtt ttctattaat 36720
   tgtttttatt ttttattgtg gtaaatttat ataacataaa atttgccatt ttaattgttt 36780
   tgttattgtt gtttttgaga cagggtctca ccccagtgcc caggctggag tgcagtggtg 36840
   cgatcatggc tcactgcagc ctcagcctcc agggctccag tgatcctctc acctcagcct 36900
   ctctagtagc cgggactaca ggcatacact accacatctg gctgattttt tgtatttttt 36960
   ttttattgta gagacccgct atgttgccca ggctggtctc aactcctgga ctcaagccat 37020
   cctcccacct caccctccca aagtgctggg attacaggca tgagccacaa cacccagcca 37080
   ttttaatttt tttttttttt tttgagatgg agtctcactc tatcgcccag gctggagtgc 37140
   agtggcgtgg tatcaactca ctgcaacctc tgcctcccag gttcaagcga ctctcctgcc 37200
   tcagcctcct cccgagtagc tgggattaca ggtgcccatc actatgcctg gctaattttt 37260
   gtatttttta gcagagacgg ggtttcacca tgttggccag gctggtcttg aactcctaac 37320
   ctggtgatcc gcccgcctcg gcctcccaaa atgctgagat tacaggtgtg agccaccgtg 37380
   cccggccttt ttttgttttt gagacagggt cttgccctgt cacccagact ggagtgcaat 37440
   ggtgggctct tggctcactg cagcctccgc ctcccaggct caagttgtgc acctccacac 37500
   ctggctaact gtattttatg tagagacaga tttcaccatg ttgcccaggc tgggcttgaa 37560
   atggactcaa gcagtccacc cacctcagcc tcccaaagtg ctgagattac aggcgcgagc 37620
   caccgcaccc agcccatttt acctattctg cagttgacag ttcagtggca ttcagtcagt 37680
   tcacgaggta accatcactg ccattcatct ccagactact tcaccttctc ggcagatgtc 37740
   cgaaactgtc cgcattgaac acactcctca tctccctctg acagccacca ttctactttg 37800
   tatctctctc tgccttctct aggtacctca tgtaagtgga attataccaa tatttgccct 37860
   tgtgtgactg gcttctttca tgtgacatgg tgtcctcaag gttcatctgt gttatagcct 37920
   gtgtcagaat ttccttcctt aaagcctgaa taataacccg ttgtaaaggc tgggcgcggt 37980
   ggctcacacc ctctaatccc agcattttgg gagtccgagg tgggcagatc acttgaggtc 38040
   aggagtttga gaccagcctg gccaacatag tgaaaccctg gctctactaa aagtacaaaa 38100
   ttagctgggt gtggtggcgc gcacctgtaa tcccagttac tcaggaggct gaggcaggag 38160
   aatcgcttgt acccgggagg cagaggttgc agtgaaccaa gattgtgcct ctgcagtcca 38220
   gcctgggtaa cagagtgaga cttcctgtct caaaaaaaaa aaaaatcatc ggatggatgg 38280
   acggaccact tcttgttatt tatccatcca cgggtgctag gtttcttcca cctttggttg 38340
   tcgtgaataa ggccactatg aacatttcct tccgtggtga aggttttgta ctagtgagga 38400
   aaaggcgtgt ttgtggtgtt gcataggatt ctggtaagaa agtttgcact aaccataagt 38460
   atttgtacta cattaaaatg aaagctcagg ggccgggcgc ggtggctcac gcctgtaatc 38520
   ccagcacttt gggaggccag ggcgggcgga tcatgaggtc aggagatcaa gaccatcctg 38580
   gccaacatgg tgaaaccccg tctctactaa aaataccaaa aaactagcca ggtgtggtgg 38640
   cgggcacctg tagtcccagc tacttgggag gctgaggcag gagaatggcg tgaacccggg 38700
   aggcggagct tgcggtgagc cgagatcgct tcactgcact cgagcctggg caacagagca 38760
   agactccgtc tcacgcaaaa ctctgtctca cgcaagactc cgtctcaaaa aaaaaaagag 38820
   ttcagggttt atgaaactgg ccagccgcgt aaagtttgct gtgttgtttt tgtgcccggg 38880
   aggagtgtgg ccagggtgtc acgtcacaca gtacacgttt ctcagatggt ggttctccag 38940
   actgctgtcc caaagtctgt tttgcatctg ggttcccaca gacccaccct ccacggtgag 39000
   cctgattttg gccagggtag ctggaatctt gcttgtcttt cagcccggca gctgtaccag 39060
   tccagggtcc acagctagtg gcttttagga aggaatttgt tcagttggct ttgacacatg 39120
   gccccctagg gtccacagct ctgtagtgat gtggatgttg ttatctacaa agacacatga 39180
   tccttcgtgt ccagatgaaa gtgatgatgt ctttgcagct gcccagcaag gctgtgtgtg 39240
   tgtgtgtgtg tgtgtgtgtg tgtgtgtgtg tggtgtgtgt gtggtgtgtg tgtgtgtatg 39300
   ggggagggag gcaccctttc catctggggg tgtgtgtgtg tggggtgtgt gtgtgtgtgt 39360
   gcgcgtgtgt gtggtgtgtg gtgtgtgtgt gtgtatgggg gaggcaccct ttccatctgg 39420
   gtccaagaga ctgggcctgg ggaagacgct tctttttatc tacttagaga ctttgtttta 39480
   tttgtatttt tttgagacag ggtctcactc tgtcacccag gctggggtat ggtgatatga 39540
   gcatagctca ctgcagcctc ggcctcccag gctgaagcga tcctcccacc tcagccttct 39600
   gaatagctgg gactgtaggc gtgcgtcacc atactgagct attgtttttt ttgtttggtt 39660
   ggtttaattt tttttgatac agatggagtc ttgctatgtt gcccagacta gtctcaaact 39720
   cctgaactca agtgattctc ccacctcagt ttcccgacat tctgggatca caggtgtgag 39780
   ccactgctgt ctccctgttt tattaactgc tgaaagacct agataaagaa agtctgaaaa 39840
   gacttactat cagagcacca tcctaagatg attccctctg actcaatgga gagggagggg 39900
   agcttttcct tcaggcctgg gtggcaggag cccaggtgct ccaggcccca tttgccccag 39960
   gccaaatcac tcgggaactt ggatgcagct gtctttcagg gtaacccaaa ggaaccagat 40020
   ccccgcaggc agtaggcttc tgggctgtcc tctcctccta cgtcagctca gtaagagccc 40080
   ttcgaaggga tgctgtgtcg gaggccccaa aagcccaggc tcatccctga gatgcacagg 40140
   gtgggctggg cttaggcagc gctcgagcat ctcctggacg gtgaccccag agagtgtgga 40200
   gacggagagt ccttgagagt cactgagaga cgtggctgcc ctgccttccc aagaggggct 40260
   ctgagtcatt ccccacactc acctgcccct acccaccctc acctggcccc cagcctcacc 40320
   tacccccaca tctgtaccga tccctttacc cgcaccttcc ctacccaccc tcacctcccc 40380
   tgtaccttca cctcccccac tcacccgccc ctgcaccctc acctgtcccc caccttcacc 40440
   taacccccac cctcacctgc cctcccctca cctggcctcc ttccgttggg gaaggggttg 40500
   taaggggcgg cccccaaact gtctgtcctg gtgccctgca gagaaaacag tacgtgaggg 40560
   ccgcagtcca aaagcttgag tcctggaagg tggaggagac agggatgtgt tgggaagggc 40620
   cccatggtct tggatccctt ctcgactgtc aatggggcct tcatgggagc gccagtctag 40680
   tgatgcacag ctgggtgccc ggcgggtggc tgaggaggcc taaagtccga ggcggcaaga 40740
   gctcttccag aggctgttgt cctaatcgct ctggcatact caggcgggca cgtagttagg 40800
   agctgattgg agaggagaga cccccacacc aatactggga tttgactttc aggctaaact 40860
   tgagaagtgt ggcctctgct gtcctgccag agctctccag ccagtgccca gggctctcca 40920
   gccagtgccc gggggtctcc accagtgccc gggggtctcc gccagtgcca ggggtctccg 40980
   ccagtgccca ggggtctccg ccagtgctca ggagtcttgg tttctttgtc ttacagccct 41040
   ttgttttgac ctctctgagc caaggccaaa acccagacag gcagccccac gacctcagca 41100
   tcgacatcta cagccggaca ctgttctgga cgtgcgaggc caccaatacc atcaacgtcc 41160
   acaggctgag cggggaagcc atgggggtgg tgctgcgtgg ggaccgcgac aagcccaggg 41220
   ccatcgtcgt caacgcggag cgagggtagg aggccaacgg gtgggtgggg gtgctgcccg 41280
   tccaggcgtg cccgccgtgt cttatgccga atgccagcct ctcacaggct ggggagactt 41340
   tccacctggg gatccaatgg gtggctttcc agggtcccaa aagcaaacac aggtttttca 41400
   cagcccgtcc gggaaagcag aaagccccaa ggggctggaa ggggaaaggg ggagctctgc 41460
   tgagaggtta caaggcagcg ctggccgacg ggagttgcag ttgataggtt ttgtatcatc 41520
   cttgttaaac ttgaaccctg tgcagaaatc ccttccacgg catgggggct gcctgttgac 41580
   tcgctcctgt tccaccacag ggagctcctg ggcttcttcc tcccagaggc ccccgacgct 41640
   cccacctgtt ggtcgtcaga gcttctggtt ggtgggaagg cacccaggac cttgaggtct 41700
   ccagagagaa aagccaggga aagagggaga ccgaaaccca tgtgacatga aactcaggct 41760
   ccaaactgag cacgggaacg tttggggaca ggagcgcgat ggccttcctc agatagctgg 41820
   ggggctggca tgaagacggg agctacagcc agcacaggtc ctgggccggg agcccagaga 41880
   ttgagccctg actctgtcac ttactggcca cgtgaccttg ggcgggtggc atagcctctt 41940
   ggagactcag tttcctcatt ggtaggagtg acggccacag tggtgcggcc tctgcagcac 42000
   acggggggct cggtgggcgg aagccccggg tctataaggc ggctgtgcag gagccagccg 42060
   agctggtctc ccaacagcca gggctccggg gtccttagca gctgtggggg gcctgcacct 42120
   gtttcccatg gctgctgtca gaaattacca gaagccaggt ggctgagagt aatggacact 42180
   tgttctctca cagttcctga gggctgaagc ccgagatcga ggtgtgggca gggccctgcg 42240
   ccctctgaag gctctgaggg aacctttggg cttctggtgg ctccaggcac cccttgactt 42300
   gtggtcctgt cactccagtc tctctgtctg gctgcacatg gcgtggcctc ttctgtacca 42360
   ttgaaggaca cttcagttgg atttagggcc taccctcacc cattgtggtc gtatcttgat 42420
   ccttcatgac atttgtaaag accctgcttc caaataagct cacattctga ggttctgggg 42480
   tgagcgggaa tttggagagc attgttcaac tagtatagaa tgtgacctgt cagcctcggg 42540
   cagccctgag aggcaggggc tttccacagc ccagctgggt gccctgggct ccgtgctgtc 42600
   cgaggagacg ccatccccac acccgtcctt cacccgccac cctcccgcag gtacctgtac 42660
   ttcaccaaca tgcaggaccg ggcagccaag atcgaacgcg cagccctgga cggcaccgag 42720
   cgcgaggtcc tcttcaccac cggcctcatc cgccctgtgg ccctggtggt agacaacaca 42780
   ctgggcaagc tgttctgggt ggacgcggac ctgaagcgca ttgagagctg tgacctgtca 42840
   ggtacgcgcc ccggggcctg ccctaaccgc agacacccgg ccttcattgt cagtaatggc 42900
   agcagctgcc acattgtccg agacctgccg tgagcccagt gccgcgccag gggctttgtg 42960
   tgtagcgtgt tttgtcctca cactgacagc tgtaggctgg ggttctgagt gagccccaca 43020
   gggcagaggc agaaaatgag tctcagagag ggtgagcgag ctgcttgggg ccccacagca 43080
   ggagatggag caggactgca gcctagcctc tgcccccagc acctgcgcaa gaagctgctc 43140
   tgctctggac tgtgttaggc tgcgagggct ggagagaaat gagagttggt gcttagagag 43200
   ggggcgcagg tccccatggc ttttcctctt atgatgaggt agatgggtga agggaggggc 43260
   catgcttgca ggggccagtg accgaggccc gccgttggaa ctgatggcct tcatcccgag 43320
   cccagcccag gtgggagcag ggctttccga gggcttgtct tgggtcggcc tgcttccagg 43380
   gactctgctg cagctcccac ccctgtccaa agcatggaat cccccaggct ccctggcagt 43440
   cctgtcaacc tctgtcctcc caagctgagt gtggggcaag ttctggaggt cagcactgct 43500
   caggggggcc cacgggctgc ttgcaggggc caaccgcctg accctggagg acgccaacat 43560
   cgtgcagcct ctgggcctga ccatccttgg caagcatctc tactggatcg accgccagca 43620
   gcagatgatc gagcgtgtgg agaagaccac cggggacaag cggactcgca tccagggccg 43680
   tgtcgcccac ctcactggca tccatgcagt ggaggaagtc agcctggagg agttctgtac 43740
   gtgggggctg gcagtggggt gggcagggtg gcctctaaac ccgacccctg gaggaggctg 43800
   gaggccagtg caagatcctg tgtggcctca gccaggcggt ggtctctgcc agatgccaac 43860
   tgttgcccgc tggggttcag cgacatgtcc gaatgtcccg aggcctctga ggttgttttc 43920
   ttttgccgca gaacaaatca ccacgaacag cgttttaaga caacaccaac tctttttttt 43980
   tttttttttt tgagtcagga tcttgctctg ttgcccaggc tggggtgccc tggtgcaaac 44040
   acagttcact gcagcctcga cctctgggct taattaagtg aacaccttgc ctcagcctcc 44100
   caggtagctg ggactacagg tgggcaccac cacacctggc taattttttt ttgtagagac 44160
   ggggtttccc catgttgccc aggctggtct gcaactcctg ggcacaagct atctgcctgc 44220
   tgtggcctcc caaagtgcta ggattatagg tgtgagccac tggcctgaca acacccacgg 44280
   attgtctctc agttctgtaa ggcaaagtcc aggcacagcg tggctcacct gggttctctg 44340
   ctcagggtct cacggggcca gaatcaaggt gtcaggaacg ctgggccctc agcggaggct 44400
   ctgtggagaa attagcttcc ttgctcactc agcaggtagc agttgtggga tcgaggttct 44460
   gttttctctc tggttattgg tcggggacca ctctcagctc ctagaggcca ccacaggtcc 44520
   ttgccccgtg gccctctctg cctcagcagt gggggctccc tgcgtcagtc cctcccacac 44580
   cttgagtctc tctgatttgc ttctaaaggg ccctgtgatt cggctcagcc acctttagat 44640
   taggttagcc tcccctttga tagactccaa gtcggctgat taataacctt aatcacatct 44700
   gcagaatccc ttctgccaca taaggtcatg acgccgtgct ggggactggg gtgggaaatt 44760
   acggggtcat ttaggattct gcctgccact gccttgctgt gtcccagggc ttgggggagg 44820
   ggcctccaca gctgggacca cagtccttcc tcccctccat ggtaaccatc tgaggattac 44880
   ttgagaccag cctgggcaac atggtgagaa cccatcccta caaaaaatac aaacaaaaag 44940
   ggaccaggct gggcttggtg gctcatgcct ataatcccag cactttggga gaccaaggtg 45000
   ggctgatcac ttgaggttgg gagttcgaga ccagcctgcc caacatagtg aaatcccgtc 45060
   tctactaaaa atacaaaaat tagctgggtg tggtggcagg cgcctgtatt cccagctact 45120
   ggggaggctg aggtgggaga attacttgaa cctgggaggc ggaagttgca gtgagccaaa 45180
   attacgccac tgcactccag cctaggcaat agagtgagac tccgtctcaa aaaaaaaaaa 45240
   gggccagggg tggtagtgac aaagagaccc tatcccaaaa aaaccgaaca ctgaatcctt 45300
   gagactgagt aaggacactg tgaaattttt ctgggtgggg cagggaacag agcgtcttct 45360
   gtcatttctt ccacctgggt gtggtcagct ctccctccaa gctgcctcct cttcttctca 45420
   ttgtccgggt gttggacaca tttggttaac tggatagaat aacgcgagtt cccagggact 45480
   tggtccattt gctattttat tttattttat tttattttat tttatttatt tatttattta 45540
   tttatttatt tattgagatg gagtttcgtt tttgtcgccc aggctggagt gcagtggcgc 45600
   gatctcggtt cactgcaacc tctgcctccc aggttcaagt gattctccta cctcagcctt 45660
   ccaagtaact gggattacag gcacccacca ccataccagg ctaatttttt tgtattttta 45720
   gtagagacgg gttttcgcca ttttgcccag gctggtcttc aactcctagc ctcaggtgat 45780
   ccacgcacct cggcctccca aagtgctggg attacaggca tgagccacca cgcctggcac 45840
   catttgctat tttaattccc atgtgtatta gtgtcccacg gctgctgtaa caaatgacca 45900
   caaactggat ggcttaaagc aacagaaatg gattccccca atgtgctgga gaccagaagc 45960
   ctgcgaccaa actgttggga gggctgtgct tcctctgggg gctccaggga ggatctattt 46020
   gttggccctt ccagtgctgt gggtgccagc gttccacact tgtggatgcg ccgcctcaac 46080
   ctctgcccat cttcatgtgt ccatctcctt tgtgtctgcg tctttacctc ttcttcttgt 46140
   ctgtgttgcc tcttataagg acgtttgtca ttgggtttag ggcccaccca aatcatccga 46200
   gatgacctcg tcttgagatc cttaacctgc aaagaccctt tttccaaaaa aaggttatgc 46260
   tcacagattc taggccttaa gacatgggtg tatctttctg gggggcacta tccaacccct 46320
   tatacaatga aagacgggaa gagggccagg tgtggtagtt cacgcctgta atctcagcac 46380
   tttaggaagc tgaagcggga ggatcacttg agcccaggag tttacaagta gctaggcaac 46440
   atgatgagac cccatttcta caaaaagtga aaaaaaaaaa aaaaaaaaaa aagccaggtg 46500
   tggtggctca cacctgtaat cccagcactt tgggaggctg aggcaggcag atcacgaggt 46560
   caggagattg agaccatcct ggctaacacg gtgaaacccc gtctctacta aaaatacaaa 46620
   aaattatggc cgggcgcagt ggctcccgcc tgtaatccca gcactttggg aggccgaggt 46680
   gggtgaatta caaggtcaag agatcgagac catcttggct aacacggtga aaccccatca 46740
   agatcacaag gtcaagagat ggagaccatc ctggctaaca cggtgaaacc ccgtctctac 46800
   taaaaataca aaaaattagc cgggcatggt agcgggcgcc tgtagtccca gctgctcggg 46860
   aggctgaggc aggagaatgg cgtgaacccg ggaggcggag cttgcggtga gccgagatcg 46920
   ctccatgcca ctgcactcca gcctgggtga cagagtgaga ctccgtctca aaaaaaaaaa 46980
   aaaaaaaaaa aaaaaaagaa aattagccag gcacagtggc aggtgcctat tgtcccagct 47040
   acttgggagg ctaaggcagg agaatggcat gaacccggga ggtggagttt gcagtgagcc 47100
   gagatcatgc cactgcgctc cagcctgggc gatagagcaa gactctgtct caaaaaaaaa 47160
   agccaggcat ggtggtgcat gcctgtagtc ccagctactc aagaggctga ggcaggaggg 47220
   ttgttcgacc cacggagatc aaggctacag tgagccatga tcgcaccact gccctccagc 47280
   ctgggtgaca gagtgtgacc ctgtctcaaa gtaagtaaat aggaggagag acaagtgggc 47340
   agttcagact gatggtatgg gcacagtaga gactggtgca gacaggctgg cctgtgatgt 47400
   caagcaactt ctgtaattgt ttccggcatc catttgtgtg tcaatttccg tgtcagtagg 47460
   aagactctgt aggctgccaa gaggaataag tgggaggatc ctcccagaga ggccgggcct 47520
   gcaggagggc cagttctcat gagttctcat ttggccccta ccctccaggc tgtggttctg 47580
   aggtgggaga cagagcctga cctctgtttg tcttgttttg tctttgcagc agcccaccca 47640
   tgtgcccgtg acaatggtgg ctgctcccac atctgtattg ccaagggtga tgggacacca 47700
   cggtgctcat gcccagtcca cctcgtgctc ctgcagaacc tgctgacctg tggaggtagg 47760
   tgtgacctag gtgctccttt ggggtgatgg acaggtacct gattctctgc ctgctaggct 47820
   gctgcctggc atccttttaa aatcacagtc cctgtggcat ccagtttcca aagctgattg 47880
   tgtcttcctt tgccctcctt tcttttctac tatgtgcatt cggtgctatg aattttcctc 47940
   taagtactgc gtttcctgca tctcacaaat tttgttacat tttcattttc aggtagtttg 48000
   aatattttta cacttctcct gagatgacat ctttggctca tgtgttattt agaagtgttg 48060
   cttagtttct aaagagttgg ggcttttcca gctgtctctc tgcaactgat ttctaattta 48120
   attctactgt agtctgagag cttattttat atgatttctg ttattttaaa tgtgttgggt 48180
   gtggtgtttt tgttgttatt gtttttgtgt ctttttgttt tgttttgctt cgtttgtttt 48240
   gtttttgaga cagtgtcttg ctctgtcact caggctggag tgcaatggcg cgatctcagc 48300
   tcaccgcaac ctctgcctcc cgggttcaag tgatcctctt gcctcagcct cctgagtagc 48360
   tgggattaca ggtgcacgcc accataccca gctaattttt gtatttttag tagagacggg 48420
   gtttcaccat gttggtcagg ctggtctcga actcctgacc tcgtgatccg cccacctcgg 48480
   cctcccaaag tgctgggatt ataggcgtga gccactgtgc ctggccatta ggtgtgtttt 48540
   atcacccagc atcatgcagt ttatcttggt gaatgttctg tgtactcttg aaaagaatgt 48600
   ggattctgct gttgttgggt ggagtgttcc agaaacatca attagatcca gttggttaat 48660
   agtgctcatc aggttgtctc tatccttcct tcctgactgc ctgcttgagc tgtcagttat 48720
   tgacaggggt gtggagtctc caactctaat ggtggatttg tttatttctc ctagtagttc 48780
   tatctttttc tctccttcta cccttgatcc tcttctcccc ctagggcttc ctggtgttag 48840
   tggtgggaga gtggggtagt gaagaacctg gactttaggg ccaaagaggc cagggttcaa 48900
   atcctggctc tgtcacttcc cagttgagtg accctggctg gtgcctgaat ctctgtgagc 48960
   ctccacttcc tcctctgtga aattgagagc acttacctgg caggctgtca tgggcatcaa 49020
   gtaacagggc actccacctg gaccctgaca cgtgatgcac aggaatgcca gctgctatgc 49080
   catgggtgtg gcagtagtaa taaagtgacc atctgtatcc tcaccacagt gaagcctgtc 49140
   cagggctttc tctcctatgc ccccatgcct ccaggtggcc ttggatcctg ttggttctgt 49200
   gctctgctca gcgacctttc tcccgtggga gttcctgggg gttcagcttc atcctacaga 49260
   cagcagcaca cactggctgt gcaccctttt tttttttttt tttttttttt tgagatggag 49320
   tctcgctttt ttcgcgcagg ctgaagtgca gtggtgtgat cttggctcac tgcaacctct 49380
   acctcctggg ttcaagtgat tttcctgcct caccctccca agtagctggg attacaggct 49440
   cccaccacca cgcccggcta atttttgtat tttcagtaga gatggtgttt caccatgttg 49500
   gccaggatgg tcttgaactc ctgacctcag gtgatccgcc cacctcagcc tcccaaagtg 49560
   cagggattac aggcgtgagc caccacaccc ggagtgccgg ttgtttttag cagtttgtct 49620
   tgttcctgga gagactggct cctgcccagg agctcgggga gtagggccgc ggggtgctgc 49680
   ctcacacctc gagtttggcc gtaagcagag gggacatttt gtgactgtcc ccctcctgag 49740
   cttcccagca gcttttctcc aagttacagc ccaaaagctc aggtggattt gcaacccaac 49800
   ggtgtctgtg cacctcccac tgatgcccga actgccctgg ccaagaaacg gggccgtcag 49860
   aacgctgcac taactgcagc cttgggcctc catgccagag gccatgccct tccatccacc 49920
   accccctggc ctgggccctg ggccctcctg gctcgggaac tccaggcccc ttcctcacgg 49980
   ctcgagagac gtgtatttac cgcacaggtg cttgtcattc tcttgtggcc tcttctccag 50040
   ggagatcaca gaaggacagg gcctcactga ggtctcggac atggaccctt tgatagtggc 50100
   aggagccagg ctgggcaaga ggcggccaca gtcacctcag cagtgccatc accaccgcca 50160
   ttcagccctt ccctgagccg ggcgcgcccc tggctctggc cccagtgtcc cagttacagc 50220
   tcacaggagc ttgtggtgcc cagcggctgc ttctgattga gagtcgaggt cggaggcttt 50280
   gggaggctga gaggctgctc ggtttcacaa ctgctgaggg agacttgggc tccatctcag 50340
   gtatgcccca tgtcgccctc aacctccagc caccggtcct ccgtgtcccc catggccagg 50400
   cacggcttgc agacatctgt cgttggctcc tctcagccgt cgtgggctga ccctggcacg 50460
   tcctcctgtg gctgagccca gtggggacag ctgcttcctt ttattaccct agaactctcg 50520
   tctttgatca ggccccctcc cctatgccac acagtccctg tcactcgggt gagcccagta 50580
   gtcatgggga aggcctgcgg gttccaaaca tccaaaggct tgcgtgcagc atgacagctt 50640
   gaaaccgatg ttttttacct tgatcagatt tcagcttggc gggggctttg ctcagctttc 50700
   agtgaggcct gggccgattt cccagcatcc cctcctgagg ccagcctctg tttcctgtga 50760
   ttttctgcac aaagtgggag ggaggagtcc taggaaatgg ggggccacct cgaagcctag 50820
   gcctcctctg gcttctctgt gccagtgccc ccacgctttg tgtctgtgtc cccagcccat 50880
   gggactctgc tattccctga gtgctgccgc atgcccagcc cgcactgagg acgtggagcc 50940
   ccgaggggca ggatggcctc catggtcaca cgtaggaagt ggcctccacc ctccgatgat 51000
   cctctccctc ctccctttca gcgccctccc cgggggtgtc ctcagccctc ctgcctgtgc 51060
   tttgtcccgt cttctgcagg cgcctgggac gtgctgacag gtcctctgcc ggctcctgcc 51120
   ttgctatgcg cacgctggtc accacagagg cctggccctt cttctgtagc agtcccacac 51180
   ccgcaacagg tgtggctgct gaccacctgc tttctgcccc tctggtcctg aggagggcgc 51240
   agtgggcact caggcgtggc tgagcagatg tgtgttgccg ggaggaggaa ggactgctcc 51300
   agtcagggct gaatttccca cccggagcat ttctgctgta tttggtgtag cgcctgctgc 51360
   ttaaagctct gattcccagt tggcaccctt tcccttctgc attgaaaaac atacggatgc 51420
   atgtcttctt gcagtgaatg tgtattctcc cagcctctct tctgggttgg ggctggaggt 51480
   ggagcggcac acaggagccg cagcgatgga ggatgtgcgg gtgcagcacc ccgtacagca 51540
   gggatgccaa acccgcgctg agtccctctc aacttctgct ttgaagccca gtcacgccat 51600
   tgcctgggtt ttgctgggcg gggctgcgtg tgatgttctc ctctgtccct cccccagagc 51660
   cgcccacctg ctccccggac cagtttgcat gtgccacagg ggagatcgac tgtatccccg 51720
   gggcctggcg ctgtgacggc tttcccgagt gcgatgacca gagcgacgag gagggctgcc 51780
   ccgtgtgctc cgccgcccag ttcccctgcg cgcggggtca gtgtgtggac ctgcgcctgc 51840
   gctgcgacgg cgaggcagac tgtcaggacc gctcagacga ggcggactgt gacggtgagg 51900
   ccctccccgt caaggctctg ccaagaccct ggccctgccc tccgggatac gagcttgggg 51960
   ctgcctccgg cctcacagga gtaggggctc tgaaaacctt tgcttgcagg gagattgcca 52020
   agtctgtctt ttaggcccaa caaggaaaac tctgcagttc cacccatcct gtcccaccag 52080
   gtagtgtggc ttgaaggcag actgtgaggg tctatctcac cttcctgcat taggtcagga 52140
   gtttcacaga aacctgaggc acattcaggg gtgggctgca gaggtccatg gctcacaccc 52200
   tggaaaatcc gcccccaaaa gacagtgctg tctccactga ccagtctgtg ggatagtgct 52260
   taagcctgag tggtttctat caacatgtag aatcaggagg tataaagaga tttgctcagg 52320
   catcctgggc cctctctgac cagcaggatc ttcctttaga tcttgacagt gaaacacatc 52380
   tcttctgtgc cccctgtgag ttttctttca ttcattcatt cattcattca ttcattcatt 52440
   cattcattcg agacagagtc ttgctctgtc acccaggctg gagtgccctg gtgtaatctc 52500
   ggctcactgc aacctctgcc tccagggttc aatcgattct cctgcctcag cctcccgagt 52560
   agctgggatg acaggtgcgc accaccatgc ctggctaatt tttgtatttt tagtagagac 52620
   agggtttcac catgttggcc aggctggtct cgaactcctg acctcaggtg atccgcccgc 52680
   ctcagcctcc caaagtgctg ggattacagg catgagccac cgcgcccggc ctgagttttc 52740
   cttttatgaa ggacctgctt ggttggttgc ctgccacatg ttgtcagcac catgggccca 52800
   ggactgctga ggagctgttg atgccctcgc tctcccagag ccaccggctc tgttagataa 52860
   ttcacatgca gtctggccac tgtcctacgt cctcattcac aaagagcaga catttcgtag 52920
   aagatgaggg cctgggagta acctccctgc atgtttttct ataaaggcat agtggttaag 52980
   tccttccagc tcattgacca ttggagaatt ttatggaggc tgtagactag gggctggtaa 53040
   actaagggcc caggggccaa atccagcctg ccacctactt ttgtaaataa agttttcttg 53100
   gtgcacagcc atgcccattc attcatttgc acaatgtctg tggctgcttt catgccaaaa 53160
   gcaagagaac tgagtggtta tgctggagac ctacggcctt caaagcccca gacctcacgt 53220
   ctggcccttg acagacagag cttccccagc cctgctgcgc atcctggccc agcatgtgct 53280
   gtgtgtgtga tttcagcttg caggagccgt ggttaggaat tgtccctgtg ttggtccatt 53340
   ttgcattgct atgaaggagc acctgaggcc gggtagatta tgaaggaaag aggtctgtct 53400
   ggctcatggt tctgtaggca gcaccagtat ggcacccgca tctgctcagc ttctagtgag 53460
   gtctcaggaa gctttgactc atggtgaaag tcgaagcggg agcaggtgca tcacatggtg 53520
   agagagggag caacggagag agagagagag cgcctctccc tcttgccctc accttgagag 53580
   gagatgccag gctcctttaa gtaaccagct cccatgtgaa ctcacagtga gagcccattt 53640
   gctactgcgg agagggcacc aggcatctgc tcccatgacc caaacactgc ccaccaggcc 53700
   ctacctccaa ccttggggtc atattttatt ctgttctatg ctatgctatg ctatgccatg 53760
   ccatgccatg ccatgctatt cctattctat tatttgagac agaatctcgc tctgttgccc 53820
   aggctggagt gcagtggcat gatcttggct cactgcaacc tccacctccc aggttcaagc 53880
   gattctcctg tatcagcctc ccgagtagct gggattacag gcacacacca ccacacccgg 53940
   ctaatttttg tattttcaat agagatgggg tttcaccatg ttggccaggc tggtctcaaa 54000
   ctcctggcct caagtgatcc acctacctcg gcctcccaaa gtgccatgat tacagatgtg 54060
   agtcactgcg cccagtgagg gtcacatttc cgttgagatt tggaggggca gacgttggag 54120
   ccatctgagc cccctcgtcc cgctctagct tctcctcccg tgtgccccgc ggtgctggtg 54180
   gcaggccctt acgccggttc tggctgcatg ctctgttcca gaagctttct tccctgcttg 54240
   gttaccagaa aatcatccca tccattacaa ggacagggtc cccttatctc ccattcccag 54300
   ggcaggacac cgggggcagg gcaggtgggg aactgagcaa gttctctggg ggcaggcgtg 54360
   gctatggctc cctctgggtg ggcgtctggg gaggggtgga ggcagccgtc agcgccctgg 54420
   cttgctcttc ctccctggcc agagactgtg gccttgtgct gctcccgtgt gggctgcctg 54480
   cacctccagt gggttgtgct ccctcccctc ccctcccctc aagctctgct gagcaccact 54540
   gccttccaca gcccccactc tcgggaggcg aggctcctcg tggccattcc tgtccttggc 54600
   acccaccccc ccaccaacct ggtagagcct tgggcggggt ctgttactcc ttgcatggcg 54660
   tagacctccc cacagtaggc acctgacaca tacctcctgg ggggcaggca ggaggtgcgt 54720
   tgaggtctca gccctggcag tccctcccct gcgtggcata ggcctcgcca cagggtcatc 54780
   gagggtgggt ggagactgta ctagaccact ccccgctggt cctagaaagg gtcccatctg 54840
   tctgctctct gtttggagtc cagaccttgg ttgctgtgcc ctgcatggtg ggctgggggg 54900
   caccctccag cctctctgag tgcatggcct ctccttgcag ccatctgcct gcccaaccag 54960
   ttccggtgtg cgagcggcca gtgtgtcctc atcaaacagc agtgcgactc cttccccgac 55020
   tgtatcgacg gctccgacga gctcatgtgt ggtgagccag cttctggcac ggggaagggg 55080
   cgtccgggct gggttccccc aggaacgtgg agtttagggg aggagacgtg cctttccagc 55140
   ggggctgggg gctgtgtggg agactcaggc ggctgggagg ctccttgcgg gaggcaggga 55200
   agcctttccc agggcagcgg ccaggaggac agactgtgag ctgtgggctc ggcggctaca 55260
   gagtctgcct cagtgggcgg ggctgatggt gtccaggtgc ctgcagcacg cacccaccca 55320
   cgggaccttg ctgagcagcg tctgtcaggc agcaagatta cccgagggct gcagtggtcc 55380
   tgttccctgg cagcttactg tctggctgag gaggagtgat gttcacatat gcacacatgt 55440
   catgtgcaca cacatgtaca tgacaacatc ccacatgctc ctcaaatagc atgacctgta 55500
   cagtcacgga tatagggcct aggggatagg aggccaagac agtcagggaa gactttccag 55560
   aggcagtggc tcctgaaagg ctgtctgatt caggcaggaa gggagctgag ttcagatagg 55620
   aagtagcaat gagtcattgt gtctggggac atggccactc cttcgctgca gagggacctg 55680
   ggctgagagc tcctctctta tggctgcagt cgggagagaa gtctgttggg gggagaaggg 55740
   ggcttcctca agggactccc tgtgcccttt ggcaccttcg tgccaggtca ggcttgaggc 55800
   ctgaaggcag tggtgggggc caccaagggt cgcctcctct gctgggcaag ttcccagtct 55860
   gacgggcctg tgccgtgggc cccagctgtg ggggcgctgt tgatgcgcag ccaggcctcg 55920
   ccgccagagc ccgcacgctt ccattccgct gacttcatcg acgccctcag gatcgctggg 55980
   ccggccctgt gggagagtga atgtggcttt tgccaaagtt gagtctggag cctggaaact 56040
   tccctatggg cagccttgat agtggagtgg cccaaggagc ccacccagcc gaccctgccc 56100
   ctcccgtggc tggtgggcgg caccaggggc tgcctggctt tgctcgttca ccaacatcac 56160
   ctgggctggc cagggcgcgc tcacttctgc caccaccgag ggccctgggc gaaggagtga 56220
   ataccaggct gccttggcag ggatgtgttg agggctgtgg ggagtcggac agcggcgggg 56280
   gtcagaggag gaggagggtg caccgtgcag gctgaagggc cacgttaccc tgaggttggc 56340
   caggctcccc aggcctagcc tcccagctcc cccactttct ccccaccctc caccagtggc 56400
   aaagccagcc ccttcagggc gcacggtgtc tgcccccaag gagggcccat tccgttgggg 56460
   ttaatgttgg ccacctcttt ctgtttgtct ctggcagaaa tcaccaagcc gccctcagac 56520
   gacagcccgg cccacagcag tgccatcggg cccgtcattg gcatcatcct ctctctcttc 56580
   gtcatgggtg gtgtctattt tgtgtgccag cgcgtggtgt gccagcgcta tgcgggggcc 56640
   aacgggccct tcccgcacga gtatgtcagc gggaccccgc acgtgcccct caatttcata 56700
   gccccgggcg gttcccagca tggccccttc acaggtaagg agcctgagat atggaatgat 56760
   ctggaggagg caggagagta gtctgggcag ctttggggag tggagcaggg atgtgctacc 56820
   ccaggccctc ttgcacatgt ggcagacatt gctaatcgat cacagcattc agcctttccc 56880
   actgagcctg tgcttggcat cagaatcctt caacacagag gcctgcatgg ctgtagcaac 56940
   ccaccctttg gcactgtagg tgtggagaaa gctccttgga cttgaccttc atattctagt 57000
   aggacatgtg ctgtgttgtc cacaaatcct catgtaccct agaaatgaat gtgggggcgg 57060
   ctgggctctc tccagagctg aaggaatcac tctgtaccat acagcagctt tgtcttgagt 57120
   gcagctggga tttgtggctg agcagttaca attcctacgt ggcccaggca ccaggaacgc 57180
   aggctgtgtt tgtagatggc tgggcagccg caccgcagag ctgcaccatg ctggtttgta 57240
   tcacatgggt gaccatggta tgtctaagaa ggtggagtcc ctgtgaggtc tgcaggtgcc 57300
   cccacagctc caggccacct tgaggattgc ctctgcctgc ccagccctga gttccctctc 57360
   ccctgtcctg tcccactgtc accccaagcc ggcctcattg ggagcctgtt ggatggcagg 57420
   gtatagatgt aacctgattc tctctgggga gcggggttat ctggcttctc aagagctcct 57480
   aggagcccac agtggtggca ccatcacagt cgcagcagcc cccagagaac gcggccctgt 57540
   ctgttcctgg cgtgctctgt gctgccccgc ctgggttccc tgccccagtc gcaggcccct 57600
   tggaggaggt accatgtgtc tcccgtttca cagatgagcc ccggggagct cactctagta 57660
   gtggccagag aggcctgcgg ctcagggagc ggggcacatt tccaacagga cacaccgccc 57720
   tggtctgagt ctcgtgggta gtgggagcag aggagagcgc cctatgtctg tggggcggct 57780
   tggctgagcc tggaagccac ctgacctccc ccgtcccttc cctgccaggc atcgcatgcg 57840
   gaaagtccat gatgagctcc gtgagcctga tggggggccg gggcggggtg cccctctacg 57900
   accggaacca cgtcacaggg gcctcgtcca gcagctcgtc cagcacgaag gccacgctgt 57960
   acccgccggt gaggggcggg gccggggagg ggcggggcgg gatggggctg tgggcccctc 58020
   ccaccgtcag tgctggccac cggaggcttc ccgggttcct gggggctgtg ccaccgcctc 58080
   tgaggcatgc ttgctttctt cccttttcaa acccttctgc ttccttcttt aatgacattg 58140
   ttgattgtgg ataatctgaa aactacacaa aaatataaag agccaaaatc tcacccaaat 58200
   ccacctccta gagtggctgt tgggctccgt cagcatccag gcggccgtct gtgttccgca 58260
   cggcccagcc catcgatagc cgcctgcacc aggcctgtct gccctctgtg agcctcccca 58320
   cagggttccc tccacaaaca ccctgttctc ccacccaggg ctggctgctt cctggaaaac 58380
   agctggatgg ttttgtgcat gacagacaaa cacagggtga ttttcgtggc taaaatactc 58440
   cctggagctt ttggcagggt gaggggctgg ctccagctga gccacgcctt gagtgaaatg 58500
   actgtgagga gaataaactg ccgctgccct ccaggatcac tggggctggc tggggagaac 58560
   ccccgtttct gggagcacag tcccaggatg ccaaggcgag cttggtgccg agatgtgaac 58620
   tcctgagtgt aaacagcggg ggctgacttg acatgctttg tatgcttttc atttgttcct 58680
   gcagctgtat gcccctaagg tgagtccagc ccccttctgc ttcctctggg gcctcgccag 58740
   tgagccccac cttgctgggg ctggttcctc ctgcccttct gggtatccct cacatctggg 58800
   gtcttgtctt cttgttttct ttttcttttt tttttgagac ggagtttcac ttttgttgcc 58860
   caggcttcag tgcaatggtg tgatctctag gctcaccgca acctctgcct cccaggttca 58920
   agcagttccc ctgcctcagc ctccctagta gctgggatta caggcatgtg ccaccacgcc 58980
   cagctaattt tgtattttta gtagagatgg ggtttctcca tgttggtcag gctgatcttg 59040
   aactccctac ctcaggtgat ccgcccacct tggcctccca aagtgctggg attacaggcg 59100
   tgagccaccg cacctggcct ttttcttttc ttttcttttc ttttttctga gacagggtct 59160
   cgctctgtca cccaggctgg agtgcaatgg tgtcatcatg gctaactgca gcctctacct 59220
   tctaggctca agcaatcctc ccatctcagc ccctaagtag ctaggactgc acgcatgcat 59280
   ccccatgccc agctaatatt tacatttttt gtagagatga agtttcacta tattgcccag 59340
   gctggtctcc aactcctgga ctcgagcgat cctcctgcct cggcctcccc aggtgctggg 59400
   attacaggcg tgagccaccg tgcctggcct ggggtattgt cttcttatgg cacctgactg 59460
   tggtgggccc tgggaaggaa gtagcagaag agggttcttc ttggtttcct ggacagtaac 59520
   tgagtgttct ggaggcccca gggcctggct ttgtttaggg acaaagggaa ctggtaacca 59580
   gaagccgaga gtttaaacac ccactgccct tcttccctgc tcctgctgct gcaacccagc 59640
   ttaaccagcc aggagtgcta ggaacccaag cagggccccc gagcacacag caggcagctc 59700
   acgaattctc ttttcctgtt ctcccttggg agctgggagg atcttaatca ggcaataaga 59760
   gatggcactg agcagccagc taatttttta aatcacttta ttgtttaacc atatgactca 59820
   cccacttaaa aaagggtaca gttcagtggg ttttagtgta ttcacagatg tgtgcaaccc 59880
   tcaccacagt taattttaga acattttcct gcccctaaaa gaaactctgc atgaagccag 59940
   ctgtttttaa attagcaaag ttattttgca tcctttaaat atatgttcat ggtacaaaat 60000
   tcaaaagata cagaagagtc tgcagtccaa agagactccg cccccatgac gccaagcagg 60060
   catccctggg aggcatggcc tcctgcagtg tgtttcttct atgtcccccc aggggtcatc 60120
   tgtacatatg caagcataca agagcgtgga ctttgttttc caagccagaa gataattgta 60180
   gatttatgtg cagttgtgag aaagagcaca gacccattta tcctctgcct ggtttccccc 60240
   agtgctgcct gccatcttgc atgacttcca ttcctatcat aagcaagaca ctgataacga 60300
   ttctttcacc ttattcagat tgacataagt gttttttgtt tgttcttgag acaaacttcc 60360
   tctgtcaccc agtgggagtg cagtggcaca atcacagctc actgcagcct caaactcctg 60420
   ggctcaagcg attctcctgc ctcagtcccc tcaagtagct cagatggcag gtgtgcacca 60480
   tcatgccagg ctaattttta aattttttgt ggaggtgagg cctcactaaa tttcctgggc 60540
   tagtcttgaa ctcctgagct aaagtgatcc tcctgcctca gcctcccaaa gtggtaggat 60600
   tacaggcatg agccactgcg cctgggctga catatgtgtt ttcgtaagcc cgaaagatag 60660
   catctgaaga gtcaacattg agccttgcct tttgctgcta acgatgtata aaagctgctg 60720
   ttctgagcat ttcggaggct cccagctgcc gtgtgcaccc tgcctagagc tctaccgtaa 60780
   cccatctccg ggaggaggtg ctattgtttt cctcattttg caacaaggag gctgaagaac 60840
   tgagcatgaa ccactggcct gggtcgttcg gttggtaggc agtggggcca ggccatccaa 60900
   ctcacaacca ccttctactc tgcttccccc gcaccctgaa gtttgttctg ttttgaggac 60960
   acagccgtca cattcttggt ggctgaacag cactccttgt caggcgtggc tgggccccca 61020
   ctggagggca tcatggtcct ctctcctgct gcggttgaac cttggctgtt tcaaccactc 61080
   ctgccaagtg gccctctgaa agggacagtc catcttttct cagcagaggg ccacactggc 61140
   aaaacggtcc ctggcaccct ttctctccac ctgtctaata tagagtaaaa atggtatcat 61200
   gttaagatct tcatttatat ttattttatc atgaatgatg taagcatcat tttgtgtgtt 61260
   taagaacctt tgggcccagc gtgatggctt gcagctgtaa tctcagcact ttaggaggct 61320
   gagatgagcg gatcacttga ggccgggagt ttgagaccag cctggccaac atggagaaac 61380
   cccgtctcta gtaaaaattt aaaaattagc cgggtatggt gatcccagct acttgggagt 61440
   ctgaagcatg agaattgctt gaacatggga ggcggaggtt gcagtgagcc gagatcgcgc 61500
   cattgcactc cagcctgggc gacagagcga gactctgtct caaaaaaaaa aaaaaaaaag 61560
   aaaagaaaag aaattatcaa tctcctcttt tatggcatat atatatatat atatatatat 61620
   atatatatat atatatattt tttttttttg gttatgttca gaaaggcctt ccctgctctg 61680
   atcataaaaa acaacttatt ttcacactct ctctcttttt tttttgagac agagttttgc 61740
   tcctgttgcc caggctggag tgcagtggcg caatctcagc tcactgtaac ctccgcctcc 61800
   cgggttggag tgattctcct gccttacctt cccgagtagc tgggattata ggcatgcacc 61860
   accatgcctg gctaattttg tacttttagt agagacgggg gtttctccat gttggtcagg 61920
   ctggtctcga actcgcgacc tcaggtgatc cacccacctc ggcctcccaa agtgctggga 61980
   ttacagacgt gagccaccat gcccagccca cactctcttt cttaacgtcc tcctcctttc 62040
   gttttacgtt cacatcttta attcttctgg gatgtaatta gatttgatga gcaaggtggg 62100
   catccagctt gtttcttggc tgatggctta tgggtggcgt gaattagtcg gggtctatca 62160
   ggaggcagaa actctatgag aatttgaaca gagaaagttc cgtctacagg cttattacca 62220
   gggactggaa tagcagaaat tgaacagtga gatgtacaga gaactctaag aatgcaggaa 62280
   taggccaggc atggtggctc acacctgtca tcccagcact ttgggagacc aaggcgggtg 62340
   gatcacctga ggtcaggagt tcgagaccag cctggccaac atagtgaaac cccatctcta 62400
   ctaaaaatac aaaaaaatta gctgggtgtg gtggcgcatg cctgtaatcc cagctactcg 62460
   ggaggctgag gcaggagaat cacttgaacc tgggaggcag aggttgcagt gagccgagat 62520
   catgccactg tactccagcc tgggtggaag agcggaactc tgtctgaaaa aaaaaaaaaa 62580
   aacaagaagt tcaacttgaa gggaaaaatg ccgtattgtc tttccctttg ttatgtcacc 62640
   agggcacagt ccatcccagg ctggcgctga tccacgggct ggagaggggc tgccccagaa 62700
   gaggacatgc caggaagggc ttggctggtg ttcaggagcc caggccaggt caggtcaaga 62760
   ggtgttgagg ctggacggga gaggccagct aggggctcat gtaggatatg aggggtcggc 62820
   ccatttcaac gtggaaactg agctcttctg cttctctttc ttcttcactg cattaagatt 62880
   caataccgct tgggaagcag gtatttccct tcctataaag gatggttggg agcctgagtg 62940
   ttgggagaaa gtgtagccgc tgagttacta acaactaggg ctgccgtcaa gcctatgggg 63000
   aaagagagaa gaggacattt ggaaggagag agatcaagct gtggcaccct gggagaggac 63060
   cacagaaaag aggccagtga gggggttccc cggtggcatc tgaaggtgtg gcccaaccag 63120
   gaggtccaga ggctgccagc cgagtggccc aggagaggga acctcacagg ggctgagtgg 63180
   gacccaagcc ctatccaccg tcctaaccac ccacatttct cgggaacaag acctcccaca 63240
   gtggcctccc cggcagtgga aatagccaaa ctggcaacat ggactttctt caactgcccg 63300
   ggcgatgctg cctcagtgcc ccagggcagg caggaagctc ccacacccat tctggaatga 63360
   ggggttggag gaaggctgag ctgagcaaag gacccatctc tgctctggtt ggtggggagg 63420
   gagcccatta tacaagagac ccctcagggc tcagtgaggg gtgacagaga cttggggagt 63480
   agtggctgtc actgcagagg tgagagggtt tggagagaag gtacatgcct ttttggccac 63540
   attgagtagc acctggtagc cagttagtaa cgtgtattgg ataaacaaaa gattaaacgg 63600
   atgcaaaaaa aaatgttggc tttgcttctt tttacccaaa cctcagttcc ctcaagtaga 63660
   ttctgggaac accccctacc tggctggact gttgtgaagt ttaaataagc caggttaact 63720
   tcacctcctc ctttaagaca cagctcagac actgcctcct ccaagaagcc ccctctggct 63780
   tcctgtgtga atatgacggc cctctgggct ctagggtatc ttagaacaat gcttccttat 63840
   ggctttggaa ccccgctgtc tcctggattg ggagcaaatg caggggagga gccacacctg 63900
   actaatctct gggtctccca gcacataagt ggcataaggg cagggctgtg cccgcttcag 63960
   gcacttactg aaggatgtac ttggcagagg gtaggcagcc ggcggatgag cccctcactc 64020
   tccccagctg actgcgtggg cgggaaaggc gggttcagga gacccagcct ccctgggctg 64080
   tcaccacctc tgcacatcca gccccattga tcaagggttc aatttttggg gtcctgttgg 64140
   gaggccagga gactctctcc aggcacttct tccaggtctt tgtgttaggg tgtgtgtgtg 64200
   tgtgtgtgtg tgtgtgtgtg tgtgttgttt gttttatttt atttatttat ttatttattt 64260
   atttatttat ttatttattt tgagacgcag tctcgctctg ttgcccaggt tggagggtgg 64320
   tggcatgatc tcggctcact gcaagctccg cctcccgggt tcacgccatt ctcctgcctc 64380
   actcttcctg agtagccgga ttacaggcgc acgcaccatg cctggctaat tattttgttt 64440
   ttttagtaga gacagggttt cgccacgttg cccaggctgg tcttgaatcc ctggcctcaa 64500
   gcgatccgcc cgcctcagcc tcccaaagtg ctgggattac aggcgtgagc caccgtgccc 64560
   gcccagccta ggggtacatg aaactttttt tttttttttt ttgagacaga gtttcactct 64620
   gtcctcaggc tggagtgcag tggcgtgatc tcggcgtact gcaatctccg cctcccggtt 64680
   caagcgattc tcctgcctca gcctcccgag tagctgggat tgcaggcacg cgccaccaca 64740
   cccagctaat ttttgtattt ttagtagaga cgggctttca ccatgtggga caggatggtc 64800
   tcgatctcct gacctcgtga tccgcccgcc tcagcctccg aaagtgctgg gattacaggc 64860
   ctgagccacc gtgcccagcc atgatgtttt gatacaggca tataacgtat aataatcaca 64920
   tcagggtaaa tgatgtaacc atcacatcaa gcatttatcc tttgtgttac aaaaaaaaat 64980
   ctaattatac tttcctactt attctttttt tttttttttt ttgagacgga gtctccctca 65040
   gtcgcccagg ctggagtgca gtggcatgat ctcagttcac tgcaagctct gcctcctagc 65100
   tctgcctcct gggttcatgc cattctcctg tctcagcctc gcgagtagct gggactacag 65160
   gcgcctgcca ccgtgcccgg ctaatttttt tttttgtatt tttggtagag acagggtttc 65220
   accgtgttag ccaggatggt ctcgatctcc tgacctcata atccgcccgt ctcggcctcc 65280
   caaagtgctg ggattacagg catgagccac cgcccccagc ctatttattc ttaaatgtac 65340
   aataaattat tgttgactcc agtcaccctg ctgtgctacc aaatacggat cttcttcatt 65400
   ctatctaact gtatttctgt acctgttaac catctctcct ccacctcacc ccccaaaccc 65460
   actacccttc tcagcctctg gtaaccatcc ttctactctc tatctctatg agttcaattg 65520
   tattaatttt tagctccccg gccgggcacg gtggctcacg cctgtaatcc cagcacttca 65580
   ggaggctgag gcaggtggat cacgaggtca ggagtttgag accagcctgg ccaacatggt 65640
   ggaaccccat ctctactaaa aacacaaaaa ttagctgggc gtggtggtgg gcgcttgtag 65700
   tcccagctac ttgggaggct gaggcaggag aatcgcttga aactgggagg cagaggttgc 65760
   agtgagccaa gattgcgcca ctgcactcca gtctgggtga cagagtaaga ttccatcccg 65820
   aaaaaaaaaa agtttagctc ccacaaataa gtgagaacac gtgaagtttc tctttctgtg 65880
   cctcgcttgt ttcacttaac ataatgacct ccagttccat ccacgttgtt gctttgttat 65940
   aaatgacagg atcttggtca ggcgcagtgg ctcatgcctg taatcccagc actttgggag 66000
   gctgaggtgg actgatcatg aggtcaagag atcgagacca tcctggctaa cacagtgaaa 66060
   ccccgtctct actaaaaata caagaaatta gccgggcgtg gtggtgggca cccatttccg 66120
   ccccttctcg ggacgctgat gcacgacata ttacccatcc ccggaagact aatcctcccc 66180
   cactctatat tgtacctctt cctttctcct ccacgcgatt ccccgagtaa cccgtcttcc 66240
   ctccctcctc ggattacgct cacctttccg cttcaatcac gttgctccgt ccccttcccc 66300
   attcgtacca ctcctcactt tcgtcttcct acccccacta tcccttttcg tcctctctat 66360
   tccttactta ctcctccccc ttctcttcat acttcattcc ctccgctctt cccactcgcg 66420
   ctcccacttt cacctagttg ccctcaccta cgttgccatc tcgccccttc ttcagctctc 66480
   ggcctctcac ccatctgtcc tctctcttac ctctctcctc atctcgctca gacatctctc 66540
   tagactatcc ctcactttac cttctcagtc gtcttcttcc tatccttcgt tctccatgat 66600
   cttcacgtcg ccatctcttt tcgccccttt catatgtctc tcttcatgtt ctcactatca 66660
   ttctcatgat cactatcgtt ctcactactt atcactcccc tctttcttca tcaattcctc 66720
   tccgtcattc tcgtctctct cttacaaccg ccttccttgt gctatctaac tcaaccatgc 66780
   ctctcctact ctctctctat cgcccctcca tcgcttatgc atcctcttct attgcacacc 66840
   cgcccctcca tcgcttatgc atcctcttct attgcacacc gcccctccat cgcttatgca 66900
   tcctcttcta ttgcacatcc tcttctattg cac 66933
<210> 12
   <211> 21
   <212> DNA
   <213> Artificial Sequence
   <220>
   <223> Artificial sequence is a primer.
<400> 12
   ctgagcggaa ttcgtgagac c 21
<210> 13
   <211> 23
   <212> DNA
   <213> Artificial Sequence
   <220>
   <223> Artificial sequence is a primer.
<400> 13
   ttggtctcac gtattccgct cga 23
<210> 14
   <211> 20
   <212> DNA
   <213> Artificial Sequence
   <220>
   <223> Artificial sequence is a primer.
<400> 14
   ctcgagaatt ctggatcctc 20
<210> 15
   <211> 22
   <212> DNA
   <213> Artificial Sequence
   <220>
   <223> Artificial sequence is a primer.
<400> 15
   ttgaggatcc agaattctcg ag 22
<210> 16
   <211> 21
   <212> DNA
   <213> Artificial Sequence
   <220>
   <223> Artificial sequence is a primer.
<400> 16
   tgtatgcgaa ttcgctgcgc g 21
<210> 17
   <211> 23
   <212> DNA
   <213> Artificial Sequence
   <220>
   <223> Artificial sequence is a primer.
<400> 17
   ttcgcgcagc gaattcgcat aca 23
<210> 18
   <211> 21
   <212> DNA
   <213> Artificial Sequence
   <220>
   <223> Artificial sequence is a primer.
<400> 18
   gtccactgaa ttctcagtga g 21
<210> 19
   <211> 23
   <212> DNA
   <213> Artificial Sequence
   <220>
   <223> Artificial sequence is a primer.
<400> 19
   ttgtcactga gaattcagtg gac 23
<210> 20
   <211> 21
   <212> DNA
   <213> Artificial Sequence
   <220>
   <223> Artificial sequence is a primer.
<400> 20
   gaatccgaat tcctggtcag c 21
<210> 21
   <211> 23
   <212> DNA
   <213> Artificial Sequence
   <220>
   <223> Artificial sequence is a primer.
<400> 21
   ttgctgacca ggaattcgga ttc 23
<210> 22
   <211> 33
   <212> DNA
   <213> Artificial Sequence
   <220>
   <223> Artificial sequence is a primer.
<400> 22
   cuacuacuac uactgagcgg aattcgtgag acc 33
<210> 23
   <211> 32
   <212> DNA
   <213> Artificial Sequence
   <220>
   <223> Artificial sequence is a primer.
<400> 23
   cuacuacuac uactcgagaa ttctggatcc tc 32
<210> 24
   <211> 33
   <212> DNA
   <213> Artificial Sequence
   <220>
   <223> Artificial sequence is a primer.
<400> 24
   cuacuacuac uatgtatgcg aattcgctgc gcg 33
<210> 25
   <211> 33
   <212> DNA
   <213> Artificial Sequence
   <220>
   <223> Artificial sequence is a primer.
<400> 25
   cuacuacuac uagtccactg aattctcagt gag 33
<210> 26
   <211> 33
   <212> DNA
   <213> Artificial Sequence
   <220>
   <223> Artificial sequence is a primer.
<400> 26
   cuacuacuac uagaatccga attcctggtc agc 33
<210> 27
   <211> 45
   <212> DNA
   <213> Artificial Sequence
   <220>
   <223> Artificial sequence is a primer.
<400> 27
   aactggaaga attcgcggcc gcaggaattt tttttttttt ttttt 45
<210> 28
   <211> 13
   <212> DNA
   <213> Artificial Sequence
   <220>
   <223> Artificial sequence is a primer.
<400> 28
   aattcggcac gag 13
<210> 29
   <211> 9
   <212> DNA
   <213> Artificial Sequence
   <220>
   <223> Artificial sequence is a primer.
<400> 29
   ctcgtgccg 9
<210> 30
   <211> 14
   <212> DNA
   <213> Artificial Sequence
   <220>
   <223> Artificial sequence is a primer.
<400> 30
   gtacgacggc cagt 14
<210> 31
   <211> 16
   <212> DNA
   <213> Artificial Sequence
   <220>
   <223> Artificial sequence is a primer.
<400> 31
   aacagctatg accatg 16
<210> 32
   <211> 18
   <212> DNA
   <213> Artificial Sequence
   <220>
   <223> Artificial sequence is a primer.
<400> 32
   ccaagttctg agaagtcc 18
<210> 33
   <211> 20
   <212> DNA
   <213> Artificial Sequence
   <220>
   <223> Artificial sequence is a primer.
<400> 33
   aatacctgaa accatacctg 20
<210> 34
   <211> 57
   <212> DNA
   <213> Artificial Sequence
   <220>
   <223> Artificial sequence is a primer.
<400> 34
   agctgctcgt agctgtctct ccctggatca cgggtacatg tactggacag actgggt 57
<210> 35
   <211> 56
   <212> DNA
   <213> Artificial Sequence
   <220>
   <223> Artificial sequence is a primer.
<400> 35
   tgagacgccc ggattgagcg ggcagggata gcttattccc tgtgccgcat tacggc 56
<210> 36
   <211> 27
   <212> DNA
   <213> Artificial Sequence
   <220>
   <223> Artificial sequence is a primer.
<400> 36
   agctgctcgt agctgtctct ccctgga 27
<210> 37
   <211> 27
   <212> DNA
   <213> Artificial Sequence
   <220>
   <223> Artificial sequence is a primer.
<400> 37
   gccgtaatgc ggcacaggga ataagct 27
<210> 38
   <211> 20
   <212> DNA
   <213> Artificial Sequence
   <220>
   <223> Artificial sequence is a primer.
<400> 38
   gagaggctat atccctgggc 20
<210> 39
   <211> 20
   <212> DNA
   <213> Artificial Sequence
   <220>
   <223> Artificial sequence is a primer.
<400> 39
   acagcacgtg tttaaagggg 20
<210> 40
   <211> 163
   <212> DNA
   <213> Homo sapiens
<400> 40
   actaaagcgc cgccgccgcg ccatggagcc cgagtgagct cggcgcgggc ccgtccggcc     60
   gccggacaac atggaggcag ctccgcccgg gccgccgtgg ccgctgctgc tgctgctgct    120
   gctgctgctg gcgctgtgcg gctgcccggc ccccgccgcg gcc                        163
<210> 41
   <211> 419
   <212> DNA
   <213> Homo sapiens
<400> 41
   gccccacagc ctcgccgctc ctgctatttg ccaaccgccg ggacgtacgg ctggtggacg     60
   ccggcggagt caagctggag tccaccatcg tggtcagcgg cctggaggat gcggccgcag    120
   tggacttcca gttttccaag ggagccgtgt actggacaga cgtgagcgag gaggccatca    180
   agcagaccta cctgaaccag acgggggccg ccgtgcagaa cgtggtcatc tccggcctgg    240
   tctctcccga cggcctcgcc tgcgactggg tgggcaagaa gctgtactgg acggactcag    300
   agaccaaccg catcgaggtg gccaacctca atggcacatc ccggaaggtg ctcttctggc    360
   aggaccttga ccagccgagg gccatcgcct tggaccccgc tcacgggtaa accctgctg     419
<210> 42
   <211> 221
   <212> DNA
   <213> Homo sapiens
<400> 42
   ccccgtcaca ggtacatgta ctggacagac tggggtgaga cgccccggat tgagcgggca     60
   gggatggatg gcagcacccg gaagatcatt gtggactcgg acatttactg gcccaatgga    120
   ctgaccatcg acctggagga gcagaagctc tactgggctg acgccaagct cagcttcatc    180
   caccgtgcca acctggacgg ctcgttccgg taggtaccca c                        221
<210> 43
   <211> 221
   <212> DNA
   <213> Homo sapiens
<400> 43
   tccctgactg caggcagaag gtggtggagg gcagcctgac gcaccccttc gccctgacgc     60
   tctccgggga cactctgtac tggacagact ggcagacccg ctccatccat gcctgcaaca    120
   agcgcactgg ggggaagagg aaggagatcc tgagtgccct atactcaccc atggacatcc    180
   aggtgctgag ccaggagcgg cagccttttt gtgagtgccg g                        221
<210> 44
   <211> 156
   <212> DNA
   <213> Homo sapiens
<400> 44
   tttctcagtc cacactcgct gtgaggagga caatggcggc tggtcccacc tgtgcctgct     60
   gtccccaagc gagccttttt acacatgcgc ctgccccacg ggtgtgcaga tgcaggacaa    120
   cggcaggacg tgtaaggcag gtgaggcggt gggacg                              156
<210> 45
   <211> 416
   <212> DNA
   <213> Homo sapiens
<400> 45
   ctccacagga gccgaggagg tgctgctgct ggcccggcgg acggacctac ggaggatctc     60
   gctggacacg ccggacttca ccgacatcgt gctgcaggtg gacgacatcc ggcacgccat    120
   tgccatcgac tacgacccgc tagagggcta tgtctactgg acagatgacg aggtgcgggc    180
   catccgcagg gcgtacctgg acgggtctgg ggcgcagacg ctggtcaaca ccgagatcaa    240
   cgaccccgat ggcatcgcgg tcgactgggt ggcccgaaac ctctactgga ccgacacggg    300
   cacggaccgc atcgaggtga cgcgcctcaa cggcacctcc cgcaagatcc tggtgtcgga    360
   ggacctggac gagccccgag ccatcgcact gcaccccgtg atggggtaag acgggc        416
<210> 46
   <211> 198
   <212> DNA
   <213> Homo sapiens
<400> 46
   ttcttctcca gcctcatgta ctggacagac tggggagaga accctaaaat cgagtgtgcc     60
   aacttggatg ggcaggagcg gcgtgtgctg gtcaatgcct ccctcgggtg gcccaacggc    120
   ctggccctgg acctgcagga ggggaagctc tactggggag acgccaagac agacaagatc    180
   gaggtgaggc tcctgtgg                                                  198
<210> 47
   <211> 244
   <212> DNA
   <213> Homo sapiens
<400> 47
   ccgtcctgca ggtgatcaat gttgatggga cgaagaggcg gaccctcctg gaggacaagc     60
   tcccgcacat tttcgggttc acgctgctgg gggacttcat ctactggact gactggcagc    120
   gccgcagcat cgagcgggtg cacaaggtca aggccagccg ggacgtcatc attgaccagc    180
   tgcccgacct gatggggctc aaagctgtga atgtggccaa ggtcgtcggt gagtccgggg    240
   ggtc                                                                 244
<210> 48
   <211> 313
   <212> DNA
   <213> Homo sapiens
<400> 48
   gttcgcttcc aggaaccaac ccgtgtgcgg acaggaacgg ggggtgcagc cacctgtgct 60
   tctgcacacc ccacgcaacc cggtgtggct gccccatcgg cctggagctg ctgagtgaca 120
   tgaagacctg catcgtgcct gaggcctttt tggtcttcac cagcagagcc gccatccaca 180
   ggatctccct cgagaccaat aacaacgacg tggccatccc gctcacgggc gtcaaggagg 240
   cctcagccct ggactttgat gtgtccaaca accacatcta ctggacagac gtcagcctga 300
   aggtagcgtg ggc 313
<210> 49
   <211> 255
   <212> DNA
   <213> Homo sapiens
<400> 49
   cctgctgcca gaccatcagc cgcgccttca tgaacgggag ctcggtggag cacgtggtgg 60
   agtttggcct tgactacccc gagggcatgg ccgttgactg gatgggcaag aacctctact 120
   gggccgacac tgggaccaac agaatcgaag tggcgcggct ggacgggcag ttccggcaag 180
   tcctcgtgtg gagggacttg gacaacccga ggtcgctggc cctggatccc accaaggggt 240
   aagtgtttgc ctgtc 255
<210> 50
   <211> 210
   <212> DNA
   <213> Homo sapiens
<400> 50
   gtgccttcca gctacatcta ctggaccgag tggggcggca agccgaggat cgtgcgggcc 60
   ttcatggacg ggaccaactg catgacgctg gtggacaagg tgggccgggc caacgacctc 120
   accattgact acgctgacca gcgcctctac tggaccgacc tggacaccaa catgatcgag 180
   tcgtccaaca tgctgggtga gggccgggct 210
<210> 51
   <211> 352
   <212> DNA
   <213> Homo sapiens
<400> 51
   gtgttcatgc aggtcaggag cgggtcgtga ttgccgacga tctcccgcac ccgttcggtc 60
   tgacgcagta cagcgattat atctactgga cagactggaa tctgcacagc attgagcggg 120
   ccgacaagac tagcggccgg aaccgcaccc tcatccaggg ccacctggac ttcgtgatgg 180
   acatcctggt gttccactcc tcccgccagg atggcctcaa tgactgtatg cacaacaacg 240
   ggcagtgtgg gcagctgtgc cttgccatcc ccggcggcca ccgctgcggc tgcgcctcac 300
   actacaccct ggaccccagc agccgcaact gcagccgtaa gtgcctcatg gt 352
<210> 52
   <211> 225
   <212> DNA
   <213> Homo sapiens
<400> 52
   gcctcctcta cgcccaccac cttcttgctg ttcagccaga aatctgccat cagtcggatg   60
   atcccggacg accagcacag cccggatctc atcctgcccc tgcatggact gaggaacgtc  120
   aaagccatcg actatgaccc actggacaag ttcatctact gggtggatgg gcgccagaac  180
   atcaagcgag ccaaggacga cgggacccag gcaggtgccc tgtgg                   225
<210> 53
   <211> 235
   <212> DNA
   <213> Homo sapiens
<400> 53
   ctttgtctta cagccctttg ttttgacctc tctgagccaa ggccaaaacc cagacaggca   60
   gccccacgac ctcagcatcg acatctacag ccggacactg ttctggacgt gcgaggccac  120
   caataccatc aacgtccaca ggctgagcgg ggaagccatg ggggtggtgc tgcgtgggga   180
   ccgcgacaag cccagggcca tcgtcgtcaa cgcggagcga gggtaggagg ccaac       235
<210> 54
   <211> 218
   <212> DNA
   <213> Homo sapiens
<400> 54
   ccaccctccc gcaggtacct gtacttcacc aacatgcagg accgggcagc caagatcgaa   60
   cgcgcagccc tggacggcac cgagcgcgag gtcctcttca ccaccggcct catccgccct  120
   gtggccctgg tggtggacaa cacactgggc aagctgttct gggtggacgc ggacctgaag  180
   cgcattgaga gctgtgacct gtcaggtacg cgccccgg                           218
<210> 55
   <211> 234
   <212> DNA
   <213> Homo sapiens
<400> 55
   ggctgcttgc aggggccaac cgcctgaccc tggaggacgc caacatcgtg cagcctctgg   60
   gcctgaccat ccttggcaag catctctact ggatcgaccg ccagcagcag atgatcgagc  120
   gtgtggagaa gaccaccggg gacaagcgga ctcgcatcca gggccgtgtc gcccacctca  180
   ctggcatcca tgcagtggag gaagtcagcc tggaggagtt ctgtacgtgg gggc         234
<210> 56
   <211> 157
   <212> DNA
   <213> Homo sapiens
<400> 56
   ttgtctttgc agcagcccac ccatgtgccc gtgacaatgg tggctgctcc cacatctgta 60
   ttgccaaggg tgatgggaca ccacggtgct catgcccagt ccacctcgtg ctcctgcaga 120
   acctgctgac ctgtggaggt aggtgtgacc taggtgc 157
<210> 57
   <211> 272
   <212> DNA
   <213> Homo sapiens
<400> 57
   gttctcctct gtccctcccc cagagccgcc cacctgctcc ccggaccagt ttgcatgtgc 60
   cacaggggag atcgactgta tccccggggc ctggcgctgt gacggctttc ccgagtgcga 120
   tgaccagagc gacgaggagg gctgccccgt gtgctccgcc gcccagttcc cctgcgcgcg 180
   gggtcagtgt gtggacctgc gcctgcgctg cgacggcgag gcagactgtc aggaccgctc 240
   agacgaggtg gactgtgacg gtgaggccct cc 272
<210> 58
   <211> 134
   <212> DNA
   <213> Homo sapiens
<400> 58
   tctccttgca gccatctgcc tgcccaacca gttccggtgt gcgagcggcc agtgtgtcct 60
   catcaaacag cagtgcgact ccttccccga ctgtatcgac ggctccgacg agctcatgtg 120
   tggtgagcca gctt 134
<210> 59
   <211> 274
   <212> DNA
   <213> Homo sapiens
<400> 59
   gtttgtctct ggcagaaatc accaagccgc cctcagacga cagcccggcc cacagcagtg 60
   ccatcgggcc cgtcattggc atcatcctct ctctcttcgt catgggtggt gtctattttg 120
   tgtgccagcg cgtggtgtgc cagcgctatg cgggggccaa cgggcccttc ccgcacgagt 180
   atgtcagcgg gaccccgcac gtgcccctca atttcatagc cccgggcggt tcccagcatg 240
   gccccttcac aggtaaggag cctgagatat ggaa 274
<210> 60
   <211> 164
   <212> DNA
   <213> Homo sapiens
<400> 60
   cttccctgcc aggcatcgca tgcggaaagt ccatgatgag ctccgtgagc ctgatggggg 60
   gccggggcgg ggtgcccctc tacgaccgga accacgtcac aggggcctcg tccagcagct 120
   cgtccagcac gaaggccacg ctgtacccgc cggtgagggg cggg 164
<210> 61
   <211> 130
   <212> DNA
   <213> Homo sapiens
<400> 61
   ttggctctcc tcagatcctg aacccgccgc cctccccggc cacggacccc tccctgtaca     60
   acatggacat gttctactct tcaaacattc cggccactgc gagaccgtac aggtaggaca    120
   tcccctgcag                                                           130
<210> 62
   <211> 496
   <212> DNA
   <213> Homo sapiens
<400> 62
   tcaaacattc cggccactgc gagaccgtac aggccctaca tcattcgagg aatggcgccc     60
   ccgacgacgc cctgcagcac cgacgtgtgt gacagcgact acagcgccag ccgctggaag    120
   gccagcaagt actacctgga tttgaactcg gactcagacc cctatccacc cccacccacg    180
   ccccacagcc agtacctgtc ggcggaggac agctgcccgc cctcgcccgc caccgagagg    240
   agctacttcc atctcttccc gccccctccg tccccctgca cggactcatc ctgacctcgg    300
   ccgggccact ctggcttctc tgtgcccctg taaatagttt taaatatgaa caaagaaaaa    360
   aatatatttt atgatttaaa aaataaatat aattgggatt ttaaaaacat gagaaatgtg    420
   aactgtgatg gggtgggcag ggctgggaga actttgtaca gtggagaaat atttataaac    480
   ttaattttgt aaaaca                                                    496
<210> 63
   <211> 3081
   <212> DNA
   <213> Homo sapiens
<400> 63
   cccgccagcc cagcccagcc caaccctact ccctccccac gccagggcag cagccgttgc 60
   tcagagagaa ggtggaggaa gaaatccaga ccctagcacg cgcgcaccat catggaccat 120
   tatgattctc agcaaaccaa cgattacatg cagccagaag aggactggga ccgggacctg 180
   ctcctggacc cggcctggga gaagcagcag agaaagacat tcacggcatg gtgtaactcc 240
   cacctccgga aggcggggac acagatcgag aacatcgaag aggacttccg ggatggcctg 300
   aagctcatgc tgctgctgga ggtcatctca ggtgaacgct tggccaagcc agagcgaggc 360
   aagatgagag tgcacaagat ctccaacgtc aacaaggccc tggatttcat agccagcaaa 420
   ggcgtcaaac tggtgtccat cggagccgaa gaaatcgtgg atgggaatgt gaagatgacc 480
   ctgggcatga tctggaccat catcctgcgc tttgccatcc aggacatctc cgtggaagag 540
   acttcagcca aggaagggct gctcctgtgg tgtcagagaa agacagcccc ttacaaaaat 600
   gtcaacatcc agaacttcca cataagctgg aaggatggcc tcggcttctg tgctttgatc 660
   caccgacacc ggcccgagct gattgactac gggaagctgc ggaaggatga tccactcaca 720
   aatctgaata cggcttttga cgtggcagag aagtacctgg acatccccaa gatgctggat 780
   gccgaagaca tcgttggaac tgcccgaccg gatgagaaag ccatcatgac ttacgtgtct 840
   agcttctacc acgccttctc tggagcccag aaggcggaga cagcagccaa tcgcatctgc 900
   aaggtgttgg ccgtcaacca ggagaacgag cagcttatgg aagactacga gaagctggcc 960
   agtgatctgt tggagtggat ccgccgcaca atcccgtggc tggagaaccg ggtgcccgag 1020
   aacaccatgc atgccatgca acagaagctg gaggacttcc gggactaccg gcgcctgcac 1080
   aagccgccca aggtgcagga gaagtgccag ctggagatca acttcaacac gctgcagacc 1140
   aagctgcggc tcagcaaccg gcctgccttc atgccctctg agggcaggat ggtctcggac 1200
   atcaacaatg cctggggctg cctggagcag gtggagaagg gctatgagga gtggttgctg 1260
   aatgagatcc ggaggctgga gcgactggac cacctggcag agaagttccg gcagaaggcc 1320
   tccatccacg aggcctggac tgacggcaaa gaggccatgc tgcgacagaa ggactatgag 1380
   accgccaccc tctcggagat caaggccttg ctcaagaagc atgaggcctt cgagagtgac 1440
   ctggctgccc accaggaccg tgtggagcag attgccgcca tcgcacagga gctcaatgag 1500
   ctggactatt atgactcacc cagtgtcaac gcccgttgcc aaaagatctg tgaccagtgg 1560
   gacaatctgg gggccctaac tcagaagcga agggaagctc tggagcggac cgagaaactg 1620
   ctggagacca ttgaccagct gtacttggag tatgccaagc gggctgcacc cttcaacaac 1680
   tggatggagg gggccatgga ggacctgcag gacaccttca ttgtgcacac cattgaggag 1740
   atccagggac tgaccacagc ccatgagcag ttcaaggcca ccctccctga tgccgacaag 1800
   gagcgcctgg ccatcctggg catccacaat gaggtgtcca agattgtcca gacctaccac 1860
   gtcaatatgg cgggcaccaa cccctacaca accatcacgc ctcaggagat caatggcaaa 1920
   tgggaccacg tgcggcagct ggtgcctcgg agggaccaag ctctgacgga ggagcatgcc 1980
   cgacagcagc acaatgagag gctacgcaag cagtttggag cccaggccaa tgtcatcggg 2040
   ccctggatcc agaccaagat ggaggagatc gggaggatct ccattgagat gcatgggacc 2100
   ctggaggacc agctcagcca cctgcggcag tatgagaaga gcatcgtcaa ctacaagcca 2160
   aagattgatc agctggaggg cgaccaccag ctcatccagg aggcgctcat cttcgacaac 2220
   aagcacacca actacaccat ggagcacatc cgtgtgggct gggagcagct gctcaccacc 2280
   atcgccagga ccatcaatga ggtagagaac cagatcctga cccgggatgc caagggcatc 2340
   agccaggagc agatgaatga gttccgggcc tccttcaacc actttgaccg ggatcactcc 2400
   ggcacactgg gtcccgagga gttcaaagcc tgcctcatca gcttgggtta tgatattggc 2460
   aacgaccccc agggagaagc agaatttgcc cgcatcatga gcattgtgga ccccaaccgc 2520
   ctgggggtag tgacattcca ggccttcatt gacttcatgt cccgcgagac agccgacaca 2580
   gatacagcag accaagtcat ggcttccttc aagatcctgg ctggggacaa gaactacatt 2640
   accatggacg agctgcgccg cgagctgcca cccgaccagg ctgagtactg catcgcgcgg 2700
   atggccccct acaccggccc cgactccgtg ccaggtgctc tggactacat gtccttctcc 2760
   acggcgctgt acggcgagag tgacctctaa tccaccccgc ccggccgccc tcgtcttgtg 2820
   cgccgtgccc acagatgtga aatgaatgta atctaataga agcctaatca gcccaccatg 2880
   ttctccactg aaaaatcctc tttctttggg gtttttcttt ctttcttttt tgattttgca 2940
   ctggacggtg acgtcagcct gtacaggctc ccaggggtgg cgtcaaatgc tattgaaatt 3000
   gcgctgaatc gtatgctttt tccttttgat aaataaacaa tgtaaaaatg tttcaaaaac 3060
   ctaataaaat aaataaatac g 3081
<210> 64
   <211> 1324
   <212> DNA
   <213> Homo sapiens
<220>
   <221> misc_feature
   <222> (1)...(1324)
   <223> n = A,T,C or G
<400> 64
   ggccgcccgg cgcccccagc agnccgagcc ggggcgcaca gncggggcgc agcccgcgcc     60
   ccccgccgcg attgacatga tgtttccaca aagcaggcat tcgggctcct cgcacctacc    120
   ccagcaactc aaattcacca cctcggactc ctgcgaccgc atcaaagacg aatttcagct    180
   actgcaagct cagtaccaca gcctcaagct cgaatgtgac aagttggcca gtgagaagtc    240
   agagatgcag cgtcactatg tgatgtacta cgagatgtcc tacggcttga acatcgagat    300
   gcacaaacag gctgagatcg tcaaaaggct gaacgggatt tgtgcccagg tcctgcccta    360
   cctctcccaa gagcaccagc agcaggtctt gggagccatt gagagggcca agcaggtcac    420
   cgctcccgag ctgaactcta tcatccgaca gcagctccaa gcccaccagc tgtcccagct    480
   gcaggccctg gccctgccct tgaccccact acccgtgggg ctgcagccgc cttcgctgcc    540
   ggcggtcagc gcaggcaccg gcctcctctc gctgtccgcg ctgggttccc aggcccacct    600
   ctccaaggaa gacaagaacg ggcacgatgg tgacacccac caggaggatg atggcgagaa    660
   gtcggattag caggggggcg ggacagggag gttgggaggg gggacagagg ggagacagag    720
   gcacggagag aaaggaatgt ttagcacaag acacagcgga gctcgggatt ggctaatctc    780
   ccatagtatt tatggtggcg ccggcggggc cccagcccag cttgcaggcc acctctagct    840
   ttcttcctac cccattccgg cttccctcct cctcccctgc agcctggtta ggtggatacc    900
   tgccctgaca tgtgaggcaa gctaaggcct ggagggtcag atgggagacc aggtcccaag    960
   ggagcaagac ctgcgaagcg cagcagcccc ggcccttccc ccgttttgaa catgtgtaac   1020
   cgacagtctg ccctgggcca cagccctctc accctggtac tgcatgcacg caatgctagc   1080
   tgcccctttc ccgtcctggg caccccgagt ctcccccgac cccgggtccc aggtatgctc   1140
   ccacctccac ctgccccact caccacctct gctagttcca gacacctcca cgcccacctg   1200
   gtcctctccc atcgcccaca aaaggggggg cacgagggac gagcttagct gagctgggag   1260
   gagcagggtg agggtgggcg acccaggatt ccccctcccc ttcccaaata aagatgaggg   1320
   tact                                                                1324
<210> 65
   <211> 2377
   <212> DNA
   <213> Homo sapiens
<400> 65
   ggtgacaaag agccaacaga gacaatagga gacttgtcaa tttgtcttga tgggctacag      60
   ttagagtctg aagttgttac caatggtgaa actacatgtt cagaaagtgc ttctcagaat     120
   gatgatggct ccagatccaa ggatgaaaca agagtgagca caaatggatc agatgaccct     180
   gaagatgcag gagctggtga aaataggaga gtcagtggga ataattctcc atcactctca     240
   aatggtggtt ttaaaccttc tagacctcca agaccttcac gaccaccacc acccacccca     300
   cgtagaccag catctgtcaa tggttcacca tctgccactt ctgaaagtga tgggtctagt     360
   acaggctctc tgccgccgac aaatacaaat acaaatacat ctgaaggagc aacatctgga     420
   ttaataattc ctcttactat atctggaggc tcaggcccta ggccattaaa tcctgtaact     480
   caagctccct tgccacctgg ttgggagcag agagtggacc agcacgggcg agtttactat     540
   gtagatcatg ttgagaaaag aacaacatgg gatagaccag aacctctacc tcctggctgg     600
   gaacggcggg ttgacaacat gggacgtatt tattatgttg accatttcac aagaacaaca     660
   acgtggcaga ggccaacact ggaatccgtc cggaactatg aacaatggca gctacagcgt     720
   agtcagcttc aaggagcaat gcagcagttt aaccagagat tcatttatgg gaatcaagat     780
   ttatttgcta catcacaaag taaagaattt gatcctcttg gtccattgcc acctggatgg     840
   gagaagagaa cagacagcaa tggcagagta tatttcgtca accacaacac acgaattaca     900
   caatgggaag accccagaag tcaaggtcaa ttaaatgaaa agcccttacc tgaaggttgg     960
   gaaatgagat tcacagtgga tggaattcca tattttgtgg accacaatag aagaactacc    1020
   acctatatag atccccgcac aggaaaatct gccctagaca atggacctca gatagcctat    1080
   gttcgggact tcaaagcaaa ggttcagtat ttccggttct ggtgtcagca actggccatg    1140
   ccacagcaca taaagattac agtgacaaga aaaacattgt ttgaggattc ctttcaacag    1200
   ataatgagct tcagtcccca agatctgcga agacgtttgt gggtgatttt tccaggagaa    1260
   gaaggtttag attatggagg tgtagcaaga gaatggttct ttcttttgtc acatgaagtg    1320
   ttgaacccaa tgtattgcct gtttgaatat gcagggaagg ataactactg cttgcagata    1380
   aaccccgctt cttacatcaa tccagatcac ctgaaatatt ttcgttttat tggcagattt    1440
   attgccatgg ctctgttcca tgggaaattc atagacacgg gtttttcttt accattctat    1500
   aagcgtatct tgaacaaacc agttggactc aaggatttag aatctattga tccagaattt    1560
   tacaattctc tcatctgggt taaggaaaac aatattgagg aatgtgattt ggaaatgtac    1620
   ttctccgttg acaaagaaat tctaggtgaa attaagagtc atgatctgaa acctaatggt    1680
   ggcaatattc ttgtaacaga agaaaataaa gaggaataca tcagaatggt agctgagtgg    1740
   aggttgtctc gaggtgttga agaacagaca caagctttct ttgaaggctt taatgaaatt    1800
   cttccccagc aatatttgca atactttgat gcaaaggaat tagaggtcct tttatgtgga    1860
   atgcaagaga ttgatttgaa tgactggcaa agacatgcca tctaccgtca ttatgcaagg    1920
   accagcaaac aaatcatgtg gttttggcag tttgttaaag aaattgataa tgagaagaga    1980
   atgagacttc tgcagtttgt tactggaacc tgccgattgc cagtaggagg atttgctgat    2040
   ctcatgggga gcaatggacc acagaaattc tgcattgaaa aagttgggaa agaaaattgg    2100
   ctacccagaa gtcatacctg ttttaatcgc ctggacctgc caccatacaa gagctatgag    2160
   caactgaagg aaaagctgtt gtttgccata gaagaaacag aaggatttgg acaagagtaa    2220
   cttctgagaa cttgcaccat gaatgggcaa gaacttattt gcaatgtttg tccttctctg    2280
   cctgttgcac atcttgtaaa attggacaat ggctctttag agagttatct gagtgtaagt    2340
   aaattaatgt tctcatttaa aaaaaaaaaa aaaaaaa                             2377
<210> 66
   <211> 1295
   <212> DNA
   <213> Homo sapiens
<400> 66
   gggccgccgc ccacccgggc cttgcctcta cctcagtcgt tgccccccga ttttcggctg      60
   gagcccacgg ccccggccct cagcccccgc tctagcttcg ccagtagctc ggccagcgac     120
   gcgagcaagc cgtccagccc ccggggcagc ctgctgctgg acggggcggg ggctggcgga     180
   gctggaggta gccggccctg cagcaatcgc accagcggca tcagcatggg ctacgaccag    240
   cgccacggga gccccttgcc agcggggccg tgcctgtttg gcccacccct ggccggagca    300
   ccggcaggct attctcccgg aggggtcccg tccgcctacc cggagctcca cgccgccctg    360
   gaccgattgt acgctcagcg gcccgcgggg ttcggctgcc aggaaagccg ccactcgtat    420
   cccccggccc tgggcagccc tggagctcta gccggggccc gagtgggagc ggcggggccc    480
   ttggagagac ggggggcgca acccggacga cactctgtga ccggctacgg ggactgcgcc     540
   gtgggcgccc ggtaccagga cgagctaaca gctttgcttc gcctgacggt gggcaccggt    600
   gggcgagaag ccggagcccg cggagaaccc tcggggattg agccgtcggg tctggaggag    660
   ccaccaggtc ctttcgttcc ggaggccgcc cgggcccgga tgcgggagcc agaggccagg    720
   gaggactact tcggcacctg tatcaagtgc aacaaaggca tctatgggca gagcaatgcc    780
   tgccaggccc tggacagcct ctaccacacc cagtgctttg tttgctgctc ttgtgggcga    840
   actttgcgtt gcaaggcttt ctacagtgtc aatggctctg tgtactgtga ggaagattat    900
   ctgttttcag ggtttcagga ggcagctgag aaatgctgtg tctgtggtca cttgattttg    960
   gagaagatcc tacaagcaat ggggaagtcc tatcatccag gctgtttccg atgcattgtt   1020
   tgcaacaagt gcctggatgg catccccttc acagtggact tctccaacca agtatactgt   1080
   gtcaccgact accacaaaaa ttatgctcct aagtgtgcag cctgtggcca acccatcctc   1140
   ccctctgagg gctgtgagga catcgtgagg gtgatatcca tggaccggga ttatcacttt   1200
   gagtgctacc actgtgagga ctgccggatg cagctgagtg atgaggaagg ctgctgctgt   1260
   ttccctctgg atgggcactt gctctgccat ggttg                              1295
<210> 67
   <211> 3411
   <212> DNA
   <213> Homo sapiens
<400> 67
   gggcccgggg tcccgccacc accgcgcgcg ggacagattg attcactttg gagctgtaag     60
   tactgatgta ttagggtgca gcgctcattg ttcattgacg cagagtccca aaatgaatat    120
   ccaagagcag ggtttcccct tggacctcgg agcaagtttc accgaagatg ctccccgacc    180
   cccagtgcct ggtgaggagg gagaactggt gtccacagac ccgaggcccg ccagctacag    240
   tttctgctcc gggaaaggtg ttggcattaa aggtgagact tcgacggcca ctccgaggcg    300
   ctcggatctg gacctggggt atgagcctga gggcagtgcc tcccccaccc caccatactt    360
   gaagtgggct gagtcactgc attccctgct ggatgaccaa gatgggataa gcctgttcag    420
   gactttcctg aagcaggagg gctgtgccga cttgctggac ttctggtttg cctgcactgg    480
   cttcaggaag ctggagccct gtgactcgaa cgaggagaag aggctgaagc tggcgagagc    540
   catctaccga aagtacattc ttgataacaa tggcatcgtg tcccggcaga ccaagccagc    600
   caccaagagc ttcataaagg gctgcatcat gaagcagctg atcgatcctg ccatgtttga    660
   ccaggcccag accgaaatcc aggccactat ggaggaaaac acctatccct ccttccttaa    720
   gtctgatatt tatttggaat atacgaggac aggctcggag agccccaaag tctgtagtga    780
   ccagagctct gggtcaggga cagggaaggg catatctgga tacctgccga ccttaaatga    840
   agatgaggaa tggaagtgtg accaggacat ggacgaggac gatggcagag acgctgctcc    900
   ccccggaaga ctccctcaga agctgctcct ggagacagct gccccgaggg tctcctccag    960
   tagacggtac agcgaaggca gagagttcag gtatggatcc tggcgggagc cagtcaaccc   1020
   ctattatgtc aatgccggct atgccctggc cccagccacc agtgccaacg acagcgagca   1080
   gcagagcctg tccagcgatg cagacaccct gtccctcacg gacagcagcg tggatgggat   1140
   ccccccatac aggatccgta agcagcaccg cagggagatg caggagagcg cgcaggtcaa   1200
   tgggcgggtg cccctacctc acattccccg cacgtaccgg gtgccgaagg aggtccgcgt   1260
   ggagcctcag aagttcgcgg aggagctcat ccaccgcctg gaggctgtgc agcgcacgcg   1320
   ggaggccgag gagaagctgg aggagcggct gaagcgcgtg cgcatggagg aggaaggtga   1380
   ggacggcgat ccatcgtcag ggcccccagg gccgtgtcac aagctgcctc ccgcccccgc   1440
   ttggcaccac ttcccgcccc gcttgtgttg gacatgggct tgtgccgggc tccgggatgc   1500
   acacgaggag aaccctgaga gcatcctgga cgagcacgta cagcgtgtgc tgaggacaac   1560
   tggccgccag tcgcctgggc ctggccatcg ctccccggac agtgggcacg tggccaagat   1620
   gccagtggca ctggggggtg ccgcctcggg gcacgggaag cacgtaccca agtcaggggc 1680
   gaagctggac gcggccggcc tgcaccacca ccgacacgtc caccaccacg tccaccacag 1740
   cacagcccgg cccaaggagc aggtggaggc cgaggccacc cgcagggccc agagcagctt 1800
   cgcctggggc ctggaaccac acagccatgg ggcaaggtcc cgaggctact cagagagtgt 1860
   tggcgctgcc cccaacgcca gtgatggcct cgcccacagt gggaaggtgg gcgttgcgtg 1920
   caaaagaaat gccaagaagg ctgagtcggg gaagagcgcc agcaccgagg tgccaggtgc 1980
   ctcggaggat gcggagaaga accagaaaat catgcagtgg atcattgagg gggaaaagga 2040
   gatcagcagg caccgcagga ccggccacgg gtcttcgggg acgaggaagc cacagcccca 2100
   tgagaactcc agaccyttgt cccttgagca cccctgggcc ggccctcagc tccggacctc 2160
   cgtgcagccc tcccacctct tcatccaaga ccccaccatg ccaccccacc cagctcccaa 2220
   ccccctaacc cagctggagg aggcgcgccg acgtctggag gaggaagaaa agagagccag 2280
   ccgagcaccc tccaagcaga ggtatgtgca ggaggttatg cggcggggac gcgcctgcgt 2340
   caggccagcg tgcgcgccgg tgctgcacgt ggtaccagcc gtgtcggaca tggagctctc 2400
   cgagacagag acaagatcgc agaggaaggt gggcggcggg agtgcccagc cgtgtgacag 2460
   catcgttgtg gcgtactact tctgcgggga acccatcccc taccgcaccc tggtgagggg 2520
   ccgcgctgtc accctgggcc agttcaagga gctgctgacc aaaaagggca gctacagata 2580
   ctacttcaag aaagtgagcg acgagtttga ctgtggggtg gtgtttgagg aggttcgaga 2640
   ggacgaggcc gtcctgcccg tctttgagga gaagatcatc ggcaaagtgg agaaggtgga 2700
   ctgataggct ggtgggctgg ccgctgtgcc aggcgaggcc cttggcgggc acgggtgtca 2760
   cggccaggca gatgacctcg tactcaggag cccgatgggg aacagtgttg ggtgtaccac 2820
   ccatccctgt ggtctacccg tgtctagagg caggtagggg gtccctccaa gtggtccaca 2880
   agcttctgtc ctgcccccaa ggaggcagcc tggaccactc ctcatagcaa tacttggagg 2940
   gcccagccca agtgaggcag ccgaggtccc tgctgccagc ttcaggtgac ccccccccat 3000
   cccccggcac ctcccttggg cacgtgtgct gggatctact ttccctctgg gatttgccca 3060
   cgtacccagg tctggctggg gcccaggccc ggatgcagag gcctgcaggg cctctgtcaa 3120
   ttgtacgcgc caccaagtgc cttcaacaca gcttgtctct tgcctgccac tgtgtgaatc 3180
   ggcgacggag cactgcacct gcctccagcc gccggctgtg cagtcctggg tcctcctttc 3240
   tgagggcccg tgtaaatatg tacatttctc aggctagggc cagcaggggc tgcccgagtc 3300
   tgtttttcat gcgatgacac ttgtacaatt atcttttcaa aggtacttgg ataataatga 3360
   aataaaactg tttttgaacc tgaataaaaa aaaaaaaaaa aaaaaaaaaa a 3411
<210> 68
   <211> 3140
   <212> DNA
   <213> Homo sapiens
<400> 68
   ggctgcgagt acctccatgg tcccggtggc tgtgacggcg gcagtggcgc ctgtcctgtc 60
   cataaacagc gatttctcag atttgcggga aattaaaaag caactgctgc ttattgcggg 120
   ccttacccgg gagcggggcc tactacacag tagcaaatgg tcggcggagt tggctttctc 180
   tctccctgca ttgcctctgg ccgagctgca accgcctccg cctattacag aggaagatgc 240
   ccaggatatg gatgcctata ccctggccaa ggcctacttt gacgttaaag agtatgatcg 300
   ggcagcacat ttcctgcatg gctgcaatag caagaaagcc tattttctgt atatgtattc 360
   cagatatctg tctggagaaa aaaagaagga cgatgaaaca gttgatagct taggccccct 420
   ggaaaaagga caagtgaaaa atgaggcgct tagagaattg agagtggagc tcagcaaaaa 480
   acaccaagct cgagaacttg atggatttgg actttatctg tatggtgtgg tgcttcgaaa 540
   actggacttg gttaaagagg ccattgatgt gtttgtggaa gctactcatg ttttgccctt 600
   gcattgggga gcctggttag aactctgtaa cctgatcaca gacaaagaga tgctgaagtt 660
   cctgtctttg ccagacacct ggatgaaaga gttttttctg gctcatatat acacagagtt 720
   gcagttgata gaggaggccc tgcaaaagta tcagaatctc attgatgtgg gcttctctaa 780
   gagctcgtat attgtttccc aaattgcagt tgcctatcac aatatcagag atattgacaa 840
   agccctctcc atttttaatg agctaaggaa acaagaccct tacaggattg aaaatatgga 900
   cacattctcc aaccttcttt atgtcaggag catgaaatcg gagttgagtt atctggctca 960
   taacctctgt gagattgata aataccgtgt agaaacgtgc tgtgtaattg gcaattatta 1020
   cagtttacgt tctcagcatg agaaagcagc cttatatttc cagagagccc tgaaattaaa 1080
   tcctcggtat cttggtgcct ggacactaat gggacatgag tacatggaga tgaagaacac 1140
   gtctgctgct atccaggctt atagacatgc cattgaggtc aacaaacggg actacagagc 1200
   ttggtatggc ctcgggcaga cctatgaaat ccttaagatg ccattttact gcctttatta 1260
   ttatagacgg gcccaccagc ttcgacccaa tgattctcgc atgctggttg ctttaggaga 1320
   atgttacgag aaactcaatc aactagtgga agccaaaaag tgttattgga gagcttacgc 1380
   cgtgggagat gtggagaaaa tggctctggt gaaactggca aagcttcatg aacagttgac 1440
   tgagtcagaa caggctgccc agtgttacat caaatatatc caagatatct attcctgtgg 1500
   ggaaatagta gaacacttgg aggaaagcac tgcctttcgc tatctggccc agtactattt 1560
   taagtgcaaa ctgtgggatg aagcttcaac ttgtgcacaa aagtgttgtg catttaatga 1620
   tacccgggaa gaaggtaagg ccttactccg gcaaatccta cagcttcgga accaaggcga 1680
   gactcctacc accgaggtgc ctgctccctt tttcctacct gcttcactct ctgctaacaa 1740
   tacccccaca cgcagagttt ctccactcaa cttgtcttct gtcacgccat agttggctac 1800
   tctcaagcca gcacattgtt agacccatct taattaagcc ttacctccat gtaaagaaca 1860
   gcacgtctgt tccaaggacc tcagctcttc ttgtttctac agatggcaac agctccatag 1920
   ggacagcttg tataattacc ttcagaggcc aactgacaga atcctggcag gaacagacat 1980
   tatcttgcca gttagaagta cttctgtctc acttatgtcc aaagagtggc tatagatctt 2040
   ggccttcttc cctgaatgct tttttttttt ggcccccaag aaagtccctt ttatagcact 2100
   ttagcacagg caatgctaca ggaacaaagt ttcaatgctg ctgagagtga aagaaaggag 2160
   gaaagtctgc cactctaccc tgagctggca gtagggcact gagtaccctt aggaagaagt 2220
   cagagcaatg gatacaaatg accttgctct tggatttgct gagcatgatc cctattctga 2280
   tgtcagagat taggtttaaa tggaatagag ctatccattt gttcttactc tctagggaga 2340
   caatcttcca aaacagtttt gggggggtct tctaaagctt tcaaattgga agtaacttta 2400
   ttcaactaga gttgaataaa agaagggcaa aaataatctc acagagcttg gaactgctga 2460
   tagcccttac tgagggcaaa agatggctat attgttagct atactcctac caaagcaagc 2520
   aaggagatag gattatagat aatttcacgg acatttggaa ataacattgg tgattataca 2580
   gacaagaata aactcacttc aagctggtct gttttaataa attttcaacg taattgtcta 2640
   tttttttccc tcccatctgc aacagaatac atttttttca gcctttatct agatgaggta 2700
   aagggaatca ttcttatggt gctcttggag agtttcaggc ctgtgcatgt gtgtacagca 2760
   ggaggtaata tgctataatg tctgctgtaa tatatttgca cagtagatgc tatggatcat 2820
   tctgagctca gggtccagac tttattctta ttcccagaat tttgtgttac gtttttacct 2880
   cctaacatat gacacttcat cttatattaa ggaaggttta gaatatctaa tacgacttga 2940
   attcatttgt tactaagcct tctcaggcaa gctgtatact agttactggt ctccactgcc 3000
   atgccttttc aaggttccca tggtccagaa tgatgtttga ttcttaattt ttctgtccct 3060
   tttataattt gttttaatga ttttgctaca tttggaattc aataaaaaat gtgaacaata 3120
   ataaaaaaaa aaaaaaaaaa 3140
<210> 69
   <211> 3513
   <212> DNA
   <213> Homo sapiens
<400> 69
   ccgtgtacca ggtgctgcta gtgggaagca cgctgctgaa ggaagtgcct tccgggctgc 60
   agctggagca gttgccttct cagagcctgc tgacccacat cccaacggcg gggctgccca 120
   cttcgctagg aggaggcctg ccttactgcc accaggcctg gctggatttc cgaaggcggc 180
   tggaagctct actacagaac tgccaggcag cttgtgccct gctccagggg gccatcgaaa 240
   gtgtgaaggc tgtgccccag cccatggagc ctggggaggt cggtcagctg ctacagcaga 300
   cagaggtcct gatgcagcag gtgctagact cgccatggct ggcatggcta caatgccagg 360
   ggggccggga gctgacatgg ctgaagcaag aggtcccaga ggtgaccctg agcccagact 420
   acaggacggc aatggacaag gctgacgagc tatatgaccg ggtggatgga ttgctgcacc 480
   aactgaccct gcagagcaac cagcgaatac aggccctaga gttggtccaa acactggagg 540
   cccgggaaag cggactgcac cagattgaag tgtggctgca gcaggtgggc tggccagcac    600
   tggaggaggc tggggagccc tcgctggaca tgctgctcca ggcccaaggc tcttttcagg    660
   agctgtacca ggttgcccag gagcaggtca ggcaagggga gaagtttctg cagccgctga    720
   ctggctggga ggcggctgaa ctggaccccc ctggggcacg ctttctggcc ctgcgagccc    780
   agctgactga attctctagg gctttggccc agcggtgcca gcggctggcg gatgctgaga    840
   ggctgtttca gctcttcagg gaggccttga cgtgggctga ggaggggcag cgagtgttgg    900
   cagagctgga gcaggaacgc ccgggggttg tgttgcagca gctgcagctg cactggacca    960
   ggcaccctga cttgcctcct gcccacttcc gaaagatgtg ggctctggcc acggggctgg   1020
   gctcagaggc catccgccag gagtgccgct gggcctgggc gcggtgccag gacacctggc   1080
   tggccctgga ccaaaagctt gaggcttcac tgaagctacc accggtgggc agcacagcta   1140
   gcctgtgtgt cagccaggtc cccgctgcac ctgcccaccc tcccctgagg aaggcctaca   1200
   gcttcgatcg gaatctgggg cagagtctca gtgaacctgc ctgccactgc caccatgcgg   1260
   ccactattgc tgcctgccgc agaccagagg ctggaggagg tgccctgccc caggcatccc   1320
   ctactgtgcc tccaccaggc agctctgacc ccaggagcct caacaggcta cagctggtgc   1380
   tggcagagat ggtggccacg gagcgggagt atgtccgggc tctagagtac actatggaga   1440
   actatttccc cgagctggat cgccccgatg tgccccaggg cctccgcggt cagcgtgccc   1500
   acctctttgg caacctggag aagctgcggg acttccactg ccacttcttc ctgcgtgagc   1560
   tggaggcttg cacccggcac ccaccacgag tggcctatgc cttcctgcgc catagggtgc   1620
   agtttgggat gtacgcgctc tacagcaaga ataagcctcg ctccgatgcc ctgatgtcaa   1680
   gctatgggca caccttcttc aaggacaagc agcaagcact gggggaccac ctggacctgg   1740
   cctcctacct gctaaagccc atccagcgca tgggcaagta cgcactgctg ctgcaggagc   1800
   tggcacgggc ctgcgggggc cccacgcagg agctcagtgc gctgcgggag gcccagagcc   1860
   ttgtgcactt ccagctgcgg cacggaaacg acctgctggc catggacgcc atccagggct   1920
   gtgatgttaa cctcaaggaa caggggcagc tggtgcgaca ggatgagttt gtggtgcgca   1980
   ctgggcgcca caagtccgtg cgccgcatct tcctttttga ggagctgctg ctcttcagca   2040
   agcctcgcca tgggcccaca ggggttgaca catttgccta caagcgctcc ttcaagatgg   2100
   cagaccttgg tctcactgag tgctgtggga acagcaacct gcgcttcgag atctggttcc   2160
   gccgccgcaa ggccagggac acctttgtgc tgcaggcctc cagcctggct atcaagcagg   2220
   cctggacagc tgacatctcc cacctgcttt ggaggcaggc cgtccacaac aaggaggtgc   2280
   gcatggctga gatggtgtcc atgggtgtgg ggaacaaggc cttccgagac attgctccca   2340
   gcgaggaagc catcaacgac cgcaccgtca actatgtcct gaagtgccga gaagttcgct   2400
   ctcgggcgtc cattgccgta gccccgtttg accatgacag cctctacctg ggggcctcga   2460
   actcccttcc tggagaccct gcctcttgct ctgttctggg gtccctcaac ctgcacctgt   2520
   acagagaccc agctcttctg ggtctccgct gtcccctgta tcccagcttc ctagaggaag   2580
   cagcactgga ggctgaggca gagctgggcg gccagccctc tttgactgct gaggactcag   2640
   agatctcgtc ccaatgccca tcagccagtg gctccagtgg ctctgacagc agctgtgtgt   2700
   cagggcaggc cctgggtagg ggcctggagg acttaccctg tgtctgagcc cgggactgga   2760
   cgagcagtag atccagcagc ctgcagctcc aaggaacatt gcctctctgg atctgctgtg   2820
   accagggtgt ggctgacacc tgggctacct ccaacctaca tgtgcaacgc tgttgactac   2880
   cctttctgat gtgtgtggcc attggactaa ctggcacggg gcctctctag ggaagtctgg   2940
   ttgtagagcc tgaataggct cctggcccca tgaccccttc tcctgtcccc agctcccatc   3000
   ccagttgtgg gttaagaata ggctagagca gacattgggt gtttccatgc tgtaggctgg   3060
   tgggggacca tgtgcctcta ggcagtgact agggtgcccc cacccctcag gaagaacaca   3120
   ggtgggctcc tagcagctga tccccaatgc ctggccttaa agccgagctc agttaccata   3180
   gggacaggtc cacctctact gggccctcat gcttgccttt cctggccccc aggcccagcc   3240
   cctttttact ggggcagttt cgttattttg acttgatgcc ttttgaataa ctttcaatag   3300
   aattgtctaa aattatctta ctggttgtta ggcctttggt gtctcagaga aggagtctag   3360
   gtctttgatg tgtgatttaa tcttttattt gtttataata aaaaatagac tgatttgtaa   3420
   aaaaaaaaaa aaaaaaaaaa aaaaaaaaaa aaaaaaaaaa aaaaaaaaaa aaaaaaaaaa   3480
   aaaaaaaaaa aaaaaaaaaa aaaaaaaaaa aaa                                3513
<210> 70
   <211> 3597
   <212> DNA
   <213> Homo sapiens
<400> 70
   catgccagtt acttcctcag gaaaatattt tcttgccttc ttctttcagt atggttttaa 60
   atttgggaac agtggataac ccaagtgtcc cacaggccaa ggtatattcc aatggcagca 120
   tgatccctgc acccaaagcc agcccctaaa gcctacccct tgtgcacccg cagcctggta 180
   agtgagcttg gctgcttgtg aggagctaca agtgaaagag aagttatttt aaataaatcc 240
   caaagtttga ggcagactgt ccaggactgt tcccaggaag aagcaggagt tacccacagg 300
   aaaagtctct gacctggtcc cctcaggccc agctacctgc gcccaccagc agtgaaggtt 360
   gatgtactgg cccagcatct ccacctcccc catgcaacca ggtccctggt accgtgtctc 420
   ccgttgcatg tctggcttct gcctgtgctc ctcctgccac gagcatcctc cctgtccctc 480
   ctcattccac cgtgtctctc ctgcacacat agcctctgtc ccagggcgat ttatccactt 540
   gagtacagga gctgctcaga cctctcagcc cagccctctg tgactgcccc agccccatcc 600
   taccccaccc aaagctgcct tcctggctgt aggagctccc tcgtctagcc aaggccctat 660
   gggtccccat ccgaggatcc acaagcaatg acttcccaaa tgacctccac tgcaagaaga 720
   atccttacca ctgtttccag agccgtgaac gatgctgtga tggcccaggt ctcagcacca 780
   ccctctgtga cctaaaaaga aaagctcaat ttccatctgt cttctttccc aggaccaagg 840
   ggacacagta atgtgaagtc aaatacttaa ccgagcaaag ggccagtgtt gttatcagtc 900
   aaggacaaac ctcccacctc acagacagcc aagcagtgag ggaaagacag acagacatag 960
   gtaggaaggt gctctgcagg cacaaggccc agagaagccc ctctccggga acttcccctg 1020
   ctccttccag gaacagtgag cccagtgagc agtcccagcc agctcttcaa ggccttcaag 1080
   gggtctttcc atgactgagt cacctccagg agctcacctg acccccagag aagacctacc 1140
   ccaggcagct ccgtgccctg gcttctcccc atgccccaaa tcccccccag ccatccctcc 1200
   tggtcctcgt ctacatcaag ggcctcttcc cctcttcctc ccagctctca ggacaggtga 1260
   ctgggagacc ttgaaccctc agcctcttcc tttaaaaaaa acaaaacaaa acaaaactgt 1320
   gggccattta tttgggattt tggagttgtt tggtttttgt ttttatatct taatagttcg 1380
   aaagtaagaa gggagccctg ctatggatgt taagtccaaa ttactcggtt agtgggagca 1440
   aaacctatga cttccaaggg gatgaggaga ggttcagagg acaggaggag cctcccccat 1500
   tgaaaaaaaa aaaatgggtc aggacattcc ctggatgagg acaatgctag gggtggcatc 1560
   tcacatggct gctgctattc ctggtgcttc cccacacttt tgacagatgg agtccttctc 1620
   ctaccgcctc ctgccacctc accctacagg cattctctat gtaggaaaca agagccttat 1680
   cttatagagt ggggagctga gacacagcct caggtaacac tgacacagct cccgaatgag 1740
   gctgggacac tctgcaaacc tctcctcatg gtgctaaggg tggcatgctc ttgacaggaa 1800
   acctaaatga ccactcctct catttggaaa gtaatccact gcagtaaaag tttcagacat 1860
   gcaagagaga gttttttttt ttttttacta caaatttttg ctcccccata aaattatttt 1920
   tttattagag ggagtatcca agttttaaaa gtatatagaa ttttttggtt gtaagagaaa 1980
   tacatactca ttaggatccc gattaaattc cttgagtaga ctggtgccta ccagaaagca 2040
   aagcaaagtt aaacaaaacg aaacaaaatc cttcatatac aaaaagaact ttctgtttgt 2100
   attggcagag gtagtgaggt gattcaggta ggctgaaaat cctgggttgc gggagcctca 2160
   ctttattcca ttcccacccg ctttgatgtc tatgcttggc tctctgggct gcccctggta 2220
   ctgccgaatc ctacacatct cttatcagct ttcctcaaac tttaaggagg ctctgtgagg 2280
   gatgggtcat gggaagaccc aagctttccc tccgccagga ttgcaaaagc aagtagactt 2340
   ggtctatgca gctcttcttc caacaatttc tttatttgga attagaactt cctttgttag 2400
   tatctttgat cttttgactc aagcacattt tggaagggct cccttacaaa agtagaattt 2460
   aaaacagagg atacagttaa agagcaaccc aaaggacgct taagaaaccg agaccacttc 2520
   accgaacagg actaaggaac actttcgtgc acagaagtca gccgcaatcc aggcacagga 2580
   cgaagatggg atacacgtgc tcatctgtct gtcctccttt cctctccctc cccgacgttc 2640
   tagttagctt gttgacttgt taaaccttct gttcttaaaa tgaaaagcta gcttacctca 2700
   aagaatcttg tttccattcg gaaaccaacg attttgtgtt ttagaatgga cagccctccc 2760
   ctcaccactc cctaccttgg cctggtgtcc ttgagacata cggtctttgc ttagtcgtgt 2820
   gttggctgct ttgagcagga acgaggcctc caggccctga ggtgggaagg aaggattgga 2880
   tgccactgcc ctcctcccca ctttagcatg taggggccag cccatctctt ccagcagggt 2940
   cctgctgagt taccatagca accagcaact ccagggtacc acaacagaca atggctcagc 3000
   gagccgacgt gtggggatga tgcaggggtt ttggcccagc cagaggaccc agagttgagc 3060
   ttcaaatgct agagaagggg agaaacagga tggaagggtg gtttaaggaa ctggcagggg 3120
   tctttgagtc acatagagaa gccgttgaag gaggtagggc aggttatctc tgttccagtc 3180
   acccccttcc agccccatcc cacttctgtt tcaaactaaa gctcccacct cgaacattga 3240
   ccctttgtta gaacaaagca aagcatatct ttaaacaaca gtgttaaaat gagcctcaaa 3300
   tgtatgtgga tgagatctct aagaagaggg tcttctggtt ttgattttta aagaagagta 3360
   tcctagtaaa atattaaaaa aaaattaaaa agtttttaaa aaggaaacct atgctattta 3420
   aattggagcc cagttgtaac ttggtaaagg caagcttctg tacctttgtt ataattaatt 3480
   gtatacctgt gtatgtaaat ataaggcatt cctattttgc agttcagaac aaaaaaaact 3540
   tatttgtaat atagaataaa gtttattaaa aaataagaaa aaaaaaaaaa aaaaaaa 3597
<210> 71
   <211> 855
   <212> DNA
   <213> Homo sapiens
<400> 71
   cgctcaatta tctactcgag tctagactcg aggcggccgc ccattgtgca ctaaagcagg 60
   ggatagcaac ggcgtccctc ctccccgctc agctgcagcc cgcagtcctc acagtggtaa 120
   catgccacgt ggtagtctct gtccatggac accacacgga tggttgtctc gcagccctgt 180
   gcagggagga taggacgggc acaggaggcg cattttggtg caaaaaccgt gtgatagtct 240
   cgcacgcagt agatgttgtt ctccacgtcc acggtgaagg gaaccccgtc caggcactca 300
   ttgcacacgg agcaccggaa gcagcctggg tggtaggact tgcccagggc ctgcaggatc 360
   atttccatga tgagatgtcc acacacgctg catttgtcgg ccgtctgctg gaacccggag 420
   tacaggaagt cctcctggca gtacactttc tcacccacgt tgtagaacgc cttcccacgg 480
   agtcgtctcc cacacgagtc gcaggtgaag cagtcagtgt gataaagact ccccattgcc 540
   tggcacgcct gctgggctcc gtagatgcca agcccacact tgatgcaaat gccgaagtag 600
   tcccgcgccg tgcgcgcctc gagcgcccgc tccagctccc gggtgagcgc ctccagccgc 660
   cgctcggccg cgcttgggcc gccctcccgg ccagggggca gcgggagtgc aggcagcggg 720
   aagggggccg gccccgcagg ctccggggag cgagcgggcg cggggcaggc gccgggcggg 780
   aggaagtcag cgtaggcttt agtgcacaat gggaattcgg atccggtcga cactagttct 840
   aggatctctt ttttt 855
<210> 72
   <211> 3791
   <212> DNA
   <213> Homo sapiens
<400> 72
   acagacggcg ggtgaacatg gcgtcctcga cttggtctga gacgtgatag gcctgccttc      60
   tggttgaaga tgtggcgagt gaaaaaactg agcctcagcc tgtcgccttc gccccagacg     120
   ggaaaaccat ctatgagaac tcctctccgt gaacttaccc tgcagcccgg tgccctcacc     180
   acctctggaa aaagatcccc cgcttgctcc tcgctgaccc catcactgtg caagctgggg     240
   ctgcaggaag gcagcaacaa ctcgtctcca gtggattttg taaataacaa gaggacagac     300
   ttatcttcag aacatttcag tcattcctca aagtggctag aaacttgtca gcatgaatca     360
   gatgagcagc ctctagatcc aattccccaa attagctcta ctcctaaaac gtctgaggaa     420
   gcagtagacc cactgggcaa ttatatggtt aaaaccatcg tccttgtacc atctccactg     480
   gggcagcaac aagacatgat atttgaggcc cgtttagata ccatggcaga gacaaacagc     540
   atatctttaa atggaccttt gagaacagac gatctggtga gagaggaggt ggcaccctgc     600
   atgggagaca ggttttcaga agttgctgct gtatctgaga aacctatctt tcaggaatct     660
   ccgtcccatc tcttagagga gtctccacca aatccctgtt ctgaacaact acattgctcc     720
   aaggaaagcc tgagcagtag aactgaggct gtgcgtgagg acttagtacc ttctgaaagt     780
   aacgccttct tgccttcctc tgttctctgg ctttcccctt caactgcctt ggcagcagat     840
   ttccgtgtca atcatgtgga cccagaggag gaaattgtag agcatggagc tatggaggaa     900
   agagaaatga ggtttcccac acatcctaag gagtctgaaa cagaagatca agcacttgtc     960
   tcaagtgtgg aagatattct gtccacatgc ctgacaccaa atctagtaga aatggaatcc    1020
   caagaagctc caggcccagc agtagaagat gttggtagga ttcttggctc tgatacagag    1080
   tcttggatgt ccccactggc ctggctggaa aaaggtgtaa atacctccgt catgctggaa    1140
   aatctccgcc aaagcttatc ccttccctcg atgcttcggg atgctgcaat tggcactacc    1200
   cctttctcta cttgctcggt ggggacttgg tttactcctt cagcaccaca ggaaaagagt    1260
   acaaacacat cccagacagg cctggttggc accaagcaca gtacttctga gacagagcag    1320
   ctcctgtgtg gccggcctcc agatctgact gccttgtctc gacatgactt ggaagataac    1380
   ctgctgagct ctcttgtcat tgtggagttt ctctcccgcc agcttcggga ctggaagagc    1440
   cagctggctg tccctcaccc agaaacccag gacagtagca cacagactga cacatctcac    1500
   agtgggataa ctaataaact tcagcatctt aaggagagcc atgagatggg acaggcccta    1560
   cagcaggcca gaaatgtcat gcaatcatgg gtgcttatct ctaaagagct gatatccttg    1620
   cttcacctat ccctgttgca tttagaagaa gataagacta ctgtgaatca ggagtctcgg    1680
   cgtgcagaaa cattggtctg ttgctgtttt gatttgctga agaaattgag ggcaaagctc    1740
   cagagcctca aagcagaaag ggaggaggca aggcacagag aggaaatggc tctcagaggc    1800
   aaggatgcgg cagagatagt gttggaggct ttctgtgcac acgccagcca gcgcatcagc    1860
   cagctggaac aggacctagc atccatgcgg gaattcagag gccttctgaa ggatgcccag    1920
   acccaactgg tagggcttca tgccaagcaa gaagagctgg ttcagcagac agtgagtctt    1980
   acttctacct tgcaacaaga ctggaggtcc atgcaactgg attatacaac atggacagct    2040
   ttgctgagtc ggtcccgaca actcacagag aaactcacag tcaagagcca gcaagccctg    2100
   caggaacgtg atgtggcaat tgaggaaaag caggaggttt ctagggtgct ggaacaagtc    2160
   tctgcccagt tagaggagtg caaaggccaa acagaacaac tggagttgga aaacattcgt    2220
   ctagcaacag atctccgggc tcagttgcag attctggcca acatggacag ccagctaaaa    2280
   gagctacaga gtcagcatac ccattgtgcc caggacctgg ctatgaagga tgagttactc    2340
   tgccagctta cccagagcaa tgaggagcag gctgctcaat gcgtaaagga agagatggca    2400
   ctaaaacaca tgcaggcaga actgcagcag caacaagctg tcctggccaa agaggtgcgg    2460
   gacctgaaag agaccttgga gtttgcagac caggagaatc aggttgctca cctggagctg    2520
   ggtcaggttg agtgtcaatt gaaaaccaca ctggaagtgc tccgggagcg cagcttgcag    2580
   tgtgagaacc tcaaggacac tgtagagaac ctaacggcta aactggccag caccatagca    2640
   gataaccagg agcaagatct ggagaaaaca cggcagtact ctcaaaagct agggctgctg    2700
   actgagcaac tacagagcct gactctcttt ctacagacaa aactaaagga gaagactgaa    2760
   caagagaccc ttctgctgag tacagcctgt cctcccaccc aggaacaccc tctgcctaat    2820
   gacaggacct tcctgggaag catcttgaca gcagtggcag atgaagagcc agaatcaact    2880
   cctgtgccct tgcttggaag tgacaagagt gctttcaccc gagtagcatc aatggtttcc    2940
   cttcagcccg cagagacccc aggcatggag gagagcctgg cagaaatgag tattatgact    3000
   actgagcttc agagtctttg ttccctgcta caagagtcta aagaagaagc catcaggact    3060
   ctgcagcgaa aaatttgtga gctgcaagct aggctgcagg cccaggaaga acagcatcag    3120
   gaagtccaga aggcaaaaga agcagacata gagaagctga accaggcctt gtgcttgcgc    3180
   tacaagaatg aaaaggagct ccaggaagtg atacagcaga atgagaagat cctagaacag    3240
   atagacaaga gtggcgagct cataagcctt agagaggagg tgacccacct tacccgctca    3300
   cttcggcgtg cggagacaga gaccaaagtg ctccaggagg cctggcaggc cagctggact    3360
   ccaactgcca gcctatggcc accaattgga tccaggagaa agtgtggctc tctcaggagg    3420
   tggacaaact gagagtgatg ttcctggaga tgaaaaatga gaaggaaaac tcctgatcaa    3480
   gttccagagc ccatagaaat atcctagagg agaaccttcg gcgctctgac aaggagttag    3540
   aaaaactaga tgacattgtt cagcatattt ataagaccct gctctctatt ccagaggtgg    3600
   tgaggggatg caaagaacta cagggattgc tggaatttct gagctaagaa actgaaagcc    3660
   agaatttgtt tcacctcttt ttacctgcaa taccccctta ccccaatacc aagaccaact    3720
   ggcatagagc caactgagat aaatgctatt taaataaagt gtatttaatg aaaaaaaaaa    3780
   aaaaaaaaaa a                                                         3791
<210> 73
   <211> 1683
   <212> DNA
   <213> Homo sapiens
<400> 73
   ctctgagtgt ccagtggtca gttgccccag gatggggacc acagccagag cagccttggt      60
   cttgacctat ttggctgttg cttctgctgc ctctgaggga ggcttcacgg ctacaggaca     120
   gaggcagctg aggccagagc actttcaaga agttggctac gcagctcccc cctccccacc     180
   cctatcccga agcctcccca tggatcaccc tgactcctct cagcatggcc ctccctttga     240
   gggacagagt caagtgcagc cccctccctc tcaggaggcc acccctctcc aacaggaaaa     300
   gctgctacct gcccaactcc ctgctgaaaa ggaagtgggt ccccctctcc ctcaggaagc     360
   tgtccccctc caaaaagagc tgccctctct ccagcacccc aatgaacaga aggaaggaac     420
   gccagctcca tttggggacc agagccatcc agaacctgag tcctggaatg cagcccagca     480
   ctgccaacag gaccggtccc aagggggctg gggccaccgg ctggatggct tcccccctgg     540
   gcggccttct ccagacaatc tgaaccaaat ctgccttcct aaccgtcagc atgtggtata     600
   tggtccctgg aacctaccac agtccagcta ctcccacctc actcgccagg gtgagaccct     660
   caatttcctg gagattggat attcccgctg ctgccactgc cgcagccaca caaaccgcct     720
   agagtgtgcc aaacttgtgt gggaggaagc aatgagccga ttctgtgagg ccgagttctc     780
   ggtcaagacc cgaccccact ggtgctgcac gcggcagggg gaggctcggt tctcctgctt     840
   ccaggaggaa gctccccagc cacactacca gctccgggcc tgccccagcc atcagcctga     900
   tatttcctcg ggtcttgagc tgcctttccc tcctggggtg cccacattgg acaatatcaa     960
   gaacatctgc cacctgaggc gcttccgctc tgtgccacgc aacctgccag ctactgaccc    1020
   cctacaaagg gagctgctgg cactgatcca gctggagagg gagttccagc gctgctgccg    1080
   ccaggggaac aatcacacct gtacatggaa ggcctgggag gatacccttg acaaatactg    1140
   tgaccgggag tatgctgtga agacccacca ccacttgtgt tgccgccacc ctcccagccc    1200
   tactcgggat gagtgctttg cccgtcgggc tccttacccc aactatgacc gggacatctt    1260
   gaccattgac atcagtcgag tcacccccaa cctcatgggc cacctctgtg gaaaccaaag    1320
   agttctcacc aagcataaac atattcctgg gctgatccac aacatgactg cccgctgctg    1380
   tgacctgcca tttccagaac aggcctgctg tgcagaggag gagaaattaa ccttcatcaa    1440
   tgatctgtgt ggtccccgac gtaacatctg gcgagaccct gccctctgct gttacctgag    1500
   tcctggggat gaacaggtca actgcttcaa catcaattat ctgaggaacg tggctctagt    1560
   gtctggagac actgagaacg ccaagggcca gggggagcag ggctcaactg gaggaacaaa    1620
   tatcagctcc acctctgagc ccaaggaaga atgagtcacc ccagagccct agagggtcag    1680
   atg                                                                  1683
<210> 74
   <211> 1696
   <212> DNA
   <213> Homo sapiens
<400> 74
   cacctaaaag ccaaaatggg aaaggaaaag actcatatca acattgtcgt cattggacac      60
   gtagattcgg gcaagtccac cactactggc catctgatct ataaatgcgg tggcatcgac     120
   aaaagaacca ttgaaaaatt tgagaaggag gctgctgaga tgggaaaggg ctccttcaag     180
   tatgcctggg tcttggataa actgaaagct gagcgtgaac gtggtatcac cattgatatc     240
   tccttgtgga aatttgagac cagcaagtac tatgtgacta tcattgatgc cccaggacac     300
   agagacttta tcaaaaacat gattacaggg acatctcagg ctgactgtgc tgtcctgatt     360
   gttgctgctg gtgttggtga atttgaagct ggtatctcca agaatgggca gacccgagag     420
   catgcccttc tggcttacac actgggtgtg aaacaactaa ttgtcggtgt taacaaaatg     480
   gattccactg agccacccta cagccagaag agatatgagg aaattgttaa ggaagtcagc     540
   acttacatta agaaaattgg ctacaacccc gacacagtag catttgtgcc aatttctggt     600
   tggaatggtg acaacatgct ggagccaagt gctaacatgc cttggttcaa gggatggaaa     660
   gtcacccgta aggatggcaa tgccagtgga accacgctgc ttgaggctgt ggactgcatc     720
   ctaccaccaa ctcgtccaac tgacaagccc ttgcgcctgc ctctccagga tgtctacaaa 780
   attggtggta ttggtactgt tcctgttggc cgagtggaga ctggtgttct caaacccggt 840
   atggtggtca cctttgctcc agtcaacgtt acaacggaag taaaatctgt cgaaatgcac 900
   catgaagctt tgagtgaagc tcttcctggg gacaatgtgg gcttcaatgt caagaatgtg 960
   tctgtcaagg atgttcgtcg tggcaacgtt gctggtgaca gcaaaaatga cccaccaatg 1020
   gaagcagctg gcttcactgc tcaggtgatt atcctgaacc atccaggcca aataagcgcc 1080
   ggctatgccc ctgtattgga ttgccacacg gctcacattg catgcaagtt tgctgagctg 1140
   aaggaaaaga ttgatcgccg ttctggtaaa aagctggaag atggccctaa attcttgaag 1200
   tctggtgatg ctgccattgt tgatatggtt cctggcaagc ccatgtgtgt tgagagcttc 1260
   tcagactatc cacctttggg tcgctttgct gttcgtgata tgagacagac agttgcggtg 1320
   ggtgtcatca aagcagtgga caagaaggct gctggagctg gcaaggtcac caagtctgcc 1380
   cagaaagctc agaaggctaa atgaatatta tccctaatac ctgccacccc actcttaatc 1440
   agtggtggaa gaacggtctc agaactgttt gtttcaattg gccatttaag tttagtagta 1500
   aaagactggt taatgataac aatgcatcgt aaaaccttca gaaggaaagg agaatgtttt 1560
   gtggaccact ttggtttctt tttttgcgtg tggcagtttt aagttattag tttttaaaat 1620
   cagtactttt taatggaaac aacttgacca aaaatttgtc acagaatttt gagacccatt 1680
   aaaaaagtta aatgag 1696
<210> 75
   <211> 7680
   <212> DNA
   <213> Homo sapiens
<400> 75
<210> 76
   <211> 1316
   <212> DNA
   <213> Homo sapiens
<400> 76
   tcctaatacg actcactata gggctcgagc ggccgcccgg gcaggtcgaa tgcaggcgac      60
   ttgcgagctg ggagcgattt aaaacgcttt ggattccccc ggcctgggtg gggagagcga     120
   gctgggtgcc ccctagattc cccgcccccg cacctcatga gccgaccctc ggctccatgg     180
   agcccggcaa ttatgccacc ttggatggag ccaaggatat cgaaggcttg ctgggagcgg     240
   gaggggggcg gaatctggtc gcccactccc ctctgaccag ccacccagcg gcgcctacgc     300
   tgatgcctgc tgtcaactat gcccccttgg atctgccagg ctcggcggag ccgccaaagc      360
   aatgccaccc atgccctggg gtgccccagg ggacgtcccc agctcccgtg ccttatggtt     420
   actttggagg cgggtactac tcctgccgag tgtcccggag ctcgctgaaa ccctgtgccc     480
   aggcagccac cctggccgcg taccccgcgg agactcccac ggccggggaa gagtacccca     540
   gtcgccccac tgagtttgcc ttctatccgg gatatccggg aacctaccac gctatggcca     600
   gttacctgga cgtgtctgtg gtgcagactc tgggtgctcc tggagaaccg cgacatgact     660
   ccctgttgcc tgtggacagt taccagtctt gggctctcgc tggtggctgg aacagccaga     720
   tgtgttgcca gggagaacag aacccaccag gtcccttttg gaaggcagca tttgcagact      780
   ccagcgggca gcaccctcct gacgcctgcg cctttcgtcg cggccgcaag aaacgcattc     840
   cgtacagcaa ggggcagttg cgggagctgg agcgggagta tgcggctaac aagttcatca     900
   ccaaggacaa gaggcgcaag atctcggcag ccaccagcct ctcggagcgc cagattacca     960
   tctggtttca gaaccgccgg gtcaaagaga agaaggttct cgccaaggtg aagaacagcg    1020
   ctacccctta agagatctcc ttgcctgggt gggaggagcg aaagtggggg tgtcctgggg    1080
   agaccagaaa cctgccaagc ccaggctggg gccaaggact ctgctgagag gcccctagag    1140
   acaacaccct tcccaggcca ctggctgctg gactgttcct caggagcggc ctgggtaccc    1200
   agtatgtgca gggagacgga accccatgtg acaggcccac tccaccaggg ttcccaaaga    1260
   acctggccca gtcataatca ttcatcctca cagtggcaat aatcacgata accagt        1316
<210> 77
   <211> 566
   <212> DNA
   <213> Homo sapiens
<400> 77
   cccaccaaac ccataaagag ggtgggtcga cccacgcgtc cgcggacgcg tgggaaatta      60
   ttgaattgga aacagaaata gaaaagttta aagctgagaa cgcatcttta gctaaacttc     120
   gcattgaacg agaaagtgcc ttggaaaaac tcaggaaaga aattgcagac ttcgaacaac     180
   agaaagcaaa agaattagct cgaatagaag agtttaaaaa ggaggagatg aggaagctac     240
   aaaaggaacg taaagttttt gaaaagtata ctacagctgc aagaactttt ccagataaaa     300
   aggaacgtga agaaatacag actttaaaac agcaaatagc agatttacgg gaagatttga     360
   aaagaaagga gaccaaatgg tcaagtacac acagccgtct cagaagccag atacaaatgt     420
   tagtcagaga gaacacagac ctccgggaag aaataaaagt gatggaaaga ttccgactgg     480
   atgcctggaa gagagcagaa gccatagaga gcagcctcga ggtggagaag aaggacaagc     540
   ttgcgaacac atctgttcga tttcaa                                          566
<210> 78
   <211> 5067
   <212> DNA
   <213> Homo sapiens
<400> 78
   gcccggacac ctgtctgcag catggataag tatgacgacc tgggcctgga ggccagtaaa      60
   ttcatcgagg acctgaacat gtatgaggcc tctaaggatg ggctcttccg agtggacaag     120
   ggtgcaggca acaaccccga gtttgaggaa actcgcaggg tgttcgccac caagatggcc     180
   aaaatccacc tccagcagca gcagcagcag ctcctgcagg aggagactct gcccaggggg     240
   agtagaggcc ctgtcaatgg agggggccgc ctgggcccac aggcccgttg ggaagttgtg     300
   ggcagcaagc tgactgtgga tggtgctgcc aagcctcctc ttgctgcctc gacaggggca     360
   cctggggcag tcaccaccct cgctgctggg cagcccccgt acccaccgca ggagcagaga     420
   tccaggccat acctgcatgg cacgaggcat ggcagccagg actgtggttc cagggagagc     480
   ctggcgactt ctgagatgtc tgctttccac cagccaggcc cctgtgagga tccttcctgc     540
   ctcactcatg gagactatta tgacaacctc tccttggcaa gcccaaagtg gggtgacaaa     600
   ccaggagtgt cccccagcat cggcctgagt gtagggagtg ggtggcctag ctccccgggg     660
   agtgacccac cactgcccaa accctgcggg gaccatcccc taaatcaccg acagctctcc     720
   ctgagctcca gcaggtcttc tgagggtagc ctcggtggtc agaatagtgg cattggtggc     780
   cgcagcagcg agaagccaac aggcctttgg tccactgcct cctcccagcg ggtgagccct     840
   ggcctgcctt ccccaaactt ggagaacgga gcaccagctg tggggcctgt tcagcccagg     900
   actccttctg tgtcagcacc cttggccctg agctgcccca ggcaaggagg tcttccaaga     960
   tcaaactcgg ggctgggggg tgaggtttca ggtgtgatgt ccaaacccaa tgtggacccc    1020
   caaccctggt tccaggatgg gcccaaatct tacctttcca gttctgcccc gtcatcctcg    1080
   ccagctggcc tggacggttc acagcagggt gcggtccctg ggctggggcc gaagcctggc    1140
   tgcacagacc ttggcactgg tcccaagctc agccccacca gtcttgtcca tccagtgatg    1200
   tccaccctgc ctgagttatc ttgtaaagag ggtcccctgg gctggtcttc tgatggtagc    1260
   ctgggatctg tgctcctgga cagccccagc tcccctaggg taaggctgcc ctgccagccc    1320
   ctcgtcccag gtcctgagct gagaccctct gctgctgagt tgaaattaga agccctcacc    1380
   caacgtctgg agcgagagat ggatgctcac ccgaaggctg attactttgg agcctgtgtg    1440
   aaatgcagca aaggggtgtt tggggctggc caggcctgtc aggccatggg gaacctctac    1500
   catgacacat gcttcacctg tgcagcttgc agccggaagc tgagaggaaa agccttttat    1560
   tttgtcaacg gcaaagtgtt ttgtgaagaa gacttcctgt actctggttt ccagcagtcg    1620
   gctgacaggt gttttctttg tggacatctg atcatggaca tgatcctgca agccctgggg    1680
   aagtcctacc accccggctg tttccgctgt gtcatctgta atgagtgttt ggatggggtg    1740
   cccttcaccg tggactcaga gaacaagatc tactgtgtcc gagattacca caaggtgctg    1800
   gcccccaagt gtgcagcctg tgggcttccc atccttccac ctgagggctc agatgagacc    1860
   atccgtgtcg tgtccatgga cagagactac cacgtggagt gttaccactg cgaggactgt    1920
   ggtctggagc tcaatgatga agatggccac cgctgttatc cgctggagga ccacctgttc    1980
   tgtcactcct gccacgtgaa gaggctggag aagagaccct catctacagc ccttcaccag    2040
   caccacttct agccagagcc acttgcagac atcacggcag gggatgagga gccggggttg    2100
   ctgctgctgc ttccggtggc ccctggggtg gaagtggggt aggggaagag gaggggcagg    2160
   agggagagtt cctgtgagca tgtggggggt gcctttcctt taaccaggga ggtgaacact    2220
   acctgcctcc tgcgtgtatt ttccaagtgc ttttctctgt tgccacattt tcctcaggtt    2280
   actcaggaaa atgctccagc atgtgcgagc acatgacctg aggttgcatc atagcaccaa    2340
   aggaatcctc ctgtcccctc tgggaacatt tcatgcttca gagggagagg tttttattga    2400
   gcttgtttca caatatcccc ttgaagggac agctcagctg ccaatacatt caaccctttc    2460
   tcttccttca ggaaaatacc tatacccaaa tgttccctcc cccgacatat atcatggcat    2520
   gacttaaggc ttcttttcac ctgagagctt cagttcttct gcagaatggc tgcaaattta    2580
   attgcattaa ggcaagaagg aagctctaat gtgtgctttg tatcctaaga taaatttgct    2640
   tagaaaacca gagtcaagat ttgaaatagg tgaggcaggg tttcctcctt agacactgac    2700
   agcattctcc gtaccccttc aaatccttac tctcctaaag gcagctgagt ccgcgacaga    2760
   aatttgccct atgggagtaa aacatacttt gggagaagaa cttggtgcag gcaccaggat    2820
   tttttttttt gcccacgtgt ttgcgctgtt tttctctgga gttctcaaga gttggtgact    2880
   tggaaggccg cttctgcaag gcaagtctca ggaacccatg caggtacatc gcttgcacct    2940
   gtttttagct tatttaatga cgggcttttg ggaagagctg cccgcatact gagagacagc    3000
   ttcttataaa caaggagagt ttttgtgtgt gcgagatctc taagccagcg tgggagggag    3060
   cgcctcagga taagttatta tattcatttc gttggtttct ctcctgccca attcttggca    3120
   caggcattat gtttgaagaa accaggataa ggtacactgc ttttgtctgt ttaatttttt    3180
   tagttgtttc ccttcacttt cagtcttcca cacacaaaaa atacctcaca gagcttcacc    3240
   aaatcacaga ttcaggagga atttggcttt cacactggac tcagatacct tcttcagtgt    3300
   gttggaaatc actggcttca cacaggccca actccaactg gtcagggcag agtgatcgta    3360
   actaaaggtc agtggggaat agatccgatt cagtgctttt gccttatgca tttcagcatc    3420
   ctggctcccc agggtggcag gagctgagga agggccacac actggcaaga tttcaagacc    3480
   actctctgca ctgaagaggt aaaatttgca ctgcaagtca catccctgag gccagaggtc    3540
   agtacccttt ggtatttcga ttagaagaag ctgcaaaaga aaggcagccc attttaccat     3600
   tgccagccag gccggggaca caggagccgg tgtgtgcact ctgcctccta acattgcacc     3660
   cagagcaaga ggactgggtg ctgggctgca gaggccggtc agtggagccc ctagcacgtg     3720
   tgaactcagg cttttcattg ggcccggctc cacttctagg ccatgttttg actcatttgg     3780
   taaccattgc ctgtaagcag cacagaattg gtgccatgga ttatcttttc catgttgatg     3840
   gaattcattc tgttggaatc ctttggccag atgtcacttc agccagggtg tgcatcatca     3900
   ttggttcttt ttcacaggct gagcctcctg aaaacccatg aacgctgggg ctggggaagt     3960
   gaaccctgag gtggggaccc tctcttccca tcaaatcatc cagctcagtg tggggcgtgg     4020
   caggggggta aatgaagcca gccaatgtgt taacctgtct ctgtcaacct aagaatgttg     4080
   gccttactga cacacctttg ctccatgttc aagaccagaa gtagctggga tttgtttgca     4140
   aattgggtaa ttagtttaaa aatctgtgat tacattttta aatgaaattt tcaaagtggc     4200
   ctagattgag gtgattcaga taggtttgcg aatataccat tttatattgt tgagaaagaa     4260
   caaaaaggga atttccagat gtcctagaaa tcctagcaac agatttctct ggttgtcagt     4320
   ttccctggag aaggcgccag ataggaatct ccaatcagtt gtttttctct tcgcttcagg     4380
   cccttacaca aaagccatga agagatgttc acctacccgg tattttaaat gttctgtaaa     4440
   ttattagcca aatagaactg taatggggtt gtatttatgg gcgcctagaa agaaaacaca     4500
   aggacttggt aggccaggaa gaaaagattt taaaatttag aatgaatagc ccttctgggt     4560
   tttctttttg acaattcttg gacttgaggt aaaacaagga ggattgtggc cggatttcag     4620
   atcccaaagc cagcctccat cttaggcctt tgcctcattg tgccttttag gttttcttac     4680
   ccaccgtctc ctgttttgtc ttttttttct tttctcctac ccctatcttg ggacattcag     4740
   aaactgcctg ggtggtttga gaagagacaa cccagtttga tctgcaatac aaggatccat     4800
   tcgtaatctc tctctcactg atgttattcc cccatctgcc gtcttggttc atctcaccac     4860
   agaagggcat ttagtcctac ccagccatcg gctgcggtat gacagcagga tggcacttcc     4920
   catttctctg tggttagtgc tcgagtgaaa acctctttca gctgagtcct ctgaggttct     4980
   gctgttgagt cctgggtggc tgatggaatg attgaggagg tctggtcacc ctcaagcgcc     5040
   gtcatcgcct tgtttccatg ggcttct                                          5067
<210> 79
   <211> 950
   <212> DNA
   <213> Homo sapiens
<400> 79
   tcgaccggat ccgaattccc attgtgcact aaagcgtctc cctgctccgc ggcccgggct       60
   ggcgggcggg cgctcggctg gcggctgcag cagcagaggg agacccgcgg caaccccggc      120
   aacccagggc tcggcgtcgc tgccaccatg acgggaagca atatgtcgga cgccttggcc      180
   aacgccgtgt gccagcgctg ccaggcccgc ttctcccccg ccgagcgcat tgtcaacagc      240
   aatggggagc tgtaccatga gcactgcttc gtgtgtgccc agtgcttccg gcccttcccc      300
   gaggggctct tctatgagtt tgaaggccgg aagtactgcg aacacgactt ccaaatgctg      360
   tttgctccgt gctgtggatc ctgcggtgag ttcatcattg gccgcgtcat caaggccatg      420
   aacaacaact ggcacccggg ctgcttccgc tgcgagctgt gtgatgtgga gctggctgac      480
   ctgggctttg tgaagaatgc cggcaggcat ctctgccggc cttgccacaa ccgtgagaag      540
   gccaagggcc tgggcaagta catctgccag cggtgccacc tggtcatcga cgagcagccc      600
   ctcatgttca ggagcgacgc ctaccaccct gaccacttca actgcaccca ctgtgggaag      660
   gagctgacag ccgaggcccg cgagctgaag ggtgagctct actgcctgcc ctgccatgac      720
   aagatgggcg ttcccatctg cggggcctgc cgccggccca tcgagggccg agtggtcaac      780
   gcgctgggca agcagtggca cgtggagcac tttgtctgtg ccaagtgtga gaagccattc      840
   ctggggcacc ggcactatga gaagaagggc ctggcctact gcgagcttta gtgcacaatg      900
   ggcggccgcc tcgagtctag actcgagtag ataattgagc ggaatttctt                 950
<210> 80
   <211> 2346
   <212> DNA
   <213> Homo sapiens
<400> 80
   ccgccgtcgc ccccgcctcc ccctgcctca gcggctgccc ccgccagcgg gccgcccgct      60
   cccccgggcc ttgcagcggg ccccggcccg gctggagggg ccccgacccc agctctggtg     120
   gcgggcagca gcgccgcggc ccccttccct cacggggact cggccctgaa cgagcaggag     180
   aaggagttgc agcggcggct gaagcgtctc tacccggccg tggacgaaca agagacgccg     240
   ctgcctcggt cctggagccc gaaggacaag ttcagctaca tcggcctctc tcagaacaac     300
   ctgcgggtgc actacaaagg tcatggcaaa accccaaaag atgccgcgtc agttcgagcc     360
   acgcatccaa taccagcagc ctgtgggatt tattattttg aagtaaaaat tgtcagtaag      420
   ggaagagatg gttacatggg aattggtctt tctgctcaag gtgtgaacat gaatagacta     480
   ccaggttggg ataagcattc atatggttac catggggatg atggacattc gttttgttct     540
   tctggaactg gacaacctta tggaccaact ttcactactg gtgatgtcat tggctgttgt     600
   gttaatctta tcaacaatac ctgcttttac accaagaatg gacatagttt aggtattgct     660
   ttcactgacc taccgccaaa tttgtatcct actgtggggc ttcaaacacc aggagaagtg     720
   gtcgatgcca attttgggca acatcctttc gtgtttgata tagaagacta tatgcgggag     780
   tggagaacca aaatccaggc acagatagat cgatttccta tcggagatcg agaaggagaa     840
   tggcagacca tgatacaaaa aatggtttca tcttatttag tccaccatgg gtactgtgcc     900
   acagcagagg cctttgccag atctacagac cagaccgttc tagaagaatt agcttccatt     960
   aagaatagac aaagaattca gaaattggta ttagcaggaa gaatgggaga agccattgaa    1020
   acaacacaac agttataccc aagtttactt gaaagaaatc ctaatctcct tttcacatta    1080
   aaagtgcgtc agtttataga aatggtgaat ggtacagata gtgaagtacg atgtttggga    1140
   ggccgaagtc caaagtctca agacagttat cctgttagtc ctcgaccttt tagtagtcca    1200
   agtatgagcc ccagccatgg aatgaatatc cacaatttag catcaggcaa aggaagcacc    1260
   gcacattttt caggttttga aagttgtagt aatggtgtaa tatcaaataa agcacatcaa    1320
   tcatattgcc atagtaataa acaccagtca tccaacttga atgtaccaga actaaacagt    1380
   ataaatatgt caagatcaca gcaagttaat aacttcacca gtaatgatgt agacatggaa    1440
   acagatcact actccaatgg agttggagaa acttcatcca atggtttcct aaatggtagc    1500
   tctaaacatg accacgaaat ggaagattgt gacaccgaaa tggaagttga ttcaagtcag    1560
   ttgagacgcc agttgtgtgg aggaagtcag gccgccatag aaagaatgat ccactttgga    1620
   cgagagctgc aagcaatgag tgaacagcta aggagagact gtggcaagaa cactgcaaac    1680
   aaaaaaatgt tgaaggatgc attcagtcta ctagcatatt cagatccctg gaacagccca    1740
   gttggaaatc agcttgaccc gattcagaga gaacctgtgt gctcagctct taacagtgca    1800
   atattagaaa cccacaatct gccaaagcaa cctccacttg ccctagcaat gggacaggcc    1860
   acacaatgtc taggactgat ggctcgatca ggaattggat cctgcgcatt tgccacagtg    1920
   gaagactacc tacattagct atgcatttca agagctcaca cttatattgt ggcatatagt    1980
   caacatggaa gtagaccagc tctgctgatt tgaaatttag attttttaaa ttatgtactg     2040
   gggacaggtt tttgtcgctt tacattgctt cctagtttac agcatgatgc aaatgatttt    2100
   ctaacttagt gttaggagaa attattttcc atctttaacc tcttagttgt ctaagagtta    2160
   aatattactg aatttcagac gttcaaattg atcatcacaa atcctttaaa acaattacct    2220
   aaaagaaacc aaaaatcctg ccttctttgt gggggagggg ggagagaggg gaaggaaatg    2280
   gaacaagttg tgtttgtgtt agcatgtggg tgatgtaaac ttcaaattgg gagatgttcc    2340
   gacccc                                                              2346
<210> 81
   <211> 2512
   <212> DNA
   <213> Homo sapiens
<400> 81
   caatgcactg acggatatga gtgggatcct gtgagacagc aatgcaaaga tattgatgaa      60
   tgtgacattg tcccagacgc ttgtaaaggt ggaatgaagt gtgtcaacca ctatggagga     120
   tacctctgcc ttccgaaaac agcccagatt attgtcaata atgaacagcc tcagcaggaa     180
   acacaaccag cagaaggaac ctcaggggca accaccgggg ttgtagctgc cagcagcatg     240
   gcaaccagtg gagtgttgcc cgggggtggt tttgtggcca gtgctgctgc agtcgcaggc     300
   cctgaaatgc agactggccg aaataacttt gtcatccggc ggaacccagc tgaccctcag     360
   cgcattccct ccaacccttc ccaccgtatc cagtgtgcag caggctacga gcaaagtgaa     420
   cacaacgtgt gccaagacat agacgagtgc actgcaggga cgcacaactg tagagcagac     480
   caagtgtgca tcaatttacg ggatcctttt gcatgtcagt gccctcctgg atatcagaag     540
   cgaggggagc agtgcgtaga catagatgaa tgtaccatcc ctccatattg ccaccaaaga     600
   tgcgtgaata caccaggctc attttattgc cagtgcagtc ctgggtttca attggcagca     660
   aacaactata cctgcgtaga tataaatgaa tgtgatgcca gcaatcaatg tgctcagcag     720
   tgctacaaca ttcttggttc attcatctgt cagtgcaatc aaggatatga gctaagcagt     780
   gacaggctca actgtgaaga cattgatgaa tgcagaacct caagctacct gtgtcaatat     840
   caatgtgtca atgaacctgg gaaattctca tgtatgtgcc cccagggata ccaagtggtg     900
   agaagtagaa catgtcaaga tataaatgag tgtgagacca caaatgaatg ccgggaggat     960
   gaaatgtgtt ggaattatca tggcggcttc cgttgttatc cacgaaatcc ttgtcaagat    1020
   ccctacattc taacaccaga gaaccgatgt gtttgcccag tctcaaatgc catgtgccga    1080
   gaactgcccc agtcaatagt ctacaaatac atgagcatcc gatctgatag gtctgtgcca    1140
   tcagacatct tccagataca ggccacaact atttatgcca acaccatcaa tacttttcgg    1200
   attaaatctg gaaatgaaaa tggagagttc tacctacgac aaacaagtcc tgtaagtgca    1260
   atgcttgtgc tcgtgaagtc attatcagga ccaagagaac atatcgtgga cctggagatg    1320
   ctgacagtca gcagtatagg gaccttccgc acaagctctg tgttaagatt gacaataata    1380
   gtggggccat tttcatttta gtcttttcta agagtcaacc acaggcattt aagtcagcca    1440
   aagaatattg ttaccttaaa gcactatttt atttatagat atatctagtg catctacatc    1500
   tctatactgt acactcaccc ataacaaaca attacaccat ggtataaagt gggcatttaa    1560
   tatgtaaaga ttcaaagttt gtctttatta ctatatgtaa attagacatt aatccactaa    1620
   actggtcttc ttcaagagag ctaagtatac actatctggt gaaacttgga ttctttccta    1680
   taaaagtggg accaagcaat gatgatcttc tgtggtgctt aaggaaactt actagagctc    1740
   cactaacagt ctcataagga ggcagccatc ataaccattg aatagcatgc aagggtaaga    1800
   atgagttttt aactgctttg taagaaaatg gaaaaggtca ataaagatat atttctttag    1860
   aaaatgggga tctgccatat ttgtgttggt ttttattttc atatccagcc taaaggtggt    1920
   tgtttattat atagtaataa atcattgctg tacaacatgc tggtttctgt agggtatttt    1980
   taattttgtc agaaatttta gattgtgaat attttgtaaa aaacagtaag caaaattttc    2040
   cagaattccc aaaatgaacc agataccccc tagaaaatta tactattgag aaatctatgg    2100
   ggaggatatg agaaaataaa ttccttctaa accacattgg aactgacctg aagaagcaaa    2160
   ctcggaaaat ataataacat ccctgaattc aggcattcac aagatgcaga acaaaatgga    2220
   taaaaggtat ttcactggag aagttttaat ttctaagtaa aatttaaatc ctaacacttc    2280
   actaatttat aactaaaatt tctcatcttc gtacttgatg ctcacagagg aagaaaatga    2340
   tgatggtttt tattcctggc atccagagtg acagtgaact taagcaaatt accctcctac    2400
   ccaattctat ggaatatttt atacgtctcc ttgtttaaaa tctgactgct ttactttgat    2460
   gtatcatatt tttaaataaa aataaatatt cctttagaag atcactctaa aa            2512
<210> 82
   <211> 2306
   <212> DNA
   <213> Homo sapiens
<400> 82
   gggcgggagc tgcacgcgcc gtggctccgg atctcttcgt ctttgcagcg tacgcccgag      60
   tcggtcagcg ccggaggacc tcagcagcca tgtcgaagcc ccatagtgaa gccgggactg     120
   ccttcattca gacccagcag ctgcacgcag ccatggctga cacattcctg gagcacatgt     180
   gccgcctgga cattgattca ccacccatca cagcccggaa cactggcatc atctgtacca     240
   ttggcccagc ttcccgatca gtggagacgt tgaaggagat gattaagtct ggaatgaatg     300
   tggctcgtct gaacttctct catggaactc atgagtacca tgcggagacc atcaagaatg     360
   tgcgcacagc cacggaaagc tttgcttctg acccctacct ctaccggccc gttgctgtgg     420
   ctctagacac taaaggacct gagatccgaa ctgggctcat caagggcagc ggcactgcag     480
   agctggagct gaagaaggga gccactctca aaatcacgct ggataacgcc tacatggaaa     540
   agtgtgacga gaacatcctg tggctggact acaagaacat ctgcaaggtg gtggaagtgg     600
   gcagcaagat ctacgtggat gatgggctta tttctctcca ggtgaagcag aaaggtgccg     660
   acttcctggt gacggaggtg gaaaatggtg gctccttggg cagcaagaag ggtgtgaacc     720
   ttcctggggc tgctgtggac ttgcctgctg tgtcggagaa ggacatccag gatctgaagt     780
   ttggggtcga gcaggatgtt gatatggtgt ttgcgtcatt catccgcaag gcatctgatg     840
   tccatgaagt taggaaggtc ctgggagaga agggaaagaa catcaagatt atcagcaaaa     900
   tcgagaatca tgagggggtt cggaggtttg atgaaatcct ggaggccagt gatgggatca   960
   tggtggctcg tggtgatcta ggcattgaga ttcctgcaga gaaggtcttc cttgctcaga    1020
   agatgatgat tggacggtgc aaccgagctg ggaagcctgt catctgtgct actcagatgc    1080
   tggagagcat gatcaagaag ccccgcccca ctcgggctga aggcagtgat gtggccaatg    1140
   cagtcctgga tggagccgac tgcatcatgc tgtctggaga aacagccaaa ggggactatc    1200
   ctctggaggc tgtgcgcatg cagcacctga ttgcccgtga ggcagaggct gccatctacc    1260
   acttgcaatt atttgaggaa ctccgccgcc tggcgcccat taccagcgac cccacagaag    1320
   ccaccgccgt gggtgccgtg gaggcctcct tcaagtgctg cagtggggcc ataatcgtcc    1380
   tcaccaagtc tggcaggtct gctcaccagg tggccagata ccgcccacgt gcccccatca    1440
   ttgctgtgac ccggaatccc cagacagctc gtcaggccca cctgtaccgt ggcatcttcc    1500
   ctgtgctgtg caaggaccca gtccaggagg cctgggctga ggacgtggac ctccgggtga    1560
   actttgccat gaatgttggc aaggcccgag gcttcttcaa gaagggagat gtggtcattg    1620
   tgctgaccgg atggcgccct ggctccggct tcaccaacac catgcgtgtt gttcctgtgc    1680
   cgtgatggac cccagagccc ctcctccagc ccctgtccca cccccttccc ccagcccatc     1740
   cattaggcca gcaacgcttg tagaactcac tctgggctgt aacgtggcac tggtaggttg    1800
   ggacaccagg gaagaagatc aacgcctcac tgaaacatgg ctgtgtttgc agcctgctct    1860
   agtgggacag cccagagcct ggctgcccat catgtggccc cacccaatca agggaagaag    1920
   gaggaatgct ggactggagg cccctggagc cagatggcaa gagggtgaca gcttcctttc    1980
   ctgtgtgtac tctgtccagt tcctttagaa aaaatggatg cccagaggac tcccaaccct    2040
   ggcttggggt caagaaacag ccagcaagag ttaggggcct tagggcactg ggctgttgtt    2100
   ccattgaagc cgactctggc cctggccctt acttgcttct ctagctctct aggcctctcc    2160
   agtttgcacc tgtccccacc ctccactcag ctgtcctgca gcaaacactc caccctccac    2220
   cttccatttt cccccactac tgcagcacct ccaggcctgt tgctatagag cctacctgta    2280
   tgtcaataaa caacagctga agcacc                                          2306
<210> 83
   <211> 2656
   <212> DNA
   <213> Homo sapiens
<400> 83
   gaattcgcgg ccgcagagtc cccgggccaa gatggctgcg cggtgctcca cacgctggtt      60
   gctggtggtt gtggggaccc cgcggctgcc ggctatatcg ggtagagggg cccggccgcc     120
   cagggagggc gtggtggggg catggctgag ccgcaagctg agcgtccccg cctttgcgtc     180
   ttccctgacc tcttgcggcc cccgagcgct gctgacattg agacctggtg tcagccttac     240
   aggaacaaaa cataaccctt tcatttgtac tgcctccttc cacacgagtg cccctttggc    300
   caaagaagat tattatcaga tattaggagt gcctcgaaat gccagccaga aagagatcaa     360
   gaaagcctat tatcagcttg ccaagaagta tcaccctgac acaaataagg atgatcccaa     420
   agccaaggag aagttctccc agctggcaga agcctatgag gttttgagtg atgaggtgaa     480
   gaggaagcag tacgatgcct acggctctgc aggcttcgat cctggggcca gcggctccca     540
   gcatagctac tggaagggag gccccactgt ggaccccgag gagctgttca ggaagatctt     600
   tggcgagttc tcatcctctt catttggaga tttccagacc gtgtttgatc agcctcagga     660
   atacttcatg gagttgacat tcaatcaagc tgcaaagggg gtcaacaagg agttcaccgt     720
   gaacatcatg gacacgtgtg agcgctgcaa cggcaagggg aacgagcccg gcaccaaggt     780
   gcagcattgc cactactgtg gcggctccgg catggaaacc atcaacacag gcccttttgt     840
   gatgcgttcc acgtgtagga gatgtggtgg ccgcggctcc atcatcatat cgccctgtgt     900
   ggtctgcagg ggagcaggac aagccaagca gaaaaagcga gtgatgatcc ctgtgcctgc     960
   aggagtcgag gatggccaga ccgtgaggat gcctgtggga aaaagggaaa ttttcattac    1020
   gttcagggtg cagaaaagcc ctgtgttccg gagggacggc gcagacatcc actccgacct    1080
   ctttatttct atagctcagg ctcttcttgg gggaacagcc agagcccagg gcctgtacga    1140
   gacgatcaac gtgacgatcc cccctgggac tcagacagac cagaagattc ggatgggtgg    1200
   gaaaggcatc ccccggatta acagctacgg ctacggagac cactacatcc acatcaagat    1260
   acgagttcca aagaggctaa cgagccggca gcagagcctg atcctgagct acgccgagga    1320
   cgagacagat gtggagggga cggtgaacgg cgtcaccctc accagctctg gtggcagcac    1380
   catggatagc tccgcaggaa gcaaggctag gcgtgaggct ggggaggacg aggagggatt    1440
   cctttccaaa cttaagaaaa tgtttacctc atgatatccc agccgaggaa aaagatccac    1500
   tggaaactag gccgggaagc agcagcccct ccaagggcca gggcacctgg gagacgggag    1560
   gattccagaa cagcagcact gagctcccac ccgcagagcc tctggacggc cttggcaaca    1620
   gcaaaatcat gggacaacac ctctctccac ggaaaggtca cagtggacag cccgggcagt    1680
   aggatgcagc cccagaggct ggtggcagtt tcctgtccat tggtaggtga cggccccctg    1740
   gtcagcagag gagaggttag atcttgcagg ctaaaactct aatttggaat tgaatattgt    1800
   ggatatctta gttaaaggcc atgcttacag cttagaaatg aagccttaag ctgcatcaag    1860
   ttacgaagtg attaatttcc ttctcagcaa acctccggga ggttccagaa tgagttcttc    1920
   ctgacaggtt gtcttcactg ggagcgtggg gcccccaggc cccaccagca ccgtcctccc    1980
   ctaatgaggg gccctgccga ggcatcagct gctctgctca gttagttttt attcccgggg    2040
   taccaagcag ctgcacagtc ggtgcctggg aagcacgtta aaggcccaga gagatcctgg    2100
   gggttctgct ctgaccgtgt gggtggtgat ccttgtcagg atgtacagtc cttgctccca    2160
   ccccatccgg gatggccgcc tgtccctgac tattgagtcc tgttgttgta agccaggcat    2220
   ggagggctcc tgcccttctg ctgagccaca gcccattgca gcactgtgct ggccagactt    2280
   cagctgcctt gggaactgaa gccctgccac tgttgctagt caggggcttg gttctcccac    2340
   ttacactgtt gacatctatt ttctgaagtg tgtttaaatt attcagtgct aatcattgtt    2400
   ttttcctttg taaatgttga ttcagaaaag gaaagcacag gctaagcagt tgaaggttcc    2460
   ccaccattca gtgagagcag aacccccatt ccccagcctc tgctggtagc atgtcgcagt   2520
   ttccatgtgt ttcaggatct tcgggctgtc gttagacagg ttaatgaaga acacttctca    2580
   acagtttcct ttttgttttc ctttataatt cactaaaata aagcatctat tagtgtctga    2640
   aaaaaaaaaa aaaaaa                                                    2656
<210> 84
   <211> 2217
   <212> DNA
   <213> Homo sapiens
<400> 84
   gcggacccgg cgccgaggcg gccacccgag acgcggcgcg cacgctccgg cctgcgcagc      60
   ccggcccggc catggcggcc ccccgcccgt ctcccgcgat ctccgtttcg gtctcggctc     120
   cggcttttta cgccccgcag aagaagttcg gccctgtggt ggccccaaag cccaaagtga     180
   atcccttccg gcccggggac agcgagcctc ccccggcacc cggggcccag cgcgcacaga     240
   tgggccgggt gggcgagatt cccccgccgc ccccggaaga ctttcccctg cctccacctc     300
   cccttgctgg ggatggcgac gatgcagagg gtgctctggg aggtgccttc ccgccgcccc     360
   ctcccccgat cgaggaatca tttccccctg cgcctctgga ggaggagatc ttcccttccc     420
   cgccgcctcc tccggaggag gagggagggc ctgaggcccc cataccgccc ccaccacagc     480
   ccagggagaa ggtgagcagt attgatttgg agatcgactc tctgtcctca ctgctggatg     540
   acatgaccaa gaatgatcct ttcaaagccc gggtgtcatc tggatatgtg cccccaccag     600
   tggccactcc attcagttcc aagtccagta ccaagcctgc agccgggggc acagcacccc     660
   tgcctccttg gaagtcccct tccagctccc agcctctgcc ccaggttccg gctccggctc     720
   agagccagac acagttccat gttcagcccc agccccagcc caagcctcag gtccaactcc     780
   atgtccagtc ccagacccag cctgtgtctt tggctaacac ccagccccga gggcccccag     840
   cctcatctcc ggctccagcc cctaagtttt ctccagtgac tcctaagttt actcctgtgg     900
   cttccaagtt cagtcctgga gccccaggtg gatctgggtc acaaccaaat caaaaattgg    960
   ggcaccccga agctctttct gctggcacag gctcccctca acctcccagc ttcacctatg   1020
   cccagcagag ggagaagccc cgagtgcagg agaagcagca ccccgtgccc ccaccggctc   1080
   agaaccaaaa ccaggtgcgc tcccctgggg ccccagggcc cctgactctg aaggaggtgg   1140
   aggagctgga gcagctgacc cagcagctaa tgcaggacat ggagcatcct cagaggcaga   1200
   atgtggctgt caacgaactc tgcggccgat gccatcaacc cctggcccgg gcgcagccag   1260
   ccgtccgcgc tctagggcag ctgttccaca tcgcctgctt cacctgccac cagtgtgcgc   1320
   agcagctcca gggccagcag ttctacagtc tggagggggc gccgtactgc gagggctgtt   1380
   acactgacac cctggagaag tgtaacacct gcggggagcc catcactgac cgcatgctga   1440
   gggccacggg caaggcctat cacccgcact gcttcacctg tgtggtctgc gcccgccccc   1500
   tggagggcac ctccttcatc gtggaccagg ccaaccggcc ccactgtgtc cccgactacc   1560
   acaagcagta cgccccgagg tgctccgtct gctctgagcc catcatgcct gagcctggcc   1620
   gagatgagac tgtgcgagtg gtcgccctgg acaagaactt ccacatgaag tgttacaagt   1680
   gtgaggactg cgggaagccc ctgtcgattg aggcagatga caatggctgc ttccccctgg  1740
   acggtcacgt gctctgtcgg aagtgccaca ctgctagagc ccagacctga gtgaggacag   1800
   gccctcttca gaccgcagtc catgccccat tgtggaccac ccacactgag accacctgcc   1860
   cccacctcag ttattgtttt gatgtctagc ccctcccatt tccaacccct ccctagcatc   1920
   ccaggtgccc tgacccagga cccaacatgg tctagggatg caggatcccc gccctggggt   1980
   ctggtcctcg cccatcctgc agggattgcc caccgtcttc cagacacccc acctgagggg   2040
   ggcaccaggt ttagtgctgc tgctttcact gctgcacccg cgccctcggc cggccccccg    2100
   agcagccttt gtactctgct tgcggagggc tgggagaccc tccaggacat tcccaccctc   2160
   ccccatgctg ccaagttgta gctatagcta caaataaaaa aaaaccttgt tttccag      2217
<210> 85
   <211> 8906
   <212> DNA
   <213> Homo sapiens
<400> 85
   gaggcggcca aggacctggc cgacatcgcg gccttcttcc gatccgggtt tcgaaaaaac     60
   gatgaaatga aagctatgga tgttttacca attttgaagg aaaaagttgc atacctttca   120
   ggtgggagag ataaacgtgg aggtcccatt ttaacgtttc cggcccgcag caatcatgac    180
   agaatacgac aggaggatct caggagactc atttcctatc tagcctgtat tcccagcgag    240
   gaggtctgca agcgtggctt cacggtgatc gtggacatgc gtgggtccaa gtgggactcc    300
   atcaagcccc ttctgaagat cctgcaggag tccttcccct gctgcatcca tgtggccctg    360
   atcatcaagc cagacaactt ctggcagaaa cagaggacta attttggcag ttctaaattt    420
   gaatttgaga caaatatggt ctctttagaa ggccttacca aagtagttga tccttctcag    480
   ctaactcctg agtttgatgg ctgcctggaa tacaaccacg aagaatggat tgaaatcaga    540
   gttgcttttg aagactacat tagcaatgcc acccacatgc tgtctcggct ggaggaactt    600
   caggacatcc tagctaagaa ggagctgcct caggatttag agggggctcg gaatatgatc    660
   gaggaacatt ctcagctgaa gaagaaggtg attaaggccc ccatcgagga cctggatttg    720
   gagggacaga agctgcttca gaggatacag agcagtgaaa gctttcccaa aaagaactca    780
   ggctcaggca atgcggacct gcagaacctc ttgcccaagg tgtccaccat gctggaccgg    840
   ctgcactcga cacggcagca tctgcaccag atgtggcatg tgaggaagct gaagctggac    900
   cagtgcttcc agctgaggct gtttgaacag gatgctgaga agatgtttga ctggatcaca    960
   cacaacaaag gcctgtttct aaacagctac acagagattg ggaccagcca ccctcatgcc   1020
   atggagcttc agacgcagca caatcacttt gccatgaact gtatgaacgt gtatgtaaat   1080
   ataaaccgca tcatgtcggt ggccaatcgt ctggtggagt ctggccacta tgcctcgcag   1140
   cagatcaggc agatcgcgag tcagctggag caggagtgga aggcgtttgc ggcagccctg   1200
   gatgagcgga gcaccttgct ggacatgtcc tccattttcc accagaaggc cgaaaagtat   1260
   atgagcaacg tggattcatg gtgtaaagct tgcggtgagg tagaccttcc ctcagagctg   1320
   caggacctag aagatgccat tcatcaccac cagggaatat atgaacatat cactcttgct   1380
   tattctgagg tcagccaaga tgggaagtcg ctccttgaca agctccagcg gcccttgact   1440
   cccggcagct ccgattccct gacagcctct gccaactact ccaaggccgt gcaccatgtc 1500
   ctggatgtca tccacgaggt gctgcaccac cagcggcacg tgagaacaat ctggcaacac 1560
   cgcaaggtcc ggctgcatca gaggctgcag ctgtgtgttt tccagcagga agttcagcag 1620
   gtgctagact ggatcgagaa ccacggagaa gcatttctga gcaaacatac aggtgtgggg 1680
   aaatctcttc atcgggccag agcattgcag aaacgtcatg aagattttga agaagtggca 1740
   cagaacacat acaccaatgc ggataaatta ctggaagcag cagaacagct ggctcagact 1800
   ggggaatgtg accccgaaga gatttatcag gctgcccatc agctggaaga ccggattcaa 1860
   gatttcgttc ggcgtgttga gcagcgaaag atcctactgg acatgtcagt gtcctttcac 1920
   acccatgtga aagagctgtg gacgtggctg gaggagctgc agaaggagct gctggacgac 1980
   gtgtatgccg agtcggtgga ggccgtgcag gacctcatca agcgctttgg ccagcagcag 2040
   cagaccaccc tgcaggtgac tgtcaacgtg atcaaggaag gggaggacct catccagcag 2100
   ctcagggact ctgccatctc cagtaacaag accccccaca acagctccat caaccacatt 2160
   gagacggtgc tgcagcagct ggacgaggcg cagtcgcaga tggaggagct cttccaggag 2220
   cgcaagatca agctggagct cttcctgcac gtgcgcatct tcgagaggga cgccatcgac 2280
   attatctcag acctcgagtc ttggaatgat gagctttctc agcaaatgaa tgacttcgac 2340
   acagaagatc tcacgattgc agagcagcgc ctccagcacc atgcagacaa agccttgacc 2400
   atgaacaact tgacttttga cgtcatccac caagggcaag atcttctgca gtatgtcaat 2460
   gaggtccagg cctctggtgt ggagctgctg tgtgatagag atgtagacat ggcaactcgg 2520
   gtccaggacc tgctggagtt tcttcatgaa aaacagcagg aattggattt agccgcagag 2580
   cagcatcgga aacacctgga gcagtgcgtg cagctgcgcc acctgcaggc agaagtgaaa 2640
   caggtgctgg gttggatccg caacggagag tccatgttaa atgccggact tatcacagcc 2700
   agctcgttac aagaggcaga gcagctccag cgagagcacg agcagttcca gcatgccatt 2760
   gagaaaacac atcagagcgc gctgcaggtg cagcagaagg cagaagccat gctacaggcc 2820
   aaccactacg acatggacat gatccgggac tgcgccgaga aggtggcgtc tcactggcaa 2880
   cagctcatgc tcaagatgga agatcgcctc aagctcgtca acgcctctgt cgctttctac 2940
   aaaacctcag agcaggtctg cagcgtcctc gagagcctgg aacaggagta caagagagaa 3000
   gaagactggt gtggcggggc ggataagctg ggcccaaact ctgagacgga ccacgtgacg 3060
   cccatgatca gcaagcacct ggagcagaag gaggcattcc tgaaggcttg cacccttgct 3120
   cggaggaatg cagacgtctt cctgaaatac ctgcacagga acagcgtgaa catgccagga 3180
   atggtgacgc acatcaaagc tcctgaacag caagtgaaaa atatcttgaa tgaactcttc 3240
   caacgggaga acagggtatt gcattactgg accatgagga agagacggct ggaccagtgt 3300
   cagcagtacg tggtctttga gaggagtgcc aagcaggctt tggaatggat ccatgacaat 3360
   ggcgagttct acctttccac acacacctcc acgggctcca gtatacagca cacccaggag 3420
   ctcctgaaag agcacgagga gttccagata actgcaaagc aaaccaaaga gagagtgaag 3480
   ctattgatac agctggctga tggcttttgt gaaaaagggc atgcccatgc ggcagagata 3540
   aaaaaatgtg ttactgctgt ggataagagg tacagagatt tctctctgcg gatggagaag 3600
   tacaggacct ctttggaaaa agccctgggg atttcttcag attccaacaa atcgagtaaa 3660
   agtctccagc tagatatcat tccagccagt atccctggct cagaggtgaa acttcgagat 3720
   gctgctcatg aacttaatga agagaagcgg aaatctgccc gcaggaaaga gttcataatg 3780
   gctgagctca ttcaaactga aaaggcttat gtaagagacc tccgggaatg tatggatacg 3840
   tacctgtggg aaatgaccag tggcgtggaa gagattccac ctggcattgt aaacaaagaa 3900
   ctcatcatct tcggaaacat gcaagaaatc tacgaatttc ataataacat attcctaaag 3960
   gagctggaaa aatatgaaca gttgccagag gatgttggac attgttttgt tacttggcca 4020
   gacaagtttc agatgtatgt cacatattgc aaaaataagc ctgattctac tcagctgata 4080
   ttggaacatg cagggtccta ttttgacgag atacagcagc gacatggatt agccaattcc 4140
   atttcttcct accttattaa accagttcag cgaataacga aatatcagct ccttttaaaa 4200
   gagctgctga cgtgctgtga ggaaggaaag ggagagatta aagatggcct ggaggtgatg 4260
   ctcagcgtgc cgaagcgagc caatgacgcc atgcacctca gcatgctgga agggtttgat 4320
   gaaaacattg agtctcaggg agaactcatc ctacaggaat ccttccaagt gtgggaccca 4380
   aaaaccttaa ttcgaaaggg tcgagaacgg catctcttcc tttttgaaat gtccttagta 4440
   tttagtaaag aagtgaaaga ttccagtggg agaagcaagt acctttataa aagcaaattg 4500
   tttacctcag agttgggtgt cacagaacat gttgaaggag acccttgcaa atttgcactg 4560
   tgggtgggga gaacaccaac ttcagataat aaaattgtcc ttaaggcttc cagcatagag 4620
   aacaagcagg actggataaa gcatatccgc gaagtcatcc aggagcggac gatccacctg 4680
   aagggagccc tgaaggagcc cattcacatc cctaagaccg ctcccgccac aagacagaag 4740
   ggaaggaggg atggagagga tctggacagc caaggagacg gcagcagcca gcctgatacg 4800
   atttccatcg cctcacggac gtctcagaac acgctggaca gcgataagct ctctggtggc 4860
   tgtgagctga cagtggtgat ccatgacttc accgcttgca acagcaacga gctgaccatc 4920
   cgacggggcc agaccgtgga agttctggag cggccgcatg acaagcctga ctggtgtctg 4980
   gtgcggacca ctgaccgctc cccagcggca gaaggcctgg tcccctgtgg ttcactgtgc 5040
   atcgcccact ccagaagtag catggaaatg gagggcatct tcaaccacaa agactcgctc 5100
   tccgtctcca gcaatgacgc cagtccaccc gcatccgtgg cttccctcca gccccacatg 5160
   atcggggccc agagctcgcc gggccccaag cggccgggca acaccctgcg caagtggctc 5220
   accagccccg tgcggcggct cagcagcggc aaggccgacg ggcacgtgaa gaagctggcg 5280
   cacaagcaca agaagagccg cgaggtccgc aagagcgccg acgccggctc gcagaaggac 5340
   tccgacgaca gtgcggccac cccgcaggac gagacggtcg aggagagagg ccggaacgag 5400
   ggcctgagca gcggtactct ctccaaatcc tcctcctcgg ggatgcagag ctgtggagaa 5460
   gaggaaggcg aggagggggc cgacgccgtg cccctgccgc cacccatggc catccagcag 5520
   cacagcctcc tccagccaga ctcacaggat gacaaggcct cttctcggtt attagtccgc 5580
   cccaccagct ccgaaacacc gagtgcagcc gagctcgtca gtgcaattga ggaactcgtg 5640
   aaaagcaaga tggcactgga ggatcgcccc agctcactcc ttgttgacca gggagatagt 5700
   agcagccctt ccttcaaccc ttcggataat tcccttctct cttcctcctc gcccattgat 5760
   gagatggaag aaaggaaatc cagctcttta aagagaagac actacgtttt gcaagaacta 5820
   gtggagacag agcgtgacta tgtgcgggac cttggctatg tggttgaggg ctacatggca 5880
   cttatgaaag aagatggtgt tcctgatgac atgaaaggaa aagacaaaat tgtgttcggc 5940
   aacatccatc agatttacga ctggcacaga gacttttttt taggagagtt agagaagtgc 6000
   cttgaagatc cagaaaaact aggatccctt tttgttaaac acgagagaag gttgcacatg 6060
   tacatagctt attgtcaaaa taaaccaaag tctgagcaca ttgtctcaga atacattgat 6120
   accttttttg aggacttaaa gcagcgtctt ggccacaggt tacagctcac agatctgttg 6180
   atcaaaccag tgcagagaat catgaagtat cagctgttac tgaaggactt cctcaagtat 6240
   tccaaaaagg ccagcctgga tacatcagaa ttagagagag ctgtggaagt catgtgcata 6300
   gtacccaggc ggtgcaacga catgatgaac gtggggcggc tgcaaggatt cgacgggaaa 6360
   atcgttgccc agggtaaact gctcttgcag gacacattct tggtcacaga ccaagatgca 6420
   ggacttctgc ctcgctgcag agagaggcgc atcttcctct ttgagcagat cgtcatattc 6480
   agcgaaccac ttgataaaaa gaagggcttc tccatgccgg gattcctgtt taagaacagt 6540
   atcaaggtga gttgcctttg cctggaggaa aatgtggaaa atgatccctg taaatttgct 6600
   ctgacatcga ggacgggtga cgtggtagag accttcattt tgcattcatc tagtccaagt 6660
   gtccggcaaa cttggatcca tgaaatcaac caaattttag aaaaccagcg caatttttta 6720
   aatgccttga catcgccaat cgagtaccag aggaaccaca gcgggggcgg cggcggcggc 6780
   ggcagcgggg cagcggcggg ggtgggggca gcggcggcgg cggggccccc agtggcggca 6840
   gcggccacag tggcggcccc agcagctgcg gcggcgcccc cagcacgagc aggagccggc 6900
   cctcccggat cccccagcct gtccgacacc acccccccgt gctggtctcc tctgcagcct 6960
   cgagccaggc agaggcagac aagatgtcag agtgaaagca gcagcagtag caacatctcc 7020
   accatgttgg tgacacacga ttacacggca gtgaaggagg atgagatcaa cgtctaccaa 7080
   ggagaggtcg ttcaaattct ggccagcaac cagcagaaca tgtttctggt gttccgagcc 7140
   gccactgacc agtgccccgc agctgagggc tggattccag gctttgtcct gggccacacc 7200
   agtgcagtca tcgtggagaa cccggacggg actctcaaga agtcaacatc ttggcacaca 7260
   gcactccgtt taaggaaaaa atctgagaaa aaagataaag acggcaaaag ggaaggcaag 7320
   ttagagaacg gttatcggaa gtcacgggaa ggactcagca acaaggtatc tgtgaagctt 7380
   ctcaatccca actacattta tgacgttccc ccagaattcg tcattccatt gagtgaggtc 7440
   acgtgtgaga caggggagac cgttgttctt agatgtcgag tctgtggccg ccccaaagcc 7500
   tcaattacct ggaagggccc tgaacacaac accttgaaca acgatggtca ctacagcatc 7560
   tcctacagtg acctgggaga ggccacgctg aagattgtgg gcgtgaccac ggaagatgac 7620
   ggcatctaca cgtgcatcgc tgtcaatgac atgggttcag cctcatcatc ggccagcctg 7680
   agggtcctag gtccagggat ggatgggatc atggtgacct ggaaagacaa ctttgactcc 7740
   ttctacagtg aagtggctga gcttggcagg ggcagattct ctgtcgttaa gaaatgtgat 7800
   cagaaaggaa ccaagcgagc agtggccact aagtttgtga acaagaagtt gatgaagcgc 7860
   gaccaggtca cccatgagct tggcatcctg cagagcctcc agcaccccct gcttgtcggc 7920
   ctcctcgaca cctttgagac ccccaccagc tacatcctgg tcttagaaat ggctgaccag 7980
   ggtcgcctcc tggactgcgt ggtgcgatgg ggaagcctca ctgaagggaa gatcagggcg 8040
   cacctggggg aggttctgga agctgtccgg tacctgcaca actgcaggat agcacacctg 8100
   gacctaaagc ctgagaatat cctggtggat gagagtttag ccaagccaac catcaaactg 8160
   gctgactttg gagatgctgt tcagctcaac acgacctact acatccacca gttactgggg 8220
   aaccctgaat tcgcagcccc tgaaatcatc ctcgggaacc ctgtctccct gacctcggat 8280
   acgtggagtg ttggagtgct cacatacgta cttcttagtg gcgtgtcccc cttcctggat 8340
   gacagtgtgg aagagacctg cctgaacatt tgccgcttag actttagctt cccagatgac 8400
   tactttaaag gagtgagcca gaaggccaag gagttcgtgt gcttcctcct gcaggaggac 8460
   cccgccaagc gtccctcggc tgcgctggcc ctccaggagc agtggctgca ggccggcaac 8520
   ggcagaagca cgggcgtcct cgacacgtcc agactgactt ccttcattga gcggcgcaaa 8580
   caccagaatg atgttcgacc tatccgtagc attaaaaact ttctgcagag caggcttctg 8640
   cctagagttt gacctatcca gaagttcttt ctcattctct ttcacctgcc aatcagctgt 8700
   taatctgaat tttcaagaga aaacaagcaa acataactga tcagctgccg gtatgttcat 8760
   cgtgtgaaat tgcattccaa gtgagctgtg ctcagcagtg cttggacaca gagctgcaag 8820
   ctgcgctggg gtggaggacc gtcacttaca ctctgccaag gacggaggtc gcattgctgt 8880
   atcacagtat tttttacgga tttctg 8906
<210> 86
   <211> 1204
   <212> DNA
   <213> Homo sapiens
<400> 86
   tcggcggcgg tggtatcggc ggcagctgtg agggggttcc gggaagatgg tgctgatcaa 60
   ggaattccgt gtggttttgc catgttctgt tcaggagtat caggttgggc agctttactc 120
   tgttgcagaa gctagtaaga atgagactgg tggtggagaa ggaattgaag tcttaaagaa 180
   tgaaccttat gagaaggatg gagaaaaggg acagtatacg cacaaaattt atcacctaaa 240
   gagcaaagtg cctgcattcg tgaggatgat tgctcccgag ggctccttgg tgtttcatga 300
   gaaagcctgg aatgcgtacc cctactgtag aacaattgta acgaatgaat atatgaaaga 360
   tgatttcttc attaaaatcg aaacatggca caaaccagac ttgggaacat tagaaaatgt 420
   acatggttta gatccaaaca catggaaaac tgttgaaatt gtccatatag atattgcaga 480
   tagaagtcaa gttgaaccag cagactacaa agctgatgaa gacccagcat tattccagtc 540
   agtcaagacc aagagaggcc ctttgggacc caactggaag aaggagctgg caaacagccc 600
   tgactgtccc cagatgtgtg cctataagct ggtgaccatc aaattcaagt ggtggggact 660
   gcaaagcaaa gtagaaaact tcattcaaaa gcaagaaaaa cggatattta caaacttcca 720
   tcgccagctt ttttgttgga ttgacaagtg gatcgatctc acgatggaag acattaggag 780
   aatggaagac gagactcaga aagaactaga aacaatgcgt aagaggggtt ccgttcgagg 840
   cacgtcggct gctgatgtct agatgagtcc cctgtagggt cagagacaat gtcaaactgt 900
   ttacgtaatc aaggtcaagt gaggggaaca agcgcagcca gtgatgagtg aacaacaatc 960
   tgaccagtat cttgcagtgt tgacgtttcc cagatgtgtg cttgtgatga tacacacaca 1020
   tgcacaggtt ctcaaccacg tgtgtatata tgtatgtgtg catatgtctg tagctgtata 1080
   taaagcgcat gtagagctac agatccagat acacacactt gtgtatatat gtacatacag 1140
   acatactgaa gggattagta caatttctcc aaagtactgt acctatcttc agcaagaatg 1200
   caaa 1204
<210> 87
   <211> 892
   <212> PRT
   <213> Homo sapiens
<400> 87
   Met Asp His Tyr Asp Ser Gln Gln Thr Asn Asp Tyr Met Gln Pro Glu
    1               5                  10                  15
   Glu Asp Trp Asp Arg Asp Leu Leu Leu Asp Pro Ala Trp Glu Lys Gln
               20                  25                  30
   Gln Arg Lys Thr Phe Thr Ala Trp Cys Asn Ser His Leu Arg Lys Ala
           35                  40                  45
   Gly Thr Gln Ile Glu Asn Ile Glu Glu Asp Phe Arg Asp Gly Leu Lys
       50                  55                  60
   Leu Met Leu Leu Leu Glu Val Ile Ser Gly Glu Arg Leu Ala Lys Pro
   65                  70                  75                  80
   Glu Arg Gly Lys Met Arg Val His Lys Ile Ser Asn Val Asn Lys Ala
                   85                  90                  95
   Leu Asp Phe Ile Ala Ser Lys Gly Val Lys Leu Val Ser Ile Gly Ala
               100                 105                 110
   Glu Glu Ile Val Asp Gly Asn Val Lys Met Thr Leu Gly Met Ile Trp
           115                 120                 125
   Thr Ile Ile Leu Arg Phe Ala Ile Gln Asp Ile Ser Val Glu Glu Thr
       130                 135                 140
   Ser Ala Lys Glu Gly Leu Leu Leu Trp Cys Gln Arg Lys Thr Ala Pro
   145                 150                 155                 160
   Tyr Lys Asn Val Asn Ile Gln Asn Phe His Ile Ser Trp Lys Asp Gly
                   165                 170                 175
   Leu Gly Phe Cys Ala Leu Ile His Arg His Arg Pro Glu Leu Ile Asp
               180                 185                 190
   Tyr Gly Lys Leu Arg Lys Asp Asp Pro Leu Thr Asn Leu Asn Thr Ala
           195                 200                 205
   Phe Asp Val Ala Glu Lys Tyr Leu Asp Ile Pro Lys Met Leu Asp Ala
       210                 215                 220
   Glu Asp Ile Val Gly Thr Ala Arg Pro Asp Glu Lys Ala Ile Met Thr
   225                 230                 235                 240
   Tyr Val Ser Ser Phe Tyr His Ala Phe Ser Gly Ala Gln Lys Ala Glu
                   245                 250                 255
   Thr Ala Ala Asn Arg Ile Cys Lys Val Leu Ala Val Asn Gln Glu Asn
               260                 265                 270
   Glu Gln Leu Met Glu Asp Tyr Glu Lys Leu Ala Ser Asp Leu Leu Glu
           275                 280                 285
   Trp Ile Arg Arg Thr Ile Pro Trp Leu Glu Asn Arg Val Pro Glu Asn
       290                 295                 300
   Thr Met His Ala Met Gln Gln Lys Leu Glu Asp Phe Arg Asp Tyr Arg
   305                 310                 315                 320
   Arg Leu His Lys Pro Pro Lys Val Gln Glu Lys Cys Gln Leu Glu Ile
                   325                 330                 335
   Asn Phe Asn Thr Leu Gln Thr Lys Leu Arg Leu Ser Asn Arg Pro Ala
               340                 345                 350
   Phe Met Pro Ser Glu Gly Arg Met Val Ser Asp Ile Asn Asn Ala Trp
           355                 360                 365
   Gly Cys Leu Glu Gln Val Glu Lys Gly Tyr Glu Glu Trp Leu Leu Asn
       370                 375                 380
   Glu Ile Arg Arg Leu Glu Arg Leu Asp His Leu Ala Glu Lys Phe Arg
   385                 390                 395                 400
   Gln Lys Ala Ser Ile His Glu Ala Trp Thr Asp Gly Lys Glu Ala Met
                   405                 410                 415
   Leu Arg Gln Lys Asp Tyr Glu Thr Ala Thr Leu Ser Glu Ile Lys Ala
   420 425 430
   Leu Leu Lys Lys His Glu Ala Phe Glu Ser Asp Leu Ala Ala His Gln
   435 440 445
   Asp Arg Val Glu Gln Ile Ala Ala Ile Ala Gln Glu Leu Asn Glu Leu
   450 455 460
   Asp Tyr Tyr Asp Ser Pro Ser Val Asn Ala Arg Cys Gln Lys Ile Cys
   465 470 475 480
   Asp Gln Trp Asp Asn Leu Gly Ala Leu Thr Gln Lys Arg Arg Glu Ala
   485 490 495
   Leu Glu Arg Thr Glu Lys Leu Leu Glu Thr Ile Asp Gln Leu Tyr Leu
   500 505 510
   Glu Tyr Ala Lys Arg Ala Ala Pro Phe Asn Asn Trp Met Glu Gly Ala
   515 520 525
   Met Glu Asp Leu Gln Asp Thr Phe Ile Val His Thr Ile Glu Glu Ile
   530 535 540
   Gln Gly Leu Thr Thr Ala His Glu Gln Phe Lys Ala Thr Leu Pro Asp
   545 550 555 560
   Ala Asp Lys Glu Arg Leu Ala Ile Leu Gly Ile His Asn Glu Val Ser
   565 570 575
   Lys Ile Val Gln Thr Tyr His Val Asn Met Ala Gly Thr Asn Pro Tyr
   580 585 590
   Thr Thr Ile Thr Pro Gln Glu Ile Asn Gly Lys Trp Asp His Val Arg
   595 600 605
   Gln Leu Val Pro Arg Arg Asp Gln Ala Leu Thr Glu Glu His Ala Arg
   610 615 620
   Gln Gln His Asn Glu Arg Leu Arg Lys Gln Phe Gly Ala Gln Ala Asn
   625 630 635 640
   Val Ile Gly Pro Trp Ile Gln Thr Lys Met Glu Glu Ile Gly Arg Ile
   645 650 655
   Ser Ile Glu Met His Gly Thr Leu Glu Asp Gln Leu Ser His Leu Arg
   660 665 670
   Gln Tyr Glu Lys Ser Ile Val Asn Tyr Lys Pro Lys Ile Asp Gln Leu
   675 680 685
   Glu Gly Asp His Gln Leu Ile Gln Glu Ala Leu Ile Phe Asp Asn Lys
   690 695 700
   His Thr Asn Tyr Thr Met Glu His Ile Arg Val Gly Trp Glu Gln Leu
   705 710 715 720
   Leu Thr Thr Ile Ala Arg Thr Ile Asn Glu Val Glu Asn Gln Ile Leu
   725 730 735
   Thr Arg Asp Ala Lys Gly Ile Ser Gln Glu Gln Met Asn Glu Phe Arg
   740 745 750
   Ala Ser Phe Asn His Phe Asp Arg Asp His Ser Gly Thr Leu Gly Pro
   755 760 765
   Glu Glu Phe Lys Ala Cys Leu Ile Ser Leu Gly Tyr Asp Ile Gly Asn
   770 775 780
   Asp Pro Gln Gly Glu Ala Glu Phe Ala Arg Ile Met Ser Ile Val Asp
   785 790 795 800
   Pro Asn Arg Leu Gly Val Val Thr Phe Gln Ala Phe Ile Asp Phe Met
   805 810 815
   Ser Arg Glu Thr Ala Asp Thr Asp Thr Ala Asp Gln Val Met Ala Ser
   820 825 830
   Phe Lys Ile Leu Ala Gly Asp Lys Asn Tyr Ile Thr Met Asp Glu Leu
           835                 840                 845
   Arg Arg Glu Leu Pro Pro Asp Gln Ala Glu Tyr Cys Ile Ala Arg Met
       850                 855                 860
   Ala Pro Tyr Thr Gly Pro Asp Ser Val Pro Gly Ala Leu Asp Tyr Met
   865                 870                 875                 880
   Ser Phe Ser Thr Ala Leu Tyr Gly Glu Ser Asp Leu
                   885                 890
<210> 88
   <211> 197
   <212> PRT
   <213> Homo sapiens
<400> 88
   Met Met Phe Pro Gln Ser Arg His Ser Gly Ser Ser His Leu Pro Gln
   1               5                   10                  15
   Gln Leu Lys Phe Thr Thr Ser Asp Ser Cys Asp Arg Ile Lys Asp Glu
               20                  25                  30
   Phe Gln Leu Leu Gln Ala Gln Tyr His Ser Leu Lys Leu Glu Cys Asp
           35                  40                  45
   Lys Leu Ala Ser Glu Lys Ser Glu Met Gln Arg His Tyr Val Met Tyr
       50                  55                  60
   Tyr Glu Met Ser Tyr Gly Leu Asn Ile Glu Met His Lys Gln Ala Glu
   65                  70                  75                  80
   Ile Val Lys Arg Leu Asn Gly Ile Cys Ala Gln Val Leu Pro Tyr Leu
                   85                  90                  95
   Ser Gln Glu His Gln Gln Gln Val Leu Gly Ala Ile Glu Arg Ala Lys
               100                 105                 110
   Gln Val Thr Ala Pro Glu Leu Asn Ser Ile Ile Arg Gln Gln Leu Gln
           115                 120                 125
   Ala His Gln Leu Ser Gln Leu Gln Ala Leu Ala Leu Pro Leu Thr Pro
       130                 135                 140
   Leu Pro Val Gly Leu Gln Pro Pro Ser Leu Pro Ala Val Ser Ala Gly
   145                 150                 155                 160
   Thr Gly Leu Leu Ser Leu Ser Ala Leu Gly Ser Gln Ala His Leu Ser
                   165                 170                 175
   Lys Glu Asp Lys Asn Gly His Asp Gly Asp Thr His Gln Glu Asp Asp
               180                 185                 190
   Gly Glu Lys Ser Asp
           195
<210> 89
   <211> 739
   <212> PRT
   <213> Homo sapiens
<400> 89
   Gly Asp Lys Glu Pro Thr Glu Thr Ile Gly Asp Leu Ser Ile Cys Leu
    1               5                  10                  15
   Asp Gly Leu Gln Leu Glu Ser Glu Val Val Thr Asn Gly Glu Thr Thr
               20                  25                  30
   Cys Ser Glu Ser Ala Ser Gln Asn Asp Asp Gly Ser Arg Ser Lys Asp
           35                  40                  45
   Glu Thr Arg Val Ser Thr Asn Gly Ser Asp Asp Pro Glu Asp Ala Gly
   50 55 60
   Ala Gly Glu Asn Arg Arg Val Ser Gly Asn Asn Ser Pro Ser Leu Ser
   65 70 75 80
   Asn Gly Gly Phe Lys Pro Ser Arg Pro Pro Arg Pro Ser Arg Pro Pro
   85 90 95
   Pro Pro Thr Pro Arg Arg Pro Ala Ser Val Asn Gly Ser Pro Ser Ala
   100 105 110
   Thr Ser Glu Ser Asp Gly Ser Ser Thr Gly Ser Leu Pro Pro Thr Asn
   115 120 125
   Thr Asn Thr Asn Thr Ser Glu Gly Ala Thr Ser Gly Leu Ile Ile Pro
   130 135 140
   Leu Thr Ile Ser Gly Gly Ser Gly Pro Arg Pro Leu Asn Pro Val Thr
   145 150 155 160
   Gln Ala Pro Leu Pro Pro Gly Trp Glu Gln Arg Val Asp Gln His Gly
   165 170 175
   Arg Val Tyr Tyr Val Asp His Val Glu Lys Arg Thr Thr Trp Asp Arg
   180 185 190
   Pro Glu Pro Leu Pro Pro Gly Trp Glu Arg Arg Val Asp Asn Met Gly
   195 200 205
   Arg Ile Tyr Tyr Val Asp His Phe Thr Arg Thr Thr Thr Trp Gln Arg
   210 215 220
   Pro Thr Leu Glu Ser Val Arg Asn Tyr Glu Gln Trp Gln Leu Gln Arg
   225 230 235 240
   Ser Gln Leu Gln Gly Ala Met Gln Gln Phe Asn Gln Arg Phe Ile Tyr
   245 250 255
   Gly Asn Gln Asp Leu Phe Ala Thr Ser Gln Ser Lys Glu Phe Asp Pro
   260 265 270
   Leu Gly Pro Leu Pro Pro Gly Trp Glu Lys Arg Thr Asp Ser Asn Gly
   275 280 285
   Arg Val Tyr Phe Val Asn His Asn Thr Arg Ile Thr Gln Trp Glu Asp
   290 295 300
   Pro Arg Ser Gln Gly Gln Leu Asn Glu Lys Pro Leu Pro Glu Gly Trp
   305 310 315 320
   Glu Met Arg Phe Thr Val Asp Gly Ile Pro Tyr Phe Val Asp His Asn
   325 330 335
   Arg Arg Thr Thr Thr Tyr Ile Asp Pro Arg Thr Gly Lys Ser Ala Leu
   340 345 350
   Asp Asn Gly Pro Gln Ile Ala Tyr Val Arg Asp Phe Lys Ala Lys Val
   355 360 365
   Gln Tyr Phe Arg Phe Trp Cys Gln Gln Leu Ala Met Pro Gln His Ile
   370 375 380
   Lys Ile Thr Val Thr Arg Lys Thr Leu Phe Glu Asp Ser Phe Gln Gln
   385 390 395 400
   Ile Met Ser Phe Ser Pro Gln Asp Leu Arg Arg Arg Leu Trp Val Ile
   405 410 415
   Phe Pro Gly Glu Glu Gly Leu Asp Tyr Gly Gly Val Ala Arg Glu Trp
   420 425 430
   Phe Phe Leu Leu Ser His Glu Val Leu Asn Pro Met Tyr Cys Leu Phe
   435 440 445
   Glu Tyr Ala Gly Lys Asp Asn Tyr Cys Leu Gln Ile Asn Pro Ala Ser
   450 455 460
   Tyr Ile Asn Pro Asp His Leu Lys Tyr Phe Arg Phe Ile Gly Arg Phe
   465 470 475 480
   Ile Ala Met Ala Leu Phe His Gly Lys Phe Ile Asp Thr Gly Phe Ser
   485 490 495
   Leu Pro Phe Tyr Lys Arg Ile Leu Asn Lys Pro Val Gly Leu Lys Asp
   500 505 510
   Leu Glu Ser Ile Asp Pro Glu Phe Tyr Asn Ser Leu Ile Trp Val Lys
   515 520 525
   Glu Asn Asn Ile Glu Glu Cys Asp Leu Glu Met Tyr Phe Ser Val Asp
   530 535 540
   Lys Glu Ile Leu Gly Glu Ile Lys Ser His Asp Leu Lys Pro Asn Gly
   545 550 555 560
   Gly Asn Ile Leu Val Thr Glu Glu Asn Lys Glu Glu Tyr Ile Arg Met
   565 570 575
   Val Ala Glu Trp Arg Leu Ser Arg Gly Val Glu Glu Gln Thr Gln Ala
   580 585 590
   Phe Phe Glu Gly Phe Asn Glu Ile Leu Pro Gln Gln Tyr Leu Gln Tyr
   595 600 605
   Phe Asp Ala Lys Glu Leu Glu Val Leu Leu Cys Gly Met Gln Glu Ile
   610 615 620
   Asp Leu Asn Asp Trp Gln Arg His Ala Ile Tyr Arg His Tyr Ala Arg
   625 630 635 640
   Thr Ser Lys Gln Ile Met Trp Phe Trp Gln Phe Val Lys Glu Ile Asp
   645 650 655
   Asn Glu Lys Arg Met Arg Leu Leu Gln Phe Val Thr Gly Thr Cys Arg
   660 665 670
   Leu Pro Val Gly Gly Phe Ala Asp Leu Met Gly Ser Asn Gly Pro Gln
   675 680 685
   Lys Phe Cys Ile Glu Lys Val Gly Lys Glu Asn Trp Leu Pro Arg Ser
   690 695 700
   His Thr Cys Phe Asn Arg Leu Asp Leu Pro Pro Tyr Lys Ser Tyr Glu
   705 710 715 720
   Gln Leu Lys Glu Lys Leu Leu Phe Ala Ile Glu Glu Thr Glu Gly Phe
   725 730 735
   Gly Gln Glu
<210> 90
   <211> 431
   <212> PRT
   <213> Homo sapiens
<400> 90
   Gly Pro Pro Pro Thr Arg Ala Leu Pro Leu Pro Gln Ser Leu Pro Pro
   1 5 10 15
   Asp Phe Arg Leu Glu Pro Thr Ala Pro Ala Leu Ser Pro Arg Ser Ser
   20 25 30
   Phe Ala Ser Ser Ser Ala Ser Asp Ala Ser Lys Pro Ser Ser Pro Arg
   35 40 45
   Gly Ser Leu Leu Leu Asp Gly Ala Gly Ala Gly Gly Ala Gly Gly Ser
   50 55 60
   Arg Pro Cys Ser Asn Arg Thr Ser Gly Ile Ser Met Gly Tyr Asp Gln
   65 70 75 80
   Arg His Gly Ser Pro Leu Pro Ala Gly Pro Cys Leu Phe Gly Pro Pro
                       85                  90                  95
   Leu Ala Gly Ala Pro Ala Gly Tyr Ser Pro Gly Gly Val Pro Ser Ala
               100                 105                 110
   Tyr Pro Glu Leu His Ala Ala Leu Asp Arg Leu Tyr Ala Gln Arg Pro
           115                 120                 125
   Ala Gly Phe Gly Cys Gln Glu Ser Arg His Ser Tyr Pro Pro Ala Leu
       130                 135                 140
   Gly Ser Pro Gly Ala Leu Ala Gly Ala Arg Val Gly Ala Ala Gly Pro
   145                 150                 155                 160
   Leu Glu Arg Arg Gly Ala Gln Pro Gly Arg His Ser Val Thr Gly Tyr
                   165                 170                 175
   Gly Asp Cys Ala Val Gly Ala Arg Tyr Gln Asp Glu Leu Thr Ala Leu
               180                 185                 190
   Leu Arg Leu Thr Val Gly Thr Gly Gly Arg Glu Ala Gly Ala Arg Gly
           195                 200                 205
   Glu Pro Ser Gly Ile Glu Pro Ser Gly Leu Glu Glu Pro Pro Gly Pro
       210                 215                 220
   Phe Val Pro Glu Ala Ala Arg Ala Arg Met Arg Glu Pro Glu Ala Arg
   225                 230                 235                 240
   Glu Asp Tyr Phe Gly Thr Cys Ile Lys Cys Asn Lys Gly Ile Tyr Gly
                   245                 250                 255
   Gln Ser Asn Ala Cys Gln Ala Leu Asp Ser Leu Tyr His Thr Gln Cys
               260                 265                 270
   Phe Val Cys Cys Ser Cys Gly Arg Thr Leu Arg Cys Lys Ala Phe Tyr
           275                 280                 285
   Ser Val Asn Gly Ser Val Tyr Cys Glu Glu Asp Tyr Leu Phe Ser Gly
       290                 295                 300
   Phe Gln Glu Ala Ala Glu Lys Cys Cys Val Cys Gly His Leu Ile Leu
   305                 310                 315                 320
   Glu Lys Ile Leu Gln Ala Met Gly Lys Ser Tyr His Pro Gly Cys Phe
                   325                 330                 335
   Arg Cys Ile Val Cys Asn Lys Cys Leu Asp Gly Ile Pro Phe Thr Val
               340                 345                 350
   Asp Phe Ser Asn Gln Val Tyr Cys Val Thr Asp Tyr His Lys Asn Tyr
           355                 360                 365
   Ala Pro Lys Cys Ala Ala Cys Gly Gln Pro Ile Leu Pro Ser Glu Gly
       370                 375                 380
   Cys Glu Asp Ile Val Arg Val Ile Ser Met Asp Arg Asp Tyr His Phe
   385                 390                 395                 400
   Glu Cys Tyr His Cys Glu Asp Cys Arg Met Gln Leu Ser Asp Glu Glu
                   405                 410                 415
   Gly Cys Cys Cys Phe Pro Leu Asp Gly His Leu Leu Cys His Gly
               420                 425                 430
<210> 91
   <211> 900
   <212> PRT
   <213> Homo sapiens
<400> 91
   Gly Pro Gly Ser Arg His His Arg Ala Arg Asp Arg Leu Ile His Phe
   1 5 10 15
   Gly Ala Val Ser Thr Asp Val Leu Gly Cys Ser Ala His Cys Ser Leu
               20                  25                  30
   Thr Gln Ser Pro Lys Met Asn Ile Gln Glu Gln Gly Phe Pro Leu Asp
           35                  40                  45
   Leu Gly Ala Ser Phe Thr Glu Asp Ala Pro Arg Pro Pro Val Pro Gly
       50                  55                  60
   Glu Glu Gly Glu Leu Val Ser Thr Asp Pro Arg Pro Ala Ser Tyr Ser
   65                  70                  75                  80
   Phe Cys Ser Gly Lys Gly Val Gly Ile Lys Gly Glu Thr Ser Thr Ala
                   85                  90                  95
   Thr Pro Arg Arg Ser Asp Leu Asp Leu Gly Tyr Glu Pro Glu Gly Ser
               100                 105                 110
   Ala Ser Pro Thr Pro Pro Tyr Leu Lys Trp Ala Glu Ser Leu His Ser
           115                 120                 125
   Leu Leu Asp Asp Gln Asp Gly Ile Ser Leu Phe Arg Thr Phe Leu Lys
       130                 135                 140
   Gln Glu Gly Cys Ala Asp Leu Leu Asp Phe Trp Phe Ala Cys Thr Gly
   145                 150                 155                 160
   Phe Arg Lys Leu Glu Pro Cys Asp Ser Asn Glu Glu Lys Arg Leu Lys
                   165                 170                 175
   Leu Ala Arg Ala Ile Tyr Arg Lys Tyr Ile Leu Asp Asn Asn Gly Ile
               180                 185                 190
   Val Ser Arg Gln Thr Lys Pro Ala Thr Lys Ser Phe Ile Lys Gly Cys
           195                 200                 205
   Ile Met Lys Gln Leu Ile Asp Pro Ala Met Phe Asp Gln Ala Gln Thr
       210                 215                 220
   Glu Ile Gln Ala Thr Met Glu Glu Asn Thr Tyr Pro Ser Phe Leu Lys
   225                 230                 235                 240
   Ser Asp Ile Tyr Leu Glu Tyr Thr Arg Thr Gly Ser Glu Ser Pro Lys
                   245                 250                 255
   Val Cys Ser Asp Gln Ser Ser Gly Ser Gly Thr Gly Lys Gly Ile Ser
               260                 265                 270
   Gly Tyr Leu Pro Thr Leu Asn Glu Asp Glu Glu Trp Lys Cys Asp Gln
           275                 280                 285
   Asp Met Asp Glu Asp Asp Gly Arg Asp Ala Ala Pro Pro Gly Arg Leu
       290                 295                 300
   Pro Gln Lys Leu Leu Leu Glu Thr Ala Ala Pro Arg Val Ser Ser Ser
   305                 310                 315                 320
   Arg Arg Tyr Ser Glu Gly Arg Glu Phe Arg Tyr Gly Ser Trp Arg Glu
                   325                 330                 335
   Pro Val Asn Pro Tyr Tyr Val Asn Ala Gly Tyr Ala Leu Ala Pro Ala
               340                 345                 350
   Thr Ser Ala Asn Asp Ser Glu Gln Gln Ser Leu Ser Ser Asp Ala Asp
           355                 360                 365
   Thr Leu Ser Leu Thr Asp Ser Ser Val Asp Gly Ile Pro Pro Tyr Arg
       370                 375                 380
   Ile Arg Lys Gln His Arg Arg Glu Met Gln Glu Ser Ala Gln Val Asn
   385                 390                 395                 400
   Gly Arg Val Pro Leu Pro His Ile Pro Arg Thr Tyr Arg Val Pro Lys
                   405                 410                 415
   Glu Val Arg Val Glu Pro Gln Lys Phe Ala Glu Glu Leu Ile His Arg
               420                 425                 430
   Leu Glu Ala Val Gln Arg Thr Arg Glu Ala Glu Glu Lys Leu Glu Glu
           435                 440                 445
   Arg Leu Lys Arg Val Arg Met Glu Glu Glu Gly Glu Asp Gly Asp Pro
   450 455 460
   Ser Ser Gly Pro Pro Gly Pro Cys His Lys Leu Pro Pro Ala Pro Ala
   465 470 475 480
   Trp His His Phe Pro Pro Arg Leu Cys Trp Thr Trp Ala Cys Ala Gly
   485 490 495
   Leu Arg Asp Ala His Glu Glu Asn Pro Glu Ser Ile Leu Asp Glu His
   500 505 510
   Val Gln Arg Val Leu Arg Thr Thr Gly Arg Gln Ser Pro Gly Pro Gly
   515 520 525
   His Arg Ser Pro Asp Ser Gly His Val Ala Lys Met Pro Val Ala Leu
   530 535 540
   Gly Gly Ala Ala Ser Gly His Gly Lys His Val Pro Lys Ser Gly Ala
   545 550 555 560
   Lys Leu Asp Ala Ala Gly Leu His His His Arg His Val His His His
   565 570 575
   Val His His Ser Thr Ala Arg Pro Lys Glu Gln Val Glu Ala Glu Ala
   580 585 590
   Thr Arg Arg Ala Gln Ser Ser Phe Ala Trp Gly Leu Glu Pro His Ser
   595 600 605
   His Gly Ala Arg Ser Arg Gly Tyr Ser Glu Ser Val Gly Ala Ala Pro
   610 615 620
   Asn Ala Ser Asp Gly Leu Ala His Ser Gly Lys Val Gly Val Ala Cys
   625 630 635 640
   Lys Arg Asn Ala Lys Lys Ala Glu Ser Gly Lys Ser Ala Ser Thr Glu
   645 650 655
   Val Pro Gly Ala Ser Glu Asp Ala Glu Lys Asn Gln Lys Ile Met Gln
   660 665 670
   Trp Ile Ile Glu Gly Glu Lys Glu Ile Ser Arg His Arg Arg Thr Gly
   675 680 685
   His Gly Ser Ser Gly Thr Arg Lys Pro Gln Pro His Glu Asn Ser Arg
   690 695 700
   Pro Leu Ser Leu Glu His Pro Trp Ala Gly Pro Gln Leu Arg Thr Ser
   705 710 715 720
   Val Gln Pro Ser His Leu Phe Ile Gln Asp Pro Thr Met Pro Pro His
   725 730 735
   Pro Ala Pro Asn Pro Leu Thr Gln Leu Glu Glu Ala Arg Arg Arg Leu
   740 745 750
   Glu Glu Glu Glu Lys Arg Ala Ser Arg Ala Pro Ser Lys Gln Arg Tyr
   755 760 765
   Val Gln Glu Val Met Arg Arg Gly Arg Ala Cys Val Arg Pro Ala Cys
   770 775 780
   Ala Pro Val Leu His Val Val Pro Ala Val Ser Asp Met Glu Leu Ser
   785 790 795 800
   Glu Thr Glu Thr Arg Ser Gln Arg Lys Val Gly Gly Gly Ser Ala Gln
   805 810 815
   Pro Cys Asp Ser Ile Val Val Ala Tyr Tyr Phe Cys Gly Glu Pro Ile
   820 825 830
   Pro Tyr Arg Thr Leu Val Arg Gly Arg Ala Val Thr Leu Gly Gln Phe
   835 840 845
   Lys Glu Leu Leu Thr Lys Lys Gly Ser Tyr Arg Tyr Tyr Phe Lys Lys
   850 855 860
   Val Ser Asp Glu Phe Asp Cys Gly Val Val Phe Glu Glu Val Arg Glu
   865 870 875 880
   Asp Glu Ala Val Leu Pro Val Phe Glu Glu Lys Ile Ile Gly Lys Val
   885 890 895
   Glu Lys Val Asp
   900
<210> 92
   <211> 591
   <212> PRT
   <213> Homo sapiens
<400> 92
   Met Val Pro Val Ala Val Thr Ala Ala Val Ala Pro Val Leu Ser Ile
   1               5                   10                  15
   Asn Ser Asp Phe Ser Asp Leu Arg Glu Ile Lys Lys Gln Leu Leu Leu
               20                  25                  30
   Ile Ala Gly Leu Thr Arg Glu Arg Gly Leu Leu His Ser Ser Lys Trp
           35                  40                  45
   Ser Ala Glu Leu Ala Phe Ser Leu Pro Ala Leu Pro Leu Ala Glu Leu
       50                  55                  60
   Gln Pro Pro Pro Pro Ile Thr Glu Glu Asp Ala Gln Asp Met Asp Ala
   65                  70                  75                  80
   Tyr Thr Leu Ala Lys Ala Tyr Phe Asp Val Lys Glu Tyr Asp Arg Ala
                   85                  90                  95
   Ala His Phe Leu His Gly Cys Asn Ser Lys Lys Ala Tyr Phe Leu Tyr
               100                 105                 110
   Met Tyr Ser Arg Tyr Leu Ser Gly Glu Lys Lys Lys Asp Asp Glu Thr
           115                 120                 125
   Val Asp Ser Leu Gly Pro Leu Glu Lys Gly Gln Val Lys Asn Glu Ala
       130                 135                 140
   Leu Arg Glu Leu Arg Val Glu Leu Ser Lys Lys His Gln Ala Arg Glu
   145                 150                 155                 160
   Leu Asp Gly Phe Gly Leu Tyr Leu Tyr Gly Val Val Leu Arg Lys Leu
                   165                 170                 175
   Asp Leu Val Lys Glu Ala Ile Asp Val Phe Val Glu Ala Thr His Val
               180                 185                 190
   Leu Pro Leu His Trp Gly Ala Trp Leu Glu Leu Cys Asn Leu Ile Thr
           195                 200                 205
   Asp Lys Glu Met Leu Lys Phe Leu Ser Leu Pro Asp Thr Trp Met Lys
       210                 215                 220
   Glu Phe Phe Leu Ala His Ile Tyr Thr Glu Leu Gln Leu Ile Glu Glu
   225                 230                 235                 240
   Ala Leu Gln Lys Tyr Gln Asn Leu Ile Asp Val Gly Phe Ser Lys Ser
                   245                 250                 255
   Ser Tyr Ile Val Ser Gln Ile Ala Val Ala Tyr His Asn Ile Arg Asp
               260                 265                 270
   Ile Asp Lys Ala Leu Ser Ile Phe Asn Glu Leu Arg Lys Gln Asp Pro
           275                 280                 285
   Tyr Arg Ile Glu Asn Met Asp Thr Phe Ser Asn Leu Leu Tyr Val Arg
       290                 295                 300
   Ser Met Lys Ser Glu Leu Ser Tyr Leu Ala His Asn Leu Cys Glu Ile
   305                 310                 315                 320
   Asp Lys Tyr Arg Val Glu Thr Cys Cys Val Ile Gly Asn Tyr Tyr Ser
                   325                 330                 335
   Leu Arg Ser Gln His Glu Lys Ala Ala Leu Tyr Phe Gln Arg Ala Leu
               340                 345                 350
   Lys Leu Asn Pro Arg Tyr Leu Gly Ala Trp Thr Leu Met Gly His Glu
           355                 360                 365
   Tyr Met Glu Met Lys Asn Thr Ser Ala Ala Ile Gln Ala Tyr Arg His
       370                 375                 380
   Ala Ile Glu Val Asn Lys Arg Asp Tyr Arg Ala Trp Tyr Gly Leu Gly
   385                 390                 395                 400
   Gln Thr Tyr Glu Ile Leu Lys Met Pro Phe Tyr Cys Leu Tyr Tyr Tyr
                   405                 410                 415
   Arg Arg Ala His Gln Leu Arg Pro Asn Asp Ser Arg Met Leu Val Ala
               420                 425                 430
   Leu Gly Glu Cys Tyr Glu Lys Leu Asn Gln Leu Val Glu Ala Lys Lys
           435                 440                 445
   Cys Tyr Trp Arg Ala Tyr Ala Val Gly Asp Val Glu Lys Met Ala Leu
       450                 455                 460
   Val Lys Leu Ala Lys Leu His Glu Gln Leu Thr Glu Ser Glu Gln Ala
   465                 470                 475                 480
   Ala Gln Cys Tyr Ile Lys Tyr Ile Gln Asp Ile Tyr Ser Cys Gly Glu
                   485                 490                 495
   Ile Val Glu His Leu Glu Glu Ser Thr Ala Phe Arg Tyr Leu Ala Gln
               500                 505                 510
   Tyr Tyr Phe Lys Cys Lys Leu Trp Asp Glu Ala Ser Thr Cys Ala Gln
           515                 520                 525
   Lys Cys Cys Ala Phe Asn Asp Thr Arg Glu Glu Gly Lys Ala Leu Leu
       530                 535                 540
   Arg Gln Ile Leu Gln Leu Arg Asn Gln Gly Glu Thr Pro Thr Thr Glu
   545                 550                 555                 560
   Val Pro Ala Pro Phe Phe Leu Pro Ala Ser Leu Ser Ala Asn Asn Thr
                   565                 570                 575
   Pro Thr Arg Arg Val Ser Pro Leu Asn Leu Ser Ser Val Thr Pro
               580                 585                 590
<210> 93
   <211> 914
   <212> PRT
   <213> Homo sapiens
<400> 93
   Val Tyr Gln Val Leu Leu Val Gly Ser Thr Leu Leu Lys Glu Val Pro
   1 5 10 15
   Ser Gly Leu Gln Leu Glu Gln Leu Pro Ser Gln Ser Leu Leu Thr His
   20 25 30
   Ile Pro Thr Ala Gly Leu Pro Thr Ser Leu Gly Gly Gly Leu Pro Tyr
   35 40 45
   Cys His Gln Ala Trp Leu Asp Phe Arg Arg Arg Leu Glu Ala Leu Leu
   50 55 60
   Gln Asn Cys Gln Ala Ala Cys Ala Leu Leu Gln Gly Ala Ile Glu Ser
   65 70 75 80
   Val Lys Ala Val Pro Gln Pro Met Glu Pro Gly Glu Val Gly Gln Leu
   85 90 95
   Leu Gln Gln Thr Glu Val Leu Met Gln Gln Val Leu Asp Ser Pro Trp
   100 105 110
   Leu Ala Trp Leu Gln Cys Gln Gly Gly Arg Glu Leu Thr Trp Leu Lys
   115 120 125
   Gln Glu Val Pro Glu Val Thr Leu Ser Pro Asp Tyr Arg Thr Ala Met
   130 135 140
   Asp Lys Ala Asp Glu Leu Tyr Asp Arg Val Asp Gly Leu Leu His Gln
   145 150 155 160
   Leu Thr Leu Gln Ser Asn Gln Arg Ile Gln Ala Leu Glu Leu Val Gln
   165 170 175
   Thr Leu Glu Ala Arg Glu Ser Gly Leu His Gln Ile Glu Val Trp Leu
   180 185 190
   Gln Gln Val Gly Trp Pro Ala Leu Glu Glu Ala Gly Glu Pro Ser Leu
   195 200 205
   Asp Met Leu Leu Gln Ala Gln Gly Ser Phe Gln Glu Leu Tyr Gln Val
   210 215 220
   Ala Gln Glu Gln Val Arg Gln Gly Glu Lys Phe Leu Gln Pro Leu Thr
   225 230 235 240
   Gly Trp Glu Ala Ala Glu Leu Asp Pro Pro Gly Ala Arg Phe Leu Ala
   245 250 255
   Leu Arg Ala Gln Leu Thr Glu Phe Ser Arg Ala Leu Ala Gln Arg Cys
   260 265 270
   Gln Arg Leu Ala Asp Ala Glu Arg Leu Phe Gln Leu Phe Arg Glu Ala
   275 280 285
   Leu Thr Trp Ala Glu Glu Gly Gln Arg Val Leu Ala Glu Leu Glu Gln
   290 295 300
   Glu Arg Pro Gly Val Val Leu Gln Gln Leu Gln Leu His Trp Thr Arg
   305 310 315 320
   His Pro Asp Leu Pro Pro Ala His Phe Arg Lys Met Trp Ala Leu Ala
   325 330 335
   Thr Gly Leu Gly Ser Glu Ala Ile Arg Gln Glu Cys Arg Trp Ala Trp
   340 345 350
   Ala Arg Cys Gln Asp Thr Trp Leu Ala Leu Asp Gln Lys Leu Glu Ala
   355 360 365
   Ser Leu Lys Leu Pro Pro Val Gly Ser Thr Ala Ser Leu Cys Val Ser
   370 375 380
   Gln Val Pro Ala Ala Pro Ala His Pro Pro Leu Arg Lys Ala Tyr Ser
   385 390 395 400
   Phe Asp Arg Asn Leu Gly Gln Ser Leu Ser Glu Pro Ala Cys His Cys
   405 410 415
   His His Ala Ala Thr Ile Ala Ala Cys Arg Arg Pro Glu Ala Gly Gly
   420 425 430
   Gly Ala Leu Pro Gln Ala Ser Pro Thr Val Pro Pro Pro Gly Ser Ser
   435 440 445
   Asp Pro Arg Ser Leu Asn Arg Leu Gln Leu Val Leu Ala Glu Met Val
   450 455 460
   Ala Thr Glu Arg Glu Tyr Val Arg Ala Leu Glu Tyr Thr Met Glu Asn
   465 470 475 480
   Tyr Phe Pro Glu Leu Asp Arg Pro Asp Val Pro Gln Gly Leu Arg Gly
   485 490 495
   Gln Arg Ala His Leu Phe Gly Asn Leu Glu Lys Leu Arg Asp Phe His
   500 505 510
   Cys His Phe Phe Leu Arg Glu Leu Glu Ala Cys Thr Arg His Pro Pro
   515 520 525
   Arg Val Ala Tyr Ala Phe Leu Arg His Arg Val Gln Phe Gly Met Tyr
   530 535 540
   Ala Leu Tyr Ser Lys Asn Lys Pro Arg Ser Asp Ala Leu Met Ser Ser
   545 550 555 560
   Tyr Gly His Thr Phe Phe Lys Asp Lys Gln Gln Ala Leu Gly Asp His
   565 570 575
   Leu Asp Leu Ala Ser Tyr Leu Leu Lys Pro Ile Gln Arg Met Gly Lys
   580 585 590
   Tyr Ala Leu Leu Leu Gln Glu Leu Ala Arg Ala Cys Gly Gly Pro Thr
   595 600 605
   Gln Glu Leu Ser Ala Leu Arg Glu Ala Gln Ser Leu Val His Phe Gln
   610 615 620
   Leu Arg His Gly Asn Asp Leu Leu Ala Met Asp Ala Ile Gln Gly Cys
   625 630 635 640
   Asp Val Asn Leu Lys Glu Gln Gly Gln Leu Val Arg Gln Asp Glu Phe
   645 650 655
   Val Val Arg Thr Gly Arg His Lys Ser Val Arg Arg Ile Phe Leu Phe
   660 665 670
   Glu Glu Leu Leu Leu Phe Ser Lys Pro Arg His Gly Pro Thr Gly Val
   675 680 685
   Asp Thr Phe Ala Tyr Lys Arg Ser Phe Lys Met Ala Asp Leu Gly Leu
   690 695 700
   Thr Glu Cys Cys Gly Asn Ser Asn Leu Arg Phe Glu Ile Trp Phe Arg
   705 710 715 720
   Arg Arg Lys Ala Arg Asp Thr Phe Val Leu Gln Ala Ser Ser Leu Ala
   725 730 735
   Ile Lys Gln Ala Trp Thr Ala Asp Ile Ser His Leu Leu Trp Arg Gln
   740 745 750
   Ala Val His Asn Lys Glu Val Arg Met Ala Glu Met Val Ser Met Gly
   755 760 765
   Val Gly Asn Lys Ala Phe Arg Asp Ile Ala Pro Ser Glu Glu Ala Ile
   770 775 780
   Asn Asp Arg Thr Val Asn Tyr Val Leu Lys Cys Arg Glu Val Arg Ser
   785 790 795 800
   Arg Ala Ser Ile Ala Val Ala Pro Phe Asp His Asp Ser Leu Tyr Leu
   805 810 815
   Gly Ala Ser Asn Ser Leu Pro Gly Asp Pro Ala Ser Cys Ser Val Leu
   820 825 830
   Gly Ser Leu Asn Leu His Leu Tyr Arg Asp Pro Ala Leu Leu Gly Leu
   835 840 845
   Arg Cys Pro Leu Tyr Pro Ser Phe Leu Glu Glu Ala Ala Leu Glu Ala
   850 855 860
   Glu Ala Glu Leu Gly Gly Gln Pro Ser Leu Thr Ala Glu Asp Ser Glu
   865 870 875 880
   Ile Ser Ser Gln Cys Pro Ser Ala Ser Gly Ser Ser Gly Ser Asp Ser
   885 890 895
   Ser Cys Val Ser Gly Gln Ala Leu Gly Arg Gly Leu Glu Asp Leu Pro
   900 905 910
   Cys Val
<210> 94
   <211> 277
<210> 95
   <211> 1120
   <212> PRT
   <213> Homo sapiens
<400> 95
   Met Trp Arg Val Lys Lys Leu Ser Leu Ser Leu Ser Pro Ser Pro Gln
    1               5                  10                  15
   Thr Gly Lys Pro Ser Met Arg Thr Pro Leu Arg Glu Leu Thr Leu Gln
               20                  25                  30
   Pro Gly Ala Leu Thr Thr Ser Gly Lys Arg Ser Pro Ala Cys Ser Ser
           35                  40                  45
   Leu Thr Pro Ser Leu Cys Lys Leu Gly Leu Gln Glu Gly Ser Asn Asn
   50 55 60
   Ser Ser Pro Val Asp Phe Val Asn Asn Lys Arg Thr Asp Leu Ser Ser
   65 70 75 80
   Glu His Phe Ser His Ser Ser Lys Trp Leu Glu Thr Cys Gln His Glu
   85 90 95
   Ser Asp Glu Gln Pro Leu Asp Pro Ile Pro Gln Ile Ser Ser Thr Pro
   100 105 110
   Lys Thr Ser Glu Glu Ala Val Asp Pro Leu Gly Asn Tyr Met Val Lys
   115 120 125
   Thr Ile Val Leu Val Pro Ser Pro Leu Gly Gln Gln Gln Asp Met Ile
   130 135 140
   Phe Glu Ala Arg Leu Asp Thr Met Ala Glu Thr Asn Ser Ile Ser Leu
   145 150 155 160
   Asn Gly Pro Leu Arg Thr Asp Asp Leu Val Arg Glu Glu Val Ala Pro
   165 170 175
   Cys Met Gly Asp Arg Phe Ser Glu Val Ala Ala Val Ser Glu Lys Pro
   180 185 190
   Ile Phe Gln Glu Ser Pro Ser His Leu Leu Glu Glu Ser Pro Pro Asn
   195 200 205
   Pro Cys Ser Glu Gln Leu His Cys Ser Lys Glu Ser Leu Ser Ser Arg
   210 215 220
   Thr Glu Ala Val Arg Glu Asp Leu Val Pro Ser Glu Ser Asn Ala Phe
   225 230 235 240
   Leu Pro Ser Ser Val Leu Trp Leu Ser Pro Ser Thr Ala Leu Ala Ala
   245 250 255
   Asp Phe Arg Val Asn His Val Asp Pro Glu Glu Glu Ile Val Glu His
   260 265 270
   Gly Ala Met Glu Glu Arg Glu Met Arg Phe Pro Thr His Pro Lys Glu
   275 280 285
   Ser Glu Thr Glu Asp Gln Ala Leu Val Ser Ser Val Glu Asp Ile Leu
   290 295 300
   Ser Thr Cys Leu Thr Pro Asn Leu Val Glu Met Glu Ser Gln Glu Ala
   305 310 315 320
   Pro Gly Pro Ala Val Glu Asp Val Gly Arg Ile Leu Gly Ser Asp Thr
   325 330 335
   Glu Ser Trp Met Ser Pro Leu Ala Trp Leu Glu Lys Gly Val Asn Thr
   340 345 350
   Ser Val Met Leu Glu Asn Leu Arg Gln Ser Leu Ser Leu Pro Ser Met
   355 360 365
   Leu Arg Asp Ala Ala Ile Gly Thr Thr Pro Phe Ser Thr Cys Ser Val
   370 375 380
   Gly Thr Trp Phe Thr Pro Ser Ala Pro Gln Glu Lys Ser Thr Asn Thr
   385 390 395 400
   Ser Gln Thr Gly Leu Val Gly Thr Lys His Ser Thr Ser Glu Thr Glu
   405 410 415
   Gln Leu Leu Cys Gly Arg Pro Pro Asp Leu Thr Ala Leu Ser Arg His
   420 425 430
   Asp Leu Glu Asp Asn Leu Leu Ser Ser Leu Val Ile Val Glu Phe Leu
   435 440 445
   Ser Arg Gln Leu Arg Asp Trp Lys Ser Gln Leu Ala Val Pro His Pro
   450 455 460
   Glu Thr Gln Asp Ser Ser Thr Gln Thr Asp Thr Ser His Ser Gly Ile
   465                 470                 475                 480
   Thr Asn Lys Leu Gln His Leu Lys Glu Ser His Glu Met Gly Gln Ala
                   485                 490                 495
   Leu Gln Gln Ala Arg Asn Val Met Gln Ser Trp Val Leu Ile Ser Lys
               500                 505                 510
   Glu Leu Ile Ser Leu Leu His Leu Ser Leu Leu His Leu Glu Glu Asp
           515                 520                 525
   Lys Thr Thr Val Asn Gln Glu Ser Arg Arg Ala Glu Thr Leu Val Cys
       530                 535                 540
   Cys Cys Phe Asp Leu Leu Lys Lys Leu Arg Ala Lys Leu Gln Ser Leu
   545                 550                 555                 560
   Lys Ala Glu Arg Glu Glu Ala Arg His Arg Glu Glu Met Ala Leu Arg
                   565                 570                 575
   Gly Lys Asp Ala Ala Glu Ile Val Leu Glu Ala Phe Cys Ala His Ala
               580                 585                 590
   Ser Gln Arg Ile Ser Gln Leu Glu Gln Asp Leu Ala Ser Met Arg Glu
           595                 600                 605
   Phe Arg Gly Leu Leu Lys Asp Ala Gln Thr Gln Leu Val Gly Leu His
       610                 615                 620
   Ala Lys Gln Glu Glu Leu Val Gln Gln Thr Val Ser Leu Thr Ser Thr
   625                 630                 635                 640
   Leu Gln Gln Asp Trp Arg Ser Met Gln Leu Asp Tyr Thr Thr Trp Thr
                   645                 650                 655
   Ala Leu Leu Ser Arg Ser Arg Gln Leu Thr Glu Lys Leu Thr Val Lys
               660                 665                 670
   Ser Gln Gln Ala Leu Gln Glu Arg Asp Val Ala Ile Glu Glu Lys Gln
           675                 680                 685
   Glu Val Ser Arg Val Leu Glu Gln Val Ser Ala Gln Leu Glu Glu Cys
       690                 695                 700
   Lys Gly Gln Thr Glu Gln Leu Glu Leu Glu Asn Ile Arg Leu Ala Thr
   705                 710                 715                 720
   Asp Leu Arg Ala Gln Leu Gln Ile Leu Ala Asn Met Asp Ser Gln Leu
                   725                 730                 735
   Lys Glu Leu Gln Ser Gln His Thr His Cys Ala Gln Asp Leu Ala Met
               740                 745                 750
   Lys Asp Glu Leu Leu Cys Gln Leu Thr Gln Ser Asn Glu Glu Gln Ala
           755                 760                 765
   Ala Gln Cys Val Lys Glu Glu Met Ala Leu Lys His Met Gln Ala Glu
       770                 775                 780
   Leu Gln Gln Gln Gln Ala Val Leu Ala Lys Glu Val Arg Asp Leu Lys
   785                 790                 795                 800
   Glu Thr Leu Glu Phe Ala Asp Gln Glu Asn Gln Val Ala His Leu Glu
                   805                 810                 815
   Leu Gly Gln Val Glu Cys Gln Leu Lys Thr Thr Leu Glu Val Leu Arg
               820                 825                 830
   Glu Arg Ser Leu Gln Cys Glu Asn Leu Lys Asp Thr Val Glu Asn Leu
           835                 840                 845
   Thr Ala Lys Leu Ala Ser Thr Ile Ala Asp Asn Gln Glu Gln Asp Leu
       850                 855                 860
   Glu Lys Thr Arg Gln Tyr Ser Gln Lys Leu Gly Leu Leu Thr Glu Gln
   865                 870                 875                 880
   Leu Gln Ser Leu Thr Leu Phe Leu Gln Thr Lys Leu Lys Glu Lys Thr
                   885                 890                 895
   Glu Gln Glu Thr Leu Leu Leu Ser Thr Ala Cys Pro Pro Thr Gln Glu
   900 905 910
   His Pro Leu Pro Asn Asp Arg Thr Phe Leu Gly Ser Ile Leu Thr Ala
   915 920 925
   Val Ala Asp Glu Glu Pro Glu Ser Thr Pro Val Pro Leu Leu Gly Ser
   930 935 940
   Asp Lys Ser Ala Phe Thr Arg Val Ala Ser Met Val Ser Leu Gln Pro
   945 950 955 960
   Ala Glu Thr Pro Gly Met Glu Glu Ser Leu Ala Glu Met Ser Ile Met
   965 970 975
   Thr Thr Glu Leu Gln Ser Leu Cys Ser Leu Leu Gln Glu Ser Lys Glu
   980 985 990
   Glu Ala Ile Arg Thr Leu Gln Arg Lys Ile Cys Glu Leu Gln Ala Arg
   995 1000 1005
   Leu Gln Ala Gln Glu Glu Gln His Gln Glu Val Gln Lys Ala Lys Glu
   1010 1015 1020
   Ala Asp Ile Glu Lys Leu Asn Gln Ala Leu Cys Leu Arg Tyr Lys Asn
   1025 1030 1035 1040
   Glu Lys Glu Leu Gln Glu Val Ile Gln Gln Asn Glu Lys Ile Leu Glu
   1045 1050 1055
   Gln Ile Asp Lys Ser Gly Glu Leu Ile Ser Leu Arg Glu Glu Val Thr
   1060 1065 1070
   His Leu Thr Arg Ser Leu Arg Arg Ala Glu Thr Glu Thr Lys Val Leu
   1075 1080 1085
   Gln Glu Ala Trp Gln Ala Ser Trp Thr Pro Thr Ala Ser Leu Trp Pro
   1090 1095 1100
   Pro Ile Gly Ser Arg Arg Lys Cys Gly Ser Leu Arg Arg Trp Thr Asn
   1105 1110 1115 1120
<210> 96
   <211> 540
   <212> PRT
   <213> Homo sapiens
<400> 96
   Met Gly Thr Thr Ala Arg Ala Ala Leu Val Leu Thr Tyr Leu Ala Val
    1               5                  10                  15
   Ala Ser Ala Ala Ser Glu Gly Gly Phe Thr Ala Thr Gly Gln Arg Gln
               20                  25                  30
   Leu Arg Pro Glu His Phe Gln Glu Val Gly Tyr Ala Ala Pro Pro Ser
           35                  40                  45
   Pro Pro Leu Ser Arg Ser Leu Pro Met Asp His Pro Asp Ser Ser Gln
       50                  55                  60
   His Gly Pro Pro Phe Glu Gly Gln Ser Gln Val Gln Pro Pro Pro Ser
   65                  70                  75                  80
   Gln Glu Ala Thr Pro Leu Gln Gln Glu Lys Leu Leu Pro Ala Gln Leu
                   85                  90                  95
   Pro Ala Glu Lys Glu Val Gly Pro Pro Leu Pro Gln Glu Ala Val Pro
               100                 105                 110
   Leu Gln Lys Glu Leu Pro Ser Leu Gln His Pro Asn Glu Gln Lys Glu
           115                 120                 125
   Gly Thr Pro Ala Pro Phe Gly Asp Gln Ser His Pro Glu Pro Glu Ser
       130                 135                 140
   Trp Asn Ala Ala Gln His Cys Gln Gln Asp Arg Ser Gln Gly Gly Trp
   145                 150                 155                 160
   Gly His Arg Leu Asp Gly Phe Pro Pro Gly Arg Pro Ser Pro Asp Asn
                   165                 170                 175
   Leu Asn Gln Ile Cys Leu Pro Asn Arg Gln His Val Val Tyr Gly Pro
               180                 185                 190
   Trp Asn Leu Pro Gln Ser Ser Tyr Ser His Leu Thr Arg Gln Gly Glu
           195                 200                 205
   Thr Leu Asn Phe Leu Glu Ile Gly Tyr Ser Arg Cys Cys His Cys Arg
       210                 215                 220
   Ser His Thr Asn Arg Leu Glu Cys Ala Lys Leu Val Trp Glu Glu Ala
   225                 230                 235                 240
   Met Ser Arg Phe Cys Glu Ala Glu Phe Ser Val Lys Thr Arg Pro His
                   245                 250                 255
   Trp Cys Cys Thr Arg Gln Gly Glu Ala Arg Phe Ser Cys Phe Gln Glu
               260                 265                 270
   Glu Ala Pro Gln Pro His Tyr Gln Leu Arg Ala Cys Pro Ser His Gln
           275                 280                 285
   Pro Asp Ile Ser Ser Gly Leu Glu Leu Pro Phe Pro Pro Gly Val Pro
       290                 295                 300
   Thr Leu Asp Asn Ile Lys Asn Ile Cys His Leu Arg Arg Phe Arg Ser
   305                 310                 315                 320
   Val Pro Arg Asn Leu Pro Ala Thr Asp Pro Leu Gln Arg Glu Leu Leu
                   325                 330                 335
   Ala Leu Ile Gln Leu Glu Arg Glu Phe Gln Arg Cys Cys Arg Gln Gly
               340                 345                 350
   Asn Asn His Thr Cys Thr Trp Lys Ala Trp Glu Asp Thr Leu Asp Lys
           355                 360                 365
   Tyr Cys Asp Arg Glu Tyr Ala Val Lys Thr His His His Leu Cys Cys
       370                 375                 380
   Arg His Pro Pro Ser Pro Thr Arg Asp Glu Cys Phe Ala Arg Arg Ala
   385                 390                 395                 400
   Pro Tyr Pro Asn Tyr Asp Arg Asp Ile Leu Thr Ile Asp Ile Ser Arg
                   405                 410                 415
   Val Thr Pro Asn Leu Met Gly His Leu Cys Gly Asn Gln Arg Val Leu
               420                 425                 430
   Thr Lys His Lys His Ile Pro Gly Leu Ile His Asn Met Thr Ala Arg
           435                 440                 445
   Cys Cys Asp Leu Pro Phe Pro Glu Gln Ala Cys Cys Ala Glu Glu Glu
       450                 455                 460
   Lys Leu Thr Phe Ile Asn Asp Leu Cys Gly Pro Arg Arg Asn Ile Trp
   465                 470                 475                 480
   Arg Asp Pro Ala Leu Cys Cys Tyr Leu Ser Pro Gly Asp Glu Gln Val
                   485                 490                 495
   Asn Cys Phe Asn Ile Asn Tyr Leu Arg Asn Val Ala Leu Val Ser Gly
               500                 505                 510
   Asp Thr Glu Asn Ala Lys Gly Gln Gly Glu Gln Gly Ser Thr Gly Gly
           515                 520                 525
   Thr Asn Ile Ser Ser Thr Ser Glu Pro Lys Glu Glu
       530                 535                 540
<210> 97
   <211> 462
   <212> PRT
   <213> Homo sapiens
<400> 97
<210> 98
   <211> 2328
   <212> PRT
   <213> Homo sapiens
<400> 98
   Lys Ser Lys Arg Gln Ala Gln Gln Met Val Gln Pro Gln Ser Pro Val
   1               5                   10                  15
   Ala Val Ser Gln Ser Lys Pro Gly Cys Tyr Asp Asn Gly Lys His Tyr
               20                  25                  30
   Gln Ile Asn Gln Gln Trp Glu Arg Thr Tyr Leu Gly Asn Val Leu Val
           35                  40                  45
   Cys Thr Cys Tyr Gly Gly Ser Arg Gly Phe Asn Cys Glu Ser Lys Pro
       50                  55                  60
   Glu Ala Glu Glu Thr Cys Phe Asp Lys Tyr Thr Gly Asn Thr Tyr Arg
   65                  70                  75                  80
   Val Gly Asp Thr Tyr Glu Arg Pro Lys Asp Ser Met Ile Trp Asp Cys
                   85                  90                  95
   Thr Cys Ile Gly Ala Gly Arg Gly Arg Ile Ser Cys Thr Ile Ala Asn
               100                 105                 110
   Arg Cys His Glu Gly Gly Gln Ser Tyr Lys Ile Gly Asp Thr Trp Arg
           115                 120                 125
   Arg Pro His Glu Thr Gly Gly Tyr Met Leu Glu Cys Val Cys Leu Gly
       130                 135                 140
   Asn Gly Lys Gly Glu Trp Thr Cys Lys Pro Ile Ala Glu Lys Cys Phe
   145                 150                 155                 160
   Asp His Ala Ala Gly Thr Ser Tyr Val Val Gly Glu Thr Trp Glu Lys
                   165                 170                 175
   Pro Tyr Gln Gly Trp Met Met Val Asp Cys Thr Cys Leu Gly Glu Gly
               180                 185                 190
   Ser Gly Arg Ile Thr Cys Thr Ser Arg Asn Arg Cys Asn Asp Gln Asp
           195                 200                 205
   Thr Arg Thr Ser Tyr Arg Ile Gly Asp Thr Trp Ser Lys Lys Asp Asn
       210                 215                 220
   Arg Gly Asn Leu Leu Gln Cys Ile Cys Thr Gly Asn Gly Arg Gly Glu
   225                 230                 235                 240
   Trp Lys Cys Glu Arg His Thr Ser Val Gln Thr Thr Ser Ser Gly Ser
                   245                 250                 255
   Gly Pro Phe Thr Asp Val Arg Ala Ala Val Tyr Gln Pro Gln Pro His
               260                 265                 270
   Pro Gln Pro Pro Pro Tyr Gly His Cys Val Thr Asp Ser Gly Val Val
           275                 280                 285
   Tyr Ser Val Gly Met Gln Trp Leu Lys Thr Gln Gly Asn Lys Gln Met
       290                 295                 300
   Leu Cys Thr Cys Leu Gly Asn Gly Val Ser Cys Gln Glu Thr Ala Val
   305                 310                 315                 320
   Thr Gln Thr Tyr Gly Gly Asn Leu Asn Gly Glu Pro Cys Val Leu Pro
                   325                 330                 335
   Phe Thr Tyr Asn Gly Arg Thr Phe Tyr Ser Cys Thr Thr Glu Gly Arg
               340                 345                 350
   Gln Asp Gly His Leu Trp Cys Ser Thr Thr Ser Asn Tyr Glu Gln Asp
           355                 360                 365
   Gln Lys Tyr Ser Phe Cys Thr Asp His Thr Val Leu Val Gln Thr Gln
       370                 375                 380
   Gly Gly Asn Ser Asn Gly Ala Leu Cys His Phe Pro Phe Leu Tyr Asn
   385                 390                 395                 400
   Asn His Asn Tyr Thr Asp Cys Thr Ser Glu Gly Arg Arg Asp Asn Met
                   405                 410                 415
   Lys Trp Cys Gly Thr Thr Gln Asn Tyr Asp Ala Asp Gln Lys Phe Gly
               420                 425                 430
   Phe Cys Pro Met Ala Ala His Glu Glu Ile Cys Thr Thr Asn Glu Gly
           435                 440                 445
   Val Met Tyr Arg Ile Gly Asp Gln Trp Asp Lys Gln His Asp Met Gly
       450                 455                 460
   His Met Met Arg Cys Thr Cys Val Gly Asn Gly Arg Gly Glu Trp Thr
   465                 470                 475                 480
   Cys Ile Ala Tyr Ser Gln Leu Arg Asp Gln Cys Ile Val Asp Asp Ile
                   485                 490                 495
   Thr Tyr Asn Val Asn Asp Thr Phe His Lys Arg His Glu Glu Gly His
               500                 505                 510
   Met Leu Asn Cys Thr Cys Phe Gly Gln Gly Arg Gly Arg Trp Lys Cys
           515                 520                 525
   Asp Pro Val Asp Gln Cys Gln Asp Ser Glu Thr Gly Thr Phe Tyr Gln
       530                 535                 540
   Ile Gly Asp Ser Trp Glu Lys Tyr Val His Gly Val Arg Tyr Gln Cys
   545                 550                 555                 560
   Tyr Cys Tyr Gly Arg Gly Ile Gly Glu Trp His Cys Gln Pro Leu Gln
                   565                 570                 575
   Thr Tyr Pro Ser Ser Ser Gly Pro Val Glu Val Phe Ile Thr Glu Thr
               580                 585                 590
   Pro Ser Gln Pro Asn Ser His Pro Ile Gln Trp Asn Ala Pro Gln Pro
           595                 600                 605
   Ser His Ile Ser Lys Tyr Ile Leu Arg Trp Arg Pro Lys Asn Ser Val
       610                 615                 620
   Gly Arg Trp Lys Glu Ala Thr Ile Pro Gly His Leu Asn Ser Tyr Thr
   625                 630                 635                 640
   Ile Lys Gly Leu Lys Pro Gly Val Val Tyr Glu Gly Gln Leu Ile Ser
                   645                 650                 655
   Ile Gln Gln Tyr Gly His Gln Glu Val Thr Arg Phe Asp Phe Thr Thr
               660                 665                 670
   Thr Ser Thr Ser Thr Pro Val Thr Ser Asn Thr Val Thr Gly Glu Thr
           675                 680                 685
   Thr Pro Phe Ser Pro Leu Val Ala Thr Ser Glu Ser Val Thr Glu Ile
       690                 695                 700
   Thr Ala Ser Ser Phe Val Val Ser Trp Val Ser Ala Ser Asp Thr Val
   705                 710                 715                 720
   Ser Gly Phe Arg Val Glu Tyr Glu Leu Ser Glu Glu Gly Asp Glu Pro
                   725                 730                 735
   Gln Tyr Leu Asp Leu Pro Ser Thr Ala Thr Ser Val Asn Ile Pro Asp
               740                 745                 750
   Leu Leu Pro Gly Arg Lys Tyr Ile Val Asn Val Tyr Gln Ile Ser Glu
           755                 760                 765
   Asp Gly Glu Gln Ser Leu Ile Leu Ser Thr Ser Gln Thr Thr Ala Pro
       770                 775                 780
   Asp Ala Pro Pro Asp Pro Thr Val Asp Gln Val Asp Asp Thr Ser Ile
   785                 790                 795                 800
   Val Val Arg Trp Ser Arg Pro Gln Ala Pro Ile Thr Gly Tyr Arg Ile
                   805                 810                 815
   Val Tyr Ser Pro Ser Val Glu Gly Ser Ser Thr Glu Leu Asn Leu Pro
               820                 825                 830
   Glu Thr Ala Asn Ser Val Thr Leu Ser Asp Leu Gln Pro Gly Val Gln
           835                 840                 845
   Tyr Asn Ile Thr Ile Tyr Ala Val Glu Glu Asn Gln Glu Ser Thr Pro
       850                 855                 860
   Val Val Ile Gln Gln Glu Thr Thr Gly Thr Pro Arg Ser Asp Thr Val
   865                 870                 875                 880
   Pro Ser Pro Arg Asp Leu Gln Phe Val Glu Val Thr Asp Val Lys Val
                   885                 890                 895
   Thr Ile Met Trp Thr Pro Pro Glu Ser Ala Val Thr Gly Tyr Arg Val
               900                 905                 910
   Asp Val Ile Pro Val Asn Leu Pro Gly Glu His Gly Gln Arg Leu Pro
           915                 920                 925
   Ile Ser Arg Asn Thr Phe Ala Glu Val Thr Gly Leu Ser Pro Gly Val
       930                 935                 940
   Thr Tyr Tyr Phe Lys Val Phe Ala Val Ser His Gly Arg Glu Ser Lys
   945                 950                 955                 960
   Pro Leu Thr Ala Gln Gln Thr Thr Lys Leu Asp Ala Pro Thr Asn Leu
                   965                 970                 975
   Gln Phe Val Asn Glu Thr Asp Ser Thr Val Leu Val Arg Trp Thr Pro
               980                 985                 990
   Pro Arg Ala Gln Ile Thr Gly Tyr Arg Leu Thr Val Gly Leu Thr Arg
           995                 1000                1005
   Arg Gly Gln Pro Arg Gln Tyr Asn Val Gly Pro Ser Val Ser Lys Tyr
       1010                1015                1020
   Pro Leu Arg Asn Leu Gln Pro Ala Ser Glu Tyr Thr Val Ser Leu Val
   1025                1030                1035                1040
   Ala Ile Lys Gly Asn Gln Glu Ser Pro Lys Ala Thr Gly Val Phe Thr
                   1045                1050                1055
   Thr Leu Gln Pro Gly Ser Ser Ile Pro Pro Tyr Asn Thr Glu Val Thr
               1060                1065                1070
   Glu Thr Thr Ile Val Ile Thr Trp Thr Pro Ala Pro Arg Ile Gly Phe
           1075                1080                1085
   Lys Leu Gly Val Arg Pro Ser Gln Gly Gly Glu Ala Pro Arg Glu Val
       1090                1095                1100
   Thr Ser Asp Ser Gly Ser Ile Val Val Ser Gly Leu Thr Pro Gly Val
   1105                1110                1115                1120
   Glu Tyr Val Tyr Thr Ile Gln Val Leu Arg Asp Gly Gln Glu Arg Asp
                   1125                1130                1135
   Ala Pro Ile Val Asn Lys Val Val Thr Pro Leu Ser Pro Pro Thr Asn
   1140 1145 1150
   Leu His Leu Glu Ala Asn Pro Asp Thr Gly Val Leu Thr Val Ser Trp
   1155 1160 1165
   Glu Arg Ser Thr Thr Pro Asp Ile Thr Gly Tyr Arg Ile Thr Thr Thr
   1170 1175 1180
   Pro Thr Asn Gly Gln Gln Gly Asn Ser Leu Glu Glu Val Val His Ala
   1185 1190 1195 1200
   Asp Gln Ser Ser Cys Thr Phe Asp Asn Leu Ser Pro Gly Leu Glu Tyr
   1205 1210 1215
   Asn Val Ser Val Tyr Thr Val Lys Asp Asp Lys Glu Ser Val Pro Ile
   1220 1225 1230
   Ser Asp Thr Ile Ile Pro Ala Val Pro Pro Pro Thr Asp Leu Arg Phe
   1235 1240 1245
   Thr Asn Ile Gly Pro Asp Thr Met Arg Val Thr Trp Ala Pro Pro Pro
   1250 1255 1260
   Ser Ile Asp Leu Thr Asn Phe Leu Val Arg Tyr Ser Pro Val Lys Asn
   1265 1270 1275 1280
   Glu Glu Asp Val Ala Glu Leu Ser Ile Ser Pro Ser Asp Asn Ala Val
   1285 1290 1295
   Val Leu Thr Asn Leu Leu Pro Gly Thr Glu Tyr Val Val Ser Val Ser
   1300 1305 1310
   Ser Val Tyr Glu Gln His Glu Ser Thr Pro Leu Arg Gly Arg Gln Lys
   1315 1320 1325
   Thr Gly Leu Asp Ser Pro Thr Gly Ile Asp Phe Ser Asp Ile Thr Ala
   1330 1335 1340
   Asn Ser Phe Thr Val His Trp Ile Ala Pro Arg Ala Thr Ile Thr Gly
   1345 1350 1355 1360
   Tyr Arg Ile Arg His His Pro Glu His Phe Ser Gly Arg Pro Arg Glu
   1365 1370 1375
   Asp Arg Val Pro His Ser Arg Asn Ser Ile Thr Leu Thr Asn Leu Thr
   1380 1385 1390
   Pro Gly Thr Glu Tyr Val Val Ser Ile Val Ala Leu Asn Gly Arg Glu
   1395 1400 1405
   Glu Ser Pro Leu Leu Ile Gly Gln Gln Ser Thr Val Ser Asp Val Pro
   1410 1415 1420
   Arg Asp Leu Glu Val Val Ala Ala Thr Pro Thr Ser Leu Leu Ile Ser
   1425 1430 1435 1440
   Trp Asp Ala Pro Ala Val Thr Val Arg Tyr Tyr Arg Ile Thr Tyr Gly
   1445 1450 1455
   Glu Thr Gly Gly Asn Ser Pro Val Gln Glu Phe Thr Val Pro Gly Ser
   1460 1465 1470
   Lys Ser Thr Ala Thr Ile Ser Gly Leu Lys Pro Gly Val Asp Tyr Thr
   1475 1480 1485
   Ile Thr Val Tyr Ala Val Thr Gly Arg Gly Asp Ser Pro Ala Ser Ser
   1490 1495 1500
   Lys Pro Ile Ser Ile Asn Tyr Arg Thr Glu Ile Asp Lys Pro Ser Gln
   1505 1510 1515 1520
   Met Gln Val Thr Asp Val Gln Asp Asn Ser Ile Ser Val Lys Trp Leu
   1525 1530 1535
   Pro Ser Ser Ser Pro Val Thr Gly Tyr Arg Val Thr Thr Thr Pro Lys
   1540 1545 1550
   Asn Gly Pro Gly Pro Thr Lys Thr Lys Thr Ala Gly Pro Asp Gln Thr
   1555 1560 1565
   Glu Met Thr Ile Glu Gly Leu Gln Pro Thr Val Glu Tyr Val Val Ser
   1570 1575 1580
   Val Tyr Ala Gln Asn Pro Ser Gly Glu Ser Gln Pro Leu Val Gln Thr
   1585 1590 1595 1600
   Ala Val Thr Asn Ile Asp Arg Pro Lys Gly Leu Ala Phe Thr Asp Val
   1605 1610 1615
   Asp Val Asp Ser Ile Lys Ile Ala Trp Glu Ser Pro Gln Gly Gln Val
   1620 1625 1630
   Ser Arg Tyr Arg Val Thr Tyr Ser Ser Pro Glu Asp Gly Ile His Glu
   1635 1640 1645
   Leu Phe Pro Ala Pro Asp Gly Glu Glu Asp Thr Ala Glu Leu Gln Gly
   1650 1655 1660
   Leu Arg Pro Gly Ser Glu Tyr Thr Val Ser Val Val Ala Leu His Asp
   1665 1670 1675 1680
   Asp Met Glu Ser Gln Pro Leu Ile Gly Thr Gln Ser Thr Ala Ile Pro
   1685 1690 1695
   Ala Pro Thr Asp Leu Lys Phe Thr Gln Val Thr Pro Thr Ser Leu Ser
   1700 1705 1710
   Ala Gln Trp Thr Pro Pro Asn Val Gln Leu Thr Gly Tyr Arg Val Arg
   1715 1720 1725
   Val Thr Pro Lys Glu Lys Thr Gly Pro Met Lys Glu Ile Asn Leu Ala
   1730 1735 1740
   Pro Asp Ser Ser Ser Val Val Val Ser Gly Leu Met Val Ala Thr Lys
   1745 1750 1755 1760
   Tyr Glu Val Ser Val Tyr Ala Leu Lys Asp Thr Leu Thr Ser Arg Pro
   1765 1770 1775
   Ala Gln Gly Val Val Thr Thr Leu Glu Asn Val Ser Pro Pro Arg Arg
   1780 1785 1790
   Ala Arg Val Thr Asp Ala Thr Glu Thr Thr Ile Thr Ile Ser Trp Arg
   1795 1800 1805
   Thr Lys Thr Glu Thr Ile Thr Gly Phe Gln Val Asp Ala Val Pro Ala
   1810 1815 1820
   Asn Gly Gln Thr Pro Ile Gln Arg Thr Ile Lys Pro Asp Val Arg Ser
   1825 1830 1835 1840
   Tyr Thr Ile Thr Gly Leu Gln Pro Gly Thr Asp Tyr Lys Ile Tyr Leu
   1845 1850 1855
   Tyr Thr Leu Asn Asp Asn Ala Arg Ser Ser Pro Val Val Ile Asp Ala
   1860 1865 1870
   Ser Thr Ala Ile Asp Ala Pro Ser Asn Leu Arg Phe Leu Ala Thr Thr
   1875 1880 1885
   Pro Asn Ser Leu Leu Val Ser Trp Gln Pro Pro Arg Ala Arg Ile Thr
   1890 1895 1900
   Gly Tyr Ile Ile Lys Tyr Glu Lys Pro Gly Ser Pro Pro Arg Glu Val
   1905 1910 1915 1920
   Val Pro Arg Pro Arg Pro Gly Val Thr Glu Ala Thr Ile Thr Gly Leu
   1925 1930 1935
   Glu Pro Gly Thr Glu Tyr Thr Ile Tyr Val Ile Ala Leu Lys Asn Asn
   1940 1945 1950
   Gln Lys Ser Glu Pro Leu Ile Gly Arg Lys Lys Thr Asp Glu Leu Pro
   1955 1960 1965
   Gln Leu Val Thr Leu Pro His Pro Asn Leu His Gly Pro Glu Ile Leu
   1970 1975 1980
   Asp Val Pro Ser Thr Val Gln Lys Thr Pro Phe Val Thr His Pro Gly
   1985                1990                1995                2000
   Tyr Asp Thr Gly Asn Gly Ile Gln Leu Pro Gly Thr Ser Gly Gln Gln
                   2005                2010                2015
   Pro Ser Val Gly Gln Gln Met Ile Phe Glu Glu His Gly Phe Arg Arg
               2020                2025                2030
   Thr Thr Pro Pro Thr Thr Ala Thr Pro Ile Arg His Arg Pro Arg Pro
           2035                2040                2045
   Tyr Pro Pro Asn Val Gly Gln Glu Ala Leu Ser Gln Thr Thr Ile Ser
       2050                2055                2060
   Trp Ala Pro Phe Gln Asp Thr Ser Glu Tyr Ile Ile Ser Cys His Pro
   2065                2070                2075                2080
   Val Gly Thr Asp Glu Glu Pro Leu Gln Phe Arg Val Pro Gly Thr Ser
                   2085                2090                2095
   Thr Ser Ala Thr Leu Thr Gly Leu Thr Arg Gly Ala Thr Tyr Asn Ile
               2100                2105                2110
   Ile Val Glu Ala Leu Lys Asp Gln Gln Arg His Lys Val Arg Glu Glu
           2115                2120                2125
   Val Val Thr Val Gly Asn Ser Val Asn Glu Gly Leu Asn Gln Pro Thr
       2130                2135                2140
   Asp Asp Ser Cys Phe Asp Pro Tyr Thr Val Ser His Tyr Ala Val Gly
   2145                2150                2155                2160
   Asp Glu Trp Glu Arg Met Ser Glu Ser Gly Phe Lys Leu Leu Cys Gln
                   2165                2170                2175
   Cys Leu Gly Phe Gly Ser Gly His Phe Arg Cys Asp Ser Ser Arg Trp
               2180                2185                2190
   Cys His Asp Asn Gly Val Asn Tyr Lys Ile Gly Glu Lys Trp Asp Arg
           2195                2200                2205
   Gln Gly Glu Asn Gly Gln Met Met Ser Cys Thr Cys Leu Gly Asn Gly
       2210                2215                2220
   Lys Gly Glu Phe Lys Cys Asp Pro His Glu Ala Thr Cys Tyr Asp Asp
   2225                2230                2235                2240
   Gly Lys Thr Tyr His Val Gly Glu Gln Trp Gln Lys Glu Tyr Leu Gly
                   2245                2250                2255
   Ala Ile Cys Ser Cys Thr Cys Phe Gly Gly Gln Arg Gly Trp Arg Cys
               2260                2265                2270
   Asp Asn Cys Arg Arg Pro Gly Gly Glu Pro Ser Pro Glu Gly Thr Thr
           2275                2280                2285
   Gly Gln Ser Tyr Asn Gln Tyr Ser Gln Arg Tyr His Gln Arg Thr Asn
       2290                2295                2300
   Thr Asn Val Asn Cys Pro Ile Glu Cys Phe Met Pro Leu Asp Val Gln
   2305                2310                2315                2320
   Ala Asp Arg Glu Asp Ser Arg Glu
                   2325
<210> 99
   <211> 188
   <212> PRT
   <213> Homo sapiens
<400> 99
   His Gln Thr His Lys Glu Gly Gly Ser Thr His Ala Ser Ala Asp Ala
   1 5 10 15
   Trp Glu Ile Ile Glu Leu Glu Thr Glu Ile Glu Lys Phe Lys Ala Glu
   20 25 30
   Asn Ala Ser Leu Ala Lys Leu Arg Ile Glu Arg Glu Ser Ala Leu Glu
   35 40 45
   Lys Leu Arg Lys Glu Ile Ala Asp Phe Glu Gln Gln Lys Ala Lys Glu
   50 55 60
   Leu Ala Arg Ile Glu Glu Phe Lys Lys Glu Glu Met Arg Lys Leu Gln
   65 70 75 80
   Lys Glu Arg Lys Val Phe Glu Lys Tyr Thr Thr Ala Ala Arg Thr Phe
   85 90 95
   Pro Asp Lys Lys Glu Arg Glu Glu Ile Gln Thr Leu Lys Gln Gln Ile
   100 105 110
   Ala Asp Leu Arg Glu Asp Leu Lys Arg Lys Glu Thr Lys Trp Ser Ser
   115 120 125
   Thr His Ser Arg Leu Arg Ser Gln Ile Gln Met Leu Val Arg Glu Asn
   130 135 140
   Thr Asp Leu Arg Glu Glu Ile Lys Val Met Glu Arg Phe Arg Leu Asp
   145 150 155 160
   Ala Trp Lys Arg Ala Glu Ala Ile Glu Ser Ser Leu Glu Val Glu Lys
   165 170 175
   Lys Asp Lys Leu Ala Asn Thr Ser Val Arg Phe Gln
   180 185
<210> 100
   <211> 284
   <212> PRT
   <213> Homo sapiens
<400> 100
   Met Glu Pro Gly Asn Tyr Ala Thr Leu Asp Gly Ala Lys Asp Ile Glu
   1 5 10 15
   Gly Leu Leu Gly Ala Gly Gly Gly Arg Asn Leu Val Ala His Ser Pro
   20 25 30
   Leu Thr Ser His Pro Ala Ala Pro Thr Leu Met Pro Ala Val Asn Tyr
   35 40 45
   Ala Pro Leu Asp Leu Pro Gly Ser Ala Glu Pro Pro Lys Gln Cys His
   50 55 60
   Pro Cys Pro Gly Val Pro Gln Gly Thr Ser Pro Ala Pro Val Pro Tyr
   65 70 75 80
   Gly Tyr Phe Gly Gly Gly Tyr Tyr Ser Cys Arg Val Ser Arg Ser Ser
   85 90 95
   Leu Lys Pro Cys Ala Gln Ala Ala Thr Leu Ala Ala Tyr Pro Ala Glu
   100 105 110
   Thr Pro Thr Ala Gly Glu Glu Tyr Pro Ser Arg Pro Thr Glu Phe Ala
   115 120 125
   Phe Tyr Pro Gly Tyr Pro Gly Thr Tyr His Ala Met Ala Ser Tyr Leu
   130 135 140
   Asp Val Ser Val Val Gln Thr Leu Gly Ala Pro Gly Glu Pro Arg His
   145 150 155 160
   Asp Ser Leu Leu Pro Val Asp Ser Tyr Gln Ser Trp Ala Leu Ala Gly
   165 170 175
   Gly Trp Asn Ser Gln Met Cys Cys Gln Gly Glu Gln Asn Pro Pro Gly
   180 185 190
   Pro Phe Trp Lys Ala Ala Phe Ala Asp Ser Ser Gly Gln His Pro Pro
   195 200 205
   Asp Ala Cys Ala Phe Arg Arg Gly Arg Lys Lys Arg Ile Pro Tyr Ser
   210 215 220
   Lys Gly Gln Leu Arg Glu Leu Glu Arg Glu Tyr Ala Ala Asn Lys Phe
   225 230 235 240
   Ile Thr Lys Asp Lys Arg Arg Lys Ile Ser Ala Ala Thr Ser Leu Ser
   245 250 255
   Glu Arg Gln Ile Thr Ile Trp Phe Gln Asn Arg Arg Val Lys Glu Lys
   260 265 270
   Lys Val Leu Ala Lys Val Lys Asn Ser Ala Thr Pro
   275 280
<210> 101
   <211> 676
   <212> PRT
   <213> Homo sapiens
<400> 101
   Met Asp Lys Tyr Asp Asp Leu Gly Leu Glu Ala Ser Lys Phe Ile Glu
   1 5 10 15
   Asp Leu Asn Met Tyr Glu Ala Ser Lys Asp Gly Leu Phe Arg Val Asp
   20 25 30
   Lys Gly Ala Gly Asn Asn Pro Glu Phe Glu Glu Thr Arg Arg Val Phe
   35 40 45
   Ala Thr Lys Met Ala Lys Ile His Leu Gln Gln Gln Gln Gln Gln Leu
   50 55 60
   Leu Gln Glu Glu Thr Leu Pro Arg Gly Ser Arg Gly Pro Val Asn Gly
   65 70 75 80
   Gly Gly Arg Leu Gly Pro Gln Ala Arg Trp Glu Val Val Gly Ser Lys
   85 90 95
   Leu Thr Val Asp Gly Ala Ala Lys Pro Pro Leu Ala Ala Ser Thr Gly
   100 105 110
   Ala Pro Gly Ala Val Thr Thr Leu Ala Ala Gly Gln Pro Pro Tyr Pro
   115 120 125
   Pro Gln Glu Gln Arg Ser Arg Pro Tyr Leu His Gly Thr Arg His Gly
   130 135 140
   Ser Gln Asp Cys Gly Ser Arg Glu Ser Leu Ala Thr Ser Glu Met Ser
   145 150 155 160
   Ala Phe His Gln Pro Gly Pro Cys Glu Asp Pro Ser Cys Leu Thr His
   165 170 175
   Gly Asp Tyr Tyr Asp Asn Leu Ser Leu Ala Ser Pro Lys Trp Gly Asp
   180 185 190
   Lys Pro Gly Val Ser Pro Ser Ile Gly Leu Ser Val Gly Ser Gly Trp
   195 200 205
   Pro Ser Ser Pro Gly Ser Asp Pro Pro Leu Pro Lys Pro Cys Gly Asp
   210 215 220
   His Pro Leu Asn His Arg Gln Leu Ser Leu Ser Ser Ser Arg Ser Ser
   225 230 235 240
   Glu Gly Ser Leu Gly Gly Gln Asn Ser Gly Ile Gly Gly Arg Ser Ser
   245 250 255
   Glu Lys Pro Thr Gly Leu Trp Ser Thr Ala Ser Ser Gln Arg Val Ser
   260 265 270
   Pro Gly Leu Pro Ser Pro Asn Leu Glu Asn Gly Ala Pro Ala Val Gly
           275                 280                 285
   Pro Val Gln Pro Arg Thr Pro Ser Val Ser Ala Pro Leu Ala Leu Ser
       290                 295                 300
   Cys Pro Arg Gln Gly Gly Leu Pro Arg Ser Asn Ser Gly Leu Gly Gly
   305                 310                 315                 320
   Glu Val Ser Gly Val Met Ser Lys Pro Asn Val Asp Pro Gln Pro Trp
                   325                 330                 335
   Phe Gln Asp Gly Pro Lys Ser Tyr Leu Ser Ser Ser Ala Pro Ser Ser
               340                 345                 350
   Ser Pro Ala Gly Leu Asp Gly Ser Gln Gln Gly Ala Val Pro Gly Leu
           355                 360                 365
   Gly Pro Lys Pro Gly Cys Thr Asp Leu Gly Thr Gly Pro Lys Leu Ser
       370                 375                 380
   Pro Thr Ser Leu Val His Pro Val Met Ser Thr Leu Pro Glu Leu Ser
   385                 390                 395                 400
   Cys Lys Glu Gly Pro Leu Gly Trp Ser Ser Asp Gly Ser Leu Gly Ser
                   405                 410                 415
   Val Leu Leu Asp Ser Pro Ser Ser Pro Arg Val Arg Leu Pro Cys Gln
               420                 425                 430
   Pro Leu Val Pro Gly Pro Glu Leu Arg Pro Ser Ala Ala Glu Leu Lys
           435                 440                 445
   Leu Glu Ala Leu Thr Gln Arg Leu Glu Arg Glu Met Asp Ala His Pro
       450                 455                 460
   Lys Ala Asp Tyr Phe Gly Ala Cys Val Lys Cys Ser Lys Gly Val Phe
   465                 470                 475                 480
   Gly Ala Gly Gln Ala Cys Gln Ala Met Gly Asn Leu Tyr His Asp Thr
                   485                 490                 495
   Cys Phe Thr Cys Ala Ala Cys Ser Arg Lys Leu Arg Gly Lys Ala Phe
               500                 505                 510
   Tyr Phe Val Asn Gly Lys Val Phe Cys Glu Glu Asp Phe Leu Tyr Ser
           515                 520                 525
   Gly Phe Gln Gln Ser Ala Asp Arg Cys Phe Leu Cys Gly His Leu Ile
       530                 535                 540
   Met Asp Met Ile Leu Gln Ala Leu Gly Lys Ser Tyr His Pro Gly Cys
   545                 550                 555                 560
   Phe Arg Cys Val Ile Cys Asn Glu Cys Leu Asp Gly Val Pro Phe Thr
                   565                 570                 575
   Val Asp Ser Glu Asn Lys Ile Tyr Cys Val Arg Asp Tyr His Lys Val
               580                 585                 590
   Leu Ala Pro Lys Cys Ala Ala Cys Gly Leu Pro Ile Leu Pro Pro Glu
           595                 600                 605
   Gly Ser Asp Glu Thr Ile Arg Val Val Ser Met Asp Arg Asp Tyr His
       610                 615                 620
   Val Glu Cys Tyr His Cys Glu Asp Cys Gly Leu Glu Leu Asn Asp Glu
   625                 630                 635                 640
   Asp Gly His Arg Cys Tyr Pro Leu Glu Asp His Leu Phe Cys His Ser
                   645                 650                 655
   Cys His Val Lys Arg Leu Glu Lys Arg Pro Ser Ser Thr Ala Leu His
               660                 665                 670
   Gln His His Phe
           675
<210> 102
   <211> 296
   <212> PRT
   <213> Homo sapiens
<400> 102
   Ser Thr Gly Ser Glu Phe Pro Leu Cys Thr Lys Ala Ser Pro Cys Ser
   1 5 10 15
   Ala Ala Arg Ala Gly Gly Arg Ala Leu Gly Trp Arg Leu Gln Gln Gln
   20 25 30
   Arg Glu Thr Arg Gly Asn Pro Gly Asn Pro Gly Leu Gly Val Ala Ala
   35 40 45
   Thr Met Thr Gly Ser Asn Met Ser Asp Ala Leu Ala Asn Ala Val Cys
   50 55 60
   Gln Arg Cys Gln Ala Arg Phe Ser Pro Ala Glu Arg Ile Val Asn Ser
   65 70 75 80
   Asn Gly Glu Leu Tyr His Glu His Cys Phe Val Cys Ala Gln Cys Phe
   85 90 95
   Arg Pro Phe Pro Glu Gly Leu Phe Tyr Glu Phe Glu Gly Arg Lys Tyr
   100 105 110
   Cys Glu His Asp Phe Gln Met Leu Phe Ala Pro Cys Cys Gly Ser Cys
   115 120 125
   Gly Glu Phe Ile Ile Gly Arg Val Ile Lys Ala Met Asn Asn Asn Trp
   130 135 140
   His Pro Gly Cys Phe Arg Cys Glu Leu Cys Asp Val Glu Leu Ala Asp
   145 150 155 160
   Leu Gly Phe Val Lys Asn Ala Gly Arg His Leu Cys Arg Pro Cys His
   165 170 175
   Asn Arg Glu Lys Ala Lys Gly Leu Gly Lys Tyr Ile Cys Gln Arg Cys
   180 185 190
   His Leu Val Ile Asp Glu Gln Pro Leu Met Phe Arg Ser Asp Ala Tyr
   195 200 205
   His Pro Asp His Phe Asn Cys Thr His Cys Gly Lys Glu Leu Thr Ala
   210 215 220
   Glu Ala Arg Glu Leu Lys Gly Glu Leu Tyr Cys Leu Pro Cys His Asp
   225 230 235 240
   Lys Met Gly Val Pro Ile Cys Gly Ala Cys Arg Arg Pro Ile Glu Gly
   245 250 255
   Arg Val Val Asn Ala Leu Gly Lys Gln Trp His Val Glu His Phe Val
   260 265 270
   Cys Ala Lys Cys Glu Lys Pro Phe Leu Gly His Arg His Tyr Glu Lys
   275 280 285
   Lys Gly Leu Ala Tyr Cys Glu Leu
   290 295
<210> 103
   <211> 500
   <212> PRT
   <213> Homo sapiens
<400> 103
   Met Gly Ile Gly Leu Ser Ala Gln Gly Val Asn Met Asn Arg Leu Pro
   1 5 10 15
   Gly Trp Asp Lys His Ser Tyr Gly Tyr His Gly Asp Asp Gly His Ser
   20 25 30
   Phe Cys Ser Ser Gly Thr Gly Gln Pro Tyr Gly Pro Thr Phe Thr Thr
   35 40 45
   Gly Asp Val Ile Gly Cys Cys Val Asn Leu Ile Asn Asn Thr Cys Phe
   50 55 60
   Tyr Thr Lys Asn Gly His Ser Leu Gly Ile Ala Phe Thr Asp Leu Pro
   65 70 75 80
   Pro Asn Leu Tyr Pro Thr Val Gly Leu Gln Thr Pro Gly Glu Val Val
   85 90 95
   Asp Ala Asn Phe Gly Gln His Pro Phe Val Phe Asp Ile Glu Asp Tyr
   100 105 110
   Met Arg Glu Trp Arg Thr Lys Ile Gln Ala Gln Ile Asp Arg Phe Pro
   115 120 125
   Ile Gly Asp Arg Glu Gly Glu Trp Gln Thr Met Ile Gln Lys Met Val
   130 135 140
   Ser Ser Tyr Leu Val His His Gly Tyr Cys Ala Thr Ala Glu Ala Phe
   145 150 155 160
   Ala Arg Ser Thr Asp Gln Thr Val Leu Glu Glu Leu Ala Ser Ile Lys
   165 170 175
   Asn Arg Gln Arg Ile Gln Lys Leu Val Leu Ala Gly Arg Met Gly Glu
   180 185 190
   Ala Ile Glu Thr Thr Gln Gln Leu Tyr Pro Ser Leu Leu Glu Arg Asn
   195 200 205
   Pro Asn Leu Leu Phe Thr Leu Lys Val Arg Gln Phe Ile Glu Met Val
   210 215 220
   Asn Gly Thr Asp Ser Glu Val Arg Cys Leu Gly Gly Arg Ser Pro Lys
   225 230 235 240
   Ser Gln Asp Ser Tyr Pro Val Ser Pro Arg Pro Phe Ser Ser Pro Ser
   245 250 255
   Met Ser Pro Ser His Gly Met Asn Ile His Asn Leu Ala Ser Gly Lys
   260 265 270
   Gly Ser Thr Ala His Phe Ser Gly Phe Glu Ser Cys Ser Asn Gly Val
   275 280 285
   Ile Ser Asn Lys Ala His Gln Ser Tyr Cys His Ser Asn Lys His Gln
   290 295 300
   Ser Ser Asn Leu Asn Val Pro Glu Leu Asn Ser Ile Asn Met Ser Arg
   305 310 315 320
   Ser Gln Gln Val Asn Asn Phe Thr Ser Asn Asp Val Asp Met Glu Thr
   325 330 335
   Asp His Tyr Ser Asn Gly Val Gly Glu Thr Ser Ser Asn Gly Phe Leu
   340 345 350
   Asn Gly Ser Ser Lys His Asp His Glu Met Glu Asp Cys Asp Thr Glu
   355 360 365
   Met Glu Val Asp Ser Ser Gln Leu Arg Arg Gln Leu Cys Gly Gly Ser
   370 375 380
   Gln Ala Ala Ile Glu Arg Met Ile His Phe Gly Arg Glu Leu Gln Ala
   385 390 395 400
   Met Ser Glu Gln Leu Arg Arg Asp Cys Gly Lys Asn Thr Ala Asn Lys
   405 410 415
   Lys Met Leu Lys Asp Ala Phe Ser Leu Leu Ala Tyr Ser Asp Pro Trp
   420 425 430
   Asn Ser Pro Val Gly Asn Gln Leu Asp Pro Ile Gln Arg Glu Pro Val
   435 440 445
   Cys Ser Ala Leu Asn Ser Ala Ile Leu Glu Thr His Asn Leu Pro Lys
   450 455 460
   Gln Pro Pro Leu Ala Leu Ala Met Gly Gln Ala Thr Gln Cys Leu Gly
   465 470 475 480
   Leu Met Ala Arg Ser Gly Ile Gly Ser Cys Ala Phe Ala Thr Val Glu
   485 490 495
   Asp Tyr Leu His
   500
<210> 104
   <211> 387
   <212> PRT
   <213> Homo sapiens
<400> 104
   Met Ala Thr Ser Gly Val Leu Pro Gly Gly Gly Phe Val Ala Ser Ala
   1               5                   10                  15
   Ala Ala Val Ala Gly Pro Glu Met Gln Thr Gly Arg Asn Asn Phe Val
               20                  25                  30
   Ile Arg Arg Asn Pro Ala Asp Pro Gln Arg Ile Pro Ser Asn Pro Ser
           35                  40                  45
   His Arg Ile Gln Cys Ala Ala Gly Tyr Glu Gln Ser Glu His Asn Val
       50                  55                  60
   Cys Gln Asp Ile Asp Glu Cys Thr Ala Gly Thr His Asn Cys Arg Ala
   65                  70                  75                  80
   Asp Gln Val Cys Ile Asn Leu Arg Gly Ser Phe Ala Cys Gln Cys Pro
                   85                  90                  95
   Pro Gly Tyr Gln Lys Arg Gly Glu Gln Cys Val Asp Ile Asp Glu Cys
               100                 105                 110
   Thr Ile Pro Pro Tyr Cys His Gln Arg Cys Val Asn Thr Pro Gly Ser
           115                 120                 125
   Phe Tyr Cys Gln Cys Ser Pro Gly Phe Gln Leu Ala Ala Asn Asn Tyr
       130                 135                 140
   Thr Cys Val Asp Ile Asn Glu Cys Asp Ala Ser Asn Gln Cys Ala Gln
   145                 150                 155                 160
   Gln Cys Tyr Asn Ile Leu Gly Ser Phe Ile Cys Gln Cys Asn Gln Gly
                   165                 170                 175
   Tyr Glu Leu Ser Ser Asp Arg Leu Asn Cys Glu Asp Ile Asp Glu Cys
               180                 185                 190
   Arg Thr Ser Ser Tyr Leu Cys Gln Tyr Gln Cys Val Asn Glu Pro Gly
           195                 200                 205
   Lys Phe Ser Cys Met Cys Pro Gln Gly Tyr Gln Val Val Arg Ser Arg
       210                 215                 220
   Thr Cys Gln Asp Ile Asn Glu Cys Glu Thr Thr Asn Glu Cys Arg Glu
   225                 230                 235                 240
   Asp Glu Met Cys Trp Asn Tyr His Gly Gly Phe Arg Cys Tyr Pro Arg
                   245                 250                 255
   Asn Pro Cys Gln Asp Pro Tyr Ile Leu Thr Pro Glu Asn Arg Cys Val
               260                 265                 270
   Cys Pro Val Ser Asn Ala Met Cys Arg Glu Leu Pro Gln Ser Ile Val
           275                 280                 285
   Tyr Lys Tyr Met Ser Ile Arg Ser Asp Arg Ser Val Pro Ser Asp Ile
       290                 295                 300
   Phe Gln Ile Gln Ala Thr Thr Ile Tyr Ala Asn Thr Ile Asn Thr Phe
   305                 310                 315                 320
   Arg Ile Lys Ser Gly Asn Glu Asn Gly Glu Phe Tyr Leu Arg Gln Thr
                   325                 330                 335
   Ser Pro Val Ser Ala Met Leu Val Leu Val Lys Ser Leu Ser Gly Pro
               340                 345                 350
   Arg Glu His Ile Val Asp Leu Glu Met Leu Thr Val Ser Ser Ile Gly
           355                 360                 365
   Thr Phe Arg Thr Ser Ser Val Leu Arg Leu Thr Ile Ile Val Gly Pro
       370                 375                 380
   Phe Ser Phe
   385
<210> 105
   <211> 531
   <212> PRT
   <213> Homo sapiens
<400> 105
<210> 106
   <211> 480
   <212> PRT
   <213> Homo sapiens
<400> 106
   Met Ala Ala Arg Cys Ser Thr Arg Trp Leu Leu Val Val Val Gly Thr
   1 5 10 15
   Pro Arg Leu Pro Ala Ile Ser Gly Arg Gly Ala Arg Pro Pro Arg Glu
   20 25 30
   Gly Val Val Gly Ala Trp Leu Ser Arg Lys Leu Ser Val Pro Ala Phe
   35 40 45
   Ala Ser Ser Leu Thr Ser Cys Gly Pro Arg Ala Leu Leu Thr Leu Arg
   50 55 60
   Pro Gly Val Ser Leu Thr Gly Thr Lys His Asn Pro Phe Ile Cys Thr
   65 70 75 80
   Ala Ser Phe His Thr Ser Ala Pro Leu Ala Lys Glu Asp Tyr Tyr Gln
   85 90 95
   Ile Leu Gly Val Pro Arg Asn Ala Ser Gln Lys Glu Ile Lys Lys Ala
               100                 105                 110
   Tyr Tyr Gln Leu Ala Lys Lys Tyr His Pro Asp Thr Asn Lys Asp Asp
           115                 120                 125
   Pro Lys Ala Lys Glu Lys Phe Ser Gln Leu Ala Glu Ala Tyr Glu Val
       130                 135                 140
   Leu Ser Asp Glu Val Lys Arg Lys Gln Tyr Asp Ala Tyr Gly Ser Ala
   145                 150                 155                 160
   Gly Phe Asp Pro Gly Ala Ser Gly Ser Gln His Ser Tyr Trp Lys Gly
                   165                 170                 175
   Gly Pro Thr Val Asp Pro Glu Glu Leu Phe Arg Lys Ile Phe Gly Glu
               180                 185                 190
   Phe Ser Ser Ser Ser Phe Gly Asp Phe Gln Thr Val Phe Asp Gln Pro
           195                 200                 205
   Gln Glu Tyr Phe Met Glu Leu Thr Phe Asn Gln Ala Ala Lys Gly Val
       210                 215                 220
   Asn Lys Glu Phe Thr Val Asn Ile Met Asp Thr Cys Glu Arg Cys Asn
   225                 230                 235                 240
   Gly Lys Gly Asn Glu Pro Gly Thr Lys Val Gln His Cys His Tyr Cys
                   245                 250                 255
   Gly Gly Ser Gly Met Glu Thr Ile Asn Thr Gly Pro Phe Val Met Arg
               260                 265                 270
   Ser Thr Cys Arg Arg Cys Gly Gly Arg Gly Ser Ile Ile Ile Ser Pro
           275                 280                 285
   Cys Val Val Cys Arg Gly Ala Gly Gln Ala Lys Gln Lys Lys Arg Val
       290                 295                 300
   Met Ile Pro Val Pro Ala Gly Val Glu Asp Gly Gln Thr Val Arg Met
   305                 310                 315                 320
   Pro Val Gly Lys Arg Glu Ile Phe Ile Thr Phe Arg Val Gln Lys Ser
                   325                 330                 335
   Pro Val Phe Arg Arg Asp Gly Ala Asp Ile His Ser Asp Leu Phe Ile
               340                 345                 350
   Ser Ile Ala Gln Ala Leu Leu Gly Gly Thr Ala Arg Ala Gln Gly Leu
           355                 360                 365
   Tyr Glu Thr Ile Asn Val Thr Ile Pro Pro Gly Thr Gln Thr Asp Gln
       370                 375                 380
   Lys Ile Arg Met Gly Gly Lys Gly Ile Pro Arg Ile Asn Ser Tyr Gly
   385                 390                 395                 400
   Tyr Gly Asp His Tyr Ile His Ile Lys Ile Arg Val Pro Lys Arg Leu
                   405                 410                 415
   Thr Ser Arg Gln Gln Ser Leu Ile Leu Ser Tyr Ala Glu Asp Glu Thr
               420                 425                 430
   Asp Val Glu Gly Thr Val Asn Gly Val Thr Leu Thr Ser Ser Gly Gly
           435                 440                 445
   Ser Thr Met Asp Ser Ser Ala Gly Ser Lys Ala Arg Arg Glu Ala Gly
       450                 455                 460
   Glu Asp Glu Glu Gly Phe Leu Ser Lys Leu Lys Lys Met Phe Thr Ser
   465                 470                 475                 480
<210> 107
   <211> 572
   <212> PRT
   <213> Homo sapiens
<400> 107
   Met Ala Ala Pro Arg Pro Ser Pro Ala Ile Ser Val Ser Val Ser Ala
    1               5                  10                  15
   Pro Ala Phe Tyr Ala Pro Gln Lys Lys Phe Gly Pro Val Val Ala Pro
               20                  25                  30
   Lys Pro Lys Val Asn Pro Phe Arg Pro Gly Asp Ser Glu Pro Pro Pro
           35                  40                  45
   Ala Pro Gly Ala Gln Arg Ala Gln Met Gly Arg Val Gly Glu Ile Pro
       50                  55                  60
   Pro Pro Pro Pro Glu Asp Phe Pro Leu Pro Pro Pro Pro Leu Ala Gly
   65                  70                  75                  80
   Asp Gly Asp Asp Ala Glu Gly Ala Leu Gly Gly Ala Phe Pro Pro Pro
                   85                  90                  95
   Pro Pro Pro Ile Glu Glu Ser Phe Pro Pro Ala Pro Leu Glu Glu Glu
               100                 105                 110
   Ile Phe Pro Ser Pro Pro Pro Pro Pro Glu Glu Glu Gly Gly Pro Glu
           115                 120                 125
   Ala Pro Ile Pro Pro Pro Pro Gln Pro Arg Glu Lys Val Ser Ser Ile
       130                 135                 140
   Asp Leu Glu Ile Asp Ser Leu Ser Ser Leu Leu Asp Asp Met Thr Lys
   145                 150                 155                 160
   Asn Asp Pro Phe Lys Ala Arg Val Ser Ser Gly Tyr Val Pro Pro Pro
                   165                 170                 175
   Val Ala Thr Pro Phe Ser Ser Lys Ser Ser Thr Lys Pro Ala Ala Gly
               180                 185                 190
   Gly Thr Ala Pro Leu Pro Pro Trp Lys Ser Pro Ser Ser Ser Gln Pro
           195                 200                 205
   Leu Pro Gln Val Pro Ala Pro Ala Gln Ser Gln Thr Gln Phe His Val
       210                 215                 220
   Gln Pro Gln Pro Gln Pro Lys Pro Gln Val Gln Leu His Val Gln Ser
   225                 230                 235                 240
   Gln Thr Gln Pro Val Ser Leu Ala Asn Thr Gln Pro Arg Gly Pro Pro
                   245                 250                 255
   Ala Ser Ser Pro Ala Pro Ala Pro Lys Phe Ser Pro Val Thr Pro Lys
               260                 265                 270
   Phe Thr Pro Val Ala Ser Lys Phe Ser Pro Gly Ala Pro Gly Gly Ser
           275                 280                 285
   Gly Ser Gln Pro Asn Gln Lys Leu Gly His Pro Glu Ala Leu Ser Ala
       290                 295                 300
   Gly Thr Gly Ser Pro Gln Pro Pro Ser Phe Thr Tyr Ala Gln Gln Arg
   305                 310                 315                 320
   Glu Lys Pro Arg Val Gln Glu Lys Gln His Pro Val Pro Pro Pro Ala
                   325                 330                 335
   Gln Asn Gln Asn Gln Val Arg Ser Pro Gly Ala Pro Gly Pro Leu Thr
               340                 345                 350
   Leu Lys Glu Val Glu Glu Leu Glu Gln Leu Thr Gln Gln Leu Met Gln
           355                 360                 365
   Asp Met Glu His Pro Gln Arg Gln Asn Val Ala Val Asn Glu Leu Cys
       370                 375                 380
   Gly Arg Cys His Gln Pro Leu Ala Arg Ala Gln Pro Ala Val Arg Ala
   385                 390                 395                 400
   Leu Gly Gln Leu Phe His Ile Ala Cys Phe Thr Cys His Gln Cys Ala
                   405                 410                 415
   Gln Gln Leu Gln Gly Gln Gln Phe Tyr Ser Leu Glu Gly Ala Pro Tyr
   420 425 430
   Cys Glu Gly Cys Tyr Thr Asp Thr Leu Glu Lys Cys Asn Thr Cys Gly
   435 440 445
   Glu Pro Ile Thr Asp Arg Met Leu Arg Ala Thr Gly Lys Ala Tyr His
   450 455 460
   Pro His Cys Phe Thr Cys Val Val Cys Ala Arg Pro Leu Glu Gly Thr
   465 470 475 480
   Ser Phe Ile Val Asp Gln Ala Asn Arg Pro His Cys Val Pro Asp Tyr
   485 490 495
   His Lys Gln Tyr Ala Pro Arg Cys Ser Val Cys Ser Glu Pro Ile Met
   500 505 510
   Pro Glu Pro Gly Arg Asp Glu Thr Val Arg Val Val Ala Leu Asp Lys
   515 520 525
   Asn Phe His Met Lys Cys Tyr Lys Cys Glu Asp Cys Gly Lys Pro Leu
   530 535 540
   Ser Ile Glu Ala Asp Asp Asn Gly Cys Phe Pro Leu Asp Gly His Val
   545 550 555 560
   Leu Cys Arg Lys Cys His Thr Ala Arg Ala Gln Thr
   565 570
<210> 108
   <211> 2861
   <212> PRT
   <213> Homo sapiens
<400> 108
   Met Lys Ala Met Asp Val Leu Pro Ile Leu Lys Glu Lys Val Ala Tyr
   1 5 10 15
   Leu Ser Gly Gly Arg Asp Lys Arg Gly Gly Pro Ile Leu Thr Phe Pro
   20 25 30
   Ala Arg Ser Asn His Asp Arg Ile Arg Gln Glu Asp Leu Arg Arg Leu
   35 40 45
   Ile Ser Tyr Leu Ala Cys Ile Pro Ser Glu Glu Val Cys Lys Arg Gly
   50 55 60
   Phe Thr Val Ile Val Asp Met Arg Gly Ser Lys Trp Asp Ser Ile Lys
   65 70 75 80
   Pro Leu Leu Lys Ile Leu Gln Glu Ser Phe Pro Cys Cys Ile His Val
   85 90 95
   Ala Leu Ile Ile Lys Pro Asp Asn Phe Trp Gln Lys Gln Arg Thr Asn
   100 105 110
   Phe Gly Ser Ser Lys Phe Glu Phe Glu Thr Asn Met Val Ser Leu Glu
   115 120 125
   Gly Leu Thr Lys Val Val Asp Pro Ser Gln Leu Thr Pro Glu Phe Asp
   130 135 140
   Gly Cys Leu Glu Tyr Asn His Glu Glu Trp Ile Glu Ile Arg Val Ala
   145 150 155 160
   Phe Glu Asp Tyr Ile Ser Asn Ala Thr His Met Leu Ser Arg Leu Glu
   165 170 175
   Glu Leu Gln Asp Ile Leu Ala Lys Lys Glu Leu Pro Gln Asp Leu Glu
   180 185 190
   Gly Ala Arg Asn Met Ile Glu Glu His Ser Gln Leu Lys Lys Lys Val
   195 200 205
   Ile Lys Ala Pro Ile Glu Asp Leu Asp Leu Glu Gly Gln Lys Leu Leu
   210 215 220
   Gln Arg Ile Gln Ser Ser Glu Ser Phe Pro Lys Lys Asn Ser Gly Ser
   225 230 235 240
   Gly Asn Ala Asp Leu Gln Asn Leu Leu Pro Lys Val Ser Thr Met Leu
   245 250 255
   Asp Arg Leu His Ser Thr Arg Gln His Leu His Gln Met Trp His Val
   260 265 270
   Arg Lys Leu Lys Leu Asp Gln Cys Phe Gln Leu Arg Leu Phe Glu Gln
   275 280 285
   Asp Ala Glu Lys Met Phe Asp Trp Ile Thr His Asn Lys Gly Leu Phe
   290 295 300
   Leu Asn Ser Tyr Thr Glu Ile Gly Thr Ser His Pro His Ala Met Glu
   305 310 315 320
   Leu Gln Thr Gln His Asn His Phe Ala Met Asn Cys Met Asn Val Tyr
   325 330 335
   Val Asn Ile Asn Arg Ile Met Ser Val Ala Asn Arg Leu Val Glu Ser
   340 345 350
   Gly His Tyr Ala Ser Gln Gln Ile Arg Gln Ile Ala Ser Gln Leu Glu
   355 360 365
   Gln Glu Trp Lys Ala Phe Ala Ala Ala Leu Asp Glu Arg Ser Thr Leu
   370 375 380
   Leu Asp Met Ser Ser Ile Phe His Gln Lys Ala Glu Lys Tyr Met Ser
   385 390 395 400
   Asn Val Asp Ser Trp Cys Lys Ala Cys Gly Glu Val Asp Leu Pro Ser
   405 410 415
   Glu Leu Gln Asp Leu Glu Asp Ala Ile His His His Gln Gly Ile Tyr
   420 425 430
   Glu His Ile Thr Leu Ala Tyr Ser Glu Val Ser Gln Asp Gly Lys Ser
   435 440 445
   Leu Leu Asp Lys Leu Gln Arg Pro Leu Thr Pro Gly Ser Ser Asp Ser
   450 455 460
   Leu Thr Ala Ser Ala Asn Tyr Ser Lys Ala Val His His Val Leu Asp
   465 470 475 480
   Val Ile His Glu Val Leu His His Gln Arg His Val Arg Thr Ile Trp
   485 490 495
   Gln His Arg Lys Val Arg Leu His Gln Arg Leu Gln Leu Cys Val Phe
   500 505 510
   Gln Gln Glu Val Gln Gln Val Leu Asp Trp Ile Glu Asn His Gly Glu
   515 520 525
   Ala Phe Leu Ser Lys His Thr Gly Val Gly Lys Ser Leu His Arg Ala
   530 535 540
   Arg Ala Leu Gln Lys Arg His Glu Asp Phe Glu Glu Val Ala Gln Asn
   545 550 555 560
   Thr Tyr Thr Asn Ala Asp Lys Leu Leu Glu Ala Ala Glu Gln Leu Ala
   565 570 575
   Gln Thr Gly Glu Cys Asp Pro Glu Glu Ile Tyr Gln Ala Ala His Gln
   580 585 590
   Leu Glu Asp Arg Ile Gln Asp Phe Val Arg Arg Val Glu Gln Arg Lys
   595 600 605
   Ile Leu Leu Asp Met Ser Val Ser Phe His Thr His Val Lys Glu Leu
   610 615 620
   Trp Thr Trp Leu Glu Glu Leu Gln Lys Glu Leu Leu Asp Asp Val Tyr
   625 630 635 640
   Ala Glu Ser Val Glu Ala Val Gln Asp Leu Ile Lys Arg Phe Gly Gln
   645 650 655
   Gln Gln Gln Thr Thr Leu Gln Val Thr Val Asn Val Ile Lys Glu Gly
   660 665 670
   Glu Asp Leu Ile Gln Gln Leu Arg Asp Ser Ala Ile Ser Ser Asn Lys
   675 680 685
   Thr Pro His Asn Ser Ser Ile Asn His Ile Glu Thr Val Leu Gln Gln
   690 695 700
   Leu Asp Glu Ala Gln Ser Gln Met Glu Glu Leu Phe Gln Glu Arg Lys
   705 710 715 720
   Ile Lys Leu Glu Leu Phe Leu His Val Arg Ile Phe Glu Arg Asp Ala
   725 730 735
   Ile Asp Ile Ile Ser Asp Leu Glu Ser Trp Asn Asp Glu Leu Ser Gln
   740 745 750
   Gln Met Asn Asp Phe Asp Thr Glu Asp Leu Thr Ile Ala Glu Gln Arg
   755 760 765
   Leu Gln His His Ala Asp Lys Ala Leu Thr Met Asn Asn Leu Thr Phe
   770 775 780
   Asp Val Ile His Gln Gly Gln Asp Leu Leu Gln Tyr Val Asn Glu Val
   785 790 795 800
   Gln Ala Ser Gly Val Glu Leu Leu Cys Asp Arg Asp Val Asp Met Ala
   805 810 815
   Thr Arg Val Gln Asp Leu Leu Glu Phe Leu His Glu Lys Gln Gln Glu
   820 825 830
   Leu Asp Leu Ala Ala Glu Gln His Arg Lys His Leu Glu Gln Cys Val
   835 840 845
   Gln Leu Arg His Leu Gln Ala Glu Val Lys Gln Val Leu Gly Trp Ile
   850 855 860
   Arg Asn Gly Glu Ser Met Leu Asn Ala Gly Leu Ile Thr Ala Ser Ser
   865 870 875 880
   Leu Gln Glu Ala Glu Gln Leu Gln Arg Glu His Glu Gln Phe Gln His
   885 890 895
   Ala Ile Glu Lys Thr His Gln Ser Ala Leu Gln Val Gln Gln Lys Ala
   900 905 910
   Glu Ala Met Leu Gln Ala Asn His Tyr Asp Met Asp Met Ile Arg Asp
   915 920 925
   Cys Ala Glu Lys Val Ala Ser His Trp Gln Gln Leu Met Leu Lys Met
   930 935 940
   Glu Asp Arg Leu Lys Leu Val Asn Ala Ser Val Ala Phe Tyr Lys Thr
   945 950 955 960
   Ser Glu Gln Val Cys Ser Val Leu Glu Ser Leu Glu Gln Glu Tyr Lys
   965 970 975
   Arg Glu Glu Asp Trp Cys Gly Gly Ala Asp Lys Leu Gly Pro Asn Ser
   980 985 990
   Glu Thr Asp His Val Thr Pro Met Ile Ser Lys His Leu Glu Gln Lys
   995 1000 1005
   Glu Ala Phe Leu Lys Ala Cys Thr Leu Ala Arg Arg Asn Ala Asp Val
   1010 1015 1020
   Phe Leu Lys Tyr Leu His Arg Asn Ser Val Asn Met Pro Gly Met Val
   1025 1030 1035 1040
   Thr His Ile Lys Ala Pro Glu Gln Gln Val Lys Asn Ile Leu Asn Glu
   1045 1050 1055
   Leu Phe Gln Arg Glu Asn Arg Val Leu His Tyr Trp Thr Met Arg Lys
   1060 1065 1070
   Arg Arg Leu Asp Gln Cys Gln Gln Tyr Val Val Phe Glu Arg Ser Ala
   1075 1080 1085
   Lys Gln Ala Leu Glu Trp Ile His Asp Asn Gly Glu Phe Tyr Leu Ser
   1090 1095 1100
   Thr His Thr Ser Thr Gly Ser Ser Ile Gln His Thr Gln Glu Leu Leu
   1105 1110 1115 1120
   Lys Glu His Glu Glu Phe Gln Ile Thr Ala Lys Gln Thr Lys Glu Arg
   1125 1130 1135
   Val Lys Leu Leu Ile Gln Leu Ala Asp Gly Phe Cys Glu Lys Gly His
   1140 1145 1150
   Ala His Ala Ala Glu Ile Lys Lys Cys Val Thr Ala Val Asp Lys Arg
   1155 1160 1165
   Tyr Arg Asp Phe Ser Leu Arg Met Glu Lys Tyr Arg Thr Ser Leu Glu
   1170 1175 1180
   Lys Ala Leu Gly Ile Ser Ser Asp Ser Asn Lys Ser Ser Lys Ser Leu
   1185 1190 1195 1200
   Gln Leu Asp Ile Ile Pro Ala Ser Ile Pro Gly Ser Glu Val Lys Leu
   1205 1210 1215
   Arg Asp Ala Ala His Glu Leu Asn Glu Glu Lys Arg Lys Ser Ala Arg
   1220 1225 1230
   Arg Lys Glu Phe Ile Met Ala Glu Leu Ile Gln Thr Glu Lys Ala Tyr
   1235 1240 1245
   Val Arg Asp Leu Arg Glu Cys Met Asp Thr Tyr Leu Trp Glu Met Thr
   1250 1255 1260
   Ser Gly Val Glu Glu Ile Pro Pro Gly Ile Val Asn Lys Glu Leu Ile
   1265 1270 1275 1280
   Ile Phe Gly Asn Met Gln Glu Ile Tyr Glu Phe His Asn Asn Ile Phe
   1285 1290 1295
   Leu Lys Glu Leu Glu Lys Tyr Glu Gln Leu Pro Glu Asp Val Gly His
   1300 1305 1310
   Cys Phe Val Thr Trp Ala Asp Lys Phe Gln Met Tyr Val Thr Tyr Cys
   1315 1320 1325
   Lys Asn Lys Pro Asp Ser Thr Gln Leu Ile Leu Glu His Ala Gly Ser
   1330 1335 1340
   Tyr Phe Asp Glu Ile Gln Gln Arg His Gly Leu Ala Asn Ser Ile Ser
   1345 1350 1355 1360
   Ser Tyr Leu Ile Lys Pro Val Gln Arg Ile Thr Lys Tyr Gln Leu Leu
   1365 1370 1375
   Leu Lys Glu Leu Leu Thr Cys Cys Glu Glu Gly Lys Gly Glu Ile Lys
   1380 1385 1390
   Asp Gly Leu Glu Val Met Leu Ser Val Pro Lys Arg Ala Asn Asp Ala
   1395 1400 1405
   Met His Leu Ser Met Leu Glu Gly Phe Asp Glu Asn Ile Glu Ser Gln
   1410 1415 1420
   Gly Glu Leu Ile Leu Gln Glu Ser Phe Gln Val Trp Asp Pro Lys Thr
   1425 1430 1435 1440
   Leu Ile Arg Lys Gly Arg Glu Arg His Leu Phe Leu Phe Glu Met Ser
   1445 1450 1455
   Leu Val Phe Ser Lys Glu Val Lys Asp Ser Ser Gly Arg Ser Lys Tyr
   1460 1465 1470
   Leu Tyr Lys Ser Lys Leu Phe Thr Ser Glu Leu Gly Val Thr Glu His
   1475 1480 1485
   Val Glu Gly Asp Pro Cys Lys Phe Ala Leu Trp Val Gly Arg Thr Pro
   1490 1495 1500
   Thr Ser Asp Asn Lys Ile Val Leu Lys Ala Ser Ser Ile Glu Asn Lys
   1505 1510 1515 1520
   Gln Asp Trp Ile Lys His Ile Arg Glu Val Ile Gln Glu Arg Thr Ile
   1525 1530 1535
   His Leu Lys Gly Ala Leu Lys Glu Pro Ile His Ile Pro Lys Thr Ala
   1540 1545 1550
   Pro Ala Thr Arg Gln Lys Gly Arg Arg Asp Gly Glu Asp Leu Asp Ser
   1555 1560 1565
   Gln Gly Asp Gly Ser Ser Gln Pro Asp Thr Ile Ser Ile Ala Ser Arg
   1570 1575 1580
   Thr Ser Gln Asn Thr Leu Asp Ser Asp Lys Leu Ser Gly Gly Cys Glu
   1585 1590 1595 1600
   Leu Thr Val Val Ile His Asp Phe Thr Ala Cys Asn Ser Asn Glu Leu
   1605 1610 1615
   Thr Ile Arg Arg Gly Gln Thr Val Glu Val Leu Glu Arg Pro His Asp
   1620 1625 1630
   Lys Pro Asp Trp Cys Leu Val Arg Thr Thr Asp Arg Ser Pro Ala Ala
   1635 1640 1645
   Glu Gly Leu Val Pro Cys Gly Ser Leu Cys Ile Ala His Ser Arg Ser
   1650 1655 1660
   Ser Met Glu Met Glu Gly Ile Phe Asn His Lys Asp Ser Leu Ser Val
   1665 1670 1675 1680
   Ser Ser Asn Asp Ala Ser Pro Pro Ala Ser Val Ala Ser Leu Gln Pro
   1685 1690 1695
   His Met Ile Gly Ala Gln Ser Ser Pro Gly Pro Lys Arg Pro Gly Asn
   1700 1705 1710
   Thr Leu Arg Lys Trp Leu Thr Ser Pro Val Arg Arg Leu Ser Ser Gly
   1715 1720 1725
   Lys Ala Asp Gly His Val Lys Lys Leu Ala His Lys His Lys Lys Ser
   1730 1735 1740
   Arg Glu Val Arg Lys Ser Ala Asp Ala Gly Ser Gln Lys Asp Ser Asp
   1745 1750 1755 1760
   Asp Ser Ala Ala Thr Pro Gln Asp Glu Thr Val Glu Glu Arg Gly Arg
   1765 1770 1775
   Asn Glu Gly Leu Ser Ser Gly Thr Leu Ser Lys Ser Ser Ser Ser Gly
   1780 1785 1790
   Met Gln Ser Cys Gly Glu Glu Glu Gly Glu Glu Gly Ala Asp Ala Val
   1795 1800 1805
   Pro Leu Pro Pro Pro Met Ala Ile Gln Gln His Ser Leu Leu Gln Pro
   1810 1815 1820
   Asp Ser Gln Asp Asp Lys Ala Ser Ser Arg Leu Leu Val Arg Pro Thr
   1825 1830 1835 1840
   Ser Ser Glu Thr Pro Ser Ala Ala Glu Leu Val Ser Ala Ile Glu Glu
   1845 1850 1855
   Leu Val Lys Ser Lys Met Ala Leu Glu Asp Arg Pro Ser Ser Leu Leu
   1860 1865 1870
   Val Asp Gln Gly Asp Ser Ser Ser Pro Ser Phe Asn Pro Ser Asp Asn
   1875 1880 1885
   Ser Leu Leu Ser Ser Ser Ser Pro Ile Asp Glu Met Glu Glu Arg Lys
   1890 1895 1900
   Ser Ser Ser Leu Lys Arg Arg His Tyr Val Leu Gln Glu Leu Val Glu
   1905 1910 1915 1920
   Thr Glu Arg Asp Tyr Val Arg Asp Leu Gly Tyr Val Val Glu Gly Tyr
   1925 1930 1935
   Met Ala Leu Met Lys Glu Asp Gly Val Pro Asp Asp Met Lys Gly Lys
   1940 1945 1950
   Asp Lys Ile Val Phe Gly Asn Ile His Gln Ile Tyr Asp Trp His Arg
   1955 1960 1965
   Asp Phe Phe Leu Gly Glu Leu Glu Lys Cys Leu Glu Asp Pro Glu Lys
   1970 1975 1980
   Leu Gly Ser Leu Phe Val Lys His Glu Arg Arg Leu His Met Tyr Ile
   1985 1990 1995 2000
   Ala Tyr Cys Gln Asn Lys Pro Lys Ser Glu His Ile Val Ser Glu Tyr
   2005 2010 2015
   Ile Asp Thr Phe Phe Glu Asp Leu Lys Gln Arg Leu Gly His Arg Leu
   2020 2025 2030
   Gln Leu Thr Asp Leu Leu Ile Lys Pro Val Gln Arg Ile Met Lys Tyr
   2035 2040 2045
   Gln Leu Leu Leu Lys Asp Phe Leu Lys Tyr Ser Lys Lys Ala Ser Leu
   2050 2055 2060
   Asp Thr Ser Glu Leu Glu Arg Ala Val Glu Val Met Cys Ile Val Pro
   2065 2070 2075 2080
   Arg Arg Cys Asn Asp Met Met Asn Val Gly Arg Leu Gln Gly Phe Asp
   2085 2090 2095
   Gly Lys Ile Val Ala Gln Gly Lys Leu Leu Leu Gln Asp Thr Phe Leu
   2100 2105 2110
   Val Thr Asp Gln Asp Ala Gly Leu Leu Pro Arg Cys Arg Glu Arg Arg
   2115 2120 2125
   Ile Phe Leu Phe Glu Gln Ile Val Ile Phe Ser Glu Pro Leu Asp Lys
   2130 2135 2140
   Lys Lys Gly Phe Ser Met Pro Gly Phe Leu Phe Lys Asn Ser Ile Lys
   2145 2150 2155 2160
   Val Ser Cys Leu Cys Leu Glu Glu Asn Val Glu Asn Asp Pro Cys Lys
   2165 2170 2175
   Phe Ala Leu Thr Ser Arg Thr Gly Asp Val Val Glu Thr Phe Ile Leu
   2180 2185 2190
   His Ser Ser Ser Pro Ser Val Arg Gln Thr Trp Ile His Glu Ile Asn
   2195 2200 2205
   Gln Ile Leu Glu Asn Gln Arg Asn Phe Leu Asn Ala Leu Thr Ser Pro
   2210 2215 2220
   Ile Glu Tyr Gln Arg Asn His Ser Gly Gly Gly Gly Gly Gly Gly Ser
   2225 2230 2235 2240
   Gly Ala Ala Ala Gly Val Gly Ala Ala Ala Ala Ala Gly Pro Pro Val
   2245 2250 2255
   Ala Ala Ala Ala Thr Val Ala Ala Pro Ala Ala Ala Ala Ala Pro Pro
   2260 2265 2270
   Ala Arg Ala Gly Ala Gly Pro Pro Gly Ser Pro Ser Leu Ser Asp Thr
   2275 2280 2285
   Thr Pro Pro Cys Trp Ser Pro Leu Gln Pro Arg Ala Arg Gln Arg Gln
   2290 2295 2300
   Thr Arg Cys Gln Ser Glu Ser Ser Ser Ser Ser Asn Ile Ser Thr Met
   2305 2310 2315 2320
   Leu Val Thr His Asp Tyr Thr Ala Val Lys Glu Asp Glu Ile Asn Val
   2325 2330 2335
   Tyr Gln Gly Glu Val Val Gln Ile Leu Ala Ser Asn Gln Gln Asn Met
   2340 2345 2350
   Phe Leu Val Phe Arg Ala Ala Thr Asp Gln Cys Pro Ala Ala Glu Gly
   2355 2360 2365
   Trp Ile Pro Gly Phe Val Leu Gly His Thr Ser Ala Val Ile Val Glu
   2370 2375 2380
   Asn Pro Asp Gly Thr Leu Lys Lys Ser Thr Ser Trp His Thr Ala Leu
   2385 2390 2395 2400
   Arg Leu Arg Lys Lys Ser Glu Lys Lys Asp Lys Asp Gly Lys Arg Glu
   2405 2410 2415
   Gly Lys Leu Glu Asn Gly Tyr Arg Lys Ser Arg Glu Gly Leu Ser Asn
   2420 2425 2430
   Lys Val Ser Val Lys Leu Leu Asn Pro Asn Tyr Ile Tyr Asp Val Pro
   2435 2440 2445
   Pro Glu Phe Val Ile Pro Leu Ser Glu Val Thr Cys Glu Thr Gly Glu
   2450 2455 2460
   Thr Val Val Leu Arg Cys Arg Val Cys Gly Arg Pro Lys Ala Ser Ile
   2465 2470 2475 2480
   Thr Trp Lys Gly Pro Glu His Asn Thr Leu Asn Asn Asp Gly His Tyr
   2485 2490 2495
   Ser Ile Ser Tyr Ser Asp Leu Gly Glu Ala Thr Leu Lys Ile Val Gly
   2500 2505 2510
   Val Thr Thr Glu Asp Asp Gly Ile Tyr Thr Cys Ile Ala Val Asn Asp
   2515 2520 2525
   Met Gly Ser Ala Ser Ser Ser Ala Ser Leu Arg Val Leu Gly Pro Gly
   2530 2535 2540
   Met Asp Gly Ile Met Val Thr Trp Lys Asp Asn Phe Asp Ser Phe Tyr
   2545 2550 2555 2560
   Ser Glu Val Ala Glu Leu Gly Arg Gly Arg Phe Ser Val Val Lys Lys
   2565 2570 2575
   Cys Asp Gln Lys Gly Thr Lys Arg Ala Val Ala Thr Lys Phe Val Asn
   2580 2585 2590
   Lys Lys Leu Met Lys Arg Asp Gln Val Thr His Glu Leu Gly Ile Leu
   2595 2600 2605
   Gln Ser Leu Gln His Pro Leu Leu Val Gly Leu Leu Asp Thr Phe Glu
   2610 2615 2620
   Thr Pro Thr Ser Tyr Ile Leu Val Leu Glu Met Ala Asp Gln Gly Arg
   2625 2630 2635 2640
   Leu Leu Asp Cys Val Val Arg Trp Gly Ser Leu Thr Glu Gly Lys Ile
   2645 2650 2655
   Arg Ala His Leu Gly Glu Val Leu Glu Ala Val Arg Tyr Leu His Asn
   2660 2665 2670
   Cys Arg Ile Ala His Leu Asp Leu Lys Pro Glu Asn Ile Leu Val Asp
   2675 2680 2685
   Glu Ser Leu Ala Lys Pro Thr Ile Lys Leu Ala Asp Phe Gly Asp Ala
   2690 2695 2700
   Val Gln Leu Asn Thr Thr Tyr Tyr Ile His Gln Leu Leu Gly Asn Pro
   2705 2710 2715 2720
   Glu Phe Ala Ala Pro Glu Ile Ile Leu Gly Asn Pro Val Ser Leu Thr
   2725 2730 2735
   Ser Asp Thr Trp Ser Val Gly Val Leu Thr Tyr Val Leu Leu Ser Gly
   2740 2745 2750
   Val Ser Pro Phe Leu Asp Asp Ser Val Glu Glu Thr Cys Leu Asn Ile
   2755 2760 2765
   Cys Arg Leu Asp Phe Ser Phe Pro Asp Asp Tyr Phe Lys Gly Val Ser
   2770 2775 2780
   Gln Lys Ala Lys Glu Phe Val Cys Phe Leu Leu Gln Glu Asp Pro Ala
   2785 2790 2795 2800
   Lys Arg Pro Ser Ala Ala Leu Ala Leu Gln Glu Gln Trp Leu Gln Ala
   2805 2810 2815
   Gly Asn Gly Arg Ser Thr Gly Val Leu Asp Thr Ser Arg Leu Thr Ser
   2820 2825 2830
   Phe Ile Glu Arg Arg Lys His Gln Asn Asp Val Arg Pro Ile Arg Ser
   2835 2840 2845
   Ile Lys Asn Phe Leu Gln Ser Arg Leu Leu Pro Arg Val
   2850 2855 2860
<210> 109
   <211> 271
   <212> PRT
   <213> Homo sapiens
<400> 109
   Met Val Leu Ile Lys Glu Phe Arg Val Val Leu Pro Cys Ser Val Gln
   1 5 10 15
   Glu Tyr Gln Val Gly Gln Leu Tyr Ser Val Ala Glu Ala Ser Lys Asn
   20 25 30
   Glu Thr Gly Gly Gly Glu Gly Ile Glu Val Leu Lys Asn Glu Pro Tyr
   35 40 45
   Glu Lys Asp Gly Glu Lys Gly Gln Tyr Thr His Lys Ile Tyr His Leu
   50 55 60
   Lys Ser Lys Val Pro Ala Phe Val Arg Met Ile Ala Pro Glu Gly Ser
   65 70 75 80
   Leu Val Phe His Glu Lys Ala Trp Asn Ala Tyr Pro Tyr Cys Arg Thr
   85 90 95
   Ile Val Thr Asn Glu Tyr Met Lys Asp Asp Phe Phe Ile Lys Ile Glu
   100 105 110
   Thr Trp His Lys Pro Asp Leu Gly Thr Leu Glu Asn Val His Gly Leu
   115 120 125
   Asp Pro Asn Thr Trp Lys Thr Val Glu Ile Val His Ile Asp Ile Ala
   130 135 140
   Asp Arg Ser Gln Val Glu Pro Ala Asp Tyr Lys Ala Asp Glu Asp Pro
   145 150 155 160
   Ala Leu Phe Gln Ser Val Lys Thr Lys Arg Gly Pro Leu Gly Pro Asn
   165 170 175
   Trp Lys Lys Glu Leu Ala Asn Ser Pro Asp Cys Pro Gln Met Cys Ala
   180 185 190
   Tyr Lys Leu Val Thr Ile Lys Phe Lys Trp Trp Gly Leu Gln Ser Lys
   195 200 205
   Val Glu Asn Phe Ile Gln Lys Gln Glu Lys Arg Ile Phe Thr Asn Phe
   210 215 220
   His Arg Gln Leu Phe Cys Trp Ile Asp Lys Trp Ile Asp Leu Thr Met
   225 230 235 240
   Glu Asp Ile Arg Arg Met Glu Asp Glu Thr Gln Lys Glu Leu Glu Thr
   245 250 255
   Met Arg Lys Arg Gly Ser Val Arg Gly Thr Ser Ala Ala Asp Val
   260 265 270